# EUROPEAN PATENT APPLICATION

(11) **EP 4 067 487 A1**
(43) Date of publication of application: **05.10.2022**
(21) Application number: 21166740.7
(22) Date of filing: 01.04.2021
(51) Int. Cl.: C12N 9/88, C12N 9/90, C12P 5/00

(54) **CHONDROITIN-PRODUCING RECOMBINANT CELL**

(71) Applicant: Givaudan SA, 1214 Vernier (CH)
(72) Inventor: JAILLARDON, Karine, 91400 ORSAY (FR); THOMAS, Dominique, 95100 Argenteuil (FR); MERKAMM, Muriel, 91400 Orsay (FR); LOUIS, Dominique, 91400 Orsay (FR)
(74) Representative: Nony

(57) **Abstract**

The present invention relates to the field of bio-production of chondroitin.

There is a need in the art for chondroitin production methods allowing its highly efficient synthesis and secretion.

The solution proposed in the present invention is the use of a recombinant cell, in particular a recombinant yeast, comprising many modifications as described in the present text.

The present invention further proposes methods allowing the bio-production of chondroitin using the recombinant cell, in particular a recombinant yeast, of the invention.

## Description

### FIELD OF THE INVENTION

The present invention relates to the field of bio-production of chondroitin.

### BACKGROUND OF THE INVENTION

Chondroitin is a high molecular weight polysaccharide that occurs naturally in the connective tissues of people and animals. Chondroitin belongs to the family of heteropolysaccharides called glycosaminoglycans.

Glycosaminoglycans (GAGs) or mucopolysaccharides are unbranched, negatively charged polysaccharide chains composed of repeating disaccharide units including an acidic sugar and an amino sugar (N-acetylglucosamine or N-acetylgalactosamine). Because of their inflexible nature and high negative charge, GAGs are are used in the body as a lubricant or shock absorber. The cartilage matrix lining the knee joint, for example, which is rich in GAGs, can support pressures of hundreds of atmospheres by this mechanism.

Chondroitin, which is formed of D-glucuronic acid and of N-acetyl-D-galactosamine, may be sulfated. Chondroitin sulfate is important for maintenance of cartilage strength and resilience and is marketed as a nutritional supplement to reduce joint pain and promote healthy cartilage and joint function. Clinical studies support the use of chondroitin and chondroitin sulfate for treatment of osteoarthritis.

Chondroitin sulfate or chondroitin being naturally present in the extracellular matrix of conjonctive tissus of the skin, it is also used for cosmetic application, in particular to moisturize, heal and soothe the skin or as a component of hair conditioner.

Currently, chondroitin is mainly produced by extraction from cartilage of animals using chemical and enzymatic treatments to dissociate the polysaccharide from the protein and produce a polysaccharide product of varying quality. However, these methods can be laborious and expensive.

As such, microbial production of chondroitin has been suggested. Indeed, certain bacteria produce chondroitin and chondroitin-like polysaccharide polymers as a component of their capsule. However, these known bacteria, such as Pasteurella multocida or Escherichia coli, are pathogens to many mammals and produce low quantities of the polysaccharides.

As a consequence, research has turned towards recombinant microorganisms, and in particular recombinant bacteria, for the production of chondroitin. Mention may be made for example of WO2011109438, of EP2142643, or of US20090263867.

Contrary to other microorganisms that are commonly used for the production of biological molecules, yeasts are generally recognised as safe. They can grow rapidly and be cultivated at higher density, compared to bacteria, and do not require an aseptic environment. Furthermore, yeast cells can be more easily separated from the culture medium than bacteria, greatly simplifying the process for product extraction and purification. Finally, yeasts present the advantage of being more resistant to pH changes in the culture medium, and as such represent stronger fermentation systems.

However, among yeasts, distinguishing features between species may also present certain challenges. This is mainly due to the difference in metabolisms between the species and their culture conditions.

However, the inventors have found that *Saccharomyces cerevisiae* is particularly useful as a tool for the production of molecules of interest since it has a long safe history when it comes to being used by humans (such as in wine, beer, or bread) and as such is a well established model whose genetic information is well known in the art. *S*. *cerevisiae* further presents the advantage of being generally recognised as safe for humans and animals. What's more, the acidification of the medium which occurs when cultivating this yeast reduces the possibility of contamination of the bio fermenters, and therefore reduces the need to add antibiotics to the medium. Finally, many genetic tools have been developed allowing for stable modifications of its genome (integration within the chromosome).

Accordingly, there is still a need in the art for further chondroitin production methods allowing its highly efficient synthesis and secretion. In particular, there is still a need to provide production methods that are cost-efficient and supply chondrotin which is safe for human application.

In a particular case, there is still a need in the art for chondrotin production methods allowing to obtain large amounts of chondroitin of a particular and controlled size.

### SUMMARY OF THE INVENTION

The present invention accordingly relates to the following items.
**Item 1:** A recombinant yeast cell producing chondroitin wherein the recombinant yeast cell comprises:
   (a) one or more recombinant nucleic acids encoding a polypeptide having a chondroitin synthase (HCOS) activity; and
   (b) one or more recombinant nucleic acids encoding a polypeptide having UDP-Glucose dehydrogenase (UDP-GlcDH or HASB) activity; and
   (c) one or more recombinant nucleic acids encoding a polypeptide having a UDP-glucose-4-epimerase (kfoA or GNE1) activity.
   As illustrated in the examples, the recombinant yeasts of the invention allow for the production of chondroitin in a yeast cell which is not naturally able to produce this glycosaminoglycan. It is further demonstrated in the examples that the size of the chondroitin produced by the recombinant yeast may be controlled.
   Said advantageous properties can be further increased by recombining the yeast with additional modifications described here-after.
**Item 2**: The recombinant according to item 1, wherein the recombinant cell comprises one or more recombinant nucleic acids encoding a polypeptide having a chondroitinase activity wherein the polypeptide having chondroitinase activity comprises a secretion signal, and optionally an anchoring signal.
**Item 3**: A recombinant host cell producing chondroitin wherein the recombinant host cell comprises:
   (a) one or more recombinant nucleic acids encoding a polypeptide having a chondroitin synthase (HCOS) activity; and
   (b) one or more recombinant nucleic acids encoding a polypeptide having UDP-Glucose dehydrogenase (UDP-GlcDH or HASB) activity;
   (c) one or more recombinant nucleic acids encoding a polypeptide having a UDP-glucose-4-epimerase (kfoA or GNE1) activity; and
   (d) one or more recombinant nucleic acids encoding a polypeptide having a chondroitinase activity wherein the polypeptide having chondroitinase activity comprises a secretion signal or comprises a secretion signal, and optionally an anchoring signal, so that chondroitin, in particular of a desired molecular weight is produced by the host cell.
**Item 4:** The recombinant host cell according to item 2 or 3, wherein the recombinant nucleic acid encoding a polypeptide having a chondroitinase activity is obtained or derived from at least one of *Cupiennius salei, Tityus serrulatus, Bos taurus, Vespa magnifica, Macaca mulata or Apis mellifera,* and preferably from *Tityus serrulatus.*
**Item 5**: The recombinant cell according to any one of items 2 to 4, wherein the molecular weight of the chondroitin is in the range of less than 50kDa, preferably is in the range of about 20kDa to about 50kDa.
**Item 6**: The recombinant cell according to any one of items 1 to 4, wherein the molecular weight of the chondroitin is in the range of greater than 50kDa, preferably is in the range of about 50kDa to about 250kDa.
**Item 7**: The recombinant cell according to any one of items 1 to 4, wherein the molecular weight of the chondroitin is in the range of greater than 100kDa, preferably is in the range of about 100kDa to about 1500kDa.
**Item 8**: The recombinant cell according to any one of items 1 to 7, wherein the nucleic acid encoding a polypeptide having a UDP-Glucose dehydrogenase (UDP-GlcDH or HASB) activity is obtained or derived from at least one of *Arabidopsis thaliana, Chlorella virus PBCV1* or *Streptococcus zooepidemicus,* and in particular from at least one of *Arabidopsis thaliana* or *Chlorella virus PBCV1.*
**Item 9**: The recombinant cell according to any one of items 1 to 8, wherein the nucleic acid encoding a polypeptide having a chondroitin synthase (HCOS) activity is
   (i) a nucleic acid encoding a chondroitin synthase; or
   (ii) a nucleic acid encoding a chimeric polypeptide having a chondroitin synthase activity.
**Item 10:** The recombinant cell according to item 9, wherein the nucleic acids are,obtained or derived from at least one of *Pasteurella multocida, Chlorella virus PBCV1, Mycobacterium tuberculosis, Homo sapiens, Escherichia coli, Saccharomyces cerevisiae* or *Penicillium oxalicum.*
**Item 11**: The recombinant cell according to any one of items 1 to 10, wherein the nucleic acid encoding a polypeptide having a UDP-glucose-4-epimerase (kfoA or GNE1) activity is obtained or derived from a bacteria, and in particular from a bacteria selected from the group consisting of *Pseudomonas aeruginosa, Pasteurella multocida,* and *Escherichia coli.*
**Item 12:** The recombinant cell according to any one of items 1 to 11, wherein the recombinant cell further comprises at least one recombinant nucleic acid encoding one or more of:
   (i) a polypeptide having glutamine-fructose-6-phosphate amidotransferase (GFA1) activity; and/or
   (ii) a polypeptide having UDP-N-acetylglucosamine pyrophosphorylase (QRI1) activity.
**Item 13**: The recombinant cell according to any one of items 1 to 12, wherein the recombinant cell further comprises at least one recombinant nucleic acid encoding one or more of:
   (i) a polypeptide having Phosphoglucomutase-1 (PGM1) activity; and/or
   (ii) a polypeptide having UTP-glucose-1-phosphate uridylyltransferase (UGP1) activity; and/or
   (iii) a polypeptide having Glucosamine-6-phosphate N-acetyltransferase (GNA1) activity; and/or
   (iv) a polypeptide having phosphoacetylglucosamine mutase (PCM1) activity.
**Item 14:** The recombinant cell according to item 12 or 13, wherein:
   - the nucleic acid encoding the polypeptide having a glutamine-fructose-6-phosphate amidotransferase (GFA1) activity;
   - the nucleic acid encoding the polypeptide having a UDP-N-acetylglucosamine pyrophosphorylase (QRI1) activity;
   - the nucleic acid encoding the polypeptide having a Phosphoglucomutase-1 (PGM1) activity;
   - the nucleic acid encoding the polypeptide having a UTP-glucose-1-phosphate uridylyltransferase (UGP1) activity;
   - the nucleic acid encoding the polypeptide having a Glucosamine-6-phosphate N-acetyltransferase (GNA1) activity; and/or
   - the nucleic acid encoding the polypeptide having phosphoacetylglucosamine mutase (PCM1);
   are obtained or derived from *Saccharomyces cerevisiae.*
**Item 15:** The recombinant host cell according to any one of items 3 to 14, wherein the recombinant host cell is a yeast.
**Item 16:** The recombinant cell according to any one of items 1 to 15, wherein the recombinant cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and in particular belongs to the *Saccharomyces* genus.
**Item 17**: The recombinant cell according to item 16, wherein the recombinant cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*
**Item 18**: A method of producing chondroitin of a desired molecular weight comprising:
   (a) cultivating a recombinant cell as defined in any one of items 1 to 17 in a cultivation medium for a time sufficient to produce chondroitin of the desired molecular weight; and
   (b) optionally isolating or recovering the chondroitinfrom the recombinant cell and/or from the cultivation medium.
**Item 19**: The method of item 18, wherein the chondroitin has a molecular weight of from about 20kDa to about 50kDa.
**Item 20**: The method of item 18, wherein chondroitin has a molecular weight of from about 50kDa to about 150 kDa.
**Item 21**: The method of item 18, wherein chondroitin has a molecular weight of from about 150kDa to about 1500 kDa.
**Item 22**: The method according to any one of items 18 to 21, wherein the recombinant cell is a yeast belonging to the *Saccharomyces* genus, and in particular is *Saccharomyces cerevisiae.*
**Item 23**: The method according to any one of items 18 to 22, wherein the time sufficient to produce chondroitin of a desired molecule weight is a period of from about 35 hours to about 50 hours, preferably from about 40 hours to about 50 hours, preferably is about 48 hours.
**Item 24**: The method according to any one of items 18 to 23, wherein wherein the molecular weight of the produced chondroitin is controlled by regulating the pH of the cultivation medium during step (a).
**Item 25**: The method according to any one of items 18 to 24, wherein the method is carried out on an industrial scale, preferably where the cultivation medium is at least about 100L, more preferably is in the range of about 1,000L to about 3,000L, even more preferably is about 10,000L or even more preferably is about 100, 000L, or even is about 250,000L.
**Item 26**: Chondroitin obtainable from a recombinant cell according to any one of items 1 to 17, or from the method of according to any one of items 18 to 25.
**Item 27**: A cultivation medium comprising the chondroitin according to item 26.
**Item 28**: A composition comprising the chondroitin according to item 26.
**Item 29**: An industrial product or a consumer product or a consumable comprising (i) the chondroitin having a molecular weight as defined in any one of items 5 to 7, (ii) the cultivation medium of item 27 or (iii) the composition of item 28.
**Item 30**: The industrial product or the consumer product or the consumable of item 29, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product, a dental and/or an oral hygiene composition.
**Item 31**: Use of a recombinant cell as defined in any one of items 2 to 17 for the production of chondroitin having a molecular weight in the range of from about 20kDa to about 50k Da or from about 50kDa to about 1000 kDa.
**Item 32**: A method for producing chondrotin, comprising the steps of:
   (a) culturing a recombinant yeast as defined in any one of items 1 to 17 in a cultivation medium; and
   (b) recovering the chondroitin from said cultivation medium,
   wherein the chondrotin recovered in step (b) has a molecular weight controlled through the selection of:
   - the nature and origin of the nucleic acid encoding a polypeptide having a chondroitinase activity of the recombinant yeast,
   - the nature and origin of the promoter controlling the expression of the recombinant nucleic acid encoding encoding a polypeptide having a chondroitinase activity of the recombinant yeast,
   - the presence of an anchoring and/or of a secretion signal associated to the polypeptide having a chondroitinase activity of the recombinant yeast,
   - the pH of the cultivation medium during the step of culturing the recombinant yeast, and/or
   - the duration of the culturing of the recombinant yeast.

Certain embodiments of the present invention may provide one or more of the following advantages:
- a process for producing chondroitin of controlled molecular weight;
- a process for producing chondroitin of controlled molecular weight in a *Saccharomyces* yeast cell; and
- a process for producing chondrotin of controlled molecular weight by varying genetic parameters (eg regulatory sequences) and/or process parameters (pH or fermentation time

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE FIGURES

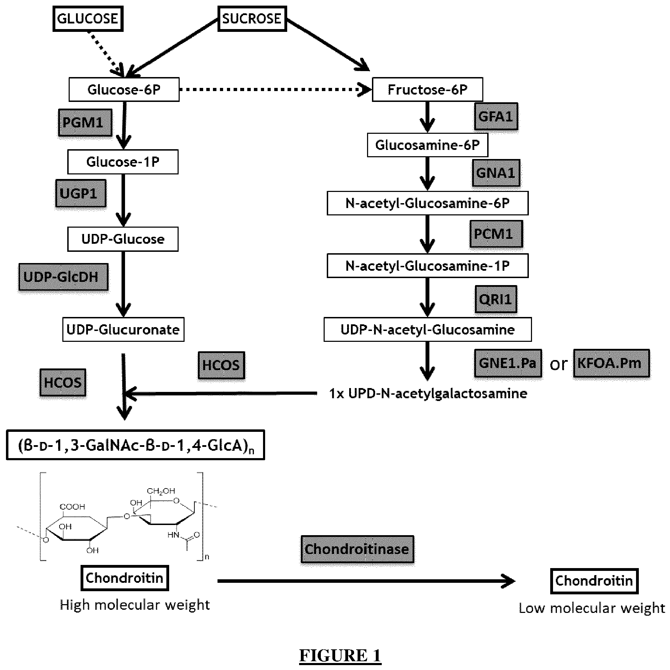

**Figure 1** shows a schematic pathway for the production of chondroitin

### SUMMARY OF THE SEQUENCES

**SEQ ID NO: 1** is a reencoded nucleic acid sequence of a polypeptide having a chondroitin synthase activity : chimeric nucleic acid comprising a fragment from a nucleic acid encoding hyaluronan synthase originating from *Pasteurella multocida* and a fragment from a nucleic acid encoding a chondroitin synthase from *Pasteurella multocida* (HCOS1-1)
**SEQ ID NO: 2** is a reencoded nucleic acid sequence of a polypeptide having a chondroitin synthase activity : chimeric nucleic acid comprising a fragment from a nucleic acid encoding a hyaluronan synthase originating from *Pasteurella multocida* and a fragment from a nucleic acid encoding a chondroitin synthase from *Pasteurella multocida* (HCOS 1-2)
**SEQ ID NO: 3** is a reencoded nucleic acid sequence of a polypeptide having a chondroitin synthase activity : chimeric nucleic acid comprising a fragment from a nucleic acid encoding a hyaluronan synthase originating from *Pasteurella multocida* and a fragment from a nucleic acid encoding a chondroitin synthase from *Pasteurella multocida* (HCOS 1-3)
**SEQ ID NO: 4**: is a nucleic acid sequence of a polypeptide having chondroitin synthase activity: chimeric nucleic acid comprising a fragment from a nucleic acid encoding a hyaluronan synthase originating from *Pasteurella multocida,* a fragment from a nucleic acid encoding a chdroitin synthase from *Pasteurella multocida* and a fragment from a nucleic acid encoding a chitin synthase 2 from *Saccharomyces cerevisiae* (HCOS.Sc)
**SEQ ID NO: 5:** is a nucleic acid sequence of a polypeptide having chondroitin synthase activity: chimeric nucleic acid comprising a fragment from a nucleic acid encoding a hyaluronan synthase originating from *Pasteurella multocida* and a fragment from a nucleic acid encoding a chitin synthase 2 from *Saccharomyces cerevisiae* (HHASA.Sc)
**SEQ ID NO: 6:** is a nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Escherichia coli* (HCOS1-Vir)
**SEQ ID NO: 7:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Escherichia coli* (HCOS2-Vir)
**SEQ ID NO: 8:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a Galactofuranosyltransferase from *Mycobacterium tuberculosis* (HCOS3-Vir)
**SEQ ID NO: 9:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Escherichia coli* (HCOS4-Vir)
**SEQ ID NO: 10:** is a nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Penicillium oxalicum* (HCOS5-Vir)
**SEQ ID NO: 11**: is a nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Pasteurella multocida* (HCOS6-Vir)
**SEQ ID NO: 12:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Pasteurella multocida* (HCOS7-Vir)
**SEQ ID NO: 13:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin synthase from *Pasteurella multocida* (HCOS8-Vir)
**SEQ ID NO: 14:** is a nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin sulfate synthase from *Homo sapiens* (HCOS9-Vir)
**SEQ ID NO: 15:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin sulfate synthase from *Homo sapiens* (HCOS10-Vir)
**SEQ ID NO: 16:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin sulfate synthase from *Homo sapiens* (HCOS 11-Vir)
**SEQ ID NO: 17:** is a reencoded nucleic acid sequence of a polypeptide having chondroitin synthase activity: chimeric nucleic acid comprising a fragment from a nucleic acid encoding a hyaluronan synthase originating from *Chlorella virus PBCV-1* and a fragment from a nucleic acid encoding a chondroitin sulfate synthase from *Homo sapiens* (HCOS12-Vir)
**SEQ ID NO: 18** is an amino acid sequence of a polypeptide having a chondroitin synthase activity : chimeric amino acid comprising a fragment from an amino acid encoding a hyaluronan synthase originating from *Pasteurella multocida* and a fragment from an amino acid encoding a chondroitin synthase from *Pasteurella multocida* (HCOS1-1)
**SEQ ID NO: 19** is an amino acid sequence of a polypeptide having a chondroitin synthase activity : chimeric amino acid comprising a fragment from an amino acid encoding a hyaluronan synthase originating from *Pasteurella multocida* and a fragment from an amino acid encoding a chondroitin synthase from *Pasteurella multocida* (HCOS 1-2)
**SEQ ID NO: 20** is an amino acid sequence of a polypeptide having a chondroitin synthase activity : chimeric amino acid comprising a fragment from an amino acid encoding a hyaluronan synthase originating from *Pasteurella multocida* and a fragment from an amino acid encoding a chondroitin synthase from *Pasteurella multocida* (HCOS 1-3)
**SEQ ID NO: 21:** is an amino acid sequence of a polypeptide having chondroitin synthase activity: chimeric amino acid comprising a fragment from an amino acid encoding a hyaluronan synthase originating from *Pasteurella multocida,* a fragment from an amino acid encoding a chdroitin synthase from *Pasteurella multocida* and a fragment from an amino acid encoding a chitin synthase 2 from *Saccharomyces cerevisiae* (HCOS.Sc)
**SEQ ID NO: 22:** is a nucleic acid sequence of a polypeptide having chondroitin synthase activity: chimeric amino acid comprising a fragment from an amino acid encoding a hyaluronan synthase originating from *Pasteurella multocida* and a fragment from an amino acid encoding a chitin synthase 2 from *Saccharomyces cerevisiae* (HHASA.Sc)
**SEQ ID NO: 23:** is an amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Escherichia coli* (HCOS 1-Vir)
**SEQ ID NO: 24:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Escherichia coli* (HCOS2-Vir)
**SEQ ID NO: 25:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Escherichia coli* (HCOS3-Vir)
**SEQ ID NO: 26:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Escherichia coli* (HCOS4-Vir)
**SEQ ID NO: 27:** is an amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Penicillium oxalicum* (HCOS5-Vir)
**SEQ ID NO: 28:** is an amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Pasteurella multocida* (HCOS6-Vir)
**SEQ ID NO: 29:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Pasteurella multocida* (HCOS7-Vir)
**SEQ ID NO: 30:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin synthase from *Pasteurella multocida* (HCOS8-Vir)
**SEQ ID NO: 31**: is an amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin sulfate synthase from *Homo sapiens* (HCOS9-Vir)
**SEQ ID NO: 32:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin sulfate synthase from *Homo sapiens* (HCOS10-Vir)
**SEQ ID NO: 33:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin sulfate synthase from *Homo sapiens* (HCOS 11-Vir)
**SEQ ID NO: 34:** is a reencoded amino acid sequence of a polypeptide having chondroitin synthase activity: chimeric amino acid comprising a fragment from an amino acid encoding a hyaluronan synthase originating from *Chlorella virus PBCV-1* and a fragment from an amino acid encoding a chondroitin sulfate synthase from *Homo sapiens* (HCOS12-Vir)
**SEQ ID NO: 35** is a nucleic acid sequence of UDP-Glucose dehydrogenase (HASB) originating from *Arabidopsis thaliana*
**SEQ ID NO: 36** is a reencoded nucleic acid sequence of UDP-Glucose dehydrogenase (HASB) originating from *Chlorella virus PBCV-1*
**SEQ ID NO: 37** is a reencoded nucleic acid sequence of UDP-Glucose dehydrogenase (HASB-A) originating from *Chlorella virus PBCV-1*
**SEQ ID NO: 38** is a nucleic acid sequence of UDP-Glucose dehydrogenase (HASB) originating from *Streptococcus zooepidemicus*
**SEQ ID NO: 39** is an amino acid sequence of UDP-Glucose dehydrogenase (HASB) originating from *Arabidopsis thaliana*
**SEQ ID NO: 40** is an amino acid sequence of UDP-Glucose dehydrogenase (HASB) originating from *Chlorella virus PBCV-1*
**SEQ ID NO: 41** is an amino acid sequence of UDP-Glucose dehydrogenase (HASB) originating from *Streptococcus zooepidemicus*
**SEQ ID NO: 42** is a nucleic acid sequence of UDP-glucose-4-epimerase (GNE1) originating from *Pseudomonas aeruginosa*
**SEQ ID NO: 43** is a nucleic acid sequence of UDP-glucose-4-epimerase (KFOA) originating from *Pasteurella multocida*
**SEQ ID NO: 44** is a nucleic acid sequence of UDP-glucose-4-epimerase (KFOA) originating from *Escherichia coli*
**SEQ ID NO: 45** is an amino acid sequence of UDP-glucose-4-epimerase (GNE1) originating from *Pseudomonas aeruginosa*
**SEQ ID NO: 46** is an amino acid sequence of UDP-glucose-4-epimerase (KFOA) originating from *Pasteurella multocida*
**SEQ ID NO: 47** is an amino acid sequence of UDP-glucose-4-epimerase (KFOA) originating from *Escherichia coli*
**SEQ ID NO: 48** is a reencoded nucleic acid sequence of hyaluronidase (HYAL) originating from *Cupiennius salei* with a N-terminal secretion signal
**SEQ ID NO: 49** is a reencoded nucleic acid sequence of hyaluronidase (HYAL) originating from *Tityus serrulatus* with a N-terminal secretion signal
**SEQ ID NO: 50** is a reencoded nucleic acid sequence of hyaluronidase (HYAL) originating from *Bos Taurus* with a N-terminal secretion signal
**SEQ ID NO: 51** is a reencoded nucleic acid sequence of hyaluronidase (HYAL) originating from *Apis mellifera* with a N-terminal secretion signal
**SEQ ID NO: 52** is a reencoded nucleic acid sequence of hyaluronidase (HYAL) originating from *Macaca mulatta* with a N-terminal secretion signal
**SEQ ID NO: 53** is a reencoded nucleic acid sequence of hyaluronidase (HYAL) originating from *Vespa magnifica* with a N-terminal secretion signal
**SEQ ID NO: 54** is a reencoded nucleic acid sequence of hyaluronidase (HYAL) originating from *Cupiennius salei* with a N-terminal secretion signal and a C-terminal anchoring signal
**SEQ ID NO: 55** is a reencoded nucleic acid sequence of hyaluronidase (HYAL) originating from *Tityus serrulatus* with a N-terminal secretion signal and a C-terminal anchoring signal
**SEQ ID NO: 56** is a reencoded nucleic acid sequence of hyaluronidase (HYAL) originating from *Bos taurus* with a N-terminal secretion signal and a C-terminal anchoring signal
**SEQ ID NO: 57** is a reencoded nucleic acid sequence of hyaluronidase (HYAL) originating *from Apis mellifera* with a N-terminal secretion signal and a C-terminal anchoring signal
**SEQ ID NO: 58** is a reencoded nucleic acid sequence of hyaluronidase (HYAL) originating from *Macaca mulatta* with a N-terminal secretion signal and a C-terminal anchoring signal
**SEQ ID NO: 59** is a reencoded nucleic acid sequence of hyaluronidase (HYAL) originating from *Vespa magnifica* with a N-terminal secretion signal and a C-terminal anchoring signal
**SEQ ID NO: 60** is an amino acid sequence of hyaluronidase (HYAL) originating from *Cupiennius salei* with a N-terminal secretion signal
**SEQ ID NO: 61** is an amino acid sequence of hyaluronidase (HYAL) originating from *Tityus serrulatus* with a N-terminal secretion signal
**SEQ ID NO: 62** is an amino acid sequence of hyaluronidase (HYAL) originating from *Bos taurus* with a N-terminal secretion signal
**SEQ ID NO: 63** is an amino acid sequence of hyaluronidase (HYAL) originating from *Apis mellifera* with a N-terminal secretion signal
**SEQ ID NO: 64** is an amino acid sequence of hyaluronidase (HYAL) originating from *Macaca mulatta* with a N-terminal secretion signal
**SEQ ID NO: 65** is an amino acid sequence of hyaluronidase (HYAL) originating from *Vespa magnified* with a N-terminal secretion signal
**SEQ ID NO: 66** is an amino acid sequence of hyaluronidase (HYAL) originating from *Cupiennius salei* with a N-terminal secretion signal and a C-terminal anchoring signal
**SEQ ID NO: 67** is an amino acid sequence of hyaluronidase (HYAL) originating from *Tityus serrulatus* with a N-terminal secretion signal and a C-terminal anchoring signal
**SEQ ID NO: 68** is an amino acid sequence of hyaluronidase (HYAL) originating from *Bos taurus* with a N-terminal secretion signal and a C-terminal anchoring signal
**SEQ ID NO: 69** is an amino acid sequence of hyaluronidase (HYAL) originating from *Apis mellifera* with a N-terminal secretion signal and a C-terminal anchoring signal
**SEQ ID NO: 70** is an amino acid sequence of hyaluronidase (HYAL) originating from *Macaca mulatta* with a N-terminal secretion signal and a C-terminal anchoring signal
**SEQ ID NO: 71** is an amino acid sequence of hyaluronidase (HYAL) originating from *Vespa magnifica* with a N-terminal secretion signal and a C-terminal anchoring signal
**SEQ ID NO: 72** is a nucleic acid sequence of glutamine-fructose-6-phosphate amidotransferase (GFA1) originating from *Saccharomyces cerevisiae*
**SEQ ID NO: 73** is a reencoded nucleic acid sequence of glutamine-fructose-6-phosphate amidotransferase (GFA1) originating from *Chlorella virus 1 (PBCV-1)*
**SEQ ID NO: 74** is a reencoded nucleic acid sequence of glutamine-fructose-6-phosphate amidotransferase (GFA1) originating from *Chlorella virus 1 (PBCV-1)*
**SEQ ID NO: 75** is an amino acid sequence of glutamine-fructose-6-phosphate amidotransferase (GFA1) originating from *Saccharomyces cerevisiae*
**SEQ ID NO: 76** is an amino acid sequence of glutamine-fructose-6-phosphate amidotransferase (GFA1) originating from *Chlorella virus 1 (PBCV-1)*
**SEQ ID NO: 77** is a nucleic acid sequence of UDP-N-acetylglucosamine pyrophosphorylase (QRI1) originating from *Saccharomyces cerevisiae*
**SEQ ID NO: 78** is an amino acid sequence of UDP-N-acetylglucosamine pyrophosphorylase (QRI1) originating from *Saccharomyces cerevisiae*
**SEQ ID NO: 79** is a nucleic acid sequence of Phosphoglucomutase-1 (PGM1) originating from *Saccharomyces cerevisiae*
**SEQ ID NO: 80** is an amino acid sequence of Phosphoglucomutase-1 (PGM1) originating from *Saccharomyces cerevisiae*
**SEQ ID NO: 81** is a nucleic acid sequence of UTP-glucose-1-phosphate uridylyltransferase (UGP1) originating from *Saccharomyces cerevisiae*
**SEQ ID NO: 82** is an amino acid sequence of UTP-glucose-1-phosphate uridylyltransferase (UGP1) originating from *Saccharomyces cerevisiae*
**SEQ ID NO: 83** is a nucleic acid sequence of Glucosamine 6-phosphate N-acetyltransferase (GNA1) originating from *Saccharomyces cerevisiae*
**SEQ ID NO: 84** is an amino acid sequence of Glucosamine 6-phosphate N-acetyltransferase (GNA1) originating from *Saccharomyces cerevisiae*
**SEQ ID NO: 85** is a nucleic acid sequence of phosphoacetylglucosamine mutase (PCM1) originating from *Saccharomyces cerevisiae*
**SEQ ID NO: 86** is an amino acid sequence of phosphoacetylglucosamine mutase (PCM1) originating from *Saccharomyces cerevisiae*
**SEQ ID NO: 87** is the nucleic acid sequence of promoter pTDH3
**SEQ ID NO: 88** is the nucleic acid sequence of promoter pTDH3.Sk
**SEQ ID NO: 89** is the nucleic acid sequence of promoter pTDH3-1.sba
**SEQ ID NO: 90** is the nucleic acid sequence of promoter pTDH3-1.Sar
**SEQ ID NO: 91** is the nucleic acid sequence of promoter pENO2
**SEQ ID NO: 92** is the nucleic acid sequence of promoter pTEF3
**SEQ ID NO: 93** is the nucleic acid sequence of promoter pTEF1
**SEQ ID NO: 94** is the nucleic acid sequence of promoter pTEF1.Ago
**SEQ ID NO: 95** is the nucleic acid sequence of promoter pTEF1.Sba
**SEQ ID NO: 96** is the nucleic acid sequence of promoter pPDC1
**SEQ ID NO: 97** is the nucleic acid sequence of promoter pCCW12
**SEQ ID NO: 98** is the nucleic acid sequence of promoter pCCW12.Sm
**SEQ ID NO: 99** is the nucleic acid sequence of promoter pCCW12.Sk
**SEQ ID NO: 100** is the nucleic acid sequence of promoter pCCW12.Sba
**SEQ ID NO: 101** is the nucleic acid sequence of promoter pCCW12.Sar
**SEQ ID NO: 102** is the nucleic acid sequence of promoter pNUP57
**SEQ ID NO: 103** is the nucleic acid sequence of promoter pCCW10.Ago
**SEQ ID NO: 104** is the nucleic acid sequence of promoter pCWP2
**SEQ ID NO: 105** is the nucleic acid sequence of promoter pFBA1
**SEQ ID NO: 106** is the nucleic acid sequence of promoter pCCW120.Sm
**SEQ ID NO: 107** is the nucleic acid sequence of promoter pCUP1
**SEQ ID NO: 108** is the nucleic acid sequence of promoter pMET6
**SEQ ID NO: 109** is the nucleic acid sequence of promoter pMET25
**SEQ ID NO: 110** is the nucleic acid sequence of promoter pSAM1
**SEQ ID NO: 111** is the nucleic acid sequence of terminator tTPI1
**SEQ ID NO: 112** is the nucleic acid sequence of terminator tMET25
**SEQ ID NO: 113** is the nucleic acid sequence of terminator tDIT1
**SEQ ID NO: 114** is the nucleic acid sequence of terminator tRPL3
**SEQ ID NO: 115** is the nucleic acid sequence of terminator tRPL3.Sm
**SEQ ID NO: 116** is the nucleic acid sequence of terminator tRPL3.sba
**SEQ ID NO: 117** is the nucleic acid sequence of terminator tRPL41B
**SEQ ID NO: 118** is the nucleic acid sequence of terminator tRPL41B.Sba
**SEQ ID NO: 1119** is the nucleic acid sequence of terminator tRPL15A
**SEQ ID NO: 120** is the nucleic acid sequence of terminator tRPL15A.Sm
**SEQ ID NO: 121** is the nucleic acid sequence of terminator tRPL15A.sba
**SEQ ID NO: 122** is the nucleic acid sequence of terminator tIDP1
**SEQ ID NO: 123** is the nucleic acid sequence of terminator tIDP1.Sba
**SEQ ID NO: 124** is the nucleic acid sequence of terminator tTEF1.sba
**SEQ ID NO: 125** is the nucleic acid sequence of the secretion sequence added in 5'
**SEQ ID NO: 126** is the amino acid sequence of the secretion sequence added in N-ter
**SEQ ID NO: 127** is the nucleic acid sequence of the anchoring sequence added in 3'
**SEQ ID NO: 128** is the amino acid sequence of the anchoring sequence added in C-ter

### DETAILED DESCRIPTION OF THE INVENTION

The inventors have conceived genetically modified cells, and especially genetically modified yeasts, having the ability to produce chondroitin, as compared to the parent cells, and especially as compared to the parent yeasts which are not naturally capable of doing so.

These genetically modified cells, are described throughout the present specification.

### Definitions

The term "chondroitin" is a polysaccharide of N-acetylgalactosamine and glucuronique acid, linked via alternating β-(1→4) and β-(1→3) glycosidic bonds (alternating polymer of β-glucuronic acid-(1→3)-N-acetyl-β-galactosamine-4-sulfate-(1→4)) and having the chemical formula (C₁₄H₂₁NO₁₁)n.

The term "Chondroitin", as used herein, is intended to cover chondroitin and its derivatives, which include but are not limited to chondroitin sulfates, useful for applications in a cosmetic product, flavor product a fragrance product, a food product, a food, a beverage, a texturant, a pharmaceutical composition, a dietary supplement, a nutraceutical, a cleaning product and/or a dental and/or an oral hygiene composition or combinations thereof.

Chondroitin sulfates include but are not limited to Chondroitin-4-sulfate, Chondroitin-6-sulfate, Chondroitin-2,6-sulfate, Chondroitin-4,6-sulfate and/or mixtures thereof.

The site of sulfation of Chondroitin-4-sulfate is carbon 4 of the N-acetylgalactosamine (GalNAc) sugar.

The site of sulfation of Chondroitin-6-sulfate is carbon 6 of the GalNAc sugar.

The site of sulfation of Chondroitin-2,6-sulfate is carbon 2 of the glucuronic acid and 6 of the GalNAc sugar.

The site of sulfation of Chondroitin-4,6-sulfate is carbons 4 and 6 of the GalNAc sugar.

Chondroitin derivatives, such as Chondroitin sulfate, may be useful in alleviating the risk of, reducing, preventing and/or treating osteoarthritis, cartilage deterioration, and osteoarthritis-related joint pain, tenderness, and/or swelling.

In particular, Chondrotin derivatives, such as Chondroitin sulfate, may be useful in alleviating the risk of, reducing, preventing and/or treating osteoarthritis; osteoarthritis-related joint pain, joint tenderness, and joint swelling; joint degeneration; and/or cartilage deterioration.

Chondrotin, in particular Chondroitin sulfate, may be used alone or with glucosamine as a dietary supplement for treatment of osteoarthritis; osteoarthritis-related joint pain, joint tenderness, and joint swelling; joint degeneration; and/or cartilage deterioration.

Chondroitin, in particular Chondroitin sulfate, may be prepared as a composition.

Chondroitin derivatives may be prepared from chondroitin through an appropriate chemical or enzymatic step.

Chondroitin may be produced in a recombinant cell.

As used herein, the term "recombinant" when used in reference to a cell indicates that the cell has been modified by the introduction of an endogenous and/or heterologous nucleic acid or protein into the cell or the alteration of a native cell, or that the cell is derived from a cell so modified. Thus, for example, recombinant cells express genes or nucleic acid that are not found within the native (non-recombinant) form of the cell or express native (eg endogenous) genes at a different level than their native level or express additional or supplementary copies of native (eg endogenous) at a different level than their native level.

As used herein, the term "recombinant", when used in reference to a nucleic acid or vector, are sequences formed/obtained by technics of genetic engineering well known to the man skilled in the art. The guidelines of the US National Institute for Health (NIH) accordingly indicate that:
*"recombinant [*...*] nucleic acids are defined as:*
*i. molecules that (a) are constructed by joining nucleic acid molecules and (b) that can replicate in a living cell, i.e., recombinant nucleic acids;",*
which reflects the conventional use of the word "recombinant" attached to nucleic acid sequences to mean recombined following the insertion of, or joining together with, another nucleic acid.

Proteins that result from the expression of recombinant DNA or recombinant vector within living cells are also termed recombinant proteins.

The term "recombinant" is accordingly synonymous with the term "genetically modified". The term "gene" is synonymous with the term "nucleic acid" or "nucleotide sequence".

A recombinant nucleic acid sequence for use in a recombinant cell, and in particular a recombinant yeast, of the invention may be provided in the form of a nucleic acid construct. The term "nucleic acid construct" refers to as a nucleic acid molecule, either single-or double-stranded, which is isolated or derived from a native (eg endogeneous) naturally occurring gene or is a heterologous nucleic acid or which has been modified to contain segments of nucleic acids which are combined and juxtaposed in a manner which would not otherwise exist in nature. The term "nucleic acid construct" is synonymous with the term "expression cassette" or "heterologous nucleic acid expression cassette" when the nucleic acid construct contains one or more regulatory elements required for expression of a coding sequence, wherein said control sequences are operably linked to said coding sequence. Nonlimiting examples of regulatory elements include promoters, enhancers, silencers, terminators, and poly-A signals.

A recombinant nucleic acid sequence for use in a recombinant cell of the invention may be provided in the form of an expression vector, wherein the polynucleotide sequence is operably linked to at least one control sequence for the expression of the polynucleotide sequence in a recombinant cell.

The terms "obtained from" or "originate from" or "originating from" a microorganism or animal generally means that a substance (e.g., a nucleic acid molecule or polypeptide) originating from the microorganism or animal is native to that microorganism or animal.

The terms "derived from" a microorganism or animal generally means that a substance (e.g., a nucleic acid molecule or polypeptide) derived from the microorganism or animal is the result of modifications brought to the substance native to (i.e. present as such) in that microorganism or animal. For example, regarding a nucleic acid sequence derived from a microorganism or animal, said nucleic acid sequence can correspond to a reencoded and/or truncated version of the native nucleic acid sequence from this microorganism or animal. Other modifications well known to the man skilled in the art can be brought to the native substance of a microorganism or animal leading to the substance used being "derived from" said microorganism or animal.

As used herein, the term "polypeptide" refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

A polypeptide encoded by a recombinant nucleic acid for use in a recombinant cell, and in particular in a recombinant yeast, of the invention may comprise a signal peptide and/or a propeptide sequence. In the event that a polypeptide expressed by a recombinant cell, and in particular a recombinant yeast, of the invention comprises a signal peptide and/or a propeptide, sequence identity may be calculated over the mature polypeptide sequence.

The term "operably linked" as used herein refers to two or more nucleic acid sequence elements that are physically linked and are in a functional relationship with each other. For instance, a promoter is operably linked to a coding sequence if the promoter is able to initiate or regulate the transcription or expression of the coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

The term "native" or "endogenous" as used herein with reference to molecules, and in particular enzymes and nucleic acids, indicates molecules that are expressed in the organism in which they originated or are found in nature.

The term "endogenous gene" means that the gene was present in the cell before any genetic modification, in the wild-type strain. Endogenous genes may be overexpressed by introducing heterologous sequences in addition to, or to replace endogenous regulatory elements, or by introducing one or more additional or supplementary copies of the gene into the chromosome or a plasmid (said additional or supplementary copies being designated "exogenous or heterologous genes" or "heterologous nucleotide sequences" or "heterologous nucleic acids" as defined herein). Endogenous genes may also be modified to modulate their expression and/or activity. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, in the down-regulation and/or attenuation of the activity of the endogenous gene product. Another way to enhance expression of endogenous genes is to introduce one or more additional or supplementary copies of the gene onto the chromosome or a plasmid (said supplementary copies being "exogenous or heterologous genes" or "heterologous nucleotide sequences" or heterologous nucleic acids" as defined here-after).

By "one or more additional or supplementary copies of a gene" according to the invention, it is for example understood in the present invention from 1 to 50 copies, in particular from 1 to 30 copies, more particularly from 1 to 20 copies, and preferably from 1 to 10 copies. Said copies may be inserted into the same locus or into different loci of a recombinant cell of the invention.

The term "exogenous gene" means that the gene was introduced into a cell, by means well known to the man skilled in the art, whereas this gene is or is not naturally occurring in the wild-type cell. Cells can express exogenous genes if these genes are introduced into the cell with all the elements allowing their expression in the cell. Transforming cells with exogenous DNA is a routine task for the man skilled in the art. Exogenous genes may be integrated into the host chromosome, or be expressed extra-chromosomally from plasmids or vectors. A variety of plasmids, which differ with respect to their origin of replication and their copy number in the cell, are all known in the art. The sequence of exogenous genes may be adapted for its expression in the cell. Indeed, the man skilled in the art knows the notion of codon usage bias and how to adapt nucleic sequences for a particular codon usage bias without modifying the deduced protein. In particular embodiments, codon optimized genes express native enzymes.

The term "heterologous gene" or "heterologous nucleic acid sequence" refers to a gene or nucleic acid sequence not normally found in a given cell in nature. As such, a heterologous nucleic acid sequence may be: (a) foreign to its host cell (i.e. is"exogenous" to the cell); (b) naturally found in the host cell (i.e. "endogenous") but present at an unnatural quantity in the cell (i.e., greater or lesser quantity than naturally found in the host cell); or (c) be naturally found in the host cell but positioned outside of its natural locus.

In the present application, all genes are referenced with their common names and with references to their nucleotide sequences and, the case arising, to their amino acid sequences. Using the references given in accession number for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other cells and designing degenerated probes to clone the corresponding gene in another organism.

The man skilled in the art knows different means to modulate, and in particular up-regulate or down-regulate, the expression of endogenous genes. For example, a way to enhance expression of, or overexpress, endogenous genes is to introduce one or more additional or supplementary copies of the gene onto the chromosome or a plasmid.

Another way is to replace the endogenous promoter of a gene with a stronger promoter. These promoters may be homologous or heterologous. Promoters particularly interesting in the present invention are described in more detail elsewhere in the present specification.

The nucleic acid expression construct may further comprise 5' and/or 3' recognition sequences and/or selection markers.

The term "inducible promoter" is used to qualify a promoter whose activity is induced, i.e. increased:
- in the presence of one or more particular metabolite(s). The higher the metabolite concentration in the medium, the stronger the promoter activity; or
- in the presence of a low concentration, or in the absence, of one or more metabolite(s). These metabolites are different from those whose increasing presence induces the activity of the promoter. The lower the metabolite concentration in the medium, the stronger the promoter activity.

The term "repressible promoter" is used to qualify a promoter whose activity is repressed, i.e. reduced:
- in the presence of one or more particular metabolite(s). The higher the metabolite concentration in the medium, the weaker the promoter activity; or
- in the presence of a low concentration, or in the absence, of one or more metabolite(s). These metabolites are different from those whose increasing presence represses the activity of the promoter. The lower the metabolite concentration in the medium, the weaker the promoter activity.

As used herein, the term "anchoring signal" when used in conjunction with a protein or polypeptide such as an enzyme (such as, for example hyaluronidase) means for example a first nucleic acid encoding a protein that is operably linked to a second nucleic acid encoding a protein or a polypeptide, or a first protein or polypeptide that is operably linked to a second protein or polypeptide, such as an enzyme (such as, for example hyaluronidase to form, for example a fusion protein), and that enables the cellular transport machinery of a cell, in particular of a S. cerevisiae cell, to correctly anchor and/or position in the membrane of the cell the second protein operably linked to the first protein.

As used herein, the term "secretion signal" when used in conjunction with a protein or polypeptide such as an enzyme (such as, for example hyaluronidase) means for example a first nucleic acid encoding a peptide or protein that is operably linked to a second nucleic acid encoding a protein, or a first protein that is linked to a second protein, such as an enzyme (such as, for example hyaluronidase to form, for example a fusion protein), and that enables the cellular transport machinery of a cell, in particular of a S. cerevisiae cell, to locate at least the second protein to the membrane of the cell and to secrete the second protein outside of the cell, after the first protein has, for example, been cleaved from the second protein.

As used herein the terms "secretion signal" and "anchoring signal" when used in conjunction with a protein or polypeptide such as an enzyme (such as, for example, hyaluronidase), mean for example a first nucleic acid encoding a peptide or protein that is operably linked to a second nucleic acid encoding a protein, or a first protein that is operably linked to a second protein, such as an enzyme (such as, for example hyaluronidase), and that enables the cellular transport machinery of a cell, in particular of a S. cerevisiae cell, to locate at least the second protein to the membrane of the cell wherein if the second protein is also operably linked to an "anchoring signal" the second protein is not secreted but remains attached to the membrane of the cell. In some cases, a secretion-anchoring signal may provide a dual secretion signal and anchoring signal function.

Sequences of secretion and anchoring signals, methods for the expression, anchoring and/or secretion of heterologous proteins, such as enzymes (such as, for example, hyaluronidase) on the surface of a cell (such, as for example, a yeast cell) are well known in the art (see for example, Ast et al (2013) Cell 152: 1134-1145, Ast and Schuldiner (2013) Crit Rev Biochem Mol Biol 48(3) 273-288, Van der Vaart et al (1997) Applied Environmental Microbiology 63(2) 615-620 and the entire contents of each of these publications are incorporated herein by reference).The "activity" of an enzyme is used interchangeably with the term "function" and designates, in the context of the invention, the capacity of an enzyme to catalyze a desired reaction. The amount of an enzyme in a host cell may be altered by modifying the transcription of the gene that encodes the enzyme. This can be achieved for example by modifying the copy number of the nucleotide sequence encoding the enzyme (e.g., by using a higher or lower copy number expression vector comprising the nucleotide sequence, or by introducing additional copies of the nucleotide sequence into the genome of the host cell or by deleting or disrupting the nucleotide sequence in the genome of the host cell), by changing the order of coding sequences on a polycistronic mRNA of an operon or breaking up an operon into individual genes each with its own control elements, or by increasing the strength of the promoter or operator to which the nucleotide sequence is operably linked.

Alternatively or in addition, the copy number of an enzyme in a host cell may be altered by modifying the level of translation of an mRNA that encodes the enzyme. This can be achieved for example by modifying the stability of the mRNA, modifying the sequence of the ribosome binding site, modifying the distance or sequence between the ribosome binding site and the start codon of the enzyme coding sequence, modifying the entire intercistronic region located "upstream of or adjacent to the 5' side of the start codon of the enzyme coding region, stabilizing the 3'-end of the mRNA transcript using hairpins and specialized sequences, modifying the codon usage of enzyme, altering expression of rare codon tRNAs used in the biosynthesis of the enzyme, and/or increasing the stability of the enzyme, as, for example, via mutation of its coding sequence.

The activity of an enzyme in a host cell can be altered in a number of ways, including, but not limited to, expressing a modified form of the enzyme that exhibits increased or decreased solubility in the host cell, expressing an altered form of the enzyme that lacks a domain through which the activity of the enzyme is inhibited, expressing a modified form of the enzyme that has a higher or lower Kcat or a lower or higher Km for the substrate, or expressing an altered form of the enzyme that is more or less affected by feed back or feed-forward regulation by another molecule in the pathway.The terms "encoding" or "coding for" refer to the process by which a polynucleotide, through the mechanisms of transcription and translation, produces an amino-acid sequence.

The gene(s) encoding the enzyme(s) considered in the present invention can be exogenous or endogenous.

The methods implemented in the present invention preferably require the use of one or more chromosomal integration constructs for the stable introduction of a heterologous nucleotide sequence into a specific location on a chromosome or for the functional disruption of one or more target genes in a genetically modified cell. In some embodiments, disruption of a target gene prevents the expression of the related functional protein. In some embodiments, disruption of a target gene results in the expression of a non-functional protein from the disrupted gene.

Parameters of chromosomal integration constructs that may be varied in the practice of the present invention include, but are not limited to, the lengths of the homologous sequences; the nucleotide sequence of the homologous sequences; the length of the integrating sequence; the nucleotide sequence of the integrating sequence; and the nucleotide sequence of the target locus. In some embodiments, an effective range for the length of each homologous sequence is 20 to 5,000 base pairs, preferentially 50 to 100 base pairs. In particular embodiments, the length of each homologous sequence is about 50 base pairs. For more information on the length of homology required for gene targeting, see D. Burke et al., Methods in yeast Genetics - A cold spring harbor laboratory course Manual (2000).

In some embodiments, (a) disrupted gene(s) in which the above-mentioned DNA construct(s) is/are intended to be inserted may advantageously comprises one or more selectable markers useful for the selection of transformed cells. Preferably, said selectable marker(s) are comprised in the DNA construct(s) according to the present invention.

In some embodiments, the selectable marker is an antibiotic resistance marker. Illustrative examples of antibiotic resistance markers include, but are not limited to the, NAT1, AUR1-C, HPH, DSDA, KAN<R>, and SH BLE gene products. The NAT 1 gene product from S. *noursei* confers resistance to nourseothricin; the AUR1-C gene product from *Saccharomyces cerevisiae* confers resistance to Auerobasidin A (AbA); the HPH gene product of *Klebsiella pneumoniae* confers resistance to Hygromycin B; the DSDA gene product of *E. coli* allows cells to grow on plates with D-serine as the sole nitrogen source; the KAN<R> gene of the Tn903 transposon confers resistance to G418; and the SH BLE gene product from *Streptoalloteichus hindustanus* confers resistance to Zeocin (bleomycin).

In some embodiments, the antibiotic resistance marker is deleted after the genetically modified cell of the invention is isolated. The man skilled in the art is able to choose suitable marker in specific genetic context.

In a particular embodiment, a recombinant cell according to the invention is devoid of any antibiotic resistance marker. This advantageously prevents the necessity to add antibiotics in the selection medium.

In some embodiments, the selectable marker rescues an auxotrophy (e.g., a nutritional auxotrophy) in the genetically modified cell. In such embodiments, a parent cell, and in particular a parent yeast, comprises a functional disruption in one or more gene products that function in an amino acid or nucleotide biosynthetic pathway, such as, for example, the HIS3, LEU2, LYS1, LYS2, MET15, TRP1, ADE2, and URA3 gene products in yeast, which renders the parent cell incapable of growing in media without supplementation with one or more nutrients (auxotrophic phenotype). The auxotrophic phenotype can then be rescued by transforming the parent cell with a chromosomal integration encoding a functional copy of the disrupted gene product (in some embodiments, the functional copy of the gene may originate from close species, such as *Kluveromyces, Candida* etc.), and the genetically modified cell generated can be selected based on the loss of the auxotrophic phenotype of the parent cell.

For each of the nucleic acid sequences comprising a promoter sequence, a coding sequence (e.g. an enzyme coding sequence), or a terminator sequence, reference sequences are described herein. The present description also encompasses nucleic acid sequences having specific percentages of nucleic acid identity with a reference nucleic acid sequence.

For each or the amino acid sequences of interest, reference sequences are described herein. The present description also encompasses amino acid sequences (e.g. enzyme amino acid sequences), having specific percentages of amino acid identity with a reference amino acid sequence.

For obvious reasons, in all the present description, a specific nucleic acid sequence or a specific amino acid sequence which complies with, respectively, the considered nucleotide or amino acid identity, should further lead to obtaining a protein (or enzyme) which displays the desired biological activity. As used herein, the "percentage of identity" between two nucleic acid sequences or between two amino acid sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences.

The terms "sequence homology" or "sequence identity" or "homology" or "identity" are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

The "fermentation" or "culture" is generally conducted in fermenters with an appropriate culture medium adapted to the cell being cultivated, containing at least one simple carbon source, and if necessary co-substrates.

The term "fermentation composition" refers to a composition which comprises genetically modified host cells and products or metabolites produced by the genetically modified host cells. An example of a fermentation composition is a whole cell broth, which can be the entire contents of a vessel (e.g., a flasks, plate, or fermentor), including cells, aqueous phase, and compounds produced from the genetically modified host cells.

The term "medium" refers to a culture medium or cultivation medium or fermentation medium.

For maximal production of chondroitin, the recombinant cells used as production hosts preferably have a high rate of carbohydrate utilization. These characteristics may be conferred by mutagenesis and selection, genetic engineering, or may be natural. Fermentation media, or "culture medium" or "cultivation medium", for the present cells may contain at least about 10 g/L of glucose and/or sucrose. Additional carbon substrates may include but are not limited to monosaccharides such as fructose, mannose, xylose and arabinose; oligosaccharides such as lactose maltose, galactose, or sucrose; polysaccharides such as starch or cellulose or mixtures thereof and unpurified mixtures from renewable feedstocks such as cheese whey permeate cornsteep liquor, sugar beet molasses, and barley malt. Other carbon substrates may include glycerol, acetate and/or ethanol.

Hence, it is contemplated that the source of carbon utilized in the present invention may encompass a wide variety of carbon containing substrates and will only be limited by the choice of cell, and in particular of yeast.

Although it is contemplated that all of the above-mentioned carbon substrates and mixtures thereof are suitable in the present invention, preferred carbon substrates are glucose, fructose, and sucrose, or mixtures of these with C5 sugars such as xylose and/or arabinose for cells, and in particular yeasts, modified to use C5 sugars, and more particularly glucose.

Preferred carbon substrates are glucose or sucrose.

In addition to an appropriate carbon source, fermentation media may contain suitable minerals, salts, cofactors, buffers and other components, known to those skilled in the art, suitable for the growth of the cultures and promotion of the enzymatic pathway necessary for the production of the desired product.

Besides, additional genetic modifications suitable for the growth of recombinant cells according to the invention may be considered.

The terms "Aerobic conditions" refers to concentrations of oxygen in the culture medium that are sufficient for an aerobic or facultative anaerobic cell, and in particular yeast, to use di-oxygene as a terminal electron acceptor.

"Microaerobic condition" refers to a culture medium in which the concentration of oxygen is less than that in air, i.e. oxygen concentration up to 6% O₂.

An "appropriate culture medium" designates a medium (e.g. a sterile, liquid medium) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like. The term "carbon source" or "carbon substrate" or "source of carbon" according to the present invention denotes any source of carbon that can be used by those skilled in the art to support the normal growth of a cell, including hexoses (such as glucose, galactose or lactose), pentoses, monosaccharides, oligosaccharides, disaccharides (such as sucrose, cellobiose or maltose), molasses, starch or its derivatives, cellulose, hemicelluloses and combinations thereof.

Culture mediums that are particularly appropriate to produce recombinant cells of the invention, and in particular recombinant yeasts of the invention, are described further below in the text.By "controlled" molecular weight of a chondroitin of the invention is intended to mean that at least 80%, in particular at least 85%, of the chondroitin produced by a method of the invention has a molecular weight comprised within a certain pre-determined molecular weight's range through the regulation of at least one parameter of the method and/or of the recombinant cell of the invention, such as for example:
- the nature and origin of the nucleic acid encoding the polypeptide having chondroitinase activity of the recombinant cell,
- the nature and origin of the promoter controlling the expression of the nucleic acid encoding the polypeptide having chondroitinase activity of the recombinant cell,
- the presence of an anchoring and/or of a secretion signal associated to the the polypeptide having chondroitinase activity of the recombinant cell,
- the pH of the culture medium during the step of culturing the recombinant cell, and/or
- the duration of the culturing of the recombinant cell.

As used herein, the term "about" refers to a reasonable range about a value as determined by the practitioner of skill. In certain embodiments, the term about refers to ± one, two, or three standard deviations. In certain embodiments, the term about refers to ± 5%, 10%, 20%, or 25%. In certain embodiments, the term about refers to ± 0.1, 0.2, or 0.3 logarithmic units, e.g. pH units.

### General features of genetic modifications introduced according to the invention

- All the genome modifications are inserted in recombinant cells, and in particular recombinant yeasts, according to known genetic engineering techniques:
- The successive nucleic acid sequences included in a gene construct that is introduced in the recombinant cell genome according to the invention are of the following structure:
   Prom₁-ORF₁-term₁-ORF₂-gene₂-term₂ - .../... -Promₙ-ORFₙ-termₙ, wherein:
   - Prom1 is a sequence regulating the expression of the coding sequence ORF1,
   - ORF1 is a nucleic acid sequence encoding a desired protein PROT1, and especially a desired enzyme PROT1,
   - Term1 is a transcription terminator sequence that mediates transcriptional termination by providing signals in the newly synthesized mRNA that trigger processes which release the mRNA from the transcriptional complex, and
   - "1", "2", .../... "n" may or may not describe the same ORF (Open Reading Frame), promoter or terminator. The order of the nucleic acid sequences does not matter. "n" is an integer usually ranging from 5 and 20. These constructs are inserted in one of the recombinant cell chromosome at a controlled location. In some embodiments, the insertion site is neither essential for the functionality of the inserted construct, nor for the viability of the resulting genetically modified cell.

As will be understood by those of skill in the art, it can be advantageous to modify a coding sequence to enhance its expression in a particular host. The genetic code is redundant with 64 possible codons, but most organisms typically use a subset of these codons. The codons that are utilized most often in a species are called optimal codons, and those not utilized very often are classified as rare or low-usage codons. Codons can be substituted to reflect the preferred codon usage of the host, in a process sometimes called "codon optimization" or"controlling for species codon bias." Codon optimization for other host cells can be readily determined using codon usage tables or can be performed using commercially available software, such as CodonOp (www.idtdna.com/CodonOptfrom) from Integrated DNA Technologies. Optimized coding sequences containing codons preferred by a particular prokaryotic or eukaryotic host (Murray et al, 1989 , Nucl Acids Res . 17: 477-508) can be prepared, for example, to increase the rate of translation or to produce recombinant RNA transcripts having desirable properties, such as a longer half-life, as compared with transcripts produced from a non-optimized sequence. Translation stop codons can also be modified to reflect host preference. For example, typical stop codons for S. cerevisiae and mammals are UAA and UGA, respectively. The typical stop codon for monocotyledonous plants is UGA, whereas insects and E. coli commonly use UAA as the stop codon (Dalphin et al, 1996, Nucl Acids Res. 24: 216-8).
- When the recombinant cell is a yeast cell, and in particular is *Saccharomyces cerevisiae* yeast cell, nucleic acid sequences introduced in the yeast genome and originating from other organisms than *Saccharomyces cerevisiae* are generally "transcoded" (generally codon-optimized"), meaning that these nucleic acid sequences are synthesized with an optimal codon usage for expression in *S.cerevisiae.* The nucleotide sequence (and not the protein sequence) of some nucleic acid sequences from S. *cerevisiae* has also been modified ("transcoded") to minimize recombination with an endogenous copy of the said gene.
- Genes may be deleted through standard procedures used in cell genetic engineering. In some embodiments, the genes targeted for deletion may be interrupted by insertion of one of the above described gene constructs, or alternatively the genes targeted for deletion are replaced by a short stretch of nucleotide.
- A nucleic acid sequence may be rendered "inducible or repressible" by deleting an endogenous copy of the nucleic acid sequences (if necessary) and placing a new copy of the ORF under the control of an inducible or repressible promoter. An inducible or repressible promoter is a promoter which activity is modulated or controlled, i.e. either increased or decreased upon a change in the environmental conditions or external stimuli. Induction or repression may be artificially controlled, which encompasses induction or repression by abiotic factors such as chemical compounds not found naturally in the cell, and in particular yeast, of interest, light, oxygen levels, heat or cold. A list and sequences of inducible or repressible promoters are described elsewhere in the present specification.

### Recombinant cells according to the invention

The inventors have conceived recombinant cells, in particular recombinant yeasts, having an ability of producing chondroitin.

The present invention relates to recombinant cells, and in particular recombinant yeasts having the ability to produce chondroitin, and wherein this ability to produce chondroitin is obtained through a plurality of alterations that have been introduced in the genome thereof, by genetic engineering methods.

This present invention pertains to a recombinant yeast cell producing chondroitin wherein the recombinant cell comprises:
(a) one or more recombinant nucleic acids encoding a polypeptide having a chondroitin synthase (HCOS) activity;
(b) one or more recombinant nucleic acids encoding a polypeptide having UDP-Glucose dehydrogenase (UDP-GlcDH or HASB) activity; and
(c) one or more recombinant nucleic acids encoding a polypeptide having a UDP-glucose-4-epimerase (kfoA or GNE1) activity.

The present invention further relates to a recombinant host cell producing chondroitin wherein the recombinant host cell comprises:
(a) one or more recombinant nucleic acids encoding a polypeptide having a chondroitin synthase (HCOS) activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having a UDP-glucose-4-epimerase (kfoA or GNE1) activity; and
(d) one or more recombinant nucleic acids encoding a polypeptide having a chondroitinase activity wherein the polypeptide having chondroitinase activity comprises a secretion signal, and optionally an anchoring signal, so that chondroitin, in particular of a desired molecular weight is produced by the host cell.

The inventors have found that an ability to produce chondroitin by cells, and in particular by yeast cells, may be reached by introducing in the genome of these cells a plurality of genetic alterations.

The production of chondroitin by cells of the invention, and in particular by yeast cells of the invention, has been achieved by optimizing the endogenous metabolism of UDP-Glucose and UDP-N-Acetyl-glucosamine and direct the subsequent artificially modified metabolic pathway mainly towards chondroitin production whereas in the same time maintaining an optimal viability of the resulting genetically modified cells.

It has been determined that a chondroitin production by recombinant cells according to the invention may be increased by increasing the conversion of glucose-6-phosphate into the successive intermediate metabolites (i) glucose-1-phosphate, UDP-glucose, UDP-glucuronate and chondroitin or (ii) fructose-6-phosphate, glucosamine-6-phosphate, N-acetyl-glucosamine-6-phosphate, N-acetyl-glucosamine-1-phosphate, UDP-N-acetyl-glucosamine, UDP-N-aceytl-galactosamine and chondroitin, while maintaining a metabolic balance allowing a good viability of the resulting recombinant cells.

Indeed, in order to obtain a viable recombinant cell of the invention, many different constructs were tested in order to obtain a viable and efficient recombinant cell, and in particular a viable recombinant yeast. In particular such recombinant yeasts were difficult to obtain because the temporary accumulation of some intermediates appeared to be toxic for yeasts.

Unexpected technical difficulties were encountered in establishing the conditions suitable for the preparation of a recombinant cell able to produce chondroitin, and in particular to produce chondroitin with a controlled molecular weight.

Indeed, after much research and experimental trial, the inventors discovered that it was possible to cultivate a recombinant cell, and in particular a recombinant yeast cell, more particularly a recombinant *Saccharomyces cerevisiae* yeast cell, able to produce chondroitin having a controlled molecular weight controlled using the following parameters:
- the selection of the nature and origin of the nucleic acid sequences encoding the polypeptide having chondroitinase activity of the recombinant cell, in particular the recombinant yeast, of the invention; and/or
- the nature and origin of the promoter controlling the expression of the nucleic acid sequences encoding the polypeptide having chondroitinase activity of the recombinant cell, in particular the recombinant yeast, of the invention; and/or
- the optional presence of an anchoring signal, in addition to a secretion signal, associated to the encoded polypeptide having chondroitinase activity of the recombinant cell, in particular the recombinant yeast, according to the invention; and/or
- the pH of the cultivation medium during the step of culturing the recombinant cell, in particular the recombinant yeast, according to the invention; and/or
- the duration of the culturing of the recombinant cell, in particular the recombinant yeast, according to the invention.

To the inventors' knowledge, this has never been achieved before.

The nucleic acid encoding a polypeptide having a chondroitinase activity in a recombinant cell of the invention may be obtained or derived from at least one of *Cupiennius salei, Tityus serrulatus, Bos taurus, Vespa magnifica, Macaca mulata* or *Apis mellifera,* and preferably from *Tityus serrulatus.*

The molecular weight of the chondroitin can be in the range of less than 50kDa, preferably in the range of about 20kDa to about 50kDa.

Alternatively, the molecular weight of the chondroitin can be in the range of greater than 50kDa, preferably in the range of about 50kDa to about 250kDa.

In another alternative, the molecular weight of the chondroitin can be in the range of greater than 100kDa, preferably in the range of about 100kDa to about 1500kDa.

The nucleic acid encoding a polypeptide having a UDP-Glucose dehydrogenase (UDP-GlcDH or HASB) activity may be obtained or derived from at least one of *Arabidopsis thaliana, Chlorella virus PBCV1* or *Streptococcus zooepidemicus,* and is in particular obtained or derived from *Arabidopsis thaliana* or *Chlorella virus PBCV1.*

The nucleic acid encoding a polypeptide having a chondroitin synthase (HCOS) activity may be
(i) a nucleic acid encoding a chondroitin synthase; or
(ii) a nucleic acid encoding a chimeric polypeptide having a chondroitin synthase activity

These nucleic acids may be obtained or derived from at least one of *Pasteurella multocida, Chlorella virus PBCV1, Mycobacterium tuberculosis, Homo sapiens, Escherichia coli, Saccharomyces cerevisiae* or *Penicillium oxalicum.*

The nucleic acid encoding a polypeptide having a UDP-glucose-4-epimerase (kfoA or GNE1) activity may be obtained or derived from a bacteria, and in particular from a bacteria selected from the group consisting of *Pseudomonas aeruginosa, Pasteurella multocida,* and *Escherichia coli.*

The recombinant cell may further comprise at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

The recombinant cell may further comprise at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

In a particular embodiment of the recombinant cell,
- the nucleic acid encoding the polypeptide having a glutamine-fructose-6-phosphate amidotransferase (GFA1) activity;
- the nucleic acid encoding the polypeptide having UDP-N-acetylglucosamine pyrophosphorylase (QRI1) activity;
- the nucleic acid encoding the polypeptide having a Phosphoglucomutase-1 (PGM1) activity;
- the nucleic acid encoding the polypeptide having a UTP-glucose-1-phosphate uridylyltransferase (UGP1) activity;
- the nucleic acid encoding the polypeptide having Glucosamine-6-phosphate N-acetyltransferase (GNA1) activity; and/or
- the nucleic acid encoding the polypeptide having phosphoacetylglucosamine mutase (PCM1);
are obtained or derived from *Saccharomyces cerevisiae.*

In a particular embodiment, the recombinant host cell is a yeast.

In particular, the recombinant cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and in particular belongs to the *Saccharomyces genus.*

In particular, the recombinant cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

Another object of the invention relates to a method of producing chondroitin of a desired molecular weight comprising:
(a) cultivating a recombinant cell as defined herein in a cultivation medium for a time sufficient to produce chondroitin of the desired molecular weight; and
(b) optionally isolating or recovering the chondroitin from the recombinant cell and/or from the cultivation medium.

In the method, the chondroitin may have a molecular weight of from about 20kDa to about 50kDa.

In another embodiment, the chondroitin may have a molecular weight of from about 50kDa to about 150 kDa.

In another embodiment, the chondroitin may have a molecular weight of from about 150kDa to about 1500 kDa.

In the method of the invention, the recombinant cell may be a yeast belonging to the *Saccharomyces* genus, and in particular may be *Saccharomyces cerevisiae.*

The time sufficient to produce chondroitin of a desired molecular weight may be a period of from about 35 hours to about 50 hours, preferably from about 40 hours to about 50 hours, preferably may be about 48 hours.

In a particular embodiment, the molecular weight of the produced chondroitin may be controlled by regulating the pH of the cultivation medium during step (a).

In an embodiment, the method is carried out on an industrial scale, preferably where the cultivation medium is at least about 100L, more preferably is in the range of about 1,000L to about 3,000L, even more preferably is about 10,000L or even more preferably is about 100,000L, or even is about 250,000L.

Another object of the invention relates to a chondrotin obtained from a recombinant cell according to the invention, or from a method according to the invention.

The invention also relates to a cultivation medium comprising chondroitin of the invention.

Another object of the invention is a composition comprising chondroitin of the invention.

The invention further relates to an industrial product or a consumer product or a consumable comprising (i) the chondroitin having a molecular weight according to the invention, (ii) the cultivation medium according to the invention or (iii) the composition according to the invention.

In particular, the industrial product or the consumer product or the consumable may be a cosmetic product, a flavour product, a fragrance product, a food product, a food, a beverage, a texturant, a pharmaceutical composition, a dietary supplement, a nutraceutical, a cleaning product, a dental and/or an oral hygiene composition

The invention also relates to the use of a recombinant cell of the invention for the production of chondroitin having a molecular weight in the range of from about 20kDa to about 50k Da or from about 50kDa to about 1000 kDa.

Another object of the invention relates to a method for producing chondroitin, comprising the steps of:
(a) culturing a recombinant yeast as defined herein in a cultivation medium; and
(b) recovering the chondroitin from said cultivation medium,
wherein the chondrotin recovered in step (b) has a molecular weight controlled through the selection of:
- the nature and origin of the recombinant nucleic acid encoding a polypeptide having a chondroitinase activity of the recombinant yeast,
- the nature and origin of the promoter controlling the expression of the recombinant nucleic acid encoding encoding a polypeptide having a chondroitinase activity of the recombinant yeast,
- the presence of an anchoring and/or of a secretion signal associated to the polypeptide having a chondroitinase activity of the recombinant yeast,
- the pH of the cultivation medium during the step of culturing the recombinant yeast, and/or
- the duration of the culturing of the recombinant yeast.

Moreover, the invention pertains to a chondroitin-producing recombinant yeast, wherein the recombinant yeast comprises:
- (A) one or more recombinant nucleic acids encoding a polypeptide having a glutamine-fructose-6-phosphate amidotransferase (GFA1) activity,
- (B) one or more recombinant nucleic acids encoding a polypeptide having a UDP-N-acetylglucosamine pyrophosphorylase (QRI1) activity,
- (C) one or more recombinant nucleic acids encoding a polypeptide having a UDP-Glucose dehydrogenase (UDP-GlcDH or HASB) activity,
- (D) one or more recombinant nucleic acids encoding a polypeptide having a chondroitin synthase (HCOS) activity, and
- (E) one or more recombinant nucleic acids encoding a polypeptide having a UDP-glucose-4-epimerase (kfoA or GNE1) activity said recombinant yeast being *Saccharomyces cerevisiae.*

In a particular embodiment, the recombinant cell according to the invention, and in particular the recombinant yeast according to the invention, further comprises (F) one nucleic acids encoding a polypeptide having a chondroitinase activity, wherein the nucleic acid encoding a polypeptide having a chondroitinase activity comprises an anchoring signal and/or a secretion signal so that chondroitin, in particular of a desired molecular weight, is produced by the host cell.

In a particular embodiment, the recombinant cell according to the invention, and in particular the recombinant yeast, comprises only one recombinant nucleic acid encoding a polypeptide having a glutamine-fructose-6-phosphate amidotransferase (GFA1) activity.

In a particular embodiment, the recombinant cell according to the invention, and in particular the recombinant yeast comprises between 1 and 10, preferably between 2 and 8 recombinant nucleic acids encoding a polypeptide having a UDP-N-acetylglucosamine pyrophosphorylase (QRI1) activity.

In a particular embodiment, the recombinant cell according to the invention, and in particular the recombinant yeast comprises between 1 and 5 recombinant nucleic acids encoding a polypeptide having a UDP-Glucose dehydrogenase (UDP-GlcDH or HASB) activity.

In another embodiment, the one or more recombinant nucleic acids encoding a polypeptide having a UDP-Glucose dehydrogenase (UDP-GlcDH or HASB) are obtained or derived from at least one of *Arabidopsis thaliana, Chlorella virus PBCV1* or *Streptococcus zooepidemicus,* and in particular are obtained or derived from *Chlorella virus PBCV1* or from *Arabidopsis thaliana.*

In another embodiment, the recombinant cell according to the invention, and in particular the recombinant yeast comprises between 1 and 5 recombinant nucleic acids encoding a polypeptide having a chondroitin synthase (HCOS) activity.

In a particular embodiment, the one or more nucleic acids encoding polypeptide having a chondroitin synthase (HCOS) activity is obtained or derived from at least one of *Pasteurella multocida, Chlorella virus PBCV1, Mycobacterium tuberculosis, Homo sapiens, Escherichia coli, Saccharomyces cerevisiae* or *Penicillium oxalicum.*

In another embodiment, the recombinant cell according to the invention, and in particular the recombinant yeast comprises between 1 and 5 recombinant nucleic acids encoding a polypeptide having a UDP-glucose-4-epimerase (kfoA or GNE1) activity.

In a particular embodiment, the one or more nucleic acids encoding a polypeptide having a UDP-glucose-4-epimerase (kfoA or GNE1) activity is obtained or derived from at least one of *Pseudomonas aeruginosa, Pasteurella multocida, and Escherichia coli.*

In a particular embodiment, the recombinant cell according to the invention, and in particular the recombinant yeast comprises only one recombinant nucleic acid encoding a polypeptide having a chondroitinase activity.

In another embodiment, the one or more recombinant nucleic acids encoding a polypeptide having a chondroitinase activity are obtained or derived from at least one of *Cupiennius salei, Tityus serrulatus, Bos taurus, Apis mellifera, Macaca mulatta,* or *Vespa magnifica.*

In another embodiment, the recombinant cell according to the invention, and in particular the recombinant yeast cell may comprise at least one recombinant nucleic acid encoding one or more:
(A) a polypeptide having a Phosphoglucomutase-1 (PGM1) activity, and/or
(B) a polypeptide having a UTP-glucose-1-phosphate uridylyltransferase (UGP1) activity, and/or
(C) a polypeptide having a Glucosamine-6-phosphate N-acetyltransferase (GNA1) activity, and/or
(D) a polypeptide having a phosphoacetylglucosamine mutase (PCM1) activity.

In particular, the recombinant cell according to the invention, and in particular the recombinant yeast cell of the invention comprises at least two, in particular at least three, and more particularly all of the modifications indicated above.

In a particular embodiment, the recombinant nucleic acid encoding polypeptide having a Phosphoglucomutase-1 (PGM1) activity, the recombinant nucleic acid encoding a polypeptide having a UTP-glucose-1-phosphate uridylyltransferase (UGP1) activity, the recombinant nucleic acid encoding a polypeptide having a glutamine-fructose-6-phosphate amidotransferase (GFA1) activity, the recombinant nucleic acid encoding polypeptide having a Glucosamine-6-phosphate N-acetyltransferase (GNA1) activity, the recombinant nucleic acid encoding a polypeptide having a phosphoacetylglucosamine mutase (PCM1) activity and the recombinant nucleic acid encoding a polypeptide having a UDP-N-acetylglucosamine pyrophosphorylase (QRI1) activity are recombinant nucleic acids obtained or derived from a yeast, preferably from *Saccharomyces cerevisiae.*

In a particular embodiment, the one or more recombinant nucleic acids encoding polypeptides defined above and comprised in a recombinant cell according to the invention, and in particular the recombinant yeast of the invention, are under the the control of a promoter selected from the group consisting of pPDC1, pTDH3, pTDH3.Sk, pTDH3-1.Sba, pCCW12, pCCW120.Sm , pTEF1, pENO2 pTEF1.Ago, and pTEF1.Sba.

In a particular embodiment, the inducible or repressible promoters mentioned in the present specification are selected from the group consisting of promoters inducible or repressible with copper or promoters inducible or repressible with methionine, in particular pCUP1.

In a particular embodiment, the recombinant nucleic acids are under the control of the promoter pCWP2.

Another object of the invention relates to a method for producing chondroitin, said method comprising the steps of:
(a) culturing a recombinant cell according to the invention, and in particular a recombinant yeast, as defined herein in a culture medium; and
(b) recovering the chondroitin from said culture medium.

In a particular embodiment, the chondroitin recovered in step (b) has a pre-determined molecular weight controlled through the selection of:
- the nature and origin of the recombinant nucleic acid encoding a polypeptide having a chondroitinase activity, of the recombinant cell, and in particular the recombinant yeast,
- the nature and origin of the promoter controlling the expression of the recombinant nucleic acid encoding a polypeptide having a chondroitinase activity of the recombinant cell and in particular of the recombinant yeast,
- the presence of an anchoring or of a secretion signal associated to the recombinant nucleic acid encoding a polypeptide having a chondroitinase activity of the recombinant cell and in particular the recombinant yeast,
- the pH of the culture medium during the step of culturing the recombinant cell and in particular thre combinant yeast, and/or
- the duration of the culturing of the recombinant cell and in particular the recombinant yeast.

In a particular embodiment, the method of the invention is for producing chondroitin having a molecular weight strictly inferior to 50 kDa, and in particular strictly inferior to 50 kDa and superior or equal to 20 kDa, wherein (c) the step of recovering the chondroitin from the culture medium is performed 48 hours after the beginning of the culturing of the recombinant cell and in particular the recombinant yeast.

In another embodiment, the method of the invention is for producing chondroitin having a molecular weight superior or equal to 50 kDa and inferior or equal to 1000 kDa. wherein (c) the step of recovering the chondroitin from the culture medium is performed 48 hours after the beginning of the culturing of the recombinant yeast.

In another embodiment, the method of the invention is for producing chondroitin having a molecular weight strictly superior to 1000 kDa, and in particular having a molecular weight comprised between strictly superior to 1000 kDa and 1.5 Million kDa, wherein (c) the step of recovering the chondroitin from the culture medium is performed 48 hours after the beginning of the culturing of the recombinant cell and in particular the recombinant yeast.

In particular, the culture medium comprises at least a carbon source, preferably a carbon source selected from the group consisting of glucose and sucrose.

Another object of the invention relates to the use of a recombinant cell and in particular the recombinant yeast of the invention as defined herein, for the production of chondroitin, in particular of chondroitin of controlled molecular weight.

### Recombinant nucleic acids encoding a polypeptide having a chondroitin synthase activity (HCOS)

A recombinant cell according to the invention, and in particular a recombinant yeast of the invention, comprises one or more recombinant nucleic acids encoding a polypeptide having a chondroitin synthase activity.

A polypeptide having a chondroitin synthase activity according to the invention means a polypeptide that converts intermediate metabolites UDP-Glucoronate and UDP-N-actyl-galactosamine into chondroitin ((β-D-1,3-GalNAc-P-D-1,4-GlcA)n).

In some embodiments, the nucleic acid encoding a polypeptide having chondroitin synthase activity may be under the control of an inducible or repressible promoter.

One or more of the recombinant nucleic acids encoding a polypeptide having chondroitin synthase activity may be under the control of a pTDH3-1.Sba or a pTDH3.Sar promoter.

The said one or more recombinant nucleic acids encoding a polypeptide having chondroitin synthase (HCOS) activity may be:
(i) a nucleic acid encoding a chondroitin synthase; or
(ii) a nucleic acid encoding a chimeric polypeptide having a chondroitin synthase activity.

The nucleic acids encoding a polypeptide having a chondroitin synthase activity may be obtained or derived from at least one of *Pasteurella multocida, Chlorella virus PBCV1, Mycobacterium tuberculosis, Homo sapiens, Escherichia coli, Saccharomyces cerevisiae* or *Penicillium oxalicum ;*

A recombinant cell according to the invention, and in particular a recombinant yeast according to the invention, may comprise one recombinant nucleic acid encoding a polypeptide having chondroitin synthase activity.

Illustratively, the chondroitin synthase gene may be inserted within the JLP1 gene and/or within the SAM3 gene, as it is shown in the examples herein.

In an embodiment of the invention, a recombinant cell, and in particular a recombinant yeast, of the invention comprises:
- one recombinant nucleic acid encoding a polypeptide having chondroitin synthase activity;
- said one recombinant nucleic acid encoding a polypeptide having chondroitin synthase activity being obtained or derived from at least one of *Pasteurella multocida, Chlorella virus PBCV1, Mycobacterium tuberculosis, Homo sapiens, Escherichia coli, Saccharomyces cerevisiae* or *Penicillium oxalicum;*
- said said one recombinant nucleic acid encoding a polypeptide having chondroitin synthase activity being under the control of an inducible or repressible promoter and/or under the control of a pTDH3-1.Sba or a pTDH3.Sar promoter.

### Recombinant nucleic acids encoding a polypeptide having a UDP-Glucose dehydrogenase activity (HASB)

A recombinant cell according to the invention, and in particular a recombinant yeast according to the invention, comprises one or more recombinant nucleic acids encoding a polypeptide having UDP-Glucose dehydrogenase (UDP-GlcDH or HASB) activity.

A polypeptide having UDP-Glucose dehydrogenase (UDP-GlcDH or HASB) activity according to the invention means a polypeptide that converts the intermediate metabolite Uridine-Diphosphate-Glucose (UDP-Glucose) into UDP-Glucuronate.

One or more recombinant nucleic acids encoding a polypeptide having a UDP-Glucose dehydrogenase activity may be under the control of a promoter selected from the group consisting of pTEF1.sba, pCCW12 and pTDH3.Sk.

The one or more recombinant nucleic acids encoding a polypeptide having a UDP-Glucose dehydrogenase activity may be obtained or derived from at least one of *Arabidopsis thaliana, Chlorella virus PBCV1* or *Streptococcus zooepidemicus,* and in particular from at least one of *Arabidopsis thaliana* or *Chlorella virus PBCV1.*

A recombinant cell according to the invention, and in particular a recombinant yeast according to the invention, may comprise between 1 and 5 recombinant nucleic acids encoding a polypeptide having UDP-Glucose dehydrogenase activity.

Illustratively, the one or more recombinant nucleic acids encoding a polypeptide having UDP-Glucose dehydrogenase activity may be inserted within the JLP1 gene and/or within the SAM3 gene of the recombinant cell, and in particular of the recombinant yeast, as it is shown in the examples herein.

In an embodiment of the invention, a recombinant cell, and in particular a recombinant yeast, of the invention comprises:
- between 1 and 5 recombinant nucleic acids encoding a polypeptide having UDP-Glucose dehydrogenase activity;
- said recombinant nucleic acid encoding a polypeptide having UDP-Glucose dehydrogenase activity being obtained or derived from at least one of *Arabidopsis thaliana, Chlorella virus PBCV1* or *Streptococcus zooepidemicus,* and in particular from at least one of *Arabidopsis thaliana* or *Chlorella virus PBCV1;*
- said recombinant nucleic acid encoding a polypeptide having UDP-Glucose dehydrogenase activity being under the control of an inducible or repressible promoter that is functional in the recombinant cells of the invention and/or under the control of a promoter selected from the group consisting of pTEF1.sba, pCCW12 and pTDH3.Sk.

### Recombinant nucleic acids encoding a polypeptide having a UDP-,glucose-4-epimerase activity (kfoA or GNE1)

A recombinant cell according to the invention, and in particular a recombinant yeast according to the invention, comprises one or more recombinant nucleic acids encoding a polypeptide having UDP-glucose-4-epimerase activity.

A polypeptide having UDP-glucose-4-epimerase activity according to the invention means a polypeptide that converts the intermediate metabolite UDP-N-acetyl-glucosamine into UDP-N-acetyl-galactosamine.

In an embodiment, one or more of the recombinant nucleic acids encoding a polypeptide having a UDP-glucose-4-epimerase activity may be under the control of a promoter selected from the group consisting of pCCW12 or pCCW120.Sm.

The one or more recombinant nucleic acids encoding a polypeptide having a UDP-glucose-4-epimerase activity may be obtained or derived from a bacteria, and in particular from a bacteria selected from the group consisting of *Pseudomonas aeruginosa, Pasteurella multocida,* and *Escherichia coli.*

A recombinant cell according to the invention, and in particular a recombinant yeast according to the invention, may comprise between 1 and 5 recombinant nucleic acids encoding a polypeptide having a UDP-glucose-4-epimerase activity.

Illustratively, the one or more recombinant nucleic acids encoding a polypeptide having a UDP-glucose-4-epimerase activity may be inserted within the JLP1 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- between 1 and 5 recombinant nucleic acids encoding a polypeptide having a UDP-glucose-4-epimerase activity;
- said recombinant nucleic acid encoding a polypeptide having a UDP-glucose-4-epimerase activity being derived or obtained from the group consisting of *Pseudomonas aeruginosa, Pasteurella multocida,* and *Escherichia coli;*
- said recombinant nucleic acid encoding a polypeptide having a UDP-glucose-4-epimerase activity being under the control of an inducible or repressible promoter that is functional in the recombinant cells of the invention and/or under the control of a promoter selected from the group consisting of pCCW12 or pCCW120.Sm.

### Recombinant nucleic acids encoding a polypeptide having a chondroitinase activity

A recombinant cell according to the invention, and in particular a recombinant yeast according to the invention, comprises one or more recombinant nucleic acids encoding a polypeptide having chondroitinase activity.

A polypeptide having chondroitinase activity according to the invention means a polypeptide that degrades chondroitin, i.e. that converts a chondroitin of a given molecular weight into a chondroitin of a lower molecular weight.

Polypeptides having a chondroitinase activity according to the invention may include hyaluronoglucosaminidases, such as chondroitinases or hyaluronidases.

As previously indicated, polypeptides having chondroitinase activity of the present invention comprise a secretion signal.

In an embodiment, polypeptides having chondroitinase activity comprise both a secretion signal and an anchoring signal.

In an embodiment, the said one or more recombinant nucleic acids encoding a polypeptide having chondroitinase activity are under the control of an inducible or repressible promoter that is functional in the recombinant cells of the invention.

One or more of the recombinant nucleic acids encoding a polypeptide having chondroitinase activity may be under the control of a promoter selected from the group consisting of pTEF1, pCCW12, pCCW12.sba, pCCW12.Sar, pPDC1, pTEF3, pTDH3, pNUP57 and pCCW10.ago, and in particular of a pCCW12.Sba promoter.

The said one or more recombinant nucleic acids encoding a polypeptide having a chondroitinase activity may be obtained or derived from at least one of *Cupiennius salei (Csa), Tityus serrulatus (Ts), Bos taurus (Bt), Apis mellifera (Am), Macaca mulatta (Mm),* or *Vespa magnifica (Vm)* and in particular from *Tityus serrulatus (Ts).*

A recombinant cell according to the invention, and in particular a recombinant yeast according to the invention, may comprise only one recombinant nucleic acid encoding a polypeptide having a chondroitinase activity.

Illustratively, the one or more recombinant nucleic acids encoding a polypeptide having chondroitinase activity may be inserted within the JLP1 gene and/or within the LYP1 gene and/or within the LEU2 gene, and in particular within the LEU2 gene.

In a preferred embodiment, the recombinant nucleic acid encoding a polypeptide having chondroitinase activity is a recombinant nucleic acid encoding a hyaluronidase. In particular, the polypeptide having chondroitinase activity is a hyaluronidase.

All the characteristics above regarding a recombinant nucleic acid encoding a polypeptide having chondroitinase activity apply to a recombinant nucleic acid encoding a hyaluronidase.

In an embodiment of the invention, a recombinant cell, and in particular a recombinant yeast, of the invention comprises:
- only one recombinant nucleic acid encoding a polypeptide having a chondroitinase activity;
- said recombinant nucleic acid encoding a polypeptide having a chondroitinase activity being derived or obtained from at least one of *Cupiennius salei (Csa), Tityus serrulatus (Ts), Bos taurus (Bt), Apis mellifera (Am), Macaca mulatta (Mm),* or *Vespa magnifica (Vm)* and in particular from *Tityus serrulatus (Ts);*
- said recombinant nucleic acid encoding a polypeptide having chondroitinase activity comprising either (i) a secretion signal and no anchoring signal or (ii) a secretion signal and an anchoring signal;
- said polypeptide having a chondroitinase is a hyaluronidase,
- said recombinant nucleic acid encoding a polypeptide having chondroitinase activity being under the control of an inducible or repressible promoter that is functional in the recombinant cells of the invention and/or under the control of a promoter selected from the group consisting of pTEF1, pCCW12, pCCW12.sba, pCCW12.Sar, pPDC1, pTEF3, pTDH3, pNUP57 and pCCW10.ago, and in particular of a pCCW12.Sba promoter.

### Recombinant nucleic acids encoding a polypeptide having Glutamine-fructose-6-phosphate amidotransferase activity (GFA1)

A recombinant cell according to the invention, and in particular a recombinant yeast cell according to the invention, may comprise only one recombinant nucleic acid encoding a polypeptide having glutamine-fructose-6-phosphate amidotransferase activity.

A polypeptide having glutamine-fructose-6-phosphate amidotransferase activity according to the invention means a polypeptide that converts fructose-6-phosphate into glucosamine-6- phosphate.

In an embodiment, one or more of the recombinant nucleic acids encoding a polypeptide having glutamine-fructose-6-phosphate amidotransferase activity are under the control of an inducible or repressible promoter that is functional in the recombinant cells of the invention.

One or more of the recombinant nucleic acids encoding a polypeptide having glutamine-fructose-6-phosphate amidotransferase activity may be under the control of a pTEF1.Ago promoter.

The said one or more recombinant nucleic acids encoding a polypeptide having glutamine-fructose-6-phosphate amidotransferase activity may be obtained or derived from *Saccharomyces cerevisiae,* as shown in the examples herein.

A recombinant cell according to the invention, and in particular a recombinant yeast cell according to the invention, may comprise only one recombinant nucleic acid encoding a polypeptide having glutamine-fructose-6-phosphate amidotransferase activity.

Illustratively, the one or more recombinant nucleic acids encoding a polypeptide having glutamine-fructose-6-phosphate amidotransferase activity may be inserted within the JLP1 gene of the recombinant cell, and in particular of the recombinant yeast cell, as it is shown in the examples herein.

In an embodiment of the invention, a recombinant cell, and in particular a recombinant yeast cell, of the invention comprises:
- only one recombinant nucleic acid encoding a polypeptide having glutamine-fructose-6-phosphate amidotransferase activity;
- said recombinant nucleic acid encoding a polypeptide having glutamine-fructose-6-phosphate amidotransferase activity originating or being derived from *Saccharomyces cerevisiae;* and
- said recombinant nucleic acid encoding a polypeptide having glutamine-fructose-6-phosphate amidotransferase activity being under the control of a pTEF1.Ago promoter.

### Recombinant nucleic acids encoding a polypeptide having a UDP-N-acetylglucosamine pyrophosphorylase activity (QRI1)

A recombinant cell according to the invention, and in particular a recombinant yeast cell according to the invention, may comprise one or more recombinant nucleic acids encoding a polypeptide having UDP-N-acetylglucosamine pyrophosphorylase activity.

A polypeptide having UDP-N-acetylglucosamine pyrophosphorylase activity according to the invention means a polypeptide that converts N-acetyl-glucosamine into UDP-N-acetyl-glucosamine.

In an embodiment, one or more of the recombinant nucleic acids encoding a polypeptide having UDP-N-acetylglucosamine pyrophosphorylase activity are under the control of an inducible or repressible promoter that is functional in the recombinant cells of the invention, such as for example the inducible or repressible promoters pMET6 or pCUP1.

One or more of the recombinant nucleic acids encoding a polypeptide having UDP-N-acetylglucosamine pyrophosphorylase activity may be under the control of the promoters pTDH3-1.Sba or pTDH3.

The said one or more recombinant nucleic acids encoding a polypeptide having UDP-N-acetylglucosamine pyrophosphorylase activity may be obtained or derived from *Saccharomyces cerevisiae,* as shown in the examples herein.

A recombinant cell according to the invention, and in particular a recombinant yeast according to the invention, may comprise between 1 and 10 recombinant nucleic acids encoding a polypeptide having UDP-N-acetylglucosamine pyrophosphorylase activity, in particular may comprise between 2 and 8 recombinant nucleic acids encoding a polypeptide having UDP-N-acetylglucosamine pyrophosphorylase activity, and for example may comprise 2, 6 or 7 recombinant nucleic acids encoding a polypeptide having UDP-N-acetylglucosamine pyrophosphorylase activity.

Illustratively, the one or more recombinant nucleic acids encoding a polypeptide having UDP-N-acetylglucosamine pyrophosphorylase activity may be inserted within the HIS3 gene and/or within the MET14 gene and/or within the JLP1 gene of the recombinant cell, and in particular of the recombinant yeast, as it is shown in the examples herein.

In an embodiment of the invention, a recombinant cell, and in particular a recombinant yeast cell, of the invention comprises:
- between 2 and 8 recombinant nucleic acids encoding a polypeptide having UDP-N-acetylglucosamine pyrophosphorylase activity;
- said one or more recombinant nucleic acids encoding a polypeptide having UDP-N-acetylglucosamine pyrophosphorylase activity originating or being derived from *Saccharomyces cerevisiae;*
- said one or more recombinant nucleic acids encoding a polypeptide having UDP-N-acetylglucosamine pyrophosphorylase activity being under the control of an inducible or repressible promoter that is functional in the recombinant cells of the invention, such as for example the inducible or repressible promoters pMET6 or pCUP1 and/or under the control of the promoters pTDH3-1.Sba or pTDH3.

### Recombinant nucleic acids encoding a polypeptide having a Phosphoglucomutase-1 activity (PGM1)

A recombinant cell according to the invention, and in particular a recombinant yeast cell according to the invention, may comprise one or more recombinant nucleic acids encoding a polypeptide having Phosphoglucomutase-1 (PGM1) activity.

A polypeptide having Phosphoglucomutase-1 (PGM1) activity according to the invention means a polypeptide that converts Glucose-6-phosphate into the intermediate metabolite Glucose-1-phosphate.

In an embodiment, one or more of the recombinant nucleic acids encoding a polypeptide having Phosphoglucomutase-1 (PGM1) activity are under the control of an inducible or repressible promoter that is functional in recombiant cells of the invention.

One or more of the recombinant nucleic acids encoding a polypeptide having Phosphoglucomutase-1 (PGM1) activity may be under the control of the promoter pTDH3.

The one or more recombinant nucleic acids encoding a polypeptide having Phosphoglucomutase-1 (PGM1) activity may be obtained or derived from *Saccharomyces cerevisiae,* as shown in the examples herein.

A recombinant cell according to the invention, and in particular a recombinant yeast cell according to the invention, may comprise only one recombinant nucleic acid encoding a polypeptide having Phosphoglucomutase-1 (PGM1) activity.

Illustratively, the one or more recombinant nucleic acids encoding a polypeptide having Phosphoglucomutase-1 activity may be inserted within the MET14 gene of the recombinant cell, and in particular of the recombinant yeast.

In an embodiment of the invention, a recombinant cell, and in particular a recombinant yeast, of the invention comprises:
- only one recombinant nucleic acid encoding a polypeptide having Phosphoglucomutase-1 activity;
- said recombinant nucleic acid encoding a polypeptide having Phosphoglucomutase-1 activity originating or being derived from *Saccharomyces cerevisiae;* and
- said recombinant nucleic acid encoding a polypeptide having Phosphoglucomutase-1 activity being under the control of an inducible or repressible promoter that is functional in recombiant cells of the invention and/or under the promoter pTDH3.

### Recombinant nucleic acids encoding a polypeptide having a UTP-glucose-1-phosphate uridylyltransferase activity (UGP1)

A recombinant cell according to the invention, and in particular a recombinant yeast cell according to the invention, comprises one or more recombinant nucleic acids encoding a polypeptide having UTP-glucose-1-phosphate uridylyltransferase *(UGP1)* activity.

A polypeptide having UTP-glucose-1-phosphate uridylyltransferase *(UGP1)* activity according to the invention means a polypeptide that converts the intermediate metabolite Glucose-1-Phosphate into the intermediate metabolite UDP-glucose.

In an embodiment, one or more of the recombinant nucleic acids encoding a polypeptide having a UTP-glucose-1-phosphate uridylyltransferase activity are under the control of an inducible or repressible promoter that is functional in recombiant cells of the invention, such as for example the inducible or repressible promoters pSAM1 or pCUP1.

One or more of the recombinant nucleic acids encoding a polypeptide having UTP-glucose-1-phosphate uridylyltransferase activity may be under the control of a pFBA1, a pENO2 or a pPDC1 promoter.

The one or more recombinant nucleic acids encoding a polypeptide having UTP-glucose-1-phosphate uridylyltransferase activity may be obtained or derived from *Saccharomyces cerevisiae,* as shown in the examples herein.

A recombinant cell according to the invention, and in particular a recombinant yeast cell according to the invention, may comprise between 1 and 10 recombinant nucleic acids encoding a polypeptide having UTP-glucose-1-phosphate uridylyltransferase activity, in particular may comprise between 2 and 8 recombinant nucleic acids encoding a polypeptide having UTP-glucose-1-phosphate uridylyltransferase activity, and for example may comprise 2, 6 or 8 recombinant nucleic acids encoding a polypeptide having UTP-glucose-1-phosphate uridylyltransferase activity.

Illustratively, the the one or more recombinant nucleic acids encoding a polypeptide having UTP-glucose-1-phosphate uridylyltransferase activity gene may be inserted within the HIS3 gene and/or within the MET14 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- between 2 and 8 recombinant nucleic acids encoding a polypeptide having UTP-glucose-1-phosphate uridylyltransferase activity;
- said one or more recombinant nucleic acids encoding a polypeptide having UTP-glucose-1-phosphate uridylyltransferase activity originating or being derived from *Saccharomyces cerevisiae;* and
- said one or more recombinant nucleic acids encoding a polypeptide having being under the control of an inducible or repressible promoter that is functional in the recombinant cells of the invention, such as for example the inducible or repressible promoters pSAM1 or pCUP1 and/or under the control of a promoter selected from the group consisting of pFBA1, pENO2 and pPDC1.

### Recombinant nucleic acids encoding a polypeptide having a Glucosamine-6-phosphate N-acetyltransferase (GNA1) activity

A recombinant cell according to the invention, and in particular a recombinant yeast cell according to the invention, may comprise only one recombinant nucleic acid encoding a polypeptide having Glucosamine-6-phosphate N-acetyltransferase (GNA1) activity.

A polypeptide having Glucosamine-6-phosphate N-acetyltransferase (GNA1) activity according to the invention means a polypeptide that converts Glucosamine-6-phosphate into N-acetyl-glucosamine-6-phosphate.

In an embodiment, one or more of the recombinant nucleic acids encoding a polypeptide having Glucosamine-6-phosphate N-acetyltransferase activity are under the control of an inducible or repressible promoter that is functional in the recombinant cells of the invention.

One or more of the recombinant nucleic acids encoding a polypeptide having Glucosamine-6-phosphate N-acetyltransferase activity may be under the control of the promoter pCWP2.

The said one or more recombinant nucleic acids encoding a polypeptide having Glucosamine-6-phosphate N-acetyltransferase activity may be obtained or derived from *Saccharomyces cerevisiae,* as shown in the examples herein.

A recombinant cell according to the invention, and in particular a recombinant yeast cell according to the invention, may comprise only one recombinant nucleic acid encoding a polypeptide having Glucosamine-6-phosphate N-acetyltransferase activity.

Illustratively, the one or more recombinant nucleic acids encoding a polypeptide having Glucosamine-6-phosphate N-acetyltransferase activity may be inserted within the MET14 gene and/or within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Glucosamine-6-phosphate N-acetyltransferase activity;
- said recombinant nucleic acid encoding a polypeptide having Glucosamine-6-phosphate N-acetyltransferase activity originating or being derived from *Saccharomyces cerevisiae*; and
- said recombinant nucleic acid encoding a polypeptide having Glucosamine-6-phosphate N-acetyltransferase activity being under the control of an inducible or repressible promoter that is functional in the recombinant cells of the invention and/or under the confrol of the promoter pCWP2.

### Recombinant nucleic acids encoding a polypeptide having Phosphoacetylglucosamine mutase (PCM1) activity

A recombinant cell according to the invention, and in particular a recombinant yeast cell according to the invention, may comprise one or more recombinant nucleic acids encoding a polypeptide having phosphoacetylglucosamine mutase (PCM1) activity.

A polypeptide having phosphoacetylglucosamine mutase activity according to the invention means a polypeptide that converts N-acetyl-glucosamine-6-phosphate N-acetyl-glucosamine-1-phosphate.

In an embodiment, one or more of the recombinant nucleic acids encoding a polypeptide having phosphoacetylglucosamine mutase activity are under the control of an inducible or repressible promoter that is functional in the recombinant cells of the invention.

One or more of the recombinant nucleic acids encoding a polypeptide having phosphoacetylglucosamine mutase activity may be under the control a pTEF1 promoter.

The said one or more recombinant nucleic acids encoding a polypeptide having phosphoacetylglucosamine mutase activity may be obtained or be derived from *Saccharomyces cerevisiae,* as shown in the examples herein.

A recombinant cell according to the invention, and in particular a recombinant yeast according to the invention, may comprise only one recombinant nucleic acid encoding a polypeptide having phosphoacetylglucosamine mutase activity.

Illustratively, the one or more recombinant nucleic acids encoding a polypeptide having phosphoacetylglucosamine mutase activity may be inserted within the MET14 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- only one recombinant nucleic acid encoding a polypeptide having phosphoacetylglucosamine mutase activity;
- said recombinant nucleic acid encoding a polypeptide having phosphoacetylglucosamine mutase activity originating or being derived from *Saccharomyces cerevisiae;* and
- said recombinant nucleic acid encoding a polypeptide having phosphoacetylglucosamine mutase activity being under the control of an inducible or repressible promoter that is functional in the recombinant cells of the invention and/or under the control of a pTEF1 promoter.

### CHONDROITIN SYNTHASE (HCOS)

The chondroitin synthase enzyme is a protein which is described in the art for catalyzing the conversion of UDP-glucuronate and UDP-N-acetyl-galactosamine into chondroitin.

In a particular embodiment, the nucleic acid encoding a polypeptide having a chondroitin synthase (HCOS) activity is :
(i) a nucleic acid encoding a chondroitin synthase; or
(ii) a nucleic acid encoding a chimeric polypeptide having a chondroitin synthase activity.

A chimeric polypeptide, also known as a fusion polypetide, is a polypeptide consisting of at least two domains or fragments, preferably from two distinct polypeptides, that are encoded by separate nucleic acids that have been joined so that they are transcribed and translated as a single unit, producing a single polypeptide. Chimeric polypeptides may be created *in vivo,* or *in vitro* using recombinant DNA techniques. Such techniques known in the art may notably include but not only DNA solid phase synthesis, recombinant PCR, routine molecular techniques involing the use of restriction enzymes, ligase, recombinase, using techniques such as Gibson assembly (Gibson DG, Young L, Chuang RY, Venter JC, Hutchison CA 3rd, Smith HO (2009). Nature Methods. 6 (5): 343-345) or taking advantage of linear DNA recombination *in vivo* preferably in yeast.

A chimeric polypeptide having a chondroitin synthase activity that is suitable according to the invention may be a hybride protein which is known in the art (the concept of chimaeric chondroitine synthase is described in Tracy et al.(2006) Journal of biological chemistry, 282, 337-344 and Jing and DeAngelis (2003) Glycobiology 13, 661-71) encoded by a nucleic acid comprising a nucleic acid encoding a polypeptide having a hyaluronan synthase activity and a nucleic acid encoding a polypeptide having a chondroitin synthase activity. The resulting polypeptide is a chimeric polypeptide between a hyaluronan synthase and a chondroitin synthase.

Another chimeric polypeptide having a chondroitin synthase activity that is suitable according to the invention is encoded by a nucleic acid comprising a nucleic acid encoding a polypeptide having hyaluronan synthase activity and a nucleic acid encoding a polypeptide having Galactofuranosyltransferase activity. The resulting polypeptide is a fusion protein between a hyaluronan synthase and a galactofuranosyltransferase (Galactofuranosyltransferase GlfT2 mycobacterium tuberculosis UNIPROT O53585 EC 2.1.4.288).

Another chimeric polypeptide having a chondroitin synthase activity that is suitable according to the invention is encoded by a nucleic acid comprising a nucleic acid encoding a polypeptide having hyaluronan synthase activity and a nucleic acid encoding a polypeptide having chitin synthase activity. The resulting polypeptide is a fusion protein between a hyaluronan synthase and a chitin synthase (chitin synthase CHS2 *Saccharomyces cerevisiae* UNIPROT P14180 EC 2.1.4.16).

Examples of chimeric polypeptides having a chondroitin synthase activity that are suitable according to the invention may be encoded by a nucleic acid sequence comprising:
- a fragment from a nucleic acid encoding a hyaluronan synthase originating from *Pasteurella multocida* and a fragment from a nucleic acid encoding a chondroitin synthase from *Pasteurella multocida* (such as HCOS1-1, HCOS1-2 or HCOS1-3);
- a fragment from a nucleic acid encoding a hyaluronan synthase originating from *Pasteurella multocida,* a fragment from a nucleic acid encoding a chondroitin synthase from *Pasteurella multocida* and a fragment from a nucleic acid encoding a chitin synthase 2 from *Saccharomyces cerevisiae* (such as HCOS.Sc);
- a fragment from a nucleic acid encoding a hyaluronan synthase originating from *Pasteurella multocida* and a fragment from a nucleic acid encoding a chitin synthase 2 from *Saccharomyces cerevisiae* (such as HHASA.Sc)
- a fragment from a nucleic acid encoding a hyaluronan synthase originating from *Chlorella virus PBCV-1* and a fragment from a nucleic acid encoding a chondroitin synthase from *Escherichia coli* (such as HCOS1-Vir, HCOS2-Vir, or HCOS4-Vir);
- a fragment from a nucleic acid encoding a hyaluronan synthase originating from *Chlorella virus PBCV-1* and a fragment from a nucleic acid encoding a Galactofuranosyltransferase from *Mycobaterium tuberculosis* (such as HCOS3-Vir);
- a fragment from a nucleic acid encoding a hyaluronan synthase originating from *Chlorella virus PBCV-1* and a fragment from a nucleic acid encoding a chondroitin synthase from *Penicillium oxalicum* (such as HCOS5-Vir, HCOS6-Vir, HCOS7-Vir or HCOS8- Vir);
- a fragment from a nucleic acid encoding a hyaluronan synthase originating from *Chlorella virus PBCV-1* and a fragment from a nucleic acid encoding a chondroitin sulfate synthase from *Homo sapiens* (such as HCOS9-Vir, HCOS10-Vir, HCOS11-Vir, or HCOS12-Vir).

A method implemented to measure the activity level of a polypeptide having a chondroitin synthase activity belongs to the general knowledge of the one skilled in the art.

In this regard, the one skilled in the art may advantageously refer to the method wherein the enzyme's capacity of producing chondroitin is evaluated by the presence of chondroitin in the supernatant assayed using the carbazole method (Bitter and Muir (1962) analytical biochemistry 4, 330-334).

Preferred polypeptide having a chondroitin synthase activity in the present specification is an enzyme carrying two activities EC 2.4.1.175 and 2.4.1.226.

According to a preferred embodiment, one or more recombinant nucleic acids encoding a polypeptide having a chondroitin synthase activity may be obtained or derived from organisms preferably selected in a group consisting of prokaryotic organisms and eukaryotic organisms. In some embodiments, the one or more recombinant nucleic acids encoding a polypeptide having a chondroitin synthase activity be obtained or derived from archaebacteria. In some preferred embodiments, the one or more recombinant nucleic acids encoding a polypeptide having a chondroitin synthase activity may be obtained or derived from the group consisting of *Pasteurella multocida (Pm), Chlorella virus PBCV1 (Vir), Mycobacterium tuberculosis (Mt), Homo sapiens* (Hs), *Escherichia coli (Ec), Saccharomyces cerevisiae (Sc)* or *Penicillium oxalicum (Po).*

According to a yet preferred embodiment, the one or more recombinant nucleic acids encoding a polypeptide having a chondroitin synthase activity may be selected from the group consisting of nucleic acid sequences having (i) at least 65%, advantageously at least 70%, preferably at least 80%, nucleic acid identity with a nucleic acid sequence as set forth as sequence SEQ ID NO: 1 (HCOS1-1), SEQ ID NO: 2 (HCOS1-2), SEQ ID NO: 3 (HCOS-1-3), SEQ ID NO: 4 (HCOS.Sc), SEQ ID NO: 5 (HHASA.Sc), SEQ ID NO: 6 (HCOS1-Vir), SEQ ID NO: 7 (HCOS2-Vir), SEQ ID NO: 8 (HCOS3-Vir), SEQ ID NO: 9 (HCOS4-Vir), SEQ ID NO: 10 (HCOS5-Vir), SEQ ID NO: 11 (HCOS6-Vir), SEQ ID NO: 12 (HCOS7-Vir), SEQ ID NO: 13 (HCOS8-Vir), SEQ ID NO: 14 (HCOS9-Vir), SEQ ID NO: 15 (HCOS10-Vir), SEQ ID NO: 16 (HCOS11-Vir), or SEQ ID NO: 17 (HCOS12-Vir), and (ii) a biological activity of the same nature as the nucleic acid sequence having, respectively, the nucleic sequence as set forth as sequence SEQ ID NO: 1 (HCOS1-1), SEQ ID NO: 2 (HCOS1-2), SEQ ID NO: 3 (HCOS-1-3), SEQ ID NO: 4 (HCOS.Sc), SEQ ID NO: 5 (HHASA.Sc), SEQ ID NO: 6 (HCOS1-Vir), SEQ ID NO: 7 (HCOS2-Vir), SEQ ID NO: 8 (HCOS3-Vir), SEQ ID NO: 9 (HCOS4-Vir), SEQ ID NO: 10 (HCOS5-Vir), SEQ ID NO: 11 (HCOS6-Vir), SEQ ID NO: 12 (HCOS7-Vir), SEQ ID NO: 13 (HCOS8-Vir), SEQ ID NO: 14 (HCOS9-Vir), SEQ ID NO: 15 (HCOS10-Vir), SEQ ID NO: 16 (HCOS11-Vir), or SEQ ID NO: 17 (HCOS12-Vir).

A biological activity of the same nature regarding this sequence is, as previously explained, the capacity to code for a polypeptide that converts UDP-glucuronate and UDP-N-acetyl-galactosamine into chondroitin.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the chimaeric polypeptide having a chondroitin synthase activity comprising an amino acid comprising a fragment from an amino acid encoding a hyaluronan synthase originating from *Pasteurella multocida* and a fragment from an amino acid encoding a chondroitin synthase from *Pasteurella multocida* the one skilled in the art may refer to the accession numbers Q7BLV3 and Q9CMP0, respectively, in the UniProt database, or to the sequences as set forth in SEQ ID NO: 18 (HCOS1-1), SEQ ID NO: 19 (HCOS1-2) and SEQ ID NO: 20 (HCOS-1-3) described herein.

For the amino acid sequence of the chimaeric polypeptide having a chondroitin synthase activity comprising a fragment from an amino acid encoding a hyaluronan synthase originating from *Pasteurella multocida,* a fragment from an amino acid encoding a chdroitin synthase from *Pasteurella multocida* and a fragment from an amino acid encoding a chitin synthase 2 from *Saccharomyces cerevisiae* the one skilled in the art may refer to the accession numbers Q7BLV3, Q9CMP0 and P14180, respectively, in the UniProt database, or to the sequence as set forth in SEQ ID NO: 21 (HCOS.Sc) as described herein.

For the amino acid sequence of the chimaeric polypeptide having a chondroitin synthase activity comprising a fragment from an amino acid encoding a hyaluronan synthase originating from *Pasteurella multocida* and a fragment from an amino acid encoding a chitin synthase 2 from *Saccharomyces cerevisiae* the one skilled in the art may refer to the accession numbers Q7BLV3 and P14180, respectively, in the UniProt database, or to the sequence as set forth in SEQ ID NO: 22 (HHASA.Sc) as described herein.

For the amino acid sequence of the chimaeric polypeptide having a chondroitin synthase activity comprising a fragment from an amino acid encoding a hyaluronan synthase originating from *Chlorella virus PBCV-1* and a fragment from an amino acid encoding a chondroitin synthase from *Escherichia coli* the one skilled in the art may refer to the accession numbers Q84419 and Q8L0V4, respectively, in the UniProt database, or to the sequences as set forth in SEQ ID NO: 23 (HCOS 1-Vir), SEQ ID NO: 24 (HCOS2-Vir), or SEQ ID NO: 26 (HCOS4-Vir) as described herein.

For the amino acid sequence of the chimaeric polypeptide having a chondroitin synthase activity comprising a fragment from an amino acid encoding a hyaluronan synthase originating from *Chlorella virus PBCV-1* and a fragment from an amino acid encoding a Galactofuranosyltransferase from *Mycobaterium tuberculosis* the one skilled in the art may refer to the accession numbers Q84419 and O53585, respectively, in the UniProt database, or to the sequence as set forth in SEQ ID NO: 25 (HCOS3-Vir) as described herein.

For the amino acid sequence of the chimaeric polypeptide having a chondroitin synthase activity comprising a fragment from an amino acid encoding a hyaluronan synthase originating from *Chlorella virus PBCV-1* and a fragment from an amino acid encoding a chondroitin synthase from *Penicillium oxalicum* the one skilled in the art may refer to the accession numbers Q84419 and S7Z8F8, respectively, in the UniProt database, or to the sequences as set forth in SEQ ID NO: 27 (HCOS5-Vir).

For the amino acid sequence of the chimaeric polypeptide having a chondroitin synthase activity comprising a fragment from an amino acid encoding a hyaluronan synthase originating from *Chlorella virus PBCV-1* and a fragment from an amino acid encoding a chondroitin synthase from *Pasteurella multocida* the one skilled in the art may refer to the accession numbers Q84419 and Q9CMP0, respectively, in the UniProt database, or to the sequences as set forth in SEQ ID NO: 28 (HCOS6-Vir), SEQ ID NO: 29 (HCOS7-Vir), or SEQ ID NO: 30 (HCOS8-Vir) as described herein.

For the amino acid sequence of the chimaeric polypeptide having a chondroitin synthase activity comprising a fragment from an amino acid encoding a hyaluronan synthase originating from *Chlorella virus PBCV-1* and a fragment from an amino acid encoding a chondroitin sulfate synthase from *Homo sapiens* the one skilled in the art may refer to the accession numbers Q84419 and Q86X52, respectively, in the UniProt database, or to the sequences as set forth in SEQ ID NO: 31 (HCOS9-Vir), SEQ ID NO: 32 (HCOS10-Vir), SEQ ID NO: 33 (HCOS11-Vir) or SEQ ID NO: 34 (HCOS 12-Vir) as described herein.

According to another particular embodiment, the one or more recombinant nucleic acids encoding a polypeptide having a chondroitin synthase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 18 (HCOS1-1), SEQ ID NO: 19 (HCOS1-2), SEQ ID NO: 20 (HCOS-1-3), SEQ ID NO: 21 (HCOS.Sc), SEQ ID NO: 22 (HHASA.Sc), SEQ ID NO: 23 (HCOS 1-Vir), SEQ ID NO: 24 (HCOS2-Vir), SEQ ID NO: 25 (HCOS3-Vir), SEQ ID NO: 26 (HCOS4-Vir), SEQ ID NO: 27 (HCOS5-Vir), SEQ ID NO: 28 (HCOS6-Vir), SEQ ID NO: 29 (HCOS7-Vir), SEQ ID NO: 30 (HCOS8-Vir), SEQ ID NO: 31 (HCOS9-Vir), SEQ ID NO: 32 (HCOS10-Vir), SEQ ID NO: 33 (HCOS11-Vir), and SEQ ID NO: 34 (HCOS12-Vir) and also a biological activity of the same nature.

A biological activity of the same nature regarding this sequence is as described previously, i.e. the capacity to catalyze the conversion of UDP-glucuronate and UDP-N-acetyl-galactosamine into UDP-N-acetyl-galactosamine.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having chondroitin synthase activity in the present invention is regulated by at least one promoter and at least one terminator, such as herein after defined more in details, which are present in 5' and 3' position respectively of the one or more recombinant nucleic acids encoding a polypeptide having a chondroitin synthase activity.

### UDP-GLUCOSE DEHYDROGENASE (UDP-GlcDH OR HASB)

The UDP-Glucose dehydrogenase is a protein which is known in the art to catalyze the conversion of UDP-glucose into UDP-glucuronate. The UDP-Glucose dehydrogenase originating from the genome of *Arabidopsis thaliana, Chlorella virus PBCV1* or *Streptococcus zooepidemicus* may be termed HASB.

A method implemented to measure the activity level of a polypeptide having a UDP-Glucose dehydrogenase activity belongs to the general knowledge of the one skilled in the art.

In this regard, the one skilled in the art may advantageously refer to the method described by Oka and Jigami (FEBS Journal 273, 2645-2657, 2006).

Preferred polypeptide having a UDP-Glucose dehydrogenase activity in the present specification is an enzyme having an EC number 1.1.1.22.

According to a preferred embodiment, the one or more recombinant nucleic acids encoding a polypeptide having UDP-Glucose dehydrogenase activity may be obtained or derived from organisms preferably selected in a group comprising prokaryotic organisms and eukaryotic organisms. In some embodiments, the one or more recombinant nucleic acids encoding a polypeptide having UDP-Glucose dehydrogenase activity obtained or derived from archaebacteria. In some preferred embodiments, the one or more recombinant nucleic acids encoding a polypeptide having UDP-Glucose dehydrogenase activity may obtained or derived from yeast, and especially from *Arabidopsis thaliana, Chlorella virus PBCV1* or *Streptococcus zooepidemicus,* preferably from *Arabidopsis thaliana,* or *Chlorella virus PBCV1.*

According to a yet preferred embodiment, the one or more recombinant nucleic acids encoding a polypeptide having UDP-Glucose dehydrogenase activity may be selected from the group consisting of nucleic acid sequences having (i) at least 65%, advantageously at least 70%, preferably at least 80%, nucleic acid identity with a nucleic acid sequence as set forth as sequence SEQ ID NO: 35 (At), SEQ ID NO: 36 (Vir), SEQ ID NO: 37 (Vir) and SEQ ID NO: 38 (Sz), and (ii) a biological activity of the same nature as set forth as sequence SEQ ID NO: 35 (At), SEQ ID NO: 36 (Vir), SEQ ID NO: 37 (Vir) and SEQ ID NO: 38 (Sz). The nucleic acid sequences set forth as SEQ ID NO: 35 (At), SEQ ID NO: 36 (Vir), SEQ ID NO: 37 (Vir) and SEQ ID NO: 38 (Sz) encode a polypeptide having a UDP-Glucose dehydrogenase activity obtained or derived, respectively, from *Arabidopsis thaliana (At), Chlorella virus PBCV1 (Vir)* or *Streptococcus zooepidemicus (Sz),* that may herein also be collectively termed HASB herein.

A biological activity of the same nature regarding this sequence is, as previously explained, the capacity to code for a polypeptide that converts UDP-glucose into UDP-glucuronate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having a UDP-Glucose dehydrogenase activity from *Arabidopsis thaliana, Chlorella virus PBCV1* or *Streptococcus zooepidemicus* the one skilled in the art may refer to the accession numbers NP_173979.1, NP_048965 or KIS19289, respectively, in the UniProt database, or to the sequences as set forth in SEQ ID NO: 39 (At), SEQ ID NO: 40 (Vir) and SEQ ID NO: 41 (Sz) described herein.

According to another particular embodiment, the nucleic acid(s) encoding a UDP-Glucose dehydrogenase may be nucleic acid(s) encoding an amino acid sequence selected from the group consisting of sequences having at least 55%, advantageously at least 65%, preferably at least 80%, amino acid identity with the amino acid sequence of SEQ ID NO: 39 (At), SEQ ID NO: 40 (Cv) and SEQ ID NO: 41 (Sz), and also a biological activity of the same nature as the amino acid sequences of SEQ ID NO: 39 (At), SEQ ID NO: 40 (Vir) and SEQ ID NO: 41 (Sz) and also a biological activity of the same nature.

A biological activity of the same nature regarding this sequence is as described previously, i.e. the capacity to catalyze the conversion of UDP-glucose into UDP-glucuronate.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the polypeptide having a UDP-Glucose dehydrogenase activity in the present invention is regulated by at least one promoter and at least one terminator, such as herein after defined more in detail, which are present in 5' and 3' position respectively of the nucleic acids encoding the said polypeptide having a UDP-Glucose dehydrogenase activity.

### UDP-GLUCOSE-4-EPIMERASE (kfoA or GNE1)

The UDP-glucose-4-epimerase is a protein which is known in the art to catalyze the conversion of UDP-N-acetyl-glucosamine into UDP-N-acetyl-galactosamine. The UDP-glucose-4-epimerase obtained or derived from *Pseudomonas aeruginosa, Pasteurella multocida,* or *Escherichia coli* may be termed kfoA or GNE1.

A method implemented to measure the activity level of a polypeptide having a UDP-glucose-4-epimerase activity belongs to the general knowledge of the one skilled in the art.

In this regard, the one skilled in the art may advantageously refer to the method described in Creuzenet et al. (2000, Journal of Biological Chemistry 275, 19060-67).

Preferred polypeptide having a UDP-glucose-4-epimerase activity in the present specification is an enzyme having an EC number 5.1.3.7.

According to a preferred embodiment, the one or more recombinant nucleic acids encoding a polypeptide having a UDP-glucose-4-epimerase activity may be obtained or derived from organisms preferably selected in a group consisting of prokaryotic organisms and eukaryotic organisms. In some embodiments, the one or more recombinant nucleic acids encoding a polypeptide having a UDP-glucose-4-epimerase activity may be obtained or derived from archaebacteria. In some preferred embodiments, the one or more recombinant nucleic acids encoding a polypeptide having a UDP-glucose-4-epimerase activitymay be obtained or derived from *Pseudomonas aeruginosa, Pasteurella multocida (Pm), or Escherichia coli (Ec).*

According to a yet preferred embodiment, the one or more recombinant nucleic acids encoding a polypeptide having a UDP-glucose-4-epimerase activitymay be selected from the group consisting of nucleic acid sequences having (i) at least 65%, advantageously at least 70%, preferably at least 80%, nucleic acid identity with a nucleic acid sequence as set forth as sequence SEQ ID NO: 42 (Pa), SEQ ID NO: 43 (Pm), and SEQ ID NO: 44 (Ec), and (ii) a biological activity of the same nature as set forth as sequence SEQ ID NO: 42 (Pa), SEQ ID NO: 43 (Pm), and SEQ ID NO: 44 (Ec).

A biological activity of the same nature regarding this sequence is, as previously explained, the capacity to code for a polypeptide that converts UDP-N-acetyl-glucosamine into UDP-N-acetyl-galactosamine.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequences.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequences.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequences.

For the amino acid sequence of the polypeptide having a UDP-glucose-4-epimerase activity from *Pseudomonas aeruginosa, Pasteurella multocida,* and *Escherichia coli,* the one skilled in the art may refer to the accession numbers Q8KN66, AAK02370, and Q8L0V2, respectively, in the UniProt database, or to the sequences as set forth as SEQ ID NO: 45 (Pa), SEQ ID NO: 46 (Pm), and SEQ ID NO: 47 (Ec) described herein.

According to another particular embodiment, the one or more recombinant nucleic acids encoding a polypeptide having a UDP-glucose-4-epimerase activity may be nucleic acid(s) encoding a polypeptide having an amino acid sequence selected from the group consisting of sequences having at least 55%, advantageously at least 65%, preferably at least 80%, amino acid identity with the amino acid sequence set forth as SEQ ID NO: 45 (Pa), SEQ ID NO: 46 (Pm), or SEQ ID NO: 47 (Ec) and also a biological activity of the same nature as the amino acid sequences of SEQ ID NO: 45 (Pa), SEQ ID NO: 46 (Pm), or SEQ ID NO: 47 (Ec).

A biological activity of the same nature regarding this sequence is as described previously, i.e. the capacity to catalyze the conversion of UDP-N-acetyl-glucosamine into UDP-N-acetyl-galactosamine.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having a UDP-glucose-4-epimerase activity in the present invention is regulated by at least one promoter and at least one terminator, such as herein after defined more in details, which are present in 5' and 3' position respectively of the one or more recombinant nucleic acids encoding a polypeptide having a UDP-glucose-4-epimerase activity.

### CHONDROITINASE (HYAL)

The chondroitinase enzyme is a protein which is described in the art for catalyzing the degradation of chondroitin molecules into smaller chondroitin molecules. As mentioned above, a polypeptide having a chondroitinase activity may be a hyaluronoglucosaminidase, such as a chondroitinase or a hyaluronidase.

In a particular embodiment, a polypeptide having a chondroitinase activity is a hyaluronidase.

The polypepide having a chondroitinase activity may encoded by the genome of *Cupiennius salei, Tityus serrulatus, Bos taurus, Apis mellifera, Macaca mulatta,* or *Vespa magnificd* and may be termed HYAL.

The polypeptide having a chondroitinase activity may possess both a secretion signal and an anchoring signal or a secretion signal and no anchoring signal signal or a secretion-anchor signal with a dual secretion and anchoring function. When the encoded polypeptide having chondroitinase activity possesses both a secretion signal and an anchoring signal, it may be termed HYAL-31.

When the encoded polyeptide having the chondroitinase activity possesses a secretion signal and no anchoring signal, it may be termed HYAL-3.

A method implemented to measure the activity level of a polypeptide having a chondroitinase activity belongs to the general knowledge of the one skilled in the art.

In this regard, the one skilled in the art may advantageously monitor the molecular weight of the obtained chondroitin on an agarose gel.

Preferred polypeptide having a chondroitinase activity in the present specification is an enzyme having an EC number of n° EC 3.2.1.35.

According to a preferred embodiment, the one or more recombinant nucleic acids encoding a polypeptide having a chondroitinase activity may be obtained or derived from organisms preferably selected in a group comprising prokaryotic organisms and eukaryotic organisms. In some embodiments, one or more recombinant nucleic acids encoding a polypeptide having a chondroitinase activity may be obtained or derived from archaebacteria. In some embodiments, one or more recombinant nucleic acids encoding a polypeptide having a chondroitinase activity may be obtained or derived from organisms preferably selected from yeasts. In some other preferred embodiments, the one or more recombinant nucleic acids encoding a polypeptide having a chondroitinase activity may be obtained or derived from *Cupiennius salei (Csa), Tityus serrulatus (Ts), Bos taurus (Bt), Apis mellifera (Am), Macaca mulatta (Mm),* or *Vespa magnifica (Vm)* and in particular from *Tityus serrulatus (Ts).*

According to a yet preferred embodiment, the one or more recombinant nucleic acids encoding a polypeptide having a chondroitinase activity may be selected from the group consisting of nucleic acid sequences having (i) at least 65%, advantageously at least 70%, preferably at least 80%, nucleic acid identity with a nucleic acid of SEQ ID NO: 48 (Csa), SEQ ID NO: 49 (Ts), SEQ ID NO: 50 (Bt), SEQ ID NO: 51 (Am), SEQ ID NO: 52 (Mm) or SEQ ID NO: 53 (Vm), (ii) encoding a polypeptide having a chondroitinase activity, comprising a secretion signal and no anchoring signal, obtained or derived from *Cupiennius salei, Tityus serrulatus, Bos taurus, Apis mellifera, Macaca mulatta,* or *Vespa magnifica,* respectively, and (iii) having a biological activity of the same nature as the sequences of SEQ ID NO: 48 (Csa), SEQ ID NO: 49 (Ts), SEQ ID NO: 50 (Bt), SEQ ID NO: 51 (Am), SEQ ID NO: 52 (Mm) or SEQ ID NO: 53 (Vm).

According to another preferred embodiment, the one or more recombinant nucleic acids encoding a polypeptide having a chondroitinase activity may be selected from the group consisting of nucleic acid sequences having at least 65%, advantageously at least 70%, preferably at least 80%, nucleic acid identity with a nucleic acid of SEQ ID NO: 54 (Csa), SEQ ID NO: 55 (Ts), SEQ ID NO: 56 (Bt), SEQ ID NO: 57 (Am), SEQ ID NO: 58 (Mm) or SEQ ID NO: 59 (Vm), (ii) encoding a polypeptide having a chondroitinase activity comprising a secretion signal and an anchoring signal obtained or derived from *Cupiennius salei, Tityus serrulatus, Bos taurus, Apis mellifera, Macaca mulatta,* or *Vespa magnifica,* respectively, and (iii) having a biological activity of the same nature as the sequences of of SEQ ID NO: 54 (Csa), SEQ ID NO: 55 (Ts), SEQ ID NO: 56 (Bt), SEQ ID NO: 57 (Am), SEQ ID NO: 58 (Mm) or SEQ ID NO: 59 (Vm).

A biological activity of the same nature regarding this sequence is, as previously explained, the capacity to code for a polypeptide that catalyzes the degradation of chondroitin molecules into smaller chondroitin molecules.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature, as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature, as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature, as the said reference nucleic acid sequence.

For the amino acid sequence of the hyaluronidase from *Cupiennius salei, Tityus serrulatus, Bos taurus, Apis mellifera, Macaca mulatta,* or *Vespa magnifica,* the one skilled in the art may refer to the accession numbers A0A0S4JYH2, P85841, Q7YS45, Q08169, G7ML68 or P86875, respectively in the UniProt database or to SEQ ID NO: 60 (Csa), SEQ ID NO: 61 (Ts), SEQ ID NO: 62 (Bt), SEQ ID NO: 63 (Am), SEQ ID NO: 64 (Mm) or SEQ ID NO: 65 (Vm), described herein.

According to another particular embodiment, the one or more recombinant nucleic acids encoding a polypeptide having a chondroitinase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 60 (Csa), SEQ ID NO: 61 (Ts), SEQ ID NO: 62 (Bt), SEQ ID NO: 63 (Am), SEQ ID NO: 64 (Mm) or SEQ ID NO: 65 (Vm) which comprise a secretion signal and no anchoring signal and also a biological activity of the same nature as the sequences SEQ ID NO: 60 (Csa), SEQ ID NO: 61 (Ts), SEQ ID NO: 62 (Bt), SEQ ID NO: 63 (Am), SEQ ID NO: 64 (Mm) or SEQ ID NO: 65 (Vm).

According to another particular embodiment, the one or more recombinant nucleic acids encoding a polypeptide having a chondroitinase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 66 (Csa), SEQ ID NO: 67 (Ts), SEQ ID NO: 68 (Bt), SEQ ID NO: 69 (Am), SEQ ID NO: 70 (Mm) or SEQ ID NO: 71 (Vm) which comprise a secretion signal and an anchoring signal and also a biological activity of the same nature as the amino acid sequences of SEQ ID NO: 66 (Csa), SEQ ID NO: 67 (Ts), SEQ ID NO: 68 (Bt), SEQ ID NO: 69 (Am), SEQ ID NO: 70 (Mm) or SEQ ID NO: 71 (Vm).

A biological activity of the same nature regarding this sequence is as described previously, i.e. the capacity to catalyze the degradation of chondroitin molecules into smaller chondroitin molecules.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having a chondroitinase activity in the present invention is regulated by at least one promoter and at least one terminator, such as herein after defined more in details, which are present in 5' and 3' position respectively of the one or more recombinant nucleic acids encoding a polypeptide having chondroitinase activity.

### GLUTAMINE-FRUCTOSE-6-PHOSPHATE AMIDOTRANSFERASE (GFA1)

The glutamine-fructose-6-phosphate amidotransferase enzyme is a protein which is described in the art for catalyzing the conversion of fructose-6-phosphate into glucosamine-6-phosphate. The glutamine-fructose-6-phosphate amidotransferase originating from Saccharomyces cerevisiae may be termed GFA1.

A method implemented to measure the activity level of a polypeptide having glutamine-fructose-6-phosphate amidotransferase activity belongs to the general knowledge of the one skilled in the art.

In this regard, the one skilled in the art may advantageously refer to the method described by Shiga Shibatan and Hiroaki Kitazawa (Plant Biotechnology 26, 149-152, 2009).

Preferred polypeptide having glutamine-fructose-6-phosphate amidotransferase activity in the present invention is an enzyme having an EC number of n° EC 2.6.1.16.

According to a preferred embodiment, the the one or more recombinant nucleic acids encoding a polypeptide having glutamine-fructose-6-phosphate amidotransferase activity may originate or be derived from organisms preferably selected in a group comprising prokaryotic organisms and eukaryotic organisms. In some embodiments, the one or more recombinant nucleic acids encoding a polypeptide having glutamine-fructose-6-phosphate amidotransferase activity may originate or be derived from archaebacteria. In some embodiments, the one or more recombinant nucleic acids encoding a polypeptide having glutamine-fructose-6-phosphate amidotransferase activity may originate or be derived from organisms preferably selected from Bacillus subtilis, and yeasts. In some other preferred embodiments, the one or more recombinant nucleic acids encoding a polypeptide having glutamine-fructose-6-phosphate amidotransferase activity may originate or be derived from a yeast, and especially from Saccharomyces cerevisiae.

According to a yet preferred embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 72 (Sc), and (ii) a biological activity of the same nature as the nucleic acid sequence as set forth as sequence SEQ ID NO: 72. The nucleic acid as set forth as sequence SEQ ID NO: 72 encodes a polypeptide having glutamine-fructose-6-phosphate amidotransferase activity obtained or derived from *Saccharomyces cerevisiae,* that may also be termed GFA1.

According to yet another embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 73 or SEQ ID NO: 74, and (ii) a biological activity of the same nature as the nucleic acid sequence as set forth as sequence SEQ ID NO: 73 or SEQ ID NO: 74. The nucleic acid sequence as set forth as sequence SEQ ID NO: 73 or SEQ ID NO: 74 encodes a polypeptide having glutamine-fructose-6-phosphate amidotransferase activity originating from *Chlorella virus PBCV1.*

A biological activity of the same nature regarding this sequence is, as previously explained, the capacity to code for a polypeptide that converts fructose-6-phosphate into glucosamine-6-phosphate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having glutamine-fructose-6-phosphate amidotransferase activity originating from *Saccharomyces cerevisiae,* the one skilled in the art may refer to the accession number NP8012818 in the UniProt database, or to the sequence SEQ ID NO: 75 described herein.

For the amino acid sequence of the polypeptide having glutamine-fructose-6-phosphate amidotransferase activity originating from *Chlorella virus PBCV1,* the one skilled in the art may also refer to the accession number NP_048448 in the UniProt database, or to the sequence SEQ ID NO: 76 described herein.

According to another particular embodiment, the one or more recombinant nucleic acids encoding a polypeptide having glutamine-fructose-6-phosphate amidotransferase activity may be nucleic acid(s) encoding polypeptide having an amino acid sequence selected from the group consisting of sequences having at least 35%, advantageously at least 65%, preferably at least 80%, amino acid identity with the amino acid sequence of SEQ ID NO: 75or with SEQ ID NO: 76, and also a biological activity of the same nature as the amino acid sequence of SEQ ID NO: 75 or with SEQ ID NO: 76.

A biological activity of the same nature regarding this sequence is as described previously, i.e. the capacity to catalyze the conversion of of fructose-6-phosphate into glucosamine-6-phosphate.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the polypeptide having glutamine-fructose-6-phosphate amidotransferase activity in the present invention is regulated by at least one promoter and at least one terminator, such as herein after defined more in details, which are present in 5' and 3' position respectively of one or more recombinant nucleic acids encoding a polypeptide having glutamine-fructose-6-phosphate amidotransferase activity.

### UDP-N-ACETYLGLUCOSAMINE PYROPHOSPHORYLASE (QRI1)

The UDP-N-acetylglucosamine pyrophosphorylase enzyme is a protein which is described in the art for catalyzing the conversion of N-acetyl-glucosmine-6-phosphate into UDP-N-acetyl-glucose. The UDP-N-acetylglucosamine pyrophosphorylase originating from *Saccharomyces cerevisiae* may be termed QRI1.

A method implemented to measure the activity level of a polypeptide having UDP-N-acetylglucosamine pyrophosphorylase activity belongs to the general knowledge of the one skilled in the art.

In this regard, the one skilled in the art may advantageously refer to the method described by Mio et al. (The Journal of Biological Chemistry, Col. 273, No 23, June 5, 1998, 14392-14397) except that UDP-N-acetyl-glucosamine is detected by LC MS/MS using a Synergi RP Fusion column.

Preferred polypeptide having UDP-N-acetylglucosamine pyrophosphorylase activity in the present invention is an enzyme having an EC number of n° EC 2.7.7.23

According to a preferred embodiment, the one or more recombinant nucleic acids encoding a polypeptide having UDP-N-acetylglucosamine pyrophosphorylase activity may originate or be derived from organisms preferably selected in a group consisting of prokaryotic organisms and eukaryotic organisms. In some embodiments, the one or more recombinant nucleic acids encoding a polypeptide having UDP-N-acetylglucosamine pyrophosphorylase activity may originate or be derived from archaebacteria. In some embodiments, the one or more recombinant nucleic acids encoding a polypeptide having UDP-N-acetylglucosamine pyrophosphorylase activity may originate or be derived from organisms preferably selected from *Bacillus subtilis,* and yeasts. In some other preferred embodiments, the one or more recombinant nucleic acids encoding a polypeptide having UDP-N-acetylglucosamine pyrophosphorylase activity may originate or be derived from yeasts, and especially from *Saccharomyces cerevisiae.*

According to a yet preferred embodiment, the one or more recombinant nucleic acids encoding a polypeptide having UDP-N-acetylglucosamine pyrophosphorylase activity may be selected from the group consisting of nucleic acid sequences having (i) at least 65%, advantageously at least 70%, preferably at least 80%, nucleic acid identity with a nucleic acid sequence as set forth as SEQ ID NO: 77, and (ii) a biological activity of the same nature as the nucleic acid sequence as set forth as SEQ ID NO: 77. The nucleic acid sequence set forth as SEQ ID NO: 77 encodes a polypeptide having UDP-N-acetylglucosamine pyrophosphorylase activity originating from *Saccharomyces cerevisiae,* that may also be termed QRI1.

A biological activity of the same nature regarding this sequence is, as previously explained, the capacity to code for a polypeptide that converts N-acetyl-glucosmine-6-phosphate into UDP-N-acetyl-glucose.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UDP-N-acetylglucosamine pyrophosphorylase activity originating from *Saccharomyces cerevisiae,* the one skilled in the art may refer to the accession number NP_010180 in the UniProt database, or to SEQ ID NO. 78 described herein.

According to another particular embodiment, the one or more recombinant nucleic acids encoding a polypeptide having UDP-N-acetylglucosamine pyrophosphorylase activity may be nucleic acid(s) encoding polypeptide having an amino acid sequence selected from the group consisting of sequences having at least 35%, advantageously at least 45%, preferably at least 80%, amino acid identity with the amino acid sequence set forth as sequence SEQ ID NO. 78, and also a biological activity of the same nature as the amino acid sequence set forth as sequence SEQ ID NO. 78.

A biological activity of the same nature regarding this sequence is as described previously, i.e. the capacity to catalyze the conversion of of N-acetyl-glucosmine-6-phosphate into UDP-N-acetyl-glucose.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 45% amino acid identity with a reference amino acid sequence encompasses amino acid sequences having at least 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-N-acetylglucosamine pyrophosphorylase activity in the present invention is regulated by at least one promoter and at least one terminator, such as herein after defined more in details, which are present in 5' and 3' position respectively of the one or more recombinant nucleic acids encoding a polypeptide having UDP-N-acetylglucosamine pyrophosphorylase activity.

### PHOSPHOGLUCOMUTASE-1 (PGM1)

The Phosphoglucomutase-1 enzyme is a protein which is described in the art for catalyzing the conversion of Glucose-6-phosphate into Glucose-1-phosphate. The Phosphoglucomutase-1 originating from *Saccharomyces cerevisiae* may be termed PGM1.

A method implemented to measure the activity level of a polypeptide having Phosphoglucomutase-1 activity belongs to the general knowledge of the one skilled in the art.

In this regard, the one skilled in the art may advantageously refer to the method described by Tiwari and Bhat (Biochemical and Biophysical Research Communications 366, 340-345, 2008).

Preferred polypeptide having a Phosphoglucomutase-1 activity in the present invention is an enzyme having an EC number of n° 5.4.2.2.

According to a preferred embodiment, the one or more recombinant nucleic acids encoding a polypeptide having Phosphoglucomutase-1 activity may originate from organisms preferably selected in a group consisting of prokaryotic organisms and eukaryotic organisms. In some embodiments, the one or more recombinant nucleic acids encoding a polypeptide having Phosphoglucomutase-1 activity may originate or be derived from archaebacteria. In some embodiments, the one or more recombinant nucleic acids encoding a polypeptide having Phosphoglucomutase-1 activity may originate or be derived from organisms preferably selected from bacteria. In a preferred embodiment, the one or more recombinant nucleic acids encoding a polypeptide having Phosphoglucomutase-1 activity may orginate or be derived from *Saccharomyces cerevisiae.*

According to an embodiment, the one or more recombinant nucleic acids encoding a polypeptide having Phosphoglucomutase-1 activity may be selected from the group consisting of nucleic acid sequences having (i) at least 80% nucleic acid identity with the nucleic acid sequence set forth as sequence SEQ ID NO: 79, which originates from *Saccharomyces cerevisiae,* and (ii) a biological activity of the same nature as the nucleic acid sequence set forth as sequence SEQ ID NO: 79.

A biological activity of the same nature regarding this sequence is, as previously explained, the capacity to code for a polypeptide that converts Glucose-6-phosphate into Glucose-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the peptide having a Phosphoglucomutase-1 activity from *Saccharomyces cerevisiae,* the one skilled in the art may refer to the accession number NP33401 in the UniProt database, or to SEQ ID NO: 80 described herein.

According to another particular embodiment, the one or more recombinant nucleic acids encoding a polypeptide having Phosphoglucomutase-1 activity may be nucleic acid(s) encoding polypeptide having an amino acid sequence selected from the group consisting of sequences having at least 80% amino acid identity with the amino acid sequence set forth as sequence SEQ ID NO: 80, and also a biological activity of the same nature as the amino acid sequence set forth as sequence SEQ ID NO: 80.

A biological activity of the same nature regarding this sequence is as described previously, i.e. the capacity to catalyze the conversion of Glucose-6-phosphate into Glucose-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having a Phosphoglucomutase-1 activity in the present invention is regulated by at least one promoter and at least one terminator, such as herein after defined more in details, which are present in 5' and 3' position respectively of the one or more recombinant nucleic acids encoding a polypeptide having a Phosphoglucomutase-1 activity.

### UTP-GLUCOSE-1-PHOSPHATE URIDYLYLTRANSFERASE (UGP1)

The UTP-glucose-1-phosphate uridylyltransferase enzyme is a protein which is described in the art for catalyzing the conversion of Glucose-1-Phosphate into UDP-glucose. The UTP-glucose-1-phosphate uridylyltransferase originating from *Saccharomyces cerevisiae* may be termed UGP1.

A method implemented to measure the activity level of a polypeptide having a UTP-glucose-1-phosphate uridylyltransferase activity belongs to the general knowledge of the one skilled in the art.

In this regard, the one skilled in the art may advantageously refer to the method described by Roeben (J. Mol. Biol 364, 551-560, 2006).

Preferred polypeptide having a UTP-glucose-1-phosphate uridylyltransferase activity in the present invention is an enzyme having an EC number of n° 2.7.7.9.

According to a preferred embodiment, the one or more recombinant nucleic acids encoding a polypeptide having UTP-glucose-1-phosphate uridylyltransferase activity mayoriginate or be derived from organisms preferably selected in a group consisting of prokaryotic organisms and eukaryotic organisms. In some embodiments, the one or more recombinant nucleic acids encoding a polypeptide having UTP-glucose-1-phosphate uridylyltransferase activity may originate or be derived from archaebacteria. In some embodiments, the one or more recombinant nucleic acids encoding a polypeptide having UTP-glucose-1-phosphate uridylyltransferase activity may originate or be derived from organisms preferably selected from bacteria. In a preferred embodiment, the one or more recombinant nucleic acids encoding a polypeptide having UTP-glucose-1-phosphate uridylyltransferase activity may originate or be derived from *Saccharomyces cerevisiae.*

According to a particular embodiment, the one or more recombinant nucleic acids encoding a polypeptide having a UTP-glucose-1-phosphate uridylyltransferase activity may be selected from the group consisting of nucleic acid sequences having (i) at least 80% nucleic acid identity with a nucleic acid as set forth in SEQ ID NO: 81,originating from *Saccharomyces cerevisiae,* and (ii) a biological activity of the same nature as nucleic acid as set forth in SEQ ID NO: 81.

A biological activity of the same nature regarding this sequence is, as previously explained, the capacity to code for a polypeptide that converts Glucose-1-phosphate into UDP-glucose.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having a UTP-glucose-1-phosphate uridylyltransferase activity originating from *Saccharomyces cerevisiae,* the one skilled in the art may refer to the accession number NP_32861 in the UniProt database, or to the sequence set forth as sequence SEQ ID NO: 82 described herein.

According to another particular embodiment, the one or more recombinant nucleic acids encoding a polypeptide having UTP-glucose-1-phosphate uridylyltransferase activity may be nucleic acid(s) encoding polypeptide having an amino acid sequence selected from the group consisting of sequences having at least 80% amino acid identity with the amino acid sequence set forth as SEQ ID NO: 82, and also a biological activity of the same nature as the amino acid sequence set forth as SEQ ID NO: 82.

A biological activity of the same nature regarding this sequence is as described previously, i.e. the capacity to catalyze the conversion of Glucose-1-phosphate into UDP-glucose.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having a UTP-glucose-1-phosphate uridylyltransferase activity in the present invention is regulated by at least one promoter and at least one terminator, such as herein after defined more in details, which are present in 5' and 3' position respectively of the one or more recombinant nucleic acids encoding a polypeptide having a UTP-glucose-1-phosphate uridylyltransferase activity.

### GLUCOSAMINE-6-PHOSPHATE N-ACETYLTRANSFERASE (GNA1)

The Glucosamine-6-phosphate N-acetyltransferase enzyme is a protein which is described in the art for catalyzing the conversion of Glucosamine-6-phosphate into N-acetyl-glucosamine-6-phosphate. The Glucosamine-6-phosphate N-acetyltransferase originating from *Saccharomyces cerevisiae* may be termed GNA1.

A method implemented to measure the activity level of a polypeptide having a Glucosamine-6-phosphate N-acetyltransferase activity belongs to the general knowledge of the one skilled in the art.

In this regard, the one skilled in the art may advantageously refer to the method described by Li et al. (Anal. Biochem. 370, 142-146, 2007).

Preferred polypeptide having a Glucosamine-6-phosphate N-acetyltransferase activity in the present invention is an enzyme having an EC number of n° 2.3.1.4.

According to a preferred embodiment, the one or more recombinant nucleic acids encoding a polypeptide having Glucosamine-6-phosphate N-acetyltransferase activity may originate or be derived from organisms preferably selected in a group consisting of prokaryotic organisms and eukaryotic organisms. In some preferred embodiments, the one or more recombinant nucleic acids encoding a polypeptide having Glucosamine-6-phosphate N-acetyltransferase activity may originate or be derived from a yeast, and especially from *Saccharomyces cerevisiae.*

According to a particular embodiment, the one or more recombinant nucleic acids encoding a polypeptide having a Glucosamine-6-phosphate N-acetyltransferase activity may be selected from the group consisting of nucleic acid sequences having (i) at least 80% nucleic acid identity with a nucleic acid sequence as set forth as sequence SEQ ID NO: 83, and (ii) a biological activity of the same nature as the nucleic acid sequence as set forth as sequence SEQ ID NO: 83. The nucleic acid sequence as set forth as sequence SEQ ID NO: 83 encodes a polypeptide having a Glucosamine-6-phosphate N-acetyltransferase activity originating from *Saccharomyces cerevisiae,* that may also be termed GNA1.

A biological activity of the same nature regarding this sequence is, as previously explained, the capacity to code for a polypeptide that converts Glucosamine-6-phosphate into N-acetyl-glucosamine-6-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having a Glucosamine-6-phosphate N-acetyltransferase activity obtained or derived from *Saccharomyces cerevisiae,* the one skilled in the art may refer to the accession number NP_116637 in the UniProt database, or to SEQ ID NO. 84 described herein.

According to another particular embodiment, the one or more recombinant nucleic acids encoding a polypeptide having Glucosamine-6-phosphate N-acetyltransferase activity may be nucleic acid(s) encoding polypeptide having an amino acid sequence selected from the group consisting of sequences having at least 80% amino acid identity with the amino acid sequence set forth as SEQ ID NO. 84, and also a biological activity of the same nature as the amino acid sequence set forth as SEQ ID NO: 84.

A biological activity of the same nature regarding this sequence is as described previously, i.e. the capacity to catalyze the conversion of Glucosamine-6-phosphate into N-acetyl-glucosamine-6-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having a Glucosamine-6-phosphate N-acetyltransferase activity in the present invention is regulated by at least one promoter and at least one terminator, such as herein after defined more in details, which are present in 5' and 3' position respectively of the one or more recombinant nucleic acids encoding a polypeptide having a Glucosamine-6-phosphate N-acetyltransferase activity.

### PHOSPHOACETYLGLUCOSAMINE MUTASE (PCM1)

The phosphoacetylglucosamine mutase enzyme is a protein which is described in the art for catalyzing the conversion of N-acetyl-glucosamine-6-phosphate into N-acetyl-glucosamine-1-phosphate. The phosphoacetylglucosamine mutase originating from *Saccharomyces cerevisiae* may be termed PCM1.

A method implemented to measure the activity level of a polypeptide having phosphoacetylglucosamine mutase activity belongs to the general knowledge of the one skilled in the art.

In this regard, the one skilled in the art may advantageously refer to the method described by Bandini et al. (Molecular Microbiology 85(3), 513-534, 2012).

Preferred polypeptide having a phosphoacetylglucosamine mutase activity in the present invention is an enzyme having an EC number of n° 5.4.2.3.

According to a preferred embodiment, the one or more recombinant nucleic acids encoding a polypeptide having phosphoacetylglucosamine mutase activity may originate or be derived from organisms preferably selected in a group consisting of prokaryotic organisms and eukaryotic organisms. In some preferred embodiments, the the one or more recombinant nucleic acids encoding a polypeptide having phosphoacetylglucosamine mutase activity may originate or be derived from a yeast, and especially from *Saccharomyces cerevisiae.*

According to a particular embodiment, the the one or more recombinant nucleic acids encoding a polypeptide having a phosphoacetylglucosamine mutase activity may be selected from the group consisting of nucleic acid sequences having (i) at least 80% nucleic acid identity with a nucleic acid sequence set forth as SEQ ID NO: 85, and (ii) a biological activity of the same nature as the nucleic acid sequence set forth as SEQ ID NO: 85. The nucleic acid set forth as SEQ ID NO: 85 encodes a polypeptide having phosphoacetylglucosamine mutase activity originating from *Saccharomyces,* that may also be termed PCM1.

A biological activity of the same nature regarding this sequence is, as previously explained, the capacity to code for a polypeptide that converts N-acetyl-glucosamine-6-phosphate into N-acetyl-glucosamine-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having a phosphoacetylglucosamine mutase activity originating from *Saccharomyces cerevisiae,* the one skilled in the art may refer to the accession number NP_010856 in the UniProt database, or to SEQ ID NO: 86 described herein.

According to another particular embodiment, the one or more recombinant nucleic acids encoding a polypeptide having phosphoacetylglucosamine mutase activity may be nucleic acid(s) encoding polypeptide having an amino acid sequence selected from the group consisting of sequences having at least 80% amino acid identity with the amino acid sequence set forth as SEQ ID NO: 86, and also a biological activity of the same nature as the amino acid sequence set forth as SEQ ID NO: 86.

A biological activity of the same nature regarding this sequence is as described previously, i.e. the capacity to catalyze the conversion of N-acetyl-glucosamine-6-phosphate into N-acetyl-glucosamine-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having a phosphoacetylglucosamine mutase activity in the present invention is regulated by at least one promoter and at least one terminator, such as herein after defined more in detail, which are present in 5' and 3' position respectively of the one or more recombinant nucleic acids encoding a polypeptide having a phosphoacetylglucosamine mutase activity.

### PROMOTERS

As disclosed herein, the expression of the genes of interest that have been genetically engineered for obtaining a recombinant cell according to the invention comprise appropriate regulatory sequences that are functional in the recombinant cells of the invention, and in particular in recombinant yeast cells of the invention, including in particular *Saccharomyces cerevisiae.*

Various promoters may be used for the desired expression of the coding sequences of interest.

Promoters according to the invention may be selected from the group consisting of the following promoters:
- pTDH3 (SEQ ID NO: 87),
- pTDH3.Sk (SEQ ID NO: 88)
- pTDH3-1.Sba (SEQ ID NO: 89),
- pTDH3.Sar (SEQ ID NO: 90),
- pENO2 (SEQ ID NO: 91),
- pTEF3 (SEQ ID NO: 92),
- pTEF1 (SEQ ID NO: 93),
- pTEF1.Ago (SEQ ID NO: 94),
- pTEF1.sba (SEQ ID NO: 95),
- pPDC1 (SEQ ID NO: 96),
- pCCW12 (SEQ ID NO: 97),
- pCCW12.Sm (SEQ ID NO: 98),
- pCCW12.sk (SEQ ID NO: 99),
- pCCW12.sba (SEQ ID NO: 100),
- pCCW12.sar (SEQ ID NO: 101),
- pNUP57 (SEQ ID NO: 102),
- pCCW10.ago (SEQ ID NO: 103),
- pCWP2 (SEQ ID NO: 104),
- pFBA1 (SEQ ID NO: 105),
- pCCW120.Sm (SEQ ID NO: 106).

Promoters more particularly interesting in the present invention may be selected from the group consisting of:
- pTDH3 (SEQ ID NO: 87)
- pTDH3.Sk (SEQ ID NO: 88)
- pTDH3-1.Sba (SEQ ID NO: 89)
- pTDH3.Sar (SEQ ID NO: 90),
- pENO2 (SEQ ID NO: 91),
- pTEF3 (SEQ ID NO: 92),
- pTEF1 (SEQ ID NO: 93),
- pTEF1.Ago (SEQ ID NO: 94),
- pTEF1.sba (SEQ ID NO: 95),
- pPDC1 (SEQ ID NO: 96),
- pCCW12 (SEQ ID NO: 97),,
- pCCW12.sba (SEQ ID NO: 100)
- pCCW120.Sm (SEQ ID NO: 106),
- pNUP57 (SEQ ID NO: 102),
- pCCW10.ago (SEQ ID NO: 103),
- pCWP2 (SEQ ID NO: 104), and
- pFBA1 (SEQ ID NO: 105)

Said promoters can in particular be selected from the group consisting of pTDH3, pTDH3.Sk, pTDH3-1.Sba, pTEF1, pTEF1.Ago, pTEF1.sba, pCCW12, pCCW120.Sm, pCWP2 and pFBA1.

Alternatively, promoters interesting in the present invention may be selected from the group consisting of:
- pNUP57 (SEQ ID NO: 102), and
- pCCW10.ago (SEQ ID NO: 103).

Another promoter of interest in the present invention may be:
- pCWP2 (SEQ ID NO: 104).

As previously mentioned, inducible or repressible promoters are promoters whose activity is controlled by the presence or absence of biotic or abiotic factors and also by the quantity of said factor. Accordingly, for some promoters, their activity will in particular be induced and thus increased when the quantity of a given factor increases or is increased, and, accordingly, the activity of these same promoters can be repressed and thus reduced when the quantity of said factor diminishes or is reduced. The quantity of said factor(s) in the culture medium of a recombinant yeast cell of the invention comprising inducible or repressible promoters can be decided and thus controlled by the man skilled in the art.

For example, increasing the quantity of copper in a culture medium of a recombinant yeast cell according to the invention comprising a pCUP1 promoter will induce and thus increase transcription of the gene under the control of this promoter. On the contrary, reducing the quantity of copper in said culture medium will lead to a repression, and thus a reduced, transcription of the gene under the control of this promoter.

In another example, increasing the quantity of methionine in a culture medium of a recombinant yeast cell according to the invention comprising a pMET6 promoter will repress and thus decrease transcription of the gene under the control of this promoter. On the contrary, reducing the quantity of methionine in said culture medium will lead to an induced, and thus an increased, transcription of the gene under the control of this promoter.

For this reason, the following promoters are referred to in the present text as being inducible or repressible promoters.

According to a first embodiment, inducible or repressible promoters according to the invention may be selected from the group comprising promoters inducible or repressible with copper, promoters inducible or repressible with methionine and promoters inducible or repressible with threonine, and are in particular CUP-1 - copper inducible or repressible.

According to this embodiment, the inducible or repressible promoter according to the invention can in particular be pCUP1 (SEQ ID NO: 107).

The activity of these promoters is thus induced by the increasing presence of methionine, copper or threonine as indicated above, and their activity diminishes, i.e. is repressed, when the quantity of methionine, copper or threonine is reduced.

According to a second embodiment, inducible or repressible promoters according to the invention may be selected from the group comprising promoters inducible or repressible with copper, promoters inducible or repressible with lysine and promoters inducible or repressible with methionine, and in particular selected from the group consisting of:
- pMET6 - methionine inducible or repressible (SEQ ID NO: 108),
- pMET25 - methionine inducible or repressible (SEQ ID NO: 109), and
- pSAM1 - methionine inducible or repressible (SEQ ID NO: 110).

According to this particular embodiment, the inducible or repressible promoter according ot the invention may, be selected from the group consisting of pMET6 and pSAM1.

The activity of these promoters is thus repressed by the increasing presence of methionine, copper, lysine or glucose as indicated above, and their activity increases, i.e. is induced, when the quantity of methionine, copper, lysine or glucose is reduced.

In a particular embodiment, inducible or repressible promoters according to the invention may be selected from the group comprising promoters inducible or repressible with copper, promoters inducible or repressible with glucose, promoters inducible or repressible with lysine, promoters inducible or repressible with methionine and promoters inducible or repressible with threonine.

Synthetic promoters as described in Blazeck & Alper (2013) Biotechnol. J. 8 46-58 can also be used.

The promoters of the invention can originate from any organism from the Saccharomycetes class and can in particular originate from an organism selected from the group consisting of at least one of *Saccharomyces cerevisiae, Saccharomyces boulardii, Saccharomyces castelii, Saccharomyces bayanus, Saccharomyces arboricola, Saccharomyces kudriavzevii, Saccharomyces mikatae, Ashbya gossypii, Kluveromyces lactis, Pichia pastoris, Candida glabrata, Candida tropicalis, Debaryomyces castelii, Yarrowia lipolitica,* and *Cyberlindnera jadinii.*

The promoters of the invention can preferably originate from an organism selected from the group consisting of *Saccharomyces cerevisiae (sc), Saccharomyces mikatae (Sm), Saccharomyces kudriavzevii (sk), Saccharomyces bayanus (sba), Saccharomyces arboricola (Sar),* and *Ashbya gossypii (Ago).*

### TERMINATORS

As it is disclosed herein, the expression of the genes of interest that have been genetically engineered for obtaining a recombinant cell according to the invention, and in particular a recombinant yeast according to the invention comprise appropriate transcription terminator sequences that are functional in recombinant cells of the invention, and in particular in recombinant yeast cells of the invention, in particular in *Saccharomyces cerevisiae.*

Said transcription terminators, identical or different, may be found in literature Yamanishi et al., (2013) ACS synthetic biology 2, 337-347.

Terminators more particularly interesting in the present invention may be selected from the group comprising:
- tTPI1 from the gene encoding for the Triose Phosphate Isomerase (SEQ ID NO: 111),
- tMET25 from the gene encoding for the O-acetyl homoserine-O-acetyl serine sulfhydrylase (SEQ ID NO: 112),
- tDIT1 (SEQ ID NO: 113),
- tRPL3 (SEQ ID NO: 114),
- tRPL3.sm (SEQ ID NO: 115),
- tRPL3.sba (SEQ ID NO: 116),
- tRPL41B (SEQ ID NO: 117),
- tRPL41B.Sba (SEQ ID NO: 118)
- tRPL15A (SEQ ID NO: 119),
- tRPL15A.Sm (SEQ ID NO: 120),
- tRPL15A.sba (SEQ ID NO: 121),
- tIDP1 (SEQ ID NO: 122),
- tIDP1.Sba (SEQ ID NO: 123), and
- tTEF1.sba (SEQ ID NO: 124).

Terminators more particularly interesting in the present invention may be selected from the group consisting in:
- tTPI1 from the gene encoding for the Triose Phosphate Isomerase (SEQ ID NO: 111),
- tMET25 from the gene encoding for the O-acetyl homoserine-O-acetyl serine sulfhydrylase (SEQ ID NO: 112)
- tDIT1 (SEQ ID NO: 113)
- tRPL3 (SEQ ID NO: 114)
- tRPL3.Sm (SEQ ID NO: 115)
- tRPL3.sba (SEQ ID NO: 116)
- tRPL41B (SEQ ID NO: 117)
   tRPL41B.Sba (SEQ ID NO: 118)
- tRPL15A (SEQ ID NO: 119)
- tRPL15A.Sm (SEQ ID NO: 120)
- tIDP1 (SEQ ID NO: 122)
- tIDP1.Sba (SEQ ID NO: 123), and
- tTEF1.sba (SEQ ID NO: 124).

In particular, said terminator may be selected from the group consisting of tTPI1, tDIT11, tRPL3, tRPL3.Sm, tRPL41B, tRPL41B.Sba, tRPL15A, tRPL15A.Sm, tIDP1, tIDP1.Sba and tTEF1.sba.

The terminators of the invention can originate from any organism from the Saccharomycetes class and can in particular originate from an organism selected from the group consisting of *Saccharomyces cerevisiae (sc), Saccharomyces mikatae (Sm),* and *Saccharomyces Bayanus (sba).*

### RECOMBINANT CELLS

Recombinant cells of the invention can be selected from the group consisting of yeasts and bacteria.

Recombinant cells of the invention, such as a recombinant host cell of the invention, are preferably recombinant yeast cells.

Generally, yeast can grow rapidly and can be cultivated at higher density as compared with bacteria, and does not require an aseptic environment in the industrial setting. Furthermore, yeast cells can be more easily separated from the culture medium compared to bacterial cells, greatly simplifying the process for product extraction and purification.

A recombinant cell of the invention, and in particular a recombinant yeast cell of the invention is preferably a *Saccharomycetales* cell.

A recombinant cell of the invention, and in particular a recombinant yeast of the invention can in particular belong to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

A recombinant cell of the invention belonging to the *Saccharomyces* genus can be selected from the group consisting of *Saccharomyces cerevisiae, Saccharomyces boulardii, Saccharomyces bayanus, Saccharomyces paradoxus, Saccharomyces mikatae, Saccharomyces castelli, Saccharomyces cariocanus, Saccharomyces kudriavzevii, Saccharomyces arboricolus, Saccharomyces pastorianus, Saccharomyces uvarum* and *Saccharomyces delbrueckii.*

A recombinant cell of the invention belonging to the *Candida* genus can be selected from the group consisting of *Candida albicans, Candida glabrata, Candida tropicalis, Candida dubliniensis, Candida parapsilosis, Candida lusitaniae* and *Candida guilliermondii.*

A recombinant cell of the invention belonging to the *Kluyveromyces* genus can be selected from the group consisting of *Kluyveromyces lactis, Kluyveromyces marxianus, Kluyveromyces polysporus, Kluyveromyces thermotoleren, Kluyveromyces dobzhanskii* and *Kluyveromyces wickerhamii.*

A recombinant cell of the invention belonging to the *Ogataea* genus can be selected from the group consisting of *Ogataea polymorpha, Ogataea histrianica, Ogataea deakii, Ogataea kolombanensis, Ogataea philodendra, Ogataea siamensis, Ogataea angusta, Ogataea parapolymorpha, Ogataea minuta, Ogataea nonfermentans* and *Ogataea kodamae.*

A recombinant cell of the invention belonging to the *Yarrowia* genus can be selected from the group consisting of *Yarrowia lypolytica, Yarrowia parophonii, Yarrowia galli, Yarrowia oslonensis, Yarrowia alimentaria, Yarrowia hollandica* and *Yarrowia yakushimensis.*

A recombinant cell of the invention belonging to the *Debaryomyces* genus can be selected from the group consisting of *Debaryomyces hansenii, Debaryomyces carsonii, Debaryomyces castellii, Debaryomyces marama, Debaryomyces occidentalis, Debaryomyces oviformis, Debaryomyces nepalensis, Debaryomyces coudertii, Debaryomyces udenii, Debaryomyces psychrosporus* and *Debaryomyces yamadae.*

A recombinant cell of the invention belonging to the *Ashbya* genus can be selected from the group consisting of *Ashbya gossypii* and *Ashbya aceri.*

A recombinant cell of the invention, and in particular a recombinant yeast cell of the invention, can in particular be selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

In a particular embodiment, the recombinant yeast cell according to the invention is of the genus *Saccharomyces, Kluyveromyces,* or *Eremothecium,* more particularly is of the species selected from the group consisting of *Saccharomyces cerevisiae, Kluyveromyces Marxianus, Ogataea polymorpha* and *Ashbya gossypii.*

In an embodiment, a recombinant host cell of the invention is a yeast selected belonging to the *Saccharomycetales* order, in particular to the *Saccharomycetaceae* family, and is in particular selected from the group consisting of *Yarrowia lipolitica, Kluyveromyces Marxianus, Ogataea polymorpha, Ashbya gossypii and Saccharomyces cerevisiae.*

A recombinant cell of the invention can most preferably be a *Saccharomyces cerevisiae* cell.

As above-mentioned, a recombinant cell according to the invention has the ability to produce chondroitin by insertion of the one or more recombinant nucleic acids according to the present invention. In certain embodiments, a recombinant yeast according to the invention has the ability to produce chondroitin having a controlled size (controlled molecular weight) by insertion of the one or more recombinant nucleic acids according to the present invention.

Methods implemented to insert a specific DNA construct within a gene belong to the general knowledge of a man skilled in the art. A related method is described in more details in the herein after examples.

However, unexpected technical difficulties were encountered because the consequences of the insertion of DNA constructs within the genome of a cell, and in particular within the genome of a yeast, such as for example in the genome of *Saccharomyces cerevisiae,* are unpredictable. In particular, the survival rate of the cells, and in particular of the yeasts, and their ability to grow and produce the desired chondroitin is also unpredictable.

In order to obtain a recombinant cell, and in particular a recombinant yeast of the invention, many different constructs were tested by the inventors in order to obtain a viable and efficient recombinant cell, and in particular yeast.

### CULTURE CONDITIONS

The present invention also relates to the use of a recombinant cell of the invention, for the production of chondritin, in particular of chondroitin of controlled molecular weight.

The present invention further relates to a method of producing chondroitin of a desired molecular weight, comprising:
(a) cultivating a recombinant cell as defined herein in a cultivation medium for a time sufficient to produce chondroitin of the desired molecular weight; and
(b) optionally isolating or recovering the chondroitin from the recombinant cell and/or from the cultivation medium. Typically, cells of the invention, and in particular yeasts of the invention, are grown at a temperature in the range of about 20°C to about 37°C, preferably at a temperature ranging from 27 to 34°C, in an appropriate culture medium.

Suitable growth media for cells of the invention, and in particular for yeasts of the invention, are common commercially prepared media such as broth that includes yeast nitrogen base, ammonium sulfate, and dextrose as the carbon/energy source or YPD Medium, a blend of peptone, yeast extract, and dextrose in optimal proportions for growing most. Other defined or synthetic growth media may also be used and the appropriate medium for growth of the particular cell, and in particular yeast, will be known by one skilled in the art of microbiology or fermentation science.

A particular medium that is suitable herein is the SY medium which comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeCl3: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM ; Nicotinic acid (C6H5NO2): 29 µM; D-Biotine: 0,82 µM; Ammonium citrate tribasic: 33 mM; and glucose or sucrose 2-30%.

Carbon sources that may be used in the culture medium include fructose, mannose, xylose and arabinose; oligosaccharides such as lactose, maltose, galactose, or sucrose; polysaccharides such as starch or cellulose or mixtures thereof; and unpurified mixtures from renewable feedstocks such as cheese whey permeate cornsteep liquor, sugar beet molasses, and barley malt.

Nitrogen sources that may be included in the culture medium include peptone, yeast extracts, meat extracts, malt extracts, urea, ammonium sulfate, ammonium chloride, ammonium nitrate, ammonium phosphate, ammonium citrate and combinations thereof.

The culture medium may further comprise trace elements (e.g., metal salts), for example magnesium salts, cobalt salts and/or manganese salts; as well as growth factors such as amino acids, vitamins, growth promoters, and the like.

Examples of vitamins that may be included are Panthotenic Acids hemicalcium, Thiamin hydrochloride; Pyridoxin hydrochloride; Myo-Inositol; Nicotinic acid; D-Biotine, folic acid, p-aminobenzoïque acid, riboflavin.

A culture medium of the invention may further include rare elements, such as CuSO₄. 5H₂O, KI, FeCl₃, ZnSO₄ ·7H₂O, MnSO₄ ·H₂O, or H₂SO₄, MgCl₂, CaCl₂, NaCl, K2HPO4, KH2PO4, ZnCl, H3BO3, MnSO4, Na2MoO4.

The term "appropriate culture medium" is above-defined.

Examples of known culture media for a recombinant cell according to the present invention are known to the person skilled in the art, and are presented in the following publication D. Burke et al., Methods in yeast Genetics - A cold spring harbor laboratory course Manual (2000).

Suitable pH ranges for the fermentation may be between pH 3.0 to pH 7.5, where pH 4 to pH 6 is preferred as the initial condition.

As mentioned elsewhere in this specification, the pH value of the culture medium can be regulated during the cultivation step of a method of the invention in order to modulate the activity of the polypeptide having chondroitin activity, which will impact the molecular weight of the chondroitin molecules produced by the recombinant cell of the invention, and in particular by the recombinant yeast of the invention..

In particular, the pH of the culture medium can be modified according to the chondroitin that is meant to be produced by the recombinant yeast. For example, the pH of the culture medium may remain at a pH of 4, of 5,5 or of 6 during the culture time.

In a particular embodiment, the pH of the culture medium may change or be changed during the time length of the culture of the recombinant cell of the invention, in particular of the recombinant yeast of the invention. As previously mentioned, *Saccharomyces cerevisiae* acidifies the medium in which it is cultivated and thus reduces the pH of its culture medium. For example, the pH of the culture medium may begin at 6, may be taken down to 4 and then brought back up to 6. In another example, the pH of the culture medium may begin at 6, remain or be maintained at 6, and then may be lowered to 4..

In a particular embodiment, the pH of the culture medium may be modulated during the cultivating step (a) of a method of the invention so that at the end of the cultivating step of a method of the invention, the pH of the cultivation medium is the same as the pH at the beginning of said cultivation step (a).

In another embodiment, the pH of the culture medium may remain the same during the time length of the culture of the recombinant cell of the invention, and in particular the recombinant yeast cell of the invention.

Said time length of the culture of a recombinant cell according to the invention, and in particular of the recombinant yeast of the invention, can vary depending on the molecular weight of the chondroitin of interest. The longer said time length, the lower the molecular weight of chondroitin in a given culture medium of a recombinant cell of the invention, and in particular a recombinant yeast cell of the invention.

The time length of the culture time of a recombinant cell of the invention, and in particular a recombinant yeast cell in the present invention can be a period of from about 35 hours to about 50 hours, preferably from about 40 hours to about 50 hours, and is in particular about 48 hours.

Fermentations may be performed under aerobic conditions or micro-aerobic conditions.

The amount of chondroitin product in the fermentation medium can be determined using a number of methods known in the art, for example, high performance liquid chromatography (HPLC) or gas chromatography (GC).

The present process may employ a batch method of fermentation. A classical batch fermentation is a closed system where the composition of the medium is set at the beginning of the fermentation and not subject to artificial alterations during the fermentation. Thus, at the beginning of the fermentation, the medium is inoculated with the desired organism or organisms, and fermentation is permitted to occur without adding anything to the system. Typically, however, a "batch" fermentation method or system is batch with respect to the addition of carbon source and attempts are often made at controlling factors such as temperature, pH and oxygen concentration. In batch systems, the metabolite and biomass compositions of the system change constantly up to the time when the fermentation is stopped. Within batch cultures cells progress through a static lag phase to a high growth log phase and finally to a stationary phase where growth rate is diminished or halted. If untreated, cells in the stationary phase will eventually die. Cells in log phase generally are responsible for the bulk of production of end product or intermediate.

A Fed-Batch system may also be used in the present invention. A Fed-Batch system is similar to a typical batch system with the exception that the carbon source substrate is added in increments as the fermentation progresses. Fed-Batch systems are useful when catabolite repression (e.g. glucose repression) is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂.

Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA , or Deshpande, Mukund V., Appl. Biochem. Biotechnol., 36, 227, (1992). Although the present invention is performed in batch mode it is contemplated that the method would be adaptable to continuous fermentation.

Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to vary. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology.

It is contemplated that the present invention may be practiced using either batch, fed- batch or continuous processes and that any known mode of fermentation would be suitable. Additionally, it is contemplated that cells may be immobilized on a substrate as whole cell catalysts and subjected to fermentation conditions for production.

In order to still improve the chondroitin production, a particular embodiment may consist of culturing the recombinant cells of the invention, in particular the recombinant yeast cells of the invention, in an appropriate culture medium, such as above-mentioned, wherein the said culture medium comprises an optimal amount of carbon source, especially glucose or sucrose.

In preferred embodiments, the carbon source comprised in said optimal culture medium consists of glucose and/or sucrose. In preferred embodiments, the said optimal culture medium comprises 1% w/w or more glucose and/or sucrose, in particular comprises 5% w/w or more glucose and/or sucrose, in particular comprises 10% w/w or more glucose and/or sucrose, in particular comprises 15% w/w or more glucose and/or sucrose. In preferred embodiments, the said optimal culture medium comprises at most 40% w/w glucose, which includes at most 35% w/w glucose.

In a preferred embodiment, the method of the invention is carried out on an industrial scale.

More particularly, the cultivation medium of the method according to the invention can be at least about 100L, more preferably in the range of about 1,000L to about 3,000L, even more preferably about 10,000L, even more preferably 100,000L, or even about 250,000L.

The invention further relates to a method for producing chondroitin as previously described, and comprising the steps of:
(a) culturing a recombinant cell of the invention in a culture medium; and
(b) recovering the chondroitin from said culture medium,
   wherein the chondroitin recovered in step (b) has a molecular weight controlled through the selection of:
   - the nature and origin of the one or more recombinant nucleic acids encoding a polypeptide having a chondroitinase activity of the recombinant cell of the invention, and in particular of the recombinant yeast of the invention,
   - the nature and origin of the promoter controlling the expression of the one or more recombinant nucleic acids encoding a polypeptide having a chondroitin activity of the recombinant cell of the invention, and in particular of the recombinant yeast of the invention,
   - the presence or the absence of an anchoring signal associated to one or more recombinant nucleic acids encoding a polypeptide having chondroitin activity of the recombinant cell of the invention, and in particular of the recombinant yeast of the invention,
   - the pH of the culture medium during the step of culturing the recombinant cell of the invention, and in particular of the recombinant yeast of the invention, and/or
   - the duration of the culturing of the recombinant cell of the invention, and in particular of the recombinant yeast of the invention.

The molecular weight of the chondroitin can be a particular molecular weight or more preferably a specific range of molecular weights such as, for example, less than 50 kDa, in the range of about 20 kDa to about 50 kDa, greater than or equal to 50 kDa, in the range of about 50 kDa to about 150 kDa, in the range of about 50 kDa to about 250 kDa, greater than or equal to 100 kDa, in the range of about 100 kDa to about 1500 kDa, in the range of about 150 kDa to about 1500 kDa, greater than 1000 kDa or greater than 1500 kDa.

The present invention also relates to the use of a recombinant cell according to the invention, and in particular a recombinant yeast of the invention, for the production of chondroitin having a molecular weight in the range of from about 20kDa to about 50kDa or from about 50kDa to about 1000 kDa.

The one or more recombinant nucleic acids encoding a polypeptide having chondroitinase activity of a recombinant cell of the invention can for example be selected from those obtained or derived from at least one of *Cupiennius salei, Tityus serrulatus, Bos taurus, Apis mellifera, Macaca mulatta,* or *Vespa magnifica,* in particular from those of sequences set forth as sequences SEQ ID NO: 48 (Csa), SEQ ID NO: 49 (Ts), SEQ ID NO: 50 (Bt), SEQ ID NO: 51 (Am), SEQ ID NO: 52 (Mm) or SEQ ID NO: 53 (Vm) when they are secreted (i.e. in the presence of a secretion signal and in the absence of an anchoring signal).

The one or more recombinant nucleic acids encoding a polypeptide having chondroitinase activity of a recombinant cell of the invention can for example be selected from those obtained or derived from at least one of *Cupiennius salei, Tityus serrulatus, Bos taurus, Apis mellifera, Macaca mulatta,* or *Vespa magnifica,* in particular from those of sequences set forth as sequences SEQ ID NO: 54 (Csa), SEQ ID NO: 55 (Ts), SEQ ID NO: 56 (Bt), SEQ ID NO: 57 (Am), SEQ ID NO: 58 (Mm) or SEQ ID NO: 59 (Vm)when they are anchored (i.e. in the presence of both a secretion signal and an anchoring signal).

The one or more recombinant nucleic acids encoding a polypeptide having chondroitinase activity of a recombinant cell of the invention can for example be placed under the control of a promoter selected from the group consisting of pTDH3, pTDH3.Sk, pTDH3-1.Sba, pTEF1, pTEF1.Ago, pTEF1.sba, pCCW12, pCCW12.Sba, pCCW120.Sm, pCWP2, pCW10.Ago, pNUP57 and pFBA1, and in particular from the group consisting of pCCW12.Sba, pCW10.Ago and pNUP57.

In particular, the promoters of the one or more recombinant nucleic acids encoding a polypeptide having chondroitinase activity of a recombinant cell of the invention, and in particular of a recombinant yeast of the invention, may be obtained or derived from *Saccharomyces Bayanus, Saccharomyces kudriavzevii, Saccharomyces mikatae, Saccharomyces arboricola* or other saccharomycetales, or *Abishia gossypii.*

The secretion signal of the present invention may for example have:
- the nucleic acid sequence set forth as SEQ ID NO: 125; and/or
- the amino acid sequence set forth as SEQ ID NO: 126.

The anchoring signal of the present invention may for example have:
- the nucleic acid sequence set forth as SEQ ID NO: 127; and/or
- the amino acid sequence set forth SEQ ID NO: 128.

The secretion signal may be fused to the polypeptide having a chondroitinase activity by creating a chimeric nucleic acid starting by a nucleic acid sequence encoding the signal peptide followed by a recombinant nucleic acid encoding a polypeptide having chondroitinase activity as previously defined .

The secretion signal and anchoring signal may be fused to the polypeptide having chondroitinase activity by creating a chimeric nucleic acid starting by a nucleic acid sequence encoding the signal peptide followed by a recombinant nucleic acid encoding a polypeptide having chondroitinase activity as previously defined followed by a nucleic acid sequence encoding for the anchoring signal.

Such chimeric nucleic acid sequences may be obtained by techniques known by the man skilled in the art such as chemical synthesis of nucleic acids or any recombination techniques such as cloning or PCR.

The invention further relates to a method as previously described, for producing chondrotin having a molecular weight less than about 10 kDa, wherein:
(a) the pH of the medium is superior to 4, and is in particular in the range of from about 5 to about 7;
(b) the one or more recombinant nucleic acids encoding a polypeptide having chondrotinase activity integrated in the genome of the recombinant cell of the invention originates or is derived from *Tityus serrulatus,* possesses a secretion signal but is devoid of an anchoring signal, and is under the control of a pCCW12.sba promoter;
(c) the polypeptide having chondrotinase activity is a hyaluronidase;
(d) the step of recovering the chondroitin from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

The invention further relates to a method as previously described, for producing chondrotin having a molecular from about 10 kDa to about 50 kDa, wherein:
(a) the pH of the medium is superior to 4, and is in particular in the range of from about 5 to about 7;
(b) the one or more recombinant nucleic acids encoding a polypeptide having chondrotinase activity integrated in the genome of the recombinant cell of the invention originates or is derived from *Tityus serrulatus,* possesses a secretion signal but is devoid of an anchoring signal, and is under the control of a pCCW10.Ago promoter;
(c) the polypeptide having chondrotinase activity is a hyaluronidase;
(d) the step of recovering the chondroitin from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

The invention further relates to a method as previously described, for producing chondrotin having a molecular from about 50 kDa to about 500 kDa, wherein:
(a) the pH of the medium is superior to 4, and is in particular in the range of from about 5 to about 7;
(b) the one or more recombinant nucleic acids encoding a polypeptide having chondrotinase activity integrated in the genome of the recombinant cell of the invention originates or is derived from *Tityus serrulatus,* possesses a secretion signal but is devoid of an anchoring signal, and is under the control of a pNUP57 promoter;
(c) the polypeptide having chondrotinase activity is a hyaluronidase;
(d) the step of recovering the chondroitin from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

Another aspect of the present invention relates to chondroitin obtained or obtainable from a recombinant cell of the invention or from a method according to the invention.

A further aspect of the invention is a cultivation medium comprising the chondroitin of the invention.

The invention further relates to a composition comprising the chondroitin according to the invention.

The invention also relates to an industrial product or a consumer product or a consumable comprising (i) chondroitin of the invention (ii) the cultivation medium comprising chondroitin of the invention or (iii) the composition comprising chondroitin of the invention.

In particular, said industrial product or consumer product or consumable according to the invention can be a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.

### PURIFICATION OF CHONDROITIN

According to a specific aspect of the invention, the fermentative production of chondroitin preferably comprises a step of isolation of the chondroitin produced from the culture medium. Recovering the chondroitin from the culture medium is a routine task for a man skilled in the art. It may be achieved by a number of techniques well known in the art including but not limiting to pervaporation, selective precipitation, filtration, centrifugation, spray drying, lyophylisation or liquid extraction. The expert in the field knows how to adapt parameters of each technique dependant on the characteristics of the material to be separated.

The yeast as model of a cell in the present invention is preferred in that the synthesized chondroitin is/are entirely exported outside the cells, thus simplifying the purification process.

Gas stripping is achieved with a stripping gas chosen among helium, argon, carbon dioxide, hydrogen, nitrogen or mixture thereof.

Liquid extraction is achieved with organic solvent as the hydrophobic phase such as pentane, hexane, heptane or dodecane. Renewal solvents may also be used.

### FORMULATIONS AND PRODUCTS

A composition of the invention may be incorporated into a formulation/product, such as a nutraceutical, pharmaceutical, veterinary, oenological or cosmetic formulation/product.

Thus, the present invention provides a formulation comprising a composition of the invention. For example, the present invention may provide a cosmetic formulation comprising the composition of the invention.

The present invention also provides a product comprising a composition of the invention. For example, the present invention may provide a cosmetic product comprising the composition of the invention.

A "cosmetic product" is intended to mean any substance or mixture intended to be placed in contact with the external parts of the human body (epidermis, hair system, nails, lips and external genital organs) or with the teeth and the mucous membranes of the oral cavity with a view exclusively or mainly to cleaning them, perfuming them, changing their appearance, protecting them, keeping them in good condition, correcting body odours and/or combinations thereof.

A "substance" is intended to mean a chemical element and its compounds in the natural state or obtained by any manufacturing process, including any additive necessary to preserve its stability and any impurity deriving from the process used but excluding any solvent which may be separated without affecting the stability of the substance or changing its composition.

A "mixture" is intended to mean a mixture or solution composed of two or more substances.

The present invention also provides the use of a composition of the invention in a nutraceutical, pharmaceutical, veterinary, oenological or cosmetic formulation/product.

Such formulations or products are hereinafter referred to as the "formulations or products of the invention".

The nutraceutical, pharmaceutical, veterinary, oenological or cosmetic formulation/product may optionally further comprise pharmaceutically/veterinary/cosmetic (including cosmetic active) ingredients, such as excipients, carriers and mixtures thereof as appropriate.

"Cosmetic" or "Cosmetic Active Ingredients" means any and all natural, naturally occurring, nature identical, synthetic synthetically produced, biosynthetically produced, sustainable, renewable and/or biodegradable compounds, ingredients, intermediates, molecules, substances, raw materials or products individually or as part of a mixture of compounds, ingredients, intermediates, molecules, substances, raw materials or products, blends, compositions, formulations (including but not limited to skin moisturizers, creams, balms, serums, oils, eye, facial makeup, wash off hair products, leave-on hair products, hair colorants (including but not limited to natural hair colorants) and/or combinations thereof), finished products and related technologies including, but not limited to components, incorporated, for example, into a cosmetic formulation (such as but not limited to natural colorants, preservatives, emulsifiers, anti-oxidants and the like which do not, for example, have an activity on the skin, hair, scalp and the like but play a role in the formulation of the finished product), delivery systems, marketing aids (such as, for example, coloured unispheres applied to translucent formulations) and methods of making anything related thereto useful in/used for/intended for:
- application by rubbing, pouring, sprinkling, spraying or otherwise directly on or to a human or animal body and/or by placing in contact with the various external and/or on surface parts of a human or animal body (including but not limited to the skin, hair, body hair, the hair system, scalp, nails, lips, external genitalia, teeth, oral and/or nasal mucosa and the like); and/or application indirectly on or to a human or animal body such as for example, application as part of a textile or application to a textile as part of a delivery device (such as a capsule) or a delivery system (such as blends or formulations) applied to a textile; and/or
- cleansing, caring, cooling, beautifying, conditioning, treating, soothing, texturizing, promoting attrativeness, protecting, maintaining, improving, enhancing, altering and/or changing an external part and/or surface of a human or animal body (such as, but not limited to the scalp) or the aesthetic appearance of a human or animal body; and/or with a view to mainly cleaning or perfuming, or protecting or maintaining in good condition or combating body odour or changing the appearance of or correcting or repairing a state of imbalance in skin, oral mucosa, scalp or hair by providing a calming, healing, repairing, or revitalization, hydration of the skin or in order to provide relief to, lubricate, moisten, tone, heal, sterilize, relieve, correct and/or remedy states of dryness, irritation, injury or fatigue, and/or with a view to correcting pigmentation disorders or providing a non-pharmaceutical prevention and/or treatment of dandruff, acne, irritation and/or inflammation and the like and/or rebalancing the bacterial flora (such as, for example, the microbiome) on the surface of the skin (such as, for example, by promoting the level of beneficial bacterial flora on the skin surface) and/or for the purposes of keeping a human or animal body in good condition for health and/or wellbeing purposes and/or for improving the appearance of a human or animal body by, for example, improving the appearance of a product applied to a human or body; and/or
- providing a cosmetic and/or dermatological function and/or benefit with a biological activity benefit (but without affecting a body's structure or function. For the avoidance of doubt, a Cosmetic or Cosmetic Active Ingredient or any part thereof may also qualify as a Functional Ingredient and/or Nutraceutical.

"Functional Ingredient" means a food ingredient or part of a food that provides medicinal or health benefits, including any of the following: a carotenoid, dietary fiber, fatty acid, saponin, antioxidant, flavonoid, isothiocyanate, phenol, polyphenol (such as resveratrol), plant sterol or stanol (phytosterols and phytostanols), a polyol, a prebiotic, a phytoestrogen, soy protein, sulfides/thiol, a vitamin, glucosamine, preservatives, hydration agents, edible gelling ingredients, edible gel mixes and gel compositions, long chain primary aliphatic saturated alcohols, colour agents, texturizing agents, emulsifiers and combinations thereof.

"Nutraceutical" means any and all natural, naturally occurring, sustainable, synthetically-produced and bio-synthetically-produced compounds, mixtures of compounds, Functional Ingredients, molecules, compositions, raw materials, and intermediates (including components and delivery devices (such as capsules) related thereto, delivery systems thereof (such as blends or formulations) and methods of making the foregoing) that are associated with health and/or cosmetic benefits, as well as improving or maintaining the appearance of the human body. For the avoidance of doubt, Nutraceuticals includes compounds that can be used as supplements to food or beverage, whether a solid formulation, capsule, tablet, liquid formulation, solution or suspension.

Alternatively, the nutraceutical, pharmaceutical, veterinary, oenological or cosmetic formulation/product may consist or consist essentially of the composition of the invention.

The cosmetic formulation/product may be an anti-aging formulation.

As used herein, references to pharmaceutically, veterinary or cosmetically acceptable excipients may refer to pharmaceutically, veterinary or cosmetically acceptable adjuvants, diluents and/or carriers as known to those skilled in the art.

By "pharmaceutically/veterinary/cosmetically acceptable" we mean that the additional components of the composition are generally safe, non-toxic, and neither biologically nor otherwise undesirable. For example, the additional components may be generally sterile and pyrogen free. Such components must be "acceptable" in the sense of being compatible with the composition of the invention and not deleterious to the recipients thereof. Thus, "pharmaceutically acceptable excipients" includes any compound(s) used in forming a part of the formulation that is intended to act merely as an excipient, i.e. not intended to have biological activity itself.

The nutraceutical, pharmaceutical, veterinary, oenological or cosmetic formulation/product may be in the form of a liquid or a solid.

Liquid dosage formulations/products for oral administration include solutions, emulsions, aqueous or oily suspensions, syrups and elixirs.

Formulations and products (e.g. pharmaceutical, veterinary or cosmetic formulations/products) described herein, such as those intended for oral administration, may be prepared according to methods known to those skilled in the art, such as by mixing the components of the formulation/product together.

The formulation or product (e.g. pharmaceutical, veterinary or cosmetic formulation/product) may contain one or more additional ingredients, such as pharmaceutical ingredients and excipients, such as sweetening agents, flavouring agents, colouring agents and preserving agents.

The formulation or product (e.g. pharmaceutical, veterinary or cosmetic formulation/product) may also contain one or more additional active ingredients, such as cosmetic or pharmaceutical active ingredients, such as hyaluronic acid, centella asiatica extract, peptides such as Matrixyl^{®} and Argireline^{®}, and mixtures thereof.

The formulation or product of the invention may contain the active ingredient(s) in admixture with non-toxic pharmaceutically acceptable excipients (or ingredients). These excipients (or ingredients) may, for example, be: inert diluents, such as calcium carbonate, sodium carbonate, lactose, calcium phosphate or sodium phosphate; granulating and disintegrating agents, for example, corn starch, maltodextrin or alginic acid; binding agents, for example, starch, gelatine or acacia; or lubricating agents, for example magnesium stearate, stearic acid, talc and mixtures thereof.

Liquid formulations or products (e.g. pharmaceutical, veterinary or cosmetic formulations/products) may be contained within a capsule, which may be uncoated or coated as defined above.

Suitable pharmaceutical or veterinary carriers include inert solid diluents or fillers, sterile aqueous solutions and various organic solvents. Examples of liquid carriers are syrup, peanut oil, olive oil, phospholipids, fatty acids, fatty acid amines, polyoxyethylene and water.

Moreover, the carrier or diluent may include any sustained release material known in the art, such as glyceryl monostearate or glyceryl distearate, alone or mixed with a wax.

Suitable pharmaceutical carriers include inert sterile aqueous solutions and various organic solvents. Examples of liquid carriers are syrup, vegetables oils, phospholipids, fatty acids, fatty acid amines, polyoxyethylene and water. Moreover, the carrier or diluent may include any sustained release material known in the art, such as glyceryl monostearate or glyceryl distearate, alone or mixed with a wax.

Suitable cosmetic carriers are typically those that are suitable for topical administration to the outer surface of the human body, such as the skin and/or hair and/or scalp.

Typically, such carriers are dermatologically acceptable.

The phrase "dermatologically acceptable carrier" means that the carrier is suitable for topical application to the keratinous tissue, has good aesthetic properties, is compatible with the actives in the composition, and will not cause any unreasonable safety or toxicity concerns.

The carrier can be in a wide variety of forms. In some instances, the solubility or dispersibility of the components (e.g. extracts, sunscreen active, additional components) may dictate the form and character of the carrier. Non-limiting examples include simple solutions (e.g. aqueous or anhydrous), dispersions, emulsions, and solid forms (e.g. gels, sticks, flowable solids, or amorphous materials).

The dermatologically acceptable carrier may be in the form of an emulsion. An emulsion may be generally classified as having a continuous aqueous phase (e.g. oil-in-water and water-in-oil-in-water) or a continuous oil phase (e.g. water-in-oil or oil-in-water). The oil phase of the present invention may comprise silicone oils, non-silicone oils such as hydrocarbon oils, esters, ethers, and the like, and mixtures thereof. The aqueous phase typically comprises water and water-soluble ingredients (e.g. water-soluble moisturizing agents, conditioning agents, anti-microbials, humectants and/or other skin care actives). However, in some instances, the aqueous phase may comprise components other than water, including but not limited to watersoluble moisturizing agents, conditioning agents, antimicrobials, humectants and/or other watersoluble skin care actives. In some instances, the non-water component of the composition comprises a humectant, such as glycerin and/or other polyol(s). Emulsions may also contain an emulsifier. Emulsifiers may be non-ionic, anionic or cationic.

The carrier may contain one or more dermatologically acceptable, hydrophilic diluents. As used herein, "diluent" includes materials in which the composition of the invention can be dispersed, dissolved, or otherwise incorporated. Hydrophilic diluents include water, organic hydrophilic diluents, such as lower monovalent alcohols (e.g., C1-C4), and low molecular weight glycols and polyols, including propylene glycol, polyethylene glycol, polypropylene glycol, glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof.

The cosmetic formulation/product may optionally include one or more additional ingredients commonly used in cosmetic compositions (e.g., colorants, skin tone agents, skin anti-aging agents, anti-inflammatory agents, sunscreen agents, combinations of these and the like), provided that the additional ingredients do not undesirably alter the anti-glycation benefits provided by the composition.

In some instances, it may be desirable to select skin tone agents that function via different biological pathways so that the actives do not interfere with one another, which could reduce the efficacy of both agents. The additional ingredients, when incorporated into the composition, should be suitable for use in contact with human skin tissue without undue toxicity, incompatibility, instability, allergic response, and the like.

The term "carrier" as used herein, may also refer to a natural product or a product originating from nature that has been transformed or modified so that it is distinct from the natural product from which it originated, such as maltodextrin.

The amount of the composition of the invention present in nutraceutical, pharmaceutical, veterinary, oenological or cosmetic formulations or products will vary depending on the application.

Typically, the amount of composition of the invention that may be present in nutraceutical, pharmaceutical, veterinary, oenological or cosmetic formulations or products will be from about 0.001 to about 50% by weight, such as from about 0.01% to about 30% or from about 1% to about 20% of the nutraceutical, pharmaceutical, veterinary, oenological or cosmetic formulations or products, such as from about 0.01 to about 20%, or from about 0.1 to 10% or from about 1 to about 5% by weight of the formulation or product.

Those of skill in the art will recognize that, due to the degenerate nature of the genetic code, a variety of DNA molecules differing in their nucleotide sequences can be used to encode a given enzyme of the disclosure. The native DNA sequence encoding the biosynthetic enzymes described above are referenced herein merely to illustrate an embodiment of the disclosure, and the disclosure includes DNA molecules of any sequence that encode the amino acid sequences of the polypeptides and proteins of the enzymes utilized in the methods of the disclosure. In similar fashion, a polypeptide can typically tolerate one or more amino acid substitutions, deletions, and insertions in its amino acid sequence without loss or significant loss of a desired activity. The disclosure includes such polypeptides with different amino acid sequences than the specific proteins described herein so long as the modified or variant polypeptides have the enzymatic anabolic or catabolic activity of the reference polypeptide. Furthermore, the amino acid sequences encoded by the DNA sequences shown herein merely illustrate embodiments of the disclosure.

Described herein are specific genes and proteins useful in the methods, compositions and organisms of the disclosure; however it will be recognized that absolute identity to such genes is not necessary. For example, changes in a particular gene or polynucleotide comprising a sequence encoding a polypeptide or enzyme can be performed and screened for activity. Typically such changes comprise conservative mutations and silent mutations. Such modified or mutated polynucleotides and polypeptides can be screened for expression of a functional enzyme using methods known in the art.

Due to the inherent degeneracy of the genetic code, other polynucleotides which encode substantially the same or functionally equivalent polypeptides can also be used to clone and express the polynucleotides encoding such enzymes

Techniques known to those skilled in the art may be suitable to identify additional homologous genes and homologous enzymes. Generally, analogous genes and/or analogous enzymes can be identified by functional analysis and will have functional similarities.

Techniques known to those skilled in the art may be suitable to identify analogous genes and analogous enzymes or any biosynthetic pathway genes, proteins, or enzymes, techniques may include, but are not limited to, cloning a gene by PCR using primers based on a published sequence of a gene/enzyme of interest, or by degenerate PCR using degenerate primers designed to amplify a conserved region among a gene of interest. Further, one skilled in the art can use techniques to identify homologous or analogous genes, proteins, or enzymes with functional homology or similarity. Techniques include examining a cell or cell culture for the catalytic activity of an enzyme through *in vitro* enzyme assays for said activity (e.g. as described herein or in Kiritani, K., Branched-Chain Amino Acids Methods Enzymology, 1970), then isolating the enzyme with said activity through purification, determining the protein sequence of the enzyme through techniques such as Edman degradation, design of PCR primers to the likely nucleic acid sequence, amplification of said DNA sequence through PCR, and cloning of said nucleic acid sequence. To identify homologous or similar genes and/or homologous or similar enzymes, analogous genes and/or analogous enzymes or proteins, techniques also include comparison of data concerning a candidate gene or enzyme with databases such as BRENDA, KEGG, or MetaCYC. The candidate gene or enzyme may be identified within the above mentioned databases in accordance with the teachings herein.

The terms "between... and..." and "ranging from... to..." should be understood as being inclusive of the limits, unless otherwise specified. Throughout this specification and the claims which follow, unless the context requires otherwise, the word "comprise", and variations such as "comprises", "comprising" and "possesses", will be understood to imply the inclusion of a stated integer or step or group of integers or steps but not the exclusion of any other integer or step or group of integer or step. The term "comprising" also means "including" as well as "consisting" e.g. a composition "comprising" X may consist exclusively of X or may include something additional e.g. X + Y. It must be noted also that, as used in this specification and the appended claims, the singular forms "a", "an" and "the" include plural referents unless the content clearly dictates otherwise. By way of example, a reference to "a gene" or "an enzyme" is a reference to "one or more genes" or "one or more enzymes".

It is to be understood that this disclosure is not limited to the particular methodology, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by the person skilled in the art. In accordance with the present disclosure there may be conventional molecular biology, microbiology, and recombinant DNA techniques employed which are within the skill of the art.

This disclosure is not limited in its application to the details of construction and the arrangement of components set forth in the following description or illustrated in the drawings. The disclosure is capable of other embodiments and of being practiced or of being carried out in various ways. Also, the phraseology and terminology used herein is for the purpose of description and should not be regarded as limiting. Preferably, the terms used herein are defined as described in "A multilingual glossary of biotechnological terms: (IUPAC Recommendations)", Leuenberger, H.G.W, Nagel, B. and Kolbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety.

The examples and figures which follow are presented by way of illustration and without implied limitation of the invention.

### EXAMPLES

### Exemple 1: Protocol for making a recombinant Saccharomyces cerevisiae strain according to the invention

All the hereinafter implemented recombinant *Saccharomyces cerevisiae* strains were constructed from standard strains using standard yeast molecular genetics procedure (Methods in yeast Genetics - A cold spring harbor laboratory course Manual (2000) by D. Burke, D. Dawson, T. Stearns CSHL Press).

Cluster of the following-mentioned genes were integrated in recombinant yeast at once using the ability of yeast to efficiently recombine free DNA ends which have sequence homology.

In addition, for a better comprehension of following genotypes:
- jlp1, his 3, leu2, sam3 and met14 are insertion sites.
- Lowercase letters mean that the considered gene is inactive, uppercase letters reflect an active gene.
- "::": following a gene name means that the gene is interrupted by what follows (if more than one gene are inserted, they are noted in brackets []). The interruption of the gene is concomitant with an entire deletion of the coding sequence but preserves the promoter. In consequence the gene followed by "::" is inactive and is noted in lowercase. If not specified the transcription of the gene inserted is controlled by the promoter of the disrupted gene.
- "gene.K1" means that the gene originates from *Kluyveromyces lactis.* When nothing is indicated after a gene in the present examples, this means that the gene is from *Saccharomyces cerevisiae.*

More particularly, the coding sequences to be cloned were artificially synthetized. For heterologous sequences (non-yeast), the nucleic sequences were modified in order to obtain a synonymous coding sequence using the yeast codon usage. Using restriction enzyme and classical cloning technology, each synthetic sequence was cloned in between a transcription promoter and a transcription terminator. Each promoter sequence is preceded by a 50 to 200 nucleotide sequence homologous to the sequence of the terminator of the upstream gene. Similarly, the terminator of each gene (a gene comprising the promoter-coding sequence-terminator) is followed by sequences homologous to the gene immediately following. So that each of the unit to be integrated have a 50-200 nucleotide overlap with both the unit upstream and the unit downstream. For the first unit, the promoter is preceded by 50-200 nucleotides homologous to the yeast chromosome nucleotide for the locus in which it will be integrated. Similarly, for the last unit, the terminator is followed by 50-200 nucleotides homologous to the yeast chromosome nucleotide for the locus in which it will be integrated.

Each unit is then PCR amplified from the plasmids constructs, yielding X unit of linear DNA having overlapping sequences. At least one of this gene is an auxotrophic marker, in order to select for recombination event. All the linear fragments are transformed in the yeast at once, and a recombinant yeast cell is selected for the auxotrophy related to the marker used. The integrity of the sequence is then verified by PCR and sequencing.

### Example 2: Comparative examples for the production of Chondroitin

Firstly, three recombinant strains are obtained: YA5809, YA5810 and YA5571.

Accordingly, these three strains are as follows:
**YA5809:** MAT-α, his3::[tRPL3-UGP1-pSAM1, pMET6-QRI1-tIDP1, HIS3]x5, jlp1::[LEU2.Sba-loxP, pTEF1.Sba-HASB.Vir-tRPL3.Sm, pTDH3.Sk-HASB-A.Vir-tTEF1.Sba, pTDH3-1.Sba-HCOS1-3.Pm-tRPL15A.Sm, pCCW12-KFOA.Pm-tRPL41B.Sba, pCCW120.Sm-KFOA.Pm-tRPL15A.Sba, pTEF1.Ago-GFA1.Vir-tRPL15A], leu2, met14::[TRP1.Sba-RS, pTDH3-PGM1-tIDP1, pCWP2-GNA1-tTPI1, pTEF1-PCM1-tRPL41B, pFBA1-UGP1-tRPL3, pTDH3-1.Sba-QRI1-tIDP1.Sba, pTEF1.Ago-GFA1-tRPL15A], trp1, ura3
**YA5810:** MAT-α, his3::[tRPL3-UGP1-pSAM1, pMET6-QRI1-tIDP1, HIS3]x5, jlp1::[LEU2.Sba-loxP, pTEF1.Sba-HASB.Vir-tRPL3.Sm, pTDH3.Sk-HASB-A.Vir-tTEF1.Sba, pTDH3-1.Sba-HCOS1-3.Pm-tRPL15A.Sm, pCCW12-KFOA.Pm-tRPL41B.Sba, pCCW120.Sm-KFOA.Pm-tRPL15A.Sba, pTEF1.Ago-GFA1.Vir-tRPL15A], leu2, met14::[TRP1.Sba-RS, pTDH3-PGMl-tIDP1, pCWP2-GNA1-tTPI1, pTEFl-PCM1-tRPL41B, pFBA1-UGP1-tRPL3, pTDH3-1.Sba-QRI1-tIDP1.Sba], trp1, ura3
**YA5571:** MAT-α, his3::[tRPL3-UGP1-pSAM1, pMET6-QRI1-tIDP1, HIS3]x5, jlp1::[LEU2.Sba-loxP, pTEF1.Sba-HASB.Vir-tRPL3.Sm, pTDH3.Sk-HASB-A.Vir-tTEF1.Sba, pTDH3-1.Sba-HCOS1-3.Pm-tRPL15A.Sm, pCCW12-KFOA.Pm-tRPL41B.Sba, pCCW120.Sm-KFOA.Pm-tRPL15A.Sba, pTEF1.Ago-GFA1.Vir-tRPL15A], leu2, trp1, ura3

HASB and HASB-A represent a nucleic acid sequence encoding a polypeptide having UDP-glucose 6-dehydrogenase activity. They differ from one another in that they are different reencoded versions of a nucleic acid sequence encoding the enzyme. HASB has the sequence SEQ ID NO: 36 and HASB-A has the sequence SEQ ID NO: 37. HCOS1-3.Pm represents a nuclic acid sequence encoding a polypeptide having chondroitin synthase activity that is a a nucleic acid comprising a nucleic acid encoding a polypeptide having a Hyaluronan synthase activity and fragments of a nucleic acid encoding a polypeptide having a chondroitin synthase activity. HCOS1-3.Pm has the sequence SEQ ID NO: 3.

These strains were incubated in 25 ml of SY medium supplemented with 2% glucose for 48 hours at 28°in Erlenmeyer flasks.

SY medium comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeCl3: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM ; Nicotinic acid (C6H5NO2): 29 µM; D-Biotine: 0,82 µM; Ammonium citrate tribasic: 33 mM; and glucose or sucrose 2-30%

Growth medium was recovered at 48h hours and assayed for chondroitin content.

An aliquot of the supernatant was loaded and ran on a 0,5% agarose gel subsequently colored with bromophenol blue.

The amount of chondroitin present in the medium was evaluated by a colorimetric determination after treatment with concentrated sulfuric acid and carbazole (Bitter and Muir (1962) analytical biochemistry 4, 330-334).

The amounts obtained with these different strains are respectively:
- YA5809: 200 mg.L⁻¹
- YA5810: 200 mg.L⁻¹
- YA5571: 200 mg.L⁻¹

In comparision, a native strain (Chérest et al. (2000) J Biol. Chem. 275: 14056-14063) from which the three other strains derive does not produce chondroitin.
It results from this experiment that a recombinant strain comprising the modifications according to the invention produces a greater amount of chondroitin when cultured in the same conditions as other strains not comprising all the genetic modifications according to the invention.

### Example 3: Examples for the production of Chondroitin of controlled molecular weight

### 1. Production of chondroitin having a molecular weight of less than 10kDa

Next, two recombinant strains are obtained: YA5887 and 5902.

Accordingly, these two strains are as follows:
**YA5887**: MAT-a, his3, jlp1::[LEU2.Kl, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2::[TRP1.Sba, pCCW12.Sba-HYAL-3.Ts-tRPL15A], sam3::[HASB.Vir-tTEF1.Sba, pTDH3-1.Sba-HCOS1-3.Pm-tRPL3.Sba, pTDH3.Sar-HCOS1-3.Pm-tRPL15A.Sm, pCCW12-KFOA.Pm-tRPL41B.Sba, pCCW120.Sm-KFOA.Pm-tRPL15A.Sba, HIS3.Sba], trp1
**YA5902:** MAT-α, his3::[pSAM1-UGP1-tRPL3, pMET6-QRI1-tIDP1, HIS3]x5, jlp1::[LEU2.Kl, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2::[TRP1.Sba, pCCW12.Sba-HYAL-3.Ts-tRPL15A], sam3::[LEU2.K1, pENO2-UGP1-tRPL3, pCWP2-GNA1-tTPI1, pTEF1-PCM1-tRPL41B, pTEF1.Sba-HASB.vir-tRPL3.Sm, pTDH3.Sk-HASB-A.Vir-tTEF1.Sba, pTDH3-1.Sba-HCOS1-3.Pm-tRPL15A.Sm, pCCW12-KFOA.Pm-tRPL15A.Sba, HIS3.Sba-loxP], trp1

HYAL-3.Ts represents a nucleic acid sequence encoding a polypeptide having chondroitinase activity associated with a secretion signal but no anchoring signal. HYAL-3.Ts has the amino acid sequence SEQ ID NO: 23.

HASB, HASB-A and HCOS-1.Pm are as defined above.

These strains were incubated in 25 ml of SY medium supplemented with 2% glucose for 48 hours at 28°in Erlenmeyer flasks.

Growth medium was recovered at 48h hours and assayed for chondroitin content.

An aliquot of the supernatant was loaded and ran on a 0,5% agarose gel subsequently colored with bromophenol blue.

The amount of chondroitin present in the medium was evaluated by a colorimetric determination after treatment with concentrated sulfuric acid and carbazole (Bitter and Muir (1962) analytical biochemistry 4, 330-334).

The amounts obtained with these different strains are respectively:
- YA5887: 600 mg.L⁻¹
- YA5902: 600 mg.L⁻¹

In comparision, a native strain (Chérest et al. (2000) J Biol. Chem. 275: 14056-14063) from which the two other strains derive does not produce chondroitin.

It results from this experiment that a recombinant strain comprising the modifications according to the invention produces a greater amount of chondroitin when cultured in the same conditions as other strains not comprising all the genetic modifications according to the invention.

Moreover, these two strains led to the production, after 48 hours, of chondroitin having a molecular weight of less than 10kDa.

### 2. Production of chondroitin having a molecular weight comprised between about 10kDa and about 50kDa

Next, two toher recombinant strains are obtained: YA5888 and 5903.

Accordingly, these two strains are as follows:
**YA5888:** MAT-a, his3, jlp1::[LEU2.Kl, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2::[TRP1.Sba, pCCW10.Ago-HYAL-3.Ts-tRPL15A], sam3::[HASB.Vir-tTEF1.Sba, pTDH3-1.Sba-HCOS1-3.Pm-tRPL3.Sba, pTDH3.Sar-HCOS1-3.Pm-tRPL15A.Sm, pCCW12-KFOA.Pm-tRPL41B.Sba, pCCW120.Sm-KFOA.Pm-tRPL15A.Sba, HIS3.Sba], trp1
**YA5903:** MAT-α, his3::[pSAM1-UGP1-tRPL3, pMET6-QRI1-tIDP1, HIS3]x5, jlp1::[LEU2.Kl, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2::[TRP1.Sba, pCCW10.Ago-HYAL-3.Ts-tRPL15A], sam3::[LEU2.K1, pENO2-UGP1-tRPL3, pCWP2-GNA1-tTPI1, pTEF1-PCM1-tRPL41B, pTEF1.Sba-HASB.vir-tRPL3.Sm, pTDH3.Sk-HASB-A.Vir-tTEF1.Sba, pTDH3-1.Sba-HCOS1-3.Pm-tRPL15A.Sm, pCCW12-KFOA.Pm-tRPL15A.Sba, HIS3.Sba-loxP], trp1

HYAL-3.Ts, HASB, HASB-A and HCOS-1.Pm are as defined above.

These strains were incubated in 25 ml of SY medium supplemented with 2% glucose for 48 hours at 28°in Erlenmeyer flasks.

Growth medium was recovered at 48h hours and assayed for chondroitin content.

An aliquot of the supernatant was loaded and ran on a 0,5% agarose gel subsequently colored with bromophenol blue.

The amount of chondroitin present in the medium was evaluated by a colorimetric determination after treatment with concentrated sulfuric acid and carbazole (Bitter and Muir (1962) analytical biochemistry 4, 330-334).

The amounts obtained with these different strains are respectively:
- YA5888: 600 mg.L⁻¹
- YA5903: 600 mg.L⁻¹

In comparision, a native strain (Chérest et al. (2000) J Biol. Chem. 275: 14056-14063) from which the two other strains derive does not produce chondroitin.

It results from this experiment that a recombinant strain comprising the modifications according to the invention produces a greater amount of chondroitin when cultured in the same conditions as other strains not comprising all the genetic modifications according to the invention.

Moreover, these two strains led to the production, after 48 hours, of chondroitin having a molecular weight ranging from about 10kDa to about 50kDa.

### 3. Production of chondroitin having a molecular weight comprised between about 50kDa and about 500kDa

Next, two toher recombinant strains are obtained: YA5889 and 5904.

Accordingly, these two strains are as follows:
**YA5889:** MAT-a, his3, jlp1::[LEU2.Kl, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2::[TRP1.Sba, pNUP57-HYAL-3.Ts-tRPL15A], sam3::[HASB.Vir-tTEF1.Sba, pTDH3-1.Sba-HCOS1-3.Pm-tRPL3.Sba, pTDH3.Sar-HCOS1-3.Pm-tRPL15A.Sm, pCCW12-KFOA.Pm-tRPL41B.Sba, pCCW120.Sm-KFOA.Pm-tRPL15A.Sba, HIS3.Sba], trp1
**YA5904:** MAT-α, his3::[pSAM1-UGP1-tRPL3, pMET6-QRI1-tIDP1, HIS3]x5, jlp1::[LEU2.Kl, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2::[TRP1.Sba, pNUP57-HYAL-3.Ts-tRPL15A], sam3::[LEU2.K1, pENO2-UGP1-tRPL3, pCWP2-GNA1-tTPI1, pTEF1-PCM1-tRPL41B, pTEF1.Sba-HASB.vir-tRPL3.Sm, pTDH3.Sk-HASB-A.Vir-tTEF1.Sba, pTDH3-1.Sba-HCOS1-3.Pm-tRPL15A.Sm, pCCW12-KFOA.Pm-tRPL15A.Sba, HIS3.Sba-loxP], trp1

HYAL-3.Ts, HASB, HASB-A and HCOS-1.Pm are as defined above.

These strains were incubated in 25 ml of SY medium supplemented with 2% glucose for 48 hours at 28°in Erlenmeyer flasks.

Growth medium was recovered at 48h hours and assayed for chondroitin content.

An aliquot of the supernatant was loaded and ran on a 0,5% agarose gel subsequently colored with bromophenol blue.

The amount of chondroitin present in the medium was evaluated by a colorimetric determination after treatment with concentrated sulfuric acid and carbazole (Bitter and Muir (1962) analytical biochemistry 4, 330-334).

The amounts obtained with these different strains are respectively:
- YA5889: 600 mg.L⁻¹
- YA5904: 600 mg.L⁻¹

In comparision, a native strain (Chérest et al. (2000) J Biol. Chem. 275: 14056-14063) from which the two other strains derive does not produce chondroitin.

It results from this experiment that a recombinant strain comprising the modifications according to the invention produces a greater amount of chondroitin when cultured in the same conditions as other strains not comprising all the genetic modifications according to the invention.

Moreover, these two strains led to the production, after 48 hours, of chondroitin having a molecular weight ranging from about 10kDa to about 50kDa.

### SEQUENCES

SEQ ID NO: 1 is a reencoded nucleic acid sequence of a polypeptide having a chondroitin
synthase activity: chimeric nucleic acid comprising a fragment from a nucleic acid encoding a
hyaluronan synthase originating from Pasteurella multocida and a fragment from a nucleic
acid encoding a chondroitin synthase from Pasteurella multocida (HCOS1-1):
ATGAACACCCTTTCCCAGGCAATTAAGGCTTATAATAGTAATGATTATCAACTAGCCCTCAAGCTG
TTCGAAAAGTCGGCAGAAATCTACGGCAGAAAGATAGTTGAgTTCCAAATTACGAAGTGCAAGGA
GAAACTTTCTGCGCACCCATCCGTTAACAGTGCGCATCTCTCGGTTAACAAAGAAGAGAAGGTGAA
TGTTTGCGACAGTCCATTAGACATAGCCACTCAATTATTGTTATCGAATGTGAAAAAACTCGTCCTT
AGTGATAGCGAGAAAAATACACTAAAGAATAAATGGAAGTTGTTGACTGAAAAGAAGTCTGAGAA
CGCAGAGGTCCGGGCCGTAGCTTTAGTTCCCAAGGACTTTCCGAAAGATTTAGTGCTTGCGCCGTT
ACCAGACCATGTGAACGATTTTACGTGGTATAAGAAGCGGAAGAAACGCCTAGGAATCAAACCAG
AACACCAACACGTTGGCCTCTCCATTATTGTGACAACGTTCAACCGTCCTGCCATCCTCAGCATCAC
CCTGGCTTGCCTCGTCAATCAAAAGACCCATTATCCTTTTGAGGTGATCGTGACCGACGATGGTTCT
CAAGAGGATTTATCGCCTATAATCCGTCAATATGAGAACAAGCTAGACATCCGTTATGTTCGGCAA
AAGGACTATGGGTATCAGCTGTGCGCCGTTAGAAACCTTGGTCTAAGGACAGCGAAGTATGACTTT
GTCTCTATCTTGGATTGCGACATGGCCCCGCAGCAGCTATGGGTTCACTCTTACCTTACAGAACTAT
TAGAGGACGATGATTTGACCATAATAGGCCCCAGGAAGTACATTGACACGCAACACATAGATCCC
AAGGATTTCCTTAATAACGCCTCTCTGTTAGAGTCGTTGCCAGAGGTTAAGACCAATAATTCCGTCG
CGGCTAAGGGCGAGGGGACCGTATCTTTAGACTGGCGTTTGGAACAATTTGAGAAGACCGAGAAC
TTGAGGCTATCCGATAGCCCTTTCCGATTCTTCGCAGCTGGGAATGTGGCTTTCGCCAAGAAGTGGC
TTAACAAGTCAGGATTCTTCGACGAGGAATTCAATCACTGGGGAGGTGAAGATGTAGAGTTCGGTT
ATCGTCTGTTTCGGTACGGTTCGTTCTTCAAAACTATAGACGGCATCATGGCCTATCATCAGGAACC
GCCAGGTAAAGAAAACGAAACTGACAGAGAAGCGGGCAAGAACATTACCCTCGATATAATGAGG
GAGAAGGTGCCTTACATCTACCGTAAACTCCTGCCTATAGAAGACAGTCATATCAACCGAGTACCA
TTGGTATCAATTTACATCCCGGCCTACAACTGTGCTAACTATATTCAAAGATGTGTTGATTCAGCTT
TGAATCAAACGGTAGTTGATCTCGAAGTGTGCATTTGCAACGACGGTAGTACTGACAACACGCTGG
AAGTTATTAACAAGCTGTATGGTAATAATCCGCGTGTGCGTATAATGTCTAAACCCAATGGCGGCA
TTGCGAGTGCATCCAACGCAGCGGTCAGCTTCGCAAAGGGTTATTACATAGGACAGTTGGACAGCG
ACGATTACTTAGAACCCGACGCAGTGGAGTTATGTCTCAAGGAATTTCTTAAGGATAAGACCCTTG
CGTGCGTTTACACCACTAATCGTAACGTCAACCCAGATGGCTCTTTAATAGCCAATGGCTATAACT
GGCCAGAGTTCAGTCGTGAGAAGTTGACTACGGCCATGATTGCTCATCACTTCCGGATGTTTACCA
TTCGTGCTTGGCATCTGACGGATGGGTTCAATGAGAAGATTGAGAACGCTGTTGACTACGACATGT
TTCTCAAGCTCAGTGAAGTTGGTAAATTTAAGCATCTGAACAAAATATGTTATAATCGGGTGTTAC
ACGGCGATAACACCTCAATCAAGAAgCTTGGCATACAAAAGAAGAATCATTTCGTAGTTGTCAATC
AGTCTCTAAACCGCCAAGGTATAACTTATTATAACTACGATGAATTTGATGATCTCGATGAGAGTC

GGAAATACATTTTCAACAAGACTGCAGAGTATCAAGAAGAGATAGATATTCTTAAAGATATTAAG
ATCATTCAGAACAAAGACGCCAAAATAGCTGTCTCCATTTTCTATCCGAACACTCTAAACGGGTTG
GTGAAGAAGCTAAATAATATTATAGAGTATAATAAGAATATCTTCGTTATTGTACTTCATGTCGAT
AAGAATCACTTAACCCCAGACATCAAGAAGGAGATATTAGCCTTCTACCATAAGCATCAGGTGAAC
ATCTTATTGAACAATGACATCTCCTACTATACATCAAATCGTCTGATTAAGACAGAAGCCCATTTGA
GTAACATTAACAAGCTaAGTCAATTAAATCTTAACTGCGAATACATTATATTCGACAATCACGATTC
CTTATTTGTGAAGAACGATTCCTATGCATACATGAAGAAGTATGATGTTGGAATGAACTTCTCTGC
ATTGACTCATGATTGGATTGAAAAGATAAACGCTCACCCGCCATTTAAGAAGCTgATCAAAACTTA
CTTCAATGACAATGATCTTAAGTCGATGAATGTAAAGGGTGCCTCCCAGGGAATGTTTATGACATA
CGCATTAGCCCACGAGTTATTGACGATCATCAAGGAGGTGATAACCTCTTGTCAATCCATTGACTC
CGTCCCCGAATACAACACAGAAGATATTTGGTTTCAGTTTGCACTTTTAATTCTGGAAAAGAAGAC
CGGCCACGTATTCAACAAGACAAGCACTCTCACGTATATGCCATGGGAACGTAAACTGCAGTGGAC
GAATGAACAAATAGAGTCCGCAAAGAGGGGCGAAAACATTCCGGTAAACAAGTTCATCATTAACA
GCATTACCCTTTAA

SEQ ID NO: 2 is a reencoded nucleic acid sequence of a polypeptide having a chondroitin
synthase activity : chimeric nucleic acid comprising a fragment from a nucleic acid encoding
a hyaluronan synthase originating from Pasteurella multocida and a fragment from a nucleic
acid encoding a chondroitin synthase from Pasteurella multocida (HCOS1-2):
ATGAACACCCTTTCCCAGGCAATTAAGGCTTATAATAGTAATGATTATCAACTAGCCCTCAAGCTG
TTCGAAAAGTCGGCAGAAATCTACGGCAGAAAGATAGTTGAgTTCCAAATTACGAAGTGCAAGGA
GAAACTTTCTGCGCACCCATCCGTTAACAGTGCGCATCTCTCGGTTAACAAAGAAGAGAAGGTGAA
TGTTTGCGACAGTCCATTAGACATAGCCACTCAATTATTGTTATCGAATGTGAAAAAACTCGTCCTT
AGTGATAGCGAGAAAAATACACTAAAGAATAAATGGAAGTTGTTGACTGAAAAGAAGTCTGAGAA
CGCAGAGGTCCGGGCCGTAGCTTTAGTTCCCAAGGACTTTCCGAAAGATTTAGTGCTTGCGCCGTT
ACCAGACCATGTGAACGATTTTACGTGGTATAAGAAGCGGAAGAAACGCCTAGGAATCAAACCAG
TGAACAAGAACATCGGGTTGTCAATCATAATCCCAACTTTCAATCGTAGTAGGATATTAGACATCA
CCCTGGCTTGCCTCGTCAATCAAAAGACCCATTATCCTTTTGAGGTGATCGTGACCGACGATGGTTC
TCAAGAGGATTTATCGCCTATAATCCGTCAATATGAGAACAAGCTAGACATCCGTTATGTTCGGCA
AAAGGACTATGGGTATCAGCTGTGCGCCGTTAGAAACCTTGGTCTAAGGACAGCGAAGTATGACTT
TGTCTCTATCTTGGATTGCGACATGGCCCCGCAGCAGCTATGGGTTCACTCTTACCTTACAGAACTA
TTAGAGGACGATGATTTGACCATAATAGGCCCCAGGAAGTACATTGACACGCAACACATAGATCCC
AAGGATTTCCTTAATAACGCCTCTCTGTTAGAGTCGTTGCCAGAGGTTAAGACCAATAATTCCGTCG
CGGCTAAGGGCGAGGGGACCGTATCTTTAGACTGGCGTTTGGAACAATTTGAGAAGACCGAGAAC
TTGAGGCTATCCGATAGCCCTTTCCGATTCTTCTCATGCGGAAATGTGGCCTTCTCCAAAGAGTGGC
TGAACAAAGTTGGCTGGTTCGACGAGGAATTCAATCACTGGGGAGGTGAAGATGTAGAGTTCGGTT
ATCGTCTGTTTCGGTACGGTTCGTTCTTCAAAACTATAGACGGCATCATGGCCTATCATCAGGAACC

GCCAGGTAAAGAAAACGAAACTGACAGAGAAGCGGGCAAGAACATTACCCTCGATATAATGAGG
GAGAAGGTGCCTTACATCTACCGTAAACTCCTGCCTATAGAAGACAGTCATATCAACCGAGTACCA
TTGGTATCAATTTACATCCCGGCCTACAACTGTGCTAACTATATTCAAAGATGTGTTGATTCAGCTT
TGAATCAAACGGTAGTTGATCTCGAAGTGTGCATTTGCAACGACGGTAGTACTGACAACACGCTGG
AAGTTATTAACAAGCTGTATGGTAATAATCCGCGTGTGCGTATAATGTCTAAACCCAATGGCGGCA
TTGCGAGTGCATCCAACGCAGCGGTCAGCTTCGCAAAGGGTTATTACATAGGACAGTTGGACAGCG
ACGATTACTTAGAACCCGACGCAGTGGAGTTATGTCTCAAGGAATTTCTTAAGGATAAGACCCTTG
CGTGCGTTTACACCACTAATCGTAACGTCAACCCAGATGGCTCTTTAATAGCCAATGGCTATAACT
GGCCAGAGTTCAGTCGTGAGAAGTTGACTACGGCCATGATTGCTCATCACTTCCGGATGTTTACCA
TTCGTGCTTGGCATCTGACGGATGGGTTCAATGAGAAGATTGAGAACGCTGTTGACTACGACATGT
TTCTCAAGCTCAGTGAAGTTGGTAAATTTAAGCATCTGAACAAAATATGTTATAATCGGGTGTTAC
ACGGCGATAACACCTCAATCAAGAAgCTTGGCATACAAAAGAAGAATCATTTCGTAGTTGTCAATC
AGTCTCTAAACCGCCAAGGTATAACTTATTATAACTACGATGAATTTGATGATCTCGATGAGAGTC
GGAAATACATTTTCAACAAGACTGCAGAGTATCAAGAAGAGATAGATATTCTTAAAGATATTAAG
ATCATTCAGAACAAAGACGCCAAAATAGCTGTCTCCATTTTCTATCCGAACACTCTAAACGGGTTG
GTGAAGAAGCTAAATAATATTATAGAGTATAATAAGAATATCTTCGTTATTGTACTTCATGTCGAT
AAGAATCACTTAACCCCAGACATCAAGAAGGAGATATTAGCCTTCTACCATAAGCATCAGGTGAAC
ATCTTATTGAACAATGACATCTCCTACTATACATCAAATCGTCTGATTAAGACAGAAGCCCATTTGA
GTAACATTAACAAGCTaAGTCAATTAAATCTTAACTGCGAATACATTATATTCGACAATCACGATTC
CTTATTTGTGAAGAACGATTCCTATGCATACATGAAGAAGTATGATGTTGGAATGAACTTCTCTGC
ATTGACTCATGATTGGATTGAAAAGATAAACGCTCACCCGCCATTTAAGAAGCTgATCAAAACTTA
CTTCAATGACAATGATCTTAAGTCGATGAATGTAAAGGGTGCCTCCCAGGGAATGTTTATGACATA
CGCATTAGCCCACGAGTTATTGACGATCATCAAGGAGGTGATAACCTCTTGTCAATCCATTGACTC
CGTCCCCGAATACAACACAGAAGATATTTGGTTTCAGTTTGCACTTTTAATTCTGGAAAAGAAGAC
CGGCCACGTATTCAACAAGACAAGCACTCTCACGTATATGCCATGGGAACGTAAACTGCAGTGGAC
GAATGAACAAATAGAGTCCGCAAAGAGGGGCGAAAACATTCCGGTAAACAAGTTCATCATTAACA
GCATTACCCTTTAA

SEQ ID NO: 3 is a reencoded nucleic acid sequence of a polypeptide having a chondroitin
synthase activity : chimeric nucleic acid comprising a fragment from a nucleic acid encoding
a hyaluronan synthase originating from Pasteurella multocida and a fragment from a nucleic
acid encoding a chondroitin synthase from Pasteurella multocida (HCOS1-3):
ATGAACACCCTTTCCCAGGCAATTAAGGCTTATAATAGTAATGATTATCAACTAGCCCTCAAGCTG
TTCGAAAAGTCGGCAGAAATCTACGGCAGAAAGATAGTTGAGTTCCAAATTACGAAGTGCAAGGA
GAAACTTTCTGCGCACCCATCCGTTAACAGTGCGCATCTCTCGGTTAACAAAGAAGAGAAGGTGAA
TGTTTGCGACAGTCCATTAGACATAGCCACTCAATTATTGTTATCGAATGTGAAAAAACTCGTCCTT
AGTGATAGCGAGAAAAATACACTAAAGAATAAATGGAAGTTGTTGACTGAAAAGAAGTCTGAGAA

CGCAGAGGTCCGGGCCGTAGCTTTAGTCCCAAAGGATTTCCCAAAGACTTAGTTTTAGCGCCACT
ACCAGACCATGTGAACGATTTTACCTGGTACAAGAATAGAAAGAAGAGCCTGGGCATAAAGCCTG
TGAACAAGAACATCGGGTTGTCAATCATAATCCCAACTTTCAATCGTAGTAGGATATTAGACATTA
CCCTGGCGTGTCTAGTTAATCAGAAAACTAACTATCCGTTTGAGGTGGTTGTTGCAGATGATGGGT
CGAAGGAAAATCTATTGACTATAGTTCAGAAATACGAGCAGAAACTAGACATTAAGTATGTGAGA
CAGAAGGACTATGGGTATCAGCTGTGCGCCGTTAGAAACCTTGGTCTAAGGACAGCGAAGTATGA
CTTTGTCTCTATCTTGGATTGCGACATGGCCCCGCAGCAGCTATGGGTTCACTCTTACCTTACAGAG
TTACTGGAAGACAACGACATCGTCTTGATCGGGCCAAGGAAATACGTTGATACCCACAACATCACG
GCTGAGCAGTTTCTGAACGACCCATATTTAATTGAGAGTTTACCGGAAACCGCCACAAACAATAAT
CCATCGATTACCAGTAAGGGTAACATCTCATTAGATTGGAGATTAGAACATTTTAAGAAGACAGAC
AACCTAAGACTATGCGACTCCCCGTTCAGGTATTTCTCATGCGGAAATGTGGCCTTCTCCAAAGAG
TGGCTGAACAAAGTTGGCTGGTTCGACGAAGAATTTAATCACTGGGGCGGCGAAGACGTCGAATTC
GGCTACAGGTTATTTGCAAAAGGGTGCTTCTTTAGGGTTATCGACGGTGGTATGGCCTACCATCAA
GAGCCACCAGGAAAGGAGAACGAAACGGATCGTGAGGCCGGTAAGTCTATCACTCTTAAAATAGT
AAAGGAGAAAGTGCCCTACATCTACAGGAAGTTGTTGCCGATTGAGGACTCCCACATAAACCGAG
TACCATTGGTATCAATTTACATCCCGGCCTACAACTGTGCTAACTATATTCAAAGATGTGTTGATTC
AGCTTTGAATCAAACGGTAGTTGATCTCGAAGTGTGCATTTGCAACGACGGTAGTACTGACAACAC
GCTGGAAGTTATTAACAAGCTGTATGGTAATAATCCGCGTGTGCGTATAATGTCTAAACCCAATGG
CGGCATTGCGAGTGCATCCAACGCAGCGGTCAGCTTCGCAAAGGGTTATTACATAGGACAGTTGGA
CAGCGACGATTACTTAGAACCCGACGCAGTGGAGTTATGTCTCAAGGAATTTCTTAAGGATAAGAC
CCTTGCGTGCGTTTACACCACTAATCGTAACGTCAACCCAGATGGCTCTTTAATAGCCAATGGCTAT
AACTGGCCAGAGTTCAGTCGTGAGAAGTTGACTACGGCCATGATTGCTCATCACTTCCGGATGTTT
ACCATTCGTGCTTGGCATCTGACGGATGGGTTCAATGAGAAGATTGAGAACGCTGTTGACTACGAC
ATGTTTCTCAAGCTCAGTGAAGTTGGTAAATTTAAGCATCTGAACAAAATATGTTATAATCGGGTG
TTACACGGCGATAACACCTCAATCAAGAAGCTTGGCATACAAAAGAAGAATCATTTCGTAGTTGTC
AATCAGTCTCTAAACCGCCAAGGTATAACTTATTATAACTACGATGAATTTGATGATCTCGATGAG
AGTCGGAAATACATTTTCAACAAGACTGCAGAGTATCAAGAAGAGATAGATATTCTTAAAGATATT
AAGATCATTCAGAACAAAGACGCCAAAATAGCTGTCTCCATTTTCTATCCGAACACTCTAAACGGG
TTGGTGAAGAAGCTAAATAATATTATAGAGTATAATAAGAATATCTTCGTTATTGTACTTCATGTCG
ATAAGAATCACTTAACCCCAGACATCAAGAAGGAGATATTAGCCTTCTACCATAAGCATCAGGTGA
ACATCTTATTGAACAATGACATCTCCTACTATACATCAAATCGTCTGATTAAGACAGAAGCCCATTT
GAGTAACATTAACAAGCTAAGTCAATTAAATCTTAACTGCGAATACATTATATTCGACAATCACGA
TTCCTTATTTGTGAAGAACGATTCCTATGCATACATGAAGAAGTATGATGTTGGAATGAACTTCTCT
GCATTGACTCATGATTGGATTGAAAAGATAAACGCTCACCCGCCATTTAAGAAGCTGATCAAAACT
TACTTCAATGACAATGATCTTAAGTCGATGAATGTAAAGGGTGCCTCCCAGGGAATGTTTATGACA
TACGCATTAGCCCACGAGTTATTGACGATCATCAAGGAGGTGATAACCTCTTGTCAATCCATTGAC
TCCGTCCCCGAATACAACACAGAAGATATTTGGTTTCAGTTTGCACTTTTAATTCTGGAAAAGAAG
ACCGGCCACGTATTCAACAAGACAAGCACTCTCACGTATATGCCATGGGAACGTAAACTGCAGTGG

ACGAATGAACAAATAGAGTCCGCAAAGAGGGGCGAAAACATTCCGGTAAACAAGTTCATCATTAA
CAGCATTACCCTTTAA

SEQ ID NO: 4 is a nucleic acid sequence of a polypeptide having chondroitin synthase
activity: chimeric nucleic acid comprising a fragment from a nucleic acid encoding a
hyaluronan synthase originating from Pasteurella multocida, a fragment from a nucleic acid
encoding a chdroitin synthase from Pasteurella multocida and a fragment from a nucleic acid
encoding a chitin synthase 2 from Saccharomyces cerevisiae (HCOS.Sc)
ATGAACACCCTTTCCCAGGCAATTAAGGCTTATAATAGTAATGATTATCAACTAGCCCTCAAGCTG
TTCGAAAAGTCGGCAGAAATCTACGGCAGAAAGATAGTTGAGTTCCAAATTACGAAGTGCAAGGA
GAAACTTTCTGCGCACCCATCCGTTAACAGTGCGCATCTCTCGGTTAACAAAGAAGAGAAGGTGAA
TGTTTGCGACAGTCCATTAGACATAGCCACTCAATTATTGTTATCGAATGTGAAAAAACTCGTCCTT
AGTGATAGCGAGAAAAATACACTAAAGAATAAATGGAAGTTGTTGACTGAAAAGAAGTCTGAGAA
CGCAGAGGTCCGGGCCGTAGCTTTAGTCCCAAAGGATTTTCCCAAAGACTTAGTTTTAGCGCCACT
ACCAGACCATGTGAACGATTTTACCTGGTACAAGAATAGAAAGAAGAGCCTGGGCATAAAGCCTG
TGAACAAGAACATCGGGTTGTCAATCATAATCCCAACTTTCAATCGTAGTAGGATATTAGACATTA
CCCTGGCGTGTCTAGTTAATCAGAAAACTAACTATCCGTTTGAGGTGGTTGTTGCAGATGATGGGT
CGAAGGAAAATCTATTGACTATAGTTCAGAAATACGAGCAGAAACTAGACATTAAGTATGTGAGA
CAGAAGGACTATGGGTATCAGCTGTGCGCCGTTAGAAACCTTGGTCTAAGGACAGCGAAGTATGA
CTTTGTCTCTATCTTGGATTGCGACATGGCCCCGCAGCAGCTATGGGTTCACTCTTACCTTACAGAG
TTACTGGAAGACAACGACATCGTCTTGATCGGGCCAAGGAAATACGTTGATACCCACAACATCACG
GCTGAGCAGTTTCTGAACGACCCATATTTAATTGAGAGTTTACCGGAAACCGCCACAAACAATAAT
CCATCGATTACCAGTAAGGGTAACATCTCATTAGATTGGAGATTAGAACATTTTAAGAAGACAGAC
AACCTAAGACTATGCGACTCCCCGTTCAGGTATTTCTCATGCGGAAATGTGGCCTTCTCCAAAGAG
TGGCTGAACAAAGTTGGCTGGTTCGACGAAGAATTTAATCACTGGGGCGGCGAAGACGTCGAATTC
GGCTACAGGTTATTTGCAAAGGGTGCTTCTTTAGGGTTATCGACGGTGGTATGGCCTACCATCAA
GAGCCACCAGGAAAGGAGAACGAAACGGATCGTGAGGCCGGTAAGTCTATCACTCTTAAAATAGT
AAAGGAGAAAGTGCCCTACATCTACAGGAAGTTGTTGCCGATTGAGGACTCCCACATAAACCGAG
TACCATTGGTATCAATTTACATCCCGGCCTACAACTGTGCTAACTATATTCAAAGATGTGTTGATTC
AGCTTTGAATCAAACGGTAGTTGATCTCGAAGTGTGCATTTGCAACGACGGTAGTACTGACAACAC
GCTGGAAGTTATTAACAAGCTGTATGGTAATAATCCGCGTGTGCGTATAATGTCTAAACCCAATGG
CGGCATTGCGAGTGCATCCAACGCAGCGGTCAGCTTCGCAAAGGGTTATTACATAGGACAGTTGGA
CAGCGACGATTACTTAGAACCCGACGCAGTGGAGTTATGTCTCAAGGAATTTCTTAAGGATAAGAC
CCTTGCGTGCGTTTACACCACTAATCGTAACGTCAACCCAGATGGCTCTTTAATAGCCAATGGCTAT
AACTGGCCAGAGTTCAGTCGTGAGAAGTTGACTACGGCCATGATTGCTCATCACTTCCGGATGTTT
ACCATTCGTGCTTGGCATCTGACGGATGGGTTCAATGAGAAGATTGAGAACGCTGTTGACTACGAC
ATGTTTCTCAAGCTCAGTGAAGTTGGTAAATTTAAGCATCTGAACAAAATATGTTATAATCGGGTG

TTACACGGCGATAACACCTCAATCAAGAAGCTTGGCATACAAAAGAAGAATCATTTCGTAGTTGTC
AATCAGTCTCTAAACCGCCAAGGTATAACTTATTATAACTACGATGAATTTGATGATCTCGATGAG
AGTCGGAAATACATTTTCAACAAGACTGCAGAGTATCAAGAAGAGATAGATATTCTTAAAGATATT
TTTATTCAGATGCTTTTTCCTGGTTTCTATTGCAAATTCGTTCTTACCTTTATTATTTAGCAGGA
TCAATGAATTTAGTTATTAAACATGGTGAGGCCTTATTCATTTTTTTAAATACCTGATCTTTTGTGA
CTTGGCAAGTTTATTCATTATTTCCATGGGTAATAGACCCCAGGGCGAAACATTTATTCATTACC
TCCATGGTTATACTGTCTATATGTGCCACATATTCTCTAATTTGTGGGTTTGTTTTGCTTTCAAGTC
GTTAGCTTCTGGAACGGAATCCCACAAAATATTTGTCGACATCGTTATCTCATTGCTCTCCACCTAT
GGCCTATACTTTTTCTCATCACTGATGTACCTAGATCCTTGGCACATGTTTACATCATCCATACAAT
ACTTTTTGACACTTCCCGCCTTTACGTGTACTTTACAGATTTTTGCCTTCTGTAATACACACGACGTT
TCCTGGGGTACTAAAGGTTCCACACAGGAGTCCAAGCAATTGTCCAAGGCCATTGTCGTTCAAGGT
CCAGATGGGAAACAGATTGTGGAAACAGATTGGCCTCAGGAAGTTGATAAGAAGTTTTTGGAAAT
AAAAAGTCGTTTGAAAGAACCAGAATTTGAAGAATCAAGCGGCAATGAAAAACAATCCAAGAATG
ATTATTATAGAGATATAAGAACCAGAATTGTGATGATTTGGATGCTATCAAATCTAATACTGATCA
TGTCTATAATTCAAGTCTTTACACCACAAGATACTGACAATGGTTATTTGATATTCATTTTATGGTC
TGTGGCCGCTTTAGCTGCCTTTAGGGTGGTTGGTTCCATGGCCTTTTTGTTCATGAAATACTTGCGT
ATAATAGTGAGTTACAGAAATAAAGTTGAAGGTAGCGGCTCATGGGAAGTCTCTAAATTAGACTTA
CCAAATGTTTTCCACAAAAAGGGCTAA

SEQ ID NO: 5 is a nucleic acid sequence of a polypeptide having chondroitin synthase
activity: chimeric nucleic acid comprising a fragment from a nucleic acid encoding a
hyaluronan synthase originating from Pasteurella multocida and a fragment from a nucleic
acid encoding a chitin synthase 2 from Saccharomyces cerevisiae (HHASA.Sc)
ATGAACACCCTTTCCCAGGCAATTAAGGCTTATAATAGTAATGATTATCAACTAGCCCTCAAGCTG
TTCGAAAAGTCGGCAGAAATCTACGGCAGAAAGATAGTTGAgTTCCAAATTACGAAGTGCAAGGA
GAAACTTTCTGCGCACCCATCCGTTAACAGTGCGCATCTCTCGGTTAACAAAGAAGAGAAGGTGAA
TGTTTGCGACAGTCCATTAGACATAGCCACTCAATTATTGTTATCGAATGTGAAAAAACTCGTCCTT
AGTGATAGCGAGAAAAATACACTAAAGAATAAATGGAAGTTGTTGACTGAAAAGAAGTCTGAGAA
CGCAGAGGTCCGGGCCGTAGCTTTAGTTCCCAAGGACTTTCCGAAAGATTTAGTGCTTGCGCCGTT
ACCAGACCATGTGAACGATTTTACGTGGTATAAGAAGCGGAAGAAACGCCTAGGAATCAAACCAG
AACACCAACACGTTGGCCTCTCCATTATTGTGACAACGTTCAACCGTCCTGCCATCCTCAGCATCAC
CCTGGCTTGCCTCGTCAATCAAAAGACCCATTATCCTTTTGAGGTGATCGTGACCGACGATGGTTCT
CAAGAGGATTTATCGCCTATAATCCGTCAATATGAGAACAAGCTAGACATCCGTTATGTTCGGCAA
AAGGACAACGGTTTTCAGGCATCGGCCGCACGTAATATGGGCCTACGTCTAGCCAAATATGACTTC
ATTGGGCTCCTTGATTGTGATATGGCTCCCAACCCTTTGTGGGTACACTCATACGTTGCGGAACTAT
TAGAGGACGATGATTTGACCATAATAGGCCCCAGGAAGTACATTGACACGCAACACATAGATCCC
AAGGATTTCCTTAATAACGCCTCTCTGTTAGAGTCGTTGCCAGAGGTTAAGACCAATAATTCCGTCG

CGGCTAAGGGCGAGGGACCGTATCTTTAGACTGGCGTTTGGAACAATTTGAGAAGACCGAGAAC
TTGAGGCTATCCGATAGCCCTTTCCGATTCTTCGCAGCTGGGAATGTGGCTTTCGCCAAGAAGTGGC
TTAACAAGTCAGGATTCTTCGACGAGGAATTCAATCACTGGGGAGGTGAAGATGTAGAGTTCGGTT
ATCGTCTGTTTCGGTACGGTTCGTTCTTCAAAACTATAGACGGCATCATGGCCTATCATCAGGAACC
GCCAGGTAAAGAAAACGAAACTGACAGAGAAGCGGGCAAGAACATTACCCTCGATATAATGAGG
GAGAAGGTGCCTTACATCTACCGTAAACTCCTGCCTATAGAAGACAGTCATATCAACCGAGTACCA
TTGGTATCAATTTACATCCCGGCCTACAACTGTGCTAACTATATTCAAAGATGTGTTGATTCAGCTT
TGAATCAAACGGTAGTTGATCTCGAAGTGTGCATTTGCAACGACGGTAGTACTGACAACACGCTGG
AAGTTATTAACAAGCTGTATGGTAATAATCCGCGTGTGCGTATAATGTCTAAACCCAATGGCGGCA
TTGCGAGTGCATCCAACGCAGCGGTCAGCTTCGCAAAGGGTTATTACATAGGACAGTTGGACAGCG
ACGATTACTTAGAACCCGACGCAGTGGAGTTATGTCTCAAGGAATTTCTTAAGGATAAGACCCTTG
CGTGCGTTTACACCACTAATCGTAACGTCAACCCAGATGGCTCTTTAATAGCCAATGGCTATAACT
GGCCAGAGTTCAGTCGTGAGAAGTTGACTACGGCCATGATTGCTCATCACTTCCGGATGTTTACCA
TTCGTGCTTGGCATCTGACGGATGGGTTCAATGAGAAGATTGAGAACGCTGTTGACTACGACATGT
TTCTCAAGCTCAGTGAAGTTGGTAAATTTAAGCATCTGAACAAAATATGTTATAATCGGGTGTTAC
ACGGCGATAACACCTCAATCAAGAAgCTTGGCATACAAAAGAAGAATCATTTCGTAGTTGTCAATC
AGTCTCTAAACCGCCAAGGTATAACTTATTATAACTACGATGAATTTGATGATCTCGATGAGAGTC
GGAAATACATTTTCAACAAGACTGCAGAGTATCAAGAAGAGATAGATATTCTTAAAGATATTTTTA
TTCAGATGCTTTTTTCCTGGTTTTCTATTGCAAATTTCGTTCTTACCTTTTATTATTTAGCAGGATCA
ATGAATTTAGTTATTAAACATGGTGAGGCCTTATTCATTTTTTTTAAATACCTGATCTTTTGTGACTT
GGCAAGTTTATTCATTATTTCCATGGGTAATAGACCCCAGGGCGCGAAACATTTATTCATTACCTCC
ATGGTTATACTGTCTATATGTGCCACATATTCTCTAATTTGTGGGTTTGTTTTTGCTTTCAAGTCGTT
AGCTTCTGGAACGGAATCCCACAAAATATTTGTCGACATCGTTATCTCATTGCTCTCCACCTATGGC
CTATACTTTTTCTCATCACTGATGTACCTAGATCCTTGGCACATGTTTACATCATCCATACAATACTT
TTTGACACTTCCCGCCTTTACGTGTACTTTACAGATTTTGCCTTCTGTAATACACACGACGTTTCCT
GGGGTACTAAAGGTTCCACACAGGAGTCCAAGCAATTGTCCAAGGCCATTGTCGTTCAAGGTCCAG
ATGGGAAACAGATTGTGGAAACAGATTGGCCTCAGGAAGTTGATAAGAAGTTTTTGGAAATAAAA
AGTCGTTTGAAAGAACCAGAATTTGAAGAATCAAGCGGCAATGAAAAACAATCCAAGAATGATTA
TTATAGAGATATAAGAACCAGAATTGTGATGATTTGGATGCTATCAAATCTAATACTGATCATGTC
TATAATTCAAGTCTTTACACCACAAGATACTGACAATGGTTATTTGATATTCATTTTATGGTCTGTG
GCCGCTTTAGCTGCCTTTAGGGTGGTTGGTTCCATGGCCTTTTTGTTCATGAAATACTTGCGTATAA
TAGTGAGTTACAGAAATAAAGTTGAAGGTAGCGGCTCATGGGAAGTCTCTAAATTAGACTTACCAA
ATGTTTTCCACAAAAAGGGCTAA

SEQ ID NO: 6 is a nucleic acid sequence of a polypeptide having chondroitin synthase
activity: chimeric nucleic acid comprising a fragment from a nucleic acid encoding a
hyaluronan synthase originating from Chlorella virus PBCV-1 and a fragment from a nucleic
acid encoding a chondroitin synthase from Escherichia coli (HCOS1-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAGGGTGGAATGATGT
TAGGTTGGCTGTAATTATTGCCGGCTACCGTGAGGACCCGTATATGTTCCAAAAGTGTCTTGAAAG
TGTGCGTGACTCAGACTATGGGAATGTAGCTAGACTAATATGCGTTATTGACGGCGATGAAGACGA
CGACATGAGGATGGCTGCAGTGTACAAGGCTATCTATAACGACAACATCAAGAAACCTGAGTTTGT
CCTCTGTGAGAGTGACGATAAGGAGGGTGAGAGAATAGATAGCGATTTCAGCCGTGATATCTGCGT
GCTGCAACCGCATCGCGGAAAGCGTGAATGTTTGTACACAGGGTTCCAATTGGCAAAGATGGACCC
CTCAGTTAATGCCGTCGTCCTAATCGACAGTGACACTGTGTTAGAAAAGGACGCGATTCTCGAaGT
AGTATACCCGCTGGCATGCGATCCAGAAATACAGGCTGTAGCAGGCGAATGCAAAATATGGAATA
CTGACACACTGTTGAGTTTGCTGGTAGCCTGGCGATATTACAGCGCATTTTGCGTAGAGCGTAGCG
CCCAATCATTCTTCAGGACAGTACAATGCGTCGGAGGACCTCTCGGCGCCTACAAGATTGATATAA
TTAAGGAAATCAAGGACCCATGGATCAGCCAACGTTTCCTTGGCCAAAAGTGCACATACGGCGAC
GATAGACGACTCACTAATGAAATACTAATGAGGGGTAAGAAAGTAGTTTTCACCCCATTCGCTGTT
GGCTGGGGATGGTTCGACGAGGAGTTCACACACTGGGGTGGCCGTTACATTGTACAGCAAACACG
GTGGTCCAAGTCGTGGTGTAGGGAGATATGGTATACACTGTTTGCAGCATGGAAGCATGGACTTTC
GGGCATTTGGCTCGCATTCGAGTGCTTATACCAGATTACTTATTTCTTCCTGGTGATTTACCTATTCT
CCCGTTTGGCTGTTGAGGCTGACCCACGGGCGCAAACGGCCACCGTCATTGTTTCGACCACAGTTG
CGCTTATTAAGTGCGGTTACTTTAGCTTCAGAGCTAAAGACATTAGGGCCTTCTATTTCGTCCTCTA
CACGTTCGTTTACTTCTTCTGCATGATTCCGGCACGAATAACTGCGATGATGACCCTGTGGGACATA
GGTTGGGGAACCAGGGGAGGAAATGAGAAGCCTTCCGTAGGCACCAGAGTTGCTTTGTGGGCCAA
GCAATATTTGATTGCTTACATGTGGTGGGCCGCTGTCGTCGGTGCTGGCGTGTACTCCATCGTTCAT
AATTGGATGTTTGACTGGAATTCACTTTCCTACAGGTTCGCACTGGTAGGCATCTGTTCGTATATAG
TTTTCATTGTAATAGTGCTGGTAGTCTACTTTACAGGAAAGATCACGACCTGGAACTTCACGAAGCT
ACAGAAAGAATTGATCGAGGACCGCGTACTGTACGACGCAACGACCAATGCCCAGTCGGTATAA

SEQ ID NO: 7 is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a chondroitin synthase from Escherichia coli (HCOS2-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC

AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAGGGTGGAATGATGT
TAGGTTGGCTGTAATTATTGCCGGCTACCGTGAGGACCCGTATATGTTCCAAAAGTGTCTTGAAAG
TGTGCGTGACTCAGACTATGGGAATGTAGCTAGACTAATATGCGTTATTGACGGCGATGAAGACGA
CGACATGAGGATGGCTGCAGTGTACAAGGCTATCTATAACGACAACATCAAGAAACCTGAGTTTGT
CCTCTGTGAGAGTGACGATAAGGAGGGTGAGAGAATAGATAGCGATTTCAGCCGTGATATCTGCGT
GCTGCAACCGCATCGCGGAAAGCGTGAATGTTTGTACACAGGGTTCCAATTGGCAAAGATGGACCC
CTCAGTTAATGCCGTCGTCCTAATCGACAGTGACACTGTGTTAGAAAAGGACGCGATTCTCGAaGT
AGTATACCCGCTGGCATGCGATCCAGAAATACAGGCTGTAGCAGGCGAATGCAAAATATGGAATA
CTGACACACTGTTGAGTTTGCTGGTAGCCTGGCGATATTACAGCGCATTTTGCGTAGAGCGTAGCG
CCCAATCATTCTTCAGGACAGTACAATGCGTCGGAGGACCTCTCGGCGCCTACAAGATTGATATAA
TTAAGGAAATCAAGGACCCATGGATCAGCCAACGTTTCCTTGGCCAAAAGTGCACATACGGCGAC
GATAGACGACTCACTAATGAAATACTAATGAGGGGTAAGAAAGTAGTTTTCACCCCATTCGCTGTT
GGCTGGCTACACGGTGAGAATACGTCTATCAAGAAGTTACGTTACATTGTACAGCAAACACGGTGG
TCCAAGTCGTGGTGTAGGGAGATATGGTATACACTGTTTGCAGCATGGAAGCATGGACTTTCGGGC
ATTTGGCTCGCATTCGAGTGCTTATACCAGATTACTTATTTCTTCCTGGTGATTTACCTATTCTCCCG
TTTGGCTGTTGAGGCTGACCCACGGGCGCAAACGGCCACCGTCATTGTTTCGACCACAGTTGCGCT
TATTAAGTGCGGTTACTTTAGCTTCAGAGCTAAAGACATTAGGGCCTTCTATTTCGTCCTCTACACG
TTCGTTTACTTCTTCTGCATGATTCCGGCACGAATAACTGCGATGATGACCCTGTGGGACATAGGTT
GGGGAACCAGGGGAGGAAATGAGAAGCCTTCCGTAGGCACCAGAGTTGCTTTGTGGGCCAAGCAA
TATTTGATTGCTTACATGTGGTGGGCCGCTGTCGTCGGTGCTGGCGTGTACTCCATCGTTCATAATT
GGATGTTTGACTGGAATTCACTTTCCTACAGGTTCGCACTGGTAGGCATCTGTTCGTATATAGTTTT
CATTGTAATAGTGCTGGTAGTCTACTTTACAGGAAAGATCACGACCTGGAACTTCACGAAGCTACA
GAAAGAATTGATCGAGGACCGCGTACTGTACGACGCAACGACCAATGCCCAGTCGGTATAA

SEQ ID NO: 8 is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a Galactofuranosyltransferase from Mycobacterium tuberculosis (HCOS3-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAGGGTGGAATGATGT
TAGGTTGGCTGTAATTATTGCCGGCTACCGTGAGGACCCGTATATGTTCCAAAAGTGTCTTGAAAG
TGTGCGTGACTCAGACTATGGGAATGTAGCTAGACTAATATGCGTTATTGACGGCGATGAAGACGA
CGACATGAGGATGGCTGCAGTGTACAAGGCTATCTATAACGACAACATCAAGAAACCTGAGTTTGT
CCTCTGTGAGAGTGACGATAAGGAGGGTGAGAGAATAGATAGCGATTTCAGCCGTGATATCTGCGT
GCTGCAACCGCATCGCGGAAAGCGTGAATGTTTGTACACAGGGTTCCAATTGGCAAAGATGGACCC
CTCAGTTAATGCCGTCGTCCTAATCGACAGTGACACTGTGTTAGAAAAGGACGCGATTCTCGAaGT
AGTATACCCGCTGGCATGCGATCCAGAAATACAGGCTGTAGCAGGCGAATGCAAAATATGGAATA

CTGACACACTGTTGAGTTTGCTGGTAGCCTGGCGATATTACAGCGCATTTTGCGTAGAGCGTAGCG
CCCAATCATTCTTCAGGACAGTACAATGCGTCGGAGGACCTCTCGGCGCCTACAAGATTGATATAA
TTAAGGAAATCAAGGACCCATGGATCAGCCAACGTTTCCTTGGCCAAAAGTGCACATACGGCGAC
GATAGACGACTCACTAATGAAATACTAATGAGGGGTAAGAAAGTAGTTTTCACCCCATTCGCTGTT
GGCTGGCACATGGCATGGTCCGACAAGGACGACGCAATCGACCGTTACATTGTACAGCAAACACG
GTGGTCCAAGTCGTGGTGTAGGGAGATATGGTATACACTGTTTGCAGCATGGAAGCATGGACTTTC
GGGCATTTGGCTCGCATTCGAGTGCTTATACCAGATTACTTATTTCTTCCTGGTGATTTACCTATTCT
CCCGTTTGGCTGTTGAGGCTGACCCACGGGCGCAAACGGCCACCGTCATTGTTTCGACCACAGTTG
CGCTTATTAAGTGCGGTTACTTTAGCTTCAGAGCTAAAGACATTAGGGCCTTCTATTTCGTCCTCTA
CACGTTCGTTTACTTCTTCTGCATGATTCCGGCACGAATAACTGCGATGATGACCCTGTGGACATA
GGTTGGGGAACCAGGGGAGGAAATGAGAAGCCTTCCGTAGGCACCAGAGTTGCTTTGTGGGCCAA
GCAATATTTGATTGCTTACATGTGGTGGGCCGCTGTCGTCGGTGCTGGCGTGTACTCCATCGTTCAT
AATTGGATGTTTGACTGGAATTCACTTTCCTACAGGTTCGCACTGGTAGGCATCTGTTCGTATATAG
TTTTCATTGTAATAGTGCTGGTAGTCTACTTTACAGGAAAGATCACGACCTGGAACTTCACGAAGCT
ACAGAAAGAATTGATCGAGGACCGCGTACTGTACGACGCAACGACCAATGCCCAGTCGGTATAA

SEQ ID NO: 9 is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a chondroitin synthase from Escherichia coli (HCOS4-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAGGGTGGAATGATGT
TAGGTTGGCTGTAATTATTGCCGGCTACCGTGAGGACCCGTATATGTTCCAAAAGTGTCTTGAAAG
TGTGCGTGACTCAGACTATGGGAATGTAGCTAGACTAATATGCGTTATTGACGGCGATGAAGACGA
CGACATGAGGATGGCTGCAGTGTACAAGGCTATCTATAACGACAACATCAAGAAACCTGAGTTTGT
CCTCTGTGAGAGTGACGATAAGGAGGGTGAGAGAATAGATAGCGATTTCAGCCGTGATATCTGCGT
GCTGCAACCGCATCGCGGAAAGCGTGAATGTTTGTACACAGGGTTCCAATTGGCAAAGATGGACCC
CTCAGTTAATGCCGTCGTCCTAATCGACAGTGACACTGTGTTAGAAAAGGACGCGATTCTCGAaGT
AGTATACCCGCTGGCATGCGATCCAGAAATACAGGCTGTAGCAGGCGAATGCAAAATATGGAATA
CTGACACACTGTTGAGTTTGCTGGTAGCCTGGCGATATTACAGCGCATTTTGCGTAGAGCGTAGCG
CCCAATCATTCTTCAGGACAGTACAATGCGTCGGAGGACCTCTCGGCGCCTACAAGATTGATATAA
TTAAGGAAATCAAGGACCCATGGATCAGCCAACGTTTCCTTGGCCAAAAGTGCACATACGGCGAC
GATAGACGACTCACTAATGAAATACTAATGAGGGGTAAGAAAGTAGTTTTCACCCCATTCGCTGTT
GGCTGGTTCGACGAGGAGTTCACACACTGGGGTGGCCGTTACATTGTACAGCAAACACGGTGGTCC
AAGTCGTGGTGTAGGGAGATATGGTATACACTGTTTGCAGCATGGAAGCATGGACTTTCGGGCATT
TGGCTCGCATTCGAGTGCTTATACCAGATTACTTATTTCTTCCTGGTGATTTACCTATTCTCCCGTTT
GGCTGTTGAGGCTGACCCACGGGCGCAAACGGCCACCGTCATTGTTTCGACCACAGTTGCGCTTAT
TAAGTGCGGTTACTTTAGCTTCAGAGCTAAAGACATTAGGGCCTTCTATTTCGTCCTCTACACGTTC
GTTTACTTCTTCTGCATGATTCCGGCACGAATAACTGCGATGATGACCCTGTGGGACATAGGTTGG
GGAACCAGGGGAGGAAATGAGAAGCCTTCCGTAGGCACCAGAGTTGCTTTGTGGGCCAAGCAATA
TTTGATTGCTTACATGTGGTGGGCCGCTGTCGTCGGTGCTGGCGTGTACTCCATCGTTCATAATTGG
ATGTTTGACTGGAATTCACTTTCCTACAGGTTCGCACTGGTAGGCATCTGTTCGTATATAGTTTTCA
TTGTAATAGTGCTGGTAGTCTACTTTACAGGAAAGATCACGACCTGGAACTTCACGAAGCTACAGA
AAGAATTGATCGAGGACCGCGTACTGTACGACGCAACGACCAATGCCCAGTCGGTATAA

SEQ ID NO: 10 is a nucleic acid sequence of a polypeptide having chondroitin synthase
activity: chimeric nucleic acid comprising a fragment from a nucleic acid encoding a
hyaluronan synthase originating from Chlorella virus PBCV-1 and a fragment from a nucleic
acid encoding a chondroitin synthase from Penicillium oxalicum (HCOS5-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAATCCATTCACCATCCA
ATTTCGAAAACGACTCACCCCGACACAGAAAATGCAGGTGACTCAACATTGTTGTGCCCGCCCATG
GATGGTATGGACGACATATTAGTCGTTATGAAGACAGGTGTCACAGAGGCTAAGGAAAAGGTGCC
AGTACATTTTCAGACAACGCTGAGGTGTGTGCCGCACTACGTTATCTATAGCGACTTCGAAGAAGA
GATAGAAGGTGTAAAGATTTACGACGTCTTGCAAGGAATGGACCCCGATGTTATGACGCAGGTTCC
GGACTTCGATCTTTACAATCGTATTAAGAAGTTAGGGCGTCAGGGCCTTGCGGAGAACGACTTCGC
GGACGAAGCGAACTCTGCGGTGGGTAAGCCTAATAACCCTGGTTGGAAGTTAGATAAATGGAAGT
TCTTACCTATGATCATTGAGGCTCTGAGATACAAACCAAAGGCTAAGTGGTATGTTTTCATGGAAG
CAGACACTTATATATCTTGGCCGACTTTATTAGCCTGGCTAAAACACTTCAACCACAAGAAGCCGT
GGTATCTTGGCACTGAGACACAGATAGCCGATGTGATTTTCGCTCATGGTGGTTCAGGATTCATGA
TTAGTCATGCCGCGCTTAAGCTTGCTGCTGAAGAATATACCGCACGTCGTACTGAGTTGCACCACT
ACACAGACGGTCACTGGGCAGGAGACTGCGTATTAGGTAAAGTTCTTGCTGACGCAGGTGTGAACT
TGCATTTCTCCTGGCCAATTTTACAGAATTCCAATCTGGGCGAACTGGATGAGTTCAACGTTGACTT
ATACAGGCAACCTTGGTGCTTTCCAGCTGCCGCTTTCCACCACTTGAACGCTCAGAAGATCCAGGA
CTTATGGTCTTACGAGCAGAAAAGGTGGCAGGACAAGGACAAGAGGGTTCTGTTACATAGCGACG
TCTTCAGGGACCTATTATATCCGGATATAGCATTAAAACCCGCGAGGATCGGTTGGGACAACCTGA
GTGCAGACGAACAGTCTACGGCCTCTAGTTTCGAGGAATGCAGGCAAATTTGTGATCAATTAGACG
ATTGTACCCAGTTTAGTTTCAGGGACAAATCCTGTTTTACATCAAGAACTCCACGTTTGGGCGCAGC
CAATCCAAATACAGTTAGCGGATGGATGGTTGATCGTATCCGTGCGAATATGGAGAAGTCTGGACT
GTTTGCAGCATGGAAGCATGGACTTTCGGGCATTTGGCTCGCATTCGAGTGCTTATACCAGATTACT
TATTTCTTCCTGGTGATTTACCTATTCTCCCGTTTGGCTGTTGAGGCTGACCCACGGGCGCAAACGG
CCACCGTCATTGTTTCGACCACAGTTGCGCTTATTAAGTGCGGTTACTTTAGCTTCAGAGCTAAAGA
CATTAGGGCCTTCTATTTCGTCCTCTACACGTTCGTTTACTTCTTCTGCATGATTCCGGCACGAATAA
CTGCGATGATGACCCTGTGGGACATAGGTTGGGGAACCAGGGGAGGAAATGAGAAGCCTTCCGTA
GGCACCAGAGTTGCTTTGTGGGCCAAGCAATATTTGATTGCTTACATGTGGTGGGCCGCTGTCGTC
GGTGCTGGCGTGTACTCCATCGTTCATAATTGGATGTTTGACTGGAATTCACTTTCCTACAGGTTCG
CACTGGTAGGCATCTGTTCGTATATAGTTTTCATTGTAATAGTGCTGGTAGTCTACTTTACAGGAAA
GATCACGACCTGGAACTTCACGAAGCTACAGAAAGAATTGATCGAGGACCGCGTACTGTACGACG
CAACGACCAATGCCCAGTCGGTAtaa

SEQ ID NO: 11: is a nucleic acid sequence of a polypeptide having chondroitin synthase activity: chimeric nucleic acid comprising a fragment from a nucleic acid encoding a hyaluronan synthase originating from Chlorella virus PBCV-1 and a fragment from a nucleic acid encoding a chondroitin synthase from Pasteurella multocida (HCOS6-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTCTTGCCCAGGTATTATTT
AGTGAACTCAACCGGAAAAGGCTCCGGAAGTGGATTTCCCTCCGACCCAAATTAGACATTGCAACA
CAGTTATTACTATCGAATGTTAAGAAATTGACCTTAAGCGAGTCCGAAAAGAACAGCTTAAAGAAT
AAGTGGAAGAGTATAACAGGCAAGAAGTCCGAGAATGCTGAAATTAGGAAGGTTGAGTTAGTCCC
AAAGGATTTTCCCAAAGACTTAGTTTTAGCGCCACTACCAGACCATGTGAACGATTTTACCTGGTA
CAAGAATAGAAAGAAGAGCCTGGGCATAAAGCCTGTGAACAAGAACATCGGGTTGTCAATCATAA
TCCCAACTTTCAATCGTAGTAGGATATTAGACATTACCCTGGCGTGTCTAGTTAATCAGAAAACTA
ACTATCCGTTTGAGGTGGTTGTTGCAGATGATGGGTCGAAGGAAAATCTATTGACTATAGTTCAGA
AATACGAGCAGAAACTAGACATTAAGTATGTGAGACAGAAGGACTATGGGTATCAGCTGTGCGCC
GTTAGAAACCTTGGTCTAAGGACAGCGAAGTATGACTTTGTCTCTATCTTGGATTGCGACATGGCC
CCGCAGCAGCTATGGGTTCACTCTTACCTTACAGAGTTACTGGAAGACAACGACATCGTCTTGATC
GGGCCAAGGAAATACGTTGATACCCACAACATCACGGCTGAGCAGTTTCTGAACGACCCATATTTA
ATTGAGAGTTTACCGGAAACCGCCACAAACAATAATCCATCGATTACCAGTAAGGGTAACATCTCA
TTAGATTGGAGATTAGAACATTTTAAGAAGACAGACAACCTAAGACTATGCGACTCCCCGTTCAGG
TATTTCTCATGCGGAAATGTGGCCTTCTCCAAAGAGTGGCTGAACAAAGTTGGCTGGTTCGACGAA
GAATTTAATCACTGGGGCGGCGAAGACGTCGAATTCGGCTACAGGTTATTTGCAAAAGGGTGCTTC
TTTAGGGTTATCGACGGTGGTATGGCCTACCATCAAGAGCCACCAGGAAAGGAGAACGAAACGGA
TCGTGAGGCCGGTAAGTCTATCACTCTTAAAATAGTAAAGGAGAAAGTGCCCTACATCTACAGGAA
GTTGTTGCCGATTGAGGACTCCCACATACACCGTATACCGCTTGTGAGCATATACATTCCAGCGTAC
AACTGCGCCAATTATATTCAAAGGTGTGTAGATAGCGCGTTGAATCAAACAGTGGTAGATTTGGAA
GTTTGTATATGTAACGACGGAAGTACTGACAATACTTTAGAGGTGATTAACAAGCTGTATGGCAAC
AACCCGCGTGTCAGGATAATGAGCAAGCCCAATGGTGGAATTGCTTCCGCGTCTAACGCTGCCGTT
TCATTCGCGAAAGGCTACTACATTGGCCAGTTGGACTCGGATGATTACTTGGAACCTGACGCAGTG
GAGCTTTGTTTAAAGGAGTTCTTGAAGGACAAAACACTAGCCTGTGTATATACAACGAACAGAAAT
GTTAATCCTGACGGTTCCCTAATCGCTAACGGATACAATTGGCCTGAGTTCAGCAGGGAGAAGCTG
ACCACTGCTATGATCGCCCACCATTTCAGGATGTTCACAATCAGGGCTTGGCATTTAACAGATGGTT
TTAATGAGAAAATAGAGAACGCAGTAGACTACGATATGTTTCTGAAGTTATCAGAGGTCGGGAAA
TTCAAACACTTGAACAAAATTTGTTACAATAGGGTATTACACGGTGACAACACCAGTATTAAGAAT
CTTGACACCCAAAAGAAGAACCACTTCGTAGTAGTGAACCAGTCTCTGAACAGACAAAGGGTGTC
GAATTATAACTACGATGAGTTTGATAACCTGGACGAGAGCCGTAAGTACATATTCAACAAGCTGTT
TGCAGCATGGAAGCATGGACTTTCGGGCATTTGGCTCGCATTCGAGTGCTTATACCAGATTACTTAT
TTCTTCCTGGTGATTTACCTATTCTCCCGTTTGGCTGTTGAGGCTGACCCACGGGCGCAAACGGCCA

CCGTCATTGTTTCGACCACAGTTGCGCTTATTAAGTGCGGTTACTTTAGCTTCAGAGCTAAAGACAT
TAGGGCCTTCTATTTCGTCCTCTACACGTTCGTTTACTTCTTCTGCATGATTCCGGCACGAATAACTG
CGATGATGACCCTGTGGACATAGGTTGGGGAACCAGGGGAGGAAATGAGAAGCCTTCCGTAGGC
ACCAGAGTTGCTTTGTGGGCCAAGCAATATTTGATTGCTTACATGTGGTGGGCCGCTGTCGTCGGTG
CTGGCGTGTACTCCATCGTTCATAATTGGATGTTTGACTGGAATTCACTTTCCTACAGGTTCGCACT
GGTAGGCATCTGTTCGTATATAGTTTTCATTGTAATAGTGCTGGTAGTCTACTTTACAGGAAAGATC
ACGACCTGGAACTTCACGAAGCTACAGAAAGAATTGATCGAGGACCGCGTACTGTACGACGCAAC
GACCAATGCCCAGTCGGTAtaa

SEQ ID NO: 12: is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a chondroitin synthase from Pasteurella multocida (HCOS7-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAATCCCAACTTTCAAT
CGTAGTAGGATATTAGACATTACCCTGGCGTGTCTAGTTAATCAGAAAACTAACTATCCGTTTGAG
GTGGTTGTTGCAGATGATGGGTCGAAGGAAAATCTATTGACTATAGTTCAGAAATACGAGCAGAA
ACTAGACATTAAGTATGTGAGACAGAAGGACTATGGGTATCAGCTGTGCGCCGTTAGAAACCTTGG
TCTAAGGACAGCGAAGTATGACTTTGTCTCTATCTTGGATTGCGACATGGCCCCGCAGCAGCTATG
GGTTCACTCTTACCTTACAGAGTTACTGGAAGACAACGACATCGTCTTGATCGGGCCAAGGAAATA
CGTTGATACCCACAACATCACGGCTGAGCAGTTTCTGAACGACCCATATTTAATTGAGAGTTTACC
GGAAACCGCCACAAACAATAATCCATCGATTACCAGTAAGGGTAACATCTCATTAGATTGGAGATT
AGAACATTTTAAGAAGACAGACAACCTAAGACTATGCGACTCCCCGTTCAGGTATTTCTCATGCGG
AAATGTGGCCTTCTCCAAAGAGTGGCTGAACAAAGTTGGCTGGTTCGACGAAGAATTTAATCACTG
GGGCGGCGAAGACGTCGAATTCGGCTACAGGTTATTTGCAAAAGGGTGCTTCTTTAGGGTTATCGA
CGGTGGTATGGCCTACCATCAAGAGCCACCAGGAAAGGAGAACGAAACGGATCGTGAGGCCGGTA
AGTCTATCACTCTTAAAATAGTAAAGGAGAAAGTGCCCTACATCTACAGGAAGTTGTTGCCGATTG
AGGACTCCCACATACACCGTATACCGCTTGTGAGCATATACATTCCAGCGTACAACTGCGCCAATT
ATATTCAAAGGTGTGTAGATAGCGCGTTGAATCAAACAGTGGTAGATTTGGAAGTTTGTATATGTA
ACGACGGAAGTACTGACAATACTTTAGAGGTGATTAACAAGCTGTATGGCAACAACCCGCGTGTCA
GGATAATGAGCAAGCCCAATGGTGGAATTGCTTCCGCGTCTAACGCTGCCGTTTCATTCGCGAAAG
GCTACTACATTGGCCAGTTGGACTCGGATGATTACTTGGAACCTGACGCAGTGGAGCTTTGTTTAA
AGGAGTTCTTGAAGGACAAAACACTAGCCTGTGTATATACAACGAACAGAAATGTTAATCCTGACG
GTTCCCTAATCGCTAACGGATACAATTGGCCTCTGTTTGCAGCATGGAAGCATGGACTTTCGGGCA
TTTGGCTCGCATTCGAGTGCTTATACCAGATTACTTATTTCTTCCTGGTGATTTACCTATTCTCCCGT

TTGGCTGTTGAGGCTGACCCACGGGCGCAAACGGCCACCGTCATTGTTTCGACCACAGTTGCGCTT
ATTAAGTGCGGTTACTTTAGCTTCAGAGCTAAAGACATTAGGGCCTTCTATTTCGTCCTCTACACGT
TCGTTTACTTCTTCTGCATGATTCCGGCACGAATAACTGCGATGATGACCCTGTGGACATAGGTTG
GGGAACCAGGGGAGGAAATGAGAAGCCTTCCGTAGGCACCAGAGTTGCTTTGTGGGCCAAGCAAT
ATTTGATTGCTTACATGTGGTGGGCCGCTGTCGTCGGTGCTGGCGTGTACTCCATCGTTCATAATTG
GATGTTTGACTGGAATTCACTTTCCTACAGGTTCGCACTGGTAGGCATCTGTTCGTATATAGTTTTC
ATTGTAATAGTGCTGGTAGTCTACTTTACAGGAAAGATCACGACCTGGAACTTCACGAAGCTACAG
AAAGAATTGATCGAGGACCGCGTACTGTACGACGCAACGACCAATGCCCAGTCGGTAtaa

SEQ ID NO: 13: is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a chondroitin synthase from Pasteurella multocida (HCOS8-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAGGGTGGAATGATGT
TAGGTTGGCTGTAATTATTGCCGGCTACCGTGAGGACCCGTATATGTTCCAAAAGTGTCTTGAAAG
TGTGCGTGACTCAGACTATGGGAATGTAGCTAGACTAATATGCGTTATTGACGGCGATGAAGACGA
CGACATGAGGATGGCTGCAGTGTACAAGGCTATCTATAACGACAACATCAAGAAACCTGAGTTTGT
CCTCTGTGAGAGTGACGATAAGGAGGGTGAGAGAATAGATAGCGATTTCAGCCGTGATATCTGCGT
GCTGCAACCGCATCGCGGAAAGCGTGAATGTTTGTACACAGGGTTCCAATTGGCAAAGATGGACCC
CTCAGTTAATGCCGTCGTCCTAATCGACAGTGACACTGTGTTAGAAAAGGACGCGATTCTCGAaGT
AGTATACCCGCTGGCATGCGATCCAGAAATACAGGCTGTAGCAGGCGAATGCAAAATATGGAATA
CTGACACACTGTTGAGTTTGCTGGTAGCCTGGCGATATTACAGCGCATTTTGCGTAGAGCGTAGCG
CCCAATCATTCTTCAGGACAGTACAATGCGTCAGACTATGCGACTCCCCGTTCAGGTATTTCTCATG
CGGAAATGTGGCCTTCTCCAAAGAGTGGCTGAACAAAGTTGGCTGGTTCGACGAAGAATTTAATCA
CTGGGGCGGCGAAGACGTCGAATTCGGCTACAGGTTAAAGAAAGTAGTTTTCACCCCATTCGCTGT
TGGCTGGTCCGACAGCCCGACGAACGTCTTCCGTTACATTGTACAGCAAACACGGTGGTCCAAGTC
GTGGTGTAGGGAGATATGGTATACACTGTTTGCAGCATGGAAGCATGGACTTTCGGGCATTTGGCT
CGCATTCGAGTGCTTATACCAGATTACTTATTTCTTCCTGGTGATTTACCTATTCTCCCGTTTGGCTG
TTGAGGCTGACCCACGGGCGCAAACGGCCACCGTCATTGTTTCGACCACAGTTGCGCTTATTAAGT
GCGGTTACTTTAGCTTCAGAGCTAAAGACATTAGGGCCTTCTATTTCGTCCTCTACACGTTCGTTTA
CTTCTTCTGCATGATTCCGGCACGAATAACTGCGATGATGACCCTGTGGGACATAGGTTGGGGAAC
CAGGGGAGGAAATGAGAAGCCTTCCGTAGGCACCAGAGTTGCTTTGTGGGCCAAGCAATATTTGAT
TGCTTACATGTGGTGGGCCGCTGTCGTCGGTGCTGGCGTGTACTCCATCGTTCATAATTGGATGTTT
GACTGGAATTCACTTTCCTACAGGTTCGCACTGGTAGGCATCTGTTCGTATATAGTTTTCATTGTAA
TAGTGCTGGTAGTCTACTTTACAGGAAAGATCACGACCTGGAACTTCACGAAGCTACAGAAAGAAT
TGATCGAGGACCGCGTACTGTACGACGCAACGACCAATGCCCAGTCGGTAtaa

SEQ ID NO: 14: is a nucleic acid sequence of a polypeptide having chondroitin synthase activity: chimeric nucleic acid comprising a fragment from a nucleic acid encoding a hyaluronan synthase originating from Chlorella virus PBCV-1 and a fragment from a nucleic acid encoding a chondroitin sulfate synthase from Homo sapiens (HCOS9-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTCTTGCCCAGGTATTATTT
AGTGAACTCAACCGGAAAAGGCTCCGGAAGTGGATTTCCCTCCGACCCAAACACATTGGTAAGTGT
CTAAGGGAAATGTATACGACTCATGAGGACGTGGAAGTAGGAAGATGCGTTAGGAGATTCGCTGG
AGTACAGTGTGTTTGGTCTTACGAGATGCAACAGTTATTCTACGAGAACTACGAGCAGAATAAGAA
GGGATACATCCGTGATCTGCACAACTCCAAAATTCACCAAGCAATAACGCTGCATCCTAACAAGAA
TCCGCCCTATCAGTATAGGTTGCACTCTTACATGTTGAGTAGAAAGATATCTGAGCTACGTCATAG
AACTATTCAGTTGCACAGGGAGATAGTACTGATGTCTAAATACTCAAACACGGAGATCCATAAGGA
AGACCTACAGTTGGGTATACCACCTTCGTTCATGAGATTCCAGCCAAGACAGAGGGAAGAAATTTT
GGAGTGGGAGTTCTTGACTGGTAAATACTTGTATTCTGCTGTTGACGGTCAACCACCTAGGAGAGG
GATGGACTCTGCACAGCGTGAGGCTTTAGACGATATAGTGATGCAAGTAATGGAAATGATCAACG
CTAATGCAAAGACACGTGGTCGTATCATAGATTTCAAAGAGATCCAGTATGGATATCGTCGTGTGA
ACCCAATGTACGGCGCGGAGTATATTCTTGACTTATTGTTGCTATACAAGAAACATAAGGGTAAGA
AGATGACTGTCCCGGTTCGTAGGCACGCGTATTTGCAACAGACTTTCTCGAAAATACAGTTTGTAG
AGCATGAGGAACTTGACGCGCAAGAGTTAGCGAAACGTATAAACCAAGAGTCGGGGAGTTTATCC
TTCTTAAGTAATTCCCTTAAGAAGTTGGTTCCTTTCCAGCTGCCTGGAAGTAAAAGTGAGCACAAG
GAGCCAAAGGATAAGAAAATCAACATTCTTATACCTCTTTCCGGCAGATTCGACATGTTCGTTAGG
TTCATGGGTAACTTCGAGAAGACCTGCTTAATTCCTAACCAGAATGTTAAGTTGGTAGTTTTGTTAT
TCAATTCTGACAGTAACCCCGACAAAGCAAAGCAGGTTGAACTGATGAGAGACTATAGGATTAAG
TACCCAAAAGCAGATATGCAGATACTACCTGTTTCCGGAGAATTCTCAAGAGCCTTGGCTCTGGAA
GTAGGCAGCTCTCAGTTTAATAATGAGTCTCTTCTGTTCTTCTGCGACGTTGACTTAGTTTTCACGA
CAGAGTTTCTGCAGAGATGTCGTGCAAACACTGTACTAGGTCAGCAGATATATTTCCCCATTATTTT
CAGTCAGTACGATCCAAAGATTGTTTATAGTGGAAAAGTTCCATCTGACAACCATTTTGCGTTTACT
CAAAAGACAGGGTTTTGGCGTAACTACGGGTTCGGCATAACATGTATCTATAAAGGGGACCTGGTA
AGAGTTGGCGGATTCGACGTATCTATACAGGGCTGGGGTTTAGAAGACGTCGATCTATTTAATAAA
GTAGTTCAGGCAGGTTTGAAGACATTCCGTAGTCAAGAGGTAGGCGTTGTACACGTGCACCATCCA
GTATTTTGCGACCCAAACTTAGACCCGAAGCAATATAAAATGTGCTTGGGTTCCAAGGCATCGACT
TACGGTTCCACGCAGCAACTACTGTTTGCAGCATGGAAGCATGGACTTTCGGGCATTTGGCTCGCA
TTCGAGTGCTTATACCAGATTACTTATTTCTTCCTGGTGATTTACCTATTCTCCCGTTTGGCTGTTGA
GGCTGACCCACGGGCGCAAACGGCCACCGTCATTGTTTCGACCACAGTTGCGCTTATTAAGTGCGG
TTACTTTAGCTTCAGAGCTAAAGACATTAGGGCCTTCTATTTCGTCCTCTACACGTTCGTTTACTTCT
TCTGCATGATTCCGGCACGAATAACTGCGATGATGACCCTGTGGGACATAGGTTGGGGAACCAGGG
GAGGAAATGAGAAGCCTTCCGTAGGCACCAGAGTTGCTTTGTGGGCCAAGCAATATTTGATTGCTT

ACATGTGGTGGGCCGCTGTCGTCGGTGCTGGCGTGTACTCCATCGTTCATAATTGGATGTTTGACTG
GAATTCACTTTCCTACAGGTTCGCACTGGTAGGCATCTGTTCGTATATAGTTTTCATTGTAATAGTG
CTGGTAGTCTACTTTACAGGAAAGATCACGACCTGGAACTTCACGAAGCTACAGAAAGAATTGATC
GAGGACCGCGTACTGTACGACGCAACGACCAATGCCCAGTCGGTATAA

SEQ ID NO: 15: is a reencoded nucleic acid sequence of a polypeptide having chondroitin synthase activity: chimeric nucleic acid comprising a fragment from a nucleic acid encoding a hyaluronan synthase originating from Chlorella virus PBCV-1 and a fragment from a nucleic acid encoding a chondroitin sulfate synthase from Homo sapiens (HCOS10-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAAGAGATAGAAACTT
CCTATTCGTTGGTGTTATGACAGCTCAGAAGTACTTACAGACCAGGGCGGTTGCAGCATATAGAAC
CTGGTCAAAGACAATTCCAGGTAAGGTACAGTTCTTCAGCTCCGAGGGTTCTGACACCAGCGTCCC
TATCCCAGTAGTGCCTTTGCGTGGAGTGGATGATTCATACCCACCTCAGAAGAAGTCCTTCATGAT
GCTTAAGTATATGCACGACCACTATCTGGACAAATACGAGTGGTTCATGAGAGCAGACGACGACGT
TTACATAAAGGGTGATAGACTGGAGAATTTCTTGAGGTCACTAAACAGTTCAGAACCACTTTTCCT
AGGACAGACGGGCCTTGGTACGACAGAGGAGATGGGTAAGTTGGCATTGGAGCCAGGGGAGAACT
TCTGCATGGGTGGCCCTGGTGTGATTATGAGTAGGGAAGTTTTAAGAAGAATGGTTCCGCACATTG
GTAAGTGTCTAAGGGAAATGTATACGACTCATGAGGACGTGGAAGTAGGAAGATGCGTTAGGAGA
TTCGCTGGAGTACAGTGTGTTTGGTCTTACGAGATGCAACAGTTATTCTACGAGAACTACGAGCAG
AATAAGAAGGGATACATCCGTGATCTGCACAACTCCAAAATTCACCAAGCAATAACGCTGCATCCT
AACAAGAATCCGCCCTATCAGTATAGGTTGCACTCTTACATGTTGAGTAGAAAGATATCTGAGCTA
CGTCATAGAACTATTCAGTTGCACAGGGAGATAGTACTGATGTCTAAATACTCAAACACGGAGATC
CATAAGGAAGACCTACAGTTGGGTATACCACCTTCGTTCATGAGATTCCAGCCAAGACAGAGGGA
AGAAATTTTGGAGTGGGAGTTCTTGACTGGTAAATACTTGTATTCTGCTGTTGACGGTCAACCACCT
AGGAGAGGGATGGACTCTGCACAGCGTGAGGCTTTAGACGATATAGTGATGCAAGTAATGGAAAT
GATCAACGCTAATGCAAAGACACGTGGTCGTATCATAGATTTCAAAGAGATCCAGTATGGATATCG
TCGTGTGAACCCAATGTACGGCGCGGAGTATATTCTTGACTTATTGTTGCTATACAAGAAACATAA
GGGTAAGAAGATGACTGTCCCGGTTCGTAGGCACGCGTATTTGCAACAGACTTTCTCGAAAATACA
GTTTGTAGAGCATGAGGAACTTGACGCGCAAGAGTTAGCGAAACGTATAAACCAAGAGTCGGGGA
GTTTATCCTTCTTAAGTAATTCCCTTAAGAAGTTGGTTCCTTTCCAGCTGCCTGGAAGTAAAAGTGA
GCACAAGGAGCCAAAGGATAAGAAAATCAACATTCTTATACCTCTTTCCGGCAGATTCGACATGTT
CGTTAGGTTCATGGGTAACTTCGAGAAGACCTGCTTAATTCCTAACCAGAATGTTAAGTTGGTAGTT
TTGTTATTCAATTCTGACAGTAACCCCGACAAAGCAAAGCAGGTTGAACTGATGAGAGACTATAGG
ATTAAGTACCCAAAAGCAGATATGCAGATACTACCTGTTTCCGGAGAATTCTCAAGAGCCTTGGCT

CTGGAAGTAGGCAGCTCTCAGTTTAATAATGAGTCTCTTCTGTTCTTCTGCGACGTTGACTTAGTTT
TCACGACAGAGTTTCTGCAGAGATGTCGTGCAAACACTGTACTAGGTCAGCAGATATATTTCCCCA
TTATTTTCAGTCAGTACGATCCAAAGATTGTTATAGTGGAAAAGTTCCATCTGACAACCATTTTGC
GTTTACTCAAAAGACAGGGTTTTGGCGTAACTACGGGTTCGGCATAACATGTATCTATAAAGGGGA
CCTGGTAAGAGTTGGCGGATTCGACGTATCTATACAGGGCTGGGGTTTAGAAGACGTCGATCTATT
TAATAAAGTAGTTCAGGCAGGTTTGAAGACATTCCGTAGTCAAGAGGTAGGCGTTGTACACGTGCA
CCATCCAGTATTTTGCGACCCAAACTTAGACCCGAAGCAATATAAAATGTGCTTGGGTTCCAAGGC
ATCGACTTACGGTTCCACGCAGCAACTAGCAGAGATGTGGTTGGAGAAGAACGACCCATCTTATAG
CCTGTTTGCAGCATGGAAGCATGGACTTTCGGGCATTTGGCTCGCATTCGAGTGCTTATACCAGATT
ACTTATTTCTTCCTGGTGATTTACCTATTCTCCCGTTTGGCTGTTGAGGCTGACCCACGGGCGCAAA
CGGCCACCGTCATTGTTTCGACCACAGTTGCGCTTATTAAGTGCGGTTACTTTAGCTTCAGAGCTAA
AGACATTAGGGCCTTCTATTTCGTCCTCTACACGTTCGTTTACTTCTTCTGCATGATTCCGGCACGA
ATAACTGCGATGATGACCCTGTGGGACATAGGTTGGGGAACCAGGGGAGGAAATGAGAAGCCTTC
CGTAGGCACCAGAGTTGCTTTGTGGGCCAAGCAATATTTGATTGCTTACATGTGGTGGGCCGCTGT
CGTCGGTGCTGGCGTGTACTCCATCGTTCATAATTGGATGTTTGACTGGAATTCACTTTCCTACAGG
TTCGCACTGGTAGGCATCTGTTCGTATATAGTTTTCATTGTAATAGTGCTGGTAGTCTACTTTACAG
GAAAGATCACGACCTGGAACTTCACGAAGCTACAGAAAGAATTGATCGAGGACCGCGTACTGTAC
GACGCAACGACCAATGCCCAGTCGGTATAA

SEQ ID NO: 16: is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a chondroitin sulfate synthase from Homo sapiens (HCOS11-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAAGAGATAGAAACTT
CCTATTCGTTGGTGTTATGACAGCTCAGAAGTACTTACAGACCAGGGCGGTTGCAGCATATAGAAC
CTGGTCAAAGACAATTCCAGGTAAGGTACAGTTCTTCAGCTCCGAGGGTTCTGACACCAGCGTCCC
TATCCCAGTAGTGCCTTTGCGTGGAGTGGATGATTCATACCCACCTCAGAAGAAGTCCTTCATGAT
GCTTAAGTATATGCACGACCACTATCTGGACAAATACGAGTGGTTCATGAGAGCAGACGACGACGT
TTACATAAAGGGTGATAGACTGGAGAATTTCTTGAGGTCACTAAACAGTTCAGAACCACTTTTCCT
AGGACAGACGGGCCTTGGTACGACAGAGGAGATGGGTAAGTTGGCATTGGAGCCAGGGGAGAACT
TCTGCATGGGTGGCCCTGGTGTGATTATGAGTAGGGAAGTTTTAAGAAGAATGGTTCCGCACATTG
GTAAGTGTCTAAGGGAAATGTATACGACTCATGAGGACGTGGAAGTAGGAAGATGCGTTAGGAGA
TTCGCTGGAGTACAGTGTGTTTGGTCTTACGAGATGCAACAGTTATTCTACGAGAACTACGAGCAG
AATAAGAAGGGATACATCCGTGATCTGCACAACTCCAAAATTCACCAAGCAATAACGCTGCATCCT

AACAAGAATCCGCCCTATCAGTATAGGTTGCACTCTTACATGTTGCTGTTTGCAGCATGGAAGCAT
GGACTTTCGGGCATTTGGCTCGCATTCGAGTGCTTATACCAGATTACTTATTTCTTCCTGGTGATTTA
CCTATTCTCCCGTTTGGCTGTTGAGGCTGACCCACGGGCGCAAACGGCCACCGTCATTGTTTCGACC
ACAGTTGCGCTTATTAAGTGCGGTTACTTTAGCTTCAGAGCTAAAGACATTAGGGCCTTCTATTTCG
TCCTCTACACGTTCGTTTACTTCTTCTGCATGATTCCGGCACGAATAACTGCGATGATGACCCTGTG
GGACATAGGTTGGGGAACCAGGGGAGGAAATGAGAAGCCTTCCGTAGGCACCAGAGTTGCTTTGT
GGGCCAAGCAATATTTGATTGCTTACATGTGGTGGGCCGCTGTCGTCGGTGCTGGCGTGTACTCCAT
CGTTCATAATTGGATGTTTGACTGGAATTCACTTTCCTACAGGTTCGCACTGGTAGGCATCTGTTCG
TATATAGTTTTCATTGTAATAGTGCTGGTAGTCTACTTTACAGGAAAGATCACGACCTGGAACTTCA
CGAAGCTACAGAAAGAATTGATCGAGGACCGCGTACTGTACGACGCAACGACCAATGCCCAGTCG
GTATAA

SEQ ID NO: 17: is a reencoded nucleic acid sequence of a polypeptide having chondroitin
synthase activity: chimeric nucleic acid comprising a fragment from a nucleic acid encoding a
hyaluronan synthase originating from Chlorella virus PBCV-1 and a fragment from a nucleic
acid encoding a chondroitin sulfate synthase from Homo sapiens (HCOS12-Vir)
ATGGGTAAGAACATCATTATTATGGTTTCTTGGTACACTATTATTACATCCAATCTCATCGCAGTGG
GTGGCGCCTCACTCATACTAGCCCCAGCTATTACGGGCTATGTCCTTCACTGGAACATTGCCCTTTC
AACAATTTGGGGAGTGTCGGCCTACGGAATTTTCGTGTTTGGTTTCTTTCTTGCCCAGGTATTATTT
AGTGAACTCAACCGGAAAAGGCTCCGGAAGTGGATTTCCCTCCGACCCAAAAAGGATAAGAAAAT
CAACATTCTTATACCTCTTTCCGGCAGATTCGACATGTTCGTTAGGTTCATGGGTAACTTCGAGAAG
ACCTGCTTAATTCCTAACCAGAATGTTAAGTTGGTAGTTTTGTTATTCAATTCTGACAGTAACCCCG
ACAAAGCAAAGCAGGTTGAACTGATGAGAGACTATAGGATTAAGTACCCAAAAGCAGATATGCAG
ATACTACCTGTTTCCGGAGAATTCTCAAGAGCCTTGGCTCTGGAAGTAGGCAGCTCTCAGTTTAATA
ATGAGTCTCTTCTGTTCTTCTGCGACGTTGACTTAGTTTTCACGACAGAGTTTCTGCAGAGATGTCG
TGCAAACACTGTACTAGGTCAGCAGATATATTTCCCCATTATTTTCAGTCAGTACGATCCAAAGATT
GTTTATAGTGGAAAAGTTCCATCTGACAACCATTTTGCGTTTACTCAAAAGACAGGGTTTTGGCGTA
ACTACGGGTTCGGCATAACATGTATCTATAAGGGGACCTGGTAAGAGTTGGCGGATTCGACGTAT
CTATACAGGGCTGGGGTTTAGAAGACGTCGATCTATTTAATAAAGTAGTTCAGGCAGGTTTGAAGA
CATTCCGTAGTCAAGAGGTAGGCGTTGTACACGTGCACCATCCAGTATTTTGCGACCCAAACTTAG
ACCCGAAGCAATATAAAATGTGCTTGGGTTCCAAGGCATCGACTTACGGTTCCACGCAGCAACTAG
CAGAGATGTGGTTGGAGAAGAACGACCCATCTTATAGCCTGTTTGCAGCATGGAAGCATGGACTTT
CGGGCATTTGGCTCGCATTCGAGTGCTTATACCAGATTACTTATTTCTTCCTGGTGATTTACCTATTC
TCCCGTTTGGCTGTTGAGGCTGACCCACGGGCGCAAACGGCCACCGTCATTGTTTCGACCACAGTT
GCGCTTATTAAGTGCGGTTACTTTAGCTTCAGAGCTAAAGACATTAGGGCCTTCTATTTCGTCCTCT
ACACGTTCGTTTACTTCTTCTGCATGATTCCGGCACGAATAACTGCGATGATGACCCTGTGGGACAT
AGGTTGGGGAACCAGGGGAGGAAATGAGAAGCCTTCCGTAGGCACCAGAGTTGCTTTGTGGGCCA

AGCAATATTTGATTGCTTACATGTGGTGGGCCGCTGTCGTCGGTGCTGGCGTGTACTCCATCGTTCA
TAATTGGATGTTTGACTGGAATTCACTTTCCTACAGGTTCGCACTGGTAGGCATCTGTTCGTATATA
GTTTTCATTGTAATAGTGCTGGTAGTCTACTTTACAGGAAAGATCACGACCTGGAACTTCACGAAG
CTACAGAAAGAATTGATCGAGGACCGCGTACTGTACGACGCAACGACCAATGCCCAGTCGGTATA
A

SEQ ID NO: 18 is an amino acid sequence of a polypeptide having a chondroitin synthase
activity : chimeric amino acid comprising a fragment from an amino acid encoding a
hyaluronan synthase originating from Pasteurella multocida and a fragment from an amino
acid encoding a chondroitin synthase from Pasteurella multocida (HCOS1-1)
MNTLSQAIKAYNSNDYQLALKLFEKSAEIYGRKIVEFQITKCKEKLSAHPSVNSAHLSVNKEEKVNVCD
SPLDIATQLLLSNVKKLVLSDSEKNTLKNKWKLLTEKKSENAEVRAVALVPKDFPKDLVLAPLPDHVN
DFTWYKKRKKRLGIKPEHQHVGLSIIVTTFNRPAILSITLACLVNQKTHYPFEVIVTDDGSQEDLSPIIRQY
ENKLDIRYVRQKDYGYQLCAVRNLGLRTAKYDFVSILDCDMAPQQLWVHSYLTELLEDDDLTIIGPRK
YIDTQHIDPKDFLNNASLLESLPEVKTNNSVAAKGEGTVSLDWRLEQFEKTENLRLSDSPFRFFAAGNV
AFAKKWLNKSGFFDEEFNHWGGEDVEFGYRLFRYGSFFKTIDGIMAYHQEPPGKENETDREAGKNITL
DIMREKVPYIYRKLLPIEDSHINRVPLVSIYIPAYNCANYIQRCVDSALNQTVVDLEVCICNDGSTDNTLE
VINKLYGNNPRVRIMSKPNGGIASASNAAVSFAKGYYIGQLDSDDYLEPDAVELCLKEFLKDKTLACVY
TTNRNVNPDGSLIANGYNWPEFSREKLTTAMIAHHFRMFTIRAWHLTDGFNEKIENAVDYDMFLKLSE
VGKFKHLNKICYNRVLHGDNTSIKKLGIQKKNHFVVVNQSLNRQGITYYNYDEFDDLDESRKYIFNKT
AEYQEEIDILKDIKIIQNKDAKIAVSIFYPNTLNGLVKKLNNIIEYNKNIFVIVLHVDKNHLTPDIKKEILAF
YHKHQVNILLNNDISYYTSNRLIKTEAHLSNINKLSQLNLNCEYIIFDNHDSLFVKNDSYAYMKKYDVG
MNFSALTHDWIEKINAHPPFKKLIKTYFNDNDLKSMNVKGASQGMFMTYALAHELLTIIKEVITSCQSID
SVPEYNTEDIWFQFALLILEKKTGHVFNKTSTLTYMPWERKLQWTNEQIESAKRGENIPVNKFIINSITL

SEQ ID NO: 19 is an amino acid sequence of a polypeptide having a chondroitin synthase
activity : chimeric amino acid comprising a fragment from an amino acid encoding a
hyaluronan synthase originating from Pasteurella multocida and a fragment from an amino
acid encoding a chondroitin synthase from Pasteurella multocida (HCOS1-2)
MNTLSQAIKAYNSNDYQLALKLFEKSAEIYGRKIVEFQITKCKEKLSAHPSVNSAHLSVNKEEKVNVCD
SPLDIATQLLLSNVKKLVLSDSEKNTLKNKWKLLTEKKSENAEVRAVALVPKDFPKDLVLAPLPDHVN
DFTWYKKRKKRLGIKPVNKNIGLSIIIPTFNRSRILDITLACLVNQKTHYPFEVIVTDDGSQEDLSPIIRQYE
NKLDIRYVRQKDYGYQLCAVRNLGLRTAKYDFVSILDCDMAPQQLWVHSYLTELLEDDDLTIIGPRKY
IDTQHIDPKDFLNNASLLESLPEVKTNNSVAAKGEGTVSLDWRLEQFEKTENLRLSDSPFRFFSCGNVAF
SKEWLNKVGWFDEEFNHWGGEDVEFGYRLFRYGSFFKTIDGIMAYHQEPPGKENETDREAGKNITLDI
MREKVPYIYRKLLPIEDSHINRVPLVSIYIPAYNCANYIQRCVDSALNQTVVDLEVCICNDGSTDNTLEVI

NKLYGNNPRVRIMSKPNGGIASASNAAVSFAKGYYIGQLDSDDYLEPDAVELCLKEFLKDKTLACVYT
TNRNVNPDGSLIANGYNWPEFSREKLTTAMIAHHFRMFTIRAWHLTDGFNEKIENAVDYDMFLKLSEV
GKFKHLNKICYNRVLHGDNTSIKKLGIQKKNHFVVVNQSLNRQGITYYNYDEFDDLDESRKYIFNKTAE
YQEEIDILKDIKIIQNKDAKIAVSIFYPNTLNGLVKKLNNIIEYNKNIFVIVLHVDKNHLTPDIKKEILAFYH
KHQVNILLNNDISYYTSNRLIKTEAHLSNINKLSQLNLNCEYIIFDNHDSLFVKNDSYAYMKKYDVGMN
FSALTHDWIEKINAHPPFKKLIKTYFNDNDLKSMNVKGASQGMFMTYALAHELLTIIKEVITSCQSIDSV
PEYNTEDIWFQFALLILEKKTGHVFNKTSTLTYMPWERKLQWTNEQIESAKRGENIPVNKFIINSITL

SEQ ID NO: 20 is an amino acid sequence of a polypeptide having a chondroitin synthase
activity : chimeric amino acid comprising a fragment from an amino acid encoding a
hyaluronan synthase originating from Pasteurella multocida and a fragment from an amino
acid encoding a chondroitin synthase from Pasteurella multocida (HCOS1-3)
MNTLSQAIKAYNSNDYQLALKLFEKSAEIYGRKIVEFQITKCKEKLSAHPSVNSAHLSVNKEEKVNVCD
SPLDIATQLLLSNVKKLVLSDSEKNTLKNKWKLLTEKKSENAEVRAVALVPKDFPKDLVLAPLPDHVN
DFTWYKNRKKSLGIKPVNKNIGLSIIIPTFNRSRILDITLACLVNQKTNYPFEVVVADDGSKENLLTIVQK
YEQKLDIKYVRQKDYGYQLCAVRNLGLRTAKYDFVSILDCDMAPQQLWVHSYLTELLEDNDIVLIGPR
KYVDTHNITAEQFLNDPYLIESLPETATNNNPSITSKGNISLDWRLEHFKKTDNLRLCDSPFRYFSCGNV
AFSKEWLNKVGWFDEEFNHWGGEDVEFGYRLFAKGCFFRVIDGGMAYHQEPPGKENETDREAGKSIT
LKIVKEKVPYIYRKLLPIEDSHINRVPLVSIYIPAYNCANYIQRCVDSALNQTVVDLEVCICNDGSTDNTL
EVINKLYGNNPRVRIMSKPNGGIASASNAAVSFAKGYYIGQLDSDDYLEPDAVELCLKEFLKDKTLACV
YTTNRNVNPDGSLIANGYNWPEFSREKLTTAMIAHHFRMFTIRAWHLTDGFNEKIENAVDYDMFLKLS
EVGKFKHLNKICYNRVLHGDNTSIKKLGIQKKNHFVVVNQSLNRQGITYYNYDEFDDLDESRKYIFNKT
AEYQEEIDILKDIKIIQNKDAKIAVSIFYPNTLNGLVKKLNNIIEYNKNIFVIVLHVDKNHLTPDIKKEILAF
YHKHQVNILLNNDISYYTSNRLIKTEAHLSNINKLSQLNLNCEYIIFDNHDSLFVKNDSYAYMKKYDVG
MNFSALTHDWIEKINAHPPFKKLIKTYFNDNDLKSMNVKGASQGMFMTYALAHELLTIIKEVITSCQSID
SVPEYNTEDIWFQFALLILEKKTGHVFNKTSTLTYMPWERKLQWTNEQIESAKRGENIPVNKFIINSITL

SEQ ID NO: 21: is an amino acid sequence of a polypeptide having chondroitin synthase
activity: chimeric amino acid comprising a fragment from an amino acid encoding a
hyaluronan synthase originating from Pasteurella multocida, a fragment from an amino acid
encoding a chdroitin synthase from Pasteurella multocida and a fragment from an amino acid
encoding a chitin synthase 2 from Saccharomyces cerevisiae (HCOS.Sc)
MNTLSQAIKAYNSNDYQLALKLFEKSAEIYGRKIVEFQITKCKEKLSAHPSVNSAHLSVNKEEKVNVCD
SPLDIATQLLLSNVKKLVLSDSEKNTLKNKWKLLTEKKSENAEVRAVALVPKDFPKDLVLAPLPDHVN
DFTWYKNRKKSLGIKPVNKNIGLSIIIPTFNRSRILDITLACLVNQKTNYPFEVVVADDGSKENLLTIVQK
YEQKLDIKYVRQKDYGYQLCAVRNLGLRTAKYDFVSILDCDMAPQQLWVHSYLTELLEDNDIVLIGPR

KYVDTHNITAEQFLNDPYLIESLPETATNNNPSITSKGNISLDWRLEHFKKTDNLRLCDSPFRYFSCGNV
AFSKEWLNKVGWFDEEFNHWGGEDVEFGYRLFAKGCFFRVIDGGMAYHQEPPGKENETDREAGKSIT
LKIVKEKVPYIYRKLLPIEDSHINRVPLVSIYIPAYNCANYIQRCVDSALNQTVVDLEVCICNDGSTDNTL
EVINKLYGNNPRVRIMSKPNGGIASASNAAVSFAKGYYIGQLDSDDYLEPDAVELCLKEFLKDKTLACV
YTTNRNVNPDGSLIANGYNWPEFSREKLTTAMIAHHFRMFTIRAWHLTDGFNEKIENAVDYDMFLKLS
EVGKFKHLNKICYNRVLHGDNTSIKKLGIQKKNHFVVVNQSLNRQGITYYNYDEFDDLDESRKYIFNKT
AEYQEEIDILKDIFIQMLFSWFSIANFVLTFYYLAGSMNLVIKHGEALFIFFKYLIFCDLASLFIISMGNRPQ
GAKHLFITSMVILSICATYSLICGFVFAFKSLASGTESHKIFVDIVISLLSTYGLYFFSSLMYLDPWHMFTS
SIQYFLTLPAFTCTLQIFAFCNTHDVSWGTKGSTQESKQLSKAIVVQGPDGKQIVETDWPQEVDKKFLEI
KSRLKEPEFEESSGNEKQSKNDYYRDIRTRIVMIWMLSNLILIMSIIQVFTPQDTDNGYLIFILWSVAALA
AFRVVGSMAFLFMKYLRIIVSYRNKVEGSGSWEVSKLDLPNVFHKKG

SEQ ID NO: 22: is an amino acid sequence of a polypeptide having chondroitin synthase
activity: chimeric amino acid comprising a fragment from an amino acid encoding a
hyaluronan synthase originating from Pasteurella multocida and a fragment from an amino
acid encoding a chitin synthase 2 from Saccharomyces cerevisiae (HHASA.Sc)
MNTLSQAIKAYNSNDYQLALKLFEKSAEIYGRKIVEFQITKCKEKLSAHPSVNSAHLSVNKEEKVNVCD
SPLDIATQLLLSNVKKLVLSDSEKNTLKNKWKLLTEKKSENAEVRAVALVPKDFPKDLVLAPLPDHVN
DFTWYKKRKKRLGIKPEHQHVGLSIIVTTFNRPAILSITLACLVNQKTHYPFEVIVTDDGSQEDLSPIIRQY
ENKLDIRYVRQKDNGFQASAARNMGLRLAKYDFIGLLDCDMAPNPLWVHSYVAELLEDDDLTIIGPRK
YIDTQHIDPKDFLNNASLLESLPEVKTNNSVAAKGEGTVSLDWRLEQFEKTENLRLSDSPFRFFAAGNV
AFAKKWLNKSGFFDEEFNHWGGEDVEFGYRLFRYGSFFKTIDGIMAYHQEPPGKENETDREAGKNITL
DIMREKVPYIYRKLLPIEDSHINRVPLVSIYIPAYNCANYIQRCVDSALNQTVVDLEVCICNDGSTDNTLE
VINKLYGNNPRVRIMSKPNGGIASASNAAVSFAKGYYIGQLDSDDYLEPDAVELCLKEFLKDKTLACVY
TTNRNVNPDGSLIANGYNWPEFSREKLTTAMIAHHFRMFTIRAWHLTDGFNEKIENAVDYDMFLKLSE
VGKFKHLNKICYNRVLHGDNTSIKKLGIQKKNHFVVVNQSLNRQGITYYNYDEFDDLDESRKYIFNKT
AEYQEEIDILKDIFIQMLFSWFSIANFVLTFYYLAGSMNLVIKHGEALFIFFKYLIFCDLASLFIISMGNRPQ
GAKHLFITSMVILSICATYSLICGFVFAFKSLASGTESHKIFVDIVISLLSTYGLYFFSSLMYLDPWHMFTS
SIQYFLTLPAFTCTLQIFAFCNTHDVSWGTKGSTQESKQLSKAIVVQGPDGKQIVETDWPQEVDKKFLEI
KSRLKEPEFEESSGNEKQSKNDYYRDIRTRIVMIWMLSNLILIMSIIQVFTPQDTDNGYLIFILWSVAALA
AFRVVGSMAFLFMKYLRIIVSYRNKVEGSGSWEVSKLDLPNVFHKKG

SEQ ID NO: 23: is an amino acid sequence of a polypeptide having chondroitin synthase
activity: chimeric amino acid comprising a fragment from an amino acid encoding a
hyaluronan synthase originating from Chlorella virus PBCV-1 and a fragment from an amino
acid encoding a chondroitin synthase from Escherichia coli (HCOS1-Vir)

MGKNIIIMVSWYTIITSNLIAVGGASLILAPAITGYVLHWNIALSTIWGVSAYGIFVFGFFLAQVLFSELNR
KRLRKWISLRPKGWNDVRLAVIIAGYREDPYMFQKCLESVRDSDYGNVARLICVIDGDEDDDMRMAA
VYKAIYNDNIKKPEFVLCESDDKEGERIDSDFSRDICVLQPHRGKRECLYTGFQLAKMDPSVNAVVLIDS
DTVLEKDAILEVVYPLACDPEIQAVAGECKIWNTDTLLSLLVAWRYYSAFCVERSAQSFFRTVQCVGGP
LGAYKIDIIKEIKDPWISQRFLGQKCTYGDDRRLTNEILMRGKKVVFTPFAVGWGWFDEEFTHWGGRYI
VQQTRWSKSWCREIWYTLFAAWKHGLSGIWLAFECLYQITYFFLVIYLFSRLAVEADPRAQTATVIVST
TVALIKCGYFSFRAKDIRAFYFVLYTFVYFFCMIPARITAMMTLWDIGWGTRGGNEKPSVGTRVALWA
KQYLIAYMWWAAVVGAGVYSIVHNWMFDWNSLSYRFALVGICSYIVFIVIVLVVYFTGKITTWNFTKL
QKELIEDRVLYDATTNAQSV

SEQ ID NO: 24: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin synthase from Escherichia coli (HCOS2-Vir)
MGKNIIIMVSWYTIITSNLIAVGGASLILAPAITGYVLHWNIALSTIWGVSAYGIFVFGFFLAQVLFSELNR
KRLRKWISLRPKGWNDVRLAVIIAGYREDPYMFQKCLESVRDSDYGNVARLICVIDGDEDDDMRMAA
VYKAIYNDNIKKPEFVLCESDDKEGERIDSDFSRDICVLQPHRGKRECLYTGFQLAKMDPSVNAVVLIDS
DTVLEKDAILEVVYPLACDPEIQAVAGECKIWNTDTLLSLLVAWRYYSAFCVERSAQSFFRTVQCVGGP
LGAYKIDIIKEIKDPWISQRFLGQKCTYGDDRRLTNEILMRGKKVVFTPFAVGWLHGENTSIKKLRYIVQ
QTRWSKSWCREIWYTLFAAWKHGLSGIWLAFECLYQITYFFLVIYLFSRLAVEADPRAQTATVIVSTTV
ALIKCGYFSFRAKDIRAFYFVLYTFVYFFCMIPARITAMMTLWDIGWGTRGGNEKPSVGTRVALWAKQ
YLIAYMWWAAVVGAGVYSIVHNWMFDWNSLSYRFALVGICSYIVFIVIVLVVYFTGKITTWNFTKLQK
ELIEDRVLYDATTNAQSV

SEQ ID NO: 25: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a Galactofuranosyltransferase from Mycobacterium tuberculosis (HCOS3-Vir)
MGKNIIIMVSWYTIITSNLIAVGGASLILAPAITGYVLHWNIALSTIWGVSAYGIFVFGFFLAQVLFSELNR
KRLRKWISLRPKGWNDVRLAVIIAGYREDPYMFQKCLESVRDSDYGNVARLICVIDGDEDDDMRMAA
VYKAIYNDNIKKPEFVLCESDDKEGERIDSDFSRDICVLQPHRGKRECLYTGFQLAKMDPSVNAVVLIDS
DTVLEKDAILEVVYPLACDPEIQAVAGECKIWNTDTLLSLLVAWRYYSAFCVERSAQSFFRTVQCVGGP
LGAYKIDIIKEIKDPWISQRFLGQKCTYGDDRRLTNEILMRGKKVVFTPFAVGWHMAWSDKDDAIDRYI
VQQTRWSKSWCREIWYTLFAAWKHGLSGIWLAFECLYQITYFFLVIYLFSRLAVEADPRAQTATVIVST
TVALIKCGYFSFRAKDIRAFYFVLYTFVYFFCMIPARITAMMTLWDIGWGTRGGNEKPSVGTRVALWA

KQYLIAYMWWAAVVGAGVYSIVHNWMFDWNSLSYRFALVGICSYIVFIVIVLVVYFTGKITTWNFTKL
QKELIEDRVLYDATTNAQSV

SEQ ID NO: 26: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin synthase from Escherichia coli (HCOS4-Vir)
MGKNIIIMVSWYTIITSNLIAVGGASLILAPAITGYVLHWNIALSTIWGVSAYGIFVFGFFLAQVLFSELNR
KRLRKWISLRPKGWNDVRLAVIIAGYREDPYMFQKCLESVRDSDYGNVARLICVIDGDEDDDMRMAA
VYKAIYNDNIKKPEFVLCESDDKEGERIDSDFSRDICVLQPHRGKRECLYTGFQLAKMDPSVNAVVLIDS
DTVLEKDAILEVVYPLACDPEIQAVAGECKIWNTDTLLSLLVAWRYYSAFCVERSAQSFFRTVQCVGGP
LGAYKIDIIKEIKDPWISQRFLGQKCTYGDDRRLTNEILMRGKKVVFTPFAVGWFDEEFTHWGGRYIVQ
QTRWSKSWCREIWYTLFAAWKHGLSGIWLAFECLYQITYFFLVIYLFSRLAVEADPRAQTATVIVSTTV
ALIKCGYFSFRAKDIRAFYFVLYTFVYFFCMIPARITAMMTLWDIGWGTRGGNEKPSVGTRVALWAKQ
YLIAYMWWAAVVGAGVYSIVHNWMFDWNSLSYRFALVGICSYIVFIVIVLVVYFTGKITTWNFTKLQK
ELIEDRVLYDATTNAQSV

SEQ ID NO: 27: is an amino acid sequence of a polypeptide having chondroitin synthase
activity: chimeric amino acid comprising a fragment from an amino acid encoding a
hyaluronan synthase originating from Chlorella virus PBCV-1 and a fragment from an amino
acid encoding a chondroitin synthase from Penicillium oxalicum (HCOS5-Vir)
MGKNIIIMVSWYTIITSNLIAVGGASLILAPAITGYVLHWNIALSTIWGVSAYGIFVFGFFLAQVLFSELNR
KRLRKWISLRPKSIHHPISKTTHPDTENAGDSTLLCPPMDGMDDILVVMKTGVTEAKEKVPVHFQTTLR
CVPHYVIYSDFEEEIEGVKIYDVLQGMDPDVMTQVPDFDLYNRIKKLGRQGLAENDFADEANSAVGKP
NNPGWKLDKWKFLPMIIEALRYKPKAKWYVFMEADTYISWPTLLAWLKHFNHKKPWYLGTETQIAD
VIFAHGGSGFMISHAALKLAAEEYTARRTELHHYTDGHWAGDCVLGKVLADAGVNLHFSWPILQNSN
LGELDEFNVDLYRQPWCFPAAAFHHLNAQKIQDLWSYEQKRWQDKDKRVLLHSDVFRDLLYPDIALK
PARIGWDNLSADEQSTASSFEECRQICDQLDDCTQFSFRDKSCFTSRTPRLGAANPNTVSGWMVDRIRA
NMEKSGLFAAWKHGLSGIWLAFECLYQITYFFLVIYLFSRLAVEADPRAQTATVIVSTTVALIKCGYFSF
RAKDIRAFYFVLYTFVYFFCMIPARITAMMTLWDIGWGTRGGNEKPSVGTRVALWAKQYLIAYMWW
AAVVGAGVYSIVHNWMFDWNSLSYRFALVGICSYIVFIVIVLVVYFTGKITTWNFTKLQKELIEDRVLY
DATTNAQSV

SEQ ID NO: 28: is an amino acid sequence of a polypeptide having chondroitin synthase
activity: chimeric amino acid comprising a fragment from an amino acid encoding a
hyaluronan synthase originating from Chlorella virus PBCV-1 and a fragment from an amino
acid encoding a chondroitin synthase from Pasteurella multocida (HCOS6-Vir)
MGKNIIIMVSWYTIITSNLIAVGGASLILAPAITGYVLHWNIALSTIWGVSAYGIFVFGFFLAQVLFSELNR
KRLRKWISLRPKLDIATQLLLSNVKKLTLSESEKNSLKNKWKSITGKKSENAEIRKVELVPKDFPKDLVL
APLPDHVNDFTWYKNRKKSLGIKPVNKNIGLSIIIPTFNRSRILDITLACLVNQKTNYPFEVVVADDGSKE
NLLTIVQKYEQKLDIKYVRQKDYGYQLCAVRNLGLRTAKYDFVSILDCDMAPQQLWVHSYLTELLED
NDIVLIGPRKYVDTHNITAEQFLNDPYLIESLPETATNNNPSITSKGNISLDWRLEHFKKTDNLRLCDSPF
RYFSCGNVAFSKEWLNKVGWFDEEFNHWGGEDVEFGYRLFAKGCFFRVIDGGMAYHQEPPGKENETD
REAGKSITLKIVKEKVPYIYRKLLPIEDSHIHRIPLVSIYIPAYNCANYIQRCVDSALNQTVVDLEVCICND
GSTDNTLEVINKLYGNNPRVRIMSKPNGGIASASNAAVSFAKGYYIGQLDSDDYLEPDAVELCLKEFLK
DKTLACVYTTNRNVNPDGSLIANGYNWPEFSREKLTTAMIAHHFRMFTIRAWHLTDGFNEKIENAVDY
DMFLKLSEVGKFKHLNKICYNRVLHGDNTSIKNLDTQKKNHFVVVNQSLNRQRVSNYNYDEFDNLDE
SRKYIFNKLFAAWKHGLSGIWLAFECLYQITYFFLVIYLFSRLAVEADPRAQTATVIVSTTVALIKCGYFS
FRAKDIRAFYFVLYTFVYFFCMIPARITAMMTLWDIGWGTRGGNEKPSVGTRVALWAKQYLIAYMWW
AAVVGAGVYSIVHNWMFDWNSLSYRFALVGICSYIVFIVIVLVVYFTGKITTWNFTKLQKELIEDRVLY
DATTNAQSV

SEQ ID NO: 29: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin synthase from Pasteurella multocida (HCOS7-Vir)
MGKNIIIMVSWYTIITSNLIAVGGASLILAPAITGYVLHWNIALSTIWGVSAYGIFVFGFFLAQVLFSELNR
KRLRKWISLRPKIPTFNRSRILDITLACLVNQKTNYPFEVVVADDGSKENLLTIVQKYEQKLDIKYVRQK
DYGYQLCAVRNLGLRTAKYDFVSILDCDMAPQQLWVHSYLTELLEDNDIVLIGPRKYVDTHNITAEQF
LNDPYLIESLPETATNNNPSITSKGNISLDWRLEHFKKTDNLRLCDSPFRYFSCGNVAFSKEWLNKVGWF
DEEFNHWGGEDVEFGYRLFAKGCFFRVIDGGMAYHQEPPGKENETDREAGKSITLKIVKEKVPYIYRKL
LPIEDSHIHRIPLVSIYIPAYNCANYIQRCVDSALNQTVVDLEVCICNDGSTDNTLEVINKLYGNNPRVRI
MSKPNGGIASASNAAVSFAKGYYIGQLDSDDYLEPDAVELCLKEFLKDKTLACVYTTNRNVNPDGSLI
ANGYNWPLFAAWKHGLSGIWLAFECLYQITYFFLVIYLFSRLAVEADPRAQTATVIVSTTVALIKCGYF
SFRAKDIRAFYFVLYTFVYFFCMIPARITAMMTLWDIGWGTRGGNEKPSVGTRVALWAKQYLIAYMW
WAAVVGAGVYSIVHNWMFDWNSLSYRFALVGICSYIVFIVIVLVVYFTGKITTWNFTKLQKELIEDRVL
YDATTNAQSV

SEQ ID NO: 30: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin synthase from Pasteurella multocida (HCOS8-Vir)
MGKNIIIMVSWYTIITSNLIAVGGASLILAPAITGYVLHWNIALSTIWGVSAYGIFVFGFFLAQVLFSELNR
KRLRKWISLRPKGWNDVRLAVIIAGYREDPYMFQKCLESVRDSDYGNVARLICVIDGDEDDDMRMAA
VYKAIYNDNIKKPEFVLCESDDKEGERIDSDFSRDICVLQPHRGKRECLYTGFQLAKMDPSVNAVVLIDS
DTVLEKDAILEVVYPLACDPEIQAVAGECKIWNTDTLLSLLVAWRYYSAFCVERSAQSFFRTVQCVRLC
DSPFRYFSCGNVAFSKEWLNKVGWFDEEFNHWGGEDVEFGYRLKKVVFTPFAVGWSDSPTNVFRYIV
QQTRWSKSWCREIWYTLFAAWKHGLSGIWLAFECLYQITYFFLVIYLFSRLAVEADPRAQTATVIVSTT
VALIKCGYFSFRAKDIRAFYFVLYTFVYFFCMIPARITAMMTLWDIGWGTRGGNEKPSVGTRVALWAK
QYLIAYMWWAAVVGAGVYSIVHNWMFDWNSLSYRFALVGICSYIVFIVIVLVVYFTGKITTWNFTKLQ
KELIEDRVLYDATTNAQSV

SEQ ID NO: 31: is an amino acid sequence of a polypeptide having chondroitin synthase
activity: chimeric amino acid comprising a fragment from an amino acid encoding a
hyaluronan synthase originating from Chlorella virus PBCV-1 and a fragment from an amino
acid encoding a chondroitin sulfate synthase from Homo sapiens (HCOS9-Vir)
MGKNIIIMVSWYTIITSNLIAVGGASLILAPAITGYVLHWNIALSTIWGVSAYGIFVFGFFLAQVLFSELNR
KRLRKWISLRPKHIGKCLREMYTTHEDVEVGRCVRRFAGVQCVWSYEMQQLFYENYEQNKKGYIRDL
HNSKIHQAITLHPNKNPPYQYRLHSYMLSRKISELRHRTIQLHREIVLMSKYSNTEIHKEDLQLGIPPSFM
RFQPRQREEILEWEFLTGKYLYSAVDGQPPRRGMDSAQREALDDIVMQVMEMINANAKTRGRIIDFKEI
QYGYRRVNPMYGAEYILDLLLLYKKHKGKKMTVPVRRHAYLQQTFSKIQFVEHEELDAQELAKRINQ
ESGSLSFLSNSLKKLVPFQLPGSKSEHKEPKDKKINILIPLSGRFDMFVRFMGNFEKTCLIPNQNVKLVVL
LFNSDSNPDKAKQVELMRDYRIKYPKADMQILPVSGEFSRALALEVGSSQFNNESLLFFCDVDLVFTTE
FLQRCRANTVLGQQIYFPIIFSQYDPKIVYSGKVPSDNHFAFTQKTGFWRNYGFGITCIYKGDLVRVGGF
DVSIQGWGLEDVDLFNKVVQAGLKTFRSQEVGVVHVHHPVFCDPNLDPKQYKMCLGSKASTYGSTQQ
LLFAAWKHGLSGIWLAFECLYQITYFFLVIYLFSRLAVEADPRAQTATVIVSTTVALIKCGYFSFRAKDIR
AFYFVLYTFVYFFCMIPARITAMMTLWDIGWGTRGGNEKPSVGTRVALWAKQYLIAYMWWAAVVGA
GVYSIVHNWMFDWNSLSYRFALVGICSYIVFIVIVLVVYFTGKITTWNFTKLQKELIEDRVLYDATTNA
QSV

SEQ ID NO: 32: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin sulfate synthase from Homo sapiens (HCOS10-Vir)

MGKNIIIMVSWYTIITSNLIAVGGASLILAPAITGYVLHWNIALSTIWGVSAYGIFVFGFFLAQVLFSELNR
KRLRKWISLRPKRDRNFLFVGVMTAQKYLQTRAVAAYRTWSKTIPGKVQFFSSEGSDTSVPIPVVPLRG
VDDSYPPQKKSFMMLKYMHDHYLDKYEWFMRADDDVYIKGDRLENFLRSLNSSEPLFLGQTGLGTTE
EMGKLALEPGENFCMGGPGVIMSREVLRRMVPHIGKCLREMYTTHEDVEVGRCVRRFAGVQCVWSYE
MQQLFYENYEQNKKGYIRDLHNSKIHQAITLHPNKNPPYQYRLHSYMLSRKISELRHRTIQLHREIVLMS
KYSNTEIHKEDLQLGIPPSFMRFQPRQREEILEWEFLTGKYLYSAVDGQPPRRGMDSAQREALDDIVMQ
VMEMINANAKTRGRIIDFKEIQYGYRRVNPMYGAEYILDLLLLYKKHKGKKMTVPVRRHAYLQQTFSK
IQFVEHEELDAQELAKRINQESGSLSFLSNSLKKLVPFQLPGSKSEHKEPKDKKINILIPLSGRFDMFVRF
MGNFEKTCLIPNQNVKLVVLLFNSDSNPDKAKQVELMRDYRIKYPKADMQILPVSGEFSRALALEVGSS
QFNNESLLFFCDVDLVFTTEFLQRCRANTVLGQQIYFPIIFSQYDPKIVYSGKVPSDNHFAFTQKTGFWR
NYGFGITCIYKGDLVRVGGFDVSIQGWGLEDVDLFNKVVQAGLKTFRSQEVGVVHVHHPVFCDPNLDP
KQYKMCLGSKASTYGSTQQLAEMWLEKNDPSYSLFAAWKHGLSGIWLAFECLYQITYFFLVIYLFSRL
AVEADPRAQTATVIVSTTVALIKCGYFSFRAKDIRAFYFVLYTFVYFFCMIPARITAMMTLWDIGWGTR
GGNEKPSVGTRVALWAKQYLIAYMWWAAVVGAGVYSIVHNWMFDWNSLSYRFALVGICSYIVFIVIV
LVVYFTGKITTWNFTKLQKELIEDRVLYDATTNAQSV

SEQ ID NO: 33: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin sulfate synthase from Homo sapiens (HCOS11-Vir)
MGKNIIIMVSWYTIITSNLIAVGGASLILAPAITGYVLHWNIALSTIWGVSAYGIFVFGFFLAQVLFSELNR
KRLRKWISLRPKRDRNFLFVGVMTAQKYLQTRAVAAYRTWSKTIPGKVQFFSSEGSDTSVPIPVVPLRG
VDDSYPPQKKSFMMLKYMHDHYLDKYEWFMRADDDVYIKGDRLENFLRSLNSSEPLFLGQTGLGTTE
EMGKLALEPGENFCMGGPGVIMSREVLRRMVPHIGKCLREMYTTHEDVEVGRCVRRFAGVQCVWSYE
MQQLFYENYEQNKKGYIRDLHNSKIHQAITLHPNKNPPYQYRLHSYMLLFAAWKHGLSGIWLAFECLY
QITYFFLVIYLFSRLAVEADPRAQTATVIVSTTVALIKCGYFSFRAKDIRAFYFVLYTFVYFFCMIPARITA
MMTLWDIGWGTRGGNEKPSVGTRVALWAKQYLIAYMWWAAVVGAGVYSIVHNWMFDWNSLSYRF
ALVGICSYIVFIVIVLVVYFTGKITTWNFTKLQKELIEDRVLYDATTNAQSV

SEQ ID NO: 34: is a reencoded amino acid sequence of a polypeptide having chondroitin
synthase activity: chimeric amino acid comprising a fragment from an amino acid encoding a
hyaluronan synthase originating from Chlorella virus PBCV-1 and a fragment from an amino
acid encoding a chondroitin sulfate synthase from Homo sapiens (HCOS12-Vir)
MGKNIIIMVSWYTIITSNLIAVGGASLILAPAITGYVLHWNIALSTIWGVSAYGIFVFGFFLAQVLFSELNR
KRLRKWISLRPKKDKKINILIPLSGRFDMFVRFMGNFEKTCLIPNQNVKLVVLLFNSDSNPDKAKQVEL
MRDYRIKYPKADMQILPVSGEFSRALALEVGSSQFNNESLLFFCDVDLVFTTEFLQRCRANTVLGQQIYF

PIIFSQYDPKIVYSGKVPSDNHFAFTQKTGFWRNYGFGITCIYKGDLVRVGGFDVSIQGWGLEDVDLFN
KVVQAGLKTFRSQEVGVVHVHHPVFCDPNLDPKQYKMCLGSKASTYGSTQQLAEMWLEKNDPSYSLF
AAWKHGLSGIWLAFECLYQITYFFLVIYLFSRLAVEADPRAQTATVIVSTTVALIKCGYFSFRAKDIRAF
YFVLYTFVYFFCMIPARITAMMTLWDIGWGTRGGNEKPSVGTRVALWAKQYLIAYMWWAAVVGAG
VYSIVHNWMFDWNSLSYRFALVGICSYIVFIVIVLVVYFTGKITTWNFTKLQKELIEDRVLYDATTNAQS
V

SEQ ID NO: 35 is a nucleic acid sequence of UDP-Glucose dehydrogenase (HASB)
originating from Arabidopsis thaliana
ATGGTCAAAATATGCTGTATCGGGGCTGGATATGTCGGTGGGCCTACAATGGCCGTTATGGCCCTA
AAGTGTCCAGAGATTGAAGTCGTGGTAGTTGATATATCGGAGCCACGAATTAACGCATGGAACTCA
GACCGTCTACCAATTTATGAGCCAGGGTTAGAGGATGTCGTCAAACAGTGTAGAGGAAGAATTTG
TTCTTCTCTACAGATGTAGAGAAGCATGTATTTGAGTCAGATATAGTGTTTGTTTCGGTAAACACTC
CTACGAAAACGCAGGGTCTGGGTGCAGGTAAAGCAGCAGATTTGACATATTGGGAGTCCGCTGCTC
GCATGATAGCTGATGTGAGCAAATCGTCGAAAATCGTCGTTGAAAAGAGTACAGTACCAGTTCGTA
CAGCCGAGGCTATAGAAAAGATTTTAACGCACAATTCGAAGGGTATCGAATTCCAGATCTTATCAA
ATCCAGAATTCTTGGCCGAAGGGACGGCGATTAAAGACTTATATAACCCTGATAGAGTTCTAATCG
GTGGCAGGGACACCGCTGCGGACAAAAGGCCATTAAGGCGTTGCGTGACGTGTATGCCCACTGG
GTTCCTGTTGAGCAAATAATCTGTACTAATTTATGGAGTGCCGAGCTATCAAAGTTGGCTGCGAAC
GCATTTCTAGCTCAAAGGATAAGTTCAGTAAACGCAATGTCAGCGCTTTGTGAGGCAACTGGTGCT
GACGTAACCCAAGTCGCTCACGCCGTTGGAACCGACACTAGAATTGGACCGAAGTTTCTTAACGCA
TCCGTAGGCTTCGGCGGATCTTGCTTTCAGAAAGACATCCTGAATCTTATTTACATCTGCGAATGCA
ATGGTCTTCCAGAAGCAGCCAATTATTGGAAACAGGTAGTCAAGGTAAATGACTACCAAAAGATT
AGGTTTGCTAATCGAGTCGTATCTTCTATGTTCAACACCGTCTCCGGTAAGAAAATTGCTATTTTGG
GATTTGCGTTCAAGAAGGACACCGGCGACACGCGTGAAACTCCTGCCATAGATGTGTGTAATCGCC
TCGTGGCTGATAAAGCAAAGCTGTCGATCTATGATCCGCAAGTATTAGAAGAGCAGATCCGCCGTG
ATCTGTCCATGGCCCGATTCGATTGGGACCATCCAGTCCCACTCCAGCAGATCAAAGCTGAAGGTA
TCTCCGAACAGGTTAACGTTGTGTCCGACGCCTACGAGGCTACGAAGGATGCTCATGGTTTATGTG
TTTTAACCGAATGGGACGAATTCAAGTCACTTGATTTTAAGAAGATCTTTGATAATATGCAGAAAC
CCGCTTTCGTTTTCGACGGAAGAAACGTGGTCGACGCTGTGAAATTGAGAGAAATTGGATTCATAG
TATATTCCATAGGTAAACCTCTGGATAGTTGGCTCAAGGATATGCCGGCTGTTGCATAA

SEQ ID NO: 36 is a reencoded nucleic acid sequence of UDP-Glucose dehydrogenase
(HASB) originating from Chlorella virus PBCV-1
ATGAGTAGAATTGCTGTCGTTGGATGCGGTTACGTGGGTACGGCCTGCGCCGTACTTTTGGCGCAG
AAGAACGAAGTTATCGTTTTGGATATCTCGGAGGACCGGGTACAACTGATTAAGAATAAGAAGTC
ACCTATAGAAGATAAGGAAATCGAAGAATTCCTGGAGACGAAAGATTTGAATCTAACAGCGACGA

CGGATAAGGTGCTCGCCTATGAGAATGCTGAATTCGTTATAATAGCTACACCGACCGATTACGATG
TCGTCACTAGATATTTCAACACTAAGTCTGTTGAAAATGTGATAGGCGATGTCATTAAGAACACTC
AGACACACCCTACTATAGTGATCAAGAGTACTATTCCAATCGGTTTCGTGGATAAAGTTAGAGAAC
AGTTCGATTATCAAAATATTATCTTCTCGCCGGAATTCTTGAGAGAAGGACGAGCATTATATGATA
ATCTTTACCCCTCCCGTATCATCGTCGGTGATGACTCCCCAATTGCCTTAAAATTCGCGAATCTCTT
GGTCGAGGGTAGCAAAACTCCACTAGCTCCCGTATTAACTATGGGTACGCGAGAGGCCGAAGCTGT
AAAACTATTTTCAAATACATATTTGGCTATGAGAGTAGCATACTTCAATGAGCTAGACACATTTGC
AATGTCCCATGGTATGAACGCAAAAGAGATTATCGACGGAGTCACACTAGAACCAAGGATAGGGC
AGGGTTATTCGAATCCTTCATTTGGGTATGGAGCCTATTGTTTCCCAAAAGATACAAAGCAATTGTT
GGCTAATTTTGAAGGGGTTCCACAAGATATTATTGGCGCAATCGTAGAGTCTAACGAAACTAGAAA
GGAGGTTATTGTTTCTGAGGTCGAGAACAGATTTCCGACCACAGTTGGCGTATATAAGTTGGCTGC
TAAAGCTGGTTCGGATAATTTCAGGTCTAGTGCGATAGTCGATATCATGGAAAGGTTGGCTAATAA
GGGCTACCACATCAAAATATTTGAACCTACCGTTGAACAATTTGAGAATTTTGAGGTAGATAATAA
TCTCACAACTTTTGCAACCGAGTCTGATGTAATAATTGCGAACAGAGTTCCAGTCGAACATCGCAT
TCTGTTTGGGAAGAAGTTAATAACACGGGATGTCTATGGGGATAA

SEQ ID NO: 37 is a reencoded nucleic acid sequence of UDP-Glucose dehydrogenase
(HASB-A) originating from Chlorella virus PBCV-1
ATGTCACGAATCGCAGTAGTTGGCTGTGGGTACGTGGGAACCGCATGCGCTGTACTGCTGGCGCAA
AAGAATGAGGTAATCGTTCTTGATATTAGTGAGGATAGGGTCCAACTAATCAAGAACAAGAAATC
CCCGATCGAAGATAAGGAGATTGAAGAATTCTTGGAAACAAAAGACCTAAACTTAACTGCAACAA
CTGACAAAGTTTTAGCCTACGAAAACGCTGAGTTTGTGATTATAGCGACACCCACAGATTATGACG
TAGTTACCAGATACTTTAACACGAAGTCCGTAGAGAACGTCATTGGAGATGTTATAAAGAATACTC
AGACTCATCCTACGATAGTAATAAAGTCAACCATTCCCATAGGTTTCGTAGATAAGGTTAGGGAGC
AATTCGATTACCAGAACATTATATTTTCGCCAGAATTTCTGAGAGAGGGTCGCGCCCTGTATGATA
ATCTATATCCATCACGGATTATAGTGGGCGATGACTCTCCGATTGCACTTAAGTTTGCTAATCTTTT
AGTTGAGGGCTCCAAAACTCCGCTCGCCCCAGTACTTACGATGGGTACACGTGAGGCTGAAGCTGT
CAAGCTGTTTTCAAACACATACCTTGCTATGCGAGTCGCATACTTTAACGAACTAGATACCTTTGCT
ATGTCGCACGGTATGAATGCTAAAGAAATCATAGATGGCGTAACGTTGGAGCCTCGGATAGGTCA
AGGATATTCCAATCCATCTTTTGGCTACGGTGCGTATTGTTTCCCAAAGGACACGAAGCAATTATTA
GCTAACTTTGAGGGTGTTCCGCAAGATATAATTGGGGCGATAGTAGAAAGCAATGAAACACGGAA
GGAGGTAATCGTGAGTGAAGTGGAAAACCGATTCCCCACTACGGTCGGCGTTTACAAATTAGCCGC
CAAGGCTGGTTCCGACAATTTCCGATCCAGCGCAATAGTAGATATTATGGAAAGATTAGCTAATAA
GGGATACCACATTAAAATCTTTGAACCTACTGTCGAACAGTTCGAGAACTTCGAGGTTGATAATAA
CTTGACGACTTTCGCAACGGAGAGCGATGTAATTATTGCAAACCGCGTACCTGTGGAACATCGAAT
TTTGTTCGGAAAGAAGCTGATTACACGCGATGTATATGGCGATAACTAA

SEQ ID NO: 38 is a nucleic acid sequence of UDP-Glucose dehydrogenase (HASB)
originating from Streptococcus zooepidemicus
ATGAAGATATCGGTAGCGGGTTCGGGGTACGTGGGGTTATCCTTGTCAATCTTGCTTGCCCAACAT
AACGATGTGACTGTTGTAGATATAATCGACGAAAAGGTACGGCTAATTAACCAGGGCATATCTCCG
ATTAAGGATGCGGACATTGAGGAATATCTGAAGAATGCACCGTTGAATCTTACGGCTACACTAGAC
GGAGCTTCGGCTTATAGTAATGCTGATCTGATTATAATCGCAACGCCAACTAATTACGATTCAGAA
CGCAATTATTTCGACACCAGACACGTTGAAGAAGTAATTGAGCAAGTATTGGATTTAAATGCCTCC
GCTACTATAATCATCAAGAGTACCATACCCTTGGGTTTTATTAAACACGTAAGAGAGAAATACCAA
ACAGACAGAATCATCTTTTCTCCAGAGTTCTTAAGAGAGTCAAAGGCATTGTACGATAACTTATAC
CCCTCTCGTATAATAGTCAGTTATGAGAAGGATGACTCTCCAAGAGTTATACAAGCAGCTAAGGCG
TTCGCGGGTTTATTAAAAGAGGGGGCAAAGAGCAAGGATACCCCAGTTCTGTTTATGGGCTCTCAA
GAAGCTGAAGCTGTCAAGCTGTTTGCTAATACTTTTCTCGCCATGAGGGTCAGTTACTTCAACGAGC
TTGACACTTATAGCGAATCAAAAGGACTAGACGCCCAAAGAGTTATAGAAGGCGTCTGCCATGATC
AAAGGATAGGTAATCATTACAATAATCCATCCTTCGGATATGGCGGTTATTGTTTACCCAAAGACT
CAAAGCAACTTTTGGCTAATTATAGAGGCATACCTCAGTCTCTAATGTCTGCCATCGTTGAATCGAA
CAAGATCCGTAAGTCGTATTTAGCTGAACAAATATTAGATAGGGCTTCTTCACAAAAGCAGGCTGG
TGTACCTTTAACCATAGGATTTTACCGTTTGATTATGAAGTCCAACTCCGATAACTTTAGAGAATCA
GCCATTAAAGATATTATTGACATCATTAATGACTACGGTGTCAATATTGTCATTTATGAACCTATGT
TGGGAGAAGACATTGGTTATAGAGTCGTTAAAGATTTGGAACAGTTTAAGAACGAAAGTACAATT
ATTGTTAGTAACAGGTTTGAAGATGATTTAGGTGATGTTATTGATAAAGTTTATACACGTGACGTCT
TTGGTAG

SEQ ID NO: 39 is an amino acid sequence of UDP-Glucose dehydrogenase (HASB)
originating from Arabidopsis thaliana
MVKICCIGAGYVGGPTMAVMALKCPEIEVVVVDISEPRINAWNSDRLPIYEPGLEDVVKQCRGKNLFFS
TDVEKHVFESDIVFVSVNTPTKTQGLGAGKAADLTYWESAARMIADVSKSSKIVVEKSTVPVRTAEAIE
KILTHNSKGIEFQILSNPEFLAEGTAIKDLYNPDRVLIGGRDTAAGQKAIKALRDVYAHWVPVEQIICTNL
WSAELSKLAANAFLAQRISSVNAMSALCEATGADVTQVAHAVGTDTRIGPKFLNASVGFGGSCFQKDI
LNLIYICECNGLPEAANYWKQVVKVNDYQKIRFANRVVSSMFNTVSGKKIAILGFAFKKDTGDTRETPA
IDVCNRLVADKAKLSIYDPQVLEEQIRRDLSMARFDWDHPVPLQQIKAEGISEQVNVVSDAYEATKDA
HGLCVLTEWDEFKSLDFKKIFDNMQKPAFVFDGRNVVDAVKLREIGFIVYSIGKPLDSWLKDMPAVA

SEQ ID NO: 40 is an amino acid sequence of UDP-Glucose dehydrogenase (HASB)
originating from Chlorella virus PBCV1
MSRIAVVGCGYVGTACAVLLAQKNEVIVLDISEDRVQLIKNKKSPIEDKEIEEFLETKDLNLTATTDKVL
AYENAEFVIIATPTDYDVVTRYFNTKSVENVIGDVIKNTQTHPTIVIKSTIPIGFVDKVREQFDYQNIIFSPE
FLREGRALYDNLYPSRIIVGDDSPIALKFANLLVEGSKTPLAPVLTMGTREAEAVKLFSNTYLAMRVAY

FNELDTFAMSHGMNAKEIIDGVTLEPRIGQGYSNPSFGYGAYCFPKDTKQLLANFEGVPQDIIGAIVESN
ETRKEVIVSEVENRFPTTVGVYKLAAKAGSDNFRSSAIVDIMERLANKGYHIKIFEPTVEQFENFEVDNN
LTTFATESDVIIANRVPVEHRILFGKKLITRDVYGDN

SEQ ID NO: 41 is an amino acid sequence of UDP-Glucose dehydrogenase (HASB)
originating from Streptococcus zooepidemicus
MKISVAGSGYVGLSLSILLAQHNDVTVVDIIDEKVRLINQGISPIKDADIEEYLKNAPLNLTATLDGASAY
SNADLIIIATPTNYDSERNYFDTRHVEEVIEQVLDLNASATIIIKSTIPLGFIKHVREKYQTDRIIFSPEFLRE
SKALYDNLYPSRIIVSYEKDDSPRVIQAAKAFAGLLKEGAKSKDTPVLFMGSQEAEAVKLFANTFLAMR
VSYFNELDTYSESKGLDAQRVIEGVCHDQRIGNHYNNPSFGYGGYCLPKDSKQLLANYRGIPQSLMSAI
VESNKIRKSYLAEQILDRASSQKQAGVPLTIGFYRLIMKSNSDNFRESAIKDIIDIINDYGVNIVIYEPMLG
EDIGYRVVKDLEQFKNESTIIVSNRFEDDLGDVIDKVYTRDVFGRD

SEQ ID NO: 42 is a nucleic acid sequence of UDP-glucose-4-epimerase (GNE1) originating
from Pseudomonas aeruginosa
ATGATGTCCCGTTACGAGGAACTTCGTAAAGAGCTGCCTGCACAGCCGAAGGTATGGTTGATCACT
GGTGTCGCGGGCTTCATCGGGTCAAATCTGCTAGAGACCTTGCTTAAATTGGATCAGAAGGTTGTT
GGCTTGGACAACTTTGCAACTGGCCACCAGAGGAATCTGGACGAGGTGAGGTCATTGGTTTCTGAG
AAACAGTGGAGCAACTTTAAGTTTATTCAAGGGGACATTAGAAATCTGGACGACTGCAACAACGC
ATGTGCAGGCGTCGATTATGTCTTACACCAGGCAGCATTGGGCAGTGTGCCGAGGTCTATAAACGA
CCCTATAACATCGAATGCAACTAACATAGACGGTTTCTTGAACATGCTGATTGCAGCGAGGGATGC
AAAAGTTCAGTCTTTTACCTATGCCGCTTCATCCTCTACATACGGGGATCACCCCGGTTTGCCAAAG
GTGGAAGACACTATCGGCAAGCCTCTATCTCCATACGCTGTGACGAAGTATGTTAATGAGTTGTAC
GCAGACGTGTTTTCACGTTGTTATGGATTTTCAACCATCGGTCTGAGATACTTTAACGTCTTCGGCC
GTAGACAAGATCCGAATGGGGCATACGCCGCCGTAATCCCTAAGTGGACATCCTCAATGATACAG
GGTGACGACGTCTACATTAACGGAGATGGAGAGACATCACGTGACTTCTGCTACATAGAAAACAC
AGTTCAAGCAAACTTATTAGCCGCAACGGCCGGCCTAGACGCCAGAAACCAGGTGTATAATATCGC
GGTGGGTGGTAGAACGTCACTAAATCAGCTTTTCTTCGCCTTGAGAGACGGTCTAGCTGAGAACGG
CGTTAGCTACCATAGGGAACCAGTTTATAGGGATTTAGAGAAGGAGATGTGAGACACAGCTTGGC
AGATATTTCCAAGGCGGCTAAACTTCTTGGGTATGCTCCTAAATATGACGTATCAGCAGGGGTGGC
GCTTGCAATGCCATGGTATATAATGTTTCTTAAGTAA

SEQ ID NO: 43 is a nucleic acid sequence of UDP-glucose-4-epimerase (KFOA) originating
from Pasteurella multocida
ATGAACATTCTAGTGACCGGCGGTGCTGGCTATATCGGCTCCCATACCAGTTTATGCTTACTGAATA
AAGGATATAACGTGGTCATCATTGACAACCTGATCAATAGTTCATGCGAGTCGATCCGTAGGATAG
AACTGATAGCTAAGAAGAAGGTCACCTTCTACGAGCTGAACATTAATAATGAAAAGGAAGTGAAC

CAGATCCTTAAGAAACACAAATTCGATTGCATAATGCACTTCGCAGGTGCCAAGTCGGTGGCCGAG
TCACTAATTAAGCCAATTTCTACTATGACAACAACGTCTCTGGAACTTTGCAGCTGATTAATTGTG
CTATAAAGAACGACGTCGCAAATTTCATCTTTCTTCATCAGCAACTGTTTATGGTGAGTCAAAGAT
TATGCCAGTGACAGAGGACTGCCACATCGGCGGCACACTAAATCCATACGGAACATCAAAATACA
TTTCAGAGTTGATGATAAGGGACATCGCCAAGAAGTACTCAGACACTAATTTCTTATGCTTGCGTT
ATTTCAATCCTACCGGGGCCCACGAGTCCGGCATGATCGGCGAGTCTCCAGCTGATATACCATCCA
ATCTGGTGCCTTACATTTTACAGGTGGCCATGGGCAAGTTGGAGAAGTTAATGGTATTCGGCGGTG
ACTACCCAACAAAGGACGGCACGGGTGTGAGAGACTATATTCACGTGATGGACCTAGCGGAAGGT
CATGTGGCAGCCCTAAGCTACCTTTTCAGAGATAATAACACTAATTACCATGTATTCAATCTGGGC
ACGGGTAAGGGCTACAGTGTCCTTGAATTAGTAAGCACATTCGAGAAAATCTCGGGTGTCAGGATT
CCATACGAGATCGTTTCTCGTCGTGATGGGGACATCGCAGAGAGTTGGTCATCACCGGAGAAAGCG
AACAAGTACTTAAATTGGAAGGCCAAGAGGGAGCTTGAGACAATGCTAGAAGACGCATGGCGTTG
GCAGATGAAGAACCCGAATGGTTACATATAA

SEQ ID NO: 44 is a nucleic acid sequence of UDP-glucose-4-epimerase (KFOA) originating
from Escherichia coli
AATGAACATTCTAGTGACCGGCGGTGCTGGCTATATCGGCTCCCATACCAGTTTATGCTTACTGAAT
AAAGGATATAACGTGGTCATCATTGACAACCTGATCAATAGTTCATGCGAGTCGATCCGTAGGATA
GAACTGATAGCTAAGAAGGTCACCTTCTACGAGCTGAACATTAATAATGAAAAGGAAGTGAA
CCAGATCCTTAAGAAACACAAATTCGATTGCATAATGCACTTCGCAGGTGCCAAGTCGGTGGCCGA
GTCACTAATTAAGCCAATTTTCTACTATGACAACAACGTCTCTGGAACTTTGCAGCTGATTAATTGT
GCTATAAAGAACGACGTCGCAAATTTCATCTTTTCTTCATCAGCAACTGTTTATGGTGAGTCAAAGA
TTATGCCAGTGACAGAGGACTGCCACATCGGCGGCACACTAAATCCATACGGAACATCAAAATAC
ATTTCAGAGTTGATGATAAGGGACATCGCCAAGAAGTACTCAGACACTAATTTCTTATGCTTGCGT
TATTTCAATCCTACCGGGGCCCACGAGTCCGGCATGATCGGCGAGTCTCCAGCTGATATACCATCC
AATCTGGTGCCTTACATTTTACAGGTGGCCATGGGCAAGTTGGAGAAGTTAATGGTATTCGGCGGT
GACTACCCAACAAAGGACGGCACGGGTGTGAGAGACTATATTCACGTGATGGACCTAGCGGAAGG
TCATGTGGCAGCCCTAAGCTACCTTTTCAGAGATAATAACACTAATTACCATGTATTCAATCTGGGC
ACGGGTAAGGGCTACAGTGTCCTTGAATTAGTAAGCACATTCGAGAAAATCTCGGGTGTCAGGATT
CCATACGAGATCGTTTCTCGTCGTGATGGGGACATCGCAGAGAGTTGGTCATCACCGGAGAAAGCG
AACAAGTACTTAAATTGGAAGGCCAAGAGGGAGCTTGAGACAATGCTAGAAGACGCATGGCGTTG
GCAGATGAAGAACCCGAATGGTTACATATAA

SEQ ID NO: 45 is an amino acid sequence of UDP-glucose-4-epimerase (GNE1) originating
from Pseudomonas aeruginosa
MMSRYEELRKELPAQPKVWLITGVAGFIGSNLLETLLKLDQKVVGLDNFATGHQRNLDEVRSLVSEKQ
WSNFKFIQGDIRNLDDCNNACAGVDYVLHQAALGSVPRSINDPITSNATNIDGFLNMLIAARDAKVQSF

TYAASSSTYGDHPGLPKVEDTIGKPLSPYAVTKYVNELYADVFSRCYGFSTIGLRYFNVFGRRQDPNGA
YAAVIPKWTSSMIQGDDVYINGDGETSRDFCYIENTVQANLLAATAGLDARNQVYNIAVGGRTSLNQL
FFALRDGLAENGVSYHREPVYRDFREGDVRHSLADISKAAKLLGYAPKYDVSAGVALAMPWYIMFLK

SEQ ID NO: 46 is an amino acid sequence of UDP-glucose-4-epimerase (KFOA) originating
from Pasteurella multocida
MAILVTGGAGYIGSHTVVELLNANKDVVVLDNLCNSSPKSLERVAQITGKQVKFYQGDILDTALLQKIF
AENQIQSVIHFAGLKAVGESVQKPAEYYMNNVTGSLVLIQEMKKAGVWNFVFSSSATVYGDPEIIPITES
CKVGGTTNPYGTSKFMVEQILKDIAKATPEFSITILRYFNPVGAHESGLIGEDPNGIPNNLLPYISQVAIGK
LPQLSVFGSDYETHDGTGVRDYIHVVDLAIGHLKALDRHEGDAGLHIYNLGTGSGYSVLDMVKAFEKV
NDIKIPYKLVDRRPGDIATCYSDPSLAKTELNWTAARGLEQMMKDTWHWQKKNPKGYRD

SEQ ID NO: 47 is an amino acid sequence of UDP-glucose-4-epimerase (KFOA) originating
from Escherichia coli
MNILVTGGAGYIGSHTSLCLLNKGYNVVIIDNLINSSCESIRRIELIAKKKVTFYELNINNEKEVNQILKKH
KFDCIMHFAGAKSVAESLIKPIFYYDNNVSGTLQLINCAIKNDVANFIFSSSATVYGESKIMPVTEDCHIG
GTLNPYGTSKYISELMIRDIAKKYSDTNFLCLRYFNPTGAHESGMIGESPADIPSNLVPYILQVAMGKLE
KLMVFGGDYPTKDGTGVRDYIHVMDLAEGHVAALSYLFRDNNTNYHVFNLGTGKGYSVLELVSTFEK
ISGVRIPYEIVSRRDGDIAESWSSPEKANKYLNWKAKRELETMLEDAWRWQMKNPNGYI

SEQ ID NO: 48 is a reencoded nucleic acid sequence of hyaluronidase (HYAL) originating
from Cupiennius salei with a N-terminal secretion signal
ATGCAATTCAGCACTGTTGCATCAGTTGCATTTGTCGCCCTGGCGAATTTTGTAGCCGCGTTCAAGA
TTTACTGGAACGTCCCAACTTTTCAGTGCACGCATAACTACAAAATCGATTATGTCAAATTGTTGTC
CACTTACGGGATACAGGTCAATGATGGCGGTAAGTTTCAAGGAAACCAAGTGACTATCTTTTATGA
AACCCAGTTGGGTTTGTATCCACGAATCCTAAAATCTGGTAAAATGGAAAACGGCGGAATCCCTCA
ACGCGGTAACTTTGAGAAACACCTAGAAAAGGCAAGCACGGACCTCCAGAAAGTGATCCCTTGGA
AAGAGTTTAGCGGATTAGGTGTGATAGATTGGGAGGCTTGGAGACCCACATGGGAATTTAACTGG
GAACCGTTGAGGATATATCAAACCGAATCAATTAAGAGAGCTAAAGAACTACACCCTACCGCAAA
CGATTCCGCAGTAAAAGAAATTGCAGAGCGGCAATGGGAAGATTCAGCCAAGTTATACATGTTAG
AAACACTGCGGCTGGCAAAGAAACTTCGACCTCAAGCGCCTTGGTGTTACTACTTATTTCCTGATTG
CTATAATTACGTCGGAAAGAAACCAAAAGATTTCCAATGTAGTGCCTCGATACGTAAAGGTAACGA
TAAGCTAAGCTGGTTGTGGAAAGATTCTACGGCATTGTGTCCATCGATATACGTATATGAATCACA
ATTAGACAGGTATTCTTTTGAACAAAGGACATGGCGCGACAATGAGAAACTTCGGGAAGCGTTGC
GTGTAGCCACGAGAACCTCTAAAATATACCCATACGTTAACTATTTCGATAAGGAGCTTATACCGG
AGCAAGAAGTATGGAGAATGCTTGCGCAGGCAGCTGCTGTCGGTGGCAGTGGTGCGGTAATTTGG
GGCTCATCTGCTGCAGTTGCATCTGAAGAGTTATGTAAATCTTTAAAACAGTATATTATTGAAACGC

TTGGGCCGGCGGCAGAGAAGGTGGCTTGGCGTAGTGACTTATGCAGCAAAGAAATTTGTAATAATC
AGGGTCGCTGCACATTCCCGGACGATGATTATGCAAACGCATGGAAATTATTTACAGATGATACTG
TTAAGTTTTATGCTGGTAATATTACATGTAGGTGCTCCGAGAATTATTCTGGTCGTTTCTGCGAAAA
GAAGAATTAA

SEQ ID NO: 49 is a reencoded nucleic acid sequence of hyaluronidase (HYAL) originating
from Tityus serrulatus with a N-terminal secretion signal
ATGCAATTCTCTACTGTCGCTTCCGTTGCTTTCGTCGCTTTGGCTAACTTTGTTGCCGCTGCTGATTT
TAAAGTTTACTGGGAAGTGCCTTCCTTCCTTTGTTCTAAACGTTTTAAAATTAATGTAACGGAAGTT
TTAACAAGTCACGAGATTCTTGTCAATCAGGGTGAGAGTTTCAACGGTGACAAGATAGTAATCTTT
TACGAAAACCAATTGGGGAAGTACCCGCATATTGACTCAAACAATGTGGAGATCAATGGAGGAAT
ACTTCAAGTAGCCGATTTGGCGAAGCATTTGAAAGTAGCCAAGGATAATATCACTAAATTCGTCCC
GAATCCTAATTTCAACGGTGTCGGAGTGATCGACTGGGAAGCTTGGCGGCCATCATGGGAATTTAA
CTGGGGTAAGTTAAAAGTATATAAAGAAAAGAGCATTGACTTGGTCAAGTCGAAACATCCGGAGT
GGCCCTCCGACAGGGTTGAAAAGGTTGCTAAAGAGGAGTGGGAGGAGTGCCAAAGAATGGATG
GTGAAGACCCTGAAGTTAGCACAGGAAATGCGACCGAACGCAGTTTGGTGCTATTATCTATTCCCT
GACTGCTACAATTATTTCGGTAAGGATCAACCCTCTCAATTCAGCTGCTCGTCTCGAATTCAGAAGG
AAAATTCTCGTCTTTCTTGGCTCTGGAATCAATCAACAGCCATTTGCCTAAGCATTTATATCCAGGA
ATCCCATGTTACCAAATATAATATGTCCCAGCGGACATGGTGGATCGATGCGAGATTAAGAGAAGC
AATTCGAGTCAGCGAACACAGACCAAACATACCCATCTACCCTTACATTAATTATATTCTACCTGG
AACTAATCAAACTGTACCAGCAATGGACTTTAAAAGGACACTGGGTCAAATAGCTAGCCTCGGCCT
AGATGGTGCTTTGTTATGGGGATCTAGCTATCATGTTTTAACAGAATCTCAATGCAAAATCACTTCT
GATTATGTGAAATCAGTGATTGCTCCTACCGTGGCTACTGTCGTTCTCAATACAAACAGATGCTCAC
AGATAATTTGTAAGGGTCGCGGCAACTGTGTTTGGCCTGAAGAACCATTTAGTTCTTGGAAATACT
TAGTTGACCCCAAAATGCCAGTGTTCAAGCCAACCAACATCCACTGTAAATGTAAAGGTTACCTAG
GTAGATACTGTGAGATCCCAAAGTAA

SEQ ID NO: 50 is a reencoded nucleic acid sequence of hyaluronidase (HYAL) originating
from Bos Taurus with a N-terminal secretion signal
ATGCAATTTTCTACTGTTGCTTCTGTCGCTTTCGTAGCACTGGCCAATTTTGTAGCTGCTGGAAGCC
GTGACCCCGTTGTCCCAAACCAGCCTTTCACAACAATATGGAACGCAAATACGGAGTGGTGCATGA
AGAAGCATGGAGTGGACGTCGATATTTCAATCTTCGACGTGGTCACTAATCCAGGACAGACATTTA
GAGGTCCAAATATGACAATATTTTACAGTTCACAATTGGGGACCTACCCTTACTACACATCCGCGG
GTGAGCCAGTATTCGGTGGTCTACCCCAGAACGCAAGTTTGAATGCCCACTTGGCTAGGACATTTC
AAGATATTCTGGCAGCCATGCCAGAGCCCAGGTTCTCAGGTTTGGCTGTCATAGACTGGGAAGCGT
GGAGACCTCGTTGGGCATTTAATTGGGATACGAAAGACATATACAGGCAGAGGTCCAGAGCATTG
GTTCAGAAACAGCACCCGGATTGGCTGGCGCCACGTGTTGAGGCCGCAGCGCAAGACCAGTTCGA

GGGCGCTGCTGAGGAGTGGATGGCCGGCACACTTAAATTAGGGCAGGCGCTTCGTCCTCAAGGCTT
GTGGGGTTTCTATAACTTCCCGGAGTGTTACAATTATGACTTCAAATCTCCGAACTACACAGGAAG
GTGCCCTTTAAACATCTGTGCGCAGAACGACCAGTTAGGTTGGCTATGGGGTCAAAGTCGTGCACT
ATATCCATCAATCTACTTGCCGGCGGCTTTGGAAGGCACGAAGAAGACTCAGATGTTCGTTCAGCA
CCGTGTAGCAGAGGCATTTAGGGTGGCGGCAGGCGCTGGTGACCCCAAGCTACCCGTCTTACCCTA
TATGCAGCTATTTTACGATATGACAAACCACTTCTTGCCCGCGGAAGAACTGGAACATAGCTTAGG
TGAGTCAGCAGCGCAGGGTGCAGCCGGGGTTGTTCTGTGGGTTTCCTGGTTATCAACTTCGACAAA
GGAAAGCTGTCAAGCAATCAAGGAATACGTAGATACTACGTTGGGCCCTTCAATCTTGAACGTCAC
ATCTGGTGCCAGGTTGTGTTCTCAGGTATTGTGCAGTGGCCACGGGAGATGCGCGAGAAGACCTTC
ATATCCTAAAGCCAGGCTGATTCTAAATTCAACGTCGTTCTCCATCAAGCCAACACCCGGTGGTGG
TCCATTGACACTTCAAGGCGCTTTGAGTTTGGAAGACAGATTACGTATGGCAGTTGAGTTTGAATG
CAGATGTTACAGAGGATGGAGAGGGACACGTTGTGAGCAGTGGGGCATGTGGTAA

SEQ ID NO: 51 is a reencoded nucleic acid sequence of hyaluronidase (HYAL) originating
from Apis mellifera with a N-terminal secretion signal
ATGCAGTTTTCCACAGTTGCTTCCGTAGCTTTCGTAGCTTTAGCAAATTTTGTCGCAGCTTTGTTGTT
GGGCTTCGTTCAGTCTACTCCAGATAATAATAAGACTGTGCGTGAATTTAACGTGTATTGGAACGTT
CCTACATTCATGTGCCATAAGTACGGGTTGAGATTCGAAGAGGTGAGCGAGAAGTATGGCATTTTG
CAAAATTGGATGGACAAATTCAGAGGAGAGGAAATTGCGATCTTATACGACCCCGGCATGTTCCCC
GCACTGTTGAAAGACCCCAATGGGAATGTAGTAGCTAGGAATGGCGGAGTGCCCCAGCTTGGCAA
TTTAACTAAACATTTACAGGTGTTCAGGGACCACCTAATCAATCAGATTCCAGATAAAAGCTTTCC
CGGAGTTGGTGTAATAGATTTCGAGTCATGGAGACCTATTTTCAGACAGAACTGGGCCTCTTTGCA
ACCGTACAAGAAGCTATCAGTCGAAGTCGTACGTCGTGAGCACCCATTCTGGGATGATCAGAGAGT
GGAGCAAGAGGCGAAAAGAAGATTTGAAAAGTACGGCCAGCTTTTCATGGAAGAGACCTTGAAAG
CCGCAAAACGTATGCGTCCTGCAGCCAATTGGGGCTACTACGCTTACCCTTATTGCTACAACCTGA
CCCCAAACCAGCCCTCAGCTCAATGCGAGGCTACAACAATGCAGGAGAACGACAAGATGTCATGG
TTATTCGAGTCCGAGGACGTCTTACTGCCCAGCGTCTATTTGAGGTGGAACCTGACTAGCGGAGAG
AGGGTTGGTTTAGTTGGTGGTCGTGTCAAGGAAGCCCTGAGAATAGCTCGTCAGATGACCACCTCT
AGGAAGAAGGTCCTACCATACTACTGGTACAAATACCAGGATAGGAGAGACACGGACCTAAGTAG
GGCTGATCTTGAGGCGACTCTACGTAAAATCACGGACTTAGGAGCCGACGGTTTCATAATCTGGGG
ATCTTCCGATGACATCAACACCAAGGCAAAATGTTTGCAGTTCAGAGAATATTTGAATAACGAGTT
GGGCCCGGCAGTGAAGAGAATAGCCTTAAACAATAATGCGAATGACAGGCTAACAGTGGATGTGT
CTGTTGACCAGGTTTAA

SEQ ID NO: 52 is a reencoded nucleic acid sequence of hyaluronidase (HYAL) originating
from Macaca mulatta with a N-terminal secretion signal
ATGCAATTCAGTACTGTCGCTAGCGTCGCCTTTGTGGCTCTTGCAAATTTCGTCGCTGCTTCACGTG
GGCCTTTATTGCCGAACAGACCATTTGCAACGGTATGGAATGCTAACACCCAATGGTGTTTAGAGA
GGCACTCAGTAGACGTCGATGTCTCCATATTTGATGTTGTAGCAAACCCCGGCCAGACTTTTCGTGG
GCCGGACATGACTATTTTCTATTCCAGTCAGCTAGGTACTTACCCTTATTATACCCCGACGGGTGAA
CCTGTCTTTGGTGGCCTTCCACAGAACGCATCACTTATAGCGCACTTGGCTAGGACTTTCCAGGATA
TATTGGCAGCTATACCAGCCCCCGACTTTTCTGGGCTAGCCGTAATTGACTGGGAAGCATGGAGAC
CGAGATGGGCATTTAACTGGGACACGAAAGACATTTACAGGCAAAGGTCCCGTGCCTTAGTCCAA
GCACAGCATCCCGACTGGCCAGTTACTCAAGTTGAGGCTGTGGCACAAGACCAGTTCCAGGGTGCT
GCGAGGGCATGGATGGCAGGAACCTTGCAATTAGGCCGTGCTTTAAGACCAAGGGGACTATGGGG
CTTCTACGGTTTTCCCGATTGCTACAATTACGATTTCCTATCTCCTAATTACACAGGGCAATGCCCA
TCAGGGATCAGAGCACAAAATGACCAGTTAGGATGGTTGTGGGGTCAGAGTCGTGCATTATATCCC
AGTATCTACATGCCGGCTGTTTTGGAAGGAACAGGCAAATCCCAAATGTACGTGCAGCACAGGGTC
GCGGAAGCCTTTAGGGTTGCTGTTGCAGCAGGCGACCCAAACTTACCCGTCCTACCGTACGTCCAG
ATTTTCTACGATATGACCAACCACTTTCTTCCATTGGACGAGTTAGAGCATAGTCTTGGAGAATCAG
CGGCTCAGGGCGCAGCAGGAGTCGTGTTATGGGTGTCATGGGAGAATACTAGAACAAAAGAATCT
TGCCAGGCAATTAAAGAATATATGGACACTACCTTGGGGCCTTTTATTCTGAACGTCACTTCGGGT
GCGTTGTTATGCTCGCAAGCCTTATGTTCAAGCCATGGTCGTTGTGTCCGTCGTCCCTCACATCCAA
AAGCCTTGCTAATCCTTAATCCCGCAAGCTTTAGTATTCAGTTGACACCAGATGGTGGTCCCTTGTC
GTTAAGGGGAGCATTGTCACTTGAGGACCAGGCACAAATGGCTGTTGAATTCAAATGCAGGTGCTA
CCCCGGGTGGCAAGGCCCTTGGTGTGAGCAGAAGAGTATGTGGTAA

SEQ ID NO: 53 is a reencoded nucleic acid sequence of hyaluronidase (HYAL) originating
from Vespa magnifica with a N-terminal secretion signal
ATGCAATTTTCTACAGTGGCAAGTGTTGCATTCGTTGCACTAGCCAACTTTGTGGCGGCAGATAGCT
GTGGGTCAAACTGCGAAAAGAGTGAGAGACCGAAAAGGGTCTTCAACATTTACTGGAACGTACCT
ACATTCATGTGTCACCAGTACGGACTATACTTTGACGAGGTCACGAATTTTAATATAAAGCACAAC
AGCAAAGACAATTTTCAAGGGGACAAGATCGCGATCTTTTATGACCCCGGGGAGTTTCCCGCTCTG
CTGCCACTAAACTATGGTAAGTACAAGATCAGGAATGGTGGTGTTCCACAAGAGGGTAACATCACC
ATCCATCTGCAGAGATTCATAGAGTACCTAGATAAGACCTATCCGAACCGTAACTTTTCAGGCATC
GGTGTGATCGATTTCGAGAGGTGGAGACCAATTTTCAGACAGAATTGGGGTAATATGAAGATTTAC
AAGAACTTCTCCATCGATCTTGTGCGTAAAGAGCATCCTTTCTGGAATAAGAAAATGATCGAGTTG
GAAGCTTCTAAAAGATTCGAGAAATACGCCCGTCTGTTCATGGAAGAAACATTAAAGTTGGCTAAG
AAAACTAGAAAACAGGCCGATTGGGGCTACTACGGTTACCCCTATTGCTTCAACATGTCTCCTACT
AATTTCGTTCCTGACTGCGATGTCACAGCTAGGGATGAGAACAACGAGATGTCTTGGTTGTTTAAC
AACCAGAATGTCCTATTACCAAGTGTATACATTAGGAGAGAGCTAACTCCTGACCAGAGGATTGGG

CTTGTACAGGGGAGAGTGAAGGAAGCTGTGAGAATTTCAAATAAACTGAAGCACTCACCTAAAGT
CTTCAGCTATTGGTGGTATGTTTACCAAGACGAGACCAACACCTTCTTAACGGAGACCGACGTCAA
GAAGACGTTTCAGGAGATTGTGATCAACGGTGGAGATGGGATTATAATCTGGGGTTCGTCCTCTGA
TGTAAACAGCTTGTCCAAGTGTACGAGGTTAAGGGAGTACCTATTGACAGTCTTGGGACCAATTGC
TGTTAACGTGACTGAAGCAGTAAACTAA

SEQ ID NO: 54 is a reencoded nucleic acid sequence of hyaluronidase (HYAL) originating
from Cupiennius salei with a N-terminal secretion signal and a C-terminal anchoring signal
ATGCAATTCAGCACTGTTGCATCAGTTGCATTTGTCGCCCTGGCGAATTTTGTAGCCGCGTTCAAGA
TTTACTGGAACGTCCCAACTTTTCAGTGCACGCATAACTACAAAATCGATTATGTCAAATTGTTGTC
CACTTACGGGATACAGGTCAATGATGGCGGTAAGTTTCAAGGAAACCAAGTGACTATCTTTTATGA
AACCCAGTTGGGTTTGTATCCACGAATCCTAAAATCTGGTAAAATGGAAAACGGCGGAATCCCTCA
ACGCGGTAACTTTGAGAAACACCTAGAAAAGGCAAGCACGGACCTCCAGAAAGTGATCCCTTGGA
AAGAGTTTAGCGGATTAGGTGTGATAGATTGGGAGGCTTGGAGACCCACATGGGAATTTAACTGG
GAACCGTTGAGGATATATCAAACCGAATCAATTAAGAGAGCTAAAGAACTACACCCTACCGCAAA
CGATTCCGCAGTAAAAGAAATTGCAGAGCGGCAATGGGAAGATTCAGCCAAGTTATACATGTTAG
AAACACTGCGGCTGGCAAAGAAACTTCGACCTCAAGCGCCTTGGTGTTACTACTTATTTCCTGATTG
CTATAATTACGTCGGAAAGAAACCAAAAGATTTCCAATGTAGTGCCTCGATACGTAAAGGTAACGA
TAAGCTAAGCTGGTTGTGGAAAGATTCTACGGCATTGTGTCCATCGATATACGTATATGAATCACA
ATTAGACAGGTATTCTTTTGAACAAAGGACATGGCGCGACAATGAGAAACTTCGGGAAGCGTTGC
GTGTAGCCACGAGAACCTCTAAAATATACCCATACGTTAACTATTTCGATAAGGAGCTTATACCGG
AGCAAGAAGTATGGAGAATGCTTGCGCAGGCAGCTGCTGTCGGTGGCAGTGGTGCGGTAATTTGG
GGCTCATCTGCTGCAGTTGCATCTGAAGAGTTATGTAAATCTTTAAAACAGTATATTATTGAAACGC
TTGGGCCGGCGGCAGAGAAGGTGGCTTGGCGTAGTGACTTATGCAGCAAAGAAATTTGTAATAATC
AGGGTCGCTGCACATTCCCGGACGATGATTATGCAAACGCATGGAAATTATTTACAGATGATACTG
TTAAGTTTTATGCTGGTAATATTACATGTAGGTGCTCCGAGAATTATTCTGGTCGTTTCTGCGAAAA
GAAGAATGGATCCGCCATTTCTCAAATCACTGACGGTCAAATCCAAGCTACTACCACTGCTACCAC
CGAAGCTACCACCACTGCTGCCCCATCTTCCACCGTTGAAACTGTTTCTCCATCCAGCACCGAAACT
ATCTCTCAACAAACTGAAAATGGTGCTGCTAAGGCCGCTGTCGGTATGGGTGCCGGTGCTCTAGCT
GCTGCTGCTATGTTGTTATAA

SEQ ID NO: 55 is a reencoded nucleic acid sequence of hyaluronidase (HYAL) originating
from Tityus serrulatus with a N-terminal secretion signal and a C-terminal anchoring signal
ATGCAATTCTCTACTGTCGCTTCCGTTGCTTTCGTCGCTTTGGCTAACTTTGTTGCCGCTGCTGATTT
TAAAGTTTACTGGGAAGTGCCTTCCTTCCTTTGTTCTAAACGTTTTAAAATTAATGTAACGGAAGTT
TTAACAAGTCACGAGATTCTTGTCAATCAGGGTGAGAGTTTCAACGGTGACAAGATAGTAATCTTT
TACGAAAACCAATTGGGGAAGTACCCGCATATTGACTCAAACAATGTGGAGATCAATGGAGGAAT

ACTTCAAGTAGCCGATTTGGCGAAGCATTTGAAAGTAGCCAAGGATAATATCACTAAATTCGTCCC
GAATCCTAATTTCAACGGTGTCGGAGTGATCGACTGGAAGCTTGGCGGCCATCATGGGAATTTAA
CTGGGGTAAGTTAAAAGTATATAAAGAAAAGAGCATTGACTTGGTCAAGTCGAAACATCCGGAGT
GGCCCTCCGACAGGGTTGAAAAGGTTGCTAAAGAGGAGTGGAGGAGAGTGCCAAAGAATGGATG
GTGAAGACCCTGAAGTTAGCACAGGAAATGCGACCGAACGCAGTTTGGTGCTATTATCTATTCCCT
GACTGCTACAATTATTTCGGTAAGGATCAACCCTCTCAATTCAGCTGCTCGTCTCGAATTCAGAAGG
AAAATTCTCGTCTTTCTTGGCTCTGGAATCAATCAACAGCCATTTGCCTAAGCATTTATATCCAGGA
ATCCCATGTTACCAAATATAATATGTCCCAGCGGACATGGTGGATCGATGCGAGATTAAGAGAAGC
AATTCGAGTCAGCGAACACAGACCAAACATACCCATCTACCCTTACATTAATTATATTCTACCTGG
AACTAATCAAACTGTACCAGCAATGGACTTTAAAAGGACACTGGGTCAAATAGCTAGCCTCGGCCT
AGATGGTGCTTTGTTATGGGGATCTAGCTATCATGTTTTAACAGAATCTCAATGCAAAATCACTTCT
GATTATGTGAAATCAGTGATTGCTCCTACCGTGGCTACTGTCGTTCTCAATACAAACAGATGCTCAC
AGATAATTTGTAAGGGTCGCGGCAACTGTGTTTGGCCTGAAGAACCATTTAGTTCTTGGAAATACT
TAGTTGACCCCAAAATGCCAGTGTTCAAGCCAACCAACATCCACTGTAAATGTAAAGGTTACCTAG
GTAGATACTGTGAGATCCCAAAGGATCCGCCATTTCTCAAATCACTGACGGTCAAATCCAAGCTA
CTACCACTGCTACCACCGAAGCTACCACCACTGCTGCCCATCTTCCACCGTTGAAACTGTTTCTCC
ATCCAGCACCGAAACTATCTCTCAACAAACTGAAAATGGTGCTGCTAAGGCCGCTGTCGGTATGGG
TGCCGGTGCTCTAGCTGCTGCTGCTATGTTGTTATAA

SEQ ID NO: 56 is a reencoded nucleic acid sequence of hyaluronidase (HYAL) originating
from Bos taurus with a N-terminal secretion signal and a C-terminal anchoring signal
ATGCAATTTTCTACTGTTGCTTCTGTCGCTTTCGTAGCACTGGCCAATTTTGTAGCTGCTGGAAGCC
GTGACCCCGTTGTCCCAAACCAGCCTTTCACAACAATATGGAACGCAAATACGGAGTGGTGCATGA
AGAAGCATGGAGTGGACGTCGATATTTCAATCTTCGACGTGGTCACTAATCCAGGACAGACATTTA
GAGGTCCAAATATGACAATATTTTACAGTTCACAATTGGGGACCTACCCTTACTACACATCCGCGG
GTGAGCCAGTATTCGGTGGTCTACCCCAGAACGCAAGTTTGAATGCCCACTTGGCTAGGACATTTC
AAGATATTCTGGCAGCCATGCCAGAGCCCAGGTTCTCAGGTTTGGCTGTCATAGACTGGGAAGCGT
GGAGACCTCGTTGGGCATTTAATTGGGATACGAAAGACATATACAGGCAGAGGTCCAGAGCATTG
GTTCAGAAACAGCACCCGGATTGGCTGGCGCCACGTGTTGAGGCCGCAGCGCAAGACCAGTTCGA
GGGCGCTGCTGAGGAGTGGATGGCCGGCACACTTAAATTAGGGCAGGCGCTTCGTCCTCAAGGCTT
GTGGGGTTTCTATAACTTCCCGGAGTGTTACAATTATGACTTCAAATCTCCGAACTACACAGGAAG
GTGCCCTTTAAACATCTGTGCGCAGAACGACCAGTTAGGTTGGCTATGGGGTCAAAGTCGTGCACT
ATATCCATCAATCTACTTGCCGGCGGCTTTGGAAGGCACGAAGAAGACTCAGATGTTCGTTCAGCA
CCGTGTAGCAGAGGCATTTAGGGTGGCGGCAGGCGCTGGTGACCCCAAGCTACCCGTCTTACCCTA
TATGCAGCTATTTTACGATATGACAAACCACTTCTTGCCCGCGGAAGAACTGGAACATAGCTTAGG
TGAGTCAGCAGCGCAGGGTGCAGCCGGGGTTGTTCTGTGGGTTTCCTGGTTATCAACTTCGACAAA
GGAAAGCTGTCAAGCAATCAAGGAATACGTAGATACTACGTTGGGCCCTTCAATCTTGAACGTCAC
ATCTGGTGCCAGGTTGTGTTCTCAGGTATTGTGCAGTGGCCACGGAGATGCGCGAGAAGACCTTC

ATATCCTAAAGCCAGGCTGATTCTAAATTCAACGTCGTTCTCCATCAAGCCAACACCCGGTGGTGG
TCCATTGACACTTCAAGGCGCTTTGAGTTTGGAAGACAGATTACGTATGGCAGTTGAGTTTGAATG
CAGATGTTACAGAGGATGGAGAGGGACACGTTGTGAGCAGTGGGGCATGTGGGATCCGCCATTT
CTCAAATCACTGACGGTCAAATCCAAGCTACTACCACTGCTACCACCGAAGCTACCACCACTGCTG
CCCCATCTTCCACCGTTGAAACTGTTTCTCCATCCAGCACCGAAACTATCTCTCAACAAACTGAAAA
TGGTGCTGCTAAGGCCGCTGTCGGTATGGGTGCCGGTGCTCTAGCTGCTGCTGCTATGTTGTTATAA

SEQ ID NO: 57 is a reencoded nucleic acid sequence of hyaluronidase (HYAL) originating
from Apis mellifera with a N-terminal secretion signal and a C-terminal anchoring signal
ATGCAGTTTTCCACAGTTGCTTCCGTAGCTTTCGTAGCTTTAGCAAATTTTGTCGCAGCTTTGTTGTT
GGGCTTCGTTCAGTCTACTCCAGATAATAATAAGACTGTGCGTGAATTTAACGTGTATTGGAACGTT
CCTACATTCATGTGCCATAAGTACGGGTTGAGATTCGAAGAGGTGAGCGAGAAGTATGGCATTTTG
CAAAATTGGATGGACAAATTCAGAGGAGAGGAAATTGCGATCTTATACGACCCCGGCATGTTCCCC
GCACTGTTGAAAGACCCCAATGGGAATGTAGTAGCTAGGAATGGCGGAGTGCCCCAGCTTGGCAA
TTTAACTAAACATTTACAGGTGTTCAGGGACCACCTAATCAATCAGATTCCAGATAAAAGCTTTCC
CGGAGTTGGTGTAATAGATTTCGAGTCATGGAGACCTATTTTCAGACAGAACTGGGCCTCTTTGCA
ACCGTACAAGAAGCTATCAGTCGAAGTCGTACGTCGTGAGCACCCATTCTGGGATGATCAGAGAGT
GGAGCAAGAGGCGAAAAGAAGATTTGAAAAGTACGGCCAGCTTTTCATGGAAGAGACCTTGAAAG
CCGCAAAACGTATGCGTCCTGCAGCCAATTGGGGCTACTACGCTTACCCTTATTGCTACAACCTGA
CCCCAAACCAGCCCTCAGCTCAATGCGAGGCTACAACAATGCAGGAGAACGACAAGATGTCATGG
TTATTCGAGTCCGAGGACGTCTTACTGCCCAGCGTCTATTTGAGGTGGAACCTGACTAGCGGAGAG
AGGGTTGGTTTAGTTGGTGGTCGTGTCAAGGAAGCCCTGAGAATAGCTCGTCAGATGACCACCTCT
AGGAAGAAGGTCCTACCATACTACTGGTACAAATACCAGGATAGGAGAGACACGGACCTAAGTAG
GGCTGATCTTGAGGCGACTCTACGTAAAATCACGGACTTAGGAGCCGACGGTTTCATAATCTGGGG
ATCTTCCGATGACATCAACACCAAGGCAAAATGTTTGCAGTTCAGAGAATATTTGAATAACGAGTT
GGGCCCGGCAGTGAAGAGAATAGCCTTAAACAATAATGCGAATGACAGGCTAACAGTGGATGTGT
CTGTTGACCAGGTTGGATCCGCCATTTCTCAAATCACTGACGGTCAAATCCAAGCTACTACCACTGC
TACCACCGAAGCTACCACCACTGCTGCCCCATCTTCCACCGTTGAAACTGTTTCTCCATCCAGCACC
GAAACTATCTCTCAACAAACTGAAAATGGTGCTGCTAAGGCCGCTGTCGGTATGGGTGCCGGTGCT
CTAGCTGCTGCTGCTATGTTGTTATAA

SEQ ID NO: 58 is a reencoded nucleic acid sequence of hyaluronidase (HYAL) originating
from Macaca mulatta with a N-terminal secretion signal and a C-terminal anchoring signal
ATGCAATTCAGTACTGTCGCTAGCGTCGCCTTTGTGGCTCTTGCAAATTTCGTCGCTGCTTCACGTG
GGCCTTTATTGCCGAACAGACCATTTGCAACGGTATGGAATGCTAACACCCAATGGTGTTTAGAGA
GGCACTCAGTAGACGTCGATGTCTCCATATTTGATGTTGTAGCAAACCCCGGCCAGACTTTTCGTGG
GCCGGACATGACTATTTTCTATTCCAGTCAGCTAGGTACTTACCCTTATTATACCCCGACGGGTGAA

CCTGTCTTTGGTGGCCTTCCACAGAACGCATCACTTATAGCGCACTTGGCTAGGACTTTCCAGGATA
TATTGGCAGCTATACCAGCCCCCGACTTTTCTGGGCTAGCCGTAATTGACTGGGAAGCATGGAGAC
CGAGATGGGCATTTAACTGGGACACGAAAGACATTTACAGGCAAAGGTCCCGTGCCTTAGTCCAA
GCACAGCATCCCGACTGGCCAGTTACTCAAGTTGAGGCTGTGGCACAAGACCAGTTCCAGGGTGCT
GCGAGGGCATGGATGGCAGGAACCTTGCAATTAGGCCGTGCTTTAAGACCAAGGGGACTATGGGG
CTTCTACGGTTTTCCCGATTGCTACAATTACGATTTCCTATCTCCTAATTACACAGGGCAATGCCCA
TCAGGGATCAGAGCACAAAATGACCAGTTAGGATGGTTGTGGGTCAGAGTCGTGCATTATATCCC
AGTATCTACATGCCGGCTGTTTTGGAAGGAACAGGCAAATCCCAAATGTACGTGCAGCACAGGGTC
GCGGAAGCCTTTAGGGTTGCTGTTGCAGCAGGCGACCCAAACTTACCCGTCCTACCGTACGTCCAG
ATTTTCTACGATATGACCAACCACTTTCTTCCATTGGACGAGTTAGAGCATAGTCTTGGAGAATCAG
CGGCTCAGGGCGCAGCAGGAGTCGTGTTATGGGTGTCATGGAGAATACTAGAACAAAAGAATCT
TGCCAGGCAATTAAAGAATATATGGACACTACCTTGGGGCCTTTATTCTGAACGTCACTTCGGGT
GCGTTGTTATGCTCGCAAGCCTTATGTTCAAGCCATGGTCGTTGTGTCCGTCGTCCCTCACATCCAA
AAGCCTTGCTAATCCTTAATCCCGCAAGCTTTAGTATTCAGTTGACACCAGATGGTGGTCCCTTGTC
GTTAAGGGGAGCATTGTCACTTGAGGACCAGGCACAAATGGCTGTTGAATTCAAATGCAGGTGCTA
CCCCGGGTGGCAAGGCCCTTGGTGTGAGCAGAAGAGTATGTGGGGATCCGCCATTTCTCAAATCAC
TGACGGTCAAATCCAAGCTACTACCACTGCTACCACCGAAGCTACCACCACTGCTGCCCCATCTTC
CACCGTTGAAACTGTTTCTCCATCCAGCACCGAAACTATCTCTCAACAAACTGAAAATGGTGCTGC
TAAGGCCGCTGTCGGTATGGGTGCCGGTGCTCTAGCTGCTGCTGCTATGTTGTTATAA

SEQ ID NO: 59 is a reencoded nucleic acid sequence of hyaluronidase (HYAL) originating
from Vespa magnifica with a N-terminal secretion signal and a C-terminal anchoring signal
ATGCAATTTTCTACAGTGGCAAGTGTTGCATTCGTTGCACTAGCCAACTTTGTGGCGGCAGATAGCT
GTGGGTCAAACTGCGAAAAGAGTGAGAGACCGAAAAGGGTCTTCAACATTTACTGGAACGTACCT
ACATTCATGTGTCACCAGTACGGACTATACTTTGACGAGGTCACGAATTTTAATATAAAGCACAAC
AGCAAAGACAATTTTCAAGGGGACAAGATCGCGATCTTTTATGACCCCGGGGAGTTTCCCGCTCTG
CTGCCACTAAACTATGGTAAGTACAAGATCAGGAATGGTGGTGTTCCACAAGAGGGTAACATCACC
ATCCATCTGCAGAGATTCATAGAGTACCTAGATAAGACCTATCCGAACCGTAACTTTTCAGGCATC
GGTGTGATCGATTTCGAGAGGTGGAGACCAATTTTCAGACAGAATTGGGGTAATATGAAGATTTAC
AAGAACTTCTCCATCGATCTTGTGCGTAAAGAGCATCCTTTCTGGAATAAGAAAATGATCGAGTTG
GAAGCTTCTAAAAGATTCGAGAAATACGCCCGTCTGTTCATGGAAGAAACATTAAAGTTGGCTAAG
AAAACTAGAAAACAGGCCGATTGGGGCTACTACGGTTACCCCTATTGCTTCAACATGTCTCCTACT
AATTTCGTTCCTGACTGCGATGTCACAGCTAGGGATGAGAACAACGAGATGTCTTGGTTGTTTAAC
AACCAGAATGTCCTATTACCAAGTGTATACATTAGGAGAGAGCTAACTCCTGACCAGAGGATTGGG
CTTGTACAGGGGAGAGTGAAGGAAGCTGTGAGAATTTCAAATAAACTGAAGCACTCACCTAAAGT
CTTCAGCTATTGGTGGTATGTTTACCAAGACGAGACCAACACCTTCTTAACGGAGACCGACGTCAA
GAAGACGTTTCAGGAGATTGTGATCAACGGTGGAGATGGGATTATAATCTGGGGTTCGTCCTCTGA
TGTAAACAGCTTGTCCAAGTGTACGAGGTTAAGGGAGTACCTATTGACAGTCTTGGGACCAATTGC

TGTTAACGTGACTGAAGCAGTAAACGGATCCGCCATTTCTCAAATCACTGACGGTCAAATCCAAGC
TACTACCACTGCTACCACCGAAGCTACCACCACTGCTGCCCCATCTTCCACCGTTGAAACTGTTTCT
CCATCCAGCACCGAAACTATCTCTCAACAAACTGAAAATGGTGCTGCTAAGGCCGCTGTCGGTATG
GGTGCCGGTGCTCTAGCTGCTGCTGCTATGTTGTTATAA

SEQ ID NO: 60 is an amino acid sequence of hyaluronidase (HYAL) originating from
Cupiennius salei with a N-terminal secretion signal
MQFSTVASVAFVALANFVAAFKIYWNVPTFQCTHNYKIDYVKLLSTYGIQVNDGGKFQGNQVTIFYET
QLGLYPRILKSGKMENGGIPQRGNFEKHLEKASTDLQKVIPWKEFSGLGVIDWEAWRPTWEFNWEPLRI
YQTESIKRAKELHPTANDSAVKEIAERQWEDSAKLYMLETLRLAKKLRPQAPWCYYLFPDCYNYVGK
KPKDFQCSASIRKGNDKLSWLWKDSTALCPSIYVYESQLDRYSFEQRTWRDNEKLREALRVATRTSKIY
PYVNYFDKELIPEQEVWRMLAQAAAVGGSGAVIWGSSAAVASEELCKSLKQYIIETLGPAAEKVAWRS
DLCSKEICNNQGRCTFPDDDYANAWKLFTDDTVKFYAGNITCRCSENYSGRFCEKKN

SEQ ID NO: 61 is an amino acid sequence of hyaluronidase (HYAL) originating from Tityus
serrulatus with a N-terminal secretion signal
MQFSTVASVAFVALANFVAAADFKVYWEVPSFLCSKRFKINVTEVLTSHEILVNQGESFNGDKIVIFYE
NQLGKYPHIDSNNVEINGGILQVADLAKHLKVAKDNITKFVPNPNFNGVGVIDWEAWRPSWEFNWGK
LKVYKEKSIDLVKSKHPEWPSDRVEKVAKEEWEESAKEWMVKTLKLAQEMRPNAVWCYYLFPDCYN
YFGKDQPSQFSCSSRIQKENSRLSWLWNQSTAICLSIYIQESHVTKYNMSQRTWWIDARLREAIRVSEHR
PNIPIYPYINYILPGTNQTVPAMDFKRTLGQIASLGLDGALLWGSSYHVLTESQCKITSDYVKSVIAPTVA
TVVLNTNRCSQIICKGRGNCVWPEEPFSSWKYLVDPKMPVFKPTNIHCKCKGYLGRYCEIPK

SEQ ID NO: 62 is an amino acid sequence of hyaluronidase (HYAL) originating from Bos
taurus with a N-terminal secretion signal
MQFSTVASVAFVALANFVAAGSRDPVVPNQPFTTIWNANTEWCMKKHGVDVDISIFDVVTNPGQTFRG
PNMTIFYSSQLGTYPYYTSAGEPVFGGLPQNASLNAHLARTFQDILAAMPEPRFSGLAVIDWEAWRPRW
AFNWDTKDIYRQRSRALVQKQHPDWLAPRVEAAAQDQFEGAAEEWMAGTLKLGQALRPQGLWGFY
NFPECYNYDFKSPNYTGRCPLNICAQNDQLGWLWGQSRALYPSIYLPAALEGTKKTQMFVQHRVAEAF
RVAAGAGDPKLPVLPYMQLFYDMTNHFLPAEELEHSLGESAAQGAAGVVLWVSWLSTSTKESCQAIK
EYVDTTLGPSILNVTSGARLCSQVLCSGHGRCARRPSYPKARLILNSTSFSIKPTPGGGPLTLQGALSLED
RLRMAVEFECRCYRGWRGTRCEQWGMW

SEQ ID NO: 63 is an amino acid sequence of hyaluronidase (HYAL) originating from Apis
mellifera with a N-terminal secretion signal
MQFSTVASVAFVALANFVAALLLGFVQSTPDNNKTVREFNVYWNVPTFMCHKYGLRFEEVSEKYGIL
QNWMDKFRGEEIAILYDPGMFPALLKDPNGNVVARNGGVPQLGNLTKHLQVFRDHLINQIPDKSFPGV
GVIDFESWRPIFRQNWASLQPYKKLSVEVVRREHPFWDDQRVEQEAKRRFEKYGQLFMEETLKAAKR
MRPAANWGYYAYPYCYNLTPNQPSAQCEATTMQENDKMSWLFESEDVLLPSVYLRWNLTSGERVGL
VGGRVKEALRIARQMTTSRKKVLPYYWYKYQDRRDTDLSRADLEATLRKITDLGADGFIIWGSSDDIN
TKAKCLQFREYLNNELGPAVKRIALNNNANDRLTVDVSVDQV

SEQ ID NO: 64 is an amino acid sequence of hyaluronidase (HYAL) originating from
Macaca mulatta with a N-terminal secretion signal
MQFSTVASVAFVALANFVAASRGPLLPNRPFATVWNANTQWCLERHSVDVDVSIFDVVANPGQTFRG
PDMTIFYSSQLGTYPYYTPTGEPVFGGLPQNASLIAHLARTFQDILAAIPAPDFSGLAVIDWEAWRPRWA
FNWDTKDIYRQRSRALVQAQHPDWPVTQVEAVAQDQFQGAARAWMAGTLQLGRALRPRGLWGFYG
FPDCYNYDFLSPNYTGQCPSGIRAQNDQLGWLWGQSRALYPSIYMPAVLEGTGKSQMYVQHRVAEAF
RVAVAAGDPNLPVLPYVQIFYDMTNHFLPLDELEHSLGESAAQGAAGVVLWVSWENTRTKESCQAIKE
YMDTTLGPFILNVTSGALLCSQALCSSHGRCVRRPSHPKALLILNPASFSIQLTPDGGPLSLRGALSLEDQ
AQMAVEFKCRCYPGWQGPWCEQKSMW

SEQ ID NO: 65 is an amino acid sequence of hyaluronidase (HYAL) originating from Vespa
magnifica with a N-terminal secretion signal
MQFSTVASVAFVALANFVAADSCGSNCEKSERPKRVFNIYWNVPTFMCHQYGLYFDEVTNFNIKHNSK
DNFQGDKIAIFYDPGEFPALLPLNYGKYKIRNGGVPQEGNITIHLQRFIEYLDKTYPNRNFSGIGVIDFER
WRPIFRQNWGNMKIYKNFSIDLVRKEHPFWNKKMIELEASKRFEKYARLFMEETLKLAKKTRKQADW
GYYGYPYCFNMSPTNFVPDCDVTARDENNEMSWLFNNQNVLLPSVYIRRELTPDQRIGLVQGRVKEAV
RISNKLKHSPKVFSYWWYVYQDETNTFLTETDVKKTFQEIVINGGDGIIIWGSSSDVNSLSKCTRLREYL
LTVLGPIAVNVTEAVN

SEQ ID NO: 66 is an amino acid sequence of hyaluronidase (HYAL) originating from
Cupiennius salei with a N-terminal secretion signal and a C-terminal anchoring signal
MQFSTVASVAFVALANFVAAFKIYWNVPTFQCTHNYKIDYVKLLSTYGIQVNDGGKFQGNQVTIFYET
QLGLYPRILKSGKMENGGIPQRGNFEKHLEKASTDLQKVIPWKEFSGLGVIDWEAWRPTWEFNWEPLRI
YQTESIKRAKELHPTANDSAVKEIAERQWEDSAKLYMLETLRLAKKLRPQAPWCYYLFPDCYNYVGK
KPKDFQCSASIRKGNDKLSWLWKDSTALCPSIYVYESQLDRYSFEQRTWRDNEKLREALRVATRTSKIY
PYVNYFDKELIPEQEVWRMLAQAAAVGGSGAVIWGSSAAVASEELCKSLKQYIIETLGPAAEKVAWRS

DLCSKEICNNQGRCTFPDDDYANAWKLFTDDTVKFYAGNITCRCSENYSGRFCEKKNGSAISQITDGQI
QATTTATTEATTTAAPSSTVETVSPSSTETISQQTENGAAKAAVGMGAGALAAAAMLL

SEQ ID NO: 67 is an amino acid sequence of hyaluronidase (HYAL) originating from Tityus
serrulatus with a N-terminal secretion signal and a C-terminal anchoring signal
MQFSTVASVAFVALANFVAAADFKVYWEVPSFLCSKRFKINVTEVLTSHEILVNQGESFNGDKIVIFYE
NQLGKYPHIDSNNVEINGGILQVADLAKHLKVAKDNITKFVPNPNFNGVGVIDWEAWRPSWEFNWGK
LKVYKEKSIDLVKSKHPEWPSDRVEKVAKEEWEESAKEWMVKTLKLAQEMRPNAVWCYYLFPDCYN
YFGKDQPSQFSCSSRIQKENSRLSWLWNQSTAICLSIYIQESHVTKYNMSQRTWWIDARLREAIRVSEHR
PNIPIYPYINYILPGTNQTVPAMDFKRTLGQIASLGLDGALLWGSSYHVLTESQCKITSDYVKSVIAPTVA
TVVLNTNRCSQIICKGRGNCVWPEEPFSSWKYLVDPKMPVFKPTNIHCKCKGYLGRYCEIPKGSAISQIT
DGQIQATTTATTEATTTAAPSSTVETVSPSSTETISQQTENGAAKAAVGMGAGALAAAAMLL

SEQ ID NO: 68 is an amino acid sequence of hyaluronidase (HYAL) originating from Bos
taurus with a N-terminal secretion signal and a C-terminal anchoring signal
MQFSTVASVAFVALANFVAAGSRDPVVPNQPFTTIWNANTEWCMKKHGVDVDISIFDVVTNPGQTFRG
PNMTIFYSSQLGTYPYYTSAGEPVFGGLPQNASLNAHLARTFQDILAAMPEPRFSGLAVIDWEAWRPRW
AFNWDTKDIYRQRSRALVQKQHPDWLAPRVEAAAQDQFEGAAEEWMAGTLKLGQALRPQGLWGFY
NFPECYNYDFKSPNYTGRCPLNICAQNDQLGWLWGQSRALYPSIYLPAALEGTKKTQMFVQHRVAEAF
RVAAGAGDPKLPVLPYMQLFYDMTNHFLPAEELEHSLGESAAQGAAGVVLWVSWLSTSTKESCQAIK
EYVDTTLGPSILNVTSGARLCSQVLCSGHGRCARRPSYPKARLILNSTSFSIKPTPGGGPLTLQGALSLED
RLRMAVEFECRCYRGWRGTRCEQWGMWGSAISQITDGQIQATTTATTEATTTAAPSSTVETVSPSSTET
ISQQTENGAAKAAVGMGAGALAAAAMLL

SEQ ID NO: 69 is an amino acid sequence of hyaluronidase (HYAL) originating from Apis
mellifera with a N-terminal secretion signal and a C-terminal anchoring signal
MQFSTVASVAFVALANFVAALLLGFVQSTPDNNKTVREFNVYWNVPTFMCHKYGLRFEEVSEKYGIL
QNWMDKFRGEEIAILYDPGMFPALLKDPNGNVVARNGGVPQLGNLTKHLQVFRDHLINQIPDKSFPGV
GVIDFESWRPIFRQNWASLQPYKKLSVEVVRREHPFWDDQRVEQEAKRRFEKYGQLFMEETLKAAKR
MRPAANWGYYAYPYCYNLTPNQPSAQCEATTMQENDKMSWLFESEDVLLPSVYLRWNLTSGERVGL
VGGRVKEALRIARQMTTSRKKVLPYYWYKYQDRRDTDLSRADLEATLRKITDLGADGFIIWGSSDDIN
TKAKCLQFREYLNNELGPAVKRIALNNNANDRLTVDVSVDQVGSAISQITDGQIQATTTATTEATTTAA
PSSTVETVSPSSTETISQQTENGAAKAAVGMGAGALAAAAMLL

SEQ ID NO: 70 is an amino acid sequence of hyaluronidase (HYAL) originating from
Macaca mulatta with a N-terminal secretion signal and a C-terminal anchoring signal
MQFSTVASVAFVALANFVAASRGPLLPNRPFATVWNANTQWCLERHSVDVDVSIFDVVANPGQTFRG
PDMTIFYSSQLGTYPYYTPTGEPVFGGLPQNASLIAHLARTFQDILAAIPAPDFSGLAVIDWEAWRPRWA
FNWDTKDIYRQRSRALVQAQHPDWPVTQVEAVAQDQFQGAARAWMAGTLQLGRALRPRGLWGFYG
FPDCYNYDFLSPNYTGQCPSGIRAQNDQLGWLWGQSRALYPSIYMPAVLEGTGKSQMYVQHRVAEAF
RVAVAAGDPNLPVLPYVQIFYDMTNHFLPLDELEHSLGESAAQGAAGVVLWVSWENTRTKESCQAIKE
YMDTTLGPFILNVTSGALLCSQALCSSHGRCVRRPSHPKALLILNPASFSIQLTPDGGPLSLRGALSLEDQ
AQMAVEFKCRCYPGWQGPWCEQKSMWGSAISQITDGQIQATTTATTEATTTAAPSSTVETVSPSSTETI
SQQTENGAAKAAVGMGAGALAAAAMLL

SEQ ID NO: 71 is an amino acid sequence of hyaluronidase (HYAL) originating from Vespa
magnifica with a N-terminal secretion signal and a C-terminal anchoring signal
MQFSTVASVAFVALANFVAADSCGSNCEKSERPKRVFNIYWNVPTFMCHQYGLYFDEVTNFNIKHNSK
DNFQGDKIAIFYDPGEFPALLPLNYGKYKIRNGGVPQEGNITIHLQRFIEYLDKTYPNRNFSGIGVIDFER
WRPIFRQNWGNMKIYKNFSIDLVRKEHPFWNKKMIELEASKRFEKYARLFMEETLKLAKKTRKQADW
GYYGYPYCFNMSPTNFVPDCDVTARDENNEMSWLFNNQNVLLPSVYIRRELTPDQRIGLVQGRVKEAV
RISNKLKHSPKVFSYWWYVYQDETNTFLTETDVKKTFQEIVINGGDGIIIWGSSSDVNSLSKCTRLREYL
LTVLGPIAVNVTEAVNAISQITDGQIQATTTATTEATTTAAPSSTVETVSPSSTETISQQTENGAAKAAVG
MGAGALAAAAMLL

SEQ ID NO: 72 is a nucleic acid sequence of glutamine-fructose-6-phosphate
amidotransferase (GFA1) originating from Saccharomyces cerevisiae
ATGTGTGGTATCTTTGGTTACTGCAATTATCTAGTGGAAAGATCCAGAGGAGAAATTATCGACACC
TTAGTGGATGGTTTACAAAGATTAGAATATAGAGGCTATGATTCCACCGGTATTGCTATCGATGGT
GACGAAGCTGATTCTACTTTCATCTATAAGCAAATCGGTAAAGTGAGTGCTTTGAAAGAGGAGATT
ACTAAGCAAAATCCGAACAGAGACGTTACTTTTGTCTCTCATTGTGGTATTGCGCATACTAGATGG
GCTACTCACGGTCGACCAGAACAAGTTAACTGTCACCCTCAAAGATCTGACCCAGAAGACCAATTT
GTGGTCGTTCATAATGGTATCATCACAAATTTTAGAGAACTGAAGACTCTTTTAATTAACAAAGGTT
ATAAATTCGAAAGTGATACCGATACCGAGTGTATTGCTAAACTATATTTGCATTTATACAATACAA
ATTTACAAAATGGGCATGACTTAGATTTCCACGAATTAACCAAGCTAGTTCTTTTAGAACTAGAAG
GTTCATACGGGTTATTATGTAAATCTTGTCACTATCCTAATGAGGTTATCGCCACTAGAAAAGGGTC
CCCTTTACTGATTGGTGTCAAATCTGAAAAAAACTAAAAGTCGACTTCGTGGATGTGGAATTTCC
CGAAGAAAACGCTGGTCAACCGGAAATTCCATTGAAATCTAACAACAAATCATTTGGCTTGGGCCC
AAAGAAAGCTCGTGAATTTGAAGCTGGTTCCCAAAATGCCAATTTACTACCAATTGCCGCCAATGA
ATTTAACTTGAGACATTCTCAATCCAGGGCTTTCCTATCAGAAGATGGATCTCCAACACCGGTGGA
ATTTTTTGTTTCTTCGGATGCGGCATCTGTTGTTAAACATACCAAGAAGGTGCTATTTTTAGAAGAT

GACGATTTGGCTCATATTTACGATGGTGAGTTACATATTCATAGATCTAGAAGAGAAGTAGGCGCA
TCAATGACAAGGTCCATTCAAACTTTAGAGATGGAGTTAGCTCAGATCATGAAGGGCCCTTACGAC
CATTTTATGCAAAAGGAAATCTATGAGCAACCAGAATCTACTTTCAATACTATGAGAGGTAGAATC
GACTATGAAAATAATAAAGTGATATTGGGTGGTTTAAAGGCATGGTTACCAGTTGTCAGAAGAGCA
CGGAGACTGATCATGATCGCATGCGGTACTTCTTATCATTCATGTTTGGCTACTCGTGCTATCTTCG
AAGAATTATCAGATATCCCAGTTAGTGTGGAATTAGCGTCTGACTTTCTGGACAGAAAATGCCCTG
TCTTCAGAGACGATGTATGCGTGTTTGTTTCACAAAGTGGTGAAACTGCGGATACCATGCTGGCTCT
AAATTATTGTTTAGAAAGAGGAGCCTTAACTGTCGGAATTGTTAACAGTGTTGGTTCTTCTATCTCT
CGTGTCACCCACTGTGGTGTTCATATTAACGCTGGTCCTGAAATTGGTGTTGCCTCTACAAAAGCTT
ATACTTCCCAGTATATTGCCTTAGTGATGTTTGCTCTATCGCTGTCAGATGACCGTGTATCGAAAAT
AGACAGAAGAATTGAAATCATTCAAGGCTTGAAGTTAATCCCGGGCCAAATTAAGCAGGTATTAA
AGCTGGAACCAAGAATAAAAAAGCTCTGTGCGACTGAATTAAAGGATCAAAAATCTCTATTGTTAT
TGGGTAGAGGTTACCAATTTGCTGCTGCTCTGGAAGGTGCTTTGAAGATCAAAGAAATTTCTTATAT
GCATTCTGAAGGTGTTTTGGCAGGTGAGTTGAAGCACGGTGTCTTGGCCTTGGTGGACGAAAACTT
GCCAATCATTGCTTTTGGTACCAGAGACTCTCTATTCCCTAAAGTAGTTTCCTCTATTGAGCAAGTT
ACTGTAAGAAAGGGCCATCCAATTATTATTTGTAACGAAAATGATGAAGTGTGGGCGCAAAAATCT
AAATCAATCGACCTGCAAACCTTAGAAGTTCCACAAACTGTTGATTGTTTACAAGGTCTAATTAAT
ATTATTCCATTACAACTAATGTCATATTGGTTGGCTGTTAATAAAGGGATTGATGTTGATTTTCCAA
GAAACTTGGCTAAATCTGTTACCGTCGAATAA

SEQ ID NO: 73 is a reencoded nucleic acid sequence of glutamine-fructose-6-phosphate amidotransferase (GFA1) originating from Chlorella virus 1 (PBCV-1)
ATGTGCGGGATCTTCGGTGCTGTGTCGAACAATAATAGCATAGAAGTTTCCATCAAGGGTATACAG
AAGCTAGAGTACCGCGGGTACGATTCGTGTGGAATAGCCTATACAGACGGAGGCGCCATTGAACG
GATCAGGTCAATAGATGGGATCGACGACTTAAGGAAGAAAACAATAACAGAGTCTTCCCCGGTAG
CTATCGCTCATTCAAGGTGGAGTACCACTGGGATTCCAAGTGTTGTGAACGCCCACCCGCACATCT
CTCGGGGCACGTCTGGATGCGAGTCGCGCATTGCAGTCGTACACAATGGCATTATTGAAAATTACC
AGCAGATCCGCAAGTACTTGATAAATCTTGGGTATACTTTCGATTCACAGACCGACACGGAAGTCA
TTGCTCATCTAATAGACTCGCAGTATAACGGGAACATCCTGCACACCGTCCAAATGGCAGTTAAGC
ACCTCAAAGGAAGCTACGCCATAGCAGTCATGTGCCATAAGGAGTCCGGGAAGATCGTGGTGGCA
AAACAGAAGAGTCCGTTGGTATTGGGTATAGGAAGCGACGGAGCTTATTATATCGCATCGGACGTA
TTGGCTCTTCCCACTAACAAAGTGGTCTACATAAGTGACGGATTCTCTGCTGAGTTAAGTCCTGGAT
CGATGACTATCTATGATCTGGATGGCAATAAGGTAGAATACGAGGTGGAAGACGTTGAAATGGAG
CAGACGTCGATGTCTTTAGATAACTTTGATCACTACATGATCAAAGAGATCAATGAACAGCCGATA
TCAATTCTCAATACTATTAAGAACAAAGGATTCTATGCAGAGATATTTGGTGATCTAGCACATGAA
ATATTTCAGAAGATCGACAATATATTGATTCTAGCCTGCGGAACTTCGTATCATGCAGGTCTCGTCG
GGAAGCAATGGATAGAGACCATCTCACGCATCCCGGTGGATGTTCATATTGCAAGCGAGTATGAGC
CGACAATTCCACGAGCCAATACACTTGTGATAACGATTAGTCAAAGCGGCGAGACTGCGGATACA

ATCGCAGCCCTTCAACGAGCGCAAAACGCAGGAATGATATACACTTTGTGTATCTGCAACAGCCCC
AAGTCAACTCTAGTGCGCGAGTCGATAATGAAATACATAACCAAATGCGGTTCAGAAGTTTCTGTT
GCCAGTACTAAAGCATTTACGTCGCAACTTGTGGTGTTGTATATGCTTGCGAACGTTCTTGCGAACA
AAACTGATGATCTGCTAGGGGATCTTCCTCAAGCTATCGAGAGAGTAATCTGTCTTACTAATGACG
AAATGAAGCGTTGGGCGGATGAAATTTGTACTGCCAAAAGTGCCATCTTCTTAGGGAGAGGACTGA
ACGCACCTGTAGCATTTGAGGGCGCGCTAAAGTTGAAGGAAATCTCATATATCCATGCCGAGGGTT
TCCTCGGTGGAGAGTTGAAGCATGGGCCGCTGGCTTTGTTGGACGATAAGATCCCTGTAATCGTGA
CTGTTGCTGACCACGCTTATTTGGATCACATTAAGGCTAACATCGACGAGGTATTGGCACGAAACG
TTACCGTATACGCGATCGTCGATCAGTATGTTAATATTGAACCACAGGAGCGACTGCATGTAGTAA
AAGTGCCGTTTGTGAGTAAAGAGTTTCTCCCATCATCCACACAATTCCGATGCAATTGTTATCGTA
TTATGTCGCGATCAAGCTGGGCAAGAACGTTGACAAGCCACGTAACCTGGCGAAAAGTGTTACAA
CATTCtaa

SEQ ID NO: 74 is a reencoded nucleic acid sequence of glutamine-fructose-6-phosphate amidotransferase (GFA1) originating from Chlorella virus 1 (PBCV-1)
ATGTGTGGCATCTTTGGAGCAGTGTCAAACAACAACTCTATCGAGGTGTCAATCAAGGGTATTCAG
AAGCTAGAATATCGTGGGTATGATTCGTGCGGTATTGCGTATACAGATGGGGGTGCGATTGAGCGT
ATACGTTCTATTGACGGCATTGACGATCTGCGTAAGAAAACAATCACAGAATCATCACCAGTGGCC
ATTGCTCACTCGCGGTGGAGCACCACTGGAATTCCATCAGTGGTGAACGCACATCCTCATATTTCTC
GCGGAACCAGTGGGTGTGAGTCTCGTATCGCGGTAGTCCACAACGGTATCATTGAAAACTATCAGC
AGATCCGAAAATATCTCATCAATCTTGGTTATACGTTTGATAGTCAAACGGACACAGAGGTCATTG
CGCATTTGATTGATTCTCAGTACAATGGGAATATCTTGCACACCGTCCAAATGGCTGTCAAGCACCT
GAAGGGCTCTTATGCCATTGCAGTTATGTGTCATAAAGAGTCTGGTAAAATAGTCGTGGCGAAACA
GAAGTCACCCCTCGTACTTGGAATCGGCTCAGATGGTGCTTACTACATCGCTTCGGACGTGCTGGC
GCTGCCGACAAATAAAGTTGTTTATATTTCAGACGGTTTCTCCGCAGAACTATCTCCAGGGAGTAT
GACCATTTACGATCTTGATGGAAATAAAGTAGAATATGAAGTAGAGGACGTTGAAATGGAACAAA
CTAGTATGTCTCTCGATAACTTTGATCATTACATGATTAAGGAAATTAATGAGCAACCAATCAGTAT
TCTAAACACTATAAAAAATAAAGGGTTCTATGCAGAAATATTCGGTGATTTGGCTCATGAAATCTT
CCAAAAAATAGACAACATCCTGATACTGGCTTGTGGTACAAGTTATCACGCCGGTCTTGTAGGAAA
ACAGTGGATAGAGACCATCTCtAGAATCCCCGTGGATGTTCACATCGCGAGTGAATACGAACCTAC
TATTCCGAGAGCGAACACATTGGTAATCACTATTTCACAGTCGGGTGAAACTGCGGACACGATAGC
GGCTTTGCAACGGGCCCAAAACGCCGGGATGATTTATACATTGTGTATTTGCAATTCACCAAAGAG
CACTCTTGTTCGTGAGAGCATTATGAAGTACATCACGAAATGTGGTTCTGAGGTGTCAGTGGCATC
AACGAAGGCGTTTACTTCTCAGCTCGTAGTACTGTACATGCTGGCAAACGTATTGGCAAATAAAAC
CGATGATTTGCTGGGAGACCTCCCACAGGCAATAGAACGGGTAATTTGTTTGACAAATGACGAAAT
GAAACGATGGGCCGACGAAATTTGCACTGCGAAATCCGCGATCTTCCTGGGAAGAGGACTAAACG
CACCAGTTGCCTTTGAGGGAGCGTTGAAGCTCAAAGAAATCTCTTACATTCATGCAGAGGGCTTCC
TGGGAGGTGAGTTGAAACACGGCCCCCTCGCACTCCTTGATGACAAGATTCCTGTTATCGTAACCG

TAGCAGATCATGCTTATTTGGACCATATCAAAGCAAATATCGACGAAGTGCTTGCGAGGAACGTTA
CGGTATACGCCATAGTAGACCAGTATGTGAACATCGAGCCCCAGGAACGCCTTCACGTCGTCAAGG
TTCCGTTTGTATCCAAAGAATTTTCTCCGATAATTCACACTATCCCGATGCAACTGCTTTCGTATTAC
GTGGCAATTAAGCTTGGAAAGAACGTTGACAAACCAAGGAATCTTGCAAAATCCGTGACTACCTTT
TAA

SEQ ID NO: 75 is an amino acid sequence of glutamine-fructose-6-phosphate
amidotransferase (GFA1) originating from Saccharomyces cerevisiae
MCGIFGYCNYLVERSRGEIIDTLVDGLQRLEYRGYDSTGIAIDGDEADSTFIYKQIGKVSALKEEITKQNP
NRDVTFVSHCGIAHTRWATHGRPEQVNCHPQRSDPEDQFVVVHNGIITNFRELKTLLINKGYKFESDTD
TECIAKLYLHLYNTNLQNGHDLDFHELTKLVLLELEGSYGLLCKSCHYPNEVIATRKGSPLLIGVKSEKK
LKVDFVDVEFPEENAGQPEIPLKSNNKSFGLGPKKAREFEAGSQNANLLPIAANEFNLRHSQSRAFLSED
GSPTPVEFFVSSDAASVVKHTKKVLFLEDDDLAHIYDGELHIHRSRREVGASMTRSIQTLEMELAQIMK
GPYDHFMQKEIYEQPESTFNTMRGRIDYENNKVILGGLKAWLPVVRRARRLIMIACGTSYHSCLATRAI
FEELSDIPVSVELASDFLDRKCPVFRDDVCVFVSQSGETADTMLALNYCLERGALTVGIVNSVGSSISRV
THCGVHINAGPEIGVASTKAYTSQYIALVMFALSLSDDRVSKIDRRIEIIQGLKLIPGQIKQVLKLEPRIKK
LCATELKDQKSLLLLGRGYQFAAALEGALKIKEISYMHSEGVLAGELKHGVLALVDENLPIIAFGTRDS
LFPKVVSSIEQVTARKGHPIIICNENDEVWAQKSKSIDLQTLEVPQTVDCLQGLINIIPLQLMSYWLAVNK
GIDVDFPRNLAKSVTVE

SEQ ID NO: 76 is an amino acid sequence of glutamine-fructose-6-phosphate
amidotransferase (GFA1) originating from Chlorella virus 1 (PBCV-1)
MCGIFGAVSNNNSIEVSIKGIQKLEYRGYDSCGIAYTDGGAIERIRSIDGIDDLRKKTITESSPVAIAHSRW
STTGIPSVVNAHPHISRGTSGCESRIAVVHNGIIENYQQIRKYLINLGYTFDSQTDTEVIAHLIDSQYNGNI
LHTVQMAVKHLKGSYAIAVMCHKESGKIVVAKQKSPLVLGIGSDGAYYIASDVLALPTNKVVYISDGF
SAELSPGSMTIYDLDGNKVEYEVEDVEMEQTSMSLDNFDHYMIKEINEQPISILNTIKNKGFYAEIFGDL
AHEIFQKIDNILILACGTSYHAGLVGKQWIETISRIPVDVHIASEYEPTIPRANTLVITISQSGETADTIAAL
QRAQNAGMIYTLCICNSPKSTLVRESIMKYITKCGSEVSVASTKAFTSQLVVLYMLANVLANKTDDLLG
DLPQAIERVICLTNDEMKRWADEICTAKSAIFLGRGLNAPVAFEGALKLKEISYIHAEGFLGGELKHGPL
ALLDDKIPVIVTVADHAYLDHIKANIDEVLARNVTVYAIVDQYVNIEPQERLHVVKVPFVSKEFSPIIHTI
PMQLLSYYVAIKLGKNVDKPRNLAKSVTTF

SEQ ID NO: 77 is a nucleic acid sequence of UDP-N-acetylglucosamine pyrophosphorylase
(QRI1) originating from Saccharomyces cerevisiae
ATGACTGACACAAAACAGCTATTCATTGAAGCCGGACAAAGTCAACTTTTCCACAATTGGGAAAGC
TTGTCTCGCAAAGACCAAGAAGAATTGCTTTCAAACCTGGAGCAAATATCTTCCAAGAGGTCCCCT
GCAAAACTACTGGAAGACTGTCAAAATGCTATTAAATTCTCACTAGCTAACTCTTCTAAGGATACT

GGCGTCGAAATTTCACCATTGCCCCCTACTTCGTACGAGTCGCTTATTGGCAACAGTAAGAAAGAA
AATGAATACTGGCGTTTAGGCCTTGAAGCTATTGGCAAGGGTGAAGTCGCAGTGATTTTAATGGCT
GGCGGACAAGGTACGCGGTTAGGATCCTCTCAACCAAAGGGCTGTTACGACATTGGATTGCCTTCT
AAGAAATCTCTTTTTCAAATTCAAGCTGAAAAGTTGATCAGGTTGCAAGATATGGTAAAGGACAAA
AAGGTAGAAATTCCTTGGTATATTATGACATCAGGCCCCACTAGAGCGGCTACTGAGGCATACTTT
CAAGAACACAATTATTTTGGCTTGAATAAAGAACAAATTACGTTCTTCAACCAGGGAACCCTGCCT
GCCTTTGATTTAACCGGGAAGCATTTCCTAATGAAAGACCCAGTAAACCTATCTCAATCACCAGAT
GGAAATGGTGGACTCTACCGTGCCATCAAGGAAAACAAGTTGAACGAAGACTTTGATAGGAGAGG
AATCAAGCATGTTTACATGTACTGTGTCGATAATGTCCTATCTAAAATCGCAGACCCTGTATTTATT
GGTTTTGCCATCAAGCATGGCTTCGAACTGGCCACCAAAGCCGTTAGAAAGAGAGATGCGCATGA
ATCAGTTGGGTTAATTGCTACTAAAAACGAGAAACCATGTGTCATAGAATATTCTGAAATTTCCAA
TGAATTGGCTGAAGCAAAGGATAAAGATGGCTTATTAAAACTACGCGCAGGCAACATTGTAAATC
ATTATTACCTAGTGGATTTACTAAAACGTGATTTGGATCAGTGGTGTGAGAATATGCCATATCACAT
TGCGAAGAAGAAAATTCCAGCTTATGATAGTGTTACCGGCAAGTACACTAAGCCTACCGAACCAA
ACGGTATAAAATTAGAGCAATTCATATTTGATGTCTTTGACACTGTACCACTGAACAAGTTTGGGT
GCTTAGAAGTAGATAGATGCAAAGAATTTTCACCTTTAAAAAACGGTCCTGGTTCTAAGAACGATA
ATCCTGAGACCAGCAGACTAGCATATTTGAAACTAGGAACCTCGTGGTTGGAAGATGCAGGCGCTA
TTGTAAAAGATGGGGTACTAGTCGAAGTTTCCAGCAAATTGAGTTATGCAGGTGAAAATCTATCCC
AGTTCAAAGGTAAAGTCTTTGACAGAAGTGGTATAGTATTAGAAAAATAA

SEQ ID NO: 78 is an amino acid sequence of UDP-N-acetylglucosamine pyrophosphorylase
(QRI1) originating from Saccharomyces cerevisiae
MTDTKQLFIEAGQSQLFHNWESLSRKDQEELLSNLEQISSKRSPAKLLEDCQNAIKFSLANSSKDTGVEIS
PLPPTSYESLIGNSKKENEYWRLGLEAIGKGEVAVILMAGGQGTRLGSSQPKGCYDIGLPSKKSLFQIQA
EKLIRLQDMVKDKKVEIPWYIMTSGPTRAATEAYFQEHNYFGLNKEQITFFNQGTLPAFDLTGKHFLMK
DPVNLSQSPDGNGGLYRAIKENKLNEDFDRRGIKHVYMYCVDNVLSKIADPVFIGFAIKHGFELATKAV
RKRDAHESVGLIATKNEKPCVIEYSEISNELAEAKDKDGLLKLRAGNIVNHYYLVDLLKRDLDQWCEN
MPYHIAKKKIPAYDSVTGKYTKPTEPNGIKLEQFIFDVFDTVPLNKFGCLEVDRCKEFSPLKNGPGSKND
NPETSRLAYLKLGTSWLEDAGAIVKDGVLVEVSSKLSYAGENLSQFKGKVFDRSGIVLEK

SEQ ID NO: 79 is a nucleic acid sequence of Phosphoglucomutase-1 (PGM1) originating
from Saccharomyces cerevisiae
ATGTCACTTCTAATAGATTCTGTACCAACAGTTGCTTATAAGGACCAAAAACCGGGTACTTCAGGT
TTACGTAAGAAGACCAAGGTTTTCATGGATGAGCCTCATTATACTGAGAACTTCATTCAAGCAACA
ATGCAATCTATCCCTAATGGCTCAGAGGGAACCACTTTAGTTGTTGGAGGAGATGGTCGTTTCTAC
AACGATGTTATCATGAACAAGATTGCCGCAGTAGGTGCTGCAAACGGTGTCAGAAAGTTAGTCATT
GGTCAAGGCGGTTTACTTTCAACACCAGCTGCTTCTCATATAATTAGAACATACGAGGAAAAGTGT

ACCGGTGGTGGTATCATATTAACTGCCTCACACAACCCAGGCGGTCCAGAGAATGATTTAGGTATC
AAGTATAATTTACCTAATGGTGGGCCAGCTCCAGAGAGTGTCACTAACGCTATCTGGGAAGCGTCT
AAAAAATTAACTCACTATAAAATTATAAAGAACTTCCCCAAGTTGAATTTGAACAAGCTTGGTAAA
AACCAAAAATATGGCCCATTGTTAGTGGACATAATTGATCCTGCCAAAGCATACGTTCAATTTCTG
AAGGAAATTTTTGATTTTGACTTAATTAAAAGCTTCTTAGCGAAACAGCGCAAAGACAAAGGGTGG
AAGTTGTTGTTTGACTCCTTAAATGGTATTACAGGACCATATGGTAAGGCTATATTTGTTGATGAAT
TTGGTTTACCGGCAGAGGAAGTTCTTCAAAATTGGCACCCTTTACCTGATTTCGGCGGTTTACATCC
CGATCCGAATCTAACCTATGCACGAACTCTTGTTGACAGGGTTGACCGCGAAAAAATTGCCTTTGG
AGCAGCCTCCGATGGTGATGGTGATAGGAATATGATTTACGGTTATGGCCCTGCTTTCGTTTCGCCA
GGTGATTCTGTTGCCATTATTGCCGAATATGCACCCGAAATTCCATACTTCGCCAAACAAGGTATTT
ATGGCTTGGCACGTTCATTTCCTACATCCTCAGCCATTGATCGTGTTGCAGCAAAAAAGGGATTAA
GATGTTACGAAGTTCCAACCGGCTGGAAATTCTTCTGTGCCTTATTTGATGCTAAAAAGCTATCAAT
CTGTGGTGAAGAATCCTTCGGTACAGGTTCCAATCATATCAGAGAAAAGGACGGTCTATGGGCCAT
TATTGCTTGGTTAAATATCTTGGCTATCTACCATAGGCGTAACCCTGAAAAGGAAGCTTCGATCAA
AACTATTCAGGACGAATTTTGGAACGAGTATGGCCGTACTTTCTTCACAAGATACGATTACGAACA
TATCGAATGCGAGCAGGCCGAAAAAGTTGTAGCTCTTTTGAGTGAATTTGTATCAAGGCCAAACGT
TTGTGGCTCCCACTTCCCAGCTGATGAGTCTTTAACCGTTATCGATTGTGGTGATTTTTCGTATAGA
GATCTAGATGGCTCCATCTCTGAAAATCAAGGCCTTTTCGTAAAGTTTTCGAATGGGACTAAATTTG
TTTTGAGGTTATCCGGCACAGGCAGTTCTGGTGCAACAATAAGATTATACGTAGAAAAGTATACTG
ATAAAAAGGAGAACTATGGCCAAACAGCTGACGTCTTCTTGAAACCCGTCATCAACTCCATTGTAA
AATTCTTAAGATTTAAAGAAATTTTAGGAACAGACGAACCAACAGTCCGCACATAG

SEQ ID NO: 80 is an amino acid sequence Phosphoglucomutase-1 (PGM1) originating from
Saccharomyces cerevisiae
MSLLIDSVPTVAYKDQKPGTSGLRKKTKVFMDEPHYTENFIQATMQSIPNGSEGTTLVVGGDGRFYND
VIMNKIAAVGAANGVRKLVIGQGGLLSTPAASHIIRTYEEKCTGGGIILTASHNPGGPENDLGIKYNLPN
GGPAPESVTNAIWEASKKLTHYKIIKNFPKLNLNKLGKNQKYGPLLVDIIDPAKAYVQFLKEIFDFDLIKS
FLAKQRKDKGWKLLFDSLNGITGPYGKAIFVDEFGLPAEEVLQNWHPLPDFGGLHPDPNLTYARTLVD
RVDREKIAFGAASDGDGDRNMIYGYGPAFVSPGDSVAIIAEYAPEIPYFAKQGIYGLARSFPTSSAIDRV
AAKKGLRCYEVPTGWKFFCALFDAKKLSICGEESFGTGSNHIREKDGLWAIIAWLNILAIYHRRNPEKE
ASIKTIQDEFWNEYGRTFFTRYDYEHIECEQAEKVVALLSEFVSRPNVCGSHFPADESLTVIDCGDFSYR
DLDGSISENQGLFVKFSNGTKFVLRLSGTGSSGATIRLYVEKYTDKKENYGQTADVFLKPVINSIVKFLR
FKEILGTDEPTVRT

SEQ ID NO: 81 is a nucleic acid sequence of UTP-glucose-1-phosphate uridylyltransferase
(UGP1) originating from Saccharomyces cerevisiae
ATGTCCACTAAGAAGCACACCAAAACACATTCCACTTATGCATTCGAGAGCAACACAAACAGCGTT
GCTGCCTCACAAATGAGAAACGCCTTAAACAAGTTGGCGGACTCTAGTAAACTTGACGATGCTGCT
CGCGCTAAGTTTGAGAACGAACTGGATTCGTTTTTCACGCTTTTCAGGAGATATTTGGTAGAGAAG
TCTTCTAGAACCACCTTGGAATGGGACAAGATCAAGTCTCCCAACCCGGATGAAGTGGTTAAGTAT
GAAATTATTTCTCAGCAGCCCGAGAATGTCTCAAACCTTTCCAAATTGGCTGTTTTGAAGTTGAACG
GTGGGCTGGGTACCTCCATGGGCTGCGTTGGCCCTAAATCTGTTATTGAAGTGAGAGAGGGAAACA
CCTTTTTGGATTTGTCTGTTCGTCAAATTGAATACTTGAACAGACAGTACGATAGCGACGTGCCATT
GTTATTGATGAATTCTTTCAACACTGACAAGGATACGGAACACTTGATTAAGAAGTATTCCGCTAA
CAGAATCAGAATCAGATCTTTCAATCAATCCAGGTTCCCAAGAGTCTACAAGGATTCTTTATTGCCT
GTCCCCACCGAATACGATTCTCCACTGGATGCTTGGTATCCACCAGGTCACGGTGATTTGTTTGAAT
CTTTACACGTATCTGGTGAACTGGATGCCTTAATTGCCCAAGGAAGAGAAATATTATTTGTTTCTAA
CGGTGACAACTTGGGTGCTACCGTCGACTTAAAAATTTTAAACCACATGATCGAGACTGGTGCCGA
ATATATAATGGAATTGACTGATAAGACCAGAGCCGATGTTAAAGGTGGTACTTTGATTTCTTACGA
TGGTCAAGTCCGTTTATTGGAAGTCGCCCAAGTTCCAAAAGAACACATTGACGAATTCAAAAATAT
CAGAAAGTTTACCAACTTCAACACGAATAACTTATGGATCAATCTGAAAGCAGTAAAGAGGTTGAT
CGAATCGAGCAATTTGGAGATGGAAATCATTCCAAACCAAAAAACTATAACAAGAGACGGTCATG
AAATTAATGTCTTACAATTAGAAACCGCTTGTGGTGCTGCTATCAGGCATTTTGATGGTGCTCACGG
TGTTGTCGTTCCAAGATCAAGATTCTTGCCTGTCAAGACCTGTTCCGATTTGTTGCTGGTTAAATCA
GATCTATTCCGTCTGGAACACGGTTCTTTGAAGTTAGACCCATCCCGTTTTGGTCCAAACCCATTAA
TCAAGTTGGGCTCGCATTTCAAAAAGGTTTCTGGTTTTAACGCAAGAATCCCTCACATCCCAAAAA
TCGTCGAGCTAGATCATTTGACCATCACTGGTAACGTCTTTTTAGGTAAAGATGTCACTTTGAGGGG
TACTGTCATCATCGTTTGCTCCGACGGTCATAAAATCGATATTCCAAACGGCTCCATATTGGAAAAT
GTTGTCGTTACTGGTAATTTGCAAATCTTGGAACATTGA

SEQ ID NO: 82 is an amino acid sequence of UTP--glucose-1-phosphate uridylyltransferase
(UGP1) originating from Saccharomyces cerevisiae
MSTKKHTKTHSTYAFESNTNSVAASQMRNALNKLADSSKLDDAARAKFENELDSFFTLFRRYLVEKSS
RTTLEWDKIKSPNPDEVVKYEIISQQPENVSNLSKLAVLKLNGGLGTSMGCVGPKSVIEVREGNTFLDLS
VRQIEYLNRQYDSDVPLLLMNSFNTDKDTEHLIKKYSANRIRIRSFNQSRFPRVYKDSLLPVPTEYDSPL
DAWYPPGHGDLFESLHVSGELDALIAQGREILFVSNGDNLGATVDLKILNHMIETGAEYIMELTDKTRA
DVKGGTLISYDGQVRLLEVAQVPKEHIDEFKNIRKFTNFNTNNLWINLKAVKRLIESSNLEMEIIPNQKTI
TRDGHEINVLQLETACGAAIRHFDGAHGVVVPRSRFLPVKTCSDLLLVKSDLFRLEHGSLKLDPSRFGP
NPLIKLGSHFKKVSGFNARIPHIPKIVELDHLTITGNVFLGKDVTLRGTVIIVCSDGHKIDIPNGSILENVV
VTGNLQILEH

SEQ ID NO: 83 is a nucleic acid sequence of Glucosamine 6-phosphate N-acetyltransferase
(GNA1) originating from Saccharomyces cerevisiae
ATGAGCTTACCCGATGGATTTTATATAAGGCGAATGGAAGAGGGGATTTGGAACAGGTCACTGA
GACGCTAAAGGTTTTGACCACCGTGGGCACTATTACCCCCGAATCCTTCAGCAAACTCATAAAATA
CTGGAATGAAGCCACAGTATGGAATGATAACGAAGATAAAAAAATAATGCAATATAACCCCATGG
TGATTGTGGACAAGCGCACCGAGACGGTTGCCGCTACGGGGAATATCATCATCGAAAGAAAGATC
ATTCATGAACTGGGGCTATGTGGCCACATCGAGGACATTGCAGTAAACTCCAAGTATCAGGGCCAA
GGTTTGGGCAAGCTCTTGATTGATCAATTGGTAACTATCGGCTTTGACTACGGTTGTTATAAGATTA
TTTTAGATTGCGATGAGAAAAATGTCAAATTCTATGAAAAATGTGGGTTTAGCAACGCAGGCGTGG
AAATGCAAATTAGAAAATAG

SEQ ID NO: 84 is an amino acid sequence of Glucosamine 6-phosphate N-acetyltransferase
(GNA1) originating from Saccharomyces cerevisiae
MSLPDGFYIRRMEEGDLEQVTETLKVLTTVGTITPESFSKLIKYWNEATVWNDNEDKKIMQYNPMVIV
DKRTETVAATGNIIIERKIIHELGLCGHIEDIAVNSKYQGQGLGKLLIDQLVTIGFDYGCYKIILDCDEKNV
KFYEKCGFSNAGVEMQIRK

SEQ ID NO: 85 is a nucleic acid sequence of phosphoacetylglucosamine mutase (PCM1)
originating from Saccharomyces cerevisiae
ATGAAGGTTGATTACGAGCAATTGTGCAAACTCTACGATGACACGTGCCGCACAAAGAATGTGCA
GTTCAGTTACGGTACGGCCGGATTCAGAACGCTGGCCAAGAATTTGGATACGGTGATGTTCAGTAC
TGGTATACTGGCGGTTCTCAGGTCGCTGAAGCTTCAGGGTCAGTATGTGGGGGTGATGATCACGGC
GTCGCACAACCCATACCAGGACAACGGGGTCAAGATCGTGGAACCAGACGGATCGATGCTTTTGG
CCACATGGGAGCCATATGCCATGCAGTTGGCCAATGCGGCCTCTTTTGCCACTAATTTTGAAGAATT
TCGTGTTGAGTTGGCCAAGCTGATTGAACACGAAAAGATTGATTTGAATACAACCGTCGTGCCTCA
CATCGTGGTTGGGAGAGACTCTAGGGAAAGTAGTCCATACTTGCTGCGCTGCTTGACTTCCTCCAT
GGCCAGCGTCTTCCACGCGCAAGTTTTGGACCTAGGCTGTGTCACTACGCCTCAATTGCATTACATT
ACTGATTTGTCCAACAGGCGGAAACTGGAAGGAGACACAGCGCCAGTTGCCACAGAACAGGACTA
CTATTCGTTCTTTATAGGAGCCTTCAACGAGCTCTTCGCCACGTATCAGCTGGAGAAGAGGCTGTCT
GTCCCAAAATTGTTCATAGACACAGCCAATGGTATCGGTGGTCCACAGTTGAAAAAACTACTGGCC
TCCGAAGATTGGGACGTGCCAGCGGAGCAAGTTGAGGTAATCAACGACAGGTCCGATGTTCCAGA
ACTGTTGAATTTTGAATGCGGTGCGGATTATGTGAAGACTAACCAGAGATTACCCAAGGGTCTTTC
TCCATCCTCGTTTGATTCGCTATATTGCTCCTTTGATGGTGACGCAGACAGGGTTGTGTTCTACTAT
GTCGACTCAGGATCAAAATTTCATTTGTTGGATGGTGACAAAATTTCCACTTTGTTTGCAAAGTTCT
TGTCTAAACAACTAGAATTGGCACACCTAGAACATTCTTTGAAGATTGGTGTTGTGCAAACTGCCT
ATGCAAACGGCAGTTCCACCGCTTACATAAAAAATACGTTGCACTGTCCCGTGTCTTGCACTAAGA
CAGGTGTTAAACACTTGCATCATGAAGCTGCCACTCAGTACGATATTGGCATTTATTTCGAAGCAA

ATGGACATGGTACGATTATATTCAGCGAAAAATTTCATCGAACTATCAAATCTGAATTATCCAAGT
CCAAGTTAAATGGTGATACGTTAGCTTTGAGAACTTTGAAGTGTTTCTCTGAATTGATTAATCAGAC
CGTGGGAGATGCTATTTCAGACATGCTTGCTGTCCTTGCTACTTTGGCGATTTTGAAAATGTCGCCA
ATGGATTGGGATGAAGAGTATACTGATTTGCCCAACAAGCTGGTTAAGTGCATCGTTCCTGATAGG
TCAATTTTCCAAACCACGGACCAGGAAAGAAAATTGCTCAATCCAGTGGGGTTGCAAGACAAGAT
AGATCTTGTGGTAGCCAAGTATCCCATGGGAAGAAGCTTTGTCAGAGCCAGTGGTACGGAGGATGC
GGTGAGGGTTTATGCGGAATGTAAGGACTCCTCTAAGTTAGGTCAATTTTGTGACGAAGTGGTGGA
GCACGTTAAGGCATCTGCTTGA

SEQ ID NO: 86 is an amino acid sequence of phosphoacetylglucosamine mutase (PCM1)
originating from Saccharomyces cerevisiae
MKVDYEQLCKLYDDTCRTKNVQFSYGTAGFRTLAKNLDTVMFSTGILAVLRSLKLQGQYVGVMITAS
HNPYQDNGVKIVEPDGSMLLATWEPYAMQLANAASFATNFEEFRVELAKLIEHEKIDLNTTVVPHIVV
GRDSRESSPYLLRCLTSSMASVFHAQVLDLGCVTTPQLHYITDLSNRRKLEGDTAPVATEQDYYSFFIGA
FNELFATYQLEKRLSVPKLFIDTANGIGGPQLKKLLASEDWDVPAEQVEVINDRSDVPELLNFECGADY
VKTNQRLPKGLSPSSFDSLYCSFDGDADRVVFYYVDSGSKFHLLDGDKISTLFAKFLSKQLELAHLEHSL
KIGVVQTAYANGSSTAYIKNTLHCPVSCTKTGVKHLHHEAATQYDIGIYFEANGHGTIIFSEKFHRTIKSE
LSKSKLNGDTLALRTLKCFSELINQTVGDAISDMLAVLATLAILKMSPMDWDEEYTDLPNKLVKCIVPD
RSIFQTTDQERKLLNPVGLQDKIDLVVAKYPMGRSFVRASGTEDAVRVYAECKDSSKLGQFCDEVVEH
VKASA

SEQ ID NO: 87 is the nucleic acid sequence of promoter pTDH3
CTGCTGTAACCCGTACATGCCCAAAATAGGGGGCGGGTTACACAGAATATATAACATCGTAGGTGT
CTGGGTGAACAGTTTATTCCTGGCATCCACTAAATATAATGGAGCCCGCTTTTTAAGCTGGCATCCA
GAAAAAAAAAGAATCCCAGCACCAAAATATTGTTTTCTTCACCAACCATCAGTTCATAGGTCCATT
CTCTTAGCGCAACTACAGAGAACAGGGGCACAAACAGGCAAAAAACGGGCACAACCTCAATGGAG
TGATGCAACCTGCCTGGAGTAAATGATGACACAAGGCAATTGACCCACGCATGTATCTATCTCATT
TTCTTACACCTTCTATTACCTTCTGCTCTCTCTGATTTGGAAAAAGCTGAAAAAAAAGGTTGAAACC
AGTTCCCTGAAATTATTCCCCTACTTGACTAATAAGTATATAAAGACGGTAGGTATTGATTGTAATT
CTGTAAATCTATTTCTTAAACTTCTTAAATTCTACTTTTATAGTTAGTCTTTTTTTTAGTTTTAAAACA
CCAAGAACTTAGTTTCGAATAAACACACATAAACAAACAAA

SEQ ID NO: 88 is the nucleic acid sequence of promoter pTDH3.Sk
CAATTCATCGGCCCTTTTAGCGGCTACCCGCGCCATCTAAATGATAGGGCGGGTGACACTATGGTA
AATCCCATAATTAGGTGTCTGGGTGAGTGGTTCTGATGCCGGCATCCACTAAATATATTGGAGCCC
ATTTTTTACGCGGGCTTCCAGAAAAAAAGAGAATCCCAGCACCAAAAGGTGGTTCTCTTCACCAAC
CATCAGATCATAGGTCCACAACCACACATAACAGGGGCACAAAAAGGCAAAAAACGGACATAACC

TCAATGGAGTGATGCAAATTGACTGGAGCAAAAGCTGACACAAGGCATTGATTGACCTACGCATGT
ATCTGTATTCTTTTCTTACACCTTCTATTACCTTCTAACTCTTTGGGTTGGAAAAAACTGAAAAAA
AGGTTGGGACCTGGTTCCCCCAAGTTGTCCCCCTACTTGGTTATTAAATATATAAAGACAGCAAGT
GTTGATTATAATCTTGTAAATCTATAGTTCTTAATCTATACTTCTATTTATATTTTAAATTAGTCTTTT
TATTTCCAAGTCCCCAAGAACTTAGTTTCGAATAAACACACACAAATAAACACA

SEQ ID NO: 89 is the nucleic acid sequence of promoter pTDH3-1.sba
GCAGCGCTTCTTCCGCTCTAGTTTTATAGTTATTATTACTACCACCTTAAAAATACGTAAATACTC
AAAATAGTAGTGATATTCCCAACCTTATTCATCCAAGGCACATCATCATCATCAGCCATTCATCTTT
CACCTGCCATTAGTAACCCGTCTTCTCATTGAGCGGGTTACGGCAGCCACAGGCCACATTCCGAAT
GTCTGGGTGAGCGGTCCCTTTTCCAGCATCCACTAAATATCTCCGATCCCGCTTTTTAATCTGGCTT
CCTGAAAAAAAGAGAATCCCAGCACCAAAAAATGGCTCTCTTCACCAACCATCAGATCATAGGTCC
CATTCTCTTACCGCAACCGTACAGAACAGGGGAAAACGGGTACAACCTCAATGGAGTGATGCAAA
CTGACTGGAGCAAAAAGCTGACACAAGGCAATCGACCTACGTGTCTGTCTATTTTCTCACACCTTCT
ATTACCTTCTAACTCTCTGGGTTGGAAAAAACTGAAAAAAAGGTTGAGACCAGTTTCCACAAATCA
TCCCCCTGTTTGATTAATAAATATATAAAGACGACAACTATCGATCATAAACTCATAAAACTATAA
CTCCTTTACACTTCTTATTTTATAGTTATTCTATTTTAATTCTTATTGATTTTAAAACCCCAAGAACTT
AGTTTCGAAAACACACACACACAAACAATTAAAA

SEQ ID NO: 90 is the nucleic acid sequence of promoter pTDH3.Sar
GAGCTAAATATCAGCCCTTCGGGTCCTGCCTGCTACCCGGTCCTGTTCGAATAAAAACGCGGGTAA
CACGACCCAGTAACACCTGTCGTTGGGTGTCTGGGTCAGAAGTTCTGATACCGGCTTCCACTAAAT
AGATTGGGTTCCGCTCTTTACGCTGGCTTCCTGAAAAAGAGATTCCGGGCACCAAAAATTGGTC
TCTTTGCCAACCATCAGATCATAGGTCCATTCTCTTACCATAACCACACAGGATAGGGGCACCACA
GGCGAAAATGGGCACAAAATCTCAATGGAGTGATGCAAATTAGCTGGAACAAAAGCTGACACAAG
GCAATTAACCTGCGCATGTATCCATCTCCTTTTCTTACACCTTCTCTTACGTTCTAACTGTTTGGGTT
GGAAAAATTAAAAAAAAAAGGTTGAGACCAGTTTCCCCAAATCGTCCCCCTACTTGATTCATAAAT
ATATAAAGACGACAACTATTGATTATAATCTTGTAAATCTATAACTCTTTACTTTCTCCTATTTATA
ATTTAACTTAATCTTTTTAGATTTAAAACCCCAAGAACTTAGTTTCGAACAAACACACACAAATAA
ACAAAA

SEQ ID NO: 91 is the nucleic acid sequence of promoter pENO2
CGCTCAGCATCTGCTTCTTCCCAAAGATGAACGCGGCGTTATGTCACTAACGACGTGCACCAACTT
GCGGAAAGTGGAATCCCGTTCCAAAACTGGCATCCACTAATTGATACATCTACACACCGCACGCCT
TTTTTCTGAAGCCCACTTTCGTGGACTTTGCCATATGCAAAATTCATGAAGTGTGATACCAAGTCAG
CATACACCTCACTAGGGTAGTTTCTTTGGTTGTATTGATCATTTGGTTCATCGTGGTTCATTAATTTT
TTTTCTCCATTGCTTTCTGGCTTTGATCTTACTATCATTTGGATTTTTGTCGAAGGTTGTAGAATTGT

ATGTGACAAGTGGCACCAAGCATATATAAAAAAAAAAAGCATTATCTTCCTACCAGAGTTGATTGT
TAAAAACGTATTTATAGCAAACGCAATTGTAATTAATTCTTATTTGTATCTTTTCTTCCCTTGTCTC
AATCTTTTATTTTTATTTTATTTTTCTTTTCTTAGTTTCTTTCATAACACCAAGCAACTAATACTATAA
CATACAATAATA

SEQ ID NO: 92 is the nucleic acid sequence of promoter pTEF3
GGCTGATAATAGCGTATAAACAATGCATACTTTGTACGTTCAAAATACAATGCAGTAGATATATTT
ATGCATATTACATATAATACATATCACATAGGAAGCAACAGGCGCGTTGGACTTTTAATTTTCGAG
GACCGCGAATCCTTACATCACACCCAATCCCCCACAAGTGATCCCCCACACACCATAGCTTCAAAA
TGTTTCTACTCCTTTTTTACTCTTCCAGATTTTCTCGGACTCCGCGCATCGCCGTACCACTTCAAAAC
ACCCAAGCACAGCATACTAAATTTCCCCTCTTTCTTCCTCTAGGGTGTCGTTAATTACCCGTACTAA
AGGTTTGGAAAAGAAAAAAGAGACCGCCTCGTTTCTTTTTCTTCGTCGAAAAAGGCAATAAAAATT
TTTATCACGTTTCTTTTTCTTGAAAATTTTTTTTTTTGATTTTTTTCTCTTTCGATGACCTCCCATTGAT
ATTTAAGTTAATAAACGGTCTTCAATTTCTCAAGTTTCAGTTTCATTTTTCTTGTTCTATTACAACTT
TTTTTACTTCTTGCTCATTAGAAAGAAAGCATAGCAATCTAATCTAAGTTTTAATTACAAA

SEQ ID NO: 93 is the nucleic acid sequence of promoter pTEF1
GTTTAGCTTGCCTCGTCCCCGCCGGGTCACCCGGCCAGCGACATGGAGGCCCAGAATACCCTCCTT
GACAGTCTTGACGTGCGCAGCTCAGGGGCATGATGTGACTGTCGCCCGTACATTTAGCCCATACAT
CCCCATGTATAATCATTTGCATCCATACATTTTGATGGCCGCACGGCGCGAAGCAAAAATTACGGC
TCCTCGCTGCAGACCTGCGAGCAGGGAAACGCTCCCCTCACAGACGCGTTGAATTGTCCCCACGCC
GCGCCCCTGTAGAGAAATATAAAAGGTTAGGATTTGCCACTGAGGTTCTTCTTTCATATACTTCCTT
TTAAAATCTTGCTACGATACAGTTCTCACATCACATCCGAACATAAACAACC

SEQ ID NO: 94 is the nucleic acid sequence of promoter pTEF1.ago
GTTTAGCTTGCCTCGTCCCCGCCGGGTCACCCGGCCAGCGACATGGAGGCCCAGAATACCCTCCTT
GACAGTCTTGACGTGCGCAGCTCAGGGGCATGATGTGACTGTCGCCCGTACATTTAGCCCATACAT
CCCCATGTATAATCATTTGCATCCATACATTTTGATGGCCGCACGGCGCGAAGCAAAAATTACGGC
TCCTCGCTGCAGACCTGCGAGCAGGGAAACGCTCCCCTCACAGACGCGTTGAATTGTCCCCACGCC
GCGCCCCTGTAGAGAAATATAAAAGGTTAGGATTTGCCACTGAGGTTCTTCTTTCATATACTTCCTT
TTAAAATCTTGCTACGATACAGTTCTCACATCACATCCGAACATAAACAACC

SEQ ID NO: 95 is the nucleic acid sequence of promoter pTEF1.Sba
CGCCAACAAACCTTCGAACACTTTAATTTTCGAGGACCGCAGATCCTCACATCACACCCACACCCA
AGACTGCTTCCCCCACACACCCTGCATCTGTACACTTTCTTCTGCTCTGTTTTCTCTCCGGCGTTCT
CTCGGGTCGCCCGCATCGCCGCGCCGGCTGGAACCGCCCACGCACCGCATATTGCAAATCGCCTGC
CCCCTCTTGCTCCTTTTTGAGGGCGCGCCGTTACCCGCGCCCAGGGTCCGGAAAAAGAAACAAGGC

TCTACCGCGTTTCTTTTTCCTTGTCGAAAAAGGCAAAAATGAAAATTTTTATCACGTTTCTTTTTTTT
TGAAAAATTTTTTTTTTGGTTTTTTTTCTTTCGATGGCCTCCCATTGATATTTAAGTTAATAAATGGT
TTTCAGTTTTCAAGTTTCAGTTTGTGTTCTTCTTTGCTAACTTTCACTTACACCTCGAAAGAAAGTAT
AGCAATCTAATCTTAGTTTTAATTACAA

SEQ ID NO: 96 is the nucleic acid sequence of promoter pPDC1
TTATTTACCTATCTCTAAACTTCAACACCTTATATCATAACTAATATTTCTTGAGATAAGCACACTG
CACCCATACCTTCCTTAAAAACGTAGCTTCCAGTTTTTGGTGGTTCCGGCTTCCTTCCCGATTCCGCC
CGCTAAACGCATATTTTTGTTGCCTGGTGGCATTTGCAAAATGCATAACCTATGCATTTAAAAGATT
ATGTATGCTCTTCTGACTTTTCGTGTGATGAGGCTCGTGGAAAAAATGAATAATTTATGAATTTGAG
AACAATTTTGTGTTGTTACGGTATTTTACTATGGAATAATCAATCAATTGAGGATTTTATGCAAATA
TCGTTTGAATATTTTTCCGACCCTTTGAGTACTTTTCTTCATAATTGCATAATATTGTCCGCTGCCCC
TTTTTCTGTTAGACGGTGTCTTGATCTACTTGCTATCGTTCAACACCACCTTATTTTCTAACTATTTTT
TTTTTAGCTCATTTGAATCAGCTTATGGTGATGGCACATTTTTGCATAAACCTAGCTGTCCTCGTTG
AACATAGGAAAAAAAAATATATAAACAAGGCTCTTTCACTCTCCTTGCAATCAGATTTGGGTTTGT
TCCCTTTATTTTCATATTTCTTGTCATATTCCTTTCTCAATTATTATTTTCTACTCATAACCTCACGCA
AAATAACACAGTCAAATCAATCAAA

SEQ ID NO: 97 is the nucleic acid sequence of promoter pCCW12
aaccagggcaaagcaaaataaaagAAACTTAATACGTTATGCCGTAATGAAGGGCTACCAAAAACGATAATCTC
AACTGTAAACAGGTACAATGCGGACCCTTTTGCCACAAAACATACATCATTCATTGCCGGAAAAAG
AAAGAAGTGAAGACAGCAGTGCAGCCAGCCATGTTGCGCCAATCTAATTATAGATGCTGGTGCCCT
GAGGATGTATCTGGAGCCAGCCATGGCATCATGCGCTACCGCCGGATGTAAAATCCGACACGCAA
AAGAAAACCTTCGAGGTTGCGCACTTCGCCCACCCATGAACCACACGGTTAGTCCAAAAGGGGCA
GTTCAGATTCCAGATGCGGGAATTAGCTTGCTGCCACCCTCACCTCACTAACGCTGCGGTGTGCGG
ATACTTCATGCTATTTATAGACGCGCGTGTCGGAATCAGCACGCGCAAGAACCAAATGGGAAAATC
GGAATGGGTCCAGAACTGCTTTGAGTGCTGGCTATTGGCGTCTGATTTCCGTTTTGGGAATCCTTTG
CCGCGCGCCCCTCTCAAAACTCCGCACAAGTCCCAGAAAGCGGGAAAGAAATAAAACGCCACCAA
AAAAAAAAATAAAAGCCAATCCTCGAAGCGTGGGTGGTAGGCCCTGGATTATCCCGTACAAGTAT
TTCTCAGGAGTAAAAAAACCGTTTGTTTTGGAATTCCCCATTTCGCGGCCACCTACGCCGCTATCTT
TGCAACAACTATCTGCGATAACTCAGCAAATTTTGCATATTCGTGTTGCAGTATTGCGATAATGGG
AGTCTTACTTCCAACATAACGGCAGAAAGAAATGTGAGAAAATTTTGCATCCTTTGCCTCCGTTCA
AGTATATAAAGTCGGCATGCTTGATAATCTTTCTTTCCATCCTACATTGTTCTAATTATTCTTATTCT
CCTTTATTCTTTCCTAACATACCAAGAAATTAATCTTctgtcattcgcttaaacactatatcaata

SEQ ID NO: 98 is the nucleic acid sequence of promoter pCCW12.Sm
CGTTGTGCTGTAGTGAAGGAAGACTAAAAAGGATAATCACAGTTGTAAAGAGGTATAATGCGGAC
CCTTTTGCCACAAAACACACATCTTTCGTTTCCAAAATAAGAAAGAAGAAAGCAAAAAGATTAGCA
GCCACGTTGCTGCGATCTAATTATAGACGGTGGCGTCATCATTCTCACCCAAGATTGTGTCTTGAAC
CTGCCACGGGTCCTGCGTTATCGCCGGATGTAAAACTAGACATGCAAAAAAAGGACCTTCCAGGTA
GCGTGCTCCACACCACCCATGACCACCACAGTTAGTCCAAAAGAGGCAGCACCACTTCCCGATGGG
GGAATTAGATTGCTGCCACCCTCACCTCACTAATGCTGCGGTGTGCGGATATGCCCTGCTATATATA
GCTCCGCGTTTTTGAACCAGCACAGCGCGAGCACCAAAAAGGAAAATCGCATAGGCCCAGAACTG
ATTTCAGCACGGGCTATTGGCGTTGGGTTTCCGTTCTGGGAAACCTTCGCCGCGTCCCCCTCACAAA
CCTCCGCACAAGTTCGAGCAAGCGGAAAAAACGAAAAACGCCATTAATACTAAATAAAGCAAAT
CCTCGAAGCGTGGGTGGCAAGCCCCTGGATTTTTCCGCACAAGTACTCTTCTCAGGAGTAAAAAA
CCCGTTTGTTTTGGAATTCCCCATTTCGCGGCCATCTACGCCGGTATCTTCGCAATATCTATCAGCG
ATAACTCAGCAATTTTAATATTCGTGTTGCAGTGCTGCGATAGCGGGAGTCTTGTTTGTAACATAAC
GGCAGAAAGGAATGAGAGAAAATTTTCCATTCTTTGGCCTCCGTTCAAGTATATAAAGCCGGCATG
CTTGGTAATCTTTCTCTCTCTTCTGTATTGTTTCTATAATACTTTTATCTTCTAATTATTTTCTGAAAA
AACCAAGAAATTAATCTTCTGTCATTCGCTTAAACACTATATCAATA

SEQ ID NO: 99 is the nucleic acid sequence of promoter pCCW12.Sk
TGTAAAATCCTACACGCAAAAAACCTCTTGGGTTGCGCGTTTTCCACCACCCACGACCCGCACAAT
CAATCCAAAAGGGGCAACGCCAGTTCCCAATGCGGGAATTAGCTCCTCACCCCCCTCACCCGCTAA
CGCTGCGGTGTGCGGACACGCAGCAGTATTTATAGATCCTCGTGTTGGAACCAGCCCGCGTGAGCA
CCAAATTGGAAAGTCGCAATGGGCCCAGAACCGCTTTCAGTACTGGGCCATTGGCGTCTAGTTTCC
GTTTTGAGCGTCCTTCGCCGCGTCTCTCTGTGAAATCCCCGCACAAGTCTGAGCAGGCAAAAAAAA
AAAACGCCACCAAAAATAAATAAAGCCAATCCTCGAAGGATGAGTAGGAAAGGAAGCCCCTGGTT
TTTTCCCGCACGAATATTTTTCAGGAGTGAAAAAATCCGTTTGTTTTGGAATTCCCCATTTCGCGCT
CACCTACGCCGGTATCTTTGGAACAACTATCAGCGATAACTCAGCAAAATTTGCATATCCGTGCTG
CAATAGTGCGATAGTGGGATTGGAGTCTTGTTGCATCATAACGGCAGAAAGGAATGAATAAAAA
TTTTCCGTTCTTTGTCTCCGTTCAAGTATATAAAGTCGGCATGCTTGATTATCTTTTCTTCTCTTCTAC
TACATTTCTATTTCACTTTCTACTCTATTCTTCCCTGAAAAACCCAAGAAAATAATCTTCTGTCATTC
GCTTAAACAATATATCAAAA

SEQ ID NO: 100 is the nucleic acid sequence of promoter pCCW12.Sba
ACCATGCCACGGTGCTGGCCCACTTCCACCCACGACCCGCACGGTTGGCCCGAAAGGGGCAACAC
CAGTTCCCAATACGGAAATTAGTCCGCCACCACCCTCACCTCGCTAGAGCTGCGGTGTGCGGGCGT
GCATCGCTATTTATAGACATGCCTGCTGGCGTCACCGCGCGCGAGCACCAAACAGGAAAATCGCAC
TGGGCCCAGAACCACGCTATGCGCTGGGCCGATGGCGTCCGGTTTCCCTTTGGGAGCCCCCTGCCA
CGTTCGCCTAACAAATCCCCGCACCGGCTTGAGAAAAAGCGAAAAGCGAAAAAAAAAAATCAAC

GCCACCAAAATTAAAAAAAAAGAGCCATCCTCGAAGGGTGAATAGTAGCCCCTGACTTTTCCCGCA
CAGACAGACACCTTTCAGGAGTGAACAAAAAAGCAGTTTGTTTTGGAATTCCCCCATTTCGCGGTG
GCCTGCGCAGGTATCTCTGCGTCAACTATCAGCGATAACTCAGCAAATTTTGCATATTCGTGTTGCG
ATACTACGATAATGGGAGTCTGTCGCCTAATAACGGCAACAAGGAATGAGAGAGAAAAATTTTCTT
CATTCTCCAGCTCCCGTTCAAGTATATAAGGTCGGCATGGTCGATTGTCTTTCCTTCTCTTCAGTTAC
GTCTCTCTATTTACATTATTCTTATTTTTATTTAATAAAAACCCCAAGAAATTAATCTTCTGTCATTC
GCTTAAACACTATATCAAAA

SEQ ID NO: 101 is the nucleic acid sequence of promoter pCCW12.Sar
AAAAAACAACCTTCTGCCAACCTGCGTGCTTCTCACCACCCATGACCCACACAATTGACCCGAATG
GGGCAACTCCAGTTCCCAATACGGAATTAACTCGCCACCATATTTACCGCGTTGAAGCTGTGGTG
TGCGGACACTCCGTACTATTTATAGACCCACGCGGTGGAACCAGCACGCGCGCACTAAACAGG
AAAATCGCATTGAGTCCAGAACCGCCACCAGCACTTGGCCATTGGCGTCTAATTTCCGTTTTCGGC
GCCCCTCACCGCGTCCTTCTAACAAAGCGCGCACAAGCTTGAGCAAGTGAAAAGAAAATTAAAAA
TAAAAAACCGCCACCAAAACAAATAAAGCAATCTTCGAAGTGTGGGTTGGTGGAAGCCCCTGGC
TTTTCCCGCACCAGTCGTTTTCAGGAGTAAAAAAATACCCGTTTGTTTTGGAATTCCCCATTTCGCG
GCGACCTGCGCCGGTATCTTTGCAACAACTATTTGCGATAACTCAGCAAAATTTGCATATTCGTGTT
GGGATATTGCGATAGTGGGAGTCTTGTTGCATAATAACGGTAAAAAGAAGTGAAGGAAAAAAATT
TGCATCCTTTAGTCTCAGTTCAAGTATATAAAGTCGGGATATTCAATTATCTTTCTTTCTCTTGCTCA
AAGGTTTCTATATTTTTTTATAGTATTTCTTTTGTTATAAAATACCAAGAAATTAATCTTCTGTCAT
TCGCTTAAATACTACATCAATA

SEQ ID NO: 102 is the nucleic acid sequence of promoter pNUP57
TCATCTGCGCAATGACTATCAAGACCTTCTGCAAGAATTTCAAATCTCACTGAAAATCTTGACCGA
AAAGTGTCTTGAAAACCCATCAAGCCTGCAAAACCTATCTTTGACATTAGTCTCCATTATAAAAAC
GGCATAGTTGGGAGAAAACTTTCCATACTTCAATTGTGGACTGATATAAGTATTTTAGTTTTGCCCG
CATGATCATCCCACATGGCTACAGCAGTTCTCTCATAGGAAAATAGTACAATAGCTACGTGATATA
ATCTAAATAATTGTTGCCAATGTGTAATTATATCATTTTGAACGTACGCGAAATGGATTATTTTCAA
AAATTTTGTTTCTTGAAATGAGTAAAAGCAAAAGTCCAACTCTCCAAGTCGATGTAAACAACTTTTT
GCCAAAGGGACTGAAAGACTAAATCGAGGATTATCCCGTTCAAACTATTCCAGAAACGCTCGTTAG
TAACAAAAGACATACCTTGTTGACCAATTGATCAC

SEQ ID NO: 103 is the nucleic acid sequence of promoter pCCW10.ago
GGTACCACGGCAACCTCGTTCGCTGTTCATCCCCTTCGTCACACAGGACGTTGGATGCCGTAAGCA
GCGTTGCTTTTGATCCTCagGATCGGCCGGGTAACCCGCGGCtGCTTCTATTTAGTATTCATATCTCA
AGCACATCCATTCCGGCCGTTTGGGGGCGCCGCCGCACTCGTGTCCATTCCTACCGTGGCACTTAG
GGCTATCCTGTCGGAGCGCCCCGCCGACCGCCTTATCGGCACCAAAAGTAGAAGCCCCGGCCCCGC

GTGGCTCAGACTCACCATCGGTGCTATTTACTTTTCGATCAGATCGCGGCGCGCGGTGGCCGGCATT
TCCGGAAGCGGCCACGGAGCAGAGGTGGCGCATTCGAATCGCATACGTCTTCGCCACGCCGGAAA
AAAAATTTTCGGCTATATAAGGAGAGGCGGCCGTCTTGCTGCAGGCAGTTTCACTTTCTCTAAAAC
CAAAGAACATCGATTTCTTTAGTCACTCGCTTCCTTACACCGAACTCGAGGCGGCCGC

SEQ ID NO: 104 is the nucleic acid sequence of promoter pCWP2
CTAGCCTCCCCTTTTTATTTTGTGCGGTCACCGCAAGGGACAAAGCTTTTCTTAGAAAACCGTCTGA
GAAGCATAACGTACGCCATCCCCTAGACATATTAATAATGCTACAGATACTATGCTGCTCGTCTTTT
TTTGACGACCCTTTTATTGCAATGTGCAACTAATGGCAAACAACCACATAGTATCACAGTATTACAT
TGCCTCCACCGATGCGGATGTTAGGGCGCCAAGTCTGTCATGAAGCATGTTCCTGTCATAATCTTGT
ATGCAAAATACCGCGTTCTGCGCCACTGATATGCTAGGCAGCAGCAACCTATGCAGAAGATTGCTT
TTCCCACGCCTGTTTTACGTCTCCAGGGCACTTGAAACAATGCAGCGATCGCCGCCACAACACGCC
AAAGAGAAGCGAAAGTGGGCCTGGGCGGCCTCAGTTTCGGCAGAGGTAAACAACACGAACTGAAC
TGCCTTAGCTCCGAAGGGCAATTCCACAGGCACTCCGCGGGGCCCGGCCAAGGCCCAAAAGGCGT
GGAATATGCGCGTTTTGGGGCCATAACACCCAGTACCACGGCCGGAACGGGCCATATAATAAGTTT
TTCACTCTCAAGAATGGTAAACGTAAATAGGAACATCCCACTACCCTAGAAATTGCGGAAATTTCG
CGCTTATCATTAGAAAATCTGGAACCGTCCTTTTTCCTCTTTCTTGCATTTCCCTTTCCGTATTATTG
CCATTCTTTAACTGCATTTGGGGAACCGTAGACCAAAAGCCAAACAGAGAAATGTAACGTTCTAAA
AAAAAAACAACGAAAAAATTGAAAAATAAGATACAATAATCGTATATAAATCAGGCTTCTTGTTC
ATCATTTTCAATTCTCTTCTTGCCATCCCTTTTCCTATCTTTGTTCTTTTCTTCTCATAATCAAGAATA
AATAACTTCATCA

SEQ ID NO: 105 is the nucleic acid sequence of promoter pFBA1
ACGCAAGCCCTAAGAAATGAATAACAATACTGACAGTACTAAATAATTGCCTACTTGGCTTCACAT
ACGTTGCATACGTCGATATAGATAATAATGATAATGACAGCAGGATTATCGTAATACGTAATAGTT
GAAAATCTCAAAAATGTGTGGGTCATTACGTAAATAATGATAGGAATGGGATTCTTCTATTTTTCCT
TTTTCCATTCTAGCAGCCGTCGGGAAAACGTGGCATCCTCTCTTTCGGGCTCAATTGGAGTCACGCT
GCCGTGAGCATCCTCTCTTTCCATATCTAACAACTGAGCACGTAACCAATGGAAAAGCATGAGCTT
AGCGTTGCTCCAAAAAAGTATTGGATGGTTAATACCATTTGTCTGTTCTCTTCTGACTTTGACTCCT
CAAAAAAAAAAAATCTACAATCAACAGATCGCTTCAATTACGCCCTCACAAAAACTTTTTTCCTTC
TTCTTCGCCCACGTTAAATTTTATCCCTCATGTTGTCTAACGGATTTCTGCACTTGATTTATTATAAA
AAGACAAAGACATAATACTTCTCTATCAATTTCAGTTATTGTTCTTCCTTGCGTTATTCTTCTGTTCT
TCTTTTTCTTTTGTCATATATAACCATAACCAAGTAATACATATTCAAA

SEQ ID NO: 106 is the nucleic acid sequence of promoter pCCW120.Sm
CGTTGTGCTGTAGTGAAGGAAGACTAAAAAGGATAATCACAGTTGTAAAGAGGTATAATGCGGAC
CCTTTTGCCACAAAACACACATCTTTCGTTTCCAAAATAAGAAAGAAGAAAGCAAAAAGATTAGCA
GCCACGTTGCTGCGATCTAATTATAGACGGTGGCGTCATCATTCTCACCCAAGATTGTGTCTTGAAC
CTGCCACGGGTCCTGCGTTATCGCCGGATGTAAAACTAGACATGCAAAAAAAGGACCTTCCAGGTA
GCGTGCTCCACACCACCCATGACCACCACAGTTAGTCCAAAAGAGGCAGCACCACTTCCCGATGGG

GGAATTAGATTGCTGCCACCCTCACCTCACTAATGCTGCGGTGTGCGGATATGCCCTGCTATATATA
GCTCCGCGTTTTTGAACCAGCACAGCGCGAGCACCAAAAAGGAAAATCGCATAGGCCCAGAACTG
ATTTCAGCACGGGCTATTGGCGTTGGGTTTCCGTTCTGGGAAACCTTCGCCGCGTCCCCCTCACAAA
CCTCCGCACAAGTTCGAGCAAGCGGAAAAAACGAAAAACGCCATTAATACTAAATAAAGCAAAT
CCTCGAAGCGTGGGTGGCAAGCCCCTGGATTTTCCGCACAAGTACTCTTCTCAGGAGTAAAAAA
CCCGTTTGTTTTGGAATTCCCCATTTCGCGGCCATCTACGCCGGTATCTTCGCAATATCTATCAGCG
ATAACTCAGCAATTTTAATATTCGTGTTGCAGTGCTGCGATAGCGGGAGTCTTGTTTGTAACATAAC
GGCAGAAAGGAATGAGAGAAAATTTTCCATTCTTTGGCCTCCGTTCAAGTATATAAAGCCGGCATG
CTTGGTAATCTTTCTCTCTCTTCTGTATTGTTTCTATAATACTTTTATCTTCTAATTATTTTCTGAAAA
AACCAAGAAATTAATCTTCTGTCATTCGCTTAAACACTATATCAATA
CTCGAAATCCAAGGTGGTGT

SEQ ID NO: 107 is the nucleic acid sequence of promoter pCUP1
CGGCAAACTTCAACGATTTCTATGATGCATTTTATAATTAGTAAGCCGATCCCATTACCGACATTTG
GGCGCTATACGTGCATATGTTCATGTATGTATCTGTATTTAAAACACTTTTGTATTATTTTTCCTCAT
ATATGTGTATAGGTTTATACGGATGATTTAATTATTACTTCACCACCCTTTATTTCAGGCTGATATCT
TAGCCTTGTTACTAGTTAGAAAAAGACATTTTTGCTGTCAGTCACTGTCAAGAGATTCTTTTGCTGG
CATTTCTTCTAGAAGCAAAAAGAGCGATGCGTCTTTTCCGCTGAACCGTTCCAGCAAAAAAGACTA
CCAACGCAATATGGATTGTCAGAATCATATAAAAGAGAAGCAAATAACTCCTTGTCTTGTATCAAT
TGCATTATAATATCTTCTTGTTAGTGCAATATCATATAGAAGTCATCGAAATAGATATTAAGAAAA
ACAAACTGTACAATCAATCAATCAATCATCACATAAA

SEQ ID NO: 108 is the nucleic acid sequence of promoter pMET6
CCACAGGAAATATTTCACGTGACTTACAAACAGAGTCGTACGTCAGGACCGGAGTCAGGTGAAAA
AATGTGGGCCGGTAAAGGGAAAAACCAGAAACGGGACTACTATCGAACTCGTTTAGTCGCGAAC
GTGCAAAAGGCCAATATTTTTCGCTAGAGTCATCGCAGTCATGGCAGCTCTTTCGCTCTATCTCCCG
GTCGCAAAACTGTGGTAGTCATAGCTCGTTCTGCTCAATTGAGAACTGTGAATGTGAATATGGAAC
AAATGCGATAGATGCACTAATTTAAGGGAAGCTAGCTAGTTTTCCCAACTGCGAAAGAAAAAAAG
GAAAGAAAAAAAAATTCTATATAAGTGATAGATATTTCCATCTTTACTAGCATTAGTTTCTCTTTTA
CGTATTCAATATTTTTGTTAAACTCTTCCTTTATCATAAAAAAGCAAGCATCTAAGAGCATTGACAA
CACTCTAAGAAACAAAATACCAATATAATTTCAAAGTACATATCAAAA

SEQ ID NO: 109 is the nucleic acid sequence of promoter pMET25
TTACATTATCAATCCTTGCGTTTCAGCTTCCACTAATTTAGATGACTATTTCTCATCATTTGCGTCAT
CTTCTAACACCGTATATGATAATATACTAGTAACGTAAATACTAGTTAGTAGATGATAGTTGATTTT
TATTCCAACACTAAGAAATAATTTCGCCATTTCTTGAATGTATTTAAAGATATTTAATGCTATAATA
GACATTTAAATCCAATTCTTCCAACATACAATGGGAGTTTGGCCGAGTGGTTTAAGGCGTCAGATT
TAGGTGGATTTAACCTCTAAAATCTCTGATATCTTCGGATGCAAGGGTTCGAATCCCTTAGCTCTCA
TTATTTTTTGCTTTTTCTCTTGAGGTCACATGATCGCAAAATGGCAAATGGCACGTGAAGCTGTCGA

TATTGGGGAACTGTGGTGGTTGGCAAATGACTAATTAAGTTAGTCAAGGCGCCATCCTCATGAAAA
CTGTGTAACATAATAACCGAAGTGTCGAAAAGGTGGCACCTTGTCCAATTGAACACGCTCGATGAA
AAAAATAAGATATATATAAGGTTAAGTAAAGCGTCTGTTAGAAAGGAAGTTTTTCCTTTTTCTTGCT
CTCTTGTCTTTTCATCTACTATTTCCTTCGTGTAATACAGGGTCGTCAGATACATAGATACAATTCTA
TTACCCCCATCCATACA

SEQ ID NO: 110 is the nucleic acid sequence of promoter pSAM1
GAAACGGACGTAAGACGGAAATAGAATTTGAAGATAAAGTTATATATCACTACACACGAATACTT
TCTTTTTTTTTTTCACAGGAAAACTGTGGTGGCGCCCTTGCCTACTAGTGCATTTCTTTTTTCGGGT
TCTTGTCTCGAcGAAATTTTAGCCTCATCGTAGTTTTTCACTCTGGTATCGATGAAAAAGGGAAGAG
TAAAAAGTTTTCCGTTTAGTACTTAATGGGATTGGTTTGGGACGTATATATCGACTGGTGTTGTCTG
TTATTCATCGTTGTTTTTCGGTTAGCTTCGAAAAAAAAATAGAGTAAAAACCAGGAATTTACCCTA
AAAACAAGAAAAAATAAGATAAACGAAAAT

SEQ ID NO: 111 is the nucleic acid sequence of terminator tTPI1
GATTAATATAATTATATAAAAATATTATCTTCTTTTCTTTATATCTAGTGTTATGTAAAATAAATTGA
TGACTACGGAAAGCTTTTTTATATTGTTTCTTTTTCATTCTGAGCCACTTAAATTTCGTGAATGTTCT
TGTAAGGGACGGTAGATTTACAAGTGATACAACAAAAAGCAAGGCGCTTTTTCTAATAAAAAGAA
GAAAAGCATTTAACAATTGAACACCTCTATATCAACGAAGAATATTACTTTGTCTCTAAATCCTTGT
AAAATGTGTACGATCTCTATATGGGTTACTCA

SEQ ID NO: 112 is the nucleic acid sequence of terminator tMET25
GTGTGCGTAATGAGTTGTAAAATTATGTATAAACCTACTTTCTCTCACAAGTACTATACTTTTATAA
AACGAACTTTATTGAAATGAATATCCTTTTTTTCCCTTGTTACATGTCGTGACTCGTACTTTGAACCT
AAATTGTTCTAACATCAAAGAACAGTGTTAATTCGCAGTCGAGAAGAAAAATATGGTGAACAAGA
CTCATCTACTTCATGAGACTACTTTACGCCTCCTATAAAGCTGTCACACTGGATAAATTTATTGTAG
GACCAAGTTACAAAAGAGGATGATGGAGGTTT

SEQ ID NO: 113 is the nucleic acid sequence of terminator tDIT1
TAAAGTAAGAGCGCTACATTGGTCTACCTTTTTGTTCTTTTACTTAAACATTAGTTAGTTCGTTTTCT
TTTTCTCATTTTTTTATGTTTCCCCCCCAAAGTTCTGATTTTATAATATTTTATTTCACACAATTCCAT
TTAACAGAGGGGGAATAGATTCTTTAGCTTAGAAAATTAGTGATCAATATATATTTGCCTTTCTTTT
CATCTTTTCAGTGATATTAATGGTTTCGAGACACTGCAATGGCCCTAGTTGTCTAAGAGGATAGATG
TTACTGTCAAAGATGATATTTTGAATTTC

SEQ ID NO: 114 is the nucleic acid sequence of terminator tRPL3
GAAGTTTTGTTAGAAAATAAATCATTTTTAATTGAGCATTCTTATTCCTATTTTATTTAAATAGTTT
TATGTATTGTTAGCTACATACAACAGTTTAAATCAAATTTTCTTTTTCCCAAGTCCAAAATGGAGGT
TTATTTTGATGACCCGCATGCGATTATGTTTTGAAAGTATAAGACTACATACATGTACATATATTTA
AACATGTAAACCCGTCCATTATATTGCTTACTTTCTTCTTTTTTGCCGTTTTGACTTGGACCTCTGGT
TTGCTATTTCCTTACAATCTTTGCTACAAT

SEQ ID NO: 115 is the nucleic acid sequence of terminator tRPL3.sm
GAAGTTTTTAAAGCATTTTTTAGACACTTCTCATTTTTCTAAGTTTTTTTAAAATAGTTTTATGTATT
TACTACGTATCACAATTTGAAATAATTCATCTTCCCAAAAAACTAAGATTTTATCCTTGTCACGAT
CCGTAACCAGTTTATAATATTTTAGAGCTTATACACGTACGTATACACACGTGTCGGTACATGAGA
ATTACGTTCAAAATTATTCACTTTTTTTTCTCTGCCGTTTTACTTTTGAACTCTGTCTCGCTATTTCC
TTACAATCTTCGCTACAATACCACTTGCCCTTGG

SEQ ID NO: 116 is the nucleic acid sequence of terminator tRPL3.sba
GAAGTTTTCTGACAAAAACATAACGTTTTTCCAATCATTTCTTATTTTTCCGGTTTATTTAAATAGT
TTTTATGTACTATTATACGTATGACTATTTAACTTAAATTCTTCCTCCCAAGAAATCTCCCAAGTTTT
TCATTATCATGGCATACACCACTATCAGTTACAAAATGGTAGCTCAACCATATATATATCTCTATAT
ACACATATAAATGCAAACAGGTCCAAGTCACCGCTCACTGCAGTTTCTTTTGCCGTTTTGACTTCGA
TCTCTGCTTGGCTATTTTCTCACAATCCT

SEQ ID NO: 117 is the nucleic acid sequence of terminator tRPL41B
GCGGATTGAGAGCAAATCGTTAAGTTCAGGTCAAGTAAAAATTGATTTCGAAAACTAATTTCTCTT
ATACAATCCTTTGATTGGACCGTCATCCTTTCGAATATAAGATTTTGTTAAGAATATTTTAGACAGA
GATCTACTTTATATTTAATATCTAGATATTACATAATTTCCTCTCTAATAAAATATCATTAATAAAA
TAAAAATGAAGCGATTTGATTTTGTGTTGTCAACTTAGTTTGCCGCTATGCCTCTTGGGTAATGCTA
TTATTGAATCGAAGGGCTTTATTATATTACCCT

SEQ ID NO: 118 is the nucleic acid sequence of terminator tRPL41B.Sba
GCGGATTGCGAGTGATTTTTTGAGTACAGACCAAGTAAAATTGATCTATGCATAATCTTTTCTCATC
CACAATCCCTAGAATGGACCGTCATCCTTTCTAAAATGTTTTATTAAGAATATTTAAAGTGGAAGTC
AGGTTTATATCTATCAATTAAATATTACATAATTTCCCCTTAATAAAATACTGTTAATAAAATAACG
AAACGTTCATAGTTTTTCATAAATAGTCCTGATGCTTACTGCACCGGAACGTACTGTTTTAGTGTAT
TGCTCCCTTCATTGCCATTTTTCCTAGTTATTG

SEQ ID NO: 119 is the nucleic acid sequence of terminator tRPL15A
GCTGGTTGATGGAAAATATAATTTTATTGGGCAAACTTTTGTTTATCTGATGTGTTTTATACTATTAT
CTTTTTAATTAATGATTCTATATACAAACCTGTATATTTTTCTTTAACCAATTTTTTTTTTATAGAC
CTAGAGCTGTACTTTTATTCTGCTATCAAGCAAACCCCTACCCCCTCTTCTCAATCCTCCCCTCAGG
CAGAACTTATCTACCTGTATCAAGGAGCGGACGAGGGAGTCCTAATTGTTCTACGTATACCAATGC
TAGCAGCTTACATAGGTGGTGGCACTACCA

SEQ ID NO: 120 is the nucleic acid sequence of terminator tRPL15A.Sm
GCTAATTAGAACCATATATACATCGATAAAAACACTTGAATGTATTTTTCTTTTTCTTCTTATTTTTT
CATTTTCCTTCGCTTTCTATTAGCGTGCGAATCTAAAGTGTGCAGCGCTATCTTTGACTCTTTTTTTC
CTTTAGTGGGAACGAACAATATTAATCATGCCAAGTTAAGTCATTGAAGAAGTCAGTAGTATGTTA
TTCGGAGAGAAGACCCTGCTATGTATCTCAGCCTACCCAAAAAATGAATTGACTCCTCTCATAACT
TGTTAATATTCGGATGAGCACATCACGTTCG

SEQ ID NO: 121 is the nucleic acid sequence of terminator tRPL15A.sba
GCTGGCTGATGAAAAATATAGTTCTGTTGGGCAAGCATTTGTTTACCTAGCATCTCTTTTATACTAT
TATTATCTTTATATTTGATGATTTTATATACAAGTTGTATACCTTTTCTTTAACCAATTTTTTTTTTC
TAATGGTGCACCTAGAAGTACATTTTTTCTCACCAATAGATAGTCAAGATACTCCCAGCCTCTATGG
CGTTACCACGGAGCCGACAAGGGAAAGCTGCTTATCTTACATATGCAGATGCCAAGGCCCGTTACA
GGCCGCCTTATTGATGTTTAGAAATAGCTTC

SEQ ID NO: 122 is the nucleic acid sequence of terminator tIDP1
TCGAATTTACGTAGCCCAATCTACCACTTTTTTTTTCATTTTTTAAAGTGTTATACTTAGTTATGCT
CTAGGATAATGAACTACTTTTTTTTTTTTTTTTTACTGTTATCATAAATATATATACCTTATTGTTGT
TTGCAACCGTCGGTTAATTCCTTATCAAGGTTCCCCAAGTTCGGATCATTACCATCAATTTCCAACA
TTTTCATGAGTTCTTCTTCTTCATTACCGTGTTTTAGGGGGCTGTTCGCACTTCTAATAGGGCTATCA
CCAAGCTGTTCTAATTCGTCCAAAAGTT

SEQ ID NO: 123 is the nucleic acid sequence of terminator tIDP1.Sba
TCTGATCTTTGGAATGCTCCATTTACTTTTTTATTTCATATTTAGGTACGATATATAGTTACGCTTGA
GAACTATGAACTAATAACTTCCTTTTTCTCTAATACATACTTACTTCAGCTGATGGTTGCAAACAAC
AATAAAATTCCTTGTCGAGGTTCCCTAAATTAGGATCATTGCCATCGACTTCTAACATTTTCATGAG
ATCCTCCTCTTCGTTCCCATTCTTTAAAAGACTGTTTCCACTTCTAATAGGGTTATCGCCCAATTGTT
CTAGTTCGTCTAGCAATTCG

SEQ ID NO: 124 is the nucleic acid sequence of terminator tTEF1.sba
GGAGATTGATAGGACTTTTCTAGTTGCATATCTTTTATTTTTAAATCTTATCTATTAGTTAATTTTTT
GTAATTTATCCTTATATATATAGTTTGGTTATTCTAAAACATCATTTCAGTATCTAAAACCTCTCTTA
TTCATTACCTTTTTATTTAATGGTTTTTGCTACAGGCAAAAATTTAATGGTTTTTGCTACAGGCAAA
AATCCCGCCGTGGACTTATTCCACGTTAACTCGGTTACAGGGTCATGAACCATTTTGTCAATTATCG
AAATAACTTCTTCAAAAGTCCCTCTTACTT

**SEQ ID NO: 125** is a nucleic acid sequence of the secretion sequence added in 5'
   ATGCAATTTAGCACAGTCGCATCAGTAGCCTTCGTTGCCTTGGCCAACTTCGTGGCAGCA
**SEQ ID NO: 126** is an amino acid sequence of the secretion sequence added in N-ter
   MQFSTVASVAFVALANFVAA 
SEQ ID NO: 127 is a nucleic acid sequence of the anchoring sequence added in 3'
GGATCCGCCATTTCTCAAATCACTGACGGTCAAATCCAAGCTACTACCACTGCTACCACCGAAGCT
ACCACCACTGCTGCCCCATCTTCCACCGTTGAAACTGTTTCTCCATCCAGCACCGAAACTATCTCTC
AACAAACTGAAAATGGTGCTGCTAAGGCCGCTGTCGGTATGGGTGCCGGTGCTCTAGCTGCTGCTG
CTATGTTGTTATAA
**SEQ ID NO: 128** is an amino acid sequence of the anchoring sequence added in C-ter
   AISQITDGQIQATTTATTEATTTAAPSSTVETVSPSSTETISQQTENGAAKAAVGMGAGALAAAAMLL

## Claims

1. A recombinant yeast cell producing chondroitin wherein the recombinant yeast cell comprises:
(a) one or more recombinant nucleic acids encoding a polypeptide having a chondroitin synthase (HCOS) activity;
(b) one or more recombinant nucleic acids encoding a polypeptide having UDP-Glucose dehydrogenase (UDP-GlcDH or HASB) activity; and
(c) one or more recombinant nucleic acids encoding a polypeptide having a UDP-glucose-4-epimerase (kfoA or GNE1) activity.

2. The recombinant cell according to claim 1, wherein the recombinant cell comprises one or more recombinant nucleic acids encoding a polypeptide having a chondroitinase activity wherein the polypeptide having chondroitinase activity comprises a secretion signal, and optionally an anchoring signal.

3. A recombinant host cell producing chondroitin wherein the recombinant host cell comprises:
(a) one or more recombinant nucleic acids encoding a polypeptide having a chondroitin synthase (HCOS) activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having a UDP-glucose-4-epimerase (kfoA or GNE1) activity; and
(d) one or more recombinant nucleic acids encoding a polypeptide having a chondroitinase activity wherein the polypeptide having chondroitinase activity comprises a secretion signal, and optionally an anchoring signal so that chondroitin, in particular of a desired molecular weight is produced by the host cell.

4. The recombinant host cell according to claim 2 or 3, wherein the recombinant nucleic acid encoding a polypeptide having a chondroitinase activity is obtained or derived from at least one of *Cupiennius salei, Tityus serrulatus, Bos taurus, Vespa magnifica, Macaca mulata* or *Apis mellifera* and preferably from *Tityus serrulatus.*

5. The recombinant cell according to any one of claims 2 to 4 wherein the molecular weight of the chondroitin is in the range of less than 50kDa, preferably is in the range of about 20kDa to about 50kDa.

6. The recombinant cell according to any one of claims 1 to 4 wherein the molecular weight of the chondroitin is in the range of greater than 50kDa, preferably is in the range of about 50kDa to about 250kDa.

7. The recombinant cell according to any one of claims 1 to 4 wherein the molecular weight of the chondroitin is in the range of greater than 100kDa, preferably is in the range of about 100kDa to about 1500kDa.

8. The recombinant cell according to any one of claims 1 to 7, wherein the nucleic acid encoding a polypeptide having a UDP-Glucose dehydrogenase (UDP-GlcDH or HASB) activity is obtained or derived from at least one of *Arabidopsis thaliana, Chlorella virus PBCV1* or *Streptococcus zooepidemicus,* and in particular from at least one of *Arabidopsis thaliana* or *Chlorella virus PBCV1.*

9. The recombinant cell according to any one of claims 1 to 8, wherein the nucleic acid encoding a polypeptide having a chondroitin synthase (HCOS) activity is
(i) a nucleic acid encoding a chondroitin synthase; or
(ii) a nucleic acid encoding a chimeric polypeptide having a chondroitin synthase activity.

10. The recombinant cell according to claim 9, wherein the nucleic acids are obtained or derived from at least one of *Pasteurella multocida, Chlorella virus PBCV1, Mycobacterium tuberculosis, Homo Sapiens, Escherichia coli, Saccharomyces cerevisiae* or *Penicillium oxalicum.*

11. The recombinant cell according to any one of claims 1 to 10, wherein the nucleic acid encoding a polypeptide having a UDP-glucose-4-epimerase (kfoA or GNE1) activity is obtained or derived from a bacteria, and in particular from a bacteria selected from the group consisting of *Pseudomonas aeruginosa, Pasteurella multocida,* and *Escherichia coli.*

12. The recombinant cell according to any one of claims 1 to 11, wherein the recombinant cell further comprises at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

13. The recombinant cell according to any one of claims 1 to 12 wherein the recombinant cell further comprises at least one recombinant nucleic acid encoding one or more of:
(i) a polypeptide having Phosphoglucomutase-1 (PGM1) activity;
(ii) a polypeptide having UTP-glucose-1-phosphate uridylyltransferase (UGP1) activity;
(iii) a polypeptide having Glucosamine-6-phosphate N-acetyltransferase (GNA1) activity; and/or
(iv) a polypeptide having phosphoacetylglucosamine mutase (PCM1) activity.

14. The recombinant cell according to claim 12 or 13, wherein:
- the nucleic acid encoding the polypeptide having glutamine-fructose-6-phosphate amidotransferase (GFA1) activity;
- the nucleic acid encoding the polypeptide having UDP-N-acetylglucosamine pyrophosphorylase (QRI1) activity;
- the nucleic acid encoding the polypeptide having Phosphoglucomutase-1 (PGM1) activity;
- the nucleic acid encoding the polypeptide having UTP-glucose-1-phosphate uridylyltransferase (UGP1) activity;
- the nucleic acid encoding the polypeptide having Glucosamine-6-phosphate N-acetyltransferase (GNA1) activity; and/or
- the nucleic acid encoding the polypeptide having phosphoacetylglucosamine mutase (PCM1);
are obtained or derived from *Saccharomyces cerevisiae.*

15. The recombinant host cell according to any one of claims 3 to 14, wherein the recombinant host cell is a yeast.

16. The recombinant cell according to any one of claims 1 to 15 wherein the recombinant cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and in particular belongs to the *Saccharomyces* genus.

17. The recombinant cell according to claim 16, wherein the recombinant cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*

18. A method of producing chondroitin of a desired molecular weight comprising:
(a) cultivating a recombinant cell as defined in any one of claims 1 to 17 in a cultivation medium for a time sufficient to produce chondroitin of the desired molecular weight; and
(b) optionally isolating or recovering the chondroitin from the recombinant cell and/or from the cultivation medium.

19. The method of claim 18, wherein the chondroitin has a molecular weight of from about 20kDa to about 50kDa.

20. The method of claim 18, wherein the chondroitin has a molecular weight of from about 50kDa to about 150 kDa.

21. The method of claim 18, wherein the chondroitin has a molecular weight of from about 150kDa to about 1500 kDa.

22. The method according to any one of claims 18 to 21, wherein the recombinant cell is a yeast belonging to the *Saccharomyces* genus, and in particular is *Saccharomyces cerevisiae.*

23. The method according to any one of claims 18 to 22 wherein the time sufficient to produce chondroitin of a desired molecular weight is a period of from about 35 hours to about 50 hours, preferably from about 40 hours to about 50 hours, preferably is about 48 hours.

24. The method according to any one of claims 18 to 23 wherein the molecular weight of the produced chondroitin is controlled by regulating the pH of the cultivation medium during step (a).

25. The method according to any one of claims 18 to 24, wherein the method is carried out on an industrial scale, preferably where the cultivation medium is at least about 100L, more preferably is in the range of about 1,000L to about 3,000L, even more preferably is about 10,000L or even more preferably is about 100, 000L, or even is about 250, 000L.

26. Chondroitin obtainable from a recombinant cell according to any one of claims 1 to 17 or from the method according to any one of claims 18 to 25.

27. A cultivation medium comprising the chondroitin according to claim 26.

28. A composition comprising the chondroitin according to claim 26.

29. An industrial product or a consumer product or a consumable comprising (i) the chondroitin having a molecular weight as defined in any one of claims 5 to 7, (ii) the cultivation medium of claim 27 or (iii) the composition of claim 28.

30. The industrial product or the consumer product or the consumable of claim 29, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product, a dental and/or an oral hygiene composition.

31. Use of a recombinant cell as defined in any one of claims 2 to 17 for the production of chondroitin having a molecular weight in the range of from about 20kDa to about 50k Da or from about 50kDa to about 1000 kDa.

32. A method for producing chondrotin, comprising the steps of:
(a) culturing a recombinant yeast as defined in any one of claims 1 to 17 in a cultivation medium; and
(b) recovering the chondroitin from said cultivation medium,
wherein the chondrotin recovered in step (b) has a molecular weight controlled through the selection of:
- the nature and origin of the recombinant nucleic acid encoding a polypeptide having a chondroitinase activity of the recombinant yeast,
- the nature and origin of the promoter controlling the expression of the recombinant nucleic acid encoding encoding a polypeptide having a chondroitinase activity of the recombinant yeast,
- the presence of an anchoring and/or of a secretion signal associated to the polypeptide having a chondroitinase activity of the recombinant yeast,
- the pH of the cultivation medium during the step of culturing the recombinant yeast, and/or
- the duration of the culturing of the recombinant yeast.


SEQUENCE LISTING

<110> GIVAUDAN SA

<120> CHONDROITIN-PRODUCING RECOMBINANT CELL

<130> PR88405/MGG

<160> 128

<170> BiSSAP 1.3.6

<210> 1
<211> 2919
<212> DNA
<213> other sequences

<220>
<223> reencoded nucleic acid sequence of a polypeptide having a
      chondroitin synthase activity (HCOS-1)

<400> 1
atgaacaccc tttcccaggc aattaaggct tataatagta atgattatca actagccctc      60

aagctgttcg aaaagtcggc agaaatctac ggcagaaaga tagttgagtt ccaaattacg     120

aagtgcaagg agaaactttc tgcgcaccca tccgttaaca gtgcgcatct ctcggttaac     180

aaagaagaga aggtgaatgt ttgcgacagt ccattagaca tagccactca attattgtta     240

tcgaatgtga aaaaactcgt ccttagtgat agcgagaaaa atacactaaa gaataaatgg     300

aagttgttga ctgaaaagaa gtctgagaac gcagaggtcc gggccgtagc tttagttccc     360

aaggactttc cgaaagattt agtgcttgcg ccgttaccag accatgtgaa cgattttacg     420

tggtataaga agcggaagaa acgcctagga atcaaaccag aacaccaaca cgttggcctc     480

tccattattg tgacaacgtt caaccgtcct gccatcctca gcatcaccct ggcttgcctc     540

gtcaatcaaa agacccatta tccttttgag gtgatcgtga ccgacgatgg ttctcaagag     600

gatttatcgc ctataatccg tcaatatgag aacaagctag acatccgtta tgttcggcaa     660

aaggactatg ggtatcagct gtgcgccgtt agaaaccttg gtctaaggac agcgaagtat     720

gactttgtct ctatcttgga ttgcgacatg gccccgcagc agctatgggt tcactcttac     780

cttacagaac tattagagga cgatgatttg accataatag gccccaggaa gtacattgac     840

acgcaacaca tagatcccaa ggatttcctt aataacgcct ctctgttaga gtcgttgcca     900

gaggttaaga ccaataattc cgtcgcggct aagggcgagg ggaccgtatc tttagactgg     960

cgtttggaac aatttgagaa gaccgagaac ttgaggctat ccgatagccc tttccgattc    1020

ttcgcagctg ggaatgtggc tttcgccaag aagtggctta acaagtcagg attcttcgac    1080

gaggaattca atcactgggg aggtgaagat gtagagttcg gttatcgtct gtttcggtac    1140

ggttcgttct tcaaaactat agacggcatc atggcctatc atcaggaacc gccaggtaaa    1200

gaaaacgaaa ctgacagaga agcgggcaag aacattaccc tcgatataat gagggagaag 1260

gtgccttaca tctaccgtaa actcctgcct atagaagaca gtcatatcaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatatta agatcattca gaacaaagac gccaaaatag ctgtctccat tttctatccg 2160

aacactctaa acgggttggt gaagaagcta aataatatta tagagtataa taagaatatc 2220

ttcgttattg tacttcatgt cgataagaat cacttaaccc cagacatcaa gaaggagata 2280

ttagccttct accataagca tcaggtgaac atcttattga acaatgacat ctcctactat 2340

acatcaaatc gtctgattaa gacagaagcc catttgagta acattaacaa gctaagtcaa 2400

ttaaatctta actgcgaata cattatattc gacaatcacg attccttatt tgtgaagaac 2460

gattcctatg catacatgaa gaagtatgat gttggaatga acttctctgc attgactcat 2520

gattggattg aaaagataaa cgctcacccg ccatttaaga agctgatcaa aacttacttc 2580

aatgacaatg atcttaagtc gatgaatgta aagggtgcct cccagggaat gtttatgaca 2640

tacgcattag cccacgagtt attgacgatc atcaaggagg tgataacctc ttgtcaatcc 2700

attgactccg tccccgaata caacacagaa gatatttggt ttcagtttgc acttttaatt 2760

ctggaaaaga agaccggcca cgtattcaac aagacaagca ctctcacgta tatgccatgg 2820

gaacgtaaac tgcagtggac gaatgaacaa atagagtccg caaagagggg cgaaaacatt 2880

ccggtaaaca agttcatcat taacagcatt accctttaa 2919

<210> 2
<211> 2919
<212> DNA
<213> other sequences

<220>
<223> reencoded nucleic acid sequence of a polypeptide having a
      chondroitin synthase activity (HCOS1-2)

<400> 2
atgaacaccc tttcccaggc aattaaggct tataatagta atgattatca actagccctc      60

aagctgttcg aaaagtcggc agaaatctac ggcagaaaga tagttgagtt ccaaattacg     120

aagtgcaagg agaaactttc tgcgcaccca tccgttaaca gtgcgcatct ctcggttaac     180

aaagaagaga aggtgaatgt ttgcgacagt ccattagaca tagccactca attattgtta     240

tcgaatgtga aaaaactcgt ccttagtgat agcgagaaaa atacactaaa gaataaatgg     300

aagttgttga ctgaaaagaa gtctgagaac gcagaggtcc gggccgtagc tttagttccc     360

aaggactttc cgaaagattt agtgcttgcg ccgttaccag accatgtgaa cgattttacg     420

tggtataaga agcggaagaa acgcctagga atcaaaccag tgaacaagaa catcgggttg     480

tcaatcataa tcccaacttt caatcgtagt aggatattag acatcaccct ggcttgcctc     540

gtcaatcaaa agacccatta tccttttgag gtgatcgtga ccgacgatgg ttctcaagag     600

gatttatcgc ctataatccg tcaatatgag aacaagctag acatccgtta tgttcggcaa     660

aaggactatg ggtatcagct gtgcgccgtt agaaaccttg gtctaaggac agcgaagtat     720

gactttgtct ctatcttgga ttgcgacatg gccccgcagc agctatgggt tcactcttac     780

cttacagaac tattagagga cgatgatttg accataatag gccccaggaa gtacattgac     840

acgcaacaca tagatcccaa ggatttcctt aataacgcct ctctgttaga gtcgttgcca     900

gaggttaaga ccaataattc cgtcgcggct aagggcgagg ggaccgtatc tttagactgg     960

cgtttggaac aatttgagaa gaccgagaac ttgaggctat ccgatagccc tttccgattc    1020

ttctcatgcg gaaatgtggc cttctccaaa gagtggctga acaaagttgg ctggttcgac    1080

gaggaattca atcactgggg aggtgaagat gtagagttcg gttatcgtct gtttcggtac    1140

ggttcgttct tcaaaactat agacggcatc atggcctatc atcaggaacc gccaggtaaa    1200

gaaaacgaaa ctgacagaga agcgggcaag aacattaccc tcgatataat gagggagaag    1260

gtgccttaca tctaccgtaa actcctgcct atagaagaca gtcatatcaa ccgagtacca    1320

ttggtatcaa tttacatccc ggcctacaac tgtgctaact atattcaaag atgtgttgat    1380

tcagctttga atcaaacggt agttgatctc gaagtgtgca tttgcaacga cggtagtact    1440

gacaacacgc tggaagttat taacaagctg tatggtaata atccgcgtgt gcgtataatg    1500

tctaaaccca atggcggcat tgcgagtgca tccaacgcag cggtcagctt cgcaaagggt    1560

tattacatag gacagttgga cagcgacgat tacttagaac ccgacgcagt ggagttatgt    1620

ctcaaggaat ttcttaagga taagaccctt gcgtgcgttt acaccactaa tcgtaacgtc    1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatatta agatcattca gaacaaagac gccaaaatag ctgtctccat tttctatccg 2160

aacactctaa acgggttggt gaagaagcta aataatatta tagagtataa taagaatatc 2220

ttcgttattg tacttcatgt cgataagaat cacttaaccc cagacatcaa gaaggagata 2280

ttagccttct accataagca tcaggtgaac atcttattga acaatgacat ctcctactat 2340

acatcaaatc gtctgattaa gacagaagcc catttgagta acattaacaa gctaagtcaa 2400

ttaaatctta actgcgaata cattatattc gacaatcacg attccttatt tgtgaagaac 2460

gattcctatg catacatgaa gaagtatgat gttggaatga acttctctgc attgactcat 2520

gattggattg aaaagataaa cgctcacccg ccatttaaga agctgatcaa aacttacttc 2580

aatgacaatg atcttaagtc gatgaatgta aagggtgcct cccagggaat gtttatgaca 2640

tacgcattag cccacgagtt attgacgatc atcaaggagg tgataacctc ttgtcaatcc 2700

attgactccg tccccgaata caacacagaa gatatttggt ttcagtttgc acttttaatt 2760

ctggaaaaga agaccggcca cgtattcaac aagacaagca ctctcacgta tatgccatgg 2820

gaacgtaaac tgcagtggac gaatgaacaa atagagtccg caaagagggg cgaaaacatt 2880

ccggtaaaca agttcatcat taacagcatt accctttaa 2919

<210> 3
<211> 2919
<212> DNA
<213> other sequences

<220>
<223> reencoded nucleic acid sequence of a polypeptide having a
      chondroitin synthase activity (HCOS1-3)

<400> 3
atgaacaccc tttcccaggc aattaaggct tataatagta atgattatca actagccctc 60

aagctgttcg aaaagtcggc agaaatctac ggcagaaaga tagttgagtt ccaaattacg 120

aagtgcaagg agaaactttc tgcgcaccca tccgttaaca gtgcgcatct ctcggttaac 180

aaagaagaga aggtgaatgt ttgcgacagt ccattagaca tagccactca attattgtta 240

tcgaatgtga aaaaactcgt ccttagtgat agcgagaaaa atacactaaa gaataaatgg 300

aagttgttga ctgaaaagaa gtctgagaac gcagaggtcc gggccgtagc tttagtccca 360

aaggattttc ccaaagactt agttttagcg ccactaccag accatgtgaa cgattttacc 420

tggtacaaga atagaaagaa gagcctgggc ataaagcctg tgaacaagaa catcgggttg 480

tcaatcataa tcccaacttt caatcgtagt aggatattag acattaccct ggcgtgtcta 540

gttaatcaga aaactaacta tccgtttgag gtggttgttg cagatgatgg gtcgaaggaa 600

aatctattga ctatagttca gaaatacgag cagaaactag acattaagta tgtgagacag 660

aaggactatg ggtatcagct gtgcgccgtt agaaaccttg gtctaaggac agcgaagtat 720

gactttgtct ctatcttgga ttgcgacatg gccccgcagc agctatgggt tcactcttac 780

cttacagagt tactggaaga caacgacatc gtcttgatcg ggccaaggaa atacgttgat 840

acccacaaca tcacggctga gcagtttctg aacgacccat atttaattga gagtttaccg 900

gaaaccgcca caaacaataa tccatcgatt accagtaagg gtaacatctc attagattgg 960

agattagaac attttaagaa gacagacaac ctaagactat gcgactcccc gttcaggtat 1020

ttctcatgcg gaaatgtggc cttctccaaa gagtggctga acaaagttgg ctggttcgac 1080

gaagaattta atcactgggg cggcgaagac gtcgaattcg gctacaggtt atttgcaaaa 1140

gggtgcttct ttagggttat cgacggtggt atggcctacc atcaagagcc accaggaaag 1200

gagaacgaaa cggatcgtga ggccggtaag tctatcactc ttaaaatagt aaaggagaaa 1260

gtgccctaca tctacaggaa gttgttgccg attgaggact cccacataaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatatta agatcattca gaacaaagac gccaaaatag ctgtctccat tttctatccg 2160

aacactctaa acgggttggt gaagaagcta aataatatta tagagtataa taagaatatc 2220

ttcgttattg tacttcatgt cgataagaat cacttaaccc cagacatcaa gaaggagata 2280

ttagccttct accataagca tcaggtgaac atcttattga acaatgacat ctcctactat 2340

acatcaaatc gtctgattaa gacagaagcc catttgagta acattaacaa gctaagtcaa 2400

ttaaatctta actgcgaata cattatattc gacaatcacg attccttatt tgtgaagaac 2460

gattcctatg catacatgaa gaagtatgat gttggaatga acttctctgc attgactcat 2520

gattggattg aaaagataaa cgctcacccg ccatttaaga agctgatcaa aacttacttc 2580

aatgacaatg atcttaagtc gatgaatgta aagggtgcct cccagggaat gtttatgaca 2640

tacgcattag cccacgagtt attgacgatc atcaaggagg tgataacctc ttgtcaatcc 2700

attgactccg tccccgaata caacacagaa gatatttggt ttcagtttgc acttttaatt 2760

ctggaaaaga agaccggcca cgtattcaac aagacaagca ctctcacgta tatgccatgg 2820

gaacgtaaac tgcagtggac gaatgaacaa atagagtccg caaagagggg cgaaaacatt 2880

ccggtaaaca agttcatcat taacagcatt accctttaa 2919

<210> 4
<211> 3072
<212> DNA
<213> other sequences

<220>
<223> nucleic acid sequence of a polypeptide having chondroitin
      synthase activity (HCOS.Sc)

<400> 4
atgaacaccc tttcccaggc aattaaggct tataatagta atgattatca actagccctc 60

aagctgttcg aaaagtcggc agaaatctac ggcagaaaga tagttgagtt ccaaattacg 120

aagtgcaagg agaaactttc tgcgcaccca tccgttaaca gtgcgcatct ctcggttaac 180

aaagaagaga aggtgaatgt ttgcgacagt ccattagaca tagccactca attattgtta 240

tcgaatgtga aaaaactcgt ccttagtgat agcgagaaaa atacactaaa gaataaatgg 300

aagttgttga ctgaaaagaa gtctgagaac gcagaggtcc gggccgtagc tttagtccca 360

aaggattttc ccaaagactt agttttagcg ccactaccag accatgtgaa cgattttacc 420

tggtacaaga atagaaagaa gagcctgggc ataaagcctg tgaacaagaa catcgggttg 480

tcaatcataa tcccaacttt caatcgtagt aggatattag acattaccct ggcgtgtcta 540

gttaatcaga aaactaacta tccgtttgag gtggttgttg cagatgatgg gtcgaaggaa 600

aatctattga ctatagttca gaaatacgag cagaaactag acattaagta tgtgagacag 660

aaggactatg ggtatcagct gtgcgccgtt agaaaccttg gtctaaggac agcgaagtat 720

gactttgtct ctatcttgga ttgcgacatg gccccgcagc agctatgggt tcactcttac 780

cttacagagt tactggaaga caacgacatc gtcttgatcg ggccaaggaa atacgttgat 840

acccacaaca tcacggctga gcagtttctg aacgacccat atttaattga gagtttaccg 900

gaaaccgcca caaacaataa tccatcgatt accagtaagg gtaacatctc attagattgg 960

agattagaac attttaagaa gacagacaac ctaagactat gcgactcccc gttcaggtat 1020

ttctcatgcg gaaatgtggc cttctccaaa gagtggctga acaaagttgg ctggttcgac 1080

gaagaattta atcactgggg cggcgaagac gtcgaattcg gctacaggtt atttgcaaaa 1140

gggtgcttct ttagggttat cgacggtggt atggcctacc atcaagagcc accaggaaag 1200

gagaacgaaa cggatcgtga ggccggtaag tctatcactc ttaaaatagt aaaggagaaa 1260

gtgccctaca tctacaggaa gttgttgccg attgaggact cccacataaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatattt ttattcagat gcttttttcc tggttttcta ttgcaaattt cgttcttacc 2160

ttttattatt tagcaggatc aatgaattta gttattaaac atggtgaggc cttattcatt 2220

ttttttaaat acctgatctt ttgtgacttg gcaagtttat tcattatttc catgggtaat 2280

agaccccagg gcgcgaaaca tttattcatt acctccatgg ttatactgtc tatatgtgcc 2340

acatattctc taatttgtgg gtttgttttt gctttcaagt cgttagcttc tggaacggaa 2400

tcccacaaaa tatttgtcga catcgttatc tcattgctct ccacctatgg cctatacttt 2460

ttctcatcac tgatgtacct agatccttgg cacatgttta catcatccat acaatacttt 2520

ttgacacttc ccgcctttac gtgtacttta cagatttttg ccttctgtaa tacacacgac 2580

gtttcctggg gtactaaagg ttccacacag gagtccaagc aattgtccaa ggccattgtc 2640

gttcaaggtc cagatgggaa acagattgtg gaaacagatt ggcctcagga agttgataag 2700

aagtttttgg aaataaaaag tcgtttgaaa gaaccagaat ttgaagaatc aagcggcaat 2760

gaaaaacaat ccaagaatga ttattataga gatataagaa ccagaattgt gatgatttgg 2820

atgctatcaa atctaatact gatcatgtct ataattcaag tctttacacc acaagatact 2880

gacaatggtt atttgatatt catttatggg tctgtggccg ctttagctgc ctttagggtg 2940

gttggttcca tggccttttt gttcatgaaa tacttgcgta taatagtgag ttacagaaat 3000

aaagttgaag gtagcggctc atgggaagtc tctaaattag acttaccaaa tgttttccac 3060

aaaaagggct aa 3072

<210> 5
<211> 3072
<212> DNA
<213> other sequences

<220>
<223> nucleic acid sequence of a polypeptide having chondroitin
      synthase activity (HHASA.Sc)

<400> 5
atgaacaccc tttcccaggc aattaaggct tataatagta atgattatca actagccctc 60

aagctgttcg aaaagtcggc agaaatctac ggcagaaaga tagttgagtt ccaaattacg 120

aagtgcaagg agaaactttc tgcgcaccca tccgttaaca gtgcgcatct ctcggttaac 180

aaagaagaga aggtgaatgt ttgcgacagt ccattagaca tagccactca attattgtta 240

tcgaatgtga aaaaactcgt ccttagtgat agcgagaaaa atacactaaa gaataaatgg 300

aagttgttga ctgaaaagaa gtctgagaac gcagaggtcc gggccgtagc tttagttccc 360

aaggactttc cgaaagattt agtgcttgcg ccgttaccag accatgtgaa cgattttacg 420

tggtataaga agcggaagaa acgcctagga atcaaaccag aacaccaaca cgttggcctc 480

tccattattg tgacaacgtt caaccgtcct gccatcctca gcatcaccct ggcttgcctc 540

gtcaatcaaa agacccatta tccttttgag gtgatcgtga ccgacgatgg ttctcaagag 600

gatttatcgc ctataatccg tcaatatgag aacaagctag acatccgtta tgttcggcaa 660

aaggacaacg gttttcaggc atcggccgca cgtaatatgg gcctacgtct agccaaatat 720

gacttcattg ggctccttga ttgtgatatg gctcccaacc ctttgtgggt acactcatac 780

gttgcggaac tattagagga cgatgatttg accataatag gccccaggaa gtacattgac 840

acgcaacaca tagatcccaa ggatttcctt aataacgcct ctctgttaga gtcgttgcca 900

gaggttaaga ccaataattc cgtcgcggct aagggcgagg ggaccgtatc tttagactgg 960

cgtttggaac aatttgagaa gaccgagaac ttgaggctat ccgatagccc tttccgattc 1020

ttcgcagctg ggaatgtggc tttcgccaag aagtggctta acaagtcagg attcttcgac 1080

gaggaattca atcactgggg aggtgaagat gtagagttcg gttatcgtct gtttcggtac 1140

ggttcgttct tcaaaactat agacggcatc atggcctatc atcaggaacc gccaggtaaa 1200

gaaaacgaaa ctgacagaga agcgggcaag aacattaccc tcgatataat gagggagaag 1260

gtgccttaca tctaccgtaa actcctgcct atagaagaca gtcatatcaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatattt ttattcagat gcttttttcc tggttttcta ttgcaaattt cgttcttacc 2160

ttttattatt tagcaggatc aatgaattta gttattaaac atggtgaggc cttattcatt 2220

ttttttaaat acctgatctt ttgtgacttg gcaagtttat tcattatttc catgggtaat 2280

agaccccagg gcgcgaaaca tttattcatt acctccatgg ttatactgtc tatatgtgcc 2340

acatattctc taatttgtgg gtttgttttt gctttcaagt cgttagcttc tggaacggaa 2400

tcccacaaaa tatttgtcga catcgttatc tcattgctct ccacctatgg cctatacttt 2460

ttctcatcac tgatgtacct agatccttgg cacatgttta catcatccat acaatacttt 2520

ttgacacttc ccgcctttac gtgtacttta cagatttttg ccttctgtaa tacacacgac 2580

gtttcctggg gtactaaagg ttccacacag gagtccaagc aattgtccaa ggccattgtc 2640

gttcaaggtc cagatgggaa acagattgtg gaaacagatt ggcctcagga agttgataag 2700

aagtttttgg aaataaaaag tcgtttgaaa gaaccagaat ttgaagaatc aagcggcaat 2760

gaaaaacaat ccaagaatga ttattataga gatataagaa ccagaattgt gatgatttgg 2820

atgctatcaa atctaatact gatcatgtct ataattcaag tctttacacc acaagatact 2880

gacaatggtt atttgatatt cattttatgg tctgtggccg ctttagctgc ctttagggtg 2940

gttggttcca tggccttttt gttcatgaaa tacttgcgta taatagtgag ttacagaaat 3000

aaagttgaag gtagcggctc atgggaagtc tctaaattag acttaccaaa tgttttccac 3060

aaaaagggct aa 3072

<210> 6
<211> 1719
<212> DNA
<213> other sequences

<220>
<223> nucleic acid sequence of a polypeptide having chondroitin
      synthase activity (HCOS1-Vir)

<400> 6
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aagggtggaa tgatgttagg ttggctgtaa ttattgccgg ctaccgtgag 300

gacccgtata tgttccaaaa gtgtcttgaa agtgtgcgtg actcagacta tgggaatgta 360

gctagactaa tatgcgttat tgacggcgat gaagacgacg acatgaggat ggctgcagtg 420

tacaaggcta tctataacga caacatcaag aaacctgagt ttgtcctctg tgagagtgac 480

gataaggagg gtgagagaat agatagcgat ttcagccgtg atatctgcgt gctgcaaccg 540

catcgcggaa agcgtgaatg tttgtacaca gggttccaat tggcaaagat ggacccctca 600

gttaatgccg tcgtcctaat cgacagtgac actgtgttag aaaaggacgc gattctcgaa 660

gtagtatacc cgctggcatg cgatccagaa atacaggctg tagcaggcga atgcaaaata 720

tggaatactg acacactgtt gagtttgctg gtagcctggc gatattacag cgcattttgc 780

gtagagcgta gcgcccaatc attcttcagg acagtacaat gcgtcggagg acctctcggc 840

gcctacaaga ttgatataat taaggaaatc aaggacccat ggatcagcca acgtttcctt 900

ggccaaaagt gcacatacgg cgacgataga cgactcacta atgaaatact aatgaggggt 960

aagaaagtag ttttcacccc attcgctgtt ggctggggat ggttcgacga ggagttcaca 1020

cactggggtg gccgttacat tgtacagcaa acacggtggt ccaagtcgtg gtgtagggag 1080

atatggtata cactgtttgc agcatggaag catggacttt cgggcatttg gctcgcattc 1140

gagtgcttat accagattac ttatttcttc ctggtgattt acctattctc ccgtttggct 1200

gttgaggctg acccacgggc gcaaacggcc accgtcattg tttcgaccac agttgcgctt 1260

attaagtgcg gttactttag cttcagagct aaagacatta gggccttcta tttcgtcctc 1320

tacacgttcg tttacttctt ctgcatgatt ccggcacgaa taactgcgat gatgaccctg 1380

tgggacatag gttggggaac caggggagga aatgagaagc cttccgtagg caccagagtt 1440

gctttgtggg ccaagcaata tttgattgct tacatgtggt gggccgctgt cgtcggtgct 1500

ggcgtgtact ccatcgttca taattggatg tttgactgga attcactttc ctacaggttc 1560

gcactggtag gcatctgttc gtatatagtt ttcattgtaa tagtgctggt agtctacttt 1620

acaggaaaga tcacgacctg gaacttcacg aagctacaga aagaattgat cgaggaccgc 1680

gtactgtacg acgcaacgac caatgcccag tcggtataa 1719

<210> 7
<211> 1716
<212> DNA
<213> other sequences

<220>
<223> reencoded nucleic acid sequence of a polypeptide having
      chondroitin synthase activity (HCOS2-Vir)

<400> 7
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aagggtggaa tgatgttagg ttggctgtaa ttattgccgg ctaccgtgag 300

gacccgtata tgttccaaaa gtgtcttgaa agtgtgcgtg actcagacta tgggaatgta 360

gctagactaa tatgcgttat tgacggcgat gaagacgacg acatgaggat ggctgcagtg 420

tacaaggcta tctataacga caacatcaag aaacctgagt ttgtcctctg tgagagtgac 480

gataaggagg gtgagagaat agatagcgat ttcagccgtg atatctgcgt gctgcaaccg 540

catcgcggaa agcgtgaatg tttgtacaca gggttccaat tggcaaagat ggacccctca 600

gttaatgccg tcgtcctaat cgacagtgac actgtgttag aaaaggacgc gattctcgaa 660

gtagtatacc cgctggcatg cgatccagaa atacaggctg tagcaggcga atgcaaaata 720

tggaatactg acacactgtt gagtttgctg gtagcctggc gatattacag cgcattttgc 780

gtagagcgta gcgcccaatc attcttcagg acagtacaat gcgtcggagg acctctcggc 840

gcctacaaga ttgatataat taaggaaatc aaggacccat ggatcagcca acgtttcctt 900

ggccaaaagt gcacatacgg cgacgataga cgactcacta atgaaatact aatgaggggt 960

aagaaagtag ttttcacccc attcgctgtt ggctggctac acggtgagaa tacgtctatc 1020

aagaagttac gttacattgt acagcaaaca cggtggtcca agtcgtggtg tagggagata 1080

tggtatacac tgtttgcagc atggaagcat ggactttcgg gcatttggct cgcattcgag 1140

tgcttatacc agattactta tttcttcctg gtgatttacc tattctcccg tttggctgtt 1200

gaggctgacc cacgggcgca aacggccacc gtcattgttt cgaccacagt tgcgcttatt 1260

aagtgcggtt actttagctt cagagctaaa gacattaggg ccttctattt cgtcctctac 1320

acgttcgttt acttcttctg catgattccg gcacgaataa ctgcgatgat gaccctgtgg 1380

gacataggtt ggggaaccag gggaggaaat gagaagcctt ccgtaggcac cagagttgct 1440

ttgtgggcca agcaatattt gattgcttac atgtggtggg ccgctgtcgt cggtgctggc 1500

gtgtactcca tcgttcataa ttggatgttt gactggaatt cactttccta caggttcgca 1560

ctggtaggca tctgttcgta tatagttttc attgtaatag tgctggtagt ctactttaca 1620

ggaaagatca cgacctggaa cttcacgaag ctacagaaag aattgatcga ggaccgcgta 1680

ctgtacgacg caacgaccaa tgcccagtcg gtataa 1716

<210> 8
<211> 1719
<212> DNA
<213> other sequences

<220>
<223> reencoded nucleic acid sequence of a polypeptide having
      chondroitin synthase activity (HCOS3-Vir)

<400> 8
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aagggtggaa tgatgttagg ttggctgtaa ttattgccgg ctaccgtgag 300

gacccgtata tgttccaaaa gtgtcttgaa agtgtgcgtg actcagacta tgggaatgta 360

gctagactaa tatgcgttat tgacggcgat gaagacgacg acatgaggat ggctgcagtg 420

tacaaggcta tctataacga caacatcaag aaacctgagt ttgtcctctg tgagagtgac 480

gataaggagg gtgagagaat agatagcgat ttcagccgtg atatctgcgt gctgcaaccg 540

catcgcggaa agcgtgaatg tttgtacaca gggttccaat tggcaaagat ggacccctca 600

gttaatgccg tcgtcctaat cgacagtgac actgtgttag aaaaggacgc gattctcgaa 660

gtagtatacc cgctggcatg cgatccagaa atacaggctg tagcaggcga atgcaaaata 720

tggaatactg acacactgtt gagtttgctg gtagcctggc gatattacag cgcattttgc 780

gtagagcgta gcgcccaatc attcttcagg acagtacaat gcgtcggagg acctctcggc 840

gcctacaaga ttgatataat taaggaaatc aaggacccat ggatcagcca acgtttcctt 900

ggccaaaagt gcacatacgg cgacgataga cgactcacta atgaaatact aatgaggggt 960

aagaaagtag ttttcacccc attcgctgtt ggctggcaca tggcatggtc cgacaaggac 1020

gacgcaatcg accgttacat tgtacagcaa acacggtggt ccaagtcgtg gtgtagggag 1080

atatggtata cactgtttgc agcatggaag catggacttt cgggcatttg gctcgcattc 1140

gagtgcttat accagattac ttatttcttc ctggtgattt acctattctc ccgtttggct 1200

gttgaggctg acccacgggc gcaaacggcc accgtcattg tttcgaccac agttgcgctt 1260

attaagtgcg gttactttag cttcagagct aaagacatta gggccttcta tttcgtcctc 1320

tacacgttcg tttacttctt ctgcatgatt ccggcacgaa taactgcgat gatgaccctg 1380

tgggacatag gttggggaac caggggagga aatgagaagc cttccgtagg caccagagtt 1440

gctttgtggg ccaagcaata tttgattgct tacatgtggt gggccgctgt cgtcggtgct 1500

ggcgtgtact ccatcgttca taattggatg tttgactgga attcactttc ctacaggttc 1560

gcactggtag gcatctgttc gtatatagtt ttcattgtaa tagtgctggt agtctacttt 1620

acaggaaaga tcacgacctg gaacttcacg aagctacaga aagaattgat cgaggaccgc 1680

gtactgtacg acgcaacgac caatgcccag tcggtataa 1719

<210> 9
<211> 1713
<212> DNA
<213> other sequences

<220>
<223> reencoded nucleic acid sequence of a polypeptide having
      chondroitin synthase activity (HCOS4-Vir)

<400> 9
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aagggtggaa tgatgttagg ttggctgtaa ttattgccgg ctaccgtgag 300

gacccgtata tgttccaaaa gtgtcttgaa agtgtgcgtg actcagacta tgggaatgta 360

gctagactaa tatgcgttat tgacggcgat gaagacgacg acatgaggat ggctgcagtg 420

tacaaggcta tctataacga caacatcaag aaacctgagt ttgtcctctg tgagagtgac 480

gataaggagg gtgagagaat agatagcgat ttcagccgtg atatctgcgt gctgcaaccg 540

catcgcggaa agcgtgaatg tttgtacaca gggttccaat tggcaaagat ggacccctca 600

gttaatgccg tcgtcctaat cgacagtgac actgtgttag aaaaggacgc gattctcgaa 660

gtagtatacc cgctggcatg cgatccagaa atacaggctg tagcaggcga atgcaaaata 720

tggaatactg acacactgtt gagtttgctg gtagcctggc gatattacag cgcattttgc 780

gtagagcgta gcgcccaatc attcttcagg acagtacaat gcgtcggagg acctctcggc 840

gcctacaaga ttgatataat taaggaaatc aaggacccat ggatcagcca acgtttcctt 900

ggccaaaagt gcacatacgg cgacgataga cgactcacta atgaaatact aatgaggggt 960

aagaaagtag ttttcacccc attcgctgtt ggctggttcg acgaggagtt cacacactgg 1020

ggtggccgtt acattgtaca gcaaacacgg tggtccaagt cgtggtgtag ggagatatgg 1080

tatacactgt ttgcagcatg gaagcatgga ctttcgggca tttggctcgc attcgagtgc 1140

ttataccaga ttacttattt cttcctggtg atttacctat tctcccgttt ggctgttgag 1200

gctgacccac gggcgcaaac ggccaccgtc attgtttcga ccacagttgc gcttattaag 1260

tgcggttact ttagcttcag agctaaagac attagggcct tctatttcgt cctctacacg 1320

ttcgtttact tcttctgcat gattccggca cgaataactg cgatgatgac cctgtgggac 1380

ataggttggg gaaccagggg aggaaatgag aagccttccg taggcaccag agttgctttg 1440

tgggccaagc aatatttgat tgcttacatg tggtgggccg ctgtcgtcgg tgctggcgtg 1500

tactccatcg ttcataattg gatgtttgac tggaattcac tttcctacag gttcgcactg 1560

gtaggcatct gttcgtatat agttttcatt gtaatagtgc tggtagtcta ctttacagga 1620

aagatcacga cctggaactt cacgaagcta cagaaagaat tgatcgagga ccgcgtactg 1680

tacgacgcaa cgaccaatgc ccagtcggta taa 1713

<210> 10
<211> 2079
<212> DNA
<213> other sequences

<220>
<223> nucleic acid sequence of a polypeptide having chondroitin
      synthase activity (HCOS5-Vir)

<400> 10
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aatccattca ccatccaatt tcgaaaacga ctcaccccga cacagaaaat 300

gcaggtgact caacattgtt gtgcccgccc atggatggta tggacgacat attagtcgtt 360

atgaagacag gtgtcacaga ggctaaggaa aaggtgccag tacattttca gacaacgctg 420

aggtgtgtgc cgcactacgt tatctatagc gacttcgaag aagagataga aggtgtaaag 480

atttacgacg tcttgcaagg aatggacccc gatgttatga cgcaggttcc ggacttcgat 540

ctttacaatc gtattaagaa gttagggcgt cagggccttg cggagaacga cttcgcggac 600

gaagcgaact ctgcggtggg taagcctaat aaccctggtt ggaagttaga taaatggaag 660

ttcttaccta tgatcattga ggctctgaga tacaaaccaa aggctaagtg gtatgttttc 720

atggaagcag acacttatat atcttggccg actttattag cctggctaaa acacttcaac 780

cacaagaagc cgtggtatct tggcactgag acacagatag ccgatgtgat tttcgctcat 840

ggtggttcag gattcatgat tagtcatgcc gcgcttaagc ttgctgctga agaatatacc 900

gcacgtcgta ctgagttgca ccactacaca gacggtcact gggcaggaga ctgcgtatta 960

ggtaaagttc ttgctgacgc aggtgtgaac ttgcatttct cctggccaat tttacagaat 1020

tccaatctgg gcgaactgga tgagttcaac gttgacttat acaggcaacc ttggtgcttt 1080

ccagctgccg ctttccacca cttgaacgct cagaagatcc aggacttatg gtcttacgag 1140

cagaaaaggt ggcaggacaa ggacaagagg gttctgttac atagcgacgt cttcagggac 1200

ctattatatc cggatatagc attaaaaccc gcgaggatcg gttgggacaa cctgagtgca 1260

gacgaacagt ctacggcctc tagtttcgag gaatgcaggc aaatttgtga tcaattagac 1320

gattgtaccc agtttagttt cagggacaaa tcctgtttta catcaagaac tccacgtttg 1380

ggcgcagcca atccaaatac agttagcgga tggatggttg atcgtatccg tgcgaatatg 1440

gagaagtctg gactgtttgc agcatggaag catggacttt cgggcatttg gctcgcattc 1500

gagtgcttat accagattac ttatttcttc ctggtgattt acctattctc ccgtttggct 1560

gttgaggctg acccacgggc gcaaacggcc accgtcattg tttcgaccac agttgcgctt 1620

attaagtgcg gttactttag cttcagagct aaagacatta gggccttcta tttcgtcctc 1680

tacacgttcg tttacttctt ctgcatgatt ccggcacgaa taactgcgat gatgaccctg 1740

tgggacatag gttggggaac caggggagga aatgagaagc cttccgtagg caccagagtt 1800

gctttgtggg ccaagcaata tttgattgct tacatgtggt gggccgctgt cgtcggtgct 1860

ggcgtgtact ccatcgttca taattggatg tttgactgga attcactttc ctacaggttc 1920

gcactggtag gcatctgttc gtatatagtt ttcattgtaa tagtgctggt agtctacttt 1980

acaggaaaga tcacgacctg gaacttcacg aagctacaga aagaattgat cgaggaccgc 2040

gtactgtacg acgcaacgac caatgcccag tcggtataa 2079

<210> 11
<211> 2733
<212> DNA
<213> other sequences

<220>
<223> nucleic acid sequence of a polypeptide having chondroitin
      synthase activity (HCOS6-Vir)

<400> 11
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aattagacat tgcaacacag ttattactat cgaatgttaa gaaattgacc 300

ttaagcgagt ccgaaaagaa cagcttaaag aataagtgga agagtataac aggcaagaag 360

tccgagaatg ctgaaattag gaaggttgag ttagtcccaa aggattttcc caaagactta 420

gttttagcgc cactaccaga ccatgtgaac gattttacct ggtacaagaa tagaaagaag 480

agcctgggca taaagcctgt gaacaagaac atcgggttgt caatcataat cccaactttc 540

aatcgtagta ggatattaga cattaccctg gcgtgtctag ttaatcagaa aactaactat 600

ccgtttgagg tggttgttgc agatgatggg tcgaaggaaa atctattgac tatagttcag 660

aaatacgagc agaaactaga cattaagtat gtgagacaga aggactatgg gtatcagctg 720

tgcgccgtta gaaaccttgg tctaaggaca gcgaagtatg actttgtctc tatcttggat 780

tgcgacatgg ccccgcagca gctatgggtt cactcttacc ttacagagtt actggaagac 840

aacgacatcg tcttgatcgg gccaaggaaa tacgttgata cccacaacat cacggctgag 900

cagtttctga acgacccata tttaattgag agtttaccgg aaaccgccac aaacaataat 960

ccatcgatta ccagtaaggg taacatctca ttagattgga gattagaaca ttttaagaag 1020

acagacaacc taagactatg cgactccccg ttcaggtatt tctcatgcgg aaatgtggcc 1080

ttctccaaag agtggctgaa caaagttggc tggttcgacg aagaatttaa tcactggggc 1140

ggcgaagacg tcgaattcgg ctacaggtta tttgcaaaag ggtgcttctt tagggttatc 1200

gacggtggta tggcctacca tcaagagcca ccaggaaagg agaacgaaac ggatcgtgag 1260

gccggtaagt ctatcactct taaaatagta aaggagaaag tgccctacat ctacaggaag 1320

ttgttgccga ttgaggactc ccacatacac cgtataccgc ttgtgagcat atacattcca 1380

gcgtacaact gcgccaatta tattcaaagg tgtgtagata gcgcgttgaa tcaaacagtg 1440

gtagatttgg aagtttgtat atgtaacgac ggaagtactg acaatacttt agaggtgatt 1500

aacaagctgt atggcaacaa cccgcgtgtc aggataatga gcaagcccaa tggtggaatt 1560

gcttccgcgt ctaacgctgc cgtttcattc gcgaaaggct actacattgg ccagttggac 1620

tcggatgatt acttggaacc tgacgcagtg gagctttgtt taaaggagtt cttgaaggac 1680

aaaacactag cctgtgtata tacaacgaac agaaatgtta atcctgacgg ttccctaatc 1740

gctaacggat acaattggcc tgagttcagc agggagaagc tgaccactgc tatgatcgcc 1800

caccatttca ggatgttcac aatcagggct tggcatttaa cagatggttt taatgagaaa 1860

atagagaacg cagtagacta cgatatgttt ctgaagttat cagaggtcgg gaaattcaaa 1920

cacttgaaca aaatttgtta caatagggta ttacacggtg acaacaccag tattaagaat 1980

cttgacaccc aaaagaagaa ccacttcgta gtagtgaacc agtctctgaa cagacaaagg 2040

gtgtcgaatt ataactacga tgagtttgat aacctggacg agagccgtaa gtacatattc 2100

aacaagctgt ttgcagcatg gaagcatgga ctttcggcca tttggctcgc attcgagtgc 2160

ttataccaga ttacttattt cttcctggtg atttacctat tctcccgttt ggctgttgag 2220

gctgacccac gggcgcaaac ggccaccgtc attgtttcga ccacagttgc gcttattaag 2280

tgcggttact ttagcttcag agctaaagac attagggcct tctatttcgt cctctacacg 2340

ttcgtttact tcttctgcat gattccggca cgaataactg cgatgatgac cctgtgggac 2400

ataggttggg gaaccagggg aggaaatgag aagccttccg taggcaccag agttgctttg 2460

tgggccaagc aatatttgat tgcttacatg tggtgggccg ctgtcgtcgg tgctggcgtg 2520

tactccatcg ttcataattg gatgtttgac tggaattcac tttcctacag gttcgcactg 2580

gtaggcatct gttcgtatat agttttcatt gtaatagtgc tggtagtcta ctttacagga 2640

aagatcacga cctggaactt cacgaagcta cagaaagaat tgatcgagga ccgcgtactg 2700

tacgacgcaa cgaccaatgc ccagtcggta taa 2733

<210> 12
<211> 2112
<212> DNA
<213> other sequences

<220>
<223> reencoded nucleic acid sequence of a polypeptide having
      chondroitin synthase activity (HCOS7-Vir)

<400> 12
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aaatcccaac tttcaatcgt agtaggatat tagacattac cctggcgtgt 300

ctagttaatc agaaaactaa ctatccgttt gaggtggttg ttgcagatga tgggtcgaag 360

gaaaatctat tgactatagt tcagaaatac gagcagaaac tagacattaa gtatgtgaga 420

cagaaggact atgggtatca gctgtgcgcc gttagaaacc ttggtctaag gacagcgaag 480

tatgactttg tctctatctt ggattgcgac atggccccgc agcagctatg ggttcactct 540

taccttacag agttactgga agacaacgac atcgtcttga tcgggccaag gaaatacgtt 600

gatacccaca acatcacggc tgagcagttt ctgaacgacc catatttaat tgagagttta 660

ccggaaaccg ccacaaacaa taatccatcg attaccagta agggtaacat ctcattagat 720

tggagattag aacattttaa gaagacagac aacctaagac tatgcgactc cccgttcagg 780

tatttctcat gcggaaatgt ggccttctcc aaagagtggc tgaacaaagt tggctggttc 840

gacgaagaat ttaatcactg gggcggcgaa gacgtcgaat tcggctacag gttatttgca 900

aaagggtgct tctttagggt tatcgacggt ggtatggcct accatcaaga gccaccagga 960

aaggagaacg aaacggatcg tgaggccggt aagtctatca ctcttaaaat agtaaaggag 1020

aaagtgccct acatctacag gaagttgttg ccgattgagg actcccacat acaccgtata 1080

ccgcttgtga gcatatacat tccagcgtac aactgcgcca attatattca aaggtgtgta 1140

gatagcgcgt tgaatcaaac agtggtagat ttggaagttt gtatatgtaa cgacggaagt 1200

actgacaata ctttagaggt gattaacaag ctgtatggca acaacccgcg tgtcaggata 1260

atgagcaagc ccaatggtgg aattgcttcc gcgtctaacg ctgccgtttc attcgcgaaa 1320

ggctactaca ttggccagtt ggactcggat gattacttgg aacctgacgc agtggagctt 1380

tgtttaaagg agttcttgaa ggacaaaaca ctagcctgtg tatatacaac gaacagaaat 1440

gttaatcctg acggttccct aatcgctaac ggatacaatt ggcctctgtt tgcagcatgg 1500

aagcatggac tttcgggcat ttggctcgca ttcgagtgct tataccagat tacttatttc 1560

ttcctggtga tttacctatt ctcccgtttg gctgttgagg ctgacccacg ggcgcaaacg 1620

gccaccgtca ttgtttcgac cacagttgcg cttattaagt gcggttactt tagcttcaga 1680

gctaaagaca ttagggcctt ctatttcgtc ctctacacgt tcgtttactt cttctgcatg 1740

attccggcac gaataactgc gatgatgacc ctgtgggaca taggttgggg aaccagggga 1800

ggaaatgaga agccttccgt aggcaccaga gttgctttgt gggccaagca atatttgatt 1860

gcttacatgt ggtgggccgc tgtcgtcggt gctggcgtgt actccatcgt tcataattgg 1920

atgtttgact ggaattcact ttcctacagg ttcgcactgg taggcatctg ttcgtatata 1980

gttttcattg taatagtgct ggtagtctac tttacaggaa agatcacgac ctggaacttc 2040

acgaagctac agaaagaatt gatcgaggac cgcgtactgt acgacgcaac gaccaatgcc 2100

cagtcggtat aa 2112

<210> 13
<211> 1710
<212> DNA
<213> other sequences

<220>
<223> reencoded nucleic acid sequence of a polypeptide having
      chondroitin synthase activity (HCOS8-Vir)

<400> 13
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aagggtggaa tgatgttagg ttggctgtaa ttattgccgg ctaccgtgag 300

gacccgtata tgttccaaaa gtgtcttgaa agtgtgcgtg actcagacta tgggaatgta 360

gctagactaa tatgcgttat tgacggcgat gaagacgacg acatgaggat ggctgcagtg 420

tacaaggcta tctataacga caacatcaag aaacctgagt ttgtcctctg tgagagtgac 480

gataaggagg gtgagagaat agatagcgat ttcagccgtg atatctgcgt gctgcaaccg 540

catcgcggaa agcgtgaatg tttgtacaca gggttccaat tggcaaagat ggacccctca 600

gttaatgccg tcgtcctaat cgacagtgac actgtgttag aaaaggacgc gattctcgaa 660

gtagtatacc cgctggcatg cgatccagaa atacaggctg tagcaggcga atgcaaaata 720

tggaatactg acacactgtt gagtttgctg gtagcctggc gatattacag cgcattttgc 780

gtagagcgta gcgcccaatc attcttcagg acagtacaat gcgtcagact atgcgactcc 840

ccgttcaggt atttctcatg cggaaatgtg gccttctcca aagagtggct gaacaaagtt 900

ggctggttcg acgaagaatt taatcactgg ggcggcgaag acgtcgaatt cggctacagg 960

ttaaagaaag tagttttcac cccattcgct gttggctggt ccgacagccc gacgaacgtc 1020

ttccgttaca ttgtacagca aacacggtgg tccaagtcgt ggtgtaggga gatatggtat 1080

acactgtttg cagcatggaa gcatggactt tcgggcattt ggctcgcatt cgagtgctta 1140

taccagatta cttatttctt cctggtgatt tacctattct cccgtttggc tgttgaggct 1200

gacccacggg cgcaaacggc caccgtcatt gtttcgacca cagttgcgct tattaagtgc 1260

ggttacttta gcttcagagc taaagacatt agggccttct atttcgtcct ctacacgttc 1320

gtttacttct tctgcatgat tccggcacga ataactgcga tgatgaccct gtgggacata 1380

ggttggggaa ccaggggagg aaatgagaag ccttccgtag gcaccagagt tgctttgtgg 1440

gccaagcaat atttgattgc ttacatgtgg tgggccgctg tcgtcggtgc tggcgtgtac 1500

tccatcgttc ataattggat gtttgactgg aattcacttt cctacaggtt cgcactggta 1560

ggcatctgtt cgtatatagt tttcattgta atagtgctgg tagtctactt tacaggaaag 1620

atcacgacct ggaacttcac gaagctacag aaagaattga tcgaggaccg cgtactgtac 1680

gacgcaacga ccaatgccca gtcggtataa 1710

<210> 14
<211> 2499
<212> DNA

<213> other sequences

<220>
<223> a nucleic acid sequence of a polypeptide having chondroitin
      synthase activity (HCOS9-Vir)

<400> 14
atgggtaaga acatcattat tatggtttct tggtacacta ttattacatc caatctcatc      60

gcagtgggtg gcgcctcact catactagcc ccagctatta cgggctatgt ccttcactgg     120

aacattgccc tttcaacaat ttggggagtg tcggcctacg gaattttcgt gtttggtttc     180

tttcttgccc aggtattatt tagtgaactc aaccggaaaa ggctccggaa gtggatttcc     240

ctccgaccca aacacattgg taagtgtcta agggaaatgt atacgactca tgaggacgtg     300

gaagtaggaa gatgcgttag gagattcgct ggagtacagt gtgtttggtc ttacgagatg     360

caacagttat tctacgagaa ctacgagcag aataagaagg gatacatccg tgatctgcac     420

aactccaaaa ttcaccaagc aataacgctg catcctaaca agaatccgcc ctatcagtat     480

aggttgcact cttacatgtt gagtagaaag atatctgagc tacgtcatag aactattcag     540

ttgcacaggg agatagtact gatgtctaaa tactcaaaca cggagatcca taaggaagac     600

ctacagttgg gtataccacc ttcgttcatg agattccagc caagacagag ggaagaaatt     660

ttggagtggg agttcttgac tggtaaatac ttgtattctg ctgttgacgg tcaaccacct     720

aggagaggga tggactctgc acagcgtgag gctttagacg atatagtgat gcaagtaatg     780

gaaatgatca acgctaatgc aaagacacgt ggtcgtatca tagatttcaa agagatccag     840

tatggatatc gtcgtgtgaa cccaatgtac ggcgcggagt atattcttga cttattgttg     900

ctatacaaga aacataaggg taagaagatg actgtcccgg ttcgtaggca cgcgtatttg     960

caacagactt tctcgaaaat acagtttgta gagcatgagg aacttgacgc gcaagagtta    1020

gcgaaacgta taaaccaaga gtcggggagt ttatccttct taagtaattc ccttaagaag    1080

ttggttcctt tccagctgcc tggaagtaaa agtgagcaca aggagccaaa ggataagaaa    1140

atcaacattc ttatacctct ttccggcaga ttcgacatgt tcgttaggtt catgggtaac    1200

ttcgagaaga cctgcttaat tcctaaccag aatgttaagt tggtagtttt gttattcaat    1260

tctgacagta accccgacaa agcaaagcag gttgaactga tgagagacta taggattaag    1320

tacccaaaag cagatatgca gatactacct gtttccggag aattctcaag agccttggct    1380

ctggaagtag gcagctctca gtttaataat gagtctcttc tgttcttctg cgacgttgac    1440

ttagttttca cgacagagtt tctgcagaga tgtcgtgcaa acactgtact aggtcagcag    1500

atatatttcc ccattatttt cagtcagtac gatccaaaga ttgtttatag tggaaaagtt    1560

ccatctgaca accattttgc gtttactcaa aagacagggt tttggcgtaa ctacgggttc    1620

ggcataacat gtatctataa aggggacctg gtaagagttg gcggattcga cgtatctata    1680

cagggctggg gtttagaaga cgtcgatcta tttaataaag tagttcaggc aggtttgaag 1740

acattccgta gtcaagaggt aggcgttgta cacgtgcacc atccagtatt ttgcgaccca 1800

aacttagacc cgaagcaata taaaatgtgc ttgggttcca aggcatcgac ttacggttcc 1860

acgcagcaac tactgtttgc agcatggaag catggacttt cgggcatttg gctcgcattc 1920

gagtgcttat accagattac ttatttcttc ctggtgattt acctattctc ccgtttggct 1980

gttgaggctg acccacgggc gcaaacggcc accgtcattg tttcgaccac agttgcgctt 2040

attaagtgcg gttactttag cttcagagct aaagacatta gggccttcta tttcgtcctc 2100

tacacgttcg tttacttctt ctgcatgatt ccggcacgaa taactgcgat gatgaccctg 2160

tgggacatag gttggggaac caggggagga aatgagaagc cttccgtagg caccagagtt 2220

gctttgtggg ccaagcaata tttgattgct tacatgtggt gggccgctgt cgtcggtgct 2280

ggcgtgtact ccatcgttca taattggatg tttgactgga attcactttc ctacaggttc 2340

gcactggtag gcatctgttc gtatatagtt ttcattgtaa tagtgctggt agtctacttt 2400

acaggaaaga tcacgacctg gaacttcacg aagctacaga aagaattgat cgaggaccgc 2460

gtactgtacg acgcaacgac caatgcccag tcggtataa 2499

<210> 15
<211> 3006
<212> DNA
<213> other sequences

<220>
<223> reencoded nucleic acid sequence of a polypeptide having
      chondroitin synthase activity (HCOS10-Vir)

<400> 15
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aaagagatag aaacttccta ttcgttggtg ttatgacagc tcagaagtac 300

ttacagacca gggcggttgc agcatataga acctggtcaa agacaattcc aggtaaggta 360

cagttcttca gctccgaggg ttctgacacc agcgtcccta tcccagtagt gcctttgcgt 420

ggagtggatg attcataccc acctcagaag aagtccttca tgatgcttaa gtatatgcac 480

gaccactatc tggacaaata cgagtggttc atgagagcag acgacgacgt ttacataaag 540

ggtgatagac tggagaattt cttgaggtca ctaaacagtt cagaaccact tttcctagga 600

cagacgggcc ttggtacgac agaggagatg ggtaagttgg cattggagcc aggggagaac 660

ttctgcatgg gtggccctgg tgtgattatg agtagggaag ttttaagaag aatggttccg 720

cacattggta agtgtctaag ggaaatgtat acgactcatg aggacgtgga agtaggaaga 780

tgcgttagga gattcgctgg agtacagtgt gtttggtctt acgagatgca acagttattc 840

tacgagaact acgagcagaa taagaaggga tacatccgtg atctgcacaa ctccaaaatt 900

caccaagcaa taacgctgca tcctaacaag aatccgccct atcagtatag gttgcactct 960

tacatgttga gtagaaagat atctgagcta cgtcatagaa ctattcagtt gcacagggag 1020

atagtactga tgtctaaata ctcaaacacg gagatccata aggaagacct acagttgggt 1080

ataccacctt cgttcatgag attccagcca agacagaggg aagaaatttt ggagtgggag 1140

ttcttgactg gtaaatactt gtattctgct gttgacggtc aaccacctag gagagggatg 1200

gactctgcac agcgtgaggc tttagacgat atagtgatgc aagtaatgga aatgatcaac 1260

gctaatgcaa agacacgtgg tcgtatcata gatttcaaag agatccagta tggatatcgt 1320

cgtgtgaacc caatgtacgg cgcggagtat attcttgact tattgttgct atacaagaaa 1380

cataagggta agaagatgac tgtcccggtt cgtaggcacg cgtatttgca acagactttc 1440

tcgaaaatac agtttgtaga gcatgaggaa cttgacgcgc aagagttagc gaaacgtata 1500

aaccaagagt cggggagttt atccttctta agtaattccc ttaagaagtt ggttcctttc 1560

cagctgcctg gaagtaaaag tgagcacaag gagccaaagg ataagaaaat caacattctt 1620

atacctcttt ccggcagatt cgacatgttc gttaggttca tgggtaactt cgagaagacc 1680

tgcttaattc ctaaccagaa tgttaagttg gtagttttgt tattcaattc tgacagtaac 1740

cccgacaaag caaagcaggt tgaactgatg agagactata ggattaagta cccaaaagca 1800

gatatgcaga tactacctgt ttccggagaa ttctcaagag ccttggctct ggaagtaggc 1860

agctctcagt ttaataatga gtctcttctg ttcttctgcg acgttgactt agttttcacg 1920

acagagtttc tgcagagatg tcgtgcaaac actgtactag gtcagcagat atatttcccc 1980

attattttca gtcagtacga tccaaagatt gtttatagtg gaaaagttcc atctgacaac 2040

cattttgcgt ttactcaaaa gacagggttt tggcgtaact acgggttcgg cataacatgt 2100

atctataaag gggacctggt aagagttggc ggattcgacg tatctataca gggctggggt 2160

ttagaagacg tcgatctatt taataaagta gttcaggcag gtttgaagac attccgtagt 2220

caagaggtag gcgttgtaca cgtgcaccat ccagtatttt gcgacccaaa cttagacccg 2280

aagcaatata aaatgtgctt gggttccaag gcatcgactt acggttccac gcagcaacta 2340

gcagagatgt ggttggagaa gaacgaccca tcttatagcc tgtttgcagc atggaagcat 2400

ggactttcgg gcatttggct cgcattcgag tgcttatacc agattactta tttcttcctg 2460

gtgatttacc tattctcccg tttggctgtt gaggctgacc cacgggcgca aacggccacc 2520

gtcattgttt cgaccacagt tgcgcttatt aagtgcggtt actttagctt cagagctaaa 2580

gacattaggg ccttctattt cgtcctctac acgttcgttt acttcttctg catgattccg 2640

gcacgaataa ctgcgatgat gaccctgtgg gacataggtt ggggaaccag gggaggaaat 2700

gagaagcctt ccgtaggcac cagagttgct ttgtgggcca agcaatattt gattgcttac 2760

atgtggtggg ccgctgtcgt cggtgctggc gtgtactcca tcgttcataa ttggatgttt 2820

gactggaatt cactttccta caggttcgca ctggtaggca tctgttcgta tatagttttc 2880

attgtaatag tgctggtagt ctactttaca ggaaagatca cgacctggaa cttcacgaag 2940

ctacagaaag aattgatcga ggaccgcgta ctgtacgacg caacgaccaa tgcccagtcg 3000

gtataa 3006

<210> 16
<211> 1596
<212> DNA
<213> other sequences

<220>
<223> reencoded nucleic acid sequence of a polypeptide having
      chondroitin synthase activity (HCOS11-Vir)

<400> 16
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aaagagatag aaacttccta ttcgttggtg ttatgacagc tcagaagtac 300

ttacagacca gggcggttgc agcatataga acctggtcaa agacaattcc aggtaaggta 360

cagttcttca gctccgaggg ttctgacacc agcgtcccta tcccagtagt gcctttgcgt 420

ggagtggatg attcataccc acctcagaag aagtccttca tgatgcttaa gtatatgcac 480

gaccactatc tggacaaata cgagtggttc atgagagcag acgacgacgt ttacataaag 540

ggtgatagac tggagaattt cttgaggtca ctaaacagtt cagaaccact tttcctagga 600

cagacgggcc ttggtacgac agaggagatg ggtaagttgg cattggagcc aggggagaac 660

ttctgcatgg gtggccctgg tgtgattatg agtagggaag ttttaagaag aatggttccg 720

cacattggta agtgtctaag ggaaatgtat acgactcatg aggacgtgga agtaggaaga 780

tgcgttagga gattcgctgg agtacagtgt gtttggtctt acgagatgca acagttattc 840

tacgagaact acgagcagaa taagaaggga tacatccgtg atctgcacaa ctccaaaatt 900

caccaagcaa taacgctgca tcctaacaag aatccgccct atcagtatag gttgcactct 960

tacatgttgc tgtttgcagc atggaagcat ggactttcgg gcatttggct cgcattcgag 1020

tgcttatacc agattactta tttcttcctg gtgatttacc tattctcccg tttggctgtt 1080

gaggctgacc cacgggcgca aacggccacc gtcattgttt cgaccacagt tgcgcttatt 1140

aagtgcggtt actttagctt cagagctaaa gacattaggg ccttctattt cgtcctctac 1200

acgttcgttt acttcttctg catgattccg gcacgaataa ctgcgatgat gaccctgtgg 1260

gacataggtt ggggaaccag gggaggaaat gagaagcctt ccgtaggcac cagagttgct 1320

ttgtgggcca agcaatattt gattgcttac atgtggtggg ccgctgtcgt cggtgctggc 1380

gtgtactcca tcgttcataa ttggatgttt gactggaatt cactttccta caggttcgca 1440

ctggtaggca tctgttcgta tatagttttc attgtaatag tgctggtagt ctactttaca 1500

ggaaagatca cgacctggaa cttcacgaag ctacagaaag aattgatcga ggaccgcgta 1560

ctgtacgacg caacgaccaa tgcccagtcg gtataa 1596

<210> 17
<211> 1662
<212> DNA
<213> other sequences

<220>
<223> reencoded nucleic acid sequence of a polypeptide having
      chondroitin synthase activity (HCOS12-Vir)

<400> 17
atgggtaaga acatcattat tatggtttct tggtacacta ttattacatc caatctcatc 60

gcagtgggtg gcgcctcact catactagcc ccagctatta cgggctatgt ccttcactgg 120

aacattgccc tttcaacaat ttggggagtg tcggcctacg gaattttcgt gtttggtttc 180

tttcttgccc aggtattatt tagtgaactc aaccggaaaa ggctccggaa gtggatttcc 240

ctccgaccca aaaaggataa gaaaatcaac attcttatac ctctttccgg cagattcgac 300

atgttcgtta ggttcatggg taacttcgag aagacctgct taattcctaa ccagaatgtt 360

aagttggtag ttttgttatt caattctgac agtaaccccg acaaagcaaa gcaggttgaa 420

ctgatgagag actataggat taagtaccca aaagcagata tgcagatact acctgtttcc 480

ggagaattct caagagcctt ggctctggaa gtaggcagct ctcagtttaa taatgagtct 540

cttctgttct tctgcgacgt tgacttagtt ttcacgacag agtttctgca gagatgtcgt 600

gcaaacactg tactaggtca gcagatatat ttccccatta ttttcagtca gtacgatcca 660

aagattgttt atagtggaaa agttccatct gacaaccatt ttgcgtttac tcaaaagaca 720

gggttttggc gtaactacgg gttcggcata acatgtatct ataaagggga cctggtaaga 780

gttggcggat tcgacgtatc tatacagggc tggggtttag aagacgtcga tctatttaat 840

aaagtagttc aggcaggttt gaagacattc cgtagtcaag aggtaggcgt tgtacacgtg 900

caccatccag tattttgcga cccaaactta gacccgaagc aatataaaat gtgcttgggt 960

tccaaggcat cgacttacgg ttccacgcag caactagcag agatgtggtt ggagaagaac 1020

gacccatctt atagcctgtt tgcagcatgg aagcatggac tttcgggcat ttggctcgca 1080

ttcgagtgct tataccagat tacttatttc ttcctggtga tttacctatt ctcccgtttg 1140

gctgttgagg ctgacccacg ggcgcaaacg gccaccgtca ttgtttcgac cacagttgcg 1200

cttattaagt gcggttactt tagcttcaga gctaaagaca ttagggcctt ctatttcgtc 1260

ctctacacgt tcgtttactt cttctgcatg attccggcac gaataactgc gatgatgacc 1320

ctgtgggaca taggttgggg aaccagggga ggaaatgaga agccttccgt aggcaccaga 1380

gttgctttgt gggccaagca atatttgatt gcttacatgt ggtgggccgc tgtcgtcggt 1440

gctggcgtgt actccatcgt tcataattgg atgtttgact ggaattcact ttcctacagg 1500

ttcgcactgg taggcatctg ttcgtatata gttttcattg taatagtgct ggtagtctac 1560

tttacaggaa agatcacgac ctggaacttc acgaagctac agaaagaatt gatcgaggac 1620

cgcgtactgt acgacgcaac gaccaatgcc cagtcggtat aa 1662

<210> 18
<211> 972
<212> PRT
<213> other sequences

<220>
<223> an amino acid sequence of a polypeptide having a chondroitin
      synthase activity (HCOS-1)

<400> 18
Met Asn Thr Leu Ser Gln Ala Ile Lys Ala Tyr Asn Ser Asn Asp Tyr
1               5                   10                  15
Gln Leu Ala Leu Lys Leu Phe Glu Lys Ser Ala Glu Ile Tyr Gly Arg
            20                  25                  30
Lys Ile Val Glu Phe Gln Ile Thr Lys Cys Lys Glu Lys Leu Ser Ala
        35                  40                  45
His Pro Ser Val Asn Ser Ala His Leu Ser Val Asn Lys Glu Glu Lys
    50                  55                  60
Val Asn Val Cys Asp Ser Pro Leu Asp Ile Ala Thr Gln Leu Leu Leu
65                  70                  75                  80
Ser Asn Val Lys Lys Leu Val Leu Ser Asp Ser Glu Lys Asn Thr Leu
                85                  90                  95
Lys Asn Lys Trp Lys Leu Leu Thr Glu Lys Lys Ser Glu Asn Ala Glu
            100                 105                 110
Val Arg Ala Val Ala Leu Val Pro Lys Asp Phe Pro Lys Asp Leu Val
        115                 120                 125
Leu Ala Pro Leu Pro Asp His Val Asn Asp Phe Thr Trp Tyr Lys Lys
    130                 135                 140
Arg Lys Lys Arg Leu Gly Ile Lys Pro Glu His Gln His Val Gly Leu
145                 150                 155                 160
Ser Ile Ile Val Thr Thr Phe Asn Arg Pro Ala Ile Leu Ser Ile Thr
                165                 170                 175
Leu Ala Cys Leu Val Asn Gln Lys Thr His Tyr Pro Phe Glu Val Ile
            180                 185                 190
Val Thr Asp Asp Gly Ser Gln Glu Asp Leu Ser Pro Ile Ile Arg Gln
        195                 200                 205

Tyr Glu Asn Lys Leu Asp Ile Arg Tyr Val Arg Gln Lys Asp Tyr Gly
    210                 215                 220
Tyr Gln Leu Cys Ala Val Arg Asn Leu Gly Leu Arg Thr Ala Lys Tyr
225                 230                 235                 240
Asp Phe Val Ser Ile Leu Asp Cys Asp Met Ala Pro Gln Gln Leu Trp
                245                 250                 255
Val His Ser Tyr Leu Thr Glu Leu Leu Glu Asp Asp Asp Leu Thr Ile
            260                 265                 270
Ile Gly Pro Arg Lys Tyr Ile Asp Thr Gln His Ile Asp Pro Lys Asp
        275                 280                 285
Phe Leu Asn Asn Ala Ser Leu Leu Glu Ser Leu Pro Glu Val Lys Thr
    290                 295                 300
Asn Asn Ser Val Ala Ala Lys Gly Glu Gly Thr Val Ser Leu Asp Trp
305                 310                 315                 320
Arg Leu Glu Gln Phe Glu Lys Thr Glu Asn Leu Arg Leu Ser Asp Ser
                325                 330                 335
Pro Phe Arg Phe Phe Ala Ala Gly Asn Val Ala Phe Ala Lys Lys Trp
            340                 345                 350
Leu Asn Lys Ser Gly Phe Phe Asp Glu Glu Phe Asn His Trp Gly Gly
        355                 360                 365
Glu Asp Val Glu Phe Gly Tyr Arg Leu Phe Arg Tyr Gly Ser Phe Phe
    370                 375                 380
Lys Thr Ile Asp Gly Ile Met Ala Tyr His Gln Glu Pro Pro Gly Lys
385                 390                 395                 400
Glu Asn Glu Thr Asp Arg Glu Ala Gly Lys Asn Ile Thr Leu Asp Ile
                405                 410                 415
Met Arg Glu Lys Val Pro Tyr Ile Tyr Arg Lys Leu Leu Pro Ile Glu
            420                 425                 430
Asp Ser His Ile Asn Arg Val Pro Leu Val Ser Ile Tyr Ile Pro Ala
        435                 440                 445
Tyr Asn Cys Ala Asn Tyr Ile Gln Arg Cys Val Asp Ser Ala Leu Asn
    450                 455                 460
Gln Thr Val Val Asp Leu Glu Val Cys Ile Cys Asn Asp Gly Ser Thr
465                 470                 475                 480
Asp Asn Thr Leu Glu Val Ile Asn Lys Leu Tyr Gly Asn Asn Pro Arg
                485                 490                 495
Val Arg Ile Met Ser Lys Pro Asn Gly Gly Ile Ala Ser Ala Ser Asn
            500                 505                 510
Ala Ala Val Ser Phe Ala Lys Gly Tyr Tyr Ile Gly Gln Leu Asp Ser
        515                 520                 525
Asp Asp Tyr Leu Glu Pro Asp Ala Val Glu Leu Cys Leu Lys Glu Phe
    530                 535                 540
Leu Lys Asp Lys Thr Leu Ala Cys Val Tyr Thr Thr Asn Arg Asn Val
545                 550                 555                 560
Asn Pro Asp Gly Ser Leu Ile Ala Asn Gly Tyr Asn Trp Pro Glu Phe
                565                 570                 575
Ser Arg Glu Lys Leu Thr Thr Ala Met Ile Ala His His Phe Arg Met
            580                 585                 590
Phe Thr Ile Arg Ala Trp His Leu Thr Asp Gly Phe Asn Glu Lys Ile
        595                 600                 605
Glu Asn Ala Val Asp Tyr Asp Met Phe Leu Lys Leu Ser Glu Val Gly
    610                 615                 620
Lys Phe Lys His Leu Asn Lys Ile Cys Tyr Asn Arg Val Leu His Gly
625                 630                 635                 640
Asp Asn Thr Ser Ile Lys Lys Leu Gly Ile Gln Lys Lys Asn His Phe
                645                 650                 655
Val Val Val Asn Gln Ser Leu Asn Arg Gln Gly Ile Thr Tyr Tyr Asn
            660                 665                 670
Tyr Asp Glu Phe Asp Asp Leu Asp Glu Ser Arg Lys Tyr Ile Phe Asn
        675                 680                 685
Lys Thr Ala Glu Tyr Gln Glu Glu Ile Asp Ile Leu Lys Asp Ile Lys
    690                 695                 700
Ile Ile Gln Asn Lys Asp Ala Lys Ile Ala Val Ser Ile Phe Tyr Pro

705                 710                 715                 720
Asn Thr Leu Asn Gly Leu Val Lys Lys Leu Asn Asn Ile Ile Glu Tyr
                725                 730                 735
Asn Lys Asn Ile Phe Val Ile Val Leu His Val Asp Lys Asn His Leu
            740                 745                 750
Thr Pro Asp Ile Lys Lys Glu Ile Leu Ala Phe Tyr His Lys His Gln
        755                 760                 765
Val Asn Ile Leu Leu Asn Asn Asp Ile Ser Tyr Tyr Thr Ser Asn Arg
    770                 775                 780
Leu Ile Lys Thr Glu Ala His Leu Ser Asn Ile Asn Lys Leu Ser Gln
785                 790                 795                 800
Leu Asn Leu Asn Cys Glu Tyr Ile Ile Phe Asp Asn His Asp Ser Leu
                805                 810                 815
Phe Val Lys Asn Asp Ser Tyr Ala Tyr Met Lys Lys Tyr Asp Val Gly
            820                 825                 830
Met Asn Phe Ser Ala Leu Thr His Asp Trp Ile Glu Lys Ile Asn Ala
        835                 840                 845
His Pro Pro Phe Lys Lys Leu Ile Lys Thr Tyr Phe Asn Asp Asn Asp
    850                 855                 860
Leu Lys Ser Met Asn Val Lys Gly Ala Ser Gln Gly Met Phe Met Thr
865                 870                 875                 880
Tyr Ala Leu Ala His Glu Leu Leu Thr Ile Ile Lys Glu Val Ile Thr
                885                 890                 895
Ser Cys Gln Ser Ile Asp Ser Val Pro Glu Tyr Asn Thr Glu Asp Ile
            900                 905                 910
Trp Phe Gln Phe Ala Leu Leu Ile Leu Glu Lys Lys Thr Gly His Val
        915                 920                 925
Phe Asn Lys Thr Ser Thr Leu Thr Tyr Met Pro Trp Glu Arg Lys Leu
    930                 935                 940
Gln Trp Thr Asn Glu Gln Ile Glu Ser Ala Lys Arg Gly Glu Asn Ile
945                 950                 955                 960
Pro Val Asn Lys Phe Ile Ile Asn Ser Ile Thr Leu
                965                 970

<210> 19
<211> 972
<212> PRT
<213> other sequences

<220>
<223> an amino acid sequence of a polypeptide having a chondroitin
      synthase activity (HCOS1-2)

<400> 19
Met Asn Thr Leu Ser Gln Ala Ile Lys Ala Tyr Asn Ser Asn Asp Tyr
1               5                   10                  15
Gln Leu Ala Leu Lys Leu Phe Glu Lys Ser Ala Glu Ile Tyr Gly Arg
            20                  25                  30
Lys Ile Val Glu Phe Gln Ile Thr Lys Cys Lys Glu Lys Leu Ser Ala
        35                  40                  45
His Pro Ser Val Asn Ser Ala His Leu Ser Val Asn Lys Glu Glu Lys
    50                  55                  60
Val Asn Val Cys Asp Ser Pro Leu Asp Ile Ala Thr Gln Leu Leu Leu
65                  70                  75                  80
Ser Asn Val Lys Lys Leu Val Leu Ser Asp Ser Glu Lys Asn Thr Leu
                85                  90                  95
Lys Asn Lys Trp Lys Leu Leu Thr Glu Lys Lys Ser Glu Asn Ala Glu
            100                 105                 110
Val Arg Ala Val Ala Leu Val Pro Lys Asp Phe Pro Lys Asp Leu Val
        115                 120                 125
Leu Ala Pro Leu Pro Asp His Val Asn Asp Phe Thr Trp Tyr Lys Lys
    130                 135                 140

Arg Lys Lys Arg Leu Gly Ile Lys Pro Val Asn Lys Asn Ile Gly Leu
145                 150                 155                 160
Ser Ile Ile Ile Pro Thr Phe Asn Arg Ser Arg Ile Leu Asp Ile Thr
                165                 170                 175
Leu Ala Cys Leu Val Asn Gln Lys Thr His Tyr Pro Phe Glu Val Ile
            180                 185                 190
Val Thr Asp Asp Gly Ser Gln Glu Asp Leu Ser Pro Ile Ile Arg Gln
        195                 200                 205
Tyr Glu Asn Lys Leu Asp Ile Arg Tyr Val Arg Gln Lys Asp Tyr Gly
    210                 215                 220
Tyr Gln Leu Cys Ala Val Arg Asn Leu Gly Leu Arg Thr Ala Lys Tyr
225                 230                 235                 240
Asp Phe Val Ser Ile Leu Asp Cys Asp Met Ala Pro Gln Gln Leu Trp
                245                 250                 255
Val His Ser Tyr Leu Thr Glu Leu Leu Glu Asp Asp Asp Leu Thr Ile
            260                 265                 270
Ile Gly Pro Arg Lys Tyr Ile Asp Thr Gln His Ile Asp Pro Lys Asp
        275                 280                 285
Phe Leu Asn Asn Ala Ser Leu Leu Glu Ser Leu Pro Glu Val Lys Thr
    290                 295                 300
Asn Asn Ser Val Ala Ala Lys Gly Glu Gly Thr Val Ser Leu Asp Trp
305                 310                 315                 320
Arg Leu Glu Gln Phe Glu Lys Thr Glu Asn Leu Arg Leu Ser Asp Ser
                325                 330                 335
Pro Phe Arg Phe Phe Ser Cys Gly Asn Val Ala Phe Ser Lys Glu Trp
            340                 345                 350
Leu Asn Lys Val Gly Trp Phe Asp Glu Glu Phe Asn His Trp Gly Gly
        355                 360                 365
Glu Asp Val Glu Phe Gly Tyr Arg Leu Phe Arg Tyr Gly Ser Phe Phe
    370                 375                 380
Lys Thr Ile Asp Gly Ile Met Ala Tyr His Gln Glu Pro Pro Gly Lys
385                 390                 395                 400
Glu Asn Glu Thr Asp Arg Glu Ala Gly Lys Asn Ile Thr Leu Asp Ile
                405                 410                 415
Met Arg Glu Lys Val Pro Tyr Ile Tyr Arg Lys Leu Leu Pro Ile Glu
            420                 425                 430
Asp Ser His Ile Asn Arg Val Pro Leu Val Ser Ile Tyr Ile Pro Ala
        435                 440                 445
Tyr Asn Cys Ala Asn Tyr Ile Gln Arg Cys Val Asp Ser Ala Leu Asn
    450                 455                 460
Gln Thr Val Val Asp Leu Glu Val Cys Ile Cys Asn Asp Gly Ser Thr
465                 470                 475                 480
Asp Asn Thr Leu Glu Val Ile Asn Lys Leu Tyr Gly Asn Asn Pro Arg
                485                 490                 495
Val Arg Ile Met Ser Lys Pro Asn Gly Gly Ile Ala Ser Ala Ser Asn
            500                 505                 510
Ala Ala Val Ser Phe Ala Lys Gly Tyr Tyr Ile Gly Gln Leu Asp Ser
        515                 520                 525
Asp Asp Tyr Leu Glu Pro Asp Ala Val Glu Leu Cys Leu Lys Glu Phe
    530                 535                 540
Leu Lys Asp Lys Thr Leu Ala Cys Val Tyr Thr Thr Asn Arg Asn Val
545                 550                 555                 560
Asn Pro Asp Gly Ser Leu Ile Ala Asn Gly Tyr Asn Trp Pro Glu Phe
                565                 570                 575
Ser Arg Glu Lys Leu Thr Thr Ala Met Ile Ala His His Phe Arg Met
            580                 585                 590
Phe Thr Ile Arg Ala Trp His Leu Thr Asp Gly Phe Asn Glu Lys Ile
        595                 600                 605
Glu Asn Ala Val Asp Tyr Asp Met Phe Leu Lys Leu Ser Glu Val Gly
    610                 615                 620
Lys Phe Lys His Leu Asn Lys Ile Cys Tyr Asn Arg Val Leu His Gly
625                 630                 635                 640
Asp Asn Thr Ser Ile Lys Lys Leu Gly Ile Gln Lys Lys Asn His Phe

645                         650                         655
Val Val Val Asn Gln Ser Leu Asn Arg Gln Gly Ile Thr Tyr Tyr Asn
            660                         665                         670
Tyr Asp Glu Phe Asp Asp Leu Asp Glu Ser Arg Lys Tyr Ile Phe Asn
        675                         680                         685
Lys Thr Ala Glu Tyr Gln Glu Glu Ile Asp Ile Leu Lys Asp Ile Lys
    690                         695                         700
Ile Ile Gln Asn Lys Asp Ala Lys Ile Ala Val Ser Ile Phe Tyr Pro
705                         710                         715                         720
Asn Thr Leu Asn Gly Leu Val Lys Lys Leu Asn Asn Ile Ile Glu Tyr
                        725                         730                         735
Asn Lys Asn Ile Phe Val Ile Val Leu His Val Asp Lys Asn His Leu
            740                         745                         750
Thr Pro Asp Ile Lys Lys Glu Ile Leu Ala Phe Tyr His Lys His Gln
        755                         760                         765
Val Asn Ile Leu Leu Asn Asn Asp Ile Ser Tyr Tyr Thr Ser Asn Arg
    770                         775                         780
Leu Ile Lys Thr Glu Ala His Leu Ser Asn Ile Asn Lys Leu Ser Gln
785                         790                         795                         800
Leu Asn Leu Asn Cys Glu Tyr Ile Ile Phe Asp Asn His Asp Ser Leu
                        805                         810                         815
Phe Val Lys Asn Asp Ser Tyr Ala Tyr Met Lys Lys Tyr Asp Val Gly
            820                         825                         830
Met Asn Phe Ser Ala Leu Thr His Asp Trp Ile Glu Lys Ile Asn Ala
        835                         840                         845
His Pro Pro Phe Lys Lys Leu Ile Lys Thr Tyr Phe Asn Asp Asn Asp
    850                         855                         860
Leu Lys Ser Met Asn Val Lys Gly Ala Ser Gln Gly Met Phe Met Thr
865                         870                         875                         880
Tyr Ala Leu Ala His Glu Leu Leu Thr Ile Ile Lys Glu Val Ile Thr
                        885                         890                         895
Ser Cys Gln Ser Ile Asp Ser Val Pro Glu Tyr Asn Thr Glu Asp Ile
            900                         905                         910
Trp Phe Gln Phe Ala Leu Leu Ile Leu Glu Lys Lys Thr Gly His Val
        915                         920                         925
Phe Asn Lys Thr Ser Thr Leu Thr Tyr Met Pro Trp Glu Arg Lys Leu
    930                         935                         940
Gln Trp Thr Asn Glu Gln Ile Glu Ser Ala Lys Arg Gly Glu Asn Ile
945                         950                         955                         960
Pro Val Asn Lys Phe Ile Ile Asn Ser Ile Thr Leu
                        965                         970

<210> 20
<211> 972
<212> PRT
<213> other sequences

<220>
<223> amino acid sequence of a polypeptide having a chondroitin
      synthase activity (HCOS1-3)

<400> 20
Met Asn Thr Leu Ser Gln Ala Ile Lys Ala Tyr Asn Ser Asn Asp Tyr
1               5                   10                  15
Gln Leu Ala Leu Lys Leu Phe Glu Lys Ser Ala Glu Ile Tyr Gly Arg
            20                  25                  30
Lys Ile Val Glu Phe Gln Ile Thr Lys Cys Lys Glu Lys Leu Ser Ala
        35                  40                  45
His Pro Ser Val Asn Ser Ala His Leu Ser Val Asn Lys Glu Glu Lys
    50                  55                  60
Val Asn Val Cys Asp Ser Pro Leu Asp Ile Ala Thr Gln Leu Leu Leu
65                  70                  75                  80

Ser Asn Val Lys Lys Leu Val Leu Ser Asp Ser Glu Lys Asn Thr Leu
                85                  90                  95
Lys Asn Lys Trp Lys Leu Leu Thr Glu Lys Lys Ser Glu Asn Ala Glu
            100                 105                 110
Val Arg Ala Val Ala Leu Val Pro Lys Asp Phe Pro Lys Asp Leu Val
        115                 120                 125
Leu Ala Pro Leu Pro Asp His Val Asn Asp Phe Thr Trp Tyr Lys Asn
    130                 135                 140
Arg Lys Lys Ser Leu Gly Ile Lys Pro Val Asn Lys Asn Ile Gly Leu
145                 150                 155                 160
Ser Ile Ile Ile Pro Thr Phe Asn Arg Ser Arg Ile Leu Asp Ile Thr
                165                 170                 175
Leu Ala Cys Leu Val Asn Gln Lys Thr Asn Tyr Pro Phe Glu Val Val
            180                 185                 190
Val Ala Asp Asp Gly Ser Lys Glu Asn Leu Leu Thr Ile Val Gln Lys
        195                 200                 205
Tyr Glu Gln Lys Leu Asp Ile Lys Tyr Val Arg Gln Lys Asp Tyr Gly
    210                 215                 220
Tyr Gln Leu Cys Ala Val Arg Asn Leu Gly Leu Arg Thr Ala Lys Tyr
225                 230                 235                 240
Asp Phe Val Ser Ile Leu Asp Cys Asp Met Ala Pro Gln Gln Leu Trp
                245                 250                 255
Val His Ser Tyr Leu Thr Glu Leu Leu Glu Asp Asn Asp Ile Val Leu
            260                 265                 270
Ile Gly Pro Arg Lys Tyr Val Asp Thr His Asn Ile Thr Ala Glu Gln
        275                 280                 285
Phe Leu Asn Asp Pro Tyr Leu Ile Glu Ser Leu Pro Glu Thr Ala Thr
    290                 295                 300
Asn Asn Asn Pro Ser Ile Thr Ser Lys Gly Asn Ile Ser Leu Asp Trp
305                 310                 315                 320
Arg Leu Glu His Phe Lys Lys Thr Asp Asn Leu Arg Leu Cys Asp Ser
                325                 330                 335
Pro Phe Arg Tyr Phe Ser Cys Gly Asn Val Ala Phe Ser Lys Glu Trp
            340                 345                 350
Leu Asn Lys Val Gly Trp Phe Asp Glu Glu Phe Asn His Trp Gly Gly
        355                 360                 365
Glu Asp Val Glu Phe Gly Tyr Arg Leu Phe Ala Lys Gly Cys Phe Phe
    370                 375                 380
Arg Val Ile Asp Gly Gly Met Ala Tyr His Gln Glu Pro Pro Gly Lys
385                 390                 395                 400
Glu Asn Glu Thr Asp Arg Glu Ala Gly Lys Ser Ile Thr Leu Lys Ile
                405                 410                 415
Val Lys Glu Lys Val Pro Tyr Ile Tyr Arg Lys Leu Leu Pro Ile Glu
            420                 425                 430
Asp Ser His Ile Asn Arg Val Pro Leu Val Ser Ile Tyr Ile Pro Ala
        435                 440                 445
Tyr Asn Cys Ala Asn Tyr Ile Gln Arg Cys Val Asp Ser Ala Leu Asn
    450                 455                 460
Gln Thr Val Val Asp Leu Glu Val Cys Ile Cys Asn Asp Gly Ser Thr
465                 470                 475                 480
Asp Asn Thr Leu Glu Val Ile Asn Lys Leu Tyr Gly Asn Asn Pro Arg
                485                 490                 495
Val Arg Ile Met Ser Lys Pro Asn Gly Gly Ile Ala Ser Ala Ser Asn
            500                 505                 510
Ala Ala Val Ser Phe Ala Lys Gly Tyr Tyr Ile Gly Gln Leu Asp Ser
        515                 520                 525
Asp Asp Tyr Leu Glu Pro Asp Ala Val Glu Leu Cys Leu Lys Glu Phe
    530                 535                 540
Leu Lys Asp Lys Thr Leu Ala Cys Val Tyr Thr Thr Asn Arg Asn Val
545                 550                 555                 560
Asn Pro Asp Gly Ser Leu Ile Ala Asn Gly Tyr Asn Trp Pro Glu Phe
                565                 570                 575
Ser Arg Glu Lys Leu Thr Thr Ala Met Ile Ala His His Phe Arg Met

580                 585                 590
Phe Thr Ile Arg Ala Trp His Leu Thr Asp Gly Phe Asn Glu Lys Ile
        595                 600                 605
Glu Asn Ala Val Asp Tyr Asp Met Phe Leu Lys Leu Ser Glu Val Gly
    610                 615                 620
Lys Phe Lys His Leu Asn Lys Ile Cys Tyr Asn Arg Val Leu His Gly
625                 630                 635                 640
Asp Asn Thr Ser Ile Lys Lys Leu Gly Ile Gln Lys Lys Asn His Phe
                645                 650                 655
Val Val Val Asn Gln Ser Leu Asn Arg Gln Gly Ile Thr Tyr Tyr Asn
            660                 665                 670
Tyr Asp Glu Phe Asp Asp Leu Asp Glu Ser Arg Lys Tyr Ile Phe Asn
        675                 680                 685
Lys Thr Ala Glu Tyr Gln Glu Glu Ile Asp Ile Leu Lys Asp Ile Lys
    690                 695                 700
Ile Ile Gln Asn Lys Asp Ala Lys Ile Ala Val Ser Ile Phe Tyr Pro
705                 710                 715                 720
Asn Thr Leu Asn Gly Leu Val Lys Lys Leu Asn Asn Ile Ile Glu Tyr
                725                 730                 735
Asn Lys Asn Ile Phe Val Ile Val Leu His Val Asp Lys Asn His Leu
            740                 745                 750
Thr Pro Asp Ile Lys Lys Glu Ile Leu Ala Phe Tyr His Lys His Gln
        755                 760                 765
Val Asn Ile Leu Leu Asn Asn Asp Ile Ser Tyr Tyr Thr Ser Asn Arg
    770                 775                 780
Leu Ile Lys Thr Glu Ala His Leu Ser Asn Ile Asn Lys Leu Ser Gln
785                 790                 795                 800
Leu Asn Leu Asn Cys Glu Tyr Ile Ile Phe Asp Asn His Asp Ser Leu
                805                 810                 815
Phe Val Lys Asn Asp Ser Tyr Ala Tyr Met Lys Lys Tyr Asp Val Gly
            820                 825                 830
Met Asn Phe Ser Ala Leu Thr His Asp Trp Ile Glu Lys Ile Asn Ala
        835                 840                 845
His Pro Pro Phe Lys Lys Leu Ile Lys Thr Tyr Phe Asn Asp Asn Asp
    850                 855                 860
Leu Lys Ser Met Asn Val Lys Gly Ala Ser Gln Gly Met Phe Met Thr
865                 870                 875                 880
Tyr Ala Leu Ala His Glu Leu Leu Thr Ile Ile Lys Glu Val Ile Thr
                885                 890                 895
Ser Cys Gln Ser Ile Asp Ser Val Pro Glu Tyr Asn Thr Glu Asp Ile
            900                 905                 910
Trp Phe Gln Phe Ala Leu Leu Ile Leu Glu Lys Lys Thr Gly His Val
        915                 920                 925
Phe Asn Lys Thr Ser Thr Leu Thr Tyr Met Pro Trp Glu Arg Lys Leu
    930                 935                 940
Gln Trp Thr Asn Glu Gln Ile Glu Ser Ala Lys Arg Gly Glu Asn Ile
945                 950                 955                 960
Pro Val Asn Lys Phe Ile Ile Asn Ser Ile Thr Leu
                965                 970

<210> 21
<211> 1023
<212> PRT
<213> other sequences

<220>
<223> amino acid sequence of a polypeptide having chondroitin synthase
      activity (HCOS.Sc)

<400> 21
Met Asn Thr Leu Ser Gln Ala Ile Lys Ala Tyr Asn Ser Asn Asp Tyr
1               5                   10                  15

Gln Leu Ala Leu Lys Leu Phe Glu Lys Ser Ala Glu Ile Tyr Gly Arg
            20                  25                  30
Lys Ile Val Glu Phe Gln Ile Thr Lys Cys Lys Glu Lys Leu Ser Ala
        35                  40                  45
His Pro Ser Val Asn Ser Ala His Leu Ser Val Asn Lys Glu Glu Lys
    50                  55                  60
Val Asn Val Cys Asp Ser Pro Leu Asp Ile Ala Thr Gln Leu Leu Leu
65                  70                  75                  80
Ser Asn Val Lys Lys Leu Val Leu Ser Asp Ser Glu Lys Asn Thr Leu
                85                  90                  95
Lys Asn Lys Trp Lys Leu Leu Thr Glu Lys Lys Ser Glu Asn Ala Glu
            100                 105                 110
Val Arg Ala Val Ala Leu Val Pro Lys Asp Phe Pro Lys Asp Leu Val
        115                 120                 125
Leu Ala Pro Leu Pro Asp His Val Asn Asp Phe Thr Trp Tyr Lys Asn
    130                 135                 140
Arg Lys Lys Ser Leu Gly Ile Lys Pro Val Asn Lys Asn Ile Gly Leu
145                 150                 155                 160
Ser Ile Ile Ile Pro Thr Phe Asn Arg Ser Arg Ile Leu Asp Ile Thr
                165                 170                 175
Leu Ala Cys Leu Val Asn Gln Lys Thr Asn Tyr Pro Phe Glu Val Val
            180                 185                 190
Val Ala Asp Asp Gly Ser Lys Glu Asn Leu Leu Thr Ile Val Gln Lys
        195                 200                 205
Tyr Glu Gln Lys Leu Asp Ile Lys Tyr Val Arg Gln Lys Asp Tyr Gly
    210                 215                 220
Tyr Gln Leu Cys Ala Val Arg Asn Leu Gly Leu Arg Thr Ala Lys Tyr
225                 230                 235                 240
Asp Phe Val Ser Ile Leu Asp Cys Asp Met Ala Pro Gln Gln Leu Trp
                245                 250                 255
Val His Ser Tyr Leu Thr Glu Leu Leu Glu Asp Asn Asp Ile Val Leu
            260                 265                 270
Ile Gly Pro Arg Lys Tyr Val Asp Thr His Asn Ile Thr Ala Glu Gln
        275                 280                 285
Phe Leu Asn Asp Pro Tyr Leu Ile Glu Ser Leu Pro Glu Thr Ala Thr
    290                 295                 300
Asn Asn Asn Pro Ser Ile Thr Ser Lys Gly Asn Ile Ser Leu Asp Trp
305                 310                 315                 320
Arg Leu Glu His Phe Lys Lys Thr Asp Asn Leu Arg Leu Cys Asp Ser
                325                 330                 335
Pro Phe Arg Tyr Phe Ser Cys Gly Asn Val Ala Phe Ser Lys Glu Trp
            340                 345                 350
Leu Asn Lys Val Gly Trp Phe Asp Glu Glu Phe Asn His Trp Gly Gly
        355                 360                 365
Glu Asp Val Glu Phe Gly Tyr Arg Leu Phe Ala Lys Gly Cys Phe Phe
    370                 375                 380
Arg Val Ile Asp Gly Gly Met Ala Tyr His Gln Glu Pro Pro Gly Lys
385                 390                 395                 400
Glu Asn Glu Thr Asp Arg Glu Ala Gly Lys Ser Ile Thr Leu Lys Ile
                405                 410                 415
Val Lys Glu Lys Val Pro Tyr Ile Tyr Arg Lys Leu Leu Pro Ile Glu
            420                 425                 430
Asp Ser His Ile Asn Arg Val Pro Leu Val Ser Ile Tyr Ile Pro Ala
        435                 440                 445
Tyr Asn Cys Ala Asn Tyr Ile Gln Arg Cys Val Asp Ser Ala Leu Asn
    450                 455                 460
Gln Thr Val Val Asp Leu Glu Val Cys Ile Cys Asn Asp Gly Ser Thr
465                 470                 475                 480
Asp Asn Thr Leu Glu Val Ile Asn Lys Leu Tyr Gly Asn Asn Pro Arg
                485                 490                 495
Val Arg Ile Met Ser Lys Pro Asn Gly Gly Ile Ala Ser Ala Ser Asn
            500                 505                 510
Ala Ala Val Ser Phe Ala Lys Gly Tyr Tyr Ile Gly Gln Leu Asp Ser

        515                 520                 525
Asp Asp Tyr Leu Glu Pro Asp Ala Val Glu Leu Cys Leu Lys Glu Phe
    530                 535                 540
Leu Lys Asp Lys Thr Leu Ala Cys Val Tyr Thr Thr Asn Arg Asn Val
545                 550                 555                 560
Asn Pro Asp Gly Ser Leu Ile Ala Asn Gly Tyr Asn Trp Pro Glu Phe
                565                 570                 575
Ser Arg Glu Lys Leu Thr Thr Ala Met Ile Ala His His Phe Arg Met
            580                 585                 590
Phe Thr Ile Arg Ala Trp His Leu Thr Asp Gly Phe Asn Glu Lys Ile
        595                 600                 605
Glu Asn Ala Val Asp Tyr Asp Met Phe Leu Lys Leu Ser Glu Val Gly
    610                 615                 620
Lys Phe Lys His Leu Asn Lys Ile Cys Tyr Asn Arg Val Leu His Gly
625                 630                 635                 640
Asp Asn Thr Ser Ile Lys Lys Leu Gly Ile Gln Lys Lys Asn His Phe
                645                 650                 655
Val Val Val Asn Gln Ser Leu Asn Arg Gln Gly Ile Thr Tyr Tyr Asn
            660                 665                 670
Tyr Asp Glu Phe Asp Asp Leu Asp Glu Ser Arg Lys Tyr Ile Phe Asn
        675                 680                 685
Lys Thr Ala Glu Tyr Gln Glu Glu Ile Asp Ile Leu Lys Asp Ile Phe
    690                 695                 700
Ile Gln Met Leu Phe Ser Trp Phe Ser Ile Ala Asn Phe Val Leu Thr
705                 710                 715                 720
Phe Tyr Tyr Leu Ala Gly Ser Met Asn Leu Val Ile Lys His Gly Glu
                725                 730                 735
Ala Leu Phe Ile Phe Phe Lys Tyr Leu Ile Phe Cys Asp Leu Ala Ser
            740                 745                 750
Leu Phe Ile Ile Ser Met Gly Asn Arg Pro Gln Gly Ala Lys His Leu
        755                 760                 765
Phe Ile Thr Ser Met Val Ile Leu Ser Ile Cys Ala Thr Tyr Ser Leu
    770                 775                 780
Ile Cys Gly Phe Val Phe Ala Phe Lys Ser Leu Ala Ser Gly Thr Glu
785                 790                 795                 800
Ser His Lys Ile Phe Val Asp Ile Val Ile Ser Leu Leu Ser Thr Tyr
                805                 810                 815
Gly Leu Tyr Phe Phe Ser Ser Leu Met Tyr Leu Asp Pro Trp His Met
            820                 825                 830
Phe Thr Ser Ser Ile Gln Tyr Phe Leu Thr Leu Pro Ala Phe Thr Cys
        835                 840                 845
Thr Leu Gln Ile Phe Ala Phe Cys Asn Thr His Asp Val Ser Trp Gly
    850                 855                 860
Thr Lys Gly Ser Thr Gln Glu Ser Lys Gln Leu Ser Lys Ala Ile Val
865                 870                 875                 880
Val Gln Gly Pro Asp Gly Lys Gln Ile Val Glu Thr Asp Trp Pro Gln
                885                 890                 895
Glu Val Asp Lys Lys Phe Leu Glu Ile Lys Ser Arg Leu Lys Glu Pro
            900                 905                 910
Glu Phe Glu Glu Ser Ser Gly Asn Glu Lys Gln Ser Lys Asn Asp Tyr
        915                 920                 925
Tyr Arg Asp Ile Arg Thr Arg Ile Val Met Ile Trp Met Leu Ser Asn
    930                 935                 940
Leu Ile Leu Ile Met Ser Ile Ile Gln Val Phe Thr Pro Gln Asp Thr
945                 950                 955                 960
Asp Asn Gly Tyr Leu Ile Phe Ile Leu Trp Ser Val Ala Ala Leu Ala
                965                 970                 975
Ala Phe Arg Val Val Gly Ser Met Ala Phe Leu Phe Met Lys Tyr Leu
            980                 985                 990
Arg Ile Ile Val Ser Tyr Arg Asn Lys Val Glu Gly Ser Gly Ser Trp
        995                 1000                1005
Glu Val Ser Lys Leu Asp Leu Pro Asn Val Phe His Lys Lys Gly
    1010                1015                1020

<210> 22
<211> 1023
<212> PRT
<213> other sequences

<220>
<223> an amino acid sequence of a polypeptide having chondroitin
      synthase activity (HHASA.Sc)

<400> 22
Met Asn Thr Leu Ser Gln Ala Ile Lys Ala Tyr Asn Ser Asn Asp Tyr
1               5                   10                  15
Gln Leu Ala Leu Lys Leu Phe Glu Lys Ser Ala Glu Ile Tyr Gly Arg
            20                  25                  30
Lys Ile Val Glu Phe Gln Ile Thr Lys Cys Lys Glu Lys Leu Ser Ala
        35                  40                  45
His Pro Ser Val Asn Ser Ala His Leu Ser Val Asn Lys Glu Glu Lys
    50                  55                  60
Val Asn Val Cys Asp Ser Pro Leu Asp Ile Ala Thr Gln Leu Leu Leu
65                  70                  75                  80
Ser Asn Val Lys Lys Leu Val Leu Ser Asp Ser Glu Lys Asn Thr Leu
                85                  90                  95
Lys Asn Lys Trp Lys Leu Leu Thr Glu Lys Lys Ser Glu Asn Ala Glu
            100                 105                 110
Val Arg Ala Val Ala Leu Val Pro Lys Asp Phe Pro Lys Asp Leu Val
        115                 120                 125
Leu Ala Pro Leu Pro Asp His Val Asn Asp Phe Thr Trp Tyr Lys Lys
    130                 135                 140
Arg Lys Lys Arg Leu Gly Ile Lys Pro Glu His Gln His Val Gly Leu
145                 150                 155                 160
Ser Ile Ile Val Thr Thr Phe Asn Arg Pro Ala Ile Leu Ser Ile Thr
                165                 170                 175
Leu Ala Cys Leu Val Asn Gln Lys Thr His Tyr Pro Phe Glu Val Ile
            180                 185                 190
Val Thr Asp Asp Gly Ser Gln Glu Asp Leu Ser Pro Ile Ile Arg Gln
        195                 200                 205
Tyr Glu Asn Lys Leu Asp Ile Arg Tyr Val Arg Gln Lys Asp Asn Gly
    210                 215                 220
Phe Gln Ala Ser Ala Ala Arg Asn Met Gly Leu Arg Leu Ala Lys Tyr
225                 230                 235                 240
Asp Phe Ile Gly Leu Leu Asp Cys Asp Met Ala Pro Asn Pro Leu Trp
                245                 250                 255
Val His Ser Tyr Val Ala Glu Leu Leu Glu Asp Asp Asp Leu Thr Ile
            260                 265                 270
Ile Gly Pro Arg Lys Tyr Ile Asp Thr Gln His Ile Asp Pro Lys Asp
        275                 280                 285
Phe Leu Asn Asn Ala Ser Leu Leu Glu Ser Leu Pro Glu Val Lys Thr
    290                 295                 300
Asn Asn Ser Val Ala Ala Lys Gly Glu Gly Thr Val Ser Leu Asp Trp
305                 310                 315                 320
Arg Leu Glu Gln Phe Glu Lys Thr Glu Asn Leu Arg Leu Ser Asp Ser
                325                 330                 335
Pro Phe Arg Phe Phe Ala Ala Gly Asn Val Ala Phe Ala Lys Lys Trp
            340                 345                 350
Leu Asn Lys Ser Gly Phe Phe Asp Glu Glu Phe Asn His Trp Gly Gly
        355                 360                 365
Glu Asp Val Glu Phe Gly Tyr Arg Leu Phe Arg Tyr Gly Ser Phe Phe
    370                 375                 380
Lys Thr Ile Asp Gly Ile Met Ala Tyr His Gln Glu Pro Pro Gly Lys
385                 390                 395                 400
Glu Asn Glu Thr Asp Arg Glu Ala Gly Lys Asn Ile Thr Leu Asp Ile

                405                 410                 415
Met Arg Glu Lys Val Pro Tyr Ile Tyr Arg Lys Leu Leu Pro Ile Glu
            420                 425                 430
Asp Ser His Ile Asn Arg Val Pro Leu Val Ser Ile Tyr Ile Pro Ala
        435                 440                 445
Tyr Asn Cys Ala Asn Tyr Ile Gln Arg Cys Val Asp Ser Ala Leu Asn
    450                 455                 460
Gln Thr Val Val Asp Leu Glu Val Cys Ile Cys Asn Asp Gly Ser Thr
465                 470                 475                 480
Asp Asn Thr Leu Glu Val Ile Asn Lys Leu Tyr Gly Asn Asn Pro Arg
                485                 490                 495
Val Arg Ile Met Ser Lys Pro Asn Gly Gly Ile Ala Ser Ala Ser Asn
            500                 505                 510
Ala Ala Val Ser Phe Ala Lys Gly Tyr Tyr Ile Gly Gln Leu Asp Ser
        515                 520                 525
Asp Asp Tyr Leu Glu Pro Asp Ala Val Glu Leu Cys Leu Lys Glu Phe
    530                 535                 540
Leu Lys Asp Lys Thr Leu Ala Cys Val Tyr Thr Thr Asn Arg Asn Val
545                 550                 555                 560
Asn Pro Asp Gly Ser Leu Ile Ala Asn Gly Tyr Asn Trp Pro Glu Phe
                565                 570                 575
Ser Arg Glu Lys Leu Thr Thr Ala Met Ile Ala His His Phe Arg Met
            580                 585                 590
Phe Thr Ile Arg Ala Trp His Leu Thr Asp Gly Phe Asn Glu Lys Ile
        595                 600                 605
Glu Asn Ala Val Asp Tyr Asp Met Phe Leu Lys Leu Ser Glu Val Gly
    610                 615                 620
Lys Phe Lys His Leu Asn Lys Ile Cys Tyr Asn Arg Val Leu His Gly
625                 630                 635                 640
Asp Asn Thr Ser Ile Lys Lys Leu Gly Ile Gln Lys Lys Asn His Phe
                645                 650                 655
Val Val Val Asn Gln Ser Leu Asn Arg Gln Gly Ile Thr Tyr Tyr Asn
            660                 665                 670
Tyr Asp Glu Phe Asp Asp Leu Asp Glu Ser Arg Lys Tyr Ile Phe Asn
        675                 680                 685
Lys Thr Ala Glu Tyr Gln Glu Glu Ile Asp Ile Leu Lys Asp Ile Phe
    690                 695                 700
Ile Gln Met Leu Phe Ser Trp Phe Ser Ile Ala Asn Phe Val Leu Thr
705                 710                 715                 720
Phe Tyr Tyr Leu Ala Gly Ser Met Asn Leu Val Ile Lys His Gly Glu
                725                 730                 735
Ala Leu Phe Ile Phe Phe Lys Tyr Leu Ile Phe Cys Asp Leu Ala Ser
            740                 745                 750
Leu Phe Ile Ile Ser Met Gly Asn Arg Pro Gln Gly Ala Lys His Leu
        755                 760                 765
Phe Ile Thr Ser Met Val Ile Leu Ser Ile Cys Ala Thr Tyr Ser Leu
    770                 775                 780
Ile Cys Gly Phe Val Phe Ala Phe Lys Ser Leu Ala Ser Gly Thr Glu
785                 790                 795                 800
Ser His Lys Ile Phe Val Asp Ile Val Ile Ser Leu Leu Ser Thr Tyr
                805                 810                 815
Gly Leu Tyr Phe Phe Ser Ser Leu Met Tyr Leu Asp Pro Trp His Met
            820                 825                 830
Phe Thr Ser Ser Ile Gln Tyr Phe Leu Thr Leu Pro Ala Phe Thr Cys
        835                 840                 845
Thr Leu Gln Ile Phe Ala Phe Cys Asn Thr His Asp Val Ser Trp Gly
    850                 855                 860
Thr Lys Gly Ser Thr Gln Glu Ser Lys Gln Leu Ser Lys Ala Ile Val
865                 870                 875                 880
Val Gln Gly Pro Asp Gly Lys Gln Ile Val Glu Thr Asp Trp Pro Gln
                885                 890                 895
Glu Val Asp Lys Lys Phe Leu Glu Ile Lys Ser Arg Leu Lys Glu Pro
            900                 905                 910

Glu Phe Glu Glu Ser Ser Gly Asn Glu Lys Gln Ser Lys Asn Asp Tyr
        915                 920                 925
Tyr Arg Asp Ile Arg Thr Arg Ile Val Met Ile Trp Met Leu Ser Asn
    930                 935                 940
Leu Ile Leu Ile Met Ser Ile Ile Gln Val Phe Thr Pro Gln Asp Thr
945                 950                 955                 960
Asp Asn Gly Tyr Leu Ile Phe Ile Leu Trp Ser Val Ala Ala Leu Ala
                965                 970                 975
Ala Phe Arg Val Val Gly Ser Met Ala Phe Leu Phe Met Lys Tyr Leu
            980                 985                 990
Arg Ile Ile Val Ser Tyr Arg Asn Lys Val Glu Gly Ser Gly Ser Trp
        995                 1000                1005
Glu Val Ser Lys Leu Asp Leu Pro Asn Val Phe His Lys Lys Gly
    1010                1015                1020

<210> 23
<211> 572
<212> PRT
<213> other sequences

<220>
<223> amino acid sequence of a polypeptide having chondroitin synthase
      activity (HCOS1-Vir)

<400> 23
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Gly Trp Asn Asp Val Arg Leu Ala Val Ile Ile Ala
                85                  90                  95
Gly Tyr Arg Glu Asp Pro Tyr Met Phe Gln Lys Cys Leu Glu Ser Val
            100                 105                 110
Arg Asp Ser Asp Tyr Gly Asn Val Ala Arg Leu Ile Cys Val Ile Asp
        115                 120                 125
Gly Asp Glu Asp Asp Asp Met Arg Met Ala Ala Val Tyr Lys Ala Ile
    130                 135                 140
Tyr Asn Asp Asn Ile Lys Lys Pro Glu Phe Val Leu Cys Glu Ser Asp
145                 150                 155                 160
Asp Lys Glu Gly Glu Arg Ile Asp Ser Asp Phe Ser Arg Asp Ile Cys
                165                 170                 175
Val Leu Gln Pro His Arg Gly Lys Arg Glu Cys Leu Tyr Thr Gly Phe
            180                 185                 190
Gln Leu Ala Lys Met Asp Pro Ser Val Asn Ala Val Val Leu Ile Asp
        195                 200                 205
Ser Asp Thr Val Leu Glu Lys Asp Ala Ile Leu Glu Val Val Tyr Pro
    210                 215                 220
Leu Ala Cys Asp Pro Glu Ile Gln Ala Val Ala Gly Glu Cys Lys Ile
225                 230                 235                 240
Trp Asn Thr Asp Thr Leu Leu Ser Leu Leu Val Ala Trp Arg Tyr Tyr
                245                 250                 255
Ser Ala Phe Cys Val Glu Arg Ser Ala Gln Ser Phe Phe Arg Thr Val
            260                 265                 270
Gln Cys Val Gly Gly Pro Leu Gly Ala Tyr Lys Ile Asp Ile Ile Lys
        275                 280                 285
Glu Ile Lys Asp Pro Trp Ile Ser Gln Arg Phe Leu Gly Gln Lys Cys

290                 295                 300
Thr Tyr Gly Asp Asp Arg Arg Leu Thr Asn Glu Ile Leu Met Arg Gly
305                 310                 315                 320
Lys Lys Val Val Phe Thr Pro Phe Ala Val Gly Trp Gly Trp Phe Asp
                325                 330                 335
Glu Glu Phe Thr His Trp Gly Gly Arg Tyr Ile Val Gln Gln Thr Arg
            340                 345                 350
Trp Ser Lys Ser Trp Cys Arg Glu Ile Trp Tyr Thr Leu Phe Ala Ala
        355                 360                 365
Trp Lys His Gly Leu Ser Gly Ile Trp Leu Ala Phe Glu Cys Leu Tyr
    370                 375                 380
Gln Ile Thr Tyr Phe Phe Leu Val Ile Tyr Leu Phe Ser Arg Leu Ala
385                 390                 395                 400
Val Glu Ala Asp Pro Arg Ala Gln Thr Ala Thr Val Ile Val Ser Thr
                405                 410                 415
Thr Val Ala Leu Ile Lys Cys Gly Tyr Phe Ser Phe Arg Ala Lys Asp
            420                 425                 430
Ile Arg Ala Phe Tyr Phe Val Leu Tyr Thr Phe Val Tyr Phe Phe Cys
        435                 440                 445
Met Ile Pro Ala Arg Ile Thr Ala Met Met Thr Leu Trp Asp Ile Gly
    450                 455                 460
Trp Gly Thr Arg Gly Gly Asn Glu Lys Pro Ser Val Gly Thr Arg Val
465                 470                 475                 480
Ala Leu Trp Ala Lys Gln Tyr Leu Ile Ala Tyr Met Trp Trp Ala Ala
                485                 490                 495
Val Val Gly Ala Gly Val Tyr Ser Ile Val His Asn Trp Met Phe Asp
            500                 505                 510
Trp Asn Ser Leu Ser Tyr Arg Phe Ala Leu Val Gly Ile Cys Ser Tyr
        515                 520                 525
Ile Val Phe Ile Val Ile Val Leu Val Val Tyr Phe Thr Gly Lys Ile
    530                 535                 540
Thr Thr Trp Asn Phe Thr Lys Leu Gln Lys Glu Leu Ile Glu Asp Arg
545                 550                 555                 560
Val Leu Tyr Asp Ala Thr Thr Asn Ala Gln Ser Val
                565                 570

<210> 24
<211> 571
<212> PRT
<213> other sequences

<220>
<223> reencoded amino acid sequence of a polypeptide having chondroitin
      synthase activity (HCOS2-Vir)

<400> 24
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Gly Trp Asn Asp Val Arg Leu Ala Val Ile Ile Ala
                85                  90                  95
Gly Tyr Arg Glu Asp Pro Tyr Met Phe Gln Lys Cys Leu Glu Ser Val
            100                 105                 110
Arg Asp Ser Asp Tyr Gly Asn Val Ala Arg Leu Ile Cys Val Ile Asp
        115                 120                 125

Gly Asp Glu Asp Asp Asp Met Arg Met Ala Ala Val Tyr Lys Ala Ile
    130                 135                 140
Tyr Asn Asp Asn Ile Lys Lys Pro Glu Phe Val Leu Cys Glu Ser Asp
145                 150                 155                 160
Asp Lys Glu Gly Glu Arg Ile Asp Ser Asp Phe Ser Arg Asp Ile Cys
                165                 170                 175
Val Leu Gln Pro His Arg Gly Lys Arg Glu Cys Leu Tyr Thr Gly Phe
            180                 185                 190
Gln Leu Ala Lys Met Asp Pro Ser Val Asn Ala Val Val Leu Ile Asp
        195                 200                 205
Ser Asp Thr Val Leu Glu Lys Asp Ala Ile Leu Glu Val Val Tyr Pro
    210                 215                 220
Leu Ala Cys Asp Pro Glu Ile Gln Ala Val Ala Gly Glu Cys Lys Ile
225                 230                 235                 240
Trp Asn Thr Asp Thr Leu Leu Ser Leu Leu Val Ala Trp Arg Tyr Tyr
                245                 250                 255
Ser Ala Phe Cys Val Glu Arg Ser Ala Gln Ser Phe Phe Arg Thr Val
            260                 265                 270
Gln Cys Val Gly Gly Pro Leu Gly Ala Tyr Lys Ile Asp Ile Ile Lys
        275                 280                 285
Glu Ile Lys Asp Pro Trp Ile Ser Gln Arg Phe Leu Gly Gln Lys Cys
    290                 295                 300
Thr Tyr Gly Asp Asp Arg Arg Leu Thr Asn Glu Ile Leu Met Arg Gly
305                 310                 315                 320
Lys Lys Val Val Phe Thr Pro Phe Ala Val Gly Trp Leu His Gly Glu
                325                 330                 335
Asn Thr Ser Ile Lys Lys Leu Arg Tyr Ile Val Gln Gln Thr Arg Trp
            340                 345                 350
Ser Lys Ser Trp Cys Arg Glu Ile Trp Tyr Thr Leu Phe Ala Ala Trp
        355                 360                 365
Lys His Gly Leu Ser Gly Ile Trp Leu Ala Phe Glu Cys Leu Tyr Gln
    370                 375                 380
Ile Thr Tyr Phe Phe Leu Val Ile Tyr Leu Phe Ser Arg Leu Ala Val
385                 390                 395                 400
Glu Ala Asp Pro Arg Ala Gln Thr Ala Thr Val Ile Val Ser Thr Thr
                405                 410                 415
Val Ala Leu Ile Lys Cys Gly Tyr Phe Ser Phe Arg Ala Lys Asp Ile
            420                 425                 430
Arg Ala Phe Tyr Phe Val Leu Tyr Thr Phe Val Tyr Phe Phe Cys Met
        435                 440                 445
Ile Pro Ala Arg Ile Thr Ala Met Met Thr Leu Trp Asp Ile Gly Trp
    450                 455                 460
Gly Thr Arg Gly Gly Asn Glu Lys Pro Ser Val Gly Thr Arg Val Ala
465                 470                 475                 480
Leu Trp Ala Lys Gln Tyr Leu Ile Ala Tyr Met Trp Trp Ala Ala Val
                485                 490                 495
Val Gly Ala Gly Val Tyr Ser Ile Val His Asn Trp Met Phe Asp Trp
            500                 505                 510
Asn Ser Leu Ser Tyr Arg Phe Ala Leu Val Gly Ile Cys Ser Tyr Ile
        515                 520                 525
Val Phe Ile Val Ile Val Leu Val Val Tyr Phe Thr Gly Lys Ile Thr
    530                 535                 540
Thr Trp Asn Phe Thr Lys Leu Gln Lys Glu Leu Ile Glu Asp Arg Val
545                 550                 555                 560
Leu Tyr Asp Ala Thr Thr Asn Ala Gln Ser Val
                565                 570

<210> 25
<211> 572
<212> PRT
<213> other sequences

<220>
<223> reencoded amino acid sequence of a polypeptide having chondroitin
      synthase activity (HCOS3-Vir)

<400> 25
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Gly Trp Asn Asp Val Arg Leu Ala Val Ile Ile Ala
                85                  90                  95
Gly Tyr Arg Glu Asp Pro Tyr Met Phe Gln Lys Cys Leu Glu Ser Val
            100                 105                 110
Arg Asp Ser Asp Tyr Gly Asn Val Ala Arg Leu Ile Cys Val Ile Asp
        115                 120                 125
Gly Asp Glu Asp Asp Asp Met Arg Met Ala Ala Val Tyr Lys Ala Ile
    130                 135                 140
Tyr Asn Asp Asn Ile Lys Lys Pro Glu Phe Val Leu Cys Glu Ser Asp
145                 150                 155                 160
Asp Lys Glu Gly Glu Arg Ile Asp Ser Asp Phe Ser Arg Asp Ile Cys
                165                 170                 175
Val Leu Gln Pro His Arg Gly Lys Arg Glu Cys Leu Tyr Thr Gly Phe
            180                 185                 190
Gln Leu Ala Lys Met Asp Pro Ser Val Asn Ala Val Val Leu Ile Asp
        195                 200                 205
Ser Asp Thr Val Leu Glu Lys Asp Ala Ile Leu Glu Val Val Tyr Pro
    210                 215                 220
Leu Ala Cys Asp Pro Glu Ile Gln Ala Val Ala Gly Glu Cys Lys Ile
225                 230                 235                 240
Trp Asn Thr Asp Thr Leu Leu Ser Leu Leu Val Ala Trp Arg Tyr Tyr
                245                 250                 255
Ser Ala Phe Cys Val Glu Arg Ser Ala Gln Ser Phe Phe Arg Thr Val
            260                 265                 270
Gln Cys Val Gly Gly Pro Leu Gly Ala Tyr Lys Ile Asp Ile Ile Lys
        275                 280                 285
Glu Ile Lys Asp Pro Trp Ile Ser Gln Arg Phe Leu Gly Gln Lys Cys
    290                 295                 300
Thr Tyr Gly Asp Asp Arg Arg Leu Thr Asn Glu Ile Leu Met Arg Gly
305                 310                 315                 320
Lys Lys Val Val Phe Thr Pro Phe Ala Val Gly Trp His Met Ala Trp
                325                 330                 335
Ser Asp Lys Asp Asp Ala Ile Asp Arg Tyr Ile Val Gln Gln Thr Arg
            340                 345                 350
Trp Ser Lys Ser Trp Cys Arg Glu Ile Trp Tyr Thr Leu Phe Ala Ala
        355                 360                 365
Trp Lys His Gly Leu Ser Gly Ile Trp Leu Ala Phe Glu Cys Leu Tyr
    370                 375                 380
Gln Ile Thr Tyr Phe Phe Leu Val Ile Tyr Leu Phe Ser Arg Leu Ala
385                 390                 395                 400
Val Glu Ala Asp Pro Arg Ala Gln Thr Ala Thr Val Ile Val Ser Thr
                405                 410                 415
Thr Val Ala Leu Ile Lys Cys Gly Tyr Phe Ser Phe Arg Ala Lys Asp
            420                 425                 430
Ile Arg Ala Phe Tyr Phe Val Leu Tyr Thr Phe Val Tyr Phe Phe Cys
        435                 440                 445
Met Ile Pro Ala Arg Ile Thr Ala Met Met Thr Leu Trp Asp Ile Gly
    450                 455                 460

Trp Gly Thr Arg Gly Gly Asn Glu Lys Pro Ser Val Gly Thr Arg Val
465                 470                 475                 480
Ala Leu Trp Ala Lys Gln Tyr Leu Ile Ala Tyr Met Trp Trp Ala Ala
                485                 490                 495
Val Val Gly Ala Gly Val Tyr Ser Ile Val His Asn Trp Met Phe Asp
            500                 505                 510
Trp Asn Ser Leu Ser Tyr Arg Phe Ala Leu Val Gly Ile Cys Ser Tyr
        515                 520                 525
Ile Val Phe Ile Val Ile Val Leu Val Val Tyr Phe Thr Gly Lys Ile
    530                 535                 540
Thr Thr Trp Asn Phe Thr Lys Leu Gln Lys Glu Leu Ile Glu Asp Arg
545                 550                 555                 560
Val Leu Tyr Asp Ala Thr Thr Asn Ala Gln Ser Val
                565                 570

<210> 26
<211> 570
<212> PRT
<213> other sequences

<220>
<223> reencoded amino acid sequence of a polypeptide having chondroitin
      synthase activity (HCOS4-Vir)

<400> 26
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Gly Trp Asn Asp Val Arg Leu Ala Val Ile Ile Ala
                85                  90                  95
Gly Tyr Arg Glu Asp Pro Tyr Met Phe Gln Lys Cys Leu Glu Ser Val
            100                 105                 110
Arg Asp Ser Asp Tyr Gly Asn Val Ala Arg Leu Ile Cys Val Ile Asp
        115                 120                 125
Gly Asp Glu Asp Asp Asp Met Arg Met Ala Ala Val Tyr Lys Ala Ile
    130                 135                 140
Tyr Asn Asp Asn Ile Lys Lys Pro Glu Phe Val Leu Cys Glu Ser Asp
145                 150                 155                 160
Asp Lys Glu Gly Glu Arg Ile Asp Ser Asp Phe Ser Arg Asp Ile Cys
                165                 170                 175
Val Leu Gln Pro His Arg Gly Lys Arg Glu Cys Leu Tyr Thr Gly Phe
            180                 185                 190
Gln Leu Ala Lys Met Asp Pro Ser Val Asn Ala Val Val Leu Ile Asp
        195                 200                 205
Ser Asp Thr Val Leu Glu Lys Asp Ala Ile Leu Glu Val Val Tyr Pro
    210                 215                 220
Leu Ala Cys Asp Pro Glu Ile Gln Ala Val Ala Gly Glu Cys Lys Ile
225                 230                 235                 240
Trp Asn Thr Asp Thr Leu Leu Ser Leu Leu Val Ala Trp Arg Tyr Tyr
                245                 250                 255
Ser Ala Phe Cys Val Glu Arg Ser Ala Gln Ser Phe Phe Arg Thr Val
            260                 265                 270
Gln Cys Val Gly Gly Pro Leu Gly Ala Tyr Lys Ile Asp Ile Ile Lys
        275                 280                 285
Glu Ile Lys Asp Pro Trp Ile Ser Gln Arg Phe Leu Gly Gln Lys Cys

290                 295                 300
Thr Tyr Gly Asp Asp Arg Arg Leu Thr Asn Glu Ile Leu Met Arg Gly
305                 310                 315                 320
Lys Lys Val Val Phe Thr Pro Phe Ala Val Gly Trp Phe Asp Glu Glu
                325                 330                 335
Phe Thr His Trp Gly Gly Arg Tyr Ile Val Gln Gln Thr Arg Trp Ser
            340                 345                 350
Lys Ser Trp Cys Arg Glu Ile Trp Tyr Thr Leu Phe Ala Ala Trp Lys
        355                 360                 365
His Gly Leu Ser Gly Ile Trp Leu Ala Phe Glu Cys Leu Tyr Gln Ile
    370                 375                 380
Thr Tyr Phe Phe Leu Val Ile Tyr Leu Phe Ser Arg Leu Ala Val Glu
385                 390                 395                 400
Ala Asp Pro Arg Ala Gln Thr Ala Thr Val Ile Val Ser Thr Thr Val
                405                 410                 415
Ala Leu Ile Lys Cys Gly Tyr Phe Ser Phe Arg Ala Lys Asp Ile Arg
            420                 425                 430
Ala Phe Tyr Phe Val Leu Tyr Thr Phe Val Tyr Phe Phe Cys Met Ile
        435                 440                 445
Pro Ala Arg Ile Thr Ala Met Met Thr Leu Trp Asp Ile Gly Trp Gly
    450                 455                 460
Thr Arg Gly Gly Asn Glu Lys Pro Ser Val Gly Thr Arg Val Ala Leu
465                 470                 475                 480
Trp Ala Lys Gln Tyr Leu Ile Ala Tyr Met Trp Trp Ala Ala Val Val
                485                 490                 495
Gly Ala Gly Val Tyr Ser Ile Val His Asn Trp Met Phe Asp Trp Asn
            500                 505                 510
Ser Leu Ser Tyr Arg Phe Ala Leu Val Gly Ile Cys Ser Tyr Ile Val
        515                 520                 525
Phe Ile Val Ile Val Leu Val Val Tyr Phe Thr Gly Lys Ile Thr Thr
    530                 535                 540
Trp Asn Phe Thr Lys Leu Gln Lys Glu Leu Ile Glu Asp Arg Val Leu
545                 550                 555                 560
Tyr Asp Ala Thr Thr Asn Ala Gln Ser Val
                565                 570

<210> 27
<211> 692
<212> PRT
<213> other sequences

<220>
<223> amino acid sequence of a polypeptide having chondroitin synthase
      activity (HCOS5-Vir)

<400> 27
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Ser Ile His His Pro Ile Ser Lys Thr Thr His Pro
                85                  90                  95
Asp Thr Glu Asn Ala Gly Asp Ser Thr Leu Leu Cys Pro Pro Met Asp
            100                 105                 110
Gly Met Asp Asp Ile Leu Val Val Met Lys Thr Gly Val Thr Glu Ala
        115                 120                 125

Lys Glu Lys Val Pro Val His Phe Gln Thr Thr Leu Arg Cys Val Pro
    130                 135                 140
His Tyr Val Ile Tyr Ser Asp Phe Glu Glu Glu Ile Glu Gly Val Lys
145                 150                 155                 160
Ile Tyr Asp Val Leu Gln Gly Met Asp Pro Asp Val Met Thr Gln Val
                165                 170                 175
Pro Asp Phe Asp Leu Tyr Asn Arg Ile Lys Lys Leu Gly Arg Gln Gly
            180                 185                 190
Leu Ala Glu Asn Asp Phe Ala Asp Glu Ala Asn Ser Ala Val Gly Lys
        195                 200                 205
Pro Asn Asn Pro Gly Trp Lys Leu Asp Lys Trp Lys Phe Leu Pro Met
    210                 215                 220
Ile Ile Glu Ala Leu Arg Tyr Lys Pro Lys Ala Lys Trp Tyr Val Phe
225                 230                 235                 240
Met Glu Ala Asp Thr Tyr Ile Ser Trp Pro Thr Leu Leu Ala Trp Leu
                245                 250                 255
Lys His Phe Asn His Lys Lys Pro Trp Tyr Leu Gly Thr Glu Thr Gln
            260                 265                 270
Ile Ala Asp Val Ile Phe Ala His Gly Gly Ser Gly Phe Met Ile Ser
        275                 280                 285
His Ala Ala Leu Lys Leu Ala Ala Glu Glu Tyr Thr Ala Arg Arg Thr
    290                 295                 300
Glu Leu His His Tyr Thr Asp Gly His Trp Ala Gly Asp Cys Val Leu
305                 310                 315                 320
Gly Lys Val Leu Ala Asp Ala Gly Val Asn Leu His Phe Ser Trp Pro
                325                 330                 335
Ile Leu Gln Asn Ser Asn Leu Gly Glu Leu Asp Glu Phe Asn Val Asp
            340                 345                 350
Leu Tyr Arg Gln Pro Trp Cys Phe Pro Ala Ala Ala Phe His His Leu
        355                 360                 365
Asn Ala Gln Lys Ile Gln Asp Leu Trp Ser Tyr Glu Gln Lys Arg Trp
    370                 375                 380
Gln Asp Lys Asp Lys Arg Val Leu Leu His Ser Asp Val Phe Arg Asp
385                 390                 395                 400
Leu Leu Tyr Pro Asp Ile Ala Leu Lys Pro Ala Arg Ile Gly Trp Asp
                405                 410                 415
Asn Leu Ser Ala Asp Glu Gln Ser Thr Ala Ser Ser Phe Glu Glu Cys
            420                 425                 430
Arg Gln Ile Cys Asp Gln Leu Asp Asp Cys Thr Gln Phe Ser Phe Arg
        435                 440                 445
Asp Lys Ser Cys Phe Thr Ser Arg Thr Pro Arg Leu Gly Ala Ala Asn
    450                 455                 460
Pro Asn Thr Val Ser Gly Trp Met Val Asp Arg Ile Arg Ala Asn Met
465                 470                 475                 480
Glu Lys Ser Gly Leu Phe Ala Ala Trp Lys His Gly Leu Ser Gly Ile
                485                 490                 495
Trp Leu Ala Phe Glu Cys Leu Tyr Gln Ile Thr Tyr Phe Phe Leu Val
            500                 505                 510
Ile Tyr Leu Phe Ser Arg Leu Ala Val Glu Ala Asp Pro Arg Ala Gln
        515                 520                 525
Thr Ala Thr Val Ile Val Ser Thr Thr Val Ala Leu Ile Lys Cys Gly
    530                 535                 540
Tyr Phe Ser Phe Arg Ala Lys Asp Ile Arg Ala Phe Tyr Phe Val Leu
545                 550                 555                 560
Tyr Thr Phe Val Tyr Phe Phe Cys Met Ile Pro Ala Arg Ile Thr Ala
                565                 570                 575
Met Met Thr Leu Trp Asp Ile Gly Trp Gly Thr Arg Gly Gly Asn Glu
            580                 585                 590
Lys Pro Ser Val Gly Thr Arg Val Ala Leu Trp Ala Lys Gln Tyr Leu
        595                 600                 605
Ile Ala Tyr Met Trp Trp Ala Ala Val Val Gly Ala Gly Val Tyr Ser
    610                 615                 620
Ile Val His Asn Trp Met Phe Asp Trp Asn Ser Leu Ser Tyr Arg Phe

625                 630                 635                 640
Ala Leu Val Gly Ile Cys Ser Tyr Ile Val Phe Ile Val Ile Val Leu
                645                 650                 655
Val Val Tyr Phe Thr Gly Lys Ile Thr Thr Trp Asn Phe Thr Lys Leu
            660                 665                 670
Gln Lys Glu Leu Ile Glu Asp Arg Val Leu Tyr Asp Ala Thr Thr Asn
        675                 680                 685
Ala Gln Ser Val
    690

<210> 28
<211> 910
<212> PRT
<213> other sequences


<220>
<223> amino acid sequence of a polypeptide having chondroitin synthase
      activity (HCOS6-Vir)

<400> 28
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Leu Asp Ile Ala Thr Gln Leu Leu Leu Ser Asn Val
                85                  90                  95
Lys Lys Leu Thr Leu Ser Glu Ser Glu Lys Asn Ser Leu Lys Asn Lys
            100                 105                 110
Trp Lys Ser Ile Thr Gly Lys Lys Ser Glu Asn Ala Glu Ile Arg Lys
        115                 120                 125
Val Glu Leu Val Pro Lys Asp Phe Pro Lys Asp Leu Val Leu Ala Pro
    130                 135                 140
Leu Pro Asp His Val Asn Asp Phe Thr Trp Tyr Lys Asn Arg Lys Lys
145                 150                 155                 160
Ser Leu Gly Ile Lys Pro Val Asn Lys Asn Ile Gly Leu Ser Ile Ile
                165                 170                 175
Ile Pro Thr Phe Asn Arg Ser Arg Ile Leu Asp Ile Thr Leu Ala Cys
            180                 185                 190
Leu Val Asn Gln Lys Thr Asn Tyr Pro Phe Glu Val Val Val Ala Asp
        195                 200                 205
Asp Gly Ser Lys Glu Asn Leu Leu Thr Ile Val Gln Lys Tyr Glu Gln
    210                 215                 220
Lys Leu Asp Ile Lys Tyr Val Arg Gln Lys Asp Tyr Gly Tyr Gln Leu
225                 230                 235                 240
Cys Ala Val Arg Asn Leu Gly Leu Arg Thr Ala Lys Tyr Asp Phe Val
                245                 250                 255
Ser Ile Leu Asp Cys Asp Met Ala Pro Gln Gln Leu Trp Val His Ser
            260                 265                 270
Tyr Leu Thr Glu Leu Leu Glu Asp Asn Asp Ile Val Leu Ile Gly Pro
        275                 280                 285
Arg Lys Tyr Val Asp Thr His Asn Ile Thr Ala Glu Gln Phe Leu Asn
    290                 295                 300
Asp Pro Tyr Leu Ile Glu Ser Leu Pro Glu Thr Ala Thr Asn Asn Asn
305                 310                 315                 320
Pro Ser Ile Thr Ser Lys Gly Asn Ile Ser Leu Asp Trp Arg Leu Glu
                325                 330                 335

His Phe Lys Lys Thr Asp Asn Leu Arg Leu Cys Asp Ser Pro Phe Arg
                340                 345                 350
Tyr Phe Ser Cys Gly Asn Val Ala Phe Ser Lys Glu Trp Leu Asn Lys
        355                 360                 365
Val Gly Trp Phe Asp Glu Glu Phe Asn His Trp Gly Gly Glu Asp Val
    370                 375                 380
Glu Phe Gly Tyr Arg Leu Phe Ala Lys Gly Cys Phe Phe Arg Val Ile
385                 390                 395                 400
Asp Gly Gly Met Ala Tyr His Gln Glu Pro Pro Gly Lys Glu Asn Glu
                405                 410                 415
Thr Asp Arg Glu Ala Gly Lys Ser Ile Thr Leu Lys Ile Val Lys Glu
            420                 425                 430
Lys Val Pro Tyr Ile Tyr Arg Lys Leu Leu Pro Ile Glu Asp Ser His
        435                 440                 445
Ile His Arg Ile Pro Leu Val Ser Ile Tyr Ile Pro Ala Tyr Asn Cys
    450                 455                 460
Ala Asn Tyr Ile Gln Arg Cys Val Asp Ser Ala Leu Asn Gln Thr Val
465                 470                 475                 480
Val Asp Leu Glu Val Cys Ile Cys Asn Asp Gly Ser Thr Asp Asn Thr
                485                 490                 495
Leu Glu Val Ile Asn Lys Leu Tyr Gly Asn Asn Pro Arg Val Arg Ile
            500                 505                 510
Met Ser Lys Pro Asn Gly Gly Ile Ala Ser Ala Ser Asn Ala Ala Val
        515                 520                 525
Ser Phe Ala Lys Gly Tyr Tyr Ile Gly Gln Leu Asp Ser Asp Asp Tyr
    530                 535                 540
Leu Glu Pro Asp Ala Val Glu Leu Cys Leu Lys Glu Phe Leu Lys Asp
545                 550                 555                 560
Lys Thr Leu Ala Cys Val Tyr Thr Thr Asn Arg Asn Val Asn Pro Asp
                565                 570                 575
Gly Ser Leu Ile Ala Asn Gly Tyr Asn Trp Pro Glu Phe Ser Arg Glu
            580                 585                 590
Lys Leu Thr Thr Ala Met Ile Ala His His Phe Arg Met Phe Thr Ile
        595                 600                 605
Arg Ala Trp His Leu Thr Asp Gly Phe Asn Glu Lys Ile Glu Asn Ala
    610                 615                 620
Val Asp Tyr Asp Met Phe Leu Lys Leu Ser Glu Val Gly Lys Phe Lys
625                 630                 635                 640
His Leu Asn Lys Ile Cys Tyr Asn Arg Val Leu His Gly Asp Asn Thr
                645                 650                 655
Ser Ile Lys Asn Leu Asp Thr Gln Lys Lys Asn His Phe Val Val Val
            660                 665                 670
Asn Gln Ser Leu Asn Arg Gln Arg Val Ser Asn Tyr Asn Tyr Asp Glu
        675                 680                 685
Phe Asp Asn Leu Asp Glu Ser Arg Lys Tyr Ile Phe Asn Lys Leu Phe
    690                 695                 700
Ala Ala Trp Lys His Gly Leu Ser Gly Ile Trp Leu Ala Phe Glu Cys
705                 710                 715                 720
Leu Tyr Gln Ile Thr Tyr Phe Phe Leu Val Ile Tyr Leu Phe Ser Arg
                725                 730                 735
Leu Ala Val Glu Ala Asp Pro Arg Ala Gln Thr Ala Thr Val Ile Val
            740                 745                 750
Ser Thr Thr Val Ala Leu Ile Lys Cys Gly Tyr Phe Ser Phe Arg Ala
        755                 760                 765
Lys Asp Ile Arg Ala Phe Tyr Phe Val Leu Tyr Thr Phe Val Tyr Phe
    770                 775                 780
Phe Cys Met Ile Pro Ala Arg Ile Thr Ala Met Met Thr Leu Trp Asp
785                 790                 795                 800
Ile Gly Trp Gly Thr Arg Gly Gly Asn Glu Lys Pro Ser Val Gly Thr
                805                 810                 815
Arg Val Ala Leu Trp Ala Lys Gln Tyr Leu Ile Ala Tyr Met Trp Trp
            820                 825                 830
Ala Ala Val Val Gly Ala Gly Val Tyr Ser Ile Val His Asn Trp Met

835                 840                 845
Phe Asp Trp Asn Ser Leu Ser Tyr Arg Phe Ala Leu Val Gly Ile Cys
    850                 855                 860
Ser Tyr Ile Val Phe Ile Val Ile Val Leu Val Val Tyr Phe Thr Gly
865                 870                 875                 880
Lys Ile Thr Thr Trp Asn Phe Thr Lys Leu Gln Lys Glu Leu Ile Glu
                885                 890                 895
Asp Arg Val Leu Tyr Asp Ala Thr Thr Asn Ala Gln Ser Val
            900                 905                 910

<210> 29
<211> 703
<212> PRT
<213> other sequences

<220>
<223> reencoded amino acid sequence of a polypeptide having chondroitin
      synthase activity (HCOS7-Vir)

<400> 29
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Ile Pro Thr Phe Asn Arg Ser Arg Ile Leu Asp Ile
                85                  90                  95
Thr Leu Ala Cys Leu Val Asn Gln Lys Thr Asn Tyr Pro Phe Glu Val
            100                 105                 110
Val Val Ala Asp Asp Gly Ser Lys Glu Asn Leu Leu Thr Ile Val Gln
        115                 120                 125
Lys Tyr Glu Gln Lys Leu Asp Ile Lys Tyr Val Arg Gln Lys Asp Tyr
    130                 135                 140
Gly Tyr Gln Leu Cys Ala Val Arg Asn Leu Gly Leu Arg Thr Ala Lys
145                 150                 155                 160
Tyr Asp Phe Val Ser Ile Leu Asp Cys Asp Met Ala Pro Gln Gln Leu
                165                 170                 175
Trp Val His Ser Tyr Leu Thr Glu Leu Leu Glu Asp Asn Asp Ile Val
            180                 185                 190
Leu Ile Gly Pro Arg Lys Tyr Val Asp Thr His Asn Ile Thr Ala Glu
        195                 200                 205
Gln Phe Leu Asn Asp Pro Tyr Leu Ile Glu Ser Leu Pro Glu Thr Ala
    210                 215                 220
Thr Asn Asn Asn Pro Ser Ile Thr Ser Lys Gly Asn Ile Ser Leu Asp
225                 230                 235                 240
Trp Arg Leu Glu His Phe Lys Lys Thr Asp Asn Leu Arg Leu Cys Asp
                245                 250                 255
Ser Pro Phe Arg Tyr Phe Ser Cys Gly Asn Val Ala Phe Ser Lys Glu
            260                 265                 270
Trp Leu Asn Lys Val Gly Trp Phe Asp Glu Glu Phe Asn His Trp Gly
        275                 280                 285
Gly Glu Asp Val Glu Phe Gly Tyr Arg Leu Phe Ala Lys Gly Cys Phe
    290                 295                 300
Phe Arg Val Ile Asp Gly Gly Met Ala Tyr His Gln Glu Pro Pro Gly
305                 310                 315                 320
Lys Glu Asn Glu Thr Asp Arg Glu Ala Gly Lys Ser Ile Thr Leu Lys
                325                 330                 335

Ile Val Lys Glu Lys Val Pro Tyr Ile Tyr Arg Lys Leu Leu Pro Ile
            340                 345                 350
Glu Asp Ser His Ile His Arg Ile Pro Leu Val Ser Ile Tyr Ile Pro
        355                 360                 365
Ala Tyr Asn Cys Ala Asn Tyr Ile Gln Arg Cys Val Asp Ser Ala Leu
    370                 375                 380
Asn Gln Thr Val Val Asp Leu Glu Val Cys Ile Cys Asn Asp Gly Ser
385                 390                 395                 400
Thr Asp Asn Thr Leu Glu Val Ile Asn Lys Leu Tyr Gly Asn Asn Pro
                405                 410                 415
Arg Val Arg Ile Met Ser Lys Pro Asn Gly Gly Ile Ala Ser Ala Ser
            420                 425                 430
Asn Ala Ala Val Ser Phe Ala Lys Gly Tyr Tyr Ile Gly Gln Leu Asp
        435                 440                 445
Ser Asp Asp Tyr Leu Glu Pro Asp Ala Val Glu Leu Cys Leu Lys Glu
    450                 455                 460
Phe Leu Lys Asp Lys Thr Leu Ala Cys Val Tyr Thr Thr Asn Arg Asn
465                 470                 475                 480
Val Asn Pro Asp Gly Ser Leu Ile Ala Asn Gly Tyr Asn Trp Pro Leu
                485                 490                 495
Phe Ala Ala Trp Lys His Gly Leu Ser Gly Ile Trp Leu Ala Phe Glu
            500                 505                 510
Cys Leu Tyr Gln Ile Thr Tyr Phe Phe Leu Val Ile Tyr Leu Phe Ser
        515                 520                 525
Arg Leu Ala Val Glu Ala Asp Pro Arg Ala Gln Thr Ala Thr Val Ile
    530                 535                 540
Val Ser Thr Thr Val Ala Leu Ile Lys Cys Gly Tyr Phe Ser Phe Arg
545                 550                 555                 560
Ala Lys Asp Ile Arg Ala Phe Tyr Phe Val Leu Tyr Thr Phe Val Tyr
                565                 570                 575
Phe Phe Cys Met Ile Pro Ala Arg Ile Thr Ala Met Met Thr Leu Trp
            580                 585                 590
Asp Ile Gly Trp Gly Thr Arg Gly Gly Asn Glu Lys Pro Ser Val Gly
        595                 600                 605
Thr Arg Val Ala Leu Trp Ala Lys Gln Tyr Leu Ile Ala Tyr Met Trp
    610                 615                 620
Trp Ala Ala Val Val Gly Ala Gly Val Tyr Ser Ile Val His Asn Trp
625                 630                 635                 640
Met Phe Asp Trp Asn Ser Leu Ser Tyr Arg Phe Ala Leu Val Gly Ile
                645                 650                 655
Cys Ser Tyr Ile Val Phe Ile Val Ile Val Leu Val Val Tyr Phe Thr
            660                 665                 670
Gly Lys Ile Thr Thr Trp Asn Phe Thr Lys Leu Gln Lys Glu Leu Ile
        675                 680                 685
Glu Asp Arg Val Leu Tyr Asp Ala Thr Thr Asn Ala Gln Ser Val
    690                 695                 700

<210> 30
<211> 569
<212> PRT
<213> other sequences

<220>
<223> reencoded amino acid sequence of a polypeptide having chondroitin
      synthase activity (HCOS8-Vir)

<400> 30
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp

35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Gly Trp Asn Asp Val Arg Leu Ala Val Ile Ile Ala
                85                  90                  95
Gly Tyr Arg Glu Asp Pro Tyr Met Phe Gln Lys Cys Leu Glu Ser Val
            100                 105                 110
Arg Asp Ser Asp Tyr Gly Asn Val Ala Arg Leu Ile Cys Val Ile Asp
        115                 120                 125
Gly Asp Glu Asp Asp Asp Met Arg Met Ala Ala Val Tyr Lys Ala Ile
    130                 135                 140
Tyr Asn Asp Asn Ile Lys Lys Pro Glu Phe Val Leu Cys Glu Ser Asp
145                 150                 155                 160
Asp Lys Glu Gly Glu Arg Ile Asp Ser Asp Phe Ser Arg Asp Ile Cys
                165                 170                 175
Val Leu Gln Pro His Arg Gly Lys Arg Glu Cys Leu Tyr Thr Gly Phe
            180                 185                 190
Gln Leu Ala Lys Met Asp Pro Ser Val Asn Ala Val Val Leu Ile Asp
        195                 200                 205
Ser Asp Thr Val Leu Glu Lys Asp Ala Ile Leu Glu Val Val Tyr Pro
    210                 215                 220
Leu Ala Cys Asp Pro Glu Ile Gln Ala Val Ala Gly Glu Cys Lys Ile
225                 230                 235                 240
Trp Asn Thr Asp Thr Leu Leu Ser Leu Leu Val Ala Trp Arg Tyr Tyr
                245                 250                 255
Ser Ala Phe Cys Val Glu Arg Ser Ala Gln Ser Phe Phe Arg Thr Val
            260                 265                 270
Gln Cys Val Arg Leu Cys Asp Ser Pro Phe Arg Tyr Phe Ser Cys Gly
        275                 280                 285
Asn Val Ala Phe Ser Lys Glu Trp Leu Asn Lys Val Gly Trp Phe Asp
    290                 295                 300
Glu Glu Phe Asn His Trp Gly Gly Glu Asp Val Glu Phe Gly Tyr Arg
305                 310                 315                 320
Leu Lys Lys Val Val Phe Thr Pro Phe Ala Val Gly Trp Ser Asp Ser
                325                 330                 335
Pro Thr Asn Val Phe Arg Tyr Ile Val Gln Gln Thr Arg Trp Ser Lys
            340                 345                 350
Ser Trp Cys Arg Glu Ile Trp Tyr Thr Leu Phe Ala Ala Trp Lys His
        355                 360                 365
Gly Leu Ser Gly Ile Trp Leu Ala Phe Glu Cys Leu Tyr Gln Ile Thr
    370                 375                 380
Tyr Phe Phe Leu Val Ile Tyr Leu Phe Ser Arg Leu Ala Val Glu Ala
385                 390                 395                 400
Asp Pro Arg Ala Gln Thr Ala Thr Val Ile Val Ser Thr Thr Val Ala
                405                 410                 415
Leu Ile Lys Cys Gly Tyr Phe Ser Phe Arg Ala Lys Asp Ile Arg Ala
            420                 425                 430
Phe Tyr Phe Val Leu Tyr Thr Phe Val Tyr Phe Phe Cys Met Ile Pro
        435                 440                 445
Ala Arg Ile Thr Ala Met Met Thr Leu Trp Asp Ile Gly Trp Gly Thr
    450                 455                 460
Arg Gly Gly Asn Glu Lys Pro Ser Val Gly Thr Arg Val Ala Leu Trp
465                 470                 475                 480
Ala Lys Gln Tyr Leu Ile Ala Tyr Met Trp Trp Ala Ala Val Val Gly
                485                 490                 495
Ala Gly Val Tyr Ser Ile Val His Asn Trp Met Phe Asp Trp Asn Ser
            500                 505                 510
Leu Ser Tyr Arg Phe Ala Leu Val Gly Ile Cys Ser Tyr Ile Val Phe
        515                 520                 525
Ile Val Ile Val Leu Val Val Tyr Phe Thr Gly Lys Ile Thr Thr Trp
    530                 535                 540

Asn Phe Thr Lys Leu Gln Lys Glu Leu Ile Glu Asp Arg Val Leu Tyr
545                 550                 555                 560
Asp Ala Thr Thr Asn Ala Gln Ser Val
                565

<210> 31
<211> 832
<212> PRT
<213> other sequences

<220>
<223> amino acid sequence of a polypeptide having chondroitin synthase
      activity (HCOS9-Vir)

<400> 31
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys His Ile Gly Lys Cys Leu Arg Glu Met Tyr Thr Thr
                85                  90                  95
His Glu Asp Val Glu Val Gly Arg Cys Val Arg Arg Phe Ala Gly Val
            100                 105                 110
Gln Cys Val Trp Ser Tyr Glu Met Gln Gln Leu Phe Tyr Glu Asn Tyr
        115                 120                 125
Glu Gln Asn Lys Lys Gly Tyr Ile Arg Asp Leu His Asn Ser Lys Ile
    130                 135                 140
His Gln Ala Ile Thr Leu His Pro Asn Lys Asn Pro Pro Tyr Gln Tyr
145                 150                 155                 160
Arg Leu His Ser Tyr Met Leu Ser Arg Lys Ile Ser Glu Leu Arg His
                165                 170                 175
Arg Thr Ile Gln Leu His Arg Glu Ile Val Leu Met Ser Lys Tyr Ser
            180                 185                 190
Asn Thr Glu Ile His Lys Glu Asp Leu Gln Leu Gly Ile Pro Pro Ser
        195                 200                 205
Phe Met Arg Phe Gln Pro Arg Gln Arg Glu Glu Ile Leu Glu Trp Glu
    210                 215                 220
Phe Leu Thr Gly Lys Tyr Leu Tyr Ser Ala Val Asp Gly Gln Pro Pro
225                 230                 235                 240
Arg Arg Gly Met Asp Ser Ala Gln Arg Glu Ala Leu Asp Asp Ile Val
                245                 250                 255
Met Gln Val Met Glu Met Ile Asn Ala Asn Ala Lys Thr Arg Gly Arg
            260                 265                 270
Ile Ile Asp Phe Lys Glu Ile Gln Tyr Gly Tyr Arg Arg Val Asn Pro
        275                 280                 285
Met Tyr Gly Ala Glu Tyr Ile Leu Asp Leu Leu Leu Leu Tyr Lys Lys
    290                 295                 300
His Lys Gly Lys Lys Met Thr Val Pro Val Arg Arg His Ala Tyr Leu
305                 310                 315                 320
Gln Gln Thr Phe Ser Lys Ile Gln Phe Val Glu His Glu Glu Leu Asp
                325                 330                 335
Ala Gln Glu Leu Ala Lys Arg Ile Asn Gln Glu Ser Gly Ser Leu Ser
            340                 345                 350
Phe Leu Ser Asn Ser Leu Lys Lys Leu Val Pro Phe Gln Leu Pro Gly
        355                 360                 365
Ser Lys Ser Glu His Lys Glu Pro Lys Asp Lys Lys Ile Asn Ile Leu

370                 375                 380
Ile Pro Leu Ser Gly Arg Phe Asp Met Phe Val Arg Phe Met Gly Asn
385                 390                 395                 400
Phe Glu Lys Thr Cys Leu Ile Pro Asn Gln Asn Val Lys Leu Val Val
                405                 410                 415
Leu Leu Phe Asn Ser Asp Ser Asn Pro Asp Lys Ala Lys Gln Val Glu
            420                 425                 430
Leu Met Arg Asp Tyr Arg Ile Lys Tyr Pro Lys Ala Asp Met Gln Ile
        435                 440                 445
Leu Pro Val Ser Gly Glu Phe Ser Arg Ala Leu Ala Leu Glu Val Gly
    450                 455                 460
Ser Ser Gln Phe Asn Asn Glu Ser Leu Leu Phe Phe Cys Asp Val Asp
465                 470                 475                 480
Leu Val Phe Thr Thr Glu Phe Leu Gln Arg Cys Arg Ala Asn Thr Val
                485                 490                 495
Leu Gly Gln Gln Ile Tyr Phe Pro Ile Ile Phe Ser Gln Tyr Asp Pro
            500                 505                 510
Lys Ile Val Tyr Ser Gly Lys Val Pro Ser Asp Asn His Phe Ala Phe
        515                 520                 525
Thr Gln Lys Thr Gly Phe Trp Arg Asn Tyr Gly Phe Gly Ile Thr Cys
    530                 535                 540
Ile Tyr Lys Gly Asp Leu Val Arg Val Gly Gly Phe Asp Val Ser Ile
545                 550                 555                 560
Gln Gly Trp Gly Leu Glu Asp Val Asp Leu Phe Asn Lys Val Val Gln
                565                 570                 575
Ala Gly Leu Lys Thr Phe Arg Ser Gln Glu Val Gly Val Val His Val
            580                 585                 590
His His Pro Val Phe Cys Asp Pro Asn Leu Asp Pro Lys Gln Tyr Lys
        595                 600                 605
Met Cys Leu Gly Ser Lys Ala Ser Thr Tyr Gly Ser Thr Gln Gln Leu
    610                 615                 620
Leu Phe Ala Ala Trp Lys His Gly Leu Ser Gly Ile Trp Leu Ala Phe
625                 630                 635                 640
Glu Cys Leu Tyr Gln Ile Thr Tyr Phe Phe Leu Val Ile Tyr Leu Phe
                645                 650                 655
Ser Arg Leu Ala Val Glu Ala Asp Pro Arg Ala Gln Thr Ala Thr Val
            660                 665                 670
Ile Val Ser Thr Thr Val Ala Leu Ile Lys Cys Gly Tyr Phe Ser Phe
        675                 680                 685
Arg Ala Lys Asp Ile Arg Ala Phe Tyr Phe Val Leu Tyr Thr Phe Val
    690                 695                 700
Tyr Phe Phe Cys Met Ile Pro Ala Arg Ile Thr Ala Met Met Thr Leu
705                 710                 715                 720
Trp Asp Ile Gly Trp Gly Thr Arg Gly Gly Asn Glu Lys Pro Ser Val
                725                 730                 735
Gly Thr Arg Val Ala Leu Trp Ala Lys Gln Tyr Leu Ile Ala Tyr Met
            740                 745                 750
Trp Trp Ala Ala Val Val Gly Ala Gly Val Tyr Ser Ile Val His Asn
        755                 760                 765
Trp Met Phe Asp Trp Asn Ser Leu Ser Tyr Arg Phe Ala Leu Val Gly
    770                 775                 780
Ile Cys Ser Tyr Ile Val Phe Ile Val Ile Val Leu Val Val Tyr Phe
785                 790                 795                 800
Thr Gly Lys Ile Thr Thr Trp Asn Phe Thr Lys Leu Gln Lys Glu Leu
                805                 810                 815
Ile Glu Asp Arg Val Leu Tyr Asp Ala Thr Thr Asn Ala Gln Ser Val
            820                 825                 830

<210> 32
<211> 1001
<212> PRT

<213> other sequences

<220>
<223> reencoded amino acid sequence of a polypeptide having chondroitin
      synthase activity (HCOS10-Vir)

<400> 32
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Arg Asp Arg Asn Phe Leu Phe Val Gly Val Met Thr
                85                  90                  95
Ala Gln Lys Tyr Leu Gln Thr Arg Ala Val Ala Ala Tyr Arg Thr Trp
            100                 105                 110
Ser Lys Thr Ile Pro Gly Lys Val Gln Phe Phe Ser Ser Glu Gly Ser
        115                 120                 125
Asp Thr Ser Val Pro Ile Pro Val Val Pro Leu Arg Gly Val Asp Asp
    130                 135                 140
Ser Tyr Pro Pro Gln Lys Lys Ser Phe Met Met Leu Lys Tyr Met His
145                 150                 155                 160
Asp His Tyr Leu Asp Lys Tyr Glu Trp Phe Met Arg Ala Asp Asp Asp
                165                 170                 175
Val Tyr Ile Lys Gly Asp Arg Leu Glu Asn Phe Leu Arg Ser Leu Asn
            180                 185                 190
Ser Ser Glu Pro Leu Phe Leu Gly Gln Thr Gly Leu Gly Thr Thr Glu
        195                 200                 205
Glu Met Gly Lys Leu Ala Leu Glu Pro Gly Glu Asn Phe Cys Met Gly
    210                 215                 220
Gly Pro Gly Val Ile Met Ser Arg Glu Val Leu Arg Arg Met Val Pro
225                 230                 235                 240
His Ile Gly Lys Cys Leu Arg Glu Met Tyr Thr Thr His Glu Asp Val
                245                 250                 255
Glu Val Gly Arg Cys Val Arg Arg Phe Ala Gly Val Gln Cys Val Trp
            260                 265                 270
Ser Tyr Glu Met Gln Gln Leu Phe Tyr Glu Asn Tyr Glu Gln Asn Lys
        275                 280                 285
Lys Gly Tyr Ile Arg Asp Leu His Asn Ser Lys Ile His Gln Ala Ile
    290                 295                 300
Thr Leu His Pro Asn Lys Asn Pro Pro Tyr Gln Tyr Arg Leu His Ser
305                 310                 315                 320
Tyr Met Leu Ser Arg Lys Ile Ser Glu Leu Arg His Arg Thr Ile Gln
                325                 330                 335
Leu His Arg Glu Ile Val Leu Met Ser Lys Tyr Ser Asn Thr Glu Ile
            340                 345                 350
His Lys Glu Asp Leu Gln Leu Gly Ile Pro Pro Ser Phe Met Arg Phe
        355                 360                 365
Gln Pro Arg Gln Arg Glu Glu Ile Leu Glu Trp Glu Phe Leu Thr Gly
    370                 375                 380
Lys Tyr Leu Tyr Ser Ala Val Asp Gly Gln Pro Pro Arg Arg Gly Met
385                 390                 395                 400
Asp Ser Ala Gln Arg Glu Ala Leu Asp Asp Ile Val Met Gln Val Met
                405                 410                 415
Glu Met Ile Asn Ala Asn Ala Lys Thr Arg Gly Arg Ile Ile Asp Phe
            420                 425                 430
Lys Glu Ile Gln Tyr Gly Tyr Arg Arg Val Asn Pro Met Tyr Gly Ala

435                 440                 445
Glu Tyr Ile Leu Asp Leu Leu Leu Leu Tyr Lys Lys His Lys Gly Lys
    450                 455                 460
Lys Met Thr Val Pro Val Arg Arg His Ala Tyr Leu Gln Gln Thr Phe
465                 470                 475                 480
Ser Lys Ile Gln Phe Val Glu His Glu Glu Leu Asp Ala Gln Glu Leu
                485                 490                 495
Ala Lys Arg Ile Asn Gln Glu Ser Gly Ser Leu Ser Phe Leu Ser Asn
            500                 505                 510
Ser Leu Lys Lys Leu Val Pro Phe Gln Leu Pro Gly Ser Lys Ser Glu
        515                 520                 525
His Lys Glu Pro Lys Asp Lys Lys Ile Asn Ile Leu Ile Pro Leu Ser
    530                 535                 540
Gly Arg Phe Asp Met Phe Val Arg Phe Met Gly Asn Phe Glu Lys Thr
545                 550                 555                 560
Cys Leu Ile Pro Asn Gln Asn Val Lys Leu Val Val Leu Leu Phe Asn
                565                 570                 575
Ser Asp Ser Asn Pro Asp Lys Ala Lys Gln Val Glu Leu Met Arg Asp
            580                 585                 590
Tyr Arg Ile Lys Tyr Pro Lys Ala Asp Met Gln Ile Leu Pro Val Ser
        595                 600                 605
Gly Glu Phe Ser Arg Ala Leu Ala Leu Glu Val Gly Ser Ser Gln Phe
    610                 615                 620
Asn Asn Glu Ser Leu Leu Phe Phe Cys Asp Val Asp Leu Val Phe Thr
625                 630                 635                 640
Thr Glu Phe Leu Gln Arg Cys Arg Ala Asn Thr Val Leu Gly Gln Gln
                645                 650                 655
Ile Tyr Phe Pro Ile Ile Phe Ser Gln Tyr Asp Pro Lys Ile Val Tyr
            660                 665                 670
Ser Gly Lys Val Pro Ser Asp Asn His Phe Ala Phe Thr Gln Lys Thr
        675                 680                 685
Gly Phe Trp Arg Asn Tyr Gly Phe Gly Ile Thr Cys Ile Tyr Lys Gly
    690                 695                 700
Asp Leu Val Arg Val Gly Gly Phe Asp Val Ser Ile Gln Gly Trp Gly
705                 710                 715                 720
Leu Glu Asp Val Asp Leu Phe Asn Lys Val Val Gln Ala Gly Leu Lys
                725                 730                 735
Thr Phe Arg Ser Gln Glu Val Gly Val Val His Val His His Pro Val
            740                 745                 750
Phe Cys Asp Pro Asn Leu Asp Pro Lys Gln Tyr Lys Met Cys Leu Gly
        755                 760                 765
Ser Lys Ala Ser Thr Tyr Gly Ser Thr Gln Gln Leu Ala Glu Met Trp
    770                 775                 780
Leu Glu Lys Asn Asp Pro Ser Tyr Ser Leu Phe Ala Ala Trp Lys His
785                 790                 795                 800
Gly Leu Ser Gly Ile Trp Leu Ala Phe Glu Cys Leu Tyr Gln Ile Thr
                805                 810                 815
Tyr Phe Phe Leu Val Ile Tyr Leu Phe Ser Arg Leu Ala Val Glu Ala
            820                 825                 830
Asp Pro Arg Ala Gln Thr Ala Thr Val Ile Val Ser Thr Thr Val Ala
        835                 840                 845
Leu Ile Lys Cys Gly Tyr Phe Ser Phe Arg Ala Lys Asp Ile Arg Ala
    850                 855                 860
Phe Tyr Phe Val Leu Tyr Thr Phe Val Tyr Phe Phe Cys Met Ile Pro
865                 870                 875                 880
Ala Arg Ile Thr Ala Met Met Thr Leu Trp Asp Ile Gly Trp Gly Thr
                885                 890                 895
Arg Gly Gly Asn Glu Lys Pro Ser Val Gly Thr Arg Val Ala Leu Trp
            900                 905                 910
Ala Lys Gln Tyr Leu Ile Ala Tyr Met Trp Trp Ala Ala Val Val Gly
        915                 920                 925
Ala Gly Val Tyr Ser Ile Val His Asn Trp Met Phe Asp Trp Asn Ser
    930                 935                 940

Leu Ser Tyr Arg Phe Ala Leu Val Gly Ile Cys Ser Tyr Ile Val Phe
945                 950                 955                 960
Ile Val Ile Val Leu Val Val Tyr Phe Thr Gly Lys Ile Thr Thr Trp
                965                 970                 975
Asn Phe Thr Lys Leu Gln Lys Glu Leu Ile Glu Asp Arg Val Leu Tyr
            980                 985                 990
Asp Ala Thr Thr Asn Ala Gln Ser Val
        995                 1000

<210> 33
<211> 531
<212> PRT
<213> other sequences

<220>
<223> reencoded amino acid sequence of a polypeptide having chondroitin
      synthase activity (HCOS11-Vir)

<400> 33
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Arg Asp Arg Asn Phe Leu Phe Val Gly Val Met Thr
                85                  90                  95
Ala Gln Lys Tyr Leu Gln Thr Arg Ala Val Ala Ala Tyr Arg Thr Trp
            100                 105                 110
Ser Lys Thr Ile Pro Gly Lys Val Gln Phe Phe Ser Ser Glu Gly Ser
        115                 120                 125
Asp Thr Ser Val Pro Ile Pro Val Val Pro Leu Arg Gly Val Asp Asp
    130                 135                 140
Ser Tyr Pro Pro Gln Lys Lys Ser Phe Met Met Leu Lys Tyr Met His
145                 150                 155                 160
Asp His Tyr Leu Asp Lys Tyr Glu Trp Phe Met Arg Ala Asp Asp Asp
                165                 170                 175
Val Tyr Ile Lys Gly Asp Arg Leu Glu Asn Phe Leu Arg Ser Leu Asn
            180                 185                 190
Ser Ser Glu Pro Leu Phe Leu Gly Gln Thr Gly Leu Gly Thr Thr Glu
        195                 200                 205
Glu Met Gly Lys Leu Ala Leu Glu Pro Gly Glu Asn Phe Cys Met Gly
    210                 215                 220
Gly Pro Gly Val Ile Met Ser Arg Glu Val Leu Arg Arg Met Val Pro
225                 230                 235                 240
His Ile Gly Lys Cys Leu Arg Glu Met Tyr Thr Thr His Glu Asp Val
                245                 250                 255
Glu Val Gly Arg Cys Val Arg Arg Phe Ala Gly Val Gln Cys Val Trp
            260                 265                 270
Ser Tyr Glu Met Gln Gln Leu Phe Tyr Glu Asn Tyr Glu Gln Asn Lys
        275                 280                 285
Lys Gly Tyr Ile Arg Asp Leu His Asn Ser Lys Ile His Gln Ala Ile
    290                 295                 300
Thr Leu His Pro Asn Lys Asn Pro Pro Tyr Gln Tyr Arg Leu His Ser
305                 310                 315                 320
Tyr Met Leu Leu Phe Ala Ala Trp Lys His Gly Leu Ser Gly Ile Trp
                325                 330                 335
Leu Ala Phe Glu Cys Leu Tyr Gln Ile Thr Tyr Phe Phe Leu Val Ile

340                 345                 350
Tyr Leu Phe Ser Arg Leu Ala Val Glu Ala Asp Pro Arg Ala Gln Thr
        355                 360                 365
Ala Thr Val Ile Val Ser Thr Thr Val Ala Leu Ile Lys Cys Gly Tyr
    370                 375                 380
Phe Ser Phe Arg Ala Lys Asp Ile Arg Ala Phe Tyr Phe Val Leu Tyr
385                 390                 395                 400
Thr Phe Val Tyr Phe Phe Cys Met Ile Pro Ala Arg Ile Thr Ala Met
                405                 410                 415
Met Thr Leu Trp Asp Ile Gly Trp Gly Thr Arg Gly Gly Asn Glu Lys
            420                 425                 430
Pro Ser Val Gly Thr Arg Val Ala Leu Trp Ala Lys Gln Tyr Leu Ile
        435                 440                 445
Ala Tyr Met Trp Trp Ala Ala Val Val Gly Ala Gly Val Tyr Ser Ile
    450                 455                 460
Val His Asn Trp Met Phe Asp Trp Asn Ser Leu Ser Tyr Arg Phe Ala
465                 470                 475                 480
Leu Val Gly Ile Cys Ser Tyr Ile Val Phe Ile Val Ile Val Leu Val
                485                 490                 495
Val Tyr Phe Thr Gly Lys Ile Thr Thr Trp Asn Phe Thr Lys Leu Gln
            500                 505                 510
Lys Glu Leu Ile Glu Asp Arg Val Leu Tyr Asp Ala Thr Thr Asn Ala
        515                 520                 525
Gln Ser Val
    530

<210> 34
<211> 553
<212> PRT
<213> other sequences

<220>
<223> reencoded amino acid sequence of a polypeptide having chondroitin
      synthase activity (HCOS12-Vir)

<400> 34
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Lys Asp Lys Lys Ile Asn Ile Leu Ile Pro Leu Ser
                85                  90                  95
Gly Arg Phe Asp Met Phe Val Arg Phe Met Gly Asn Phe Glu Lys Thr
            100                 105                 110
Cys Leu Ile Pro Asn Gln Asn Val Lys Leu Val Val Leu Leu Phe Asn
        115                 120                 125
Ser Asp Ser Asn Pro Asp Lys Ala Lys Gln Val Glu Leu Met Arg Asp
    130                 135                 140
Tyr Arg Ile Lys Tyr Pro Lys Ala Asp Met Gln Ile Leu Pro Val Ser
145                 150                 155                 160
Gly Glu Phe Ser Arg Ala Leu Ala Leu Glu Val Gly Ser Ser Gln Phe
                165                 170                 175
Asn Asn Glu Ser Leu Leu Phe Phe Cys Asp Val Asp Leu Val Phe Thr
            180                 185                 190
Thr Glu Phe Leu Gln Arg Cys Arg Ala Asn Thr Val Leu Gly Gln Gln
        195                 200                 205

Ile Tyr Phe Pro Ile Ile Phe Ser Gln Tyr Asp Pro Lys Ile Val Tyr
    210                 215                 220
Ser Gly Lys Val Pro Ser Asp Asn His Phe Ala Phe Thr Gln Lys Thr
225                 230                 235                 240
Gly Phe Trp Arg Asn Tyr Gly Phe Gly Ile Thr Cys Ile Tyr Lys Gly
                245                 250                 255
Asp Leu Val Arg Val Gly Gly Phe Asp Val Ser Ile Gln Gly Trp Gly
            260                 265                 270
Leu Glu Asp Val Asp Leu Phe Asn Lys Val Val Gln Ala Gly Leu Lys
        275                 280                 285
Thr Phe Arg Ser Gln Glu Val Gly Val Val His Val His His Pro Val
    290                 295                 300
Phe Cys Asp Pro Asn Leu Asp Pro Lys Gln Tyr Lys Met Cys Leu Gly
305                 310                 315                 320
Ser Lys Ala Ser Thr Tyr Gly Ser Thr Gln Gln Leu Ala Glu Met Trp
                325                 330                 335
Leu Glu Lys Asn Asp Pro Ser Tyr Ser Leu Phe Ala Ala Trp Lys His
            340                 345                 350
Gly Leu Ser Gly Ile Trp Leu Ala Phe Glu Cys Leu Tyr Gln Ile Thr
        355                 360                 365
Tyr Phe Phe Leu Val Ile Tyr Leu Phe Ser Arg Leu Ala Val Glu Ala
    370                 375                 380
Asp Pro Arg Ala Gln Thr Ala Thr Val Ile Val Ser Thr Thr Val Ala
385                 390                 395                 400
Leu Ile Lys Cys Gly Tyr Phe Ser Phe Arg Ala Lys Asp Ile Arg Ala
                405                 410                 415
Phe Tyr Phe Val Leu Tyr Thr Phe Val Tyr Phe Phe Cys Met Ile Pro
            420                 425                 430
Ala Arg Ile Thr Ala Met Met Thr Leu Trp Asp Ile Gly Trp Gly Thr
        435                 440                 445
Arg Gly Gly Asn Glu Lys Pro Ser Val Gly Thr Arg Val Ala Leu Trp
    450                 455                 460
Ala Lys Gln Tyr Leu Ile Ala Tyr Met Trp Trp Ala Ala Val Val Gly
465                 470                 475                 480
Ala Gly Val Tyr Ser Ile Val His Asn Trp Met Phe Asp Trp Asn Ser
                485                 490                 495
Leu Ser Tyr Arg Phe Ala Leu Val Gly Ile Cys Ser Tyr Ile Val Phe
            500                 505                 510
Ile Val Ile Val Leu Val Val Tyr Phe Thr Gly Lys Ile Thr Thr Trp
        515                 520                 525
Asn Phe Thr Lys Leu Gln Lys Glu Leu Ile Glu Asp Arg Val Leu Tyr
    530                 535                 540
Asp Ala Thr Thr Asn Ala Gln Ser Val
545                 550

<210> 35
<211> 1446
<212> DNA
<213> Arabidopsis thaliana

<220>
<223> nucleic acid sequence of UDP-Glucose dehydrogenase (HASB)

<400> 35
atggtcaaaa tatgctgtat cggggctgga tatgtcggtg ggcctacaat ggccgttatg      60

gccctaaagt gtccagagat tgaagtcgtg gtagttgata tatcggagcc acgaattaac     120

gcatggaact cagaccgtct accaatttat gagccagggt tagaggatgt cgtcaaacag     180

tgtagaggga agaatttgtt cttctctaca gatgtagaga agcatgtatt tgagtcagat     240

atagtgtttg tttcggtaaa cactcctacg aaaacgcagg gtctgggtgc aggtaaagca 300

gcagatttga catattggga gtccgctgct cgcatgatag ctgatgtgag caaatcgtcg 360

aaaatcgtcg ttgaaaagag tacagtacca gttcgtacag ccgaggctat agaaaagatt 420

ttaacgcaca attcgaaggg tatcgaattc cagatcttat caaatccaga attcttggcc 480

gaagggacgg cgattaaaga cttatataac cctgatagag ttctaatcgg tggcagggac 540

accgctgcgg gacaaaaggc cattaaggcg ttgcgtgacg tgtatgccca ctgggttcct 600

gttgagcaaa taatctgtac taatttatgg agtgccgagc tatcaaagtt ggctgcgaac 660

gcatttctag ctcaaaggat aagttcagta aacgcaatgt cagcgctttg tgaggcaact 720

ggtgctgacg taacccaagt cgctcacgcc gttggaaccg acactagaat tggaccgaag 780

tttcttaacg catccgtagg cttcggcgga tcttgctttc agaaagacat cctgaatctt 840

atttacatct gcgaatgcaa tggtcttcca gaagcagcca attattggaa acaggtagtc 900

aaggtaaatg actaccaaaa gattaggttt gctaatcgag tcgtatcttc tatgttcaac 960

accgtctccg gtaagaaaat tgctattttg ggatttgcgt tcaagaagga caccggcgac 1020

acgcgtgaaa ctcctgccat agatgtgtgt aatcgcctcg tggctgataa agcaaagctg 1080

tcgatctatg atccgcaagt attagaagag cagatccgcc gtgatctgtc catggcccga 1140

ttcgattggg accatccagt cccactccag cagatcaaag ctgaaggtat ctccgaacag 1200

gttaacgttg tgtccgacgc ctacgaggct acgaaggatg ctcatggttt atgtgtttta 1260

accgaatggg acgaattcaa gtcacttgat tttaagaaga tctttgataa tatgcagaaa 1320

cccgctttcg ttttcgacgg aagaaacgtg gtcgacgctg tgaaattgag agaaattgga 1380

ttcatagtat attccatagg taaacctctg gatagttggc tcaaggatat gccggctgtt 1440

gcataa 1446

<210> 36
<211> 1166
<212> DNA
<213> Chlorella virus


<220>
<223> reencoded nucleic acid sequence of UDP-Glucose dehydrogenase
      (HASB)

<400> 36
atgagtagaa ttgctgtcgt tggatgcggt tacgtgggta cggcctgcgc cgtacttttg 60

gcgcagaaga acgaagttat cgttttggat atctcggagg accgggtaca actgattaag 120

aataagaagt cacctataga agataaggaa atcgaagaat tcctggagac gaaagatttg 180

aatctaacag cgacgacgga taaggtgctc gcctatgaga atgctgaatt cgttataata 240

gctacaccga ccgattacga tgtcgtcact agatatttca acactaagtc tgttgaaaat 300

gtgataggcg atgtcattaa gaacactcag acacacccta ctatagtgat caagagtact 360

attccaatcg gtttcgtgga taaagttaga gaacagttcg attatcaaaa tattatcttc 420

tcgccggaat tcttgagaga aggacgagca ttatatgata atctttaccc ctcccgtatc 480

atcgtcggtg atgactcccc aattgcctta aaattcgcga atctcttggt cgagggtagc 540

aaaactccac tagctcccgt attaactatg ggtacgcgag aggccgaagc tgtaaaacta 600

ttttcaaata catatttggc tatgagagta gcatacttca atgagctaga cacatttgca 660

atgtcccatg gtatgaacgc aaaagagatt atcgacggag tcacactaga accaaggata 720

gggcagggtt attcgaatcc ttcatttggg tatggagcct attgtttccc aaaagataca 780

aagcaattgt tggctaattt tgaaggggtt ccacaagata ttattggcgc aatcgtagag 840

tctaacgaaa ctagaaagga ggttattgtt tctgaggtcg agaacagatt tccgaccaca 900

gttggcgtat ataagttggc tgctaaagct ggttcggata atttcaggtc tagtgcgata 960

gtcgatatca tggaaaggtt ggctaataag ggctaccaca tcaaaatatt tgaacctacc 1020

gttgaacaat ttgagaattt tgaggtagat aataatctca caacttttgc aaccgagtct 1080

gatgtaataa ttgcgaacag agttccagtc gaacatcgca ttctgtttgg gaagaagtta 1140

ataacacggg atgtctatgg ggataa 1166

<210> 37
<211> 1170
<212> DNA
<213> Chlorella virus

<220>
<223> reencoded nucleic acid sequence of UDP-Glucose dehydrogenase
(HASB-A)

<400> 37
atgtcacgaa tcgcagtagt tggctgtggg tacgtgggaa ccgcatgcgc tgtactgctg 60

gcgcaaaaga atgaggtaat cgttcttgat attagtgagg atagggtcca actaatcaag 120

aacaagaaat ccccgatcga agataaggag attgaagaat tcttggaaac aaaagaccta 180

aacttaactg caacaactga caaagtttta gcctacgaaa acgctgagtt tgtgattata 240

gcgacaccca cagattatga cgtagttacc agatacttta acacgaagtc cgtagagaac 300

gtcattggag atgttataaa gaatactcag actcatccta cgatagtaat aaagtcaacc 360

attcccatag gtttcgtaga taaggttagg gagcaattcg attaccagaa cattatattt 420

tcgccagaat ttctgagaga gggtcgcgcc ctgtatgata atctatatcc atcacggatt 480

atagtgggcg atgactctcc gattgcactt aagtttgcta atcttttagt tgagggctcc 540

aaaactccgc tcgccccagt acttacgatg ggtacacgtg aggctgaagc tgtcaagctg 600

ttttcaaaca cataccttgc tatgcgagtc gcatacttta acgaactaga tacctttgct 660

atgtcgcacg gtatgaatgc taaagaaatc atagatggcg taacgttgga gcctcggata 720

ggtcaaggat attccaatcc atcttttggc tacggtgcgt attgtttccc aaaggacacg 780

aagcaattat tagctaactt tgagggtgtt ccgcaagata taattggggc gatagtagaa 840

agcaatgaaa cacggaagga ggtaatcgtg agtgaagtgg aaaaccgatt ccccactacg 900

gtcggcgttt acaaattagc cgccaaggct ggttccgaca atttccgatc cagcgcaata 960

gtagatatta tggaaagatt agctaataag ggataccaca ttaaaatctt tgaacctact 1020

gtcgaacagt tcgagaactt cgaggttgat aataacttga cgactttcgc aacggagagc 1080

gatgtaatta ttgcaaaccg cgtacctgtg gaacatcgaa ttttgttcgg aaagaagctg 1140

attacacgcg atgtatatgg cgataactaa 1170

<210> 38
<211> 1199
<212> DNA
<213> Streptococcus

<220>
<223> a nucleic acid sequence of UDP-Glucose dehydrogenase (HASB)

<400> 38
atgaagatat cggtagcggg ttcggggtac gtggggttat ccttgtcaat cttgcttgcc 60

caacataacg atgtgactgt tgtagatata atcgacgaaa aggtacggct aattaaccag 120

ggcatatctc cgattaagga tgcggacatt gaggaatatc tgaagaatgc accgttgaat 180

cttacggcta cactagacgg agcttcggct tatagtaatg ctgatctgat tataatcgca 240

acgccaacta attacgattc agaacgcaat tatttcgaca ccagacacgt tgaagaagta 300

attgagcaag tattggattt aaatgcctcc gctactataa tcatcaagag taccataccc 360

ttgggtttta ttaaacacgt aagagagaaa taccaaacag acagaatcat cttttctcca 420

gagttcttaa gagagtcaaa ggcattgtac gataacttat acccctctcg tataatagtc 480

agttatgaga aggatgactc tccaagagtt atacaagcag ctaaggcgtt cgcgggttta 540

ttaaaagagg gggcaaagag caaggatacc ccagttctgt ttatgggctc tcaagaagct 600

gaagctgtca agctgtttgc taatactttt ctcgccatga gggtcagtta cttcaacgag 660

cttgacactt atagcgaatc aaaaggacta gacgcccaaa gagttataga aggcgtctgc 720

catgatcaaa ggataggtaa tcattacaat aatccatcct tcggatatgg cggttattgt 780

ttacccaaag actcaaagca acttttggct aattatagag gcatacctca gtctctaatg 840

tctgccatcg ttgaatcgaa caagatccgt aagtcgtatt tagctgaaca aatattagat 900

agggcttctt cacaaaagca ggctggtgta cctttaacca taggatttta ccgtttgatt 960

atgaagtcca actccgataa ctttagagaa tcagccatta aagatattat tgacatcatt 1020

aatgactacg gtgtcaatat tgtcatttat gaacctatgt tgggagaaga cattggttat 1080

agagtcgtta aagatttgga acagtttaag aacgaaagta caattattgt tagtaacagg 1140

tttgaagatg atttaggtga tgttattgat aaagtttata cacgtgacgt ctttggtag 1199

<210> 39
<211> 481
<212> PRT
<213> Arabidopsis thaliana

<220>
<223> amino acid sequence of UDP-Glucose dehydrogenase (HASB)

<400> 39
Met Val Lys Ile Cys Cys Ile Gly Ala Gly Tyr Val Gly Gly Pro Thr
1               5                   10                  15
Met Ala Val Met Ala Leu Lys Cys Pro Glu Ile Glu Val Val Val Val
            20                  25                  30
Asp Ile Ser Glu Pro Arg Ile Asn Ala Trp Asn Ser Asp Arg Leu Pro
        35                  40                  45
Ile Tyr Glu Pro Gly Leu Glu Asp Val Val Lys Gln Cys Arg Gly Lys
    50                  55                  60
Asn Leu Phe Phe Ser Thr Asp Val Glu Lys His Val Phe Glu Ser Asp
65                  70                  75                  80
Ile Val Phe Val Ser Val Asn Thr Pro Thr Lys Thr Gln Gly Leu Gly
                85                  90                  95
Ala Gly Lys Ala Ala Asp Leu Thr Tyr Trp Glu Ser Ala Ala Arg Met
            100                 105                 110
Ile Ala Asp Val Ser Lys Ser Ser Lys Ile Val Val Glu Lys Ser Thr
        115                 120                 125
Val Pro Val Arg Thr Ala Glu Ala Ile Glu Lys Ile Leu Thr His Asn
    130                 135                 140
Ser Lys Gly Ile Glu Phe Gln Ile Leu Ser Asn Pro Glu Phe Leu Ala
145                 150                 155                 160
Glu Gly Thr Ala Ile Lys Asp Leu Tyr Asn Pro Asp Arg Val Leu Ile
                165                 170                 175
Gly Gly Arg Asp Thr Ala Ala Gly Gln Lys Ala Ile Lys Ala Leu Arg
            180                 185                 190
Asp Val Tyr Ala His Trp Val Pro Val Glu Gln Ile Ile Cys Thr Asn
        195                 200                 205
Leu Trp Ser Ala Glu Leu Ser Lys Leu Ala Ala Asn Ala Phe Leu Ala
    210                 215                 220
Gln Arg Ile Ser Ser Val Asn Ala Met Ser Ala Leu Cys Glu Ala Thr
225                 230                 235                 240
Gly Ala Asp Val Thr Gln Val Ala His Ala Val Gly Thr Asp Thr Arg
                245                 250                 255
Ile Gly Pro Lys Phe Leu Asn Ala Ser Val Gly Phe Gly Gly Ser Cys
            260                 265                 270
Phe Gln Lys Asp Ile Leu Asn Leu Ile Tyr Ile Cys Glu Cys Asn Gly
        275                 280                 285
Leu Pro Glu Ala Ala Asn Tyr Trp Lys Gln Val Val Lys Val Asn Asp
    290                 295                 300
Tyr Gln Lys Ile Arg Phe Ala Asn Arg Val Val Ser Ser Met Phe Asn
305                 310                 315                 320
Thr Val Ser Gly Lys Lys Ile Ala Ile Leu Gly Phe Ala Phe Lys Lys
                325                 330                 335
Asp Thr Gly Asp Thr Arg Glu Thr Pro Ala Ile Asp Val Cys Asn Arg
            340                 345                 350

Leu Val Ala Asp Lys Ala Lys Leu Ser Ile Tyr Asp Pro Gln Val Leu
        355                 360                 365
Glu Glu Gln Ile Arg Arg Asp Leu Ser Met Ala Arg Phe Asp Trp Asp
    370                 375                 380
His Pro Val Pro Leu Gln Gln Ile Lys Ala Glu Gly Ile Ser Glu Gln
385                 390                 395                 400
Val Asn Val Val Ser Asp Ala Tyr Glu Ala Thr Lys Asp Ala His Gly
                405                 410                 415
Leu Cys Val Leu Thr Glu Trp Asp Glu Phe Lys Ser Leu Asp Phe Lys
            420                 425                 430
Lys Ile Phe Asp Asn Met Gln Lys Pro Ala Phe Val Phe Asp Gly Arg
        435                 440                 445
Asn Val Val Asp Ala Val Lys Leu Arg Glu Ile Gly Phe Ile Val Tyr
    450                 455                 460
Ser Ile Gly Lys Pro Leu Asp Ser Trp Leu Lys Asp Met Pro Ala Val
465                 470                 475                 480
Ala

<210> 40
<211> 389
<212> PRT
<213> Chlorella virus

<220>
<223> amino acid sequence of UDP-Glucose dehydrogenase (HASB)

<400> 40
Met Ser Arg Ile Ala Val Val Gly Cys Gly Tyr Val Gly Thr Ala Cys
1               5                   10                  15
Ala Val Leu Leu Ala Gln Lys Asn Glu Val Ile Val Leu Asp Ile Ser
            20                  25                  30
Glu Asp Arg Val Gln Leu Ile Lys Asn Lys Lys Ser Pro Ile Glu Asp
        35                  40                  45
Lys Glu Ile Glu Glu Phe Leu Glu Thr Lys Asp Leu Asn Leu Thr Ala
    50                  55                  60
Thr Thr Asp Lys Val Leu Ala Tyr Glu Asn Ala Glu Phe Val Ile Ile
65                  70                  75                  80
Ala Thr Pro Thr Asp Tyr Asp Val Val Thr Arg Tyr Phe Asn Thr Lys
                85                  90                  95
Ser Val Glu Asn Val Ile Gly Asp Val Ile Lys Asn Thr Gln Thr His
            100                 105                 110
Pro Thr Ile Val Ile Lys Ser Thr Ile Pro Ile Gly Phe Val Asp Lys
        115                 120                 125
Val Arg Glu Gln Phe Asp Tyr Gln Asn Ile Ile Phe Ser Pro Glu Phe
    130                 135                 140
Leu Arg Glu Gly Arg Ala Leu Tyr Asp Asn Leu Tyr Pro Ser Arg Ile
145                 150                 155                 160
Ile Val Gly Asp Asp Ser Pro Ile Ala Leu Lys Phe Ala Asn Leu Leu
                165                 170                 175
Val Glu Gly Ser Lys Thr Pro Leu Ala Pro Val Leu Thr Met Gly Thr
            180                 185                 190
Arg Glu Ala Glu Ala Val Lys Leu Phe Ser Asn Thr Tyr Leu Ala Met
        195                 200                 205
Arg Val Ala Tyr Phe Asn Glu Leu Asp Thr Phe Ala Met Ser His Gly
    210                 215                 220
Met Asn Ala Lys Glu Ile Ile Asp Gly Val Thr Leu Glu Pro Arg Ile
225                 230                 235                 240
Gly Gln Gly Tyr Ser Asn Pro Ser Phe Gly Tyr Gly Ala Tyr Cys Phe
                245                 250                 255
Pro Lys Asp Thr Lys Gln Leu Leu Ala Asn Phe Glu Gly Val Pro Gln
            260                 265                 270

Asp Ile Ile Gly Ala Ile Val Glu Ser Asn Glu Thr Arg Lys Glu Val
        275                 280                 285
Ile Val Ser Glu Val Glu Asn Arg Phe Pro Thr Thr Val Gly Val Tyr
    290                 295                 300
Lys Leu Ala Ala Lys Ala Gly Ser Asp Asn Phe Arg Ser Ser Ala Ile
305                 310                 315                 320
Val Asp Ile Met Glu Arg Leu Ala Asn Lys Gly Tyr His Ile Lys Ile
                325                 330                 335
Phe Glu Pro Thr Val Glu Gln Phe Glu Asn Phe Glu Val Asp Asn Asn
            340                 345                 350
Leu Thr Thr Phe Ala Thr Glu Ser Asp Val Ile Ile Ala Asn Arg Val
        355                 360                 365
Pro Val Glu His Arg Ile Leu Phe Gly Lys Lys Leu Ile Thr Arg Asp
    370                 375                 380
Val Tyr Gly Asp Asn
385

<210> 41
<211> 401
<212> PRT
<213> Streptococcus

<220>
<223> amino acid sequence of UDP-Glucose dehydrogenase (HASB)

<400> 41
Met Lys Ile Ser Val Ala Gly Ser Gly Tyr Val Gly Leu Ser Leu Ser
1               5                   10                  15
Ile Leu Leu Ala Gln His Asn Asp Val Thr Val Val Asp Ile Ile Asp
            20                  25                  30
Glu Lys Val Arg Leu Ile Asn Gln Gly Ile Ser Pro Ile Lys Asp Ala
        35                  40                  45
Asp Ile Glu Glu Tyr Leu Lys Asn Ala Pro Leu Asn Leu Thr Ala Thr
    50                  55                  60
Leu Asp Gly Ala Ser Ala Tyr Ser Asn Ala Asp Leu Ile Ile Ile Ala
65                  70                  75                  80
Thr Pro Thr Asn Tyr Asp Ser Glu Arg Asn Tyr Phe Asp Thr Arg His
                85                  90                  95
Val Glu Glu Val Ile Glu Gln Val Leu Asp Leu Asn Ala Ser Ala Thr
            100                 105                 110
Ile Ile Ile Lys Ser Thr Ile Pro Leu Gly Phe Ile Lys His Val Arg
        115                 120                 125
Glu Lys Tyr Gln Thr Asp Arg Ile Ile Phe Ser Pro Glu Phe Leu Arg
    130                 135                 140
Glu Ser Lys Ala Leu Tyr Asp Asn Leu Tyr Pro Ser Arg Ile Ile Val
145                 150                 155                 160
Ser Tyr Glu Lys Asp Asp Ser Pro Arg Val Ile Gln Ala Ala Lys Ala
                165                 170                 175
Phe Ala Gly Leu Leu Lys Glu Gly Ala Lys Ser Lys Asp Thr Pro Val
            180                 185                 190
Leu Phe Met Gly Ser Gln Glu Ala Glu Ala Val Lys Leu Phe Ala Asn
        195                 200                 205
Thr Phe Leu Ala Met Arg Val Ser Tyr Phe Asn Glu Leu Asp Thr Tyr
    210                 215                 220
Ser Glu Ser Lys Gly Leu Asp Ala Gln Arg Val Ile Glu Gly Val Cys
225                 230                 235                 240
His Asp Gln Arg Ile Gly Asn His Tyr Asn Asn Pro Ser Phe Gly Tyr
                245                 250                 255
Gly Gly Tyr Cys Leu Pro Lys Asp Ser Lys Gln Leu Leu Ala Asn Tyr
            260                 265                 270
Arg Gly Ile Pro Gln Ser Leu Met Ser Ala Ile Val Glu Ser Asn Lys
        275                 280                 285

Ile Arg Lys Ser Tyr Leu Ala Glu Gln Ile Leu Asp Arg Ala Ser Ser
    290                 295                 300
Gln Lys Gln Ala Gly Val Pro Leu Thr Ile Gly Phe Tyr Arg Leu Ile
305                 310                 315                 320
Met Lys Ser Asn Ser Asp Asn Phe Arg Glu Ser Ala Ile Lys Asp Ile
                325                 330                 335
Ile Asp Ile Ile Asn Asp Tyr Gly Val Asn Ile Val Ile Tyr Glu Pro
            340                 345                 350
Met Leu Gly Glu Asp Ile Gly Tyr Arg Val Val Lys Asp Leu Glu Gln
        355                 360                 365
Phe Lys Asn Glu Ser Thr Ile Ile Val Ser Asn Arg Phe Glu Asp Asp
    370                 375                 380
Leu Gly Asp Val Ile Asp Lys Val Tyr Thr Arg Asp Val Phe Gly Arg
385                 390                 395                 400
Asp

<210> 42
<211> 1026
<212> DNA
<213> Pseudomonas aeruginosa

<220>
<223> nucleic acid sequence of UDP-glucose-4-epimerase (GNE1)

<400> 42
atgatgtccc gttacgagga acttcgtaaa gagctgcctg cacagccgaa ggtatggttg      60

atcactggtg tcgcgggctt catcgggtca aatctgctag agaccttgct taaattggat     120

cagaaggttg ttggcttgga caactttgca actggccacc agaggaatct ggacgaggtg     180

aggtcattgg tttctgagaa acagtggagc aactttaagt ttattcaagg ggacattaga     240

aatctggacg actgcaacaa cgcatgtgca ggcgtcgatt atgtcttaca ccaggcagca     300

ttgggcagtg tgccgaggtc tataaacgac cctataacat cgaatgcaac taacatagac     360

ggtttcttga acatgctgat tgcagcgagg gatgcaaaag ttcagtcttt tacctatgcc     420

gcttcatcct ctacatacgg ggatcacccc ggtttgccaa aggtggaaga cactatcggc     480

aagcctctat ctccatacgc tgtgacgaag tatgttaatg agttgtacgc agacgtgttt     540

tcacgttgtt atggattttc aaccatcggt ctgagatact ttaacgtctt cggccgtaga     600

caagatccga atggggcata cgccgccgta atccctaagt ggacatcctc aatgatacag     660

ggtgacgacg tctacattaa cggagatgga gagacatcac gtgacttctg ctacatagaa     720

aacacagttc aagcaaactt attagccgca acggccggcc tagacgccag aaaccaggtg     780

tataatatcg cggtgggtgg tagaacgtca ctaaatcagc ttttcttcgc cttgagagac     840

ggtctagctg agaacggcgt tagctaccat agggaaccag tttataggga ttttagagaa     900

ggagatgtga gacacagctt ggcagatatt tccaaggcgg ctaaacttct tgggtatgct     960

cctaaatatg acgtatcagc aggggtggcg cttgcaatgc catggtatat aatgtttctt    1020

aagtaa                                                               1026

<210> 43
<211> 1020
<212> DNA
<213> Pasteurella multocida

<220>
<223> nucleic acid sequence of UDP-glucose-4-epimerase (KFOA)

<400> 43
atgaacattc tagtgaccgg cggtgctggc tatatcggct cccataccag tttatgctta      60

ctgaataaag gatataacgt ggtcatcatt gacaacctga tcaatagttc atgcgagtcg     120

atccgtagga tagaactgat agctaagaag aaggtcacct tctacgagct gaacattaat     180

aatgaaaagg aagtgaacca gatccttaag aaacacaaat tcgattgcat aatgcacttc     240

gcaggtgcca agtcggtggc cgagtcacta attaagccaa ttttctacta tgacaacaac     300

gtctctggaa ctttgcagct gattaattgt gctataaaga acgacgtcgc aaatttcatc     360

ttttcttcat cagcaactgt ttatggtgag tcaaagatta tgccagtgac agaggactgc     420

cacatcggcg gcacactaaa tccatacgga acatcaaaat acatttcaga gttgatgata     480

agggacatcg ccaagaagta ctcagacact aatttcttat gcttgcgtta tttcaatcct     540

accggggccc acgagtccgg catgatcggc gagtctccag ctgatatacc atccaatctg     600

gtgccttaca ttttacaggt ggccatgggc aagttggaga agttaatggt attcggcggt     660

gactacccaa caaaggacgg cacgggtgtg agagactata ttcacgtgat ggacctagcg     720

gaaggtcatg tggcagccct aagctacctt ttcagagata ataacactaa ttaccatgta     780

ttcaatctgg gcacgggtaa gggctacagt gtccttgaat tagtaagcac attcgagaaa     840

atctcgggtg tcaggattcc atacgagatc gtttctcgtc gtgatgggga catcgcagag     900

agttggtcat caccggagaa agcgaacaag tacttaaatt ggaaggccaa gagggagctt     960

gagacaatgc tagaagacgc atggcgttgg cagatgaaga acccgaatgg ttacatataa    1020

<210> 44
<211> 1021
<212> DNA
<213> Escherichia coli

<220>
<223> nucleic acid sequence of UDP-glucose-4-epimerase (KFOA)

<400> 44
aatgaacatt ctagtgaccg gcggtgctgg ctatatcggc tcccatacca gtttatgctt      60

actgaataaa ggatataacg tggtcatcat tgacaacctg atcaatagtt catgcgagtc     120

gatccgtagg atagaactga tagctaagaa gaaggtcacc ttctacgagc tgaacattaa     180

taatgaaaag gaagtgaacc agatccttaa gaaacacaaa ttcgattgca taatgcactt     240

cgcaggtgcc aagtcggtgg ccgagtcact aattaagcca attttctact atgacaacaa 300

cgtctctgga actttgcagc tgattaattg tgctataaag aacgacgtcg caaatttcat 360

cttttcttca tcagcaactg tttatggtga gtcaaagatt atgccagtga cagaggactg 420

ccacatcggc ggcacactaa atccatacgg aacatcaaaa tacatttcag agttgatgat 480

aagggacatc gccaagaagt actcagacac taatttctta tgcttgcgtt atttcaatcc 540

taccggggcc cacgagtccg gcatgatcgg cgagtctcca gctgatatac catccaatct 600

ggtgccttac attttacagg tggccatggg caagttggag aagttaatgg tattcggcgg 660

tgactaccca acaaaggacg gcacgggtgt gagagactat attcacgtga tggacctagc 720

ggaaggtcat gtggcagccc taagctacct tttcagagat aataacacta attaccatgt 780

attcaatctg ggcacgggta agggctacag tgtccttgaa ttagtaagca cattcgagaa 840

aatctcgggt gtcaggattc catacgagat cgtttctcgt cgtgatgggg acatcgcaga 900

gagttggtca tcaccggaga aagcgaacaa gtacttaaat tggaaggcca agagggagct 960

tgagacaatg ctagaagacg catggcgttg gcagatgaag aacccgaatg gttacatata 1020

a 1021

<210> 45
<211> 341
<212> PRT
<213> Pseudomonas aeruginosa

<220>
<223> amino acid sequence of UDP-glucose-4-epimerase (GNE1)

<400> 45
Met Met Ser Arg Tyr Glu Glu Leu Arg Lys Glu Leu Pro Ala Gln Pro
1               5                   10                  15
Lys Val Trp Leu Ile Thr Gly Val Ala Gly Phe Ile Gly Ser Asn Leu
            20                  25                  30
Leu Glu Thr Leu Leu Lys Leu Asp Gln Lys Val Val Gly Leu Asp Asn
        35                  40                  45
Phe Ala Thr Gly His Gln Arg Asn Leu Asp Glu Val Arg Ser Leu Val
    50                  55                  60
Ser Glu Lys Gln Trp Ser Asn Phe Lys Phe Ile Gln Gly Asp Ile Arg
65                  70                  75                  80
Asn Leu Asp Asp Cys Asn Asn Ala Cys Ala Gly Val Asp Tyr Val Leu
                85                  90                  95
His Gln Ala Ala Leu Gly Ser Val Pro Arg Ser Ile Asn Asp Pro Ile
            100                 105                 110
Thr Ser Asn Ala Thr Asn Ile Asp Gly Phe Leu Asn Met Leu Ile Ala
        115                 120                 125
Ala Arg Asp Ala Lys Val Gln Ser Phe Thr Tyr Ala Ala Ser Ser Ser
    130                 135                 140
Thr Tyr Gly Asp His Pro Gly Leu Pro Lys Val Glu Asp Thr Ile Gly
145                 150                 155                 160
Lys Pro Leu Ser Pro Tyr Ala Val Thr Lys Tyr Val Asn Glu Leu Tyr
                165                 170                 175
Ala Asp Val Phe Ser Arg Cys Tyr Gly Phe Ser Thr Ile Gly Leu Arg

180                 185                 190
Tyr Phe Asn Val Phe Gly Arg Arg Gln Asp Pro Asn Gly Ala Tyr Ala
        195                 200                 205
Ala Val Ile Pro Lys Trp Thr Ser Ser Met Ile Gln Gly Asp Asp Val
    210                 215                 220
Tyr Ile Asn Gly Asp Gly Glu Thr Ser Arg Asp Phe Cys Tyr Ile Glu
225                 230                 235                 240
Asn Thr Val Gln Ala Asn Leu Leu Ala Ala Thr Ala Gly Leu Asp Ala
                245                 250                 255
Arg Asn Gln Val Tyr Asn Ile Ala Val Gly Gly Arg Thr Ser Leu Asn
            260                 265                 270
Gln Leu Phe Phe Ala Leu Arg Asp Gly Leu Ala Glu Asn Gly Val Ser
        275                 280                 285
Tyr His Arg Glu Pro Val Tyr Arg Asp Phe Arg Glu Gly Asp Val Arg
    290                 295                 300
His Ser Leu Ala Asp Ile Ser Lys Ala Ala Lys Leu Leu Gly Tyr Ala
305                 310                 315                 320
Pro Lys Tyr Asp Val Ser Ala Gly Val Ala Leu Ala Met Pro Trp Tyr
                325                 330                 335
Ile Met Phe Leu Lys
            340

<210> 46
<211> 338
<212> PRT
<213> Pasteurella multocida

<220>
<223> amino acid sequence of UDP-glucose-4-epimerase (KFOA)

<400> 46
Met Ala Ile Leu Val Thr Gly Gly Ala Gly Tyr Ile Gly Ser His Thr
1               5                   10                  15
Val Val Glu Leu Leu Asn Ala Asn Lys Asp Val Val Val Leu Asp Asn
            20                  25                  30
Leu Cys Asn Ser Ser Pro Lys Ser Leu Glu Arg Val Ala Gln Ile Thr
        35                  40                  45
Gly Lys Gln Val Lys Phe Tyr Gln Gly Asp Ile Leu Asp Thr Ala Leu
    50                  55                  60
Leu Gln Lys Ile Phe Ala Glu Asn Gln Ile Gln Ser Val Ile His Phe
65                  70                  75                  80
Ala Gly Leu Lys Ala Val Gly Glu Ser Val Gln Lys Pro Ala Glu Tyr
                85                  90                  95
Tyr Met Asn Asn Val Thr Gly Ser Leu Val Leu Ile Gln Glu Met Lys
            100                 105                 110
Lys Ala Gly Val Trp Asn Phe Val Phe Ser Ser Ser Ala Thr Val Tyr
        115                 120                 125
Gly Asp Pro Glu Ile Ile Pro Ile Thr Glu Ser Cys Lys Val Gly Gly
    130                 135                 140
Thr Thr Asn Pro Tyr Gly Thr Ser Lys Phe Met Val Glu Gln Ile Leu
145                 150                 155                 160
Lys Asp Ile Ala Lys Ala Thr Pro Glu Phe Ser Ile Thr Ile Leu Arg
                165                 170                 175
Tyr Phe Asn Pro Val Gly Ala His Glu Ser Gly Leu Ile Gly Glu Asp
            180                 185                 190
Pro Asn Gly Ile Pro Asn Asn Leu Leu Pro Tyr Ile Ser Gln Val Ala
        195                 200                 205
Ile Gly Lys Leu Pro Gln Leu Ser Val Phe Gly Ser Asp Tyr Glu Thr
    210                 215                 220
His Asp Gly Thr Gly Val Arg Asp Tyr Ile His Val Val Asp Leu Ala
225                 230                 235                 240
Ile Gly His Leu Lys Ala Leu Asp Arg His Glu Gly Asp Ala Gly Leu

245                 250                 255
His Ile Tyr Asn Leu Gly Thr Gly Ser Gly Tyr Ser Val Leu Asp Met
            260                 265                 270
Val Lys Ala Phe Glu Lys Val Asn Asp Ile Lys Ile Pro Tyr Lys Leu
        275                 280                 285
Val Asp Arg Arg Pro Gly Asp Ile Ala Thr Cys Tyr Ser Asp Pro Ser
    290                 295                 300
Leu Ala Lys Thr Glu Leu Asn Trp Thr Ala Ala Arg Gly Leu Glu Gln
305                 310                 315                 320
Met Met Lys Asp Thr Trp His Trp Gln Lys Lys Asn Pro Lys Gly Tyr
                325                 330                 335
Arg Asp

<210> 47
<211> 339
<212> PRT
<213> Escherichia coli

<220>
<223> amino acid sequence of UDP-glucose-4-epimerase (KFOA)

<400> 47
Met Asn Ile Leu Val Thr Gly Gly Ala Gly Tyr Ile Gly Ser His Thr
1               5                   10                  15
Ser Leu Cys Leu Leu Asn Lys Gly Tyr Asn Val Val Ile Ile Asp Asn
            20                  25                  30
Leu Ile Asn Ser Ser Cys Glu Ser Ile Arg Arg Ile Glu Leu Ile Ala
        35                  40                  45
Lys Lys Lys Val Thr Phe Tyr Glu Leu Asn Ile Asn Asn Glu Lys Glu
    50                  55                  60
Val Asn Gln Ile Leu Lys Lys His Lys Phe Asp Cys Ile Met His Phe
65                  70                  75                  80
Ala Gly Ala Lys Ser Val Ala Glu Ser Leu Ile Lys Pro Ile Phe Tyr
                85                  90                  95
Tyr Asp Asn Asn Val Ser Gly Thr Leu Gln Leu Ile Asn Cys Ala Ile
            100                 105                 110
Lys Asn Asp Val Ala Asn Phe Ile Phe Ser Ser Ser Ala Thr Val Tyr
        115                 120                 125
Gly Glu Ser Lys Ile Met Pro Val Thr Glu Asp Cys His Ile Gly Gly
    130                 135                 140
Thr Leu Asn Pro Tyr Gly Thr Ser Lys Tyr Ile Ser Glu Leu Met Ile
145                 150                 155                 160
Arg Asp Ile Ala Lys Lys Tyr Ser Asp Thr Asn Phe Leu Cys Leu Arg
                165                 170                 175
Tyr Phe Asn Pro Thr Gly Ala His Glu Ser Gly Met Ile Gly Glu Ser
            180                 185                 190
Pro Ala Asp Ile Pro Ser Asn Leu Val Pro Tyr Ile Leu Gln Val Ala
        195                 200                 205
Met Gly Lys Leu Glu Lys Leu Met Val Phe Gly Gly Asp Tyr Pro Thr
    210                 215                 220
Lys Asp Gly Thr Gly Val Arg Asp Tyr Ile His Val Met Asp Leu Ala
225                 230                 235                 240
Glu Gly His Val Ala Ala Leu Ser Tyr Leu Phe Arg Asp Asn Asn Thr
                245                 250                 255
Asn Tyr His Val Phe Asn Leu Gly Thr Gly Lys Gly Tyr Ser Val Leu
            260                 265                 270
Glu Leu Val Ser Thr Phe Glu Lys Ile Ser Gly Val Arg Ile Pro Tyr
        275                 280                 285
Glu Ile Val Ser Arg Arg Asp Gly Asp Ile Ala Glu Ser Trp Ser Ser
    290                 295                 300
Pro Glu Lys Ala Asn Lys Tyr Leu Asn Trp Lys Ala Lys Arg Glu Leu

305                 310                 315                 320
Glu Thr Met Leu Glu Asp Ala Trp Arg Trp Gln Met Lys Asn Pro Asn
                325                 330                 335
Gly Tyr Ile

<210> 48
<211> 1197
<212> DNA
<213> Cupiennius salei

<220>
<223> reencoded nucleic acid sequence of hyaluronidase (HYAL)
      originating from Cupiennius salei with a N-terminal secretion
      signal

<400> 48
atgcaattca gcactgttgc atcagttgca tttgtcgccc tggcgaattt tgtagccgcg      60

ttcaagattt actggaacgt cccaactttt cagtgcacgc ataactacaa aatcgattat     120

gtcaaattgt tgtccactta cgggatacag gtcaatgatg gcggtaagtt tcaaggaaac     180

caagtgacta tcttttatga aacccagttg ggtttgtatc cacgaatcct aaaatctggt     240

aaaatggaaa acggcggaat ccctcaacgc ggtaactttg agaaacacct agaaaaggca     300

agcacggacc tccagaaagt gatcccttgg aaagagttta gcggattagg tgtgatagat     360

tgggaggctt ggagacccac atgggaattt aactgggaac cgttgaggat atatcaaacc     420

gaatcaatta agagagctaa agaactacac cctaccgcaa acgattccgc agtaaaagaa     480

attgcagagc ggcaatggga agattcagcc aagttataca tgttagaaac actgcggctg     540

gcaaagaaac ttcgacctca agcgccttgg tgttactact tatttcctga ttgctataat     600

tacgtcggaa agaaaccaaa agatttccaa tgtagtgcct cgatacgtaa aggtaacgat     660

aagctaagct ggttgtggaa agattctacg gcattgtgtc catcgatata cgtatatgaa     720

tcacaattag acaggtattc ttttgaacaa aggacatggc gcgacaatga gaaacttcgg     780

gaagcgttgc gtgtagccac gagaacctct aaaatatacc catacgttaa ctatttcgat     840

aaggagctta taccggagca agaagtatgg agaatgcttg cgcaggcagc tgctgtcggt     900

ggcagtggtg cggtaatttg gggctcatct gctgcagttg catctgaaga gttatgtaaa     960

tctttaaaac agtatattat tgaaacgctt gggccggcgg cagagaaggt ggcttggcgt    1020

agtgacttat gcagcaaaga aatttgtaat aatcagggtc gctgcacatt cccggacgat    1080

gattatgcaa acgcatggaa attatttaca gatgatactg ttaagtttta tgctggtaat    1140

attacatgta ggtgctccga gaattattct ggtcgtttct gcgaaaagaa gaattaa       1197

<210> 49
<211> 1218
<212> DNA
<213> Tityus serrulatus

<220>
<223> reencoded nucleic acid sequence of hyaluronidase (HYAL)
      originating from Tityus serrulatus with a N-terminal secretion
      signal

<400> 49
atgcaattct ctactgtcgc ttccgttgct ttcgtcgctt tggctaactt tgttgccgct      60

gctgatttta aagtttactg ggaagtgcct tccttccttt gttctaaacg ttttaaaatt     120

aatgtaacgg aagttttaac aagtcacgag attcttgtca atcagggtga gagtttcaac     180

ggtgacaaga tagtaatctt ttacgaaaac caattgggga agtacccgca tattgactca     240

aacaatgtgg agatcaatgg aggaatactt caagtagccg atttggcgaa gcatttgaaa     300

gtagccaagg ataatatcac taaattcgtc ccgaatccta atttcaacgg tgtcggagtg     360

atcgactggg aagcttggcg gccatcatgg gaatttaact ggggtaagtt aaaagtatat     420

aaagaaaaga gcattgactt ggtcaagtcg aaacatccgg agtggccctc cgacagggtt     480

gaaaaggttg ctaaagagga gtgggaggag agtgccaaag aatggatggt gaagaccctg     540

aagttagcac aggaaatgcg accgaacgca gtttggtgct attatctatt ccctgactgc     600

tacaattatt tcggtaagga tcaaccctct caattcagct gctcgtctcg aattcagaag     660

gaaaattctc gtctttcttg gctctggaat caatcaacag ccatttgcct aagcatttat     720

atccaggaat cccatgttac caaatataat atgtcccagc ggacatggtg gatcgatgcg     780

agattaagag aagcaattcg agtcagcgaa cacagaccaa acatacccat ctacccttac     840

attaattata ttctacctgg aactaatcaa actgtaccag caatggactt taaaaggaca     900

ctgggtcaaa tagctagcct cggcctagat ggtgctttgt tatggggatc tagctatcat     960

gttttaacag aatctcaatg caaaatcact tctgattatg tgaaatcagt gattgctcct    1020

accgtggcta ctgtcgttct caatacaaac agatgctcac agataatttg taagggtcgc    1080

ggcaactgtg tttggcctga agaaccattt agttcttgga aatacttagt tgaccccaaa    1140

atgccagtgt tcaagccaac caacatccac tgtaaatgta aaggttacct aggtagatac    1200

tgtgagatcc caaagtaa                                                  1218

<210> 50
<211> 1308
<212> DNA
<213> Bos taurus

<220>
<223> reencoded nucleic acid sequence of hyaluronidase (HYAL)
      originating from Bos Taurus with a N-terminal secretion signal

<400> 50
atgcaatttt ctactgttgc ttctgtcgct ttcgtagcac tggccaattt tgtagctgct      60

ggaagccgtg accccgttgt cccaaaccag cctttcacaa caatatggaa cgcaaatacg 120

gagtggtgca tgaagaagca tggagtggac gtcgatattt caatcttcga cgtggtcact 180

aatccaggac agacatttag aggtccaaat atgacaatat tttacagttc acaattgggg 240

acctaccctt actacacatc cgcgggtgag ccagtattcg gtggtctacc ccagaacgca 300

agtttgaatg cccacttggc taggacattt caagatattc tggcagccat gccagagccc 360

aggttctcag gtttggctgt catagactgg gaagcgtgga gacctcgttg ggcatttaat 420

tgggatacga aagacatata caggcagagg tccagagcat tggttcagaa acagcacccg 480

gattggctgg cgccacgtgt tgaggccgca gcgcaagacc agttcgaggg cgctgctgag 540

gagtggatgg ccggcacact taaattaggg caggcgcttc gtcctcaagg cttgtggggt 600

ttctataact tcccggagtg ttacaattat gacttcaaat ctccgaacta cacaggaagg 660

tgccctttaa acatctgtgc gcagaacgac cagttaggtt ggctatgggg tcaaagtcgt 720

gcactatatc catcaatcta cttgccggcg gctttggaag gcacgaagaa gactcagatg 780

ttcgttcagc accgtgtagc agaggcattt agggtggcgg caggcgctgg tgaccccaag 840

ctacccgtct taccctatat gcagctattt tacgatatga caaaccactt cttgcccgcg 900

gaagaactgg aacatagctt aggtgagtca gcagcgcagg gtgcagccgg ggttgttctg 960

tgggtttcct ggttatcaac ttcgacaaag gaaagctgtc aagcaatcaa ggaatacgta 1020

gatactacgt tgggcccttc aatcttgaac gtcacatctg gtgccaggtt gtgttctcag 1080

gtattgtgca gtggccacgg gagatgcgcg agaagacctt catatcctaa agccaggctg 1140

attctaaatt caacgtcgtt ctccatcaag ccaacacccg gtggtggtcc attgacactt 1200

caaggcgctt tgagtttgga agacagatta cgtatggcag ttgagtttga atgcagatgt 1260

tacagaggat ggagagggac acgttgtgag cagtggggca tgtggtaa 1308

<210> 51
<211> 1137
<212> DNA
<213> Apis mellifera

<220>
<223> reencoded nucleic acid sequence of hyaluronidase (HYAL)
      originating from Apis mellifera with a N-terminal secretion
      signal

<400> 51
atgcagtttt ccacagttgc ttccgtagct ttcgtagctt tagcaaattt tgtcgcagct 60

ttgttgttgg gcttcgttca gtctactcca gataataata agactgtgcg tgaatttaac 120

gtgtattgga acgttcctac attcatgtgc cataagtacg ggttgagatt cgaagaggtg 180

agcgagaagt atggcatttt gcaaaattgg atggacaaat tcagaggaga ggaaattgcg 240

atcttatacg accccggcat gttccccgca ctgttgaaag accccaatgg gaatgtagta 300

gctaggaatg gcggagtgcc ccagcttggc aatttaacta aacatttaca ggtgttcagg 360

gaccacctaa tcaatcagat tccagataaa agctttcccg gagttggtgt aatagatttc 420

gagtcatgga gacctatttt cagacagaac tgggcctctt tgcaaccgta caagaagcta 480

tcagtcgaag tcgtacgtcg tgagcaccca ttctgggatg atcagagagt ggagcaagag 540

gcgaaaagaa gatttgaaaa gtacggccag cttttcatgg aagagacctt gaaagccgca 600

aaacgtatgc gtcctgcagc caattggggc tactacgctt acccttattg ctacaacctg 660

accccaaacc agccctcagc tcaatgcgag gctacaacaa tgcaggagaa cgacaagatg 720

tcatggttat tcgagtccga ggacgtctta ctgcccagcg tctatttgag gtggaacctg 780

actagcggag agagggttgg tttagttggt ggtcgtgtca aggaagccct gagaatagct 840

cgtcagatga ccacctctag gaagaaggtc ctaccatact actggtacaa ataccaggat 900

aggagagaca cggacctaag tagggctgat cttgaggcga ctctacgtaa aatcacggac 960

ttaggagccg acggtttcat aatctgggga tcttccgatg acatcaacac caaggcaaaa 1020

tgtttgcagt tcagagaata tttgaataac gagttgggcc cggcagtgaa gagaatagcc 1080

ttaaacaata atgcgaatga caggctaaca gtggatgtgt ctgttgacca ggtttaa 1137

<210> 52
<211> 1305
<212> DNA
<213> Macaca mulatta

<220>
<223> reencoded nucleic acid sequence of hyaluronidase (HYAL)
      originating from Macaca mulatta with a N-terminal secretion
      signal

<400> 52
atgcaattca gtactgtcgc tagcgtcgcc tttgtggctc ttgcaaattt cgtcgctgct 60

tcacgtgggc ctttattgcc gaacagacca tttgcaacgg tatggaatgc taacacccaa 120

tggtgtttag agaggcactc agtagacgtc gatgtctcca tatttgatgt tgtagcaaac 180

cccggccaga cttttcgtgg gccggacatg actattttct attccagtca gctaggtact 240

tacccttatt ataccccgac gggtgaacct gtctttggtg gccttccaca gaacgcatca 300

cttatagcgc acttggctag gactttccag gatatattgg cagctatacc agcccccgac 360

ttttctgggc tagccgtaat tgactgggaa gcatggagac cgagatgggc atttaactgg 420

gacacgaaag acatttacag gcaaaggtcc cgtgccttag tccaagcaca gcatcccgac 480

tggccagtta ctcaagttga ggctgtggca caagaccagt tccagggtgc tgcgagggca 540

tggatggcag gaaccttgca attaggccgt gctttaagac caaggggact atggggcttc 600

tacggttttc ccgattgcta caattacgat ttcctatctc ctaattacac agggcaatgc 660

ccatcaggga tcagagcaca aaatgaccag ttaggatggt tgtggggtca gagtcgtgca 720

ttatatccca gtatctacat gccggctgtt ttggaaggaa caggcaaatc ccaaatgtac 780

gtgcagcaca gggtcgcgga agcctttagg gttgctgttg cagcaggcga cccaaactta 840

cccgtcctac cgtacgtcca gattttctac gatatgacca accactttct tccattggac 900

gagttagagc atagtcttgg agaatcagcg gctcagggcg cagcaggagt cgtgttatgg 960

gtgtcatggg agaatactag aacaaaagaa tcttgccagg caattaaaga atatatggac 1020

actaccttgg ggccttttat tctgaacgtc acttcgggtg cgttgttatg ctcgcaagcc 1080

ttatgttcaa gccatggtcg ttgtgtccgt cgtccctcac atccaaaagc cttgctaatc 1140

cttaatcccg caagctttag tattcagttg acaccagatg gtggtccctt gtcgttaagg 1200

ggagcattgt cacttgagga ccaggcacaa atggctgttg aattcaaatg caggtgctac 1260

cccgggtggc aaggcccttg gtgtgagcag aagagtatgt ggtaa 1305

<210> 53
<211> 1083
<212> DNA
<213> Vespa magnifica

<220>
<223> reencoded nucleic acid sequence of hyaluronidase (HYAL)
      originating from Vespa magnifica with a N-terminal secretion
      signal

<400> 53
atgcaatttt ctacagtggc aagtgttgca ttcgttgcac tagccaactt tgtggcggca 60

gatagctgtg ggtcaaactg cgaaaagagt gagagaccga aaagggtctt caacatttac 120

tggaacgtac ctacattcat gtgtcaccag tacggactat actttgacga ggtcacgaat 180

tttaatataa agcacaacag caaagacaat tttcaagggg acaagatcgc gatcttttat 240

gaccccgggg agtttcccgc tctgctgcca ctaaactatg gtaagtacaa gatcaggaat 300

ggtggtgttc cacaagaggg taacatcacc atccatctgc agagattcat agagtaccta 360

gataagacct atccgaaccg taacttttca ggcatcggtg tgatcgattt cgagaggtgg 420

agaccaattt tcagacagaa ttggggtaat atgaagattt acaagaactt ctccatcgat 480

cttgtgcgta aagagcatcc tttctggaat aagaaaatga tcgagttgga agcttctaaa 540

agattcgaga aatacgcccg tctgttcatg gaagaaacat taaagttggc taagaaaact 600

agaaaacagg ccgattgggg ctactacggt tacccctatt gcttcaacat gtctcctact 660

aatttcgttc ctgactgcga tgtcacagct agggatgaga acaacgagat gtcttggttg 720

tttaacaacc agaatgtcct attaccaagt gtatacatta ggagagagct aactcctgac 780

cagaggattg ggcttgtaca ggggagagtg aaggaagctg tgagaatttc aaataaactg 840

aagcactcac ctaaagtctt cagctattgg tggtatgttt accaagacga gaccaacacc 900

ttcttaacgg agaccgacgt caagaagacg tttcaggaga ttgtgatcaa cggtggagat 960

gggattataa tctggggttc gtcctctgat gtaaacagct tgtccaagtg tacgaggtta 1020

agggagtacc tattgacagt cttgggacca attgctgtta acgtgactga agcagtaaac 1080

taa 1083

<210> 54
<211> 1407
<212> DNA
<213> Cupiennius salei

<220>
<223> reencoded nucleic acid sequence of hyaluronidase (HYAL)
      originating from Cupiennius salei with a N-terminal secretion
      signal and a C-terminal anchoring signal

<400> 54
atgcaattca gcactgttgc atcagttgca tttgtcgccc tggcgaattt tgtagccgcg 60

ttcaagattt actggaacgt cccaactttt cagtgcacgc ataactacaa aatcgattat 120

gtcaaattgt tgtccactta cgggatacag gtcaatgatg gcggtaagtt tcaaggaaac 180

caagtgacta tcttttatga aacccagttg ggtttgtatc cacgaatcct aaaatctggt 240

aaaatggaaa acggcggaat ccctcaacgc ggtaactttg agaaacacct agaaaaggca 300

agcacggacc tccagaaagt gatcccttgg aaagagttta gcggattagg tgtgatagat 360

tgggaggctt ggagacccac atgggaattt aactgggaac cgttgaggat atatcaaacc 420

gaatcaatta agagagctaa agaactacac cctaccgcaa acgattccgc agtaaaagaa 480

attgcagagc ggcaatggga agattcagcc aagttataca tgttagaaac actgcggctg 540

gcaaagaaac ttcgacctca agcgccttgg tgttactact tatttcctga ttgctataat 600

tacgtcggaa agaaaccaaa agatttccaa tgtagtgcct cgatacgtaa aggtaacgat 660

aagctaagct ggttgtggaa agattctacg gcattgtgtc catcgatata cgtatatgaa 720

tcacaattag acaggtattc ttttgaacaa aggacatggc gcgacaatga gaaacttcgg 780

gaagcgttgc gtgtagccac gagaacctct aaaatatacc catacgttaa ctatttcgat 840

aaggagctta taccggagca agaagtatgg agaatgcttg cgcaggcagc tgctgtcggt 900

ggcagtggtg cggtaatttg gggctcatct gctgcagttg catctgaaga gttatgtaaa 960

tctttaaaac agtatattat tgaaacgctt gggccggcgg cagagaaggt ggcttggcgt 1020

agtgacttat gcagcaaaga aatttgtaat aatcagggtc gctgcacatt cccggacgat 1080

gattatgcaa acgcatggaa attatttaca gatgatactg ttaagtttta tgctggtaat 1140

attacatgta ggtgctccga gaattattct ggtcgtttct gcgaaaagaa gaatggatcc 1200

gccatttctc aaatcactga cggtcaaatc caagctacta ccactgctac caccgaagct 1260

accaccactg ctgccccatc ttccaccgtt gaaactgttt ctccatccag caccgaaact 1320

atctctcaac aaactgaaaa tggtgctgct aaggccgctg tcggtatggg tgccggtgct 1380

ctagctgctg ctgctatgtt gttataa 1407

<210> 55
<211> 1428
<212> DNA
<213> Tityus serrulatus

<220>
<223> reencoded nucleic acid sequence of hyaluronidase (HYAL)
      originating from Tityus serrulatus with a N-terminal secretion
      signal and a C-terminal anchoring signal

<400> 55
atgcaattct ctactgtcgc ttccgttgct ttcgtcgctt tggctaactt tgttgccgct 60

gctgatttta aagtttactg ggaagtgcct tccttccttt gttctaaacg ttttaaaatt 120

aatgtaacgg aagttttaac aagtcacgag attcttgtca atcagggtga gagtttcaac 180

ggtgacaaga tagtaatctt ttacgaaaac caattgggga agtacccgca tattgactca 240

aacaatgtgg agatcaatgg aggaatactt caagtagccg atttggcgaa gcatttgaaa 300

gtagccaagg ataatatcac taaattcgtc ccgaatccta atttcaacgg tgtcggagtg 360

atcgactggg aagcttggcg gccatcatgg gaatttaact ggggtaagtt aaaagtatat 420

aaagaaaaga gcattgactt ggtcaagtcg aaacatccgg agtggccctc cgacagggtt 480

gaaaaggttg ctaaagagga gtgggaggag agtgccaaag aatggatggt gaagaccctg 540

aagttagcac aggaaatgcg accgaacgca gtttggtgct attatctatt ccctgactgc 600

tacaattatt tcggtaagga tcaaccctct caattcagct gctcgtctcg aattcagaag 660

gaaaattctc gtctttcttg gctctggaat caatcaacag ccatttgcct aagcatttat 720

atccaggaat cccatgttac caaatataat atgtcccagc ggacatggtg gatcgatgcg 780

agattaagag aagcaattcg agtcagcgaa cacagaccaa acatacccat ctacccttac 840

attaattata ttctacctgg aactaatcaa actgtaccag caatggactt taaaaggaca 900

ctgggtcaaa tagctagcct cggcctagat ggtgctttgt tatggggatc tagctatcat 960

gttttaacag aatctcaatg caaaatcact tctgattatg tgaaatcagt gattgctcct 1020

accgtggcta ctgtcgttct caatacaaac agatgctcac agataatttg taagggtcgc 1080

ggcaactgtg tttggcctga agaaccattt agttcttgga aatacttagt tgaccccaaa 1140

atgccagtgt tcaagccaac caacatccac tgtaaatgta aaggttacct aggtagatac 1200

tgtgagatcc caaagggatc cgccatttct caaatcactg acggtcaaat ccaagctact 1260

accactgcta ccaccgaagc taccaccact gctgccccat cttccaccgt tgaaactgtt 1320

tctccatcca gcaccgaaac tatctctcaa caaactgaaa atggtgctgc taaggccgct 1380

gtcggtatgg gtgccggtgc tctagctgct gctgctatgt tgttataa 1428

<210> 56
<211> 1518
<212> DNA
<213> Bos taurus

<220>
<223> reencoded nucleic acid sequence of hyaluronidase (HYAL)
      originating from Bos taurus with a N-terminal secretion signal
      and a C-terminal anchoring signal

<400> 56
atgcaatttt ctactgttgc ttctgtcgct ttcgtagcac tggccaattt tgtagctgct 60

ggaagccgtg accccgttgt cccaaaccag cctttcacaa caatatggaa cgcaaatacg 120

gagtggtgca tgaagaagca tggagtggac gtcgatattt caatcttcga cgtggtcact 180

aatccaggac agacatttag aggtccaaat atgacaatat tttacagttc acaattgggg 240

acctaccctt actacacatc cgcgggtgag ccagtattcg gtggtctacc ccagaacgca 300

agtttgaatg cccacttggc taggacattt caagatattc tggcagccat gccagagccc 360

aggttctcag gtttggctgt catagactgg gaagcgtgga gacctcgttg ggcatttaat 420

tgggatacga aagacatata caggcagagg tccagagcat tggttcagaa acagcacccg 480

gattggctgg cgccacgtgt tgaggccgca gcgcaagacc agttcgaggg cgctgctgag 540

gagtggatgg ccggcacact taaattaggg caggcgcttc gtcctcaagg cttgtggggt 600

ttctataact tcccggagtg ttacaattat gacttcaaat ctccgaacta cacaggaagg 660

tgccctttaa acatctgtgc gcagaacgac cagttaggtt ggctatgggg tcaaagtcgt 720

gcactatatc catcaatcta cttgccggcg gctttggaag gcacgaagaa gactcagatg 780

ttcgttcagc accgtgtagc agaggcattt agggtggcgg caggcgctgg tgaccccaag 840

ctacccgtct taccctatat gcagctattt tacgatatga caaaccactt cttgcccgcg 900

gaagaactgg aacatagctt aggtgagtca gcagcgcagg gtgcagccgg ggttgttctg 960

tgggtttcct ggttatcaac ttcgacaaag gaaagctgtc aagcaatcaa ggaatacgta 1020

gatactacgt tgggcccttc aatcttgaac gtcacatctg gtgccaggtt gtgttctcag 1080

gtattgtgca gtggccacgg gagatgcgcg agaagacctt catatcctaa agccaggctg 1140

attctaaatt caacgtcgtt ctccatcaag ccaacacccg gtggtggtcc attgacactt 1200

caaggcgctt tgagtttgga agacagatta cgtatggcag ttgagtttga atgcagatgt 1260

tacagaggat ggagagggac acgttgtgag cagtggggca tgtggggatc cgccatttct 1320

caaatcactg acggtcaaat ccaagctact accactgcta ccaccgaagc taccaccact 1380

gctgccccat cttccaccgt tgaaactgtt tctccatcca gcaccgaaac tatctctcaa 1440

caaactgaaa atggtgctgc taaggccgct gtcggtatgg gtgccggtgc tctagctgct 1500

gctgctatgt tgttataa 1518

<210> 57
<211> 1347
<212> DNA
<213> Apis mellifera

<220>
<223> reencoded nucleic acid sequence of hyaluronidase (HYAL)
      originating from Apis mellifera with a N-terminal secretion
      signal and a C-terminal anchoring signal

<400> 57
atgcagtttt ccacagttgc ttccgtagct ttcgtagctt tagcaaattt tgtcgcagct 60

ttgttgttgg gcttcgttca gtctactcca gataataata agactgtgcg tgaatttaac 120

gtgtattgga acgttcctac attcatgtgc cataagtacg ggttgagatt cgaagaggtg 180

agcgagaagt atggcatttt gcaaaattgg atggacaaat tcagaggaga ggaaattgcg 240

atcttatacg accccggcat gttccccgca ctgttgaaag accccaatgg gaatgtagta 300

gctaggaatg gcggagtgcc ccagcttggc aatttaacta aacatttaca ggtgttcagg 360

gaccacctaa tcaatcagat tccagataaa agctttcccg gagttggtgt aatagatttc 420

gagtcatgga gacctatttt cagacagaac tgggcctctt tgcaaccgta caagaagcta 480

tcagtcgaag tcgtacgtcg tgagcaccca ttctgggatg atcagagagt ggagcaagag 540

gcgaaaagaa gatttgaaaa gtacggccag cttttcatgg aagagacctt gaaagccgca 600

aaacgtatgc gtcctgcagc caattggggc tactacgctt acccttattg ctacaacctg 660

accccaaacc agccctcagc tcaatgcgag gctacaacaa tgcaggagaa cgacaagatg 720

tcatggttat tcgagtccga ggacgtctta ctgcccagcg tctatttgag gtggaacctg 780

actagcggag agagggttgg tttagttggt ggtcgtgtca aggaagccct gagaatagct 840

cgtcagatga ccacctctag gaagaaggtc ctaccatact actggtacaa ataccaggat 900

aggagagaca cggacctaag tagggctgat cttgaggcga ctctacgtaa aatcacggac 960

ttaggagccg acggtttcat aatctgggga tcttccgatg acatcaacac caaggcaaaa 1020

tgtttgcagt tcagagaata tttgaataac gagttgggcc cggcagtgaa gagaatagcc 1080

ttaaacaata atgcgaatga caggctaaca gtggatgtgt ctgttgacca ggttggatcc 1140

gccatttctc aaatcactga cggtcaaatc caagctacta ccactgctac caccgaagct 1200

accaccactg ctgccccatc ttccaccgtt gaaactgttt ctccatccag caccgaaact 1260

atctctcaac aaactgaaaa tggtgctgct aaggccgctg tcggtatggg tgccggtgct 1320

ctagctgctg ctgctatgtt gttataa 1347

<210> 58
<211> 1515
<212> DNA
<213> Macaca mulatta

<220>
<223> reencoded nucleic acid sequence of hyaluronidase (HYAL)
      originating from Macaca mulatta with a N-terminal secretion
      signal and a C-terminal anchoring signal

<400> 58
atgcaattca gtactgtcgc tagcgtcgcc tttgtggctc ttgcaaattt cgtcgctgct 60

tcacgtgggc ctttattgcc gaacagacca tttgcaacgg tatggaatgc taacacccaa 120

tggtgtttag agaggcactc agtagacgtc gatgtctcca tatttgatgt tgtagcaaac 180

cccggccaga cttttcgtgg gccggacatg actattttct attccagtca gctaggtact 240

tacccttatt ataccccgac gggtgaacct gtctttggtg gccttccaca gaacgcatca 300

cttatagcgc acttggctag gactttccag gatatattgg cagctatacc agcccccgac 360

ttttctgggc tagccgtaat tgactgggaa gcatggagac cgagatgggc atttaactgg 420

gacacgaaag acatttacag gcaaaggtcc cgtgccttag tccaagcaca gcatcccgac 480

tggccagtta ctcaagttga ggctgtggca caagaccagt tccagggtgc tgcgagggca 540

tggatggcag gaaccttgca attaggccgt gctttaagac caaggggact atggggcttc 600

tacggttttc ccgattgcta caattacgat ttcctatctc ctaattacac agggcaatgc 660

ccatcaggga tcagagcaca aaatgaccag ttaggatggt tgtggggtca gagtcgtgca 720

ttatatccca gtatctacat gccggctgtt ttggaaggaa caggcaaatc ccaaatgtac 780

gtgcagcaca gggtcgcgga agcctttagg gttgctgttg cagcaggcga cccaaactta 840

cccgtcctac cgtacgtcca gattttctac gatatgacca accactttct tccattggac 900

gagttagagc atagtcttgg agaatcagcg gctcagggcg cagcaggagt cgtgttatgg 960

gtgtcatggg agaatactag aacaaaagaa tcttgccagg caattaaaga atatatggac 1020

actaccttgg ggccttttat tctgaacgtc acttcgggtg cgttgttatg ctcgcaagcc 1080

ttatgttcaa gccatggtcg ttgtgtccgt cgtccctcac atccaaaagc cttgctaatc 1140

cttaatcccg caagctttag tattcagttg acaccagatg gtggtccctt gtcgttaagg 1200

ggagcattgt cacttgagga ccaggcacaa atggctgttg aattcaaatg caggtgctac 1260

cccgggtggc aaggcccttg gtgtgagcag aagagtatgt ggggatccgc catttctcaa 1320

atcactgacg gtcaaatcca agctactacc actgctacca ccgaagctac caccactgct 1380

gccccatctt ccaccgttga aactgtttct ccatccagca ccgaaactat ctctcaacaa 1440

actgaaaatg gtgctgctaa ggccgctgtc ggtatgggtg ccggtgctct agctgctgct 1500

gctatgttgt tataa 1515

<210> 59
<211> 1293
<212> DNA
<213> Vespa magnifica

<220>
<223> reencoded nucleic acid sequence of hyaluronidase (HYAL)
      originating from Vespa magnifica with a N-terminal secretion
      signal and a C-terminal anchoring signal

<400> 59
atgcaatttt ctacagtggc aagtgttgca ttcgttgcac tagccaactt tgtggcggca 60

gatagctgtg ggtcaaactg cgaaaagagt gagagaccga aaagggtctt caacatttac 120

tggaacgtac ctacattcat gtgtcaccag tacggactat actttgacga ggtcacgaat 180

tttaatataa agcacaacag caaagacaat tttcaagggg acaagatcgc gatcttttat 240

gaccccgggg agtttcccgc tctgctgcca ctaaactatg gtaagtacaa gatcaggaat 300

ggtggtgttc cacaagaggg taacatcacc atccatctgc agagattcat agagtaccta 360

gataagacct atccgaaccg taacttttca ggcatcggtg tgatcgattt cgagaggtgg 420

agaccaattt tcagacagaa ttggggtaat atgaagattt acaagaactt ctccatcgat 480

cttgtgcgta aagagcatcc tttctggaat aagaaaatga tcgagttgga agcttctaaa 540

agattcgaga aatacgcccg tctgttcatg gaagaaacat taaagttggc taagaaaact 600

agaaaacagg ccgattgggg ctactacggt tacccctatt gcttcaacat gtctcctact 660

aatttcgttc ctgactgcga tgtcacagct agggatgaga acaacgagat gtcttggttg 720

tttaacaacc agaatgtcct attaccaagt gtatacatta ggagagagct aactcctgac 780

cagaggattg ggcttgtaca ggggagagtg aaggaagctg tgagaatttc aaataaactg 840

aagcactcac ctaaagtctt cagctattgg tggtatgttt accaagacga gaccaacacc 900

ttcttaacgg agaccgacgt caagaagacg tttcaggaga ttgtgatcaa cggtggagat 960

gggattataa tctggggttc gtcctctgat gtaaacagct tgtccaagtg tacgaggtta 1020

agggagtacc tattgacagt cttgggacca attgctgtta acgtgactga agcagtaaac 1080

ggatccgcca tttctcaaat cactgacggt caaatccaag ctactaccac tgctaccacc 1140

gaagctacca ccactgctgc cccatcttcc accgttgaaa ctgtttctcc atccagcacc 1200

gaaactatct ctcaacaaac tgaaaatggt gctgctaagg ccgctgtcgg tatgggtgcc 1260

ggtgctctag ctgctgctgc tatgttgtta taa                                    1293

<210> 60
<211> 398
<212> PRT
<213> Cupiennius salei

<220>
<223> amino acid sequence of hyaluronidase (HYAL) originating from
      Cupiennius salei with a N-terminal secretion signal

<400> 60
Met Gln Phe Ser Thr Val Ala Ser Val Ala Phe Val Ala Leu Ala Asn
1               5                   10                  15
Phe Val Ala Ala Phe Lys Ile Tyr Trp Asn Val Pro Thr Phe Gln Cys
            20                  25                  30
Thr His Asn Tyr Lys Ile Asp Tyr Val Lys Leu Leu Ser Thr Tyr Gly
        35                  40                  45
Ile Gln Val Asn Asp Gly Gly Lys Phe Gln Gly Asn Gln Val Thr Ile
    50                  55                  60
Phe Tyr Glu Thr Gln Leu Gly Leu Tyr Pro Arg Ile Leu Lys Ser Gly
65                  70                  75                  80
Lys Met Glu Asn Gly Gly Ile Pro Gln Arg Gly Asn Phe Glu Lys His
                85                  90                  95
Leu Glu Lys Ala Ser Thr Asp Leu Gln Lys Val Ile Pro Trp Lys Glu
            100                 105                 110
Phe Ser Gly Leu Gly Val Ile Asp Trp Glu Ala Trp Arg Pro Thr Trp
        115                 120                 125
Glu Phe Asn Trp Glu Pro Leu Arg Ile Tyr Gln Thr Glu Ser Ile Lys
    130                 135                 140
Arg Ala Lys Glu Leu His Pro Thr Ala Asn Asp Ser Ala Val Lys Glu
145                 150                 155                 160
Ile Ala Glu Arg Gln Trp Glu Asp Ser Ala Lys Leu Tyr Met Leu Glu
                165                 170                 175
Thr Leu Arg Leu Ala Lys Lys Leu Arg Pro Gln Ala Pro Trp Cys Tyr
            180                 185                 190
Tyr Leu Phe Pro Asp Cys Tyr Asn Tyr Val Gly Lys Lys Pro Lys Asp
        195                 200                 205
Phe Gln Cys Ser Ala Ser Ile Arg Lys Gly Asn Asp Lys Leu Ser Trp
    210                 215                 220
Leu Trp Lys Asp Ser Thr Ala Leu Cys Pro Ser Ile Tyr Val Tyr Glu
225                 230                 235                 240
Ser Gln Leu Asp Arg Tyr Ser Phe Glu Gln Arg Thr Trp Arg Asp Asn
                245                 250                 255
Glu Lys Leu Arg Glu Ala Leu Arg Val Ala Thr Arg Thr Ser Lys Ile
            260                 265                 270
Tyr Pro Tyr Val Asn Tyr Phe Asp Lys Glu Leu Ile Pro Glu Gln Glu
        275                 280                 285
Val Trp Arg Met Leu Ala Gln Ala Ala Ala Val Gly Gly Ser Gly Ala
    290                 295                 300
Val Ile Trp Gly Ser Ser Ala Ala Val Ala Ser Glu Glu Leu Cys Lys
305                 310                 315                 320
Ser Leu Lys Gln Tyr Ile Ile Glu Thr Leu Gly Pro Ala Ala Glu Lys
                325                 330                 335
Val Ala Trp Arg Ser Asp Leu Cys Ser Lys Glu Ile Cys Asn Asn Gln
            340                 345                 350
Gly Arg Cys Thr Phe Pro Asp Asp Asp Tyr Ala Asn Ala Trp Lys Leu
        355                 360                 365
Phe Thr Asp Asp Thr Val Lys Phe Tyr Ala Gly Asn Ile Thr Cys Arg
    370                 375                 380

Cys Ser Glu Asn Tyr Ser Gly Arg Phe Cys Glu Lys Lys Asn
385                 390                 395

<210> 61
<211> 405
<212> PRT
<213> Tityus serrulatus


<220>
<223> amino acid sequence of hyaluronidase (HYAL) originating from
      Tityus serrulatus with a N-terminal secretion signal

<400> 61
Met Gln Phe Ser Thr Val Ala Ser Val Ala Phe Val Ala Leu Ala Asn
1               5                   10                  15
Phe Val Ala Ala Ala Asp Phe Lys Val Tyr Trp Glu Val Pro Ser Phe
            20                  25                  30
Leu Cys Ser Lys Arg Phe Lys Ile Asn Val Thr Glu Val Leu Thr Ser
        35                  40                  45
His Glu Ile Leu Val Asn Gln Gly Glu Ser Phe Asn Gly Asp Lys Ile
    50                  55                  60
Val Ile Phe Tyr Glu Asn Gln Leu Gly Lys Tyr Pro His Ile Asp Ser
65                  70                  75                  80
Asn Asn Val Glu Ile Asn Gly Gly Ile Leu Gln Val Ala Asp Leu Ala
                85                  90                  95
Lys His Leu Lys Val Ala Lys Asp Asn Ile Thr Lys Phe Val Pro Asn
            100                 105                 110
Pro Asn Phe Asn Gly Val Gly Val Ile Asp Trp Glu Ala Trp Arg Pro
        115                 120                 125
Ser Trp Glu Phe Asn Trp Gly Lys Leu Lys Val Tyr Lys Glu Lys Ser
    130                 135                 140
Ile Asp Leu Val Lys Ser Lys His Pro Glu Trp Pro Ser Asp Arg Val
145                 150                 155                 160
Glu Lys Val Ala Lys Glu Glu Trp Glu Glu Ser Ala Lys Glu Trp Met
                165                 170                 175
Val Lys Thr Leu Lys Leu Ala Gln Glu Met Arg Pro Asn Ala Val Trp
            180                 185                 190
Cys Tyr Tyr Leu Phe Pro Asp Cys Tyr Asn Tyr Phe Gly Lys Asp Gln
        195                 200                 205
Pro Ser Gln Phe Ser Cys Ser Ser Arg Ile Gln Lys Glu Asn Ser Arg
    210                 215                 220
Leu Ser Trp Leu Trp Asn Gln Ser Thr Ala Ile Cys Leu Ser Ile Tyr
225                 230                 235                 240
Ile Gln Glu Ser His Val Thr Lys Tyr Asn Met Ser Gln Arg Thr Trp
                245                 250                 255
Trp Ile Asp Ala Arg Leu Arg Glu Ala Ile Arg Val Ser Glu His Arg
            260                 265                 270
Pro Asn Ile Pro Ile Tyr Pro Tyr Ile Asn Tyr Ile Leu Pro Gly Thr
        275                 280                 285
Asn Gln Thr Val Pro Ala Met Asp Phe Lys Arg Thr Leu Gly Gln Ile
    290                 295                 300
Ala Ser Leu Gly Leu Asp Gly Ala Leu Leu Trp Gly Ser Ser Tyr His
305                 310                 315                 320
Val Leu Thr Glu Ser Gln Cys Lys Ile Thr Ser Asp Tyr Val Lys Ser
                325                 330                 335
Val Ile Ala Pro Thr Val Ala Thr Val Val Leu Asn Thr Asn Arg Cys
            340                 345                 350
Ser Gln Ile Ile Cys Lys Gly Arg Gly Asn Cys Val Trp Pro Glu Glu
        355                 360                 365
Pro Phe Ser Ser Trp Lys Tyr Leu Val Asp Pro Lys Met Pro Val Phe
    370                 375                 380
Lys Pro Thr Asn Ile His Cys Lys Cys Lys Gly Tyr Leu Gly Arg Tyr

385                 390                 395                 400
Cys Glu Ile Pro Lys
                405

<210> 62
<211> 435
<212> PRT
<213> Bos taurus

<220>
<223> amino acid sequence of hyaluronidase (HYAL) originating from Bos
      taurus with a N-terminal secretion signal

<400> 62
Met Gln Phe Ser Thr Val Ala Ser Val Ala Phe Val Ala Leu Ala Asn
1               5                   10                  15
Phe Val Ala Ala Gly Ser Arg Asp Pro Val Val Pro Asn Gln Pro Phe
            20                  25                  30
Thr Thr Ile Trp Asn Ala Asn Thr Glu Trp Cys Met Lys Lys His Gly
        35                  40                  45
Val Asp Val Asp Ile Ser Ile Phe Asp Val Val Thr Asn Pro Gly Gln
    50                  55                  60
Thr Phe Arg Gly Pro Asn Met Thr Ile Phe Tyr Ser Ser Gln Leu Gly
65                  70                  75                  80
Thr Tyr Pro Tyr Tyr Thr Ser Ala Gly Glu Pro Val Phe Gly Gly Leu
                85                  90                  95
Pro Gln Asn Ala Ser Leu Asn Ala His Leu Ala Arg Thr Phe Gln Asp
            100                 105                 110
Ile Leu Ala Ala Met Pro Glu Pro Arg Phe Ser Gly Leu Ala Val Ile
        115                 120                 125
Asp Trp Glu Ala Trp Arg Pro Arg Trp Ala Phe Asn Trp Asp Thr Lys
    130                 135                 140
Asp Ile Tyr Arg Gln Arg Ser Arg Ala Leu Val Gln Lys Gln His Pro
145                 150                 155                 160
Asp Trp Leu Ala Pro Arg Val Glu Ala Ala Ala Gln Asp Gln Phe Glu
                165                 170                 175
Gly Ala Ala Glu Glu Trp Met Ala Gly Thr Leu Lys Leu Gly Gln Ala
            180                 185                 190
Leu Arg Pro Gln Gly Leu Trp Gly Phe Tyr Asn Phe Pro Glu Cys Tyr
        195                 200                 205
Asn Tyr Asp Phe Lys Ser Pro Asn Tyr Thr Gly Arg Cys Pro Leu Asn
    210                 215                 220
Ile Cys Ala Gln Asn Asp Gln Leu Gly Trp Leu Trp Gly Gln Ser Arg
225                 230                 235                 240
Ala Leu Tyr Pro Ser Ile Tyr Leu Pro Ala Ala Leu Glu Gly Thr Lys
                245                 250                 255
Lys Thr Gln Met Phe Val Gln His Arg Val Ala Glu Ala Phe Arg Val
            260                 265                 270
Ala Ala Gly Ala Gly Asp Pro Lys Leu Pro Val Leu Pro Tyr Met Gln
        275                 280                 285
Leu Phe Tyr Asp Met Thr Asn His Phe Leu Pro Ala Glu Glu Leu Glu
    290                 295                 300
His Ser Leu Gly Glu Ser Ala Ala Gln Gly Ala Ala Gly Val Val Leu
305                 310                 315                 320
Trp Val Ser Trp Leu Ser Thr Ser Thr Lys Glu Ser Cys Gln Ala Ile
                325                 330                 335
Lys Glu Tyr Val Asp Thr Thr Leu Gly Pro Ser Ile Leu Asn Val Thr
            340                 345                 350
Ser Gly Ala Arg Leu Cys Ser Gln Val Leu Cys Ser Gly His Gly Arg
        355                 360                 365
Cys Ala Arg Arg Pro Ser Tyr Pro Lys Ala Arg Leu Ile Leu Asn Ser
    370                 375                 380

Thr Ser Phe Ser Ile Lys Pro Thr Pro Gly Gly Gly Pro Leu Thr Leu
385                 390                 395                 400
Gln Gly Ala Leu Ser Leu Glu Asp Arg Leu Arg Met Ala Val Glu Phe
                405                 410                 415
Glu Cys Arg Cys Tyr Arg Gly Trp Arg Gly Thr Arg Cys Glu Gln Trp
            420                 425                 430
Gly Met Trp
        435

<210> 63
<211> 378
<212> PRT
<213> Apis mellifera

<220>
<223> amino acid sequence of hyaluronidase (HYAL) originating from Apis
      mellifera with a N-terminal secretion signal

<400> 63
Met Gln Phe Ser Thr Val Ala Ser Val Ala Phe Val Ala Leu Ala Asn
1               5                   10                  15
Phe Val Ala Ala Leu Leu Leu Gly Phe Val Gln Ser Thr Pro Asp Asn
            20                  25                  30
Asn Lys Thr Val Arg Glu Phe Asn Val Tyr Trp Asn Val Pro Thr Phe
        35                  40                  45
Met Cys His Lys Tyr Gly Leu Arg Phe Glu Glu Val Ser Glu Lys Tyr
    50                  55                  60
Gly Ile Leu Gln Asn Trp Met Asp Lys Phe Arg Gly Glu Glu Ile Ala
65                  70                  75                  80
Ile Leu Tyr Asp Pro Gly Met Phe Pro Ala Leu Leu Lys Asp Pro Asn
                85                  90                  95
Gly Asn Val Val Ala Arg Asn Gly Gly Val Pro Gln Leu Gly Asn Leu
            100                 105                 110
Thr Lys His Leu Gln Val Phe Arg Asp His Leu Ile Asn Gln Ile Pro
        115                 120                 125
Asp Lys Ser Phe Pro Gly Val Gly Val Ile Asp Phe Glu Ser Trp Arg
    130                 135                 140
Pro Ile Phe Arg Gln Asn Trp Ala Ser Leu Gln Pro Tyr Lys Lys Leu
145                 150                 155                 160
Ser Val Glu Val Val Arg Arg Glu His Pro Phe Trp Asp Asp Gln Arg
                165                 170                 175
Val Glu Gln Glu Ala Lys Arg Arg Phe Glu Lys Tyr Gly Gln Leu Phe
            180                 185                 190
Met Glu Glu Thr Leu Lys Ala Ala Lys Arg Met Arg Pro Ala Ala Asn
        195                 200                 205
Trp Gly Tyr Tyr Ala Tyr Pro Tyr Cys Tyr Asn Leu Thr Pro Asn Gln
    210                 215                 220
Pro Ser Ala Gln Cys Glu Ala Thr Thr Met Gln Glu Asn Asp Lys Met
225                 230                 235                 240
Ser Trp Leu Phe Glu Ser Glu Asp Val Leu Leu Pro Ser Val Tyr Leu
                245                 250                 255
Arg Trp Asn Leu Thr Ser Gly Glu Arg Val Gly Leu Val Gly Gly Arg
            260                 265                 270
Val Lys Glu Ala Leu Arg Ile Ala Arg Gln Met Thr Thr Ser Arg Lys
        275                 280                 285
Lys Val Leu Pro Tyr Tyr Trp Tyr Lys Tyr Gln Asp Arg Arg Asp Thr
    290                 295                 300
Asp Leu Ser Arg Ala Asp Leu Glu Ala Thr Leu Arg Lys Ile Thr Asp
305                 310                 315                 320
Leu Gly Ala Asp Gly Phe Ile Ile Trp Gly Ser Ser Asp Asp Ile Asn
                325                 330                 335
Thr Lys Ala Lys Cys Leu Gln Phe Arg Glu Tyr Leu Asn Asn Glu Leu

340                 345                 350
Gly Pro Ala Val Lys Arg Ile Ala Leu Asn Asn Asn Ala Asn Asp Arg
        355                 360                 365
Leu Thr Val Asp Val Ser Val Asp Gln Val
    370                 375

<210> 64
<211> 434
<212> PRT
<213> Macaca mulatta

<220>
<223> amino acid sequence of hyaluronidase (HYAL) originating from
      Macaca mulatta with a N-terminal secretion signal

<400> 64
Met Gln Phe Ser Thr Val Ala Ser Val Ala Phe Val Ala Leu Ala Asn
1               5                   10                  15
Phe Val Ala Ala Ser Arg Gly Pro Leu Leu Pro Asn Arg Pro Phe Ala
            20                  25                  30
Thr Val Trp Asn Ala Asn Thr Gln Trp Cys Leu Glu Arg His Ser Val
        35                  40                  45
Asp Val Asp Val Ser Ile Phe Asp Val Val Ala Asn Pro Gly Gln Thr
    50                  55                  60
Phe Arg Gly Pro Asp Met Thr Ile Phe Tyr Ser Ser Gln Leu Gly Thr
65                  70                  75                  80
Tyr Pro Tyr Tyr Thr Pro Thr Gly Glu Pro Val Phe Gly Gly Leu Pro
                85                  90                  95
Gln Asn Ala Ser Leu Ile Ala His Leu Ala Arg Thr Phe Gln Asp Ile
            100                 105                 110
Leu Ala Ala Ile Pro Ala Pro Asp Phe Ser Gly Leu Ala Val Ile Asp
        115                 120                 125
Trp Glu Ala Trp Arg Pro Arg Trp Ala Phe Asn Trp Asp Thr Lys Asp
    130                 135                 140
Ile Tyr Arg Gln Arg Ser Arg Ala Leu Val Gln Ala Gln His Pro Asp
145                 150                 155                 160
Trp Pro Val Thr Gln Val Glu Ala Val Ala Gln Asp Gln Phe Gln Gly
                165                 170                 175
Ala Ala Arg Ala Trp Met Ala Gly Thr Leu Gln Leu Gly Arg Ala Leu
            180                 185                 190
Arg Pro Arg Gly Leu Trp Gly Phe Tyr Gly Phe Pro Asp Cys Tyr Asn
        195                 200                 205
Tyr Asp Phe Leu Ser Pro Asn Tyr Thr Gly Gln Cys Pro Ser Gly Ile
    210                 215                 220
Arg Ala Gln Asn Asp Gln Leu Gly Trp Leu Trp Gly Gln Ser Arg Ala
225                 230                 235                 240
Leu Tyr Pro Ser Ile Tyr Met Pro Ala Val Leu Glu Gly Thr Gly Lys
                245                 250                 255
Ser Gln Met Tyr Val Gln His Arg Val Ala Glu Ala Phe Arg Val Ala
            260                 265                 270
Val Ala Ala Gly Asp Pro Asn Leu Pro Val Leu Pro Tyr Val Gln Ile
        275                 280                 285
Phe Tyr Asp Met Thr Asn His Phe Leu Pro Leu Asp Glu Leu Glu His
    290                 295                 300
Ser Leu Gly Glu Ser Ala Ala Gln Gly Ala Ala Gly Val Val Leu Trp
305                 310                 315                 320
Val Ser Trp Glu Asn Thr Arg Thr Lys Glu Ser Cys Gln Ala Ile Lys
                325                 330                 335
Glu Tyr Met Asp Thr Thr Leu Gly Pro Phe Ile Leu Asn Val Thr Ser
            340                 345                 350
Gly Ala Leu Leu Cys Ser Gln Ala Leu Cys Ser Ser His Gly Arg Cys
        355                 360                 365

Val Arg Arg Pro Ser His Pro Lys Ala Leu Leu Ile Leu Asn Pro Ala
    370                 375                 380
Ser Phe Ser Ile Gln Leu Thr Pro Asp Gly Gly Pro Leu Ser Leu Arg
385                 390                 395                 400
Gly Ala Leu Ser Leu Glu Asp Gln Ala Gln Met Ala Val Glu Phe Lys
                405                 410                 415
Cys Arg Cys Tyr Pro Gly Trp Gln Gly Pro Trp Cys Glu Gln Lys Ser
            420                 425                 430
Met Trp

<210> 65
<211> 360
<212> PRT
<213> Vespa magnifica

<220>
<223> amino acid sequence of hyaluronidase (HYAL) originating from
      Vespa magnifica with a N-terminal secretion signal

<400> 65
Met Gln Phe Ser Thr Val Ala Ser Val Ala Phe Val Ala Leu Ala Asn
1               5                   10                  15
Phe Val Ala Ala Asp Ser Cys Gly Ser Asn Cys Glu Lys Ser Glu Arg
            20                  25                  30
Pro Lys Arg Val Phe Asn Ile Tyr Trp Asn Val Pro Thr Phe Met Cys
        35                  40                  45
His Gln Tyr Gly Leu Tyr Phe Asp Glu Val Thr Asn Phe Asn Ile Lys
    50                  55                  60
His Asn Ser Lys Asp Asn Phe Gln Gly Asp Lys Ile Ala Ile Phe Tyr
65                  70                  75                  80
Asp Pro Gly Glu Phe Pro Ala Leu Leu Pro Leu Asn Tyr Gly Lys Tyr
                85                  90                  95
Lys Ile Arg Asn Gly Gly Val Pro Gln Glu Gly Asn Ile Thr Ile His
            100                 105                 110
Leu Gln Arg Phe Ile Glu Tyr Leu Asp Lys Thr Tyr Pro Asn Arg Asn
        115                 120                 125
Phe Ser Gly Ile Gly Val Ile Asp Phe Glu Arg Trp Arg Pro Ile Phe
    130                 135                 140
Arg Gln Asn Trp Gly Asn Met Lys Ile Tyr Lys Asn Phe Ser Ile Asp
145                 150                 155                 160
Leu Val Arg Lys Glu His Pro Phe Trp Asn Lys Lys Met Ile Glu Leu
                165                 170                 175
Glu Ala Ser Lys Arg Phe Glu Lys Tyr Ala Arg Leu Phe Met Glu Glu
            180                 185                 190
Thr Leu Lys Leu Ala Lys Lys Thr Arg Lys Gln Ala Asp Trp Gly Tyr
        195                 200                 205
Tyr Gly Tyr Pro Tyr Cys Phe Asn Met Ser Pro Thr Asn Phe Val Pro
    210                 215                 220
Asp Cys Asp Val Thr Ala Arg Asp Glu Asn Asn Glu Met Ser Trp Leu
225                 230                 235                 240
Phe Asn Asn Gln Asn Val Leu Leu Pro Ser Val Tyr Ile Arg Arg Glu
                245                 250                 255
Leu Thr Pro Asp Gln Arg Ile Gly Leu Val Gln Gly Arg Val Lys Glu
            260                 265                 270
Ala Val Arg Ile Ser Asn Lys Leu Lys His Ser Pro Lys Val Phe Ser
        275                 280                 285
Tyr Trp Trp Tyr Val Tyr Gln Asp Glu Thr Asn Thr Phe Leu Thr Glu
    290                 295                 300
Thr Asp Val Lys Lys Thr Phe Gln Glu Ile Val Ile Asn Gly Gly Asp
305                 310                 315                 320
Gly Ile Ile Ile Trp Gly Ser Ser Ser Asp Val Asn Ser Leu Ser Lys

325                 330                 335
Cys Thr Arg Leu Arg Glu Tyr Leu Leu Thr Val Leu Gly Pro Ile Ala
            340                 345                 350
Val Asn Val Thr Glu Ala Val Asn
        355                 360

<210> 66
<211> 468
<212> PRT
<213> Cupiennius salei

<220>
<223> amino acid sequence of hyaluronidase (HYAL) originating from
      Cupiennius salei with a N-terminal secretion signal and a
      C-terminal anchoring signal

<400> 66
Met Gln Phe Ser Thr Val Ala Ser Val Ala Phe Val Ala Leu Ala Asn
1               5                   10                  15
Phe Val Ala Ala Phe Lys Ile Tyr Trp Asn Val Pro Thr Phe Gln Cys
            20                  25                  30
Thr His Asn Tyr Lys Ile Asp Tyr Val Lys Leu Leu Ser Thr Tyr Gly
        35                  40                  45
Ile Gln Val Asn Asp Gly Gly Lys Phe Gln Gly Asn Gln Val Thr Ile
    50                  55                  60
Phe Tyr Glu Thr Gln Leu Gly Leu Tyr Pro Arg Ile Leu Lys Ser Gly
65                  70                  75                  80
Lys Met Glu Asn Gly Gly Ile Pro Gln Arg Gly Asn Phe Glu Lys His
                85                  90                  95
Leu Glu Lys Ala Ser Thr Asp Leu Gln Lys Val Ile Pro Trp Lys Glu
            100                 105                 110
Phe Ser Gly Leu Gly Val Ile Asp Trp Glu Ala Trp Arg Pro Thr Trp
        115                 120                 125
Glu Phe Asn Trp Glu Pro Leu Arg Ile Tyr Gln Thr Glu Ser Ile Lys
    130                 135                 140
Arg Ala Lys Glu Leu His Pro Thr Ala Asn Asp Ser Ala Val Lys Glu
145                 150                 155                 160
Ile Ala Glu Arg Gln Trp Glu Asp Ser Ala Lys Leu Tyr Met Leu Glu
                165                 170                 175
Thr Leu Arg Leu Ala Lys Lys Leu Arg Pro Gln Ala Pro Trp Cys Tyr
            180                 185                 190
Tyr Leu Phe Pro Asp Cys Tyr Asn Tyr Val Gly Lys Lys Pro Lys Asp
        195                 200                 205
Phe Gln Cys Ser Ala Ser Ile Arg Lys Gly Asn Asp Lys Leu Ser Trp
    210                 215                 220
Leu Trp Lys Asp Ser Thr Ala Leu Cys Pro Ser Ile Tyr Val Tyr Glu
225                 230                 235                 240
Ser Gln Leu Asp Arg Tyr Ser Phe Glu Gln Arg Thr Trp Arg Asp Asn
                245                 250                 255
Glu Lys Leu Arg Glu Ala Leu Arg Val Ala Thr Arg Thr Ser Lys Ile
            260                 265                 270
Tyr Pro Tyr Val Asn Tyr Phe Asp Lys Glu Leu Ile Pro Glu Gln Glu
        275                 280                 285
Val Trp Arg Met Leu Ala Gln Ala Ala Ala Val Gly Gly Ser Gly Ala
    290                 295                 300
Val Ile Trp Gly Ser Ser Ala Ala Val Ala Ser Glu Glu Leu Cys Lys
305                 310                 315                 320
Ser Leu Lys Gln Tyr Ile Ile Glu Thr Leu Gly Pro Ala Ala Glu Lys
                325                 330                 335
Val Ala Trp Arg Ser Asp Leu Cys Ser Lys Glu Ile Cys Asn Asn Gln
            340                 345                 350
Gly Arg Cys Thr Phe Pro Asp Asp Asp Tyr Ala Asn Ala Trp Lys Leu

355                 360                 365
Phe Thr Asp Asp Thr Val Lys Phe Tyr Ala Gly Asn Ile Thr Cys Arg
    370                 375                 380
Cys Ser Glu Asn Tyr Ser Gly Arg Phe Cys Glu Lys Lys Asn Gly Ser
385                 390                 395                 400
Ala Ile Ser Gln Ile Thr Asp Gly Gln Ile Gln Ala Thr Thr Thr Ala
                405                 410                 415
Thr Thr Glu Ala Thr Thr Thr Ala Ala Pro Ser Ser Thr Val Glu Thr
            420                 425                 430
Val Ser Pro Ser Ser Thr Glu Thr Ile Ser Gln Gln Thr Glu Asn Gly
        435                 440                 445
Ala Ala Lys Ala Ala Val Gly Met Gly Ala Gly Ala Leu Ala Ala Ala
    450                 455                 460
Ala Met Leu Leu
465

<210> 67
<211> 475
<212> PRT
<213> Tityus serrulatus

<220>
<223> amino acid sequence of hyaluronidase (HYAL) originating from
      Tityus serrulatus with a N-terminal secretion signal and a
      C-terminal anchoring signal

<400> 67
Met Gln Phe Ser Thr Val Ala Ser Val Ala Phe Val Ala Leu Ala Asn
1               5                   10                  15
Phe Val Ala Ala Ala Asp Phe Lys Val Tyr Trp Glu Val Pro Ser Phe
            20                  25                  30
Leu Cys Ser Lys Arg Phe Lys Ile Asn Val Thr Glu Val Leu Thr Ser
        35                  40                  45
His Glu Ile Leu Val Asn Gln Gly Glu Ser Phe Asn Gly Asp Lys Ile
    50                  55                  60
Val Ile Phe Tyr Glu Asn Gln Leu Gly Lys Tyr Pro His Ile Asp Ser
65                  70                  75                  80
Asn Asn Val Glu Ile Asn Gly Gly Ile Leu Gln Val Ala Asp Leu Ala
                85                  90                  95
Lys His Leu Lys Val Ala Lys Asp Asn Ile Thr Lys Phe Val Pro Asn
            100                 105                 110
Pro Asn Phe Asn Gly Val Gly Val Ile Asp Trp Glu Ala Trp Arg Pro
        115                 120                 125
Ser Trp Glu Phe Asn Trp Gly Lys Leu Lys Val Tyr Lys Glu Lys Ser
    130                 135                 140
Ile Asp Leu Val Lys Ser Lys His Pro Glu Trp Pro Ser Asp Arg Val
145                 150                 155                 160
Glu Lys Val Ala Lys Glu Glu Trp Glu Glu Ser Ala Lys Glu Trp Met
                165                 170                 175
Val Lys Thr Leu Lys Leu Ala Gln Glu Met Arg Pro Asn Ala Val Trp
            180                 185                 190
Cys Tyr Tyr Leu Phe Pro Asp Cys Tyr Asn Tyr Phe Gly Lys Asp Gln
        195                 200                 205
Pro Ser Gln Phe Ser Cys Ser Ser Arg Ile Gln Lys Glu Asn Ser Arg
    210                 215                 220
Leu Ser Trp Leu Trp Asn Gln Ser Thr Ala Ile Cys Leu Ser Ile Tyr
225                 230                 235                 240
Ile Gln Glu Ser His Val Thr Lys Tyr Asn Met Ser Gln Arg Thr Trp
                245                 250                 255
Trp Ile Asp Ala Arg Leu Arg Glu Ala Ile Arg Val Ser Glu His Arg
            260                 265                 270
Pro Asn Ile Pro Ile Tyr Pro Tyr Ile Asn Tyr Ile Leu Pro Gly Thr

275                 280                 285
Asn Gln Thr Val Pro Ala Met Asp Phe Lys Arg Thr Leu Gly Gln Ile
    290                 295                 300
Ala Ser Leu Gly Leu Asp Gly Ala Leu Leu Trp Gly Ser Ser Tyr His
305                 310                 315                 320
Val Leu Thr Glu Ser Gln Cys Lys Ile Thr Ser Asp Tyr Val Lys Ser
                325                 330                 335
Val Ile Ala Pro Thr Val Ala Thr Val Val Leu Asn Thr Asn Arg Cys
            340                 345                 350
Ser Gln Ile Ile Cys Lys Gly Arg Gly Asn Cys Val Trp Pro Glu Glu
        355                 360                 365
Pro Phe Ser Ser Trp Lys Tyr Leu Val Asp Pro Lys Met Pro Val Phe
    370                 375                 380
Lys Pro Thr Asn Ile His Cys Lys Cys Lys Gly Tyr Leu Gly Arg Tyr
385                 390                 395                 400
Cys Glu Ile Pro Lys Gly Ser Ala Ile Ser Gln Ile Thr Asp Gly Gln
                405                 410                 415
Ile Gln Ala Thr Thr Thr Ala Thr Thr Glu Ala Thr Thr Thr Ala Ala
            420                 425                 430
Pro Ser Ser Thr Val Glu Thr Val Ser Pro Ser Ser Thr Glu Thr Ile
        435                 440                 445
Ser Gln Gln Thr Glu Asn Gly Ala Ala Lys Ala Ala Val Gly Met Gly
    450                 455                 460
Ala Gly Ala Leu Ala Ala Ala Ala Met Leu Leu
465                 470                 475

<210> 68
<211> 448
<212> PRT
<213> Bos taurus


<220>
<223> amino acid sequence of hyaluronidase (HYAL) originating from Bos
      taurus with a N-terminal secretion signal and a C-terminal
      anchoring signal

<400> 68
Met Gln Phe Ser Thr Val Ala Ser Val Ala Phe Val Ala Leu Ala Asn
1               5                   10                  15
Phe Val Ala Ala Leu Leu Leu Gly Phe Val Gln Ser Thr Pro Asp Asn
            20                  25                  30
Asn Lys Thr Val Arg Glu Phe Asn Val Tyr Trp Asn Val Pro Thr Phe
        35                  40                  45
Met Cys His Lys Tyr Gly Leu Arg Phe Glu Glu Val Ser Glu Lys Tyr
    50                  55                  60
Gly Ile Leu Gln Asn Trp Met Asp Lys Phe Arg Gly Glu Glu Ile Ala
65                  70                  75                  80
Ile Leu Tyr Asp Pro Gly Met Phe Pro Ala Leu Leu Lys Asp Pro Asn
                85                  90                  95
Gly Asn Val Val Ala Arg Asn Gly Gly Val Pro Gln Leu Gly Asn Leu
            100                 105                 110
Thr Lys His Leu Gln Val Phe Arg Asp His Leu Ile Asn Gln Ile Pro
        115                 120                 125
Asp Lys Ser Phe Pro Gly Val Gly Val Ile Asp Phe Glu Ser Trp Arg
    130                 135                 140
Pro Ile Phe Arg Gln Asn Trp Ala Ser Leu Gln Pro Tyr Lys Lys Leu
145                 150                 155                 160
Ser Val Glu Val Val Arg Arg Glu His Pro Phe Trp Asp Asp Gln Arg
                165                 170                 175
Val Glu Gln Glu Ala Lys Arg Arg Phe Glu Lys Tyr Gly Gln Leu Phe
            180                 185                 190
Met Glu Glu Thr Leu Lys Ala Ala Lys Arg Met Arg Pro Ala Ala Asn

195                 200                 205
Trp Gly Tyr Tyr Ala Tyr Pro Tyr Cys Tyr Asn Leu Thr Pro Asn Gln
    210                 215                 220
Pro Ser Ala Gln Cys Glu Ala Thr Thr Met Gln Glu Asn Asp Lys Met
225                 230                 235                 240
Ser Trp Leu Phe Glu Ser Glu Asp Val Leu Leu Pro Ser Val Tyr Leu
                245                 250                 255
Arg Trp Asn Leu Thr Ser Gly Glu Arg Val Gly Leu Val Gly Gly Arg
            260                 265                 270
Val Lys Glu Ala Leu Arg Ile Ala Arg Gln Met Thr Thr Ser Arg Lys
        275                 280                 285
Lys Val Leu Pro Tyr Tyr Trp Tyr Lys Tyr Gln Asp Arg Arg Asp Thr
    290                 295                 300
Asp Leu Ser Arg Ala Asp Leu Glu Ala Thr Leu Arg Lys Ile Thr Asp
305                 310                 315                 320
Leu Gly Ala Asp Gly Phe Ile Ile Trp Gly Ser Ser Asp Asp Ile Asn
                325                 330                 335
Thr Lys Ala Lys Cys Leu Gln Phe Arg Glu Tyr Leu Asn Asn Glu Leu
            340                 345                 350
Gly Pro Ala Val Lys Arg Ile Ala Leu Asn Asn Asn Ala Asn Asp Arg
        355                 360                 365
Leu Thr Val Asp Val Ser Val Asp Gln Val Gly Ser Ala Ile Ser Gln
    370                 375                 380
Ile Thr Asp Gly Gln Ile Gln Ala Thr Thr Thr Ala Thr Thr Glu Ala
385                 390                 395                 400
Thr Thr Thr Ala Ala Pro Ser Ser Thr Val Glu Thr Val Ser Pro Ser
                405                 410                 415
Ser Thr Glu Thr Ile Ser Gln Gln Thr Glu Asn Gly Ala Ala Lys Ala
            420                 425                 430
Ala Val Gly Met Gly Ala Gly Ala Leu Ala Ala Ala Ala Met Leu Leu
        435                 440                 445

<210> 69
<211> 448
<212> PRT
<213> Apis mellifera

<220>
<223> amino acid sequence of hyaluronidase (HYAL) originating from Apis
      mellifera with a N-terminal secretion signal and a C-terminal
      anchoring signal

<400> 69
Met Gln Phe Ser Thr Val Ala Ser Val Ala Phe Val Ala Leu Ala Asn
1               5                   10                  15
Phe Val Ala Ala Leu Leu Leu Gly Phe Val Gln Ser Thr Pro Asp Asn
            20                  25                  30
Asn Lys Thr Val Arg Glu Phe Asn Val Tyr Trp Asn Val Pro Thr Phe
        35                  40                  45
Met Cys His Lys Tyr Gly Leu Arg Phe Glu Glu Val Ser Glu Lys Tyr
    50                  55                  60
Gly Ile Leu Gln Asn Trp Met Asp Lys Phe Arg Gly Glu Glu Ile Ala
65                  70                  75                  80
Ile Leu Tyr Asp Pro Gly Met Phe Pro Ala Leu Leu Lys Asp Pro Asn
                85                  90                  95
Gly Asn Val Val Ala Arg Asn Gly Gly Val Pro Gln Leu Gly Asn Leu
            100                 105                 110
Thr Lys His Leu Gln Val Phe Arg Asp His Leu Ile Asn Gln Ile Pro
        115                 120                 125
Asp Lys Ser Phe Pro Gly Val Gly Val Ile Asp Phe Glu Ser Trp Arg

130                 135                 140
Pro Ile Phe Arg Gln Asn Trp Ala Ser Leu Gln Pro Tyr Lys Lys Leu
145                 150                 155                 160
Ser Val Glu Val Val Arg Arg Glu His Pro Phe Trp Asp Asp Gln Arg
                165                 170                 175
Val Glu Gln Glu Ala Lys Arg Arg Phe Glu Lys Tyr Gly Gln Leu Phe
            180                 185                 190
Met Glu Glu Thr Leu Lys Ala Ala Lys Arg Met Arg Pro Ala Ala Asn
        195                 200                 205
Trp Gly Tyr Tyr Ala Tyr Pro Tyr Cys Tyr Asn Leu Thr Pro Asn Gln
    210                 215                 220
Pro Ser Ala Gln Cys Glu Ala Thr Thr Met Gln Glu Asn Asp Lys Met
225                 230                 235                 240
Ser Trp Leu Phe Glu Ser Glu Asp Val Leu Leu Pro Ser Val Tyr Leu
                245                 250                 255
Arg Trp Asn Leu Thr Ser Gly Glu Arg Val Gly Leu Val Gly Gly Arg
            260                 265                 270
Val Lys Glu Ala Leu Arg Ile Ala Arg Gln Met Thr Thr Ser Arg Lys
        275                 280                 285
Lys Val Leu Pro Tyr Tyr Trp Tyr Lys Tyr Gln Asp Arg Arg Asp Thr
    290                 295                 300
Asp Leu Ser Arg Ala Asp Leu Glu Ala Thr Leu Arg Lys Ile Thr Asp
305                 310                 315                 320
Leu Gly Ala Asp Gly Phe Ile Ile Trp Gly Ser Ser Asp Asp Ile Asn
                325                 330                 335
Thr Lys Ala Lys Cys Leu Gln Phe Arg Glu Tyr Leu Asn Asn Glu Leu
            340                 345                 350
Gly Pro Ala Val Lys Arg Ile Ala Leu Asn Asn Asn Ala Asn Asp Arg
        355                 360                 365
Leu Thr Val Asp Val Ser Val Asp Gln Val Gly Ser Ala Ile Ser Gln
    370                 375                 380
Ile Thr Asp Gly Gln Ile Gln Ala Thr Thr Thr Ala Thr Thr Glu Ala
385                 390                 395                 400
Thr Thr Thr Ala Ala Pro Ser Ser Thr Val Glu Thr Val Ser Pro Ser
                405                 410                 415
Ser Thr Glu Thr Ile Ser Gln Gln Thr Glu Asn Gly Ala Ala Lys Ala
            420                 425                 430
Ala Val Gly Met Gly Ala Gly Ala Leu Ala Ala Ala Ala Met Leu Leu
        435                 440                 445

<210> 70
<211> 504
<212> PRT
<213> Macaca mulatta

<220>
<223> amino acid sequence of hyaluronidase (HYAL) originating from
      Macaca mulatta with a N-terminal secretion signal and a
      C-terminal anchoring signal

<400> 70
Met Gln Phe Ser Thr Val Ala Ser Val Ala Phe Val Ala Leu Ala Asn
1               5                   10                  15
Phe Val Ala Ala Ser Arg Gly Pro Leu Leu Pro Asn Arg Pro Phe Ala
            20                  25                  30
Thr Val Trp Asn Ala Asn Thr Gln Trp Cys Leu Glu Arg His Ser Val
        35                  40                  45
Asp Val Asp Val Ser Ile Phe Asp Val Val Ala Asn Pro Gly Gln Thr
    50                  55                  60
Phe Arg Gly Pro Asp Met Thr Ile Phe Tyr Ser Ser Gln Leu Gly Thr

65                  70                  75                  80
Tyr Pro Tyr Tyr Thr Pro Thr Gly Glu Pro Val Phe Gly Gly Leu Pro
                85                  90                  95
Gln Asn Ala Ser Leu Ile Ala His Leu Ala Arg Thr Phe Gln Asp Ile
            100                 105                 110
Leu Ala Ala Ile Pro Ala Pro Asp Phe Ser Gly Leu Ala Val Ile Asp
        115                 120                 125
Trp Glu Ala Trp Arg Pro Arg Trp Ala Phe Asn Trp Asp Thr Lys Asp
    130                 135                 140
Ile Tyr Arg Gln Arg Ser Arg Ala Leu Val Gln Ala Gln His Pro Asp
145                 150                 155                 160
Trp Pro Val Thr Gln Val Glu Ala Val Ala Gln Asp Gln Phe Gln Gly
                165                 170                 175
Ala Ala Arg Ala Trp Met Ala Gly Thr Leu Gln Leu Gly Arg Ala Leu
            180                 185                 190
Arg Pro Arg Gly Leu Trp Gly Phe Tyr Gly Phe Pro Asp Cys Tyr Asn
        195                 200                 205
Tyr Asp Phe Leu Ser Pro Asn Tyr Thr Gly Gln Cys Pro Ser Gly Ile
    210                 215                 220
Arg Ala Gln Asn Asp Gln Leu Gly Trp Leu Trp Gly Gln Ser Arg Ala
225                 230                 235                 240
Leu Tyr Pro Ser Ile Tyr Met Pro Ala Val Leu Glu Gly Thr Gly Lys
                245                 250                 255
Ser Gln Met Tyr Val Gln His Arg Val Ala Glu Ala Phe Arg Val Ala
            260                 265                 270
Val Ala Ala Gly Asp Pro Asn Leu Pro Val Leu Pro Tyr Val Gln Ile
        275                 280                 285
Phe Tyr Asp Met Thr Asn His Phe Leu Pro Leu Asp Glu Leu Glu His
    290                 295                 300
Ser Leu Gly Glu Ser Ala Ala Gln Gly Ala Ala Gly Val Val Leu Trp
305                 310                 315                 320
Val Ser Trp Glu Asn Thr Arg Thr Lys Glu Ser Cys Gln Ala Ile Lys
                325                 330                 335
Glu Tyr Met Asp Thr Thr Leu Gly Pro Phe Ile Leu Asn Val Thr Ser
            340                 345                 350
Gly Ala Leu Leu Cys Ser Gln Ala Leu Cys Ser Ser His Gly Arg Cys
        355                 360                 365
Val Arg Arg Pro Ser His Pro Lys Ala Leu Leu Ile Leu Asn Pro Ala
    370                 375                 380
Ser Phe Ser Ile Gln Leu Thr Pro Asp Gly Gly Pro Leu Ser Leu Arg
385                 390                 395                 400
Gly Ala Leu Ser Leu Glu Asp Gln Ala Gln Met Ala Val Glu Phe Lys
                405                 410                 415
Cys Arg Cys Tyr Pro Gly Trp Gln Gly Pro Trp Cys Glu Gln Lys Ser
            420                 425                 430
Met Trp Gly Ser Ala Ile Ser Gln Ile Thr Asp Gly Gln Ile Gln Ala
        435                 440                 445
Thr Thr Thr Ala Thr Thr Glu Ala Thr Thr Thr Ala Ala Pro Ser Ser
    450                 455                 460
Thr Val Glu Thr Val Ser Pro Ser Ser Thr Glu Thr Ile Ser Gln Gln
465                 470                 475                 480
Thr Glu Asn Gly Ala Ala Lys Ala Ala Val Gly Met Gly Ala Gly Ala
                485                 490                 495
Leu Ala Ala Ala Ala Met Leu Leu
            500

<210> 71
<211> 360
<212> PRT
<213> Vespa magnifica


<220>

<223> amino acid sequence of hyaluronidase (HYAL) originating from
      Vespa magnifica with a N-terminal secretion signal and a
      C-terminal anchoring signal

<400> 71
Asp Asn Phe Gln Gly Asp Lys Ile Ala Ile Phe Tyr Asp Pro Gly Glu
1               5                   10                  15
Phe Pro Ala Leu Leu Pro Leu Asn Tyr Gly Lys Tyr Lys Ile Arg Asn
            20                  25                  30
Gly Gly Val Pro Gln Glu Gly Asn Ile Thr Ile His Leu Gln Arg Phe
        35                  40                  45
Ile Glu Tyr Leu Asp Lys Thr Tyr Pro Asn Arg Asn Phe Ser Gly Ile
    50                  55                  60
Gly Val Ile Asp Phe Glu Arg Trp Arg Pro Ile Phe Arg Gln Asn Trp
65                  70                  75                  80
Gly Asn Met Lys Ile Tyr Lys Asn Phe Ser Ile Asp Leu Val Arg Lys
                85                  90                  95
Glu His Pro Phe Trp Asn Lys Lys Met Ile Glu Leu Glu Ala Ser Lys
            100                 105                 110
Arg Phe Glu Lys Tyr Ala Arg Leu Phe Met Glu Glu Thr Leu Lys Leu
        115                 120                 125
Ala Lys Lys Thr Arg Lys Gln Ala Asp Trp Gly Tyr Tyr Gly Tyr Pro
    130                 135                 140
Tyr Cys Phe Asn Met Ser Pro Thr Asn Phe Val Pro Asp Cys Asp Val
145                 150                 155                 160
Thr Ala Arg Asp Glu Asn Asn Glu Met Ser Trp Leu Phe Asn Asn Gln
                165                 170                 175
Asn Val Leu Leu Pro Ser Val Tyr Ile Arg Arg Glu Leu Thr Pro Asp
            180                 185                 190
Gln Arg Ile Gly Leu Val Gln Gly Arg Val Lys Glu Ala Val Arg Ile
        195                 200                 205
Ser Asn Lys Leu Lys His Ser Pro Lys Val Phe Ser Tyr Trp Trp Tyr
    210                 215                 220
Val Tyr Gln Asp Glu Thr Asn Thr Phe Leu Thr Glu Thr Asp Val Lys
225                 230                 235                 240
Lys Thr Phe Gln Glu Ile Val Ile Asn Gly Gly Asp Gly Ile Ile Ile
                245                 250                 255
Trp Gly Ser Ser Ser Asp Val Asn Ser Leu Ser Lys Cys Thr Arg Leu
            260                 265                 270
Arg Glu Tyr Leu Leu Thr Val Leu Gly Pro Ile Ala Val Asn Val Thr
        275                 280                 285
Glu Ala Val Asn Ala Ile Ser Gln Ile Thr Asp Gly Gln Ile Gln Ala
    290                 295                 300
Thr Thr Thr Ala Thr Thr Glu Ala Thr Thr Thr Ala Ala Pro Ser Ser
305                 310                 315                 320
Thr Val Glu Thr Val Ser Pro Ser Ser Thr Glu Thr Ile Ser Gln Gln
                325                 330                 335
Thr Glu Asn Gly Ala Ala Lys Ala Ala Val Gly Met Gly Ala Gly Ala
            340                 345                 350
Leu Ala Ala Ala Ala Met Leu Leu
        355                 360

<210> 72
<211> 2154
<212> DNA
<213> Saccharomyces cerevisiae


<220>
<223> nucleic acid sequence of glutamine-fructose-6-phosphate
      amidotransferase (GFA1) originating from Saccharomyces cerevisiae

<400> 72

atgtgtggta tctttggtta ctgcaattat ctagtggaaa gatccagagg agaaattatc 60

gacaccttag tggatggttt acaaagatta gaatatagag gctatgattc caccggtatt 120

gctatcgatg gtgacgaagc tgattctact ttcatctata agcaaatcgg taaagtgagt 180

gctttgaaag aggagattac taagcaaaat ccgaacagag acgttacttt tgtctctcat 240

tgtggtattg cgcatactag atgggctact cacggtcgac cagaacaagt taactgtcac 300

cctcaaagat ctgacccaga agaccaattt gtggtcgttc ataatggtat catcacaaat 360

tttagagaac tgaagactct tttaattaac aaaggttata aattcgaaag tgataccgat 420

accgagtgta ttgctaaact atatttgcat ttatacaata caaatttaca aaatgggcat 480

gacttagatt tccacgaatt aaccaagcta gttcttttag aactagaagg ttcatacggg 540

ttattatgta aatcttgtca ctatcctaat gaggttatcg ccactagaaa agggtcccct 600

ttactgattg gtgtcaaatc tgaaaaaaaa ctaaaagtcg acttcgtgga tgtggaattt 660

cccgaagaaa acgctggtca accggaaatt ccattgaaat ctaacaacaa atcatttggc 720

ttgggcccaa agaaagctcg tgaatttgaa gctggttccc aaaatgccaa tttactacca 780

attgccgcca atgaatttaa cttgagacat tctcaatcca gggctttcct atcagaagat 840

ggatctccaa caccggtgga attttttgtt tcttcggatg cggcatctgt tgttaaacat 900

accaagaagg tgctattttt agaagatgac gatttggctc atatttacga tggtgagtta 960

catattcata gatctagaag agaagtaggc gcatcaatga caaggtccat tcaaacttta 1020

gagatggagt tagctcagat catgaagggc ccttacgacc attttatgca aaaggaaatc 1080

tatgagcaac cagaatctac tttcaatact atgagaggta gaatcgacta tgaaaataat 1140

aaagtgatat tgggtggttt aaaggcatgg ttaccagttg tcagaagagc acggagactg 1200

atcatgatcg catgcggtac ttcttatcat tcatgtttgg ctactcgtgc tatcttcgaa 1260

gaattatcag atatcccagt tagtgtggaa ttagcgtctg actttctgga cagaaaatgc 1320

cctgtcttca gagacgatgt atgcgtgttt gtttcacaaa gtggtgaaac tgcggatacc 1380

atgctggctc taaattattg tttagaaaga ggagccttaa ctgtcggaat tgttaacagt 1440

gttggttctt ctatctctcg tgtcacccac tgtggtgttc atattaacgc tggtcctgaa 1500

attggtgttg cctctacaaa agcttatact tcccagtata ttgccttagt gatgtttgct 1560

ctatcgctgt cagatgaccg tgtatcgaaa atagacagaa gaattgaaat cattcaaggc 1620

ttgaagttaa tcccgggcca aattaagcag gtattaaagc tggaaccaag aataaaaaag 1680

ctctgtgcga ctgaattaaa ggatcaaaaa tctctattgt tattgggtag aggttaccaa 1740

tttgctgctg ctctggaagg tgctttgaag atcaaagaaa tttcttatat gcattctgaa 1800

ggtgttttgg caggtgagtt gaagcacggt gtcttggcct tggtggacga aaacttgcca 1860

atcattgctt ttggtaccag agactctcta ttccctaaag tagtttcctc tattgagcaa 1920

gttactgtaa gaaagggcca tccaattatt atttgtaacg aaaatgatga agtgtgggcg 1980

caaaaatcta aatcaatcga cctgcaaacc ttagaagttc cacaaactgt tgattgttta 2040

caaggtctaa ttaatattat tccattacaa ctaatgtcat attggttggc tgttaataaa 2100

gggattgatg ttgattttcc aagaaacttg gctaaatctg ttaccgtcga ataa 2154

<210> 73
<211> 1788
<212> DNA
<213> Chlorella virus

<220>
<223> reencoded nucleic acid sequence of glutamine-fructose-6-phosphate
      amidotransferase (GFA1) originating from Chlorella virus 1
      (PBCV-1)

<400> 73
atgtgcggga tcttcggtgc tgtgtcgaac aataatagca tagaagtttc catcaagggt 60

atacagaagc tagagtaccg cgggtacgat tcgtgtggaa tagcctatac agacggaggc 120

gccattgaac ggatcaggtc aatagatggg atcgacgact taaggaagaa aacaataaca 180

gagtcttccc cggtagctat cgctcattca aggtggagta ccactgggat tccaagtgtt 240

gtgaacgccc acccgcacat ctctcggggc acgtctggat gcgagtcgcg cattgcagtc 300

gtacacaatg gcattattga aaattaccag cagatccgca agtacttgat aaatcttggg 360

tatactttcg attcacagac cgacacggaa gtcattgctc atctaataga ctcgcagtat 420

aacgggaaca tcctgcacac cgtccaaatg gcagttaagc acctcaaagg aagctacgcc 480

atagcagtca tgtgccataa ggagtccggg aagatcgtgg tggcaaaaca gaagagtccg 540

ttggtattgg gtataggaag cgacggagct tattatatcg catcggacgt attggctctt 600

cccactaaca aagtggtcta cataagtgac ggattctctg ctgagttaag tcctggatcg 660

atgactatct atgatctgga tggcaataag gtagaatacg aggtggaaga cgttgaaatg 720

gagcagacgt cgatgtcttt agataacttt gatcactaca tgatcaaaga gatcaatgaa 780

cagccgatat caattctcaa tactattaag aacaaaggat tctatgcaga gatatttggt 840

gatctagcac atgaaatatt tcagaagatc gacaatatat tgattctagc ctgcggaact 900

tcgtatcatg caggtctcgt cgggaagcaa tggatagaga ccatctcacg catcccggtg 960

gatgttcata ttgcaagcga gtatgagccg acaattccac gagccaatac acttgtgata 1020

acgattagtc aaagcggcga gactgcggat acaatcgcag cccttcaacg agcgcaaaac 1080

gcaggaatga tatacacttt gtgtatctgc aacagcccca agtcaactct agtgcgcgag 1140

tcgataatga aatacataac caaatgcggt tcagaagttt ctgttgccag tactaaagca 1200

tttacgtcgc aacttgtggt gttgtatatg cttgcgaacg ttcttgcgaa caaaactgat 1260

gatctgctag gggatcttcc tcaagctatc gagagagtaa tctgtcttac taatgacgaa 1320

atgaagcgtt gggcggatga aatttgtact gccaaaagtg ccatcttctt agggagagga 1380

ctgaacgcac ctgtagcatt tgagggcgcg ctaaagttga aggaaatctc atatatccat 1440

gccgagggtt tcctcggtgg agagttgaag catgggccgc tggctttgtt ggacgataag 1500

atccctgtaa tcgtgactgt tgctgaccac gcttatttgg atcacattaa ggctaacatc 1560

gacgaggtat tggcacgaaa cgttaccgta tacgcgatcg tcgatcagta tgttaatatt 1620

gaaccacagg agcgactgca tgtagtaaaa gtgccgtttg tgagtaaaga gttttctccc 1680

atcatccaca caattccgat gcaattgtta tcgtattatg tcgcgatcaa gctgggcaag 1740

aacgttgaca agccacgtaa cctggcgaaa agtgttacaa cattctaa 1788

<210> 74
<211> 1788
<212> DNA
<213> Chlorella virus

<220>
<223> reencoded nucleic acid sequence of glutamine-fructose-6-phosphate
      amidotransferase (GFA1) originating from Chlorella virus 1
      (PBCV-1)

<400> 74
   atgtgtggca tctttggagc agtgtcaaac aacaactcta tcgaggtgtc aatcaagggt 60
   attcagaagc tagaatatcg tgggtatgat tcgtgcggta ttgcgtatac agatgggggt 120
   gcgattgagc gtatacgttc tattgacggc attgacgatc tgcgtaagaa aacaatcaca 180
   gaatcatcac cagtggccat tgctcactcg cggtggagca ccactggaat tccatcagtg 240
   gtgaacgcac atcctcatat ttctcgcgga accagtgggt gtgagtctcg tatcgcggta 300
   gtccacaacg gtatcattga aaactatcag cagatccgaa aatatctcat caatcttggt 360
   tatacgtttg atagtcaaac ggacacagag gtcattgcgc atttgattga ttctcagtac 420
   aatgggaata tcttgcacac cgtccaaatg gctgtcaagc acctgaaggg ctcttatgcc 480
   attgcagtta tgtgtcataa agagtctggt aaaatagtcg tggcgaaaca gaagtcaccc 540
   ctcgtacttg gaatcggctc agatggtgct tactacatcg cttcggacgt gctggcgctg 600
   ccgacaaata aagttgttta tatttcagac ggtttctccg cagaactatc tccagggagt 660
   atgaccattt acgatcttga tggaaataaa gtagaatatg aagtagagga cgttgaaatg 720
   gaacaaacta gtatgtctct cgataacttt gatcattaca tgattaagga aattaatgag 780
   caaccaatca gtattctaaa cactataaaa aataaagggt tctatgcaga aatattcggt 840
   gatttggctc atgaaatctt ccaaaaaata gacaacatcc tgatactggc ttgtggtaca 900
   agttatcacg ccggtcttgt aggaaaacag tggatagaga ccatctctag aatccccgtg 960
   gatgttcaca tcgcgagtga atacgaacct actattccga gagcgaacac attggtaatc 1020
   actatttcac agtcgggtga aactgcggac acgatagcgg ctttgcaacg ggcccaaaac 1080
   gccgggatga tttatacatt gtgtatttgc aattcaccaa agagcactct tgttcgtgag 1140
   agcattatga agtacatcac gaaatgtggt tctgaggtgt cagtggcatc aacgaaggcg 1200
   tttacttctc agctcgtagt actgtacatg ctggcaaacg tattggcaaa taaaaccgat 1260
   gatttgctgg gagacctccc acaggcaata gaacgggtaa tttgtttgac aaatgacgaa 1320
   atgaaacgat gggccgacga aatttgcact gcgaaatccg cgatcttcct gggaagagga 1380
   ctaaacgcac cagttgcctt tgagggagcg ttgaagctca aagaaatctc ttacattcat 1440
   gcagagggct tcctgggagg tgagttgaaa cacggccccc tcgcactcct tgatgacaag 1500
   attcctgtta tcgtaaccgt agcagatcat gcttatttgg accatatcaa agcaaatatc 1560
   gacgaagtgc ttgcgaggaa cgttacggta tacgccatag tagaccagta tgtgaacatc 1620
   gagccccagg aacgccttca cgtcgtcaag gttccgtttg tatccaaaga attttctccg 1680
   ataattcaca ctatcccgat gcaactgctt tcgtattacg tggcaattaa gcttggaaag 1740
   aacgttgaca aaccaaggaa tcttgcaaaa tccgtgacta ccttttaa 1788

<210> 75
<211> 717
<212> PRT
<213> Saccharomyces cerevisiae

<220>
<223> amino acid sequence of glutamine-fructose-6-phosphate
      amidotransferase (GFA1) originating from Saccharomyces cerevisiae

<400> 75
Met Cys Gly Ile Phe Gly Tyr Cys Asn Tyr Leu Val Glu Arg Ser Arg
1               5                   10                  15
Gly Glu Ile Ile Asp Thr Leu Val Asp Gly Leu Gln Arg Leu Glu Tyr
            20                  25                  30
Arg Gly Tyr Asp Ser Thr Gly Ile Ala Ile Asp Gly Asp Glu Ala Asp
        35                  40                  45
Ser Thr Phe Ile Tyr Lys Gln Ile Gly Lys Val Ser Ala Leu Lys Glu
    50                  55                  60
Glu Ile Thr Lys Gln Asn Pro Asn Arg Asp Val Thr Phe Val Ser His
65                  70                  75                  80
Cys Gly Ile Ala His Thr Arg Trp Ala Thr His Gly Arg Pro Glu Gln
                85                  90                  95
Val Asn Cys His Pro Gln Arg Ser Asp Pro Glu Asp Gln Phe Val Val
            100                 105                 110
Val His Asn Gly Ile Ile Thr Asn Phe Arg Glu Leu Lys Thr Leu Leu
        115                 120                 125
Ile Asn Lys Gly Tyr Lys Phe Glu Ser Asp Thr Asp Thr Glu Cys Ile
    130                 135                 140
Ala Lys Leu Tyr Leu His Leu Tyr Asn Thr Asn Leu Gln Asn Gly His
145                 150                 155                 160
Asp Leu Asp Phe His Glu Leu Thr Lys Leu Val Leu Leu Glu Leu Glu
                165                 170                 175

Gly Ser Tyr Gly Leu Leu Cys Lys Ser Cys His Tyr Pro Asn Glu Val
            180                 185                 190
Ile Ala Thr Arg Lys Gly Ser Pro Leu Leu Ile Gly Val Lys Ser Glu
        195                 200                 205
Lys Lys Leu Lys Val Asp Phe Val Asp Val Glu Phe Pro Glu Glu Asn
    210                 215                 220
Ala Gly Gln Pro Glu Ile Pro Leu Lys Ser Asn Asn Lys Ser Phe Gly
225                 230                 235                 240
Leu Gly Pro Lys Lys Ala Arg Glu Phe Glu Ala Gly Ser Gln Asn Ala
                245                 250                 255
Asn Leu Leu Pro Ile Ala Ala Asn Glu Phe Asn Leu Arg His Ser Gln
            260                 265                 270
Ser Arg Ala Phe Leu Ser Glu Asp Gly Ser Pro Thr Pro Val Glu Phe
        275                 280                 285
Phe Val Ser Ser Asp Ala Ala Ser Val Val Lys His Thr Lys Lys Val
    290                 295                 300
Leu Phe Leu Glu Asp Asp Asp Leu Ala His Ile Tyr Asp Gly Glu Leu
305                 310                 315                 320
His Ile His Arg Ser Arg Arg Glu Val Gly Ala Ser Met Thr Arg Ser
                325                 330                 335
Ile Gln Thr Leu Glu Met Glu Leu Ala Gln Ile Met Lys Gly Pro Tyr
            340                 345                 350
Asp His Phe Met Gln Lys Glu Ile Tyr Glu Gln Pro Glu Ser Thr Phe
        355                 360                 365
Asn Thr Met Arg Gly Arg Ile Asp Tyr Glu Asn Asn Lys Val Ile Leu
    370                 375                 380
Gly Gly Leu Lys Ala Trp Leu Pro Val Val Arg Arg Ala Arg Arg Leu
385                 390                 395                 400
Ile Met Ile Ala Cys Gly Thr Ser Tyr His Ser Cys Leu Ala Thr Arg
                405                 410                 415
Ala Ile Phe Glu Glu Leu Ser Asp Ile Pro Val Ser Val Glu Leu Ala
            420                 425                 430
Ser Asp Phe Leu Asp Arg Lys Cys Pro Val Phe Arg Asp Asp Val Cys
        435                 440                 445
Val Phe Val Ser Gln Ser Gly Glu Thr Ala Asp Thr Met Leu Ala Leu
    450                 455                 460
Asn Tyr Cys Leu Glu Arg Gly Ala Leu Thr Val Gly Ile Val Asn Ser
465                 470                 475                 480
Val Gly Ser Ser Ile Ser Arg Val Thr His Cys Gly Val His Ile Asn
                485                 490                 495
Ala Gly Pro Glu Ile Gly Val Ala Ser Thr Lys Ala Tyr Thr Ser Gln
            500                 505                 510
Tyr Ile Ala Leu Val Met Phe Ala Leu Ser Leu Ser Asp Asp Arg Val
        515                 520                 525
Ser Lys Ile Asp Arg Arg Ile Glu Ile Ile Gln Gly Leu Lys Leu Ile
    530                 535                 540
Pro Gly Gln Ile Lys Gln Val Leu Lys Leu Glu Pro Arg Ile Lys Lys
545                 550                 555                 560
Leu Cys Ala Thr Glu Leu Lys Asp Gln Lys Ser Leu Leu Leu Leu Gly
                565                 570                 575
Arg Gly Tyr Gln Phe Ala Ala Ala Leu Glu Gly Ala Leu Lys Ile Lys
            580                 585                 590
Glu Ile Ser Tyr Met His Ser Glu Gly Val Leu Ala Gly Glu Leu Lys
        595                 600                 605
His Gly Val Leu Ala Leu Val Asp Glu Asn Leu Pro Ile Ile Ala Phe
    610                 615                 620
Gly Thr Arg Asp Ser Leu Phe Pro Lys Val Val Ser Ser Ile Glu Gln
625                 630                 635                 640
Val Thr Ala Arg Lys Gly His Pro Ile Ile Ile Cys Asn Glu Asn Asp
                645                 650                 655
Glu Val Trp Ala Gln Lys Ser Lys Ser Ile Asp Leu Gln Thr Leu Glu
            660                 665                 670
Val Pro Gln Thr Val Asp Cys Leu Gln Gly Leu Ile Asn Ile Ile Pro

675                 680                 685
Leu Gln Leu Met Ser Tyr Trp Leu Ala Val Asn Lys Gly Ile Asp Val
    690                 695                 700
Asp Phe Pro Arg Asn Leu Ala Lys Ser Val Thr Val Glu
705                 710                 715

<210> 76
<211> 595
<212> PRT
<213> Chlorella virus

<220>
<223> amino acid sequence of glutamine-fructose-6-phosphate
      amidotransferase (GFA1) originating from Chlorella virus 1
      (PBCV-1)

<400> 76
Met Cys Gly Ile Phe Gly Ala Val Ser Asn Asn Asn Ser Ile Glu Val
1               5                   10                  15
Ser Ile Lys Gly Ile Gln Lys Leu Glu Tyr Arg Gly Tyr Asp Ser Cys
            20                  25                  30
Gly Ile Ala Tyr Thr Asp Gly Gly Ala Ile Glu Arg Ile Arg Ser Ile
        35                  40                  45
Asp Gly Ile Asp Asp Leu Arg Lys Lys Thr Ile Thr Glu Ser Ser Pro
    50                  55                  60
Val Ala Ile Ala His Ser Arg Trp Ser Thr Thr Gly Ile Pro Ser Val
65                  70                  75                  80
Val Asn Ala His Pro His Ile Ser Arg Gly Thr Ser Gly Cys Glu Ser
                85                  90                  95
Arg Ile Ala Val Val His Asn Gly Ile Ile Glu Asn Tyr Gln Gln Ile
            100                 105                 110
Arg Lys Tyr Leu Ile Asn Leu Gly Tyr Thr Phe Asp Ser Gln Thr Asp
        115                 120                 125
Thr Glu Val Ile Ala His Leu Ile Asp Ser Gln Tyr Asn Gly Asn Ile
    130                 135                 140
Leu His Thr Val Gln Met Ala Val Lys His Leu Lys Gly Ser Tyr Ala
145                 150                 155                 160
Ile Ala Val Met Cys His Lys Glu Ser Gly Lys Ile Val Val Ala Lys
                165                 170                 175
Gln Lys Ser Pro Leu Val Leu Gly Ile Gly Ser Asp Gly Ala Tyr Tyr
            180                 185                 190
Ile Ala Ser Asp Val Leu Ala Leu Pro Thr Asn Lys Val Val Tyr Ile
        195                 200                 205
Ser Asp Gly Phe Ser Ala Glu Leu Ser Pro Gly Ser Met Thr Ile Tyr
    210                 215                 220
Asp Leu Asp Gly Asn Lys Val Glu Tyr Glu Val Glu Asp Val Glu Met
225                 230                 235                 240
Glu Gln Thr Ser Met Ser Leu Asp Asn Phe Asp His Tyr Met Ile Lys
                245                 250                 255
Glu Ile Asn Glu Gln Pro Ile Ser Ile Leu Asn Thr Ile Lys Asn Lys
            260                 265                 270
Gly Phe Tyr Ala Glu Ile Phe Gly Asp Leu Ala His Glu Ile Phe Gln
        275                 280                 285
Lys Ile Asp Asn Ile Leu Ile Leu Ala Cys Gly Thr Ser Tyr His Ala
    290                 295                 300
Gly Leu Val Gly Lys Gln Trp Ile Glu Thr Ile Ser Arg Ile Pro Val
305                 310                 315                 320
Asp Val His Ile Ala Ser Glu Tyr Glu Pro Thr Ile Pro Arg Ala Asn
                325                 330                 335
Thr Leu Val Ile Thr Ile Ser Gln Ser Gly Glu Thr Ala Asp Thr Ile
            340                 345                 350
Ala Ala Leu Gln Arg Ala Gln Asn Ala Gly Met Ile Tyr Thr Leu Cys

355                 360                 365
Ile Cys Asn Ser Pro Lys Ser Thr Leu Val Arg Glu Ser Ile Met Lys
    370                 375                 380
Tyr Ile Thr Lys Cys Gly Ser Glu Val Ser Val Ala Ser Thr Lys Ala
385                 390                 395                 400
Phe Thr Ser Gln Leu Val Val Leu Tyr Met Leu Ala Asn Val Leu Ala
                405                 410                 415
Asn Lys Thr Asp Asp Leu Leu Gly Asp Leu Pro Gln Ala Ile Glu Arg
            420                 425                 430
Val Ile Cys Leu Thr Asn Asp Glu Met Lys Arg Trp Ala Asp Glu Ile
        435                 440                 445
Cys Thr Ala Lys Ser Ala Ile Phe Leu Gly Arg Gly Leu Asn Ala Pro
    450                 455                 460
Val Ala Phe Glu Gly Ala Leu Lys Leu Lys Glu Ile Ser Tyr Ile His
465                 470                 475                 480
Ala Glu Gly Phe Leu Gly Gly Glu Leu Lys His Gly Pro Leu Ala Leu
                485                 490                 495
Leu Asp Asp Lys Ile Pro Val Ile Val Thr Val Ala Asp His Ala Tyr
            500                 505                 510
Leu Asp His Ile Lys Ala Asn Ile Asp Glu Val Leu Ala Arg Asn Val
        515                 520                 525
Thr Val Tyr Ala Ile Val Asp Gln Tyr Val Asn Ile Glu Pro Gln Glu
    530                 535                 540
Arg Leu His Val Val Lys Val Pro Phe Val Ser Lys Glu Phe Ser Pro
545                 550                 555                 560
Ile Ile His Thr Ile Pro Met Gln Leu Leu Ser Tyr Tyr Val Ala Ile
                565                 570                 575
Lys Leu Gly Lys Asn Val Asp Lys Pro Arg Asn Leu Ala Lys Ser Val
            580                 585                 590
Thr Thr Phe
        595

<210> 77
<211> 1434
<212> DNA
<213> Saccharomyces cerevisiae

<220>
<223> nucleic acid sequence of UDP-N-acetylglucosamine
      pyrophosphorylase (QRI1) originating from Saccharomyces
      cerevisiae

<400> 77
atgactgaca caaaacagct attcattgaa gccggacaaa gtcaactttt ccacaattgg      60

gaaagcttgt ctcgcaaaga ccaagaagaa ttgctttcaa acctggagca aatatcttcc     120

aagaggtccc ctgcaaaact actggaagac tgtcaaaatg ctattaaatt ctcactagct     180

aactcttcta aggatactgg cgtcgaaatt tcaccattgc cccctacttc gtacgagtcg     240

cttattggca acagtaagaa agaaaatgaa tactggcgtt taggccttga agctattggc     300

aagggtgaag tcgcagtgat tttaatggct ggcggacaag gtacgcggtt aggatcctct     360

caaccaaagg gctgttacga cattggattg ccttctaaga aatctctttt tcaaattcaa     420

gctgaaaagt tgatcaggtt gcaagatatg gtaaaggaca aaaaggtaga aattccttgg     480

tatattatga catcaggccc cactagagcg gctactgagg catactttca agaacacaat     540

tattttggct tgaataaaga acaaattacg ttcttcaacc agggaaccct gcctgccttt     600

gatttaaccg ggaagcattt cctaatgaaa gacccagtaa acctatctca atcaccagat 660

ggaaatggtg gactctaccg tgccatcaag gaaaacaagt tgaacgaaga ctttgatagg 720

agaggaatca agcatgttta catgtactgt gtcgataatg tcctatctaa aatcgcagac 780

cctgtattta ttggttttgc catcaagcat ggcttcgaac tggccaccaa agccgttaga 840

aagagagatg cgcatgaatc agttgggtta attgctacta aaaacgagaa accatgtgtc 900

atagaatatt ctgaaatttc caatgaattg gctgaagcaa aggataaaga tggcttatta 960

aaactacgcg caggcaacat tgtaaatcat tattacctag tggatttact aaaacgtgat 1020

ttggatcagt ggtgtgagaa tatgccatat cacattgcga agaagaaaat tccagcttat 1080

gatagtgtta ccggcaagta cactaagcct accgaaccaa acggtataaa attagagcaa 1140

ttcatatttg atgtctttga cactgtacca ctgaacaagt ttgggtgctt agaagtagat 1200

agatgcaaag aattttcacc tttaaaaaac ggtcctggtt ctaagaacga taatcctgag 1260

accagcagac tagcatattt gaaactagga acctcgtggt tggaagatgc aggcgctatt 1320

gtaaaagatg gggtactagt cgaagtttcc agcaaattga gttatgcagg tgaaaatcta 1380

tcccagttca aaggtaaagt ctttgacaga agtggtatag tattagaaaa ataa 1434

<210> 78
<211> 477
<212> PRT
<213> Saccharomyces cerevisiae

<220>
<223> amino acid sequence of UDP-N-acetylglucosamine pyrophosphorylase
(QRI1) originating from Saccharomyces cerevisiae

<400> 78
Met Thr Asp Thr Lys Gln Leu Phe Ile Glu Ala Gly Gln Ser Gln Leu
1               5                   10                  15
Phe His Asn Trp Glu Ser Leu Ser Arg Lys Asp Gln Glu Glu Leu Leu
            20                  25                  30
Ser Asn Leu Glu Gln Ile Ser Ser Lys Arg Ser Pro Ala Lys Leu Leu
        35                  40                  45
Glu Asp Cys Gln Asn Ala Ile Lys Phe Ser Leu Ala Asn Ser Ser Lys
    50                  55                  60
Asp Thr Gly Val Glu Ile Ser Pro Leu Pro Pro Thr Ser Tyr Glu Ser
65                  70                  75                  80
Leu Ile Gly Asn Ser Lys Lys Glu Asn Glu Tyr Trp Arg Leu Gly Leu
                85                  90                  95
Glu Ala Ile Gly Lys Gly Glu Val Ala Val Ile Leu Met Ala Gly Gly
            100                 105                 110
Gln Gly Thr Arg Leu Gly Ser Ser Gln Pro Lys Gly Cys Tyr Asp Ile
        115                 120                 125
Gly Leu Pro Ser Lys Lys Ser Leu Phe Gln Ile Gln Ala Glu Lys Leu
    130                 135                 140
Ile Arg Leu Gln Asp Met Val Lys Asp Lys Lys Val Glu Ile Pro Trp
145                 150                 155                 160
Tyr Ile Met Thr Ser Gly Pro Thr Arg Ala Ala Thr Glu Ala Tyr Phe
                165                 170                 175

Gln Glu His Asn Tyr Phe Gly Leu Asn Lys Glu Gln Ile Thr Phe Phe
            180                 185                 190
Asn Gln Gly Thr Leu Pro Ala Phe Asp Leu Thr Gly Lys His Phe Leu
        195                 200                 205
Met Lys Asp Pro Val Asn Leu Ser Gln Ser Pro Asp Gly Asn Gly Gly
    210                 215                 220
Leu Tyr Arg Ala Ile Lys Glu Asn Lys Leu Asn Glu Asp Phe Asp Arg
225                 230                 235                 240
Arg Gly Ile Lys His Val Tyr Met Tyr Cys Val Asp Asn Val Leu Ser
                245                 250                 255
Lys Ile Ala Asp Pro Val Phe Ile Gly Phe Ala Ile Lys His Gly Phe
            260                 265                 270
Glu Leu Ala Thr Lys Ala Val Arg Lys Arg Asp Ala His Glu Ser Val
        275                 280                 285
Gly Leu Ile Ala Thr Lys Asn Glu Lys Pro Cys Val Ile Glu Tyr Ser
    290                 295                 300
Glu Ile Ser Asn Glu Leu Ala Glu Ala Lys Asp Lys Asp Gly Leu Leu
305                 310                 315                 320
Lys Leu Arg Ala Gly Asn Ile Val Asn His Tyr Tyr Leu Val Asp Leu
                325                 330                 335
Leu Lys Arg Asp Leu Asp Gln Trp Cys Glu Asn Met Pro Tyr His Ile
            340                 345                 350
Ala Lys Lys Lys Ile Pro Ala Tyr Asp Ser Val Thr Gly Lys Tyr Thr
        355                 360                 365
Lys Pro Thr Glu Pro Asn Gly Ile Lys Leu Glu Gln Phe Ile Phe Asp
    370                 375                 380
Val Phe Asp Thr Val Pro Leu Asn Lys Phe Gly Cys Leu Glu Val Asp
385                 390                 395                 400
Arg Cys Lys Glu Phe Ser Pro Leu Lys Asn Gly Pro Gly Ser Lys Asn
                405                 410                 415
Asp Asn Pro Glu Thr Ser Arg Leu Ala Tyr Leu Lys Leu Gly Thr Ser
            420                 425                 430
Trp Leu Glu Asp Ala Gly Ala Ile Val Lys Asp Gly Val Leu Val Glu
        435                 440                 445
Val Ser Ser Lys Leu Ser Tyr Ala Gly Glu Asn Leu Ser Gln Phe Lys
    450                 455                 460
Gly Lys Val Phe Asp Arg Ser Gly Ile Val Leu Glu Lys
465                 470                 475

<210> 79
<211> 1713
<212> DNA
<213> Saccharomyces cerevisiae

<220>
<223> nucleic acid sequence of Phosphoglucomutase-1 (PGM1) originating
      from Saccharomyces cerevisiae

<400> 79
atgtcacttc taatagattc tgtaccaaca gttgcttata aggaccaaaa accgggtact      60

tcaggtttac gtaagaagac caaggttttc atggatgagc ctcattatac tgagaacttc     120

attcaagcaa caatgcaatc tatccctaat ggctcagagg gaaccacttt agttgttgga     180

ggagatggtc gtttctacaa cgatgttatc atgaacaaga ttgccgcagt aggtgctgca     240

aacggtgtca gaaagttagt cattggtcaa ggcggtttac tttcaacacc agctgcttct     300

catataatta gaacatacga ggaaaagtgt accggtggtg gtatcatatt aactgcctca     360

cacaacccag gcggtccaga gaatgattta ggtatcaagt ataatttacc taatggtggg     420

ccagctccag agagtgtcac taacgctatc tgggaagcgt ctaaaaaatt aactcactat 480

aaaattataa agaacttccc caagttgaat ttgaacaagc ttggtaaaaa ccaaaaatat 540

ggcccattgt tagtggacat aattgatcct gccaaagcat acgttcaatt tctgaaggaa 600

atttttgatt ttgacttaat taaaagcttc ttagcgaaac agcgcaaaga caaagggtgg 660

aagttgttgt ttgactcctt aaatggtatt acaggaccat atggtaaggc tatatttgtt 720

gatgaatttg gtttaccggc agaggaagtt cttcaaaatt ggcacccttt acctgatttc 780

ggcggtttac atcccgatcc gaatctaacc tatgcacgaa ctcttgttga cagggttgac 840

cgcgaaaaaa ttgcctttgg agcagcctcc gatggtgatg gtgataggaa tatgatttac 900

ggttatggcc ctgctttcgt ttcgccaggt gattctgttg ccattattgc cgaatatgca 960

cccgaaattc catacttcgc caaacaaggt atttatggct tggcacgttc atttcctaca 1020

tcctcagcca ttgatcgtgt tgcagcaaaa aagggattaa gatgttacga agttccaacc 1080

ggctggaaat tcttctgtgc cttatttgat gctaaaaagc tatcaatctg tggtgaagaa 1140

tccttcggta caggttccaa tcatatcaga gaaaaggacg gtctatgggc cattattgct 1200

tggttaaata tcttggctat ctaccatagg cgtaaccctg aaaaggaagc ttcgatcaaa 1260

actattcagg acgaattttg gaacgagtat ggccgtactt tcttcacaag atacgattac 1320

gaacatatcg aatgcgagca ggccgaaaaa gttgtagctc ttttgagtga atttgtatca 1380

aggccaaacg tttgtggctc ccacttccca gctgatgagt ctttaaccgt tatcgattgt 1440

ggtgattttt cgtatagaga tctagatggc tccatctctg aaaatcaagg ccttttcgta 1500

aagttttcga atgggactaa atttgttttg aggttatccg gcacaggcag ttctggtgca 1560

acaataagat tatacgtaga aaagtatact gataaaaagg agaactatgg ccaaacagct 1620

gacgtcttct tgaaacccgt catcaactcc attgtaaaat tcttaagatt taaagaaatt 1680

ttaggaacag acgaaccaac agtccgcaca tag 1713

<210> 80
<211> 570
<212> PRT
<213> Saccharomyces cerevisiae

<220>
<223> amino acid sequence Phosphoglucomutase-1 (PGM1) originating from
Saccharomyces cerevisiae

<400> 80
Met Ser Leu Leu Ile Asp Ser Val Pro Thr Val Ala Tyr Lys Asp Gln
1               5                   10                  15
Lys Pro Gly Thr Ser Gly Leu Arg Lys Lys Thr Lys Val Phe Met Asp
            20                  25                  30
Glu Pro His Tyr Thr Glu Asn Phe Ile Gln Ala Thr Met Gln Ser Ile
        35                  40                  45

Pro Asn Gly Ser Glu Gly Thr Thr Leu Val Val Gly Gly Asp Gly Arg
    50                  55                  60
Phe Tyr Asn Asp Val Ile Met Asn Lys Ile Ala Ala Val Gly Ala Ala
65                  70                  75                  80
Asn Gly Val Arg Lys Leu Val Ile Gly Gln Gly Gly Leu Leu Ser Thr
                85                  90                  95
Pro Ala Ala Ser His Ile Ile Arg Thr Tyr Glu Glu Lys Cys Thr Gly
            100                 105                 110
Gly Gly Ile Ile Leu Thr Ala Ser His Asn Pro Gly Gly Pro Glu Asn
        115                 120                 125
Asp Leu Gly Ile Lys Tyr Asn Leu Pro Asn Gly Gly Pro Ala Pro Glu
    130                 135                 140
Ser Val Thr Asn Ala Ile Trp Glu Ala Ser Lys Lys Leu Thr His Tyr
145                 150                 155                 160
Lys Ile Ile Lys Asn Phe Pro Lys Leu Asn Leu Asn Lys Leu Gly Lys
                165                 170                 175
Asn Gln Lys Tyr Gly Pro Leu Leu Val Asp Ile Ile Asp Pro Ala Lys
            180                 185                 190
Ala Tyr Val Gln Phe Leu Lys Glu Ile Phe Asp Phe Asp Leu Ile Lys
        195                 200                 205
Ser Phe Leu Ala Lys Gln Arg Lys Asp Lys Gly Trp Lys Leu Leu Phe
    210                 215                 220
Asp Ser Leu Asn Gly Ile Thr Gly Pro Tyr Gly Lys Ala Ile Phe Val
225                 230                 235                 240
Asp Glu Phe Gly Leu Pro Ala Glu Glu Val Leu Gln Asn Trp His Pro
                245                 250                 255
Leu Pro Asp Phe Gly Gly Leu His Pro Asp Pro Asn Leu Thr Tyr Ala
            260                 265                 270
Arg Thr Leu Val Asp Arg Val Asp Arg Glu Lys Ile Ala Phe Gly Ala
        275                 280                 285
Ala Ser Asp Gly Asp Gly Asp Arg Asn Met Ile Tyr Gly Tyr Gly Pro
    290                 295                 300
Ala Phe Val Ser Pro Gly Asp Ser Val Ala Ile Ile Ala Glu Tyr Ala
305                 310                 315                 320
Pro Glu Ile Pro Tyr Phe Ala Lys Gln Gly Ile Tyr Gly Leu Ala Arg
                325                 330                 335
Ser Phe Pro Thr Ser Ser Ala Ile Asp Arg Val Ala Ala Lys Lys Gly
            340                 345                 350
Leu Arg Cys Tyr Glu Val Pro Thr Gly Trp Lys Phe Phe Cys Ala Leu
        355                 360                 365
Phe Asp Ala Lys Lys Leu Ser Ile Cys Gly Glu Glu Ser Phe Gly Thr
    370                 375                 380
Gly Ser Asn His Ile Arg Glu Lys Asp Gly Leu Trp Ala Ile Ile Ala
385                 390                 395                 400
Trp Leu Asn Ile Leu Ala Ile Tyr His Arg Arg Asn Pro Glu Lys Glu
                405                 410                 415
Ala Ser Ile Lys Thr Ile Gln Asp Glu Phe Trp Asn Glu Tyr Gly Arg
            420                 425                 430
Thr Phe Phe Thr Arg Tyr Asp Tyr Glu His Ile Glu Cys Glu Gln Ala
        435                 440                 445
Glu Lys Val Val Ala Leu Leu Ser Glu Phe Val Ser Arg Pro Asn Val
    450                 455                 460
Cys Gly Ser His Phe Pro Ala Asp Glu Ser Leu Thr Val Ile Asp Cys
465                 470                 475                 480
Gly Asp Phe Ser Tyr Arg Asp Leu Asp Gly Ser Ile Ser Glu Asn Gln
                485                 490                 495
Gly Leu Phe Val Lys Phe Ser Asn Gly Thr Lys Phe Val Leu Arg Leu
            500                 505                 510
Ser Gly Thr Gly Ser Ser Gly Ala Thr Ile Arg Leu Tyr Val Glu Lys
        515                 520                 525
Tyr Thr Asp Lys Lys Glu Asn Tyr Gly Gln Thr Ala Asp Val Phe Leu
    530                 535                 540
Lys Pro Val Ile Asn Ser Ile Val Lys Phe Leu Arg Phe Lys Glu Ile

545                 550                 555                 560
Leu Gly Thr Asp Glu Pro Thr Val Arg Thr
                565                 570

<210> 81
<211> 1500
<212> DNA
<213> Saccharomyces cerevisiae

<220>
<223> nucleic acid sequence of UTP-glucose-1-phosphate
      uridylyltransferase (UGP1) originating from Saccharomyces
      cerevisiae

<400> 81
atgtccacta agaagcacac caaaacacat tccacttatg cattcgagag caacacaaac      60

agcgttgctg cctcacaaat gagaaacgcc ttaaacaagt tggcggactc tagtaaactt     120

gacgatgctg ctcgcgctaa gtttgagaac gaactggatt cgtttttcac gcttttcagg     180

agatatttgg tagagaagtc ttctagaacc accttggaat gggacaagat caagtctccc     240

aacccggatg aagtggttaa gtatgaaatt atttctcagc agcccgagaa tgtctcaaac     300

ctttccaaat tggctgtttt gaagttgaac ggtgggctgg gtacctccat gggctgcgtt     360

ggccctaaat ctgttattga agtgagagag ggaaacacct tttggatttt gtctgttcgt     420

caaattgaat acttgaacag acagtacgat agcgacgtgc cattgttatt gatgaattct     480

ttcaacactg acaaggatac ggaacacttg attaagaagt attccgctaa cagaatcaga     540

atcagatctt tcaatcaatc caggttccca agagtctaca aggattcttt attgcctgtc     600

cccaccgaat acgattctcc actggatgct tggtatccac caggtcacgg tgatttgttt     660

gaatctttac acgtatctgg tgaactggat gccttaattg cccaaggaag agaaatatta     720

tttgtttcta acggtgacaa cttgggtgct accgtcgact taaaaatttt aaaccacatg     780

atcgagactg gtgccgaata tataatggaa ttgactgata agaccagagc cgatgttaaa     840

ggtggtactt tgatttctta cgatggtcaa gtccgtttat tggaagtcgc ccaagttcca     900

aaagaacaca ttgacgaatt caaaaatatc agaaagttta ccaacttcaa cacgaataac     960

ttatggatca atctgaaagc agtaaagagg ttgatcgaat cgagcaattt ggagatggaa    1020

atcattccaa accaaaaaac tataacaaga gacggtcatg aaattaatgt cttacaatta    1080

gaaaccgctt gtggtgctgc tatcaggcat tttgatggtg ctcacggtgt tgtcgttcca    1140

agatcaagat tcttgcctgt caagacctgt tccgatttgt tgctggttaa atcagatcta    1200

ttccgtctgg aacacggttc tttgaagtta gacccatccc gttttggtcc aaacccatta    1260

atcaagttgg gctcgcattt caaaaaggtt tctggtttta acgcaagaat ccctcacatc    1320

ccaaaaatcg tcgagctaga tcatttgacc atcactggta acgtcttttt aggtaaagat    1380

gtcactttga ggggtactgt catcatcgtt tgctccgacg gtcataaaat cgatattcca    1440

aacggctcca tattggaaaa tgttgtcgtt actggtaatt tgcaaatctt ggaacattga    1500

<210> 82
<211> 499
<212> PRT
<213> Saccharomyces cerevisiae

<220>
<223> amino acid sequence of UTP--glucose-1-phosphate
      uridylyltransferase (UGP1) originating from Saccharomyces
      cerevisiae

<400> 82
Met Ser Thr Lys Lys His Thr Lys Thr His Ser Thr Tyr Ala Phe Glu
1               5                   10                  15
Ser Asn Thr Asn Ser Val Ala Ala Ser Gln Met Arg Asn Ala Leu Asn
            20                  25                  30
Lys Leu Ala Asp Ser Ser Lys Leu Asp Asp Ala Ala Arg Ala Lys Phe
        35                  40                  45
Glu Asn Glu Leu Asp Ser Phe Phe Thr Leu Phe Arg Arg Tyr Leu Val
    50                  55                  60
Glu Lys Ser Ser Arg Thr Thr Leu Glu Trp Asp Lys Ile Lys Ser Pro
65                  70                  75                  80
Asn Pro Asp Glu Val Val Lys Tyr Glu Ile Ile Ser Gln Gln Pro Glu
                85                  90                  95
Asn Val Ser Asn Leu Ser Lys Leu Ala Val Leu Lys Leu Asn Gly Gly
            100                 105                 110
Leu Gly Thr Ser Met Gly Cys Val Gly Pro Lys Ser Val Ile Glu Val
        115                 120                 125
Arg Glu Gly Asn Thr Phe Leu Asp Leu Ser Val Arg Gln Ile Glu Tyr
    130                 135                 140
Leu Asn Arg Gln Tyr Asp Ser Asp Val Pro Leu Leu Leu Met Asn Ser
145                 150                 155                 160
Phe Asn Thr Asp Lys Asp Thr Glu His Leu Ile Lys Lys Tyr Ser Ala
                165                 170                 175
Asn Arg Ile Arg Ile Arg Ser Phe Asn Gln Ser Arg Phe Pro Arg Val
            180                 185                 190
Tyr Lys Asp Ser Leu Leu Pro Val Pro Thr Glu Tyr Asp Ser Pro Leu
        195                 200                 205
Asp Ala Trp Tyr Pro Pro Gly His Gly Asp Leu Phe Glu Ser Leu His
    210                 215                 220
Val Ser Gly Glu Leu Asp Ala Leu Ile Ala Gln Gly Arg Glu Ile Leu
225                 230                 235                 240
Phe Val Ser Asn Gly Asp Asn Leu Gly Ala Thr Val Asp Leu Lys Ile
                245                 250                 255
Leu Asn His Met Ile Glu Thr Gly Ala Glu Tyr Ile Met Glu Leu Thr
            260                 265                 270
Asp Lys Thr Arg Ala Asp Val Lys Gly Gly Thr Leu Ile Ser Tyr Asp
        275                 280                 285
Gly Gln Val Arg Leu Leu Glu Val Ala Gln Val Pro Lys Glu His Ile
    290                 295                 300
Asp Glu Phe Lys Asn Ile Arg Lys Phe Thr Asn Phe Asn Thr Asn Asn
305                 310                 315                 320
Leu Trp Ile Asn Leu Lys Ala Val Lys Arg Leu Ile Glu Ser Ser Asn
                325                 330                 335
Leu Glu Met Glu Ile Ile Pro Asn Gln Lys Thr Ile Thr Arg Asp Gly
            340                 345                 350
His Glu Ile Asn Val Leu Gln Leu Glu Thr Ala Cys Gly Ala Ala Ile
        355                 360                 365
Arg His Phe Asp Gly Ala His Gly Val Val Val Pro Arg Ser Arg Phe

370                 375                 380
Leu Pro Val Lys Thr Cys Ser Asp Leu Leu Leu Val Lys Ser Asp Leu
385                 390                 395                 400
Phe Arg Leu Glu His Gly Ser Leu Lys Leu Asp Pro Ser Arg Phe Gly
                405                 410                 415
Pro Asn Pro Leu Ile Lys Leu Gly Ser His Phe Lys Lys Val Ser Gly
            420                 425                 430
Phe Asn Ala Arg Ile Pro His Ile Pro Lys Ile Val Glu Leu Asp His
        435                 440                 445
Leu Thr Ile Thr Gly Asn Val Phe Leu Gly Lys Asp Val Thr Leu Arg
    450                 455                 460
Gly Thr Val Ile Ile Val Cys Ser Asp Gly His Lys Ile Asp Ile Pro
465                 470                 475                 480
Asn Gly Ser Ile Leu Glu Asn Val Val Val Thr Gly Asn Leu Gln Ile
                485                 490                 495
Leu Glu His

<210> 83
<211> 480
<212> DNA
<213> Saccharomyces cerevisiae

<220>
<223> nucleic acid sequence of Glucosamine 6-phosphate
      N-acetyltransferase (GNA1) originating from Saccharomyces
      cerevisiae

<400> 83
atgagcttac ccgatggatt ttatataagg cgaatggaag agggggattt ggaacaggtc      60

actgagacgc taaaggtttt gaccaccgtg ggcactatta cccccgaatc cttcagcaaa     120

ctcataaaat actggaatga agccacagta tggaatgata acgaagataa aaaaataatg     180

caatataacc ccatggtgat tgtggacaag cgcaccgaga cggttgccgc tacggggaat     240

atcatcatcg aaagaaagat cattcatgaa ctggggctat gtggccacat cgaggacatt     300

gcagtaaact ccaagtatca gggccaaggt ttgggcaagc tcttgattga tcaattggta     360

actatcggct ttgactacgg ttgttataag attattttag attgcgatga gaaaaatgtc     420

aaattctatg aaaaatgtgg gtttagcaac gcaggcgtgg aaatgcaaat tagaaaatag     480

<210> 84
<211> 159
<212> PRT
<213> Saccharomyces cerevisiae

<220>
<223> amino acid sequence of Glucosamine 6-phosphate
      N-acetyltransferase (GNA1) originating from Saccharomyces
      cerevisiae

<400> 84
Met Ser Leu Pro Asp Gly Phe Tyr Ile Arg Arg Met Glu Glu Gly Asp
1               5                   10                  15
Leu Glu Gln Val Thr Glu Thr Leu Lys Val Leu Thr Thr Val Gly Thr
            20                  25                  30

Ile Thr Pro Glu Ser Phe Ser Lys Leu Ile Lys Tyr Trp Asn Glu Ala
        35                  40                  45
Thr Val Trp Asn Asp Asn Glu Asp Lys Lys Ile Met Gln Tyr Asn Pro
    50                  55                  60
Met Val Ile Val Asp Lys Arg Thr Glu Thr Val Ala Ala Thr Gly Asn
65                  70                  75                  80
Ile Ile Ile Glu Arg Lys Ile Ile His Glu Leu Gly Leu Cys Gly His
                85                  90                  95
Ile Glu Asp Ile Ala Val Asn Ser Lys Tyr Gln Gly Gln Gly Leu Gly
            100                 105                 110
Lys Leu Leu Ile Asp Gln Leu Val Thr Ile Gly Phe Asp Tyr Gly Cys
        115                 120                 125
Tyr Lys Ile Ile Leu Asp Cys Asp Glu Lys Asn Val Lys Phe Tyr Glu
    130                 135                 140
Lys Cys Gly Phe Ser Asn Ala Gly Val Glu Met Gln Ile Arg Lys
145                 150                 155

<210> 85
<211> 1674
<212> DNA
<213> Saccharomyces cerevisiae

<220>
<223> nucleic acid sequence of phosphoacetylglucosamine mutase (PCM1)
      originating from Saccharomyces cerevisiae

<400> 85
atgaaggttg attacgagca attgtgcaaa ctctacgatg acacgtgccg cacaaagaat      60

gtgcagttca gttacggtac ggccggattc agaacgctgg ccaagaattt ggatacggtg     120

atgttcagta ctggtatact ggcggttctc aggtcgctga agcttcaggg tcagtatgtg     180

ggggtgatga tcacggcgtc gcacaaccca taccaggaca acggggtcaa gatcgtggaa     240

ccagacggat cgatgctttt ggccacatgg gagccatatg ccatgcagtt ggccaatgcg     300

gcctcttttg ccactaattt tgaagaattt cgtgttgagt tggccaagct gattgaacac     360

gaaaagattg atttgaatac aaccgtcgtg cctcacatcg tggttgggag agactctagg     420

gaaagtagtc catacttgct gcgctgcttg acttcctcca tggccagcgt cttccacgcg     480

caagttttgg acctaggctg tgtcactacg cctcaattgc attacattac tgatttgtcc     540

aacaggcgga aactggaagg agacacagcg ccagttgcca cagaacagga ctactattcg     600

ttctttatag gagccttcaa cgagctcttc gccacgtatc agctggagaa gaggctgtct     660

gtcccaaaat tgttcataga cacagccaat ggtatcggtg gtccacagtt gaaaaaacta     720

ctggcctccg aagattggga cgtgccagcg gagcaagttg aggtaatcaa cgacaggtcc     780

gatgttccag aactgttgaa ttttgaatgc ggtgcggatt atgtgaagac taaccagaga     840

ttacccaagg gtctttctcc atcctcgttt gattcgctat attgctcctt tgatggtgac     900

gcagacaggg ttgtgttcta ctatgtcgac tcaggatcaa aatttcattt gttggatggt     960

gacaaaattt ccactttgtt tgcaaagttc ttgtctaaac aactagaatt ggcacaccta    1020

gaacattctt tgaagattgg tgttgtgcaa actgcctatg caaacggcag ttccaccgct    1080

tacataaaaa atacgttgca ctgtcccgtg tcttgcacta agacaggtgt taaacacttg 1140

catcatgaag ctgccactca gtacgatatt ggcatttatt tcgaagcaaa tggacatggt 1200

acgattatat tcagcgaaaa atttcatcga actatcaaat ctgaattatc caagtccaag 1260

ttaaatggtg atacgttagc tttgagaact ttgaagtgtt tctctgaatt gattaatcag 1320

accgtgggag atgctatttc agacatgctt gctgtccttg ctactttggc gattttgaaa 1380

atgtcgccaa tggattggga tgaagagtat actgatttgc ccaacaagct ggttaagtgc 1440

atcgttcctg ataggtcaat tttccaaacc acggaccagg aaagaaaatt gctcaatcca 1500

gtggggttgc aagacaagat agatcttgtg gtagccaagt atcccatggg aagaagcttt 1560

gtcagagcca gtggtacgga ggatgcggtg agggtttatg cggaatgtaa ggactcctct 1620

aagttaggtc aatttgtgga cgaagtggtg gagcacgtta aggcatctgc ttga 1674

<210> 86
<211> 557
<212> PRT
<213> Saccharomyces cerevisiae

<220>
<223> amino acid sequence of phosphoacetylglucosamine mutase (PCM1)
      originating from Saccharomyces cerevisiae

<400> 86
Met Lys Val Asp Tyr Glu Gln Leu Cys Lys Leu Tyr Asp Asp Thr Cys
1               5                   10                  15
Arg Thr Lys Asn Val Gln Phe Ser Tyr Gly Thr Ala Gly Phe Arg Thr
            20                  25                  30
Leu Ala Lys Asn Leu Asp Thr Val Met Phe Ser Thr Gly Ile Leu Ala
        35                  40                  45
Val Leu Arg Ser Leu Lys Leu Gln Gly Gln Tyr Val Gly Val Met Ile
    50                  55                  60
Thr Ala Ser His Asn Pro Tyr Gln Asp Asn Gly Val Lys Ile Val Glu
65                  70                  75                  80
Pro Asp Gly Ser Met Leu Leu Ala Thr Trp Glu Pro Tyr Ala Met Gln
                85                  90                  95
Leu Ala Asn Ala Ala Ser Phe Ala Thr Asn Phe Glu Glu Phe Arg Val
            100                 105                 110
Glu Leu Ala Lys Leu Ile Glu His Glu Lys Ile Asp Leu Asn Thr Thr
        115                 120                 125
Val Val Pro His Ile Val Val Gly Arg Asp Ser Arg Glu Ser Ser Pro
    130                 135                 140
Tyr Leu Leu Arg Cys Leu Thr Ser Ser Met Ala Ser Val Phe His Ala
145                 150                 155                 160
Gln Val Leu Asp Leu Gly Cys Val Thr Thr Pro Gln Leu His Tyr Ile
                165                 170                 175
Thr Asp Leu Ser Asn Arg Arg Lys Leu Glu Gly Asp Thr Ala Pro Val
            180                 185                 190
Ala Thr Glu Gln Asp Tyr Tyr Ser Phe Phe Ile Gly Ala Phe Asn Glu
        195                 200                 205
Leu Phe Ala Thr Tyr Gln Leu Glu Lys Arg Leu Ser Val Pro Lys Leu
    210                 215                 220
Phe Ile Asp Thr Ala Asn Gly Ile Gly Gly Pro Gln Leu Lys Lys Leu
225                 230                 235                 240

Leu Ala Ser Glu Asp Trp Asp Val Pro Ala Glu Gln Val Glu Val Ile
                245                 250                 255
Asn Asp Arg Ser Asp Val Pro Glu Leu Leu Asn Phe Glu Cys Gly Ala
            260                 265                 270
Asp Tyr Val Lys Thr Asn Gln Arg Leu Pro Lys Gly Leu Ser Pro Ser
        275                 280                 285
Ser Phe Asp Ser Leu Tyr Cys Ser Phe Asp Gly Asp Ala Asp Arg Val
    290                 295                 300
Val Phe Tyr Tyr Val Asp Ser Gly Ser Lys Phe His Leu Leu Asp Gly
305                 310                 315                 320
Asp Lys Ile Ser Thr Leu Phe Ala Lys Phe Leu Ser Lys Gln Leu Glu
                325                 330                 335
Leu Ala His Leu Glu His Ser Leu Lys Ile Gly Val Val Gln Thr Ala
            340                 345                 350
Tyr Ala Asn Gly Ser Ser Thr Ala Tyr Ile Lys Asn Thr Leu His Cys
        355                 360                 365
Pro Val Ser Cys Thr Lys Thr Gly Val Lys His Leu His His Glu Ala
    370                 375                 380
Ala Thr Gln Tyr Asp Ile Gly Ile Tyr Phe Glu Ala Asn Gly His Gly
385                 390                 395                 400
Thr Ile Ile Phe Ser Glu Lys Phe His Arg Thr Ile Lys Ser Glu Leu
                405                 410                 415
Ser Lys Ser Lys Leu Asn Gly Asp Thr Leu Ala Leu Arg Thr Leu Lys
            420                 425                 430
Cys Phe Ser Glu Leu Ile Asn Gln Thr Val Gly Asp Ala Ile Ser Asp
        435                 440                 445
Met Leu Ala Val Leu Ala Thr Leu Ala Ile Leu Lys Met Ser Pro Met
    450                 455                 460
Asp Trp Asp Glu Glu Tyr Thr Asp Leu Pro Asn Lys Leu Val Lys Cys
465                 470                 475                 480
Ile Val Pro Asp Arg Ser Ile Phe Gln Thr Thr Asp Gln Glu Arg Lys
                485                 490                 495
Leu Leu Asn Pro Val Gly Leu Gln Asp Lys Ile Asp Leu Val Val Ala
            500                 505                 510
Lys Tyr Pro Met Gly Arg Ser Phe Val Arg Ala Ser Gly Thr Glu Asp
        515                 520                 525
Ala Val Arg Val Tyr Ala Glu Cys Lys Asp Ser Ser Lys Leu Gly Gln
    530                 535                 540
Phe Cys Asp Glu Val Val Glu His Val Lys Ala Ser Ala
545                 550                 555

<210> 87
<211> 574
<212> DNA
<213> other sequences

<220>
<223> nucleic acid sequence of promoter pTDH3

<400> 87
ctgctgtaac ccgtacatgc ccaaaatagg gggcgggtta cacagaatat ataacatcgt      60

aggtgtctgg gtgaacagtt tattcctggc atccactaaa tataatggag cccgcttttt     120

aagctggcat ccagaaaaaa aaagaatccc agcaccaaaa tattgttttc ttcaccaacc     180

atcagttcat aggtccattc tcttagcgca actacagaga acaggggcac aaacaggcaa     240

aaaacgggca caacctcaat ggagtgatgc aacctgcctg gagtaaatga tgacacaagg     300

caattgaccc acgcatgtat ctatctcatt ttcttacacc ttctattacc ttctgctctc     360

tctgatttgg aaaaagctga aaaaaaaggt tgaaaccagt tccctgaaat tattccccta 420

cttgactaat aagtatataa agacggtagg tattgattgt aattctgtaa atctatttct 480

taaacttctt aaattctact tttatagtta gtcttttttt tagttttaaa acaccaagaa 540

cttagtttcg aataaacaca cataaacaaa caaa 574

<210> 88
<211> 585
<212> DNA
<213> other sequences

<220>
<223> nucleic acid sequence of promoter pTDH3.Sk

<400> 88
caattcatcg gcccttttag cggctacccg cgccatctaa atgatagggc gggtgacact 60

atggtaaatc ccataattag gtgtctgggt gagtggttct gatgccggca tccactaaat 120

atattggagc ccatttttta cgcgggcttc cagaaaaaaa gagaatccca gcaccaaaag 180

gtggttctct tcaccaacca tcagatcata ggtccacaac cacacataac aggggcacaa 240

aaaggcaaaa aacggacata acctcaatgg agtgatgcaa attgactgga gcaaaagctg 300

acacaaggca ttgattgacc tacgcatgta tctgtattct tttcttacac cttctattac 360

cttctaactc tttgggttgg aaaaaactga aaaaaaaggt tgggacctgg ttcccccaag 420

ttgtccccct acttggttat taaatatata aagacagcaa gtgttgatta taatcttgta 480

aatctatagt tcttaatcta tacttctatt tatattttaa attagtcttt ttatttccaa 540

gtccccaaga acttagtttc gaataaacac acacaaataa acaca 585

<210> 89
<211> 700
<212> DNA
<213> other sequences

<220>
<223> nucleic acid sequence of promoter pTDH3-1.sba

<400> 89
gcagcgcttc ttccgctcta gtttttatag ttattattac taccacctta aaaatacgta 60

aatactcaaa atagtagtga tattcccaac cttattcatc caaggcacat catcatcatc 120

agccattcat ctttcacctg ccattagtaa cccgtcttct cattgagcgg gttacggcag 180

ccacaggcca cattccgaat gtctgggtga gcggtccctt ttccagcatc cactaaatat 240

ctccgatccc gctttttaat ctggcttcct gaaaaaaaga gaatcccagc accaaaaaat 300

ggctctcttc accaaccatc agatcatagg tcccattctc ttaccgcaac cgtacagaac 360

aggggaaaac gggtacaacc tcaatggagt gatgcaaact gactggagca aaaagctgac 420

acaaggcaat cgacctacgt gtctgtctat tttctcacac cttctattac cttctaactc 480

tctgggttgg aaaaaactga aaaaaaggtt gagaccagtt tccacaaatc atccccctgt 540

ttgattaata aatatataaa gacgacaact atcgatcata aactcataaa actataactc 600

ctttacactt cttattttat agttattcta ttttaattct tattgatttt aaaaccccaa 660

gaacttagtt tcgaaaacac acacacacaa acaattaaaa 700

<210> 90
<211> 602
<212> DNA
<213> other sequences

<220>
<223> nucleic acid sequence of promoter pTDH3.Sar

<400> 90
gagctaaata tcagcccttc gggtcctgcc tgctacccgg tcctgttcga ataaaaacgc 60

gggtaacacg acccagtaac acctgtcgtt gggtgtctgg gtcagaagtt ctgataccgg 120

cttccactaa atagattggg ttccgctctt tacgctggct tcctgaaaaa agagattccg 180

ggcaccaaaa aattggtctc tttgccaacc atcagatcat aggtccattc tcttaccata 240

accacacagg ataggggcac cacaggcgaa aatgggcaca aaatctcaat ggagtgatgc 300

aaattagctg gaacaaaagc tgacacaagg caattaacct gcgcatgtat ccatctcctt 360

ttcttacacc ttctcttacg ttctaactgt ttgggttgga aaaattaaaa aaaaaaggtt 420

gagaccagtt tccccaaatc gtccccctac ttgattcata aatatataaa gacgacaact 480

attgattata atcttgtaaa tctataactc tttactttct cctatttata atttaactta 540

atctttttag atttaaaacc ccaagaactt agtttcgaac aaacacacac aaataaacaa 600

aa 602

<210> 91
<211> 550
<212> DNA
<213> other sequences

<220>
<223> nucleic acid sequence of promoter pENO2

<400> 91
cgctcagcat ctgcttcttc ccaaagatga acgcggcgtt atgtcactaa cgacgtgcac 60

caacttgcgg aaagtggaat cccgttccaa aactggcatc cactaattga tacatctaca 120

caccgcacgc cttttttctg aagcccactt tcgtggactt tgccatatgc aaaattcatg 180

aagtgtgata ccaagtcagc atacacctca ctagggtagt ttctttggtt gtattgatca 240

tttggttcat cgtggttcat taattttttt tctccattgc tttctggctt tgatcttact 300

atcatttgga tttttgtcga aggttgtaga attgtatgtg acaagtggca ccaagcatat 360

ataaaaaaaa aaagcattat cttcctacca gagttgattg ttaaaaacgt atttatagca 420

aacgcaattg taattaattc ttattttgta tcttttcttc ccttgtctca atcttttatt 480

tttattttat ttttcttttc ttagtttctt tcataacacc aagcaactaa tactataaca 540

tacaataata 550

<210> 92
<211> 598
<212> DNA
<213> other sequences

<220>
<223> nucleic acid sequence of promoter pTEF3

<400> 92
ggctgataat agcgtataaa caatgcatac tttgtacgtt caaaatacaa tgcagtagat 60

atatttatgc atattacata taatacatat cacataggaa gcaacaggcg cgttggactt 120

ttaattttcg aggaccgcga atccttacat cacacccaat cccccacaag tgatccccca 180

cacaccatag cttcaaaatg tttctactcc ttttttactc ttccagattt tctcggactc 240

cgcgcatcgc cgtaccactt caaaacaccc aagcacagca tactaaattt cccctctttc 300

ttcctctagg gtgtcgttaa ttacccgtac taaaggtttg gaaaagaaaa aagagaccgc 360

ctcgtttctt tttcttcgtc gaaaaaggca ataaaaattt ttatcacgtt tctttttctt 420

gaaaattttt ttttttgatt tttttctctt tcgatgacct cccattgata tttaagttaa 480

taaacggtct tcaatttctc aagtttcagt ttcatttttc ttgttctatt acaacttttt 540

ttacttcttg ctcattagaa agaaagcata gcaatctaat ctaagtttta attacaaa 598

<210> 93
<211> 383
<212> DNA
<213> other sequences

<220>
<223> nucleic acid sequence of promoter pTEF1

<400> 93
gtttagcttg cctcgtcccc gccgggtcac ccggccagcg acatggaggc ccagaatacc 60

ctccttgaca gtcttgacgt gcgcagctca ggggcatgat gtgactgtcg cccgtacatt 120

tagcccatac atccccatgt ataatcattt gcatccatac attttgatgg ccgcacggcg 180

cgaagcaaaa attacggctc ctcgctgcag acctgcgagc agggaaacgc tcccctcaca 240

gacgcgttga attgtcccca cgccgcgccc ctgtagagaa atataaaagg ttaggatttg 300

ccactgaggt tcttctttca tatacttcct tttaaaatct tgctacgata cagttctcac 360

atcacatccg aacataaaca acc 383

<210> 94
<211> 383
<212> DNA
<213> other sequences

<220>
<223> nucleic acid sequence of promoter pTEF1.ago

<400> 94
gtttagcttg cctcgtcccc gccgggtcac ccggccagcg acatggaggc ccagaatacc 60

ctccttgaca gtcttgacgt gcgcagctca ggggcatgat gtgactgtcg cccgtacatt 120

tagcccatac atccccatgt ataatcattt gcatccatac attttgatgg ccgcacggcg 180

cgaagcaaaa attacggctc ctcgctgcag acctgcgagc agggaaacgc tcccctcaca 240

gacgcgttga attgtcccca cgccgcgccc ctgtagagaa atataaaagg ttaggatttg 300

ccactgaggt tcttctttca tatacttcct tttaaaatct tgctacgata cagttctcac 360

atcacatccg aacataaaca acc 383

<210> 95
<211> 498
<212> DNA
<213> other sequences

<220>
<223> nucleic acid sequence of promoter pTEF1.Sba

<400> 95
cgccaacaaa ccttcgaaca ctttaatttt cgaggaccgc agatcctcac atcacaccca 60

cacccaagac tgcttccccc acacaccctg catctgtaca ctttcttctg ctctgttttt 120

ctctccggcg ttctctcggg tcgcccgcat cgccgcgccg gctggaaccg cccacgcacc 180

gcatattgca aatcgcctgc cccctcttgc tcctttttga gggcgcgccg ttacccgcgc 240

ccagggtccg gaaaaagaaa caaggctcta ccgcgtttct ttttccttgt cgaaaaaggc 300

aaaaatgaaa atttttatca cgtttctttt tttttgaaaa attttttttt tggttttttt 360

tctttcgatg gcctcccatt gatatttaag ttaataaatg gttttcagtt ttcaagtttc 420

agtttgtgtt cttctttgct aactttcact tacacctcga aagaaagtat agcaatctaa 480

tcttagtttt aattacaa 498

<210> 96
<211> 700
<212> DNA

<213> other sequences

<220>
<223> nucleic acid sequence of promoter pPDC1

<400> 96
ttatttacct atctctaaac ttcaacacct tatatcataa ctaatatttc ttgagataag 60

cacactgcac ccataccttc cttaaaaacg tagcttccag tttttggtgg ttccggcttc 120

cttcccgatt ccgcccgcta aacgcatatt tttgttgcct ggtggcattt gcaaaatgca 180

taacctatgc atttaaaaga ttatgtatgc tcttctgact tttcgtgtga tgaggctcgt 240

ggaaaaaatg aataatttat gaatttgaga acaattttgt gttgttacgg tattttacta 300

tggaataatc aatcaattga ggattttatg caaatatcgt ttgaatattt ttccgaccct 360

ttgagtactt ttcttcataa ttgcataata ttgtccgctg cccctttttc tgttagacgg 420

tgtcttgatc tacttgctat cgttcaacac caccttattt tctaactatt ttttttttag 480

ctcatttgaa tcagcttatg gtgatggcac atttttgcat aaacctagct gtcctcgttg 540

aacataggaa aaaaaaatat ataaacaagg ctctttcact ctccttgcaa tcagatttgg 600

gtttgttccc tttattttca tatttcttgt catattcctt tctcaattat tattttctac 660

tcataacctc acgcaaaata acacagtcaa atcaatcaaa 700

<210> 97
<211> 924
<212> DNA
<213> other sequences

<220>
<223> nucleic acid sequence of promoter pCCW12

<400> 97
aactgtaaac aggtacaatg cggacccttt tgccacaaaa catacatcat tcattgccgg 60

aaaaagaaag aagtgaagac agcagtgcag ccagccatgt tgcgccaatc taattataga 120

tgctggtgcc ctgaggatgt atctggagcc agccatggca tcatgcgcta ccgccggatg 180

taaaatccga cacgcaaaag aaaaccttcg aggttgcgca cttcgcccac ccatgaacca 240

cacggttagt ccaaaagggg cagttcagat tccagatgcg ggaattagct tgctgccacc 300

ctcacctcac taacgctgcg gtgtgcggat acttcatgct atttatagac gcgcgtgtcg 360

gaatcagcac gcgcaagaac caaatgggaa aatcggaatg ggtccagaac tgctttgagt 420

gctggctatt ggcgtctgat ttccgttttg ggaatccttt gccgcgcgcc cctctcaaaa 480

ctccgcacaa gtcccagaaa gcgggaaaga aataaaacgc caccaaaaaa aaaaataaaa 540

gccaatcctc gaagcgtggg tggtaggccc tggattatcc cgtacaagta tttctcagga 600

gtaaaaaaac cgtttgtttt ggaattcccc atttcgcggc cacctacgcc gctatctttg 660

caacaactat ctgcgataac tcagcaaatt ttgcatattc gtgttgcagt attgcgataa 720

tgggagtctt acttccaaca taacggcaga aagaaatgtg agaaaatttt gcatcctttg 780

cctccgttca agtatataaa gtcggcatgc ttgataatct ttctttccat cctacattgt 840

tctaattatt cttattctcc tttattcttt cctaacatac caagaaatta atcttctgtc 900

attcgcttaa acactatatc aata 924

<210> 98
<211> 976
<212> DNA
<213> other sequences

<220>
<223> nucleic acid sequence of promoter pCCW12.Sm

<400> 98
cgttgtgctg tagtgaagga agactaaaaa ggataatcac agttgtaaag aggtataatg 60

cggacccttt tgccacaaaa cacacatctt tcgtttccaa aataagaaag aagaaagcaa 120

aaagattagc agccacgttg ctgcgatcta attatagacg gtggcgtcat cattctcacc 180

caagattgtg tcttgaacct gccacgggtc ctgcgttatc gccggatgta aaactagaca 240

tgcaaaaaaa ggaccttcca ggtagcgtgc tccacaccac ccatgaccac cacagttagt 300

ccaaaagagg cagcaccact tcccgatggg ggaattagat tgctgccacc ctcacctcac 360

taatgctgcg gtgtgcggat atgccctgct atatatagct ccgcgttttt gaaccagcac 420

agcgcgagca ccaaaaagga aaatcgcata ggcccagaac tgatttcagc acgggctatt 480

ggcgttgggt ttccgttctg ggaaaccttc gccgcgtccc cctcacaaac ctccgcacaa 540

gttcgagcaa gcgggaaaaa acgaaaaacg ccattaatac taaataaagc aaatcctcga 600

agcgtgggtg gcaagcccct ggatttttcc gcacaagtac tcttctcagg agtaaaaaaa 660

cccgtttgtt ttggaattcc ccatttcgcg gccatctacg ccggtatctt cgcaatatct 720

atcagcgata actcagcaat tttaatattc gtgttgcagt gctgcgatag cgggagtctt 780

gtttgtaaca taacggcaga aaggaatgag agaaaatttt ccattctttg gcctccgttc 840

aagtatataa agccggcatg cttggtaatc tttctctctc ttctgtattg tttctataat 900

acttttatct tctaattatt ttctgaaaaa accaagaaat taatcttctg tcattcgctt 960

aaacactata tcaata 976

<210> 99
<211> 750
<212> DNA
<213> other sequences

<220>
<223> nucleic acid sequence of promoter pCCW12.Sk

<400> 99
tgtaaaatcc tacacgcaaa aaacctcttg ggttgcgcgt tttccaccac ccacgacccg      60

cacaatcaat ccaaaagggg caacgccagt tcccaatgcg ggaattagct cctcaccccc     120

ctcacccgct aacgctgcgg tgtgcggaca cgcagcagta tttatagatc ctcgtgttgg     180

aaccagcccg cgtgagcacc aaattggaaa gtcgcaatgg gcccagaacc gctttcagta     240

ctgggccatt ggcgtctagt ttccgttttg agcgtccttc gccgcgtctc tctgtgaaat     300

ccccgcacaa gtctgagcag gcaaaaaaaa aaaacgccac caaaaataaa taaagccaat     360

cctcgaagga tgagtaggaa aggaagcccc tggttttttc ccgcacgaat atttttcagg     420

agtgaaaaaa tccgtttgtt ttggaattcc ccatttcgcg ctcacctacg ccggtatctt     480

tggaacaact atcagcgata actcagcaaa atttgcatat ccgtgctgca atagtgcgat     540

agtgggattg ggagtcttgt tgcatcataa cggcagaaag gaatgaataa aaattttccg     600

ttctttgtct ccgttcaagt atataaagtc ggcatgcttg attatctttt cttctcttct     660

actacatttc tatttcactt tctactctat tcttccctga aaaacccaag aaaataatct     720

tctgtcattc gcttaaacaa tatatcaaaa                                      750

<210> 100
<211> 750
<212> DNA
<213> other sequences

<220>
<223> nucleic acid sequence of promoter pCCW12.Sba

<400> 100
accatgccac ggtgctggcc ccacttccac ccacgacccg cacggttggc ccgaaagggg      60

caacaccagt tcccaatacg gaaattagtc cgccaccacc ctcacctcgc tagagctgcg     120

gtgtgcgggc gtgcatcgct atttatagac atgcctgctg gcgtcaccgc gcgcgagcac     180

caaacaggaa aatcgcactg ggcccagaac cacgctatgc gctgggccga tggcgtccgg     240

tttccctttg ggagccccct gccacgttcg cctaacaaat ccccgcaccg gcttgagaaa     300

aaagcgaaaa gcgaaaaaaa aaaatcaacg ccaccaaaat taaaaaaaaa gagccatcct     360

cgaagggtga atagtagccc ctgacttttc ccgcacagac agacaccttt caggagtgaa     420

caaaaaagca gtttgttttg gaattccccc atttcgcggt ggcctgcgca ggtatctctg     480

cgtcaactat cagcgataac tcagcaaatt ttgcatattc gtgttgcgat actacgataa     540

tgggagtctg tcgcctaata acggcaacaa ggaatgagag agaaaaattt tcttcattct     600

ccagctcccg ttcaagtata taaggtcggc atggtcgatt gtctttcctt ctcttcagtt     660

acgtctctct atttacatta ttcttatttt tatttaataa aaaccccaag aaattaatct 720

tctgtcattc gcttaaacac tatatcaaaa 750

<210> 101
<211> 750
<212> DNA
<213> other sequences

<220>
<223> nucleic acid sequence of promoter pCCW12.Sar

<400> 101
   aaaaaacaac cttctgccaa cctgcgtgct tctcaccacc catgacccac acaattgacc 60
   cgaatggggc aactccagtt cccaatacgg gaattaactc gccaccatat ttaccgcgtt 120
   gaagctgtgg tgtgcggaca ctccgtacta tttatagacc cacgcggtgg aaccagcacg 180
   cgcgcgcact aaacaggaaa atcgcattga gtccagaacc gccaccagca cttggccatt 240
   ggcgtctaat ttccgttttc ggcgcccctc accgcgtcct tctaacaaag cgcgcacaag 300
   cttgagcaag tgaaaagaaa attaaaaata aaaaaccgcc accaaaacaa ataaagcaat 360
   cttcgaagtg tgggttggtg ggaagcccct ggcttttccc gcaccagtcg ttttcaggag 420
   taaaaaaata cccgtttgtt ttggaattcc ccatttcgcg gcgacctgcg ccggtatctt 480
   tgcaacaact atttgcgata actcagcaaa atttgcatat tcgtgttggg atattgcgat 540
   agtgggagtc ttgttgcata ataacggtaa aaagaagtga aggaaaaaaa tttgcatcct 600
   ttagtctcag ttcaagtata taaagtcggg atattcaatt atctttcttt ctcttgctca 660
   aaggtttcta tatttttttt atagtatttc ttttgttata aaataccaag aaattaatct 720
   tctgtcattc gcttaaatac tacatcaata 750

<210> 102
<211> 500
<212> DNA
<213> other sequences

<220>
<223> nucleic acid sequence of promoter pNUP57

<400> 102
tcatctgcgc aatgactatc aagaccttct gcaagaattt caaatctcac tgaaaatctt 60

gaccgaaaag tgtcttgaaa acccatcaag cctgcaaaac ctatctttga cattagtctc 120

cattataaaa acggcatagt tgggagaaaa ctttccatac ttcaattgtg gactgatata 180

agtattttag ttttgcccgc atgatcatcc cacatggcta cagcagttct ctcataggaa 240

aatagtacaa tagctacgtg atataatcta aataattgtt gccaatgtgt aattatatca 300

ttttgaacgt acgcgaaatg gattattttc aaaaattttg tttcttgaaa tgagtaaaag 360

caaaagtcca actctccaag tcgatgtaaa caactttttg ccaaagggac tgaaagacta 420

aatcgaggat tatcccgttc aaactattcc agaaacgctc gttagtaaca aaagacatac 480

cttgttgacc aattgatcac 500

<210> 103
<211> 523
<212> DNA
<213> other sequences

<220>
<223> nucleic acid sequence of promoter pCCW10.ago

<400> 103
ggtaccacgg caacctcgtt cgctgttcat ccccttcgtc acacaggacg ttggatgccg 60

taagcagcgt tgcttttgat cctcaggatc ggccgggtaa cccgcggctg cttctatttt 120

agtattcata tctcaagcac atccattccg gccgtttggg ggcgccgccg cactcgtgtc 180

cattcctacc gtggcactta gggctatcct gtcggagcgc cccgccgacc gccttatcgg 240

caccaaaagt agaagccccg gccccgcgtg gctcagactc accatcggtg ctatttactt 300

ttcgatcaga tcgcggcgcg cggtggccgg catttccgga agcggccacg gagcagaggt 360

ggcgcattcg aatcgcatac gtcttcgcca cgccggaaaa aaaattttcg gctatataag 420

gagaggcggc cgtcttgctg caggcagttt cactttctct aaaaccaaag aacatcgatt 480

tctttagtca ctcgcttcct tacaccgaac tcgaggcggc cgc 523

<210> 104
<211> 943
<212> DNA
<213> other sequences

<220>
<223> nucleic acid sequence of promoter pCWP2

<400> 104
ctagcctccc ctttttattt tgtgcggtca ccgcaaggga caaagctttt cttagaaaac 60

cgtctgagaa gcataacgta cgccatcccc tagacatatt aataatgcta cagatactat 120

gctgctcgtc tttttttgac gaccctttta ttgcaatgtg caactaatgg caaacaacca 180

catagtatca cagtattaca ttgcctccac cgatgcggat gttagggcgc caagtctgtc 240

atgaagcatg ttcctgtcat aatcttgtat gcaaaatacc gcgttctgcg ccactgatat 300

gctaggcagc agcaacctat gcagaagatt gcttttccca cgcctgtttt acgtctccag 360

ggcacttgaa acaatgcagc gatcgccgcc acaacacgcc aaagagaagc gaaagtgggc 420

ctgggcggcc tcagtttcgg cagaggtaaa caacacgaac tgaactgcct tagctccgaa 480

gggcaattcc acaggcactc cgcggggccc ggccaaggcc caaaaggcgt ggaatatgcg 540

cgttttgggg ccataacacc cagtaccacg gccggaacgg gccatataat aagtttttca 600

ctctcaagaa tggtaaacgt aaataggaac atcccactac cctagaaatt gcggaaattt 660

cgcgcttatc attagaaaat ctggaaccgt cctttttcct ctttcttgca tttccctttc 720

cgtattattg ccattcttta actgcatttg gggaaccgta gaccaaaagc caaacagaga 780

aatgtaacgt tctaaaaaaa aaacaacgaa aaaattgaaa aataagatac aataatcgta 840

tataaatcag gcttcttgtt catcattttc aattctcttc ttgccatccc ttttcctatc 900

tttgttcttt tcttctcata atcaagaata aataacttca tca 943

<210> 105
<211> 650
<212> DNA
<213> other sequences

<220>
<223> nucleic acid sequence of promoter pFBA1

<400> 105
acgcaagccc taagaaatga ataacaatac tgacagtact aaataattgc ctacttggct 60

tcacatacgt tgcatacgtc gatatagata ataatgataa tgacagcagg attatcgtaa 120

tacgtaatag ttgaaaatct caaaaatgtg tgggtcatta cgtaaataat gataggaatg 180

ggattcttct atttttcctt tttccattct agcagccgtc gggaaaacgt ggcatcctct 240

ctttcgggct caattggagt cacgctgccg tgagcatcct ctctttccat atctaacaac 300

tgagcacgta accaatggaa aagcatgagc ttagcgttgc tccaaaaaag tattggatgg 360

ttaataccat ttgtctgttc tcttctgact ttgactcctc aaaaaaaaaa aatctacaat 420

caacagatcg cttcaattac gccctcacaa aaactttttt ccttcttctt cgcccacgtt 480

aaattttatc cctcatgttg tctaacggat ttctgcactt gatttattat aaaaagacaa 540

agacataata cttctctatc aatttcagtt attgttcttc cttgcgttat tcttctgttc 600

ttctttttct tttgtcatat ataaccataa ccaagtaata catattcaaa 650

<210> 106
<211> 996
<212> DNA
<213> other sequences

<220>
<223> nucleic acid sequence of promoter pCCW120.Sm

<400> 106
cgttgtgctg tagtgaagga agactaaaaa ggataatcac agttgtaaag aggtataatg 60

cggacccttt tgccacaaaa cacacatctt tcgtttccaa aataagaaag aagaaagcaa 120

aaagattagc agccacgttg ctgcgatcta attatagacg gtggcgtcat cattctcacc 180

caagattgtg tcttgaacct gccacgggtc ctgcgttatc gccggatgta aaactagaca 240

tgcaaaaaaa ggaccttcca ggtagcgtgc tccacaccac ccatgaccac cacagttagt 300

ccaaaagagg cagcaccact tcccgatggg ggaattagat tgctgccacc ctcacctcac 360

taatgctgcg gtgtgcggat atgccctgct atatatagct ccgcgttttt gaaccagcac 420

agcgcgagca ccaaaaagga aaatcgcata ggcccagaac tgatttcagc acgggctatt 480

ggcgttgggt ttccgttctg ggaaaccttc gccgcgtccc cctcacaaac ctccgcacaa 540

gttcgagcaa gcgggaaaaa acgaaaaacg ccattaatac taaataaagc aaatcctcga 600

agcgtgggtg gcaagcccct ggatttttcc gcacaagtac tcttctcagg agtaaaaaaa 660

cccgtttgtt ttggaattcc ccatttcgcg gccatctacg ccggtatctt cgcaatatct 720

atcagcgata actcagcaat tttaatattc gtgttgcagt gctgcgatag cgggagtctt 780

gtttgtaaca taacggcaga aaggaatgag agaaaatttt ccattctttg gcctccgttc 840

aagtatataa agccggcatg cttggtaatc tttctctctc ttctgtattg tttctataat 900

acttttatct tctaattatt ttctgaaaaa accaagaaat taatcttctg tcattcgctt 960

aaacactata tcaatactcg aaatccaagg tggtgt 996

<210> 107
<211> 505
<212> DNA
<213> other sequences

<220>
<223> nucleic acid sequence of promoter pCUP1

<400> 107
cggcaaactt caacgatttc tatgatgcat tttataatta gtaagccgat cccattaccg 60

acatttgggc gctatacgtg catatgttca tgtatgtatc tgtatttaaa acacttttgt 120

attatttttc ctcatatatg tgtataggtt tatacggatg atttaattat tacttcacca 180

ccctttattt caggctgata tcttagcctt gttactagtt agaaaaagac atttttgctg 240

tcagtcactg tcaagagatt cttttgctgg catttcttct agaagcaaaa agagcgatgc 300

gtcttttccg ctgaaccgtt ccagcaaaaa agactaccaa cgcaatatgg attgtcagaa 360

tcatataaaa gagaagcaaa taactccttg tcttgtatca attgcattat aatatcttct 420

tgttagtgca atatcatata gaagtcatcg aaatagatat taagaaaaac aaactgtaca 480

atcaatcaat caatcatcac ataaa 505

<210> 108
<211> 510

<212> DNA
<213> other sequences


<220>
<223> nucleic acid sequence of promoter pMET6

<400> 108
ccacaggaaa tatttcacgt gacttacaaa cagagtcgta cgtcaggacc ggagtcaggt      60

gaaaaaatgt gggccggtaa agggaaaaaa ccagaaacgg gactactatc gaactcgttt     120

agtcgcgaac gtgcaaaagg ccaatatttt tcgctagagt catcgcagtc atggcagctc     180

tttcgctcta tctcccggtc gcaaaactgt ggtagtcata gctcgttctg ctcaattgag     240

aactgtgaat gtgaatatgg aacaaatgcg atagatgcac taatttaagg gaagctagct     300

agttttccca actgcgaaag aaaaaaagga aagaaaaaaa aattctatat aagtgataga     360

tatttccatc tttactagca ttagtttctc ttttacgtat tcaatatttt tgttaaactc     420

ttcctttatc ataaaaaagc aagcatctaa gagcattgac aacactctaa gaaacaaaat     480

accaatataa tttcaaagta catatcaaaa                                      510


<210> 109
<211> 686
<212> DNA
<213> other sequences


<220>
<223> nucleic acid sequence of promoter pMET25

<400> 109
ttacattatc aatccttgcg tttcagcttc cactaattta gatgactatt tctcatcatt      60

tgcgtcatct tctaacaccg tatatgataa tatactagta acgtaaatac tagttagtag     120

atgatagttg atttttattc caacactaag aaataatttc gccatttctt gaatgtattt     180

aaagatattt aatgctataa tagacattta aatccaattc ttccaacata caatgggagt     240

ttggccgagt ggtttaaggc gtcagattta ggtggattta acctctaaaa tctctgatat     300

cttcggatgc aagggttcga atcccttagc tctcattatt ttttgctttt tctcttgagg     360

tcacatgatc gcaaaatggc aaatggcacg tgaagctgtc gatattgggg aactgtggtg     420

gttggcaaat gactaattaa gttagtcaag gcgccatcct catgaaaact gtgtaacata     480

ataaccgaag tgtcgaaaag gtggcacctt gtccaattga acacgctcga tgaaaaaaat     540

aagatatata taaggttaag taaagcgtct gttagaaagg aagtttttcc tttttcttgc     600

tctcttgtct tttcatctac tatttccttc gtgtaataca gggtcgtcag atacatagat     660

acaattctat tacccccatc cataca                                          686


<210> 110

<211> 363
<212> DNA
<213> other sequences

<220>
<223> nucleic acid sequence of promoter pSAM1

<400> 110
gaaacggacg taagacggaa atagaatttg aagataaagt tatatatcac tacacacgaa 60

tactttcttt tttttttttc acaggaaaac tgtggtggcg cccttgccta ctagtgcatt 120

tcttttttcg ggttcttgtc tcgacgaaat tttagcctca tcgtagtttt tcactctggt 180

atcgatgaaa aagggaagag taaaaagttt tccgtttagt acttaatggg attggtttgg 240

gacgtatata tcgactggtg ttgtctgtta ttcatcgttg tttttcggtt agcttcgaaa 300

aaaaaataga gtaaaaacca ggaatttacc ctaaaaacaa gaaaaaataa gataaacgaa 360

aat 363

<210> 111
<211> 300
<212> DNA
<213> other sequences

<220>
<223> nucleic acid sequence of terminator tTPI1

<400> 111
gattaatata attatataaa aatattatct tcttttcttt atatctagtg ttatgtaaaa 60

taaattgatg actacggaaa gcttttttat attgtttctt tttcattctg agccacttaa 120

atttcgtgaa tgttcttgta agggacggta gatttacaag tgatacaaca aaaagcaagg 180

cgctttttct aataaaaaga agaaaagcat ttaacaattg aacacctcta tatcaacgaa 240

gaatattact ttgtctctaa atccttgtaa aatgtgtacg atctctatat gggttactca 300

<210> 112
<211> 299
<212> DNA
<213> other sequences

<220>
<223> nucleic acid sequence of terminator tMET25

<400> 112
gtgtgcgtaa tgagttgtaa aattatgtat aaacctactt tctctcacaa gtactatact 60

tttataaaac gaactttatt gaaatgaata tccttttttt cccttgttac atgtcgtgac 120

tcgtactttg aacctaaatt gttctaacat caaagaacag tgttaattcg cagtcgagaa 180

gaaaaatatg gtgaacaaga ctcatctact tcatgagact actttacgcc tcctataaag 240

ctgtcacact ggataaattt attgtaggac caagttacaa aagaggatga tggaggttt     299

<210> 113
<211> 300
<212> DNA
<213> other sequences

<220>
<223> nucleic acid sequence of terminator tDIT1

<400> 113
taaagtaaga gcgctacatt ggtctacctt tttgttcttt tacttaaaca ttagttagtt      60

cgttttcttt ttctcatttt tttatgtttc ccccccaaag ttctgatttt ataatatttt     120

atttcacaca attccattta acagaggggg aatagattct ttagcttaga aaattagtga     180

tcaatatata tttgcctttc ttttcatctt ttcagtgata ttaatggttt cgagacactg     240

caatggccct agttgtctaa gaggatagat gttactgtca aagatgatat tttgaatttc     300

<210> 114
<211> 300
<212> DNA
<213> other sequences

<220>
<223> nucleic acid sequence of terminator tRPL3

<400> 114
gaagttttgt tagaaaataa atcatttttt aattgagcat tcttattcct attttattta      60

aatagtttta tgtattgtta gctacataca acagtttaaa tcaaattttc tttttcccaa     120

gtccaaaatg gaggtttatt ttgatgaccc gcatgcgatt atgttttgaa agtataagac     180

tacatacatg tacatatatt taaacatgta aacccgtcca ttatattgct tactttcttc     240

ttttttgccg ttttgacttg gacctctggt ttgctatttc cttacaatct ttgctacaat     300

<210> 115
<211> 304
<212> DNA
<213> other sequences

<220>
<223> nucleic acid sequence of terminator tRPL3.sm

<400> 115
gaagttttta aagcattttt tagacacttc tcatttttct aagttttttt aaaatagttt      60

tatgtattta ctacgtatca caatttgaaa taattcatct tcccaaaaaa ctaagatttt     120

tatccttgtc acgatccgta accagtttat aatattttag agcttataca cgtacgtata     180

cacacgtgtc ggtacatgag aattacgttc aaaattattc actttttttt tctctgccgt     240

tttacttttg aactctgtct cgctatttcc ttacaatctt cgctacaata ccacttgccc 300

ttgg 304

<210> 116
<211> 299
<212> DNA
<213> other sequences

<220>
<223> nucleic acid sequence of terminator tRPL3.sba

<400> 116
gaagttttct gacaaaaaca taacgttttt tccaatcatt tcttattttt ccggtttatt 60

taaatagttt ttatgtacta ttatacgtat gactatttaa cttaaattct tcctcccaag 120

aaatctccca agtttttcat tatcatggca tacaccacta tcagttacaa aatggtagct 180

caaccatata tatatctcta tatacacata taaatgcaaa caggtccaag tcaccgctca 240

ctgcagtttc ttttgccgtt ttgacttcga tctctgcttg gctattttct cacaatcct 299

<210> 117
<211> 300
<212> DNA
<213> other sequences

<220>
<223> nucleic acid sequence of terminator tRPL41B

<400> 117
gcggattgag agcaaatcgt taagttcagg tcaagtaaaa attgatttcg aaaactaatt 60

tctcttatac aatcctttga ttggaccgtc atcctttcga atataagatt ttgttaagaa 120

tattttagac agagatctac tttatattta atatctagat attacataat ttcctctcta 180

ataaaatatc attaataaaa taaaaatgaa gcgatttgat tttgtgttgt caacttagtt 240

tgccgctatg cctcttgggt aatgctatta ttgaatcgaa gggctttatt atattaccct 300

<210> 118
<211> 301
<212> DNA
<213> other sequences

<220>
<223> nucleic acid sequence of terminator tRPL41B.Sba

<400> 118
gcggattgcg agtgattttt tgagtacaga ccaagtaaaa ttgatctatg cataatcttt 60

tctcatccac aatccctaga atggaccgtc atcctttcta aaatgtttta ttaagaatat 120

ttaaagtgga agtcaggttt atatctatca attaaatatt acataatttc cccttaataa 180

aatactgtta ataaaataac gaaacgttca tagtttttca taaatagtcc tgatgcttac 240

tgcaccggaa cgtactgttt tagtgtattg ctcccttcat tgccattttt cctagttatt 300

g 301

<210> 119
<211> 300
<212> DNA
<213> other sequences

<220>
<223> nucleic acid sequence of terminator tRPL15A

<400> 119
gctggttgat ggaaaatata attttattgg gcaaactttt gtttatctga tgtgttttat 60

actattatct ttttaattaa tgattctata tacaaacctg tatatttttt ctttaaccaa 120

tttttttttt tatagaccta gagctgtact tttattctgc tatcaagcaa acccctaccc 180

cctcttctca atcctcccct caggcagaac ttatctacct gtatcaagga gcggacgagg 240

gagtcctaat tgttctacgt ataccaatgc tagcagctta cataggtggt ggcactacca 300

<210> 120
<211> 299
<212> DNA
<213> other sequences

<220>
<223> nucleic acid sequence of terminator tRPL15A.Sm

<400> 120
gctaattaga accatatata catcgataaa aacacttgaa tgtatttttc tttttcttct 60

tattttttca ttttccttcg ctttctatta gcgtgcgaat ctaaagtgtg cagcgctatc 120

tttgactctt tttttccttt agtgggaacg aacaatatta atcatgccaa gttaagtcat 180

tgaagaagtc agtagtatgt tattcggaga gaagaccctg ctatgtatct cagcctaccc 240

aaaaaatgaa ttgactcctc tcataacttg ttaatattcg gatgagcaca tcacgttcg 299

<210> 121
<211> 300
<212> DNA
<213> other sequences

<220>
<223> nucleic acid sequence of terminator tRPL15A.sba

<400> 121
gctggctgat gaaaaatata gttctgttgg gcaagcattt gtttacctag catctctttt 60

atactattat tatctttata tttgatgatt ttatatacaa gttgtatacc ttttctttaa 120

ccaatttttt tttttctaat ggtgcaccta gaagtacatt ttttctcacc aatagatagt 180

caagatactc ccagcctcta tggcgttacc acggagccga caagggaaag ctgcttatct 240

tacatatgca gatgccaagg cccgttacag gccgccttat tgatgtttag aaatagcttc 300

<210> 122
<211> 300
<212> DNA
<213> other sequences

<220>
<223> nucleic acid sequence of terminator tIDP1

<400> 122
tcgaatttac gtagcccaat ctaccacttt tttttttcat tttttaaagt gttatactta 60

gttatgctct aggataatga actacttttt tttttttttt tttactgtta tcataaatat 120

atatacctta ttgttgtttg caaccgtcgg ttaattcctt atcaaggttc cccaagttcg 180

gatcattacc atcaatttcc aacattttca tgagttcttc ttcttcatta ccgtgtttta 240

gggggctgtt cgcacttcta atagggctat caccaagctg ttctaattcg tccaaaagtt 300

<210> 123
<211> 290
<212> DNA
<213> other sequences

<220>
<223> nucleic acid sequence of terminator tIDP1.Sba

<400> 123
tctgatcttt ggaatgctcc atttactttt ttatttcata tttaggtacg atatatagtt 60

acgcttgaga actatgaact aataacttcc tttttctcta atacatactt acttcagctg 120

atggttgcaa acaacaataa aattccttgt cgaggttccc taaattagga tcattgccat 180

cgacttctaa cattttcatg agatcctcct cttcgttccc attctttaaa agactgtttc 240

cacttctaat agggttatcg cccaattgtt ctagttcgtc tagcaattcg 290

<210> 124
<211> 300
<212> DNA
<213> other sequences

<220>
<223> nucleic acid sequence of terminator tTEF1.sba

<400> 124
ggagattgat aggacttttc tagttgcata tcttttattt ttaaatctta tctattagtt 60

aattttttgt aatttatcct tatatatata gtttggttat tctaaaacat catttcagta 120

tctaaaacct ctcttattca ttaccttttt atttaatggt ttttgctaca ggcaaaaatt    180

taatggtttt tgctacaggc aaaaatcccg ccgtggactt attccacgtt aactcggtta    240

cagggtcatg aaccattttg tcaattatcg aaataacttc ttcaaaagtc cctcttactt    300

<210> 125
<211> 60
<212> DNA
<213> other sequences

<220>
<223> nucleic acid sequence of the secretion sequence added in 5â\200\231

<400> 125
atgcaattta gcacagtcgc atcagtagcc ttcgttgcct tggccaactt cgtggcagca     60

<210> 126
<211> 20
<212> PRT
<213> other sequences

<220>
<223> amino acid sequence of the secretion sequence added in N-ter

<400> 126
Met Gln Phe Ser Thr Val Ala Ser Val Ala Phe Val Ala Leu Ala Asn
1               5                   10                  15
Phe Val Ala Ala
            20

<210> 127
<211> 213
<212> DNA
<213> other sequences

<220>
<223> nucleic acid sequence of the anchoring sequence added in 3â\200\231

<400> 127
ggatccgcca tttctcaaat cactgacggt caaatccaag ctactaccac tgctaccacc     60

gaagctacca ccactgctgc cccatcttcc accgttgaaa ctgtttctcc atccagcacc    120

gaaactatct ctcaacaaac tgaaaatggt gctgctaagg ccgctgtcgg tatgggtgcc    180

ggtgctctag ctgctgctgc tatgttgtta taa                                 213

<210> 128
<211> 68
<212> PRT
<213> other sequences

<220>
<223> amino acid sequence of the anchoring sequence added in C-ter

<400> 128
Ala Ile Ser Gln Ile Thr Asp Gly Gln Ile Gln Ala Thr Thr Thr Ala
1               5                   10                  15
Thr Thr Glu Ala Thr Thr Thr Ala Ala Pro Ser Ser Thr Val Glu Thr
            20                  25                  30
Val Ser Pro Ser Ser Thr Glu Thr Ile Ser Gln Gln Thr Glu Asn Gly
        35                  40                  45
Ala Ala Lys Ala Ala Val Gly Met Gly Ala Gly Ala Leu Ala Ala Ala
    50                  55                  60
Ala Met Leu Leu
65